# EUROPEAN PATENT APPLICATION

(11) **EP 3 929 298 A1**
(43) Date of publication of application: **29.12.2021**
(21) Application number: 21174106.1
(22) Date of filing: 09.03.2016
(51) Int. Cl.: C12N 15/85, C12N 15/09, C12N 5/10

(54) **TOOLS AND METHODS FOR USING CELL DIVISION LOCI TO CONTROL PROLIFERATION OF CELLS**

(30) Priority: 09.03.2015 US 201562130258 P; 09.03.2015 US 201562130270 P
(62) Divisional of application: 16760977.5
(71) Applicant: Sinai Health System, Toronto, ON M5G 1X5 (CA)
(72) Inventor: NAGY, Andras, Toronto, Ontario M6G 4C2 (CA); MONETTI, Claudio, Toronto, Ontario M6P 4B8 (CA); LIANG, Qin, Toronto, Ontario M6E 3P6 (CA)
(74) Representative: Barker Brettell LLP

(57) **Abstract**

The present disclosure provides molecular tools, methods and kits for using cell division loci (CDLs) to control cell proliferation in animal cells. CDLs, as provided herein, are loci whose transcription product(s) are expressed during cell division. CDLs may be genetically modified, as described herein, to comprise a negative selectable marker and/or an inducible activator-based gene expression system, which allows a user to permit, ablate, and/or inhibit proliferation of the genetically modified cell(s) by adding or removing an appropriate inducer.

SEQUENCE LISTING

<110>  Sinai Health System

<120>  TOOLS AND METHODS FOR USING CELL DIVISION LOCI TO CONTROL
       PROLIFERATION OF CELLS

<130>  JA90900P.EPPD1

<140>
<141>  2016-03-09

<150>  EP16760977.5
<151>  2016-03-09

<150>  PCT/CA2016/050256
<151>  2016-03-09

<150>  US 62/130,258
<151>  2015-03-09

<150>  US 62/130,270
<151>  2015-03-09

<160>  152

<170>  PatentIn version 3.5

<210>  1
<211>  562
<212>  DNA
<213>  Artificial Sequence

<220>
<223>  Synthetic DNA

<400>  1
gtcgacggtt ctggcgtgaa acagactttg aattttgacc ttctcaagtt ggcgggagac      60

gtggagtcca acccagggcc catggccagc tacccctgtc accagcacgc cagcgccttc      120

gaccaggccg ccagaagcag gggccacagc aaccggcgga ccgccctgag acccaggcgg      180

cagcaggaag ccaccgaagt ccggctggaa cagaagatgc ccaccctgtt aaccctagaa      240

agatagtctg cgtaaaattg acgcatgcat tcttgaaata ttgctctctc tttctaaata      300

gcgcgaatcc gtcgctgtgc atttaggaca tctcagtcgc cgcttggagc tcccgtgagg      360

cgtgcttgtc aatgcggtaa gtgtcactga ttttgaacta taacgaccgc gtgagtcaaa      420

atgacgcatg attatctttt acgtgacttt taagatttaa ctcatacgat aattatattg      480

ttatttcatg ttctacttac gtgataactt attatatata tattttcttg ttatagatat      540

cgtggcggcc gcattaccgc gg                                               562

<210>  2
<211>  2031
<212>  DNA
<213>  Artificial Sequence

<220>
<223>  Synthetic DNA

<400>  2
gtcgacatta ccgcgggttt tgttacttta tagaagaaat tttgagtttt tgtttttttt      60

taataaataa ataaacataa ataaattgtt tgttgaattt attattagta tgtaagtgta     120

aatataataa aacttaatat ctattcaaat taataaataa acctcgatat acagaccgat     180

aaaacacatg cgtcaatttt acgcatgatt atctttaacg tacgtcacaa tatgattatc     240

tttctagggt taagggtgta catcgacggc ccccacggca tgggcaagac caccaccacc     300

cagctgctgg tggccctggg cagccgggac gacatcgtgt acgtgcccga gcccatgacc     360

tactggcagg tgctgggcgc cagcgagacc atcgccaaca tctacaccac acagcacagg     420

ctggaccagg gcgagatctc tgccggcgac gccgccgtgg tgatgaccag cgcccagatc     480

acaatgggca tgccctacgc cgtgaccgac gccgtgctgg cccctcacgt gggcggcgag     540

gccggctcta gccacgcccc tccccctgcc ctgaccctga tcttcgaccg gcaccccatc     600

gcccacctgc tgtgctaccc tgccgccaga tacctgatgg gcagcatgac cccccaggcc     660

gtgctggcct tcgtggccct gatccccccc accctgcccg gcaccaacat cgtgctggga     720

gccctgcccg aggaccggca catcgaccgg ctggccaagc ggcagagacc cggcgagcgg     780

ctggacctgg ccatgctggc cgccatccgg cgggtgtacg gcctgctggc caacaccgtg     840

agatacctgc agggcggagg gtcttggtgg gaggactggg gccagctgtc cggcaccgcc     900

gtgccacctc agggcgccga gccccagagc aatgccggcc ctcggcccca catcggcgac     960

accctgttta ccctgttcag agcccccgag ctgctggccc ccaacggcga cctgtacaac    1020

gtgttcgcct gggccctgga cgtgctggcc aagaggctgc ggcccatgca cgtgttcatc    1080

ctggactacg accagagccc tgccggctgc agggacgccc tgctgcagct gaccagcggc    1140

atggtgcaga cccacgtgac cacccccggc agcatcccca ccatctgcga cctggcccgg    1200

accttcgccc gggagatggg cgaggccaac ggctccggag agggcagagg aagtctgcta    1260

acatgcggtg acgtcgagga gaatcctggc ccactcgaga tggccacaac catggtgagc    1320

aagggcgagg aggataacat ggccatcatc aaggagttca tgcgcttcaa ggtgcacatg    1380

gagggctccg tgaacggcca cgagttcgag atcgagggcg agggcgaggg ccgcccctac    1440

gagggcaccc agaccgccaa gctgaaggtg accaagggtg gccccctgcc cttcgcctgg    1500

gacatcctgt cccctcagtt catgtacggc tccaaggcct acgtgaagca ccccgccgac    1560

atccccgact acttgaagct gtccttcccc gagggcttca agtgggagcg cgtgatgaac    1620

ttcgaggacg gcggcgtggt gaccgtgacc caggactcct ccctgcagga cggcgagttc    1680

atctacaagg tgaagctgcg cggcaccaac ttcccctccg acggccccgt aatgcagaag    1740

aagaccatgg gctgggaggc ctcctccgag cggatgtacc ccgaggacgg cgccctgaag 1800

ggcgagatca agcagaggct gaagctgaag gacggcggcc actacgacgc tgaggtcaag 1860

accacctaca aggccaagaa gcccgtgcag ctgcccggcg cctacaacgt caacatcaag 1920

ttggacatca cctcccacaa cgaggactac accatcgtgg aacagtacga acgcgccgag 1980

ggccgccact ccaccggcgg catggacgag ctgtacaagg ctagcaagct t 2031

<210> 3
<211> 1848
<212> DNA
<213> Artificial Sequence

<220>
<223> Plasmid DNA

<400> 3
gcggccgcat ccaggcggcc gagatccgaa gttcctatac tatttgaaga ataggaactt 60

cagatccccc gggctgcagg atctaccggg taggggaggc gcttttccca aggcagtctg 120

gagcatgcgc tttagcagcc ccgctgggca cttggcgcta cacaagtggc ctctggcctc 180

gcacacattc cacatccacc ggtaggcgcc aaccggctcc gttctttggt ggccccttcg 240

cgccaccttc tactcctccc ctagtcagga agttcccccc cgccccgcag ctcgcgtcgt 300

gcaggacgtg acaaatggaa gtagcacgtc tcactagtct cgtgcagatg gacagcaccg 360

ctgagcaatg gaagcgggta ggcctttggg gcagcggcca atagcagctt tgctccttcg 420

ctttctgggc tcagaggctg ggaaggggtg ggtccggggg cgggctcagg ggcgggctca 480

ggggcggggc gggcgcccga aggtcctccg gaggcccggc attctgcacg cttcaaaagc 540

gcacgtctgc cgcgctgttc tcctcttcct catctccggg cctttcgacc tgcagcaatt 600

aatcatcggc atagtatatc ggcatagtat aatacgacaa ggtgaggaac taaaccatgg 660

gatcggccat tgaacaagat ggattgcacg caggttctcc ggccgcttgg gtggagaggc 720

tattcggcta tgactgggca caacagacaa tcggctgctc tgatgccgcc gtgttccggc 780

tgtcagcgca ggggcgcccg gttctttttg tcaagaccga cctgtccggt gccctgaatg 840

aactgcagga cgaggcagcg cggctatcgt ggctggccac gacgggcgtt ccttgcgcag 900

ctgtgctcga cgttgtcact gaagcgggaa gggactggct gctattgggc gaagtgccgg 960

ggcaggatct cctgtcatct caccttgctc ctgccgagaa agtatccatc atggctgatg 1020

caatgcggcg gctgcatacg cttgatccgg ctacctgccc attcgaccac caagcgaaac 1080

atcgcatcga gcgagcacgt actcggatgg aagccggtct tgtcgatcag gatgatctgg 1140

acgaagagca tcaggggctc gcgccagccg aactgttcgc caggctcaag gcgcgcatgc 1200

ccgacggcga tgatctcgtc gtgacccatg gcgatgcctg cttgccgaat atcatggtgg 1260

aaaatggccg cttttctgga ttcatcgact gtggccggct gggtgtggcg gaccgctatc 1320

aggacatagc gttggctacc cgtgatattg ctgaagagct tggcggcgaa tgggctgacc 1380

gcttcctcgt gctttacggt atcgccgctc ccgattcgca gcgcatcgcc ttctatcgcc 1440

ttcttgacga gttcttctga tcgagaaatt gatgatctat taaacaataa agatgtccac 1500

atggaagttt ttcctgtcat actttgttaa gaagggtgag aacagagtac ctacattttg 1560

aatggaagga ttggagctac gggggtgggg gtggggtggg attagataaa tgcctgctct 1620

ttactgaagg ctctttacta ttgctttatg ataatgtttc atagttggat atcataattt 1680

aaacaagcaa aaccaaatta agggccagct cattcctccc actcatgatc tatagatccg 1740

gtcgacgaag ttcctatact atttgaagaa taggaacttc ggatctggcc ggcctaggga 1800

taacagggta atggatcccc cgggctgcag gaattcgata tcaagctt 1848

<210> 4
<211> 8003
<212> DNA
<213> Artificial Sequence

<220>
<223> synthetic DNA

<400> 4
ggtctacctt aagccaaggt agaaatactt ggttgaaaaa tgtttctgct gccaggcagt 60

ggcagcacac accagcactt ggggggacag aggcaggcag atctcttgag tttaaggcca 120

gcctagtcta tagagcaagt cctggaacac ccagggctac acagagaaac cttgtctcag 180

aaaaccaaac aaaaagttct gttctggtag tgttggctaa ctgctttttt attgccactt 240

cactcttgtt tgtgtggtct gtgaaaagga agacagaccc tttggcatct ttgtcagggg 300

agattgatgg cctgggggga cctccaggaa ggggcttatt gtagttttag gggttctcgt 360

ttctctgtgt tgaacttccg cagagctgtg cttcctcctg attggacctg ctgtgctcat 420

tcagatcaat taaaaggcag gttttgctgt gtgagggaga ttattgtgaa agatttagga 480

gccagcctac tctagctggg ggagggagca ctagggattg aaccccagtc tttggcccct 540

gttgcttggg tgtcctgtca ctgagctgtg cccttggccc tgtcatcagg ctttgtcagt 600

ggattcccat gtggttgtct gttttattat tttcctctgc aatggggctt gaaccatggg 660

ccacacacac acactcacac actctgctcc ttgttttgag aacgtctcac tgacttgccc 720

tggttggccc tgaggtcact ggcgtagata gactttggac ctgggactcc tacagcggcc 780

ttcccagtag taggtctaca gcaccagact aggttcctct ggtatttctg aaagggcatt 840

ggcttgtata atacaggaac attggcatct tgggaggtgt gagcctggag tctacagtgt 900

cggtcccctg tgtgtcattt ggattttgag gcagggtctc ctgtagccct gactggcctc 960

attctctggg tgctaagatt acacagatgt gtgccctgct ggggccctgg tcctgctcgt 1020

tttgcctgat agggaagcta gccacccact gagcagcctt ctcagtcctg tcttgtttat 1080

tcttttttat tttttcacag tttatgatat gactcgtctt taatggctgt aactgctgct 1140

gacctcattg cattgttcat tgaggtcatt cactattttg ataaataaac tgtagttcta 1200

aatcactaat catttgaatc ccctcctacc gctttggctt agatttcttt tcatttttct 1260

tttttttctt tttttttggt ttttttgaga cagggtttct ctgtgtagct ctggctgtcc 1320

tggaactcac tttgtagacc aggctggcct caaactctgc ctccagagtg ctggtattaa 1380

agttgtatgc cagccaggta tatgccaccc aggtatatgc cagccaggat atggctgctt 1440

tggtcctttg actttagaga tctgaaaagc aagaggtcta tggtacttac ccactaatga 1500

gcagaaatca gaaataaaat aggttcaact tagtaatttg catatagcta attgtctgaa 1560

gttcttggtg acacctgcac agcttcctaa aagacaaccg tgtgtggtct gtcctgtgcc 1620

aggtagtgac gctgtggtac cgatctccag aagtgttgct gggctcggct cgttactcca 1680

ctccggttga catctggagt atagggacca tatttgcaga actggccacc aagaagccgc 1740

ttttccacgg cgactcagag attgaccagc tcttcaggat cttcaggtaa ttgtgtttta 1800

tacctaagct gttaaaagcc tgtatggtta aaataataaa ggcaccgtgt atgtgatagg 1860

acttttgctt cctttcagag ctctgggcac tcctaacaac gaagtgtggc cagaagtcga 1920

gtccctgcag gactacaaga acacctttcc caagtggaag ccggggagcc tcgcatccca 1980

cgtcaagaac ctggacgaga acggcttgga tttgctctca gtaagtgagc tgttctttat 2040

ggttcgagcg tggtgccctt gggtgtcagt agtgacaggt aagggtgact ggatgtactc 2100

agtcctggac ttgtcagcac gaagacacag cagctgagtc ggatgcccgg taatcctgta 2160

gtgacagcga gctgcctttg aggtgtgaag tgcctggtgg tctgaggagc ctctggtgtt 2220

ggactttgtc atttacttct catccggtag gagaggctgg actcacacac cttcttgctt 2280

gagagtatgt tgatgagtta caatgactca ctcctctctg tttgctactg agccctcagt 2340

tcatcggttt acactgttga ctgcttagga tactgagctg ttcaggcttt tgacagcata 2400

actccattgc tcaggtctgg ccttgtttcc tcagaaaagc tagcaaagac taattaattc 2460

tgattttctt cagttgtctt ggtgcacaat attagcaatg caaagaacaa aattactgtt 2520

caggattaat aaaggtctcc tttaggaaat ttgctgtagg tagagcaggg tttaaaatct 2580

gagtttgtag aaggaatcaa gtagacgatt cagttggtct ttccgctccc ttgcaatcta 2640

taaacgatgc caccaaatgg ggctcagatt tgtccagacc cctacagctg tcacatagtt 2700

tgttacatat tggtattggc taacttagaa gccttcccgt agcctgggtc caatctgctg 2760

gtattatcta aagttattag ctctgtgtgt gtgtgtgtgt gtgtgtgtgt gtgtgtgtgt 2820

gtgtgtgtgt tgctgggggc tatggacaga aggcagagct ttcttgaggt ggtgtaggaa 2880

ggttggtcat tgactgttga ggcgcagccc tacttctcag tctttaaaca aaggacttca 2940

cttaacagac atcagttctg ggcaccaatt tcctctgttg agccaacacc ctgcaatagc 3000

taagcttgcc gtggccctgc tgcctgctga gcactgtggc tatcactgcc ctgtccccca 3060

ctccagcggc agactgctga gttagcaggg cctgtgtcta tcgcaggacc ccagaagcaa 3120

actgccccag ttggagacca ctggattatt gatgagactt ggcttcagtt tattttctcc 3180

cacattgatg gattcgggca agtttcttgc tgtccttgaa agacttggtg tcttaagcca 3240

aacagtgtgg cacatctttc tcatatgtct gtgcggactg acagacatat cctcaaggcc 3300

ttcatattta tttgtttgcc tttttaaaaa tttacttatt tattttacat atgcaaatac 3360

actgttgctg tcctcagaca cagtagaaga gggcatcgga tcccattaca gatggttgtg 3420

agccaccatg tggttgctgg gagttgaact caggacttct ggaagagtag tcatcgctct 3480

taattgctga gccatctctc cacctcctta atctattttc tctgaagata atatgcatga 3540

tcagatttaa gatctggagt atttaaatta gtttacaata attagtccat ctattataac 3600

tgtgtttata aaaatgctat ggggtttttc ctttgttggc ttgacgaagg ggttgtgtgg 3660

acactatcat gaaacactat aactgattgc tactttttca gttgtgaata acagatggac 3720

atttaatttc tagggtagat ataattagct agataatgtg aagatttatg ttttggtttt 3780

aagctgtctg ggtgcttatc ttataattta taacaggtta ccaaggtgtc tgtgtgtgat 3840

ttaagtatct tggagaggta aatagagggc aggcttctaa atttatttcc ctttttctct 3900

ctagaaaatg ctagtctatg atcctgccaa acgaatctct ggcaaaatgg ccctgaagca 3960

cccgtacttt gatgacttgg acaatcagat taagaagatg tagccctctg gatggatgtc 4020

cctgtctgct ggtcgtaggg gaagatcgtg ttgtttaccg ttggctctct tcctgtcttg 4080

tatagttttc tttgtttgta aactgtcatc tggacttttc ttaatttcct acgtataact 4140

taattaacat gtaaatatta ttccatatga atttaaatat aattctgtat atgtgcagat 4200

gtcactgtgg tggctgttaa ttactataac acaagtgtta attactacaa cataagactt 4260

gagtctccct agacttccca gcagccattc ctgcagctcg gagcacagtt gaaggagctg 4320

agctcaggcc tcgtgatgct ttcaagtgcc tccgtgttct ggatatatat gattcctggt 4380

cagtttcttg ccatttggaa actacaaccc acctacggac agtgtttttc tacttgtgct 4440

taagcagttg ggatgagaag gccaaagacc cgaggatgtc tagagtaatg acccccagat 4500

ggaagtgcac caaagctggc tgggtttcac agctagagat caggggctgt ccagagcagg 4560

acagcttaga acatttatga agactcccta tttttaggtt tgttgtaaag ctgttgtcta 4620

gttggattcc tgtgctctgc atggtcagag gtaggttaga ggatttgcct tggcttctaa 4680

atccaatttg aaaactgctt aaaatctcct gtcctctctt agcagtgtct aaaaatgtcc 4740

ttgtccaaat atttagctga gattcctcac tttggaaaag gagccgtatc gctgtgctgc 4800

ttagtgtaat tcttagaagc agcctgactt atctgctagc agtcaaaggg atgcctgaga 4860

cgactgctcc tcttagaact aaaggctggg atgcttaagt ttgtctactg tttggaggat 4920

ctcggtaaga ctgagcccct gttcctgtgg ccacctcagt ttaccagtac ctcagcctca 4980

gcctcctgca tttgctggag tcagggaagg tccccagccc tgagccctga cgctcctgat 5040

tgtagagact gtcagttgga ggtaaaacgt tcattgaagt agtcagcagc cacatgcatt 5100

tagggcactc cagtgtcaga gaccatcctg gaggtttcta accctgccgc tggcagtcta 5160

ctcccaagac agatcagtta gagtggtcag caaacaccaa ctgctgcaga aacctgtgtt 5220

ggtgtggttc cctgctgctg ctgggaactg ggcccaagac tagagagctt ggggctgcgg 5280

ttgatcatgg gttctgttcc tgcattacac cttgaaatcc aagccttcta atatctccct 5340

tcggatcata agttgtgaat ttggtcctcc gcccccgcca ggttttctat acttgggttt 5400

gtcttgctga cattttcaag agtcctgact aagacggtga ttagtgtgac atgacttgag 5460

aactaccgat ttgaagcaca cttgaagtta acaaattctc tcatgattat acttttaact 5520

ttttataaga ttgcttgagc tcacccagat ctctgttggg aagtaactgg gtaacaaaag 5580

ccgttgcact ggtttgacag ctaacaactg ttggtacttt gtattcagaa ggaatgaggt 5640

agcgattgaa tggctggggt gttgtttcca cagtttatac actaaaaatt tgggtagcca 5700

ggaggtagtg gcgcgcaatc tttaatctca gcagaggcag tcctatctct tgagttggag 5760

gccagcctgc ctgagttcca gaccagccag ggctacagga agaagtctta aaaaaatttt 5820

ttttccctgt ggatgtaaac ccatgagaat gactgctgta tctatcactt tctgtataat 5880

agaggtttct tccgccgcag cgctccattg gtctcagccc ctcatgttgg gggcgggaag 5940

gaggggcaag cgtccccaag cacaggaagg cctgctgaag agttggcggc tttgagggtt 6000

caaatcctgc ttgggtctga ggagtccccc aaacgatctt ggactgtaaa aacctgccag 6060

gaacaagcgc taagggatgg ttctcaacct gtgggtcgcg accccctggg gagttggaca 6120

gccctttcct gaggatcaga acaaacatcc tgcatatcag atattaacgt ggcatttcat 6180

aactgcaaac ttacagttag gaagtagcag tgacaaagaa ttttatgggt ggggtggggt 6240

gggggctcac caccatatta aagggttaca gcattaggag ggttgacaac agaaccatcg 6300

cactgaggga aagtgagtta gcacgaagag atgagcattt cccagaacac agaataagac 6360

acgtttaacc aacatcagtc tgtaacccca gcggactgtg acaagctctc acccatgccg 6420

tggagctcag aactagcatt gcacgctttt accccatctt tggttgcctc actccctttg 6480

agtagtaagt ccctcttcaa agtaatagcc tctaaacagc ctgctgcgca tgctctagtc 6540

ctttgctttc ctccacgggt aggccacagg cttaagcagc agttggaaat ggaacttggg 6600

ctttctggct cctggtttag aaaactcttg aaatggacaa gatttagtcc taggcaagtt 6660

ctgtgtagtc agcaatggaa gcaaaggggg gtggggcaaa cattccagtc aggtggaatc 6720

tgagcccttg agagtgtgag gcaggaggat cactaggcag gagttctcaa ccagcctaga 6780

cgacagcttg gaacccatcg cactccacaa tgaggacccc tcagcttctg aagagaggag 6840

agctgctagc tctgagaagg gtgtataaaa tgggcttttg gttcccagac atcagacttg 6900

aagaggcaac cgagtaagcc agctatagac taagtaagat tgtcgtacct agcctactcc 6960

ttgggagctc attcttcaga gtatgttgat ggatgaccta acgccatctc agatgctgga 7020

gcattttgct ggaaaggctc tcagcactag cgaggctgcc tcaatgtaat cctgcctcag 7080

cctgagaact gattatgagt taccgtttgg tactttatgt atttttctct cttcctagta 7140

accaaggaca aacctgcttc cttccagcca cgtggcagac actagctgta ctaaagtatt 7200

attacacacc ggtcccctgc tgccagaaca aagaactcag cctcgagaaa tttctcctat 7260

tatatttggg atccagcatg tcccaagaca gggacgactc aataatttaa tgtgcctaat 7320

ggcacggaat aaaaggatta ttcacaaaca gaaaaccgga cttaagcaga tgtttatttg 7380

aacacaaata ccataaccct ccttctcatg atgttcccaa ttacacgttg caacattttc 7440

acgaagaagc tctttctaaa acggccttct agggagcgaa taaacaggac tatatttggc 7500

taacagctgc ttctgaaaac tgagatgtca gcaaagcaag ctgttgttta aagaaaacta 7560

attacccagc taccttgggc ttggatgggg gtaaataacg ccctcccgtt tcttggcagt 7620

gatttgtctg ggattttaat actgttaaga ttttatttct cttcctccac ttctcctttg 7680

gatcaaatgg tttttaaagg attccttgtg ttcctgctgc caatggcgag tcctttgcgt 7740

ttgccgcccc tgcttcgtca ccaactgcta ttttgtttag tgcaaagcag tgtgtttaag 7800

gacacactaa actttagatg acccagtgac acagtgatcc gtatttgcaa gcgtctggtt 7860

atgcagatgt cattgtgttg tttatcagca atgtcagcaa tgtctttttt tttttttttt 7920

tatgcacagg ctttgaaact tattttggaa attcattcat tttgagggga ggcagtgagc 7980

tgggagttat acaatctctc ttt 8003

<210> 5
<211> 50
<212> DNA
<213> Artificial Sequence

<220>
<223> Synthetic DNA

<400> 5
ccctgaagca cccgtacttt gatgacttgg acaatcagat taagaagatg 50

<210> 6
<211> 50
<212> DNA
<213> Artificial Sequence

<220>
<223>  Synthetic DNA

<400>  6
tagccctctg gatggatgtc cctgtctgct ggtcgtaggg gaagatcgtg                50


<210>  7
<211>  755
<212>  DNA
<213>  Artificial Sequence

<220>
<223>  synthetic DNA

<400>  7
tgtggcacat ctttctcata tgtctgtgcg gactgacaga catatcctca aggccttcat      60

atttatttgt ttgccttttt aaaaatttac ttatttattt tacatatgca aatacactgt     120

tgctgtcctc agacacagta gaagagggca tcggatccca ttacagatgg ttgtgagcca     180

ccatgtggtt gctgggagtt gaactcagga cttctggaag agtagtcatc gctcttaatt     240

gctgagccat ctctccacct ccttaatcta ttttctctga agataatatg catgatcaga     300

tttaagatct ggagtattta aattagttta caataattag tccatctatt ataactgtgt     360

ttataaaaat gctatggggt ttttcctttg ttggcttgac gaaggggttg tgtggacact     420

atcatgaaac actataactg attgctactt tttcagttgt gaataacaga tggacattta     480

atttctaggg tagatataat tagctagata atgtgaagat ttatgttttg gttttaagct     540

gtctgggtgc ttatcttata atttataaca ggttaccaag gtgtctgtgt gtgatttaag     600

tatcttggag aggtaaatag agggcaggct tctaaattta tttccctttt tctctctaga     660

aaatgctagt ctatgatcct gccaaacgaa tctctggcaa aatggccctg aagcacccgt     720

actttgatga cttggacaat cagattaaga agatg                                755


<210>  8
<211>  842
<212>  DNA
<213>  Artificial Sequence

<220>
<223>  synthetic DNA

<400>  8
ccctctggat ggatgtccct gtctgctggt cgtaggggaa gatcgtgttg tttaccgttg      60

gctctcttcc tgtcttgtat agttttcttt gtttgtaaac tgtcatctgg acttttctta     120

atttcctacg tataacttaa ttaacatgta aatattattc catatgaatt taaatataat     180

tctgtatatg tgcagatgtc actgtggtgg ctgttaatta ctataacaca agtgttaatt     240

actacaacat aagacttgag tctccctaga cttcccagca gccattcctg cagctcggag     300

cacagttgaa ggagctgagc tcaggcctcg tgatgctttc aagtgcctcc gtgttctgga     360

tatatatgat tcctggtcag tttcttgcca tttggaaact acaacccacc tacggacagt 420

gtttttctac ttgtgcttaa gcagttggga tgagaaggcc aaagacccga ggatgtctag 480

agtaatgacc cccagatgga agtgcaccaa agctggctgg gtttcacagc tagagatcag 540

gggctgtcca gagcaggaca gcttagaaca tttatgaaga ctccctattt ttaggtttgt 600

tgtaaagctg ttgtctagtt ggattcctgt gctctgcatg gtcagaggta ggttagagga 660

tttgccttgg cttctaaatc caatttgaaa actgcttaaa atctcctgtc ctctcttagc 720

agtgtctaaa aatgtccttg tccaaatatt tagctgagat tcctcacttt ggaaaaggag 780

ccgtatcgct gtgctgctta gtgtaattct tagaagcagc ctgacttatc tgctagcagt 840

ca 842

<210> 9
<211> 30
<212> DNA
<213> Artificial Sequence

<220>
<223> oligonucleotide

<400> 9
tgtggcacat ctttctcata tgtctgtgcg 30

<210> 10
<211> 30
<212> DNA
<213> Artificial Sequence

<220>
<223> oligonucleotide

<400> 10
gaagcagcct gacttatctg ctagcagtca 30

<210> 11
<211> 2034
<212> DNA
<213> Artificial Sequence

<220>
<223> synthetic DNA

<400> 11
ggttctggcg tgaaacagac tttgaatttt gaccttctca agttggcggg agacgtggag 60

tccaacccag ggcccataac ttcgtataat gtatgctata cgaagttatt aggtctgaag 120

aggagtttac gtccagccaa gctagcttgg ctgcaggtcg tcgacggtat cgataagctt 180

gatatcgaat ttcgagggcc cctgcaggtc aattctaccg ggtaggggag gcgcttttcc 240

caaggcagtc tggagcatgc gctttagcag ccccgctggc acttggcgct acacaagtgg 300

cctctggcct cgcacacatt ccacatccac cggtagcgcc aaccggctcc gttctttggt 360

ggccccttcg cgccaccttc tactcctccc ctagtcagga agttcccccc cgccccgcag 420

ctcgcgtcgt gcaggacgtg acaaatggaa gtagcacgtc tcactagtct cgtgcagatg 480

gacagcaccg ctgagcaatg gaagcgggta ggcctttggg gcagcggcca atagcagctt 540

tgctccttcg ctttctgggc tcagaggctg ggaaggggtg ggtccggggg cgggctcagg 600

ggcgggctca ggggcggggc gggcgcgaag gtcctcccga ggcccggcat tctcgcacgc 660

ttcaaaagcg cacgtctgcc gcgctgttct cctcttcctc atctccgggc ctttcgacct 720

gcagccaata tgggatcggc cattgaacaa gatggattgc acgcaggttc tccggccgct 780

tgggtggaga ggctattcgg ctatgactgg gcacaacaga caatcggctg ctctgatgcc 840

gccgtgttcc ggctgtcagc gcaggggcgc ccggttcttt ttgtcaagac cgacctgtcc 900

ggtgccctga atgaactgca ggacgaggca gcgcggctat cgtggctggc cacgacgggc 960

gttccttgcg cagctgtgct cgacgttgtc actgaagcgg gaagggactg gctgctattg 1020

ggcgaagtgc cggggcagga tctcctgtca tctcaccttg ctcctgccga gaaagtatcc 1080

atcatggctg atgcaatgcg gcggctgcat acgcttgatc cggctacctg cccattcgac 1140

caccaagcga aacatcgcat cgagcgagca cgtactcgga tggaagccgg tcttgtcgat 1200

caggatgatc tggacgaaga gcatcagggg ctcgcgccag ccgaactgtt cgccaggctc 1260

aaggcgcgca tgcccgacgg cgatgatctc gtcgtgaccc atggcgatgc ctgcttgccg 1320

aatatcatgg tggaaaatgg ccgcttttct ggattcatcg actgtggccg gctgggtgtg 1380

gcggaccgct atcaggacat agcgttggct acccgtgata ttgctgaaga gcttggcggc 1440

gaatgggctg accgcttcct cgtgctttac ggtatcgccg ctcccgattc gcagcgcatc 1500

gccttctatc gccttcttga cgagttcttc tgaggggatc gatccgtcct gtaagtctgc 1560

agaaattgat gatctattaa acaataaaga tgtccactaa aatggaagtt tttcctgtca 1620

tactttgtta agaagggtga gaacagagta cctacatttt gaatggaagg attggagcta 1680

cgggggtggg ggtggggtgg gattagataa atgcctgctc tttactgaag gctctttact 1740

attgctttat gataatgttt catagttgga tatcataatt taaacaagca aaaccaaatt 1800

aagggccagc tcattcctcc cactcatgat ctatagatct atagatctct cgtgggatca 1860

ttgtttttct cttgattccc actttgtggt tctaagtact gtggtttcca aatgtgtcag 1920

tttcatagcc tgaagaacga gatcagcagc ctctgttcca catacacttc attctcagta 1980

ttgttttgcc aagttctaat tccatcagaa gctgactcta gagcttggcg cgcc 2034

<210> 12
<211> 1971
<212> DNA
<213> Artificial Sequence

<220>
<223>  synthetic DNA

<400>  12
ggcgcgccat aacttcgtat aatgtatgct atacgaagtt atctatggcc agctacccct      60

gtcaccagca cgccagcgcc ttcgaccagg ccgccagaag caggggccac agcaaccggc     120

ggaccgccct gagacccagg cggcagcagg aagccaccga agtccggctg gaacagaaga     180

tgcccaccct gttaagggtg tacatcgacg gcccccacgg catgggcaag accaccacca     240

cccagctgct ggtggccctg ggcagccggg acgacatcgt gtacgtgccc gagcccatga     300

cctactggca ggtgctgggc gccagcgaga ccatcgccaa catctacacc acacagcaca     360

ggctggacca gggcgagatc tctgccggcg acgccgccgt ggtgatgacc agcgcccaga     420

tcacaatggg catgccctac gccgtgaccg acgccgtgct ggcccctcac gtgggcggcg     480

aggccggctc tagccacgcc cctccccctg ccctgaccct gatcttcgac cggcacccca     540

tcgcccacct gctgtgctac cctgccgcca gatacctgat gggcagcatg accccccagg     600

ccgtgctggc cttcgtggcc ctgatccccc ccaccctgcc cggcaccaac atcgtgctgg     660

gagccctgcc cgaggaccgg cacatcgacc ggctggccaa gcggcagaga cccggcgagc     720

ggctggacct ggccatgctg gccgccatcc ggcgggtgta cggcctgctg gccaacaccg     780

tgagatacct gcagggcgga gggtcttggt gggaggactg gggccagctg tccggcaccg     840

ccgtgccacc tcagggcgcc gagccccaga gcaatgccgg ccctcggccc cacatcggcg     900

acaccctgtt taccctgttc agagcccccg agctgctggc ccccaacggc gacctgtaca     960

acgtgttcgc ctgggccctg gacgtgctgg ccaagaggct gcggcccatg cacgtgttca    1020

tcctggacta cgaccagagc cctgccggct gcagggacgc cctgctgcag ctgaccagcg    1080

gcatggtgca gacccacgtg accacccccg gcagcatccc caccatctgc gacctggccc    1140

ggaccttcgc ccgggagatg ggcgaggcca acggctccgg agagggcaga ggaagtctgc    1200

taacatgcgg tgacgtcgag gagaatcctg gcccactcga gatggccaca accatggtga    1260

gcaagggcga ggaggataac atggccatca tcaaggagtt catgcgcttc aaggtgcaca    1320

tggagggctc cgtgaacggc cacgagttcg agatcgaggg cgagggcgag ggccgcccct    1380

acgagggcac ccagaccgcc aagctgaagg tgaccaaggg tggccccctg cccttcgcct    1440

gggacatcct gtcccctcag ttcatgtacg gctccaaggc ctacgtgaag caccccgccg    1500

acatccccga ctacttgaag ctgtccttcc ccgagggctt caagtgggag cgcgtgatga    1560

acttcgagga cggcggcgtg gtgaccgtga cccaggactc ctccctgcag gacggcgagt    1620

tcatctacaa ggtgaagctg cgcggcacca acttcccctc cgacggcccc gtaatgcaga    1680

agaagaccat gggctgggag gcctcctccg agcggatgta ccccgaggac ggcgccctga    1740

agggcgagat caagcagagg ctgaagctga aggacggcgg ccactacgac gctgaggtca 1800

agaccaccta caaggccaag aagcccgtgc agctgcccgg cgcctacaac gtcaacatca 1860

agttggacat cacctcccac aacgaggact acaccatcgt ggaacagtac gaacgcgccg 1920

agggccgcca ctccaccggc ggcatggacg agctgtacaa gatctgaatt c 1971

<210> 13
<211> 1726
<212> DNA
<213> Artificial Sequence

<220>
<223> synthetic DNA

<400> 13
gcggccgcat ctagagtcga ccagcttctg atggaattag aacttggcaa aacaatactg 60

agaatgaagt gtatgtggaa cagaggctgc tgatctcgtt cttcaggcta tgaaactgac 120

acatttggaa accacagtac ttagaaccac aaagtgggaa tcaagagaaa aacaatgatc 180

ccacgagaga tctatagatc tatagatcat gagtgggagg aatgagctgg cccttaattt 240

ggttttgctt gtttaaatta tgatatccaa ctatgaaaca ttatcataaa gcaatagtaa 300

agagccttca gtaaagagca ggcatttatc taatcccacc ccacccccac ccccgtagct 360

ccaatccttc cattcaaaat gtaggtactc tgttctcacc cttcttaaca aagtatgaca 420

ggaaaaactt ccattttagt ggacatcttt attgtttaat agatcatcaa tttctgcatc 480

ccggggatct gatatcatcg atgcatgggg tcgtgcgctc ctttcggtcg ggcgctgcgg 540

gtcgtggggc gggcgtcagg caccgggctt gcgggtcatg caccaggtgc gcggtccttc 600

gggcacctcg acgtcggcgg tgacggtgaa gccgagccgc tcgtagaagg ggaggttgcg 660

gggcgcggag gtctccagga aggcgggcac cccggcgcgc tcggccgcct ccactccggg 720

gagcacgacg gcgctgccca gacccttgcc ctggtggtcg ggcgagacgc cgacggtggc 780

caggaaccac gcgggctcct tgggccggtg cggcgccagg aggccttcca tctgttgctg 840

cgcggccagc cgggaaccgc tcaactcggc catgcgcggg ccgatctcgg cgaacaccgc 900

ccccgcttcg acgctctccg gcgtggtcca gaccgccacc gcggcgccgt cgtccgcgac 960

ccacaccttg ccgatgtcga gcccgacgcg cgtgaggaag agttcttgca gctcggtgac 1020

ccgctcgatg tggcggtccg gatcgacggt gtggcgcgtg gcggggtagt cggcgaacgc 1080

ggcggcgagg gtgcgtacgg ccctggggac gtcgtcgcgg gtggcgaggc gcaccgtggg 1140

cttgtactcg gtcatggtaa gcttcagctg ctcgagatct agatggatgc aggtcgaaag 1200

gcccggagat gaggaagagg agaacagcgc ggcagacgtg cgcttttgaa gcgtgcagaa 1260

tgccgggcct ccggaggacc ttcgggcgcc cgccccgccc ctgagcccgc ccctgagccc 1320

gcccccggac ccaccccttc ccagcctctg agcccagaaa gcgaaggagc aaagctgcta 1380

ttggccgctg ccccaaaggc ctacccgctt ccattgctca gcggtgctgt ccatctgcac 1440

gagactagtg agacgtgcta cttccatttg tcacgtcctg cacgacgcga gctgcggggc 1500

gggggggaac ttcctgacta ggggaggagt agaaggtggc gcgaaggggc caccaaagaa 1560

cggagccggt tggcgcctac cggtggatgt ggaatgtgtg cgaggccaga ggccacttgt 1620

gtagcgccaa gtgcccagcg gggctgctaa agcgcatgct ccagactgcc ttgggaaaag 1680

cgcctcccct acccggtaga atttcgaggt cgacgaattc ggatcc 1726

<210> 14
<211> 847
<212> DNA
<213> Artificial Sequence

<220>
<223> synthetic DNA

<400> 14
gttacttccc ttcgagcctc aatgtcctca tttgtaaaat gacattaata cctactttta 60

gctgtgggaa ttgagtacca tgatttatac aaagcagttt gtatggtgct ggttacatga 120

gagttcagat ggtaactagt tagtaaaaaa tctctagtgt gcttgttgat tttattttat 180

tttagtattt cttaaagatc aaatttaaca tcaatcctaa actttattta gctttttctg 240

gcgcgtaaac taacatacta agttgtgtga ctataattca tttagtgact catttttagc 300

tatttttata acacattgtg ctatgggggg ttttggaact tgctggaagc tacatcagaa 360

actgccatag ttaattgcca tttcaagaat gttgtaaata actcaggtgg ccgtttaatt 420

ctcaatgtaa atataattaa ctagacgtct ttcctatatt tgtgtctcag ttttaaagct 480

atttctggat gcttgagtct taccgtaatt gataacaaaa agaggttatt gagaatatct 540

atgatttaca gagtaagtta ttctagacct caagagtgaa atgtagggga ggagacattt 600

gtgtgttaaa ctaatggaaa tgctcattta atagatattc actgaaagta ttagttttgg 660

tttattgcta gaaaagttga ggttttatgg agatttttgt aaaaaatggt ttatttccta 720

aataaatatc tctttttctt ttttctccca gaaaatgtta atctatgatc cagccaaacg 780

aatttctggc aaaatggcac tgaatcatcc atattttaat gatttggaca atcagattaa 840

gaagatg 847

<210> 15
<211> 4382
<212> DNA
<213> Artificial Sequence

<220>
<223> synthetic DNA

<400> 15

ggttctggcg tgaaacagac tttgaatttt gaccttctca agttggcggg agacgtggag 60

tccaacccag ggcccatggc cagctacccc tgtcaccagc acgccagcgc cttcgaccag 120

gccgccagaa gcaggggcca cagcaaccgg cggaccgccc tgagacccag gcggcagcag 180

gaagccaccg aagtccggct ggaacagaag atgcccaccc tgttaaccct agaaagatag 240

tctgcgtaaa attgacgcat gcattcttga aatattgctc tctctttcta aatagcgcga 300

atccgtcgct gtgcatttag gacatctcag tcgccgcttg gagctcccgt gaggcgtgct 360

tgtcaatgcg gtaagtgtca ctgattttga actataacga ccgcgtgagt caaaatgacg 420

catgattatc ttttacgtga cttttaagat ttaactcata cgataattat attgttattt 480

catgttctac ttacgtgata acttattata tatatatttt cttgttatag atatcgtggc 540

ggccgcagat ccgaagttcc tatactattt gaagaatagg aacttcagat cccccgggct 600

gcaggatcta ccgggtaggg gaggcgcttt tcccaaggca gtctggagca tgcgctttag 660

cagccccgct gggcacttgg cgctacacaa gtggcctctg gcctcgcaca cattccacat 720

ccaccggtag gcgccaaccg gctccgttct ttggtggccc cttcgcgcca ccttctactc 780

ctcccctagt caggaagttc ccccccgccc cgcagctcgc gtcgtgcagg acgtgacaaa 840

tggaagtagc acgtctcact agtctcgtgc agatggacag caccgctgag caatggaagc 900

gggtaggcct ttggggcagc ggccaatagc agctttgctc cttcgctttc tgggctcaga 960

ggctgggaag gggtgggtcc gggggcgggc tcaggggcgg gctcaggggc ggggcgggcg 1020

cccgaaggtc ctccggaggc ccggcattct gcacgcttca aaagcgcacg tctgccgcgc 1080

tgttctcctc ttcctcatct ccgggccttt cgacctgcag caattaatca tcggcatagt 1140

atatcggcat agtataatac gacaaggtga ggaactaaac catgggatcg gccattgaac 1200

aagatggatt gcacgcaggt tctccggccg cttgggtgga gaggctattc ggctatgact 1260

gggcacaaca gacaatcggc tgctctgatg ccgccgtgtt ccggctgtca gcgcaggggc 1320

gcccggttct ttttgtcaag accgacctgt ccggtgccct gaatgaactg caggacgagg 1380

cagcgcggct atcgtggctg gccacgacgg gcgttccttg cgcagctgtg ctcgacgttg 1440

tcactgaagc gggaagggac tggctgctat tgggcgaagt gccggggcag gatctcctgt 1500

catctcacct tgctcctgcc gagaaagtat ccatcatggc tgatgcaatg cggcggctgc 1560

atacgcttga tccggctacc tgcccattcg accaccaagc gaaacatcgc atcgagcgag 1620

cacgtactcg gatggaagcc ggtcttgtcg atcaggatga tctggacgaa gagcatcagg 1680

ggctcgcgcc agccgaactg ttcgccaggc tcaaggcgcg catgcccgac ggcgatgatc 1740

tcgtcgtgac ccatggcgat gcctgcttgc cgaatatcat ggtggaaaat ggccgctttt 1800

ctggattcat cgactgtggc cggctgggtg tggcggaccg ctatcaggac atagcgttgg 1860

ctacccgtga tattgctgaa gagcttggcg gcgaatgggc tgaccgcttc ctcgtgcttt 1920

acggtatcgc cgctcccgat tcgcagcgca tcgccttcta tcgccttctt gacgagttct 1980

tctgatcgag aaattgatga tctattaaac aataaagatg tccacatgga agtttttcct 2040

gtcatacttt gttaagaagg gtgagaacag agtacctaca ttttgaatgg aaggattgga 2100

gctacggggg tgggggtggg gtgggattag ataaatgcct gctctttact gaaggctctt 2160

tactattgct ttatgataat gtttcatagt tggatatcat aatttaaaca agcaaaacca 2220

aattaagggc cagctcattc ctcccactca tgatctatag atccggtcga cgaagttcct 2280

attcttcaaa tagtatagga acttcggatc tggccggcct agggataaca gggtaatgga 2340

tcccccgggc tgcaggaatt cgatatcaag ctgcgggttt tgttacttta tagaagaaat 2400

tttgagtttt tgtttttttt taataaataa ataaacataa ataaattgtt tgttgaattt 2460

attattagta tgtaagtgta aatataataa aacttaatat ctattcaaat taataaataa 2520

acctcgatat acagaccgat aaaacacatg cgtcaatttt acgcatgatt atctttaacg 2580

tacgtcacaa tatgattatc tttctagggt taagggtgta catcgacggc ccccacggca 2640

tgggcaagac caccaccacc cagctgctgg tggccctggg cagccgggac gacatcgtgt 2700

acgtgcccga gcccatgacc tactggcagg tgctgggcgc cagcgagacc atcgccaaca 2760

tctacaccac acagcacagg ctggaccagg gcgagatctc tgccggcgac gccgccgtgg 2820

tgatgaccag cgcccagatc acaatgggca tgccctacgc cgtgaccgac gccgtgctgg 2880

cccctcacgt gggcggcgag gccggctcta gccacgcccc tccccctgcc ctgaccctga 2940

tcttcgaccg gcaccccatc gcccacctgc tgtgctaccc tgccgccaga tacctgatgg 3000

gcagcatgac cccccaggcc gtgctggcct tcgtggccct gatccccccc accctgcccg 3060

gcaccaacat cgtgctggga gccctgcccg aggaccggca catcgaccgg ctggccaagc 3120

ggcagagacc cggcgagcgg ctggacctgg ccatgctggc cgccatccgg cgggtgtacg 3180

gcctgctggc caacaccgtg agatacctgc agggcggagg gtcttggtgg gaggactggg 3240

gccagctgtc cggcaccgcc gtgccacctc agggcgccga gccccagagc aatgccggcc 3300

ctcggcccca catcggcgac accctgttta ccctgttcag agcccccgag ctgctggccc 3360

ccaacggcga cctgtacaac gtgttcgcct gggccctgga cgtgctggcc aagaggctgc 3420

ggcccatgca cgtgttcatc ctggactacg accagagccc tgccggctgc agggacgccc 3480

tgctgcagct gaccagcggc atggtgcaga cccacgtgac cacccccggc agcatcccca 3540

ccatctgcga cctggcccgg accttcgccc gggagatggg cgaggccaac ggctccggag 3600

agggcagagg aagtctgcta acatgcggtg acgtcgagga gaatcctggc ccactcgaga 3660

tggccacaac catggtgagc aagggcgagg aggataacat ggccatcatc aaggagttca 3720

tgcgcttcaa ggtgcacatg gagggctccg tgaacggcca cgagttcgag atcgagggcg 3780

agggcgaggg ccgcccctac gagggcaccc agaccgccaa gctgaaggtg accaagggtg 3840

gccccctgcc cttcgcctgg gacatcctgt cccctcagtt catgtacggc tccaaggcct 3900

acgtgaagca ccccgccgac atccccgact acttgaagct gtccttcccc gagggcttca 3960

agtgggagcg cgtgatgaac ttcgaggacg gcggcgtggt gaccgtgacc caggactcct 4020

ccctgcagga cggcgagttc atctacaagg tgaagctgcg cggcaccaac ttcccctccg 4080

acggccccgt aatgcagaag aagaccatgg gctgggaggc ctcctccgag cggatgtacc 4140

ccgaggacgg cgccctgaag ggcgagatca agcagaggct gaagctgaag gacggcggcc 4200

actacgacgc tgaggtcaag accacctaca aggccaagaa gcccgtgcag ctgcccggcg 4260

cctacaacgt caacatcaag ttggacatca cctcccacaa cgaggactac accatcgtgg 4320

aacagtacga acgcgccgag ggccgccact ccaccggcgg catggacgag ctgtacaagt 4380

ag 4382

<210> 16
<211> 931
<212> DNA
<213> Artificial Sequence

<220>
<223> synthetic DNA

<400> 16
ctttctgaca aaaagtttcc atatgttata tcaacagata gttgtgtttt tattgttaac 60

tcttgtctat ttttgtctta tatatatttc tttgttatca aacttcagct gtacttcgtc 120

ttctaatttc aaaaatataa cttaaaaatg taaatattct atatgaattt aaatataatt 180

ctgtaaatgt gtgtaggtct cactgtaaca actatttgtt actataataa aactataata 240

ttgatgtcag gaatcaggaa aaaatttgag ttggcttaaa tcatctcagt ccttatggca 300

gttttatttt cctgtagttg gaactactaa aatttaggaa aatgctaagt tcaagtttcg 360

taatgctttg aagtattttt atgctctgaa tgtttaaatg ttctcatcag tttcttgcca 420

tgttgttaac tatacaacct ggctaaagat gaatattttt ctactggtat tttaattttt 480

gacctaaatg tttaagcatt cggaatgaga aaactataca gatttgagaa atgatgctaa 540

atttatagga gttttcagta acttaaaaag ctaacatgag agcatgccaa aatttgctaa 600

gtcttacaaa gatcaagggc tgtccgcaac agggaagaac agttttgaaa atttatgaac 660

tatcttattt ttaggtaggt tttgaaagct ttttgtctaa gtgaattctt atgccttggt 720

cagagtaata actgaaggag ttgcttatct tggctttcga gtctgagttt aaaactacac 780

attttgacat agtgtttatt agcagccatc taaaaaggct ctaatgtata tttaactaaa 840

attactagct ttgggaatta aactgtttaa caaataatgc tgctcattgt gattcttacc 900

tataagcagc ctaatttgaa ttatttgctg c 931

<210>  17
<211>  1623
<212>  DNA
<213>  Artificial Sequence

<220>
<223>  synthetic DNA

<400>  17
accggtaggc gccaaccggc tccgttcttt ggtggcccct tcgcgccacc ttctactcct      60

cccctagtca ggaagttccc ccccgccccg cagctcgcgt cgtgcaggac gtgacaaatg     120

gaagtagcac gtctcactag tctcgtgcag atggacagca ccgctgagca atggaagcgg     180

gtaggccttt ggggcagcgg ccaatagcag ctttgctcct tcgctttctg ggctcagagg     240

ctgggaaggg gtgggtccgg gggcgggctc aggggcgggc tcaggggcgg ggcgggcgcc     300

cgaaggtcct ccggaggccc ggcattctgc acgcttcaaa agcgcacgtc tgccgcgctg     360

ttctcctctt cctcatctcc gggcctttcg acctgcatcc atctagatct cgagcagctg     420

aagcttacca tgaccgagta caagcccacg gtgcgcctcg ccacccgcga cgacgtcccc     480

agggccgtac gcaccctcgc cgccgcgttc gccgactacc ccgccacgcg ccacaccgtc     540

gatccggacc gccacatcga gcgggtcacc gagctgcaag aactcttcct cacgcgcgtc     600

gggctcgaca tcggcaaggt gtgggtcgcg gacgacggcg ccgcggtggc ggtctggacc     660

acgccggaga gcgtcgaagc gggggcggtg ttcgccgaga tcggcccgcg catggccgag     720

ttgagcggtt cccggctggc cgcgcagcaa cagatggaag gcctcctggc gccgcaccgg     780

cccaaggagc ccgcgtggtt cctggccacc gtcggcgtct cgcccgacca ccagggcaag     840

ggtctgggca gcgccgtcgt gctccccgga gtggaggcgg ccgagcgcgc cggggtgccc     900

gccttcctgg agacctccgc gccccgcaac ctccccttct acgagcggct cggcttcacc     960

gtcaccgccg acgtcgaggt gcccgaagga ccgcgcacct ggtgcatgac ccgcaagccc    1020

ggtgcctgac gcccgcccca cgacccgcag cgcccgaccg aaaggagcgc acgaccccat    1080

gcatcgatga tatcagatcc ccgggatgca gaaattgatg atctattaaa caataaagat    1140

gtccactaaa atggaagttt ttcctgtcat actttgttaa gaagggtgag aacagagtac    1200

ctacattttg aatggaagga ttggagctac gggggtgggg gtggggtggg attagataaa    1260

tgcctgctct ttactgaagg ctctttacta ttgctttatg ataatgtttc atagttggat    1320

atcataattt aaacaagcaa aaccaaatta agggccagct cattcctccc actcatgatc    1380

tatagatcta tagatctctc gtgggatcat tgtttttctc ttgattccca ctttgtggtt    1440

ctaagtactg tggtttccaa atgtgtcagt ttcatagcct gaagaacgag atcagcagcc    1500

tctgttccac atacacttca ttctcagtat tgttttgcca agttctaatt ccatcagaag    1560

ctggtcgact ctagatgcgg cgaagttcct attcttcaaa tagtatagga acttcggccg 1620

gcc 1623

<210> 18
<211> 3990
<212> DNA
<213> Artificial Sequence

<220>
<223> synthetic DNA

<400> 18
ggatccggtt ctggcgtgaa acagactttg aattttgacc ttctcaagtt ggcgggagac 60

gtggagtcca acccagggcc cataacttcg tataatgtat gctatacgaa gttattaggt 120

ctgaagagga gtttacgtcc agccaagcta gcttggctgc aggtcgtcga cggtatcgat 180

aagcttgata tcgagggccc ctgcaggtca attctaccgg gtaggggagg cgcttttccc 240

aaggcagtct ggagcatgcg ctttagcagc cccgctgggc acttggcgct acacaagtgg 300

cctctggcct cgcacacatt ccacatccac cggtaggcgc caaccggctc cgttctttgg 360

tggccccttc gcgccacctt ctactcctcc cctagtcagg aagttccccc ccgccccgca 420

gctcgcgtcg tgcaggacgt gacaaatgga agtagcacgt ctcactagtc tcgtgcagat 480

ggacagcacc gctgagcaat ggaagcgggt aggcctttgg ggcagcggcc aatagcagct 540

ttgctccttc gctttctggg ctcagaggct gggaaggggt gggtccgggg gcgggctcag 600

gggcgggctc aggggcgggg cgggcgcccg aaggtcctcc cgaggcccgg cattctgcac 660

gcttcaaaag cgcacgtctg ccgcgctgtt ctcctcttcc tcatctccgg gcctttcgac 720

ctgcagccaa tatgggatcg gccattgaac aagatggatt gcacgcaggt tctccggccg 780

cttgggtgga gaggctattc ggctatgact gggcacaaca gacaatcggc tgctctgatg 840

ccgccgtgtt ccggctgtca gcgcaggggc gcccggttct ttttgtcaag accgacctgt 900

ccggtgccct gaatgaactg caggacgagg cagcgcggct atcgtggctg gccacgacgg 960

gcgttccttg cgcagctgtg ctcgacgttg tcactgaagc gggaagggac tggctgctat 1020

tgggcgaagt gccggggcag gatctcctgt catctcacct tgctcctgcc gagaaagtat 1080

ccatcatggc tgatgcaatg cggcggctgc atacgcttga tccggctacc tgcccattcg 1140

accaccaagc gaaacatcgc atcgagcgag cacgtactcg gatggaagcc ggtcttgtcg 1200

atcaggatga tctggacgaa gagcatcagg ggctcgcgcc agccgaactg ttcgccaggc 1260

tcaaggcgcg catgcccgac ggcgatgatc tcgtcgtgac ccatggcgat gcctgcttgc 1320

cgaatatcat ggtggaaaat ggccgctttt ctggattcat cgactgtggc cggctgggtg 1380

tggcggaccg ctatcaggac atagcgttgg ctacccgtga tattgctgaa gagcttggcg 1440

gcgaatgggc tgaccgcttc ctcgtgcttt acggtatcgc cgctcccgat tcgcagcgca 1500

tcgccttcta tcgccttctt gacgagttct tctgagggga tcgatccgct gtaagtctgc 1560

agaaattgat gatctattaa acaataaaga tgtccactaa aatggaagtt tttcctgtca 1620

tactttgtta agaagggtga gaacagagta cctacatttt gaatggaagg attggagcta 1680

cgggggtggg ggtggggtgg gattagataa atgcctgctc tttactgaag gctctttact 1740

attgctttat gataatgttt catagttgga tatcataatt taaacaagca aaaccaaatt 1800

aagggccagc tcattcctcc cactcatgat ctatagatct atagatctct cgtgggatca 1860

ttgtttttct cttgattccc actttgtggt tctaagtact gtggtttcca aatgtgtcag 1920

tttcatagcc tgaagaacga gatcagcagc ctctgttcca catacacttc attctcagta 1980

ttgttttgcc aagttctaat tccatcagaa gctgactcta gagcttggcg cgccataact 2040

tcgtataatg tatgctatac gaagttatct atggccagct acccctgtca ccagcacgcc 2100

agcgccttcg accaggccgc cagaagcagg ggccacagca accggcggac cgccctgaga 2160

cccaggcggc agcaggaagc caccgaagtc cggctggaac agaagatgcc caccctgtta 2220

agggtgtaca tcgacggccc ccacggcatg ggcaagacca ccaccaccca gctgctggtg 2280

gccctgggca gccgggacga catcgtgtac gtgcccgagc ccatgaccta ctggcaggtg 2340

ctgggcgcca gcgagaccat cgccaacatc tacaccacac agcacaggct ggaccagggc 2400

gagatctctg ccggcgacgc cgccgtggtg atgaccagcg cccagatcac aatgggcatg 2460

ccctacgccg tgaccgacgc cgtgctggcc cctcacgtgg gcggcgaggc cggctctagc 2520

cacgcccctc cccctgccct gaccctgatc ttcgaccggc accccatcgc ccacctgctg 2580

tgctaccctg ccgccagata cctgatgggc agcatgaccc cccaggccgt gctggccttc 2640

gtggccctga tcccccccac cctgcccggc accaacatcg tgctgggagc cctgcccgag 2700

gaccggcaca tcgaccggct ggccaagcgg cagagacccg gcgagcggct ggacctggcc 2760

atgctggccg ccatccggcg ggtgtacggc ctgctggcca acaccgtgag atacctgcag 2820

ggcggagggt cttggtggga ggactggggc cagctgtccg gcaccgccgt gccacctcag 2880

ggcgccgagc cccagagcaa tgccggccct cggccccaca tcggcgacac cctgtttacc 2940

ctgttcagag cccccgagct gctggccccc aacggcgacc tgtacaacgt gttcgcctgg 3000

gccctggacg tgctggccaa gaggctgcgg cccatgcacg tgttcatcct ggactacgac 3060

cagagccctg ccggctgcag ggacgccctg ctgcagctga ccagcggcat ggtgcagacc 3120

cacgtgacca cccccggcag catccccacc atctgcgacc tggcccggac cttcgcccgg 3180

gagatgggcg aggccaacgg ctccggagag ggcagaggaa gtctgctaac atgcggtgac 3240

gtcgaggaga atcctggccc actcgagatg gccacaacca tggtgagcaa gggcgaggag 3300

gataacatgg ccatcatcaa ggagttcatg cgcttcaagg tgcacatgga gggctccgtg 3360

aacggccacg agttcgagat cgagggcgag ggcgagggcc gcccctacga gggcacccag 3420

accgccaagc tgaaggtgac caagggtggc cccctgccct tcgcctggga catcctgtcc 3480

cctcagttca tgtacggctc caaggcctac gtgaagcacc ccgccgacat ccccgactac 3540

ttgaagctgt ccttccccga gggcttcaag tgggagcgcg tgatgaactt cgaggacggc 3600

ggcgtggtga ccgtgaccca ggactcctcc ctgcaggacg gcgagttcat ctacaaggtg 3660

aagctgcgcg gcaccaactt cccctccgac ggccccgtaa tgcagaagaa gaccatgggc 3720

tgggaggcct cctccgagcg gatgtacccc gaggacggcg ccctgaaggg cgagatcaag 3780

cagaggctga agctgaagga cggcggccac tacgacgctg aggtcaagac cacctacaag 3840

gccaagaagc ccgtgcagct gcccggcgcc tacaacgtca acatcaagtt ggacatcacc 3900

tcccacaacg aggactacac catcgtggaa cagtacgaac gcgccgaggg ccgccactcc 3960

accggcggca tggacgagct gtacaagtag 3990

<210> 19
<211> 3860
<212> DNA
<213> Artificial Sequence

<220>
<223> synthetic DNA

<400> 19
   ggatccggtt ctggcgtgaa acagactttg aattttgacc ttctcaagtt ggcgggagac 60
   gtggagtcca acccagggcc cataacttcg tataatgtat gctatacgaa gttattaggt 120
   ctgaagagga gtttacgtcc agccaagcta gcttggctgc aggtcgtcga cggtatcggc 180
   cgcatctaga gtcgaccagc ttctgatgga attagaactt ggcaaacaat actgagaatg 240
   aagtgtatgt ggaacagagg ctgctgatct cgttcttcag gctatgaaac tgacacattt 300
   ggaaaccaca gtacttagaa ccacaaagtg ggaatcaaga gaaaaacaat gatcccacga 360
   gagatctata gatctataga tcatgagtgg gaggaatgag ctggccctta atttggtttt 420
   gcttgtttaa attatgatat ccaactatga aacattatca taaagcaata gtaaagagcc 480
   ttcagtaaag agcaggcatt tatctaatcc cacccccacc ccacccccgt agctccaatc 540
   cttccattca aaatgtaggt actctgttct cacccttctt aacaaagtat gacaggaaaa 600
   acttccattt tagtggacat ctttattgtt taatagatca tcaatttctg catcccgggg 660
   atctgatatc atcgatgcat ggggtcgtgc gctcctttcg gtcgggcgct gcgggtcgtg 720
   gggcgggcgt caggcaccgg gcttgcgggt catgcaccag gtgcgcggtc cttcgggcac 780
   ctcgacgtcg gcggtgacgg tgaagccgag ccgctcgtag aaggggaggt tgcggggcgc 840
   ggaggtctcc aggaaggcgg gcaccccggc gcgctcggcc gcctccactc cggggagcac 900
   gacggcgctg cccagaccct tgccctggtg gtcgggcgag acgccgacgg tggccaggaa 960
   ccacgcgggc tccttgggcc ggtgcggcgc caggaggcct tccatctgtt gctgcgcggc 1020
   cagccgggaa ccgctcaact cggccatgcg cgggccgatc tcggcgaaca ccgcccccgc 1080
   ttcgacgctc tccggcgtgg tccagaccgc caccgcggcg ccgtcgtccg cgacccacac 1140
   cttgccgatg tcgagcccga cgcgcgtgag gaagagttct tgcagctcgg tgacccgctc 1200
   gatgtggcgg tccggatcga cggtgtggcg cgtggcgggg tagtcggcga acgcggcggc 1260
   gagggtgcgt acggccctgg ggacgtcgtc gcgggtggcg aggcgcaccg tgggcttgta 1320
   ctcggtcatg gtaagcttca gctgctcgag atctagatgg atgcaggtcg aaaggcccgg 1380
   agatgaggaa gaggagaaca gcgcggcaga cgtgcgcttt tgaagcgtgc agaatgccgg 1440
   gcctccggag gaccttcggg cgcccgcccc gcccctgagc ccgcccctga gcccgccccc 1500
   ggacccaccc cttcccagcc tctgagccca gaaagcgaag gagcaaagct gctattggcc 1560
   gctgccccaa aggcctaccc gcttccattg ctcagcggtg ctgtccatct gcacgagact 1620
   agtgagacgt gctacttcca tttgtcacgt cctgcacgac gcgagctgcg gggcgggggg 1680
   gaacttcctg actaggggag gagtagaagg tggcgcgaag gggccaccaa agaacggagc 1740
   cggttggcgc ctaccggtgg atgtggaatg tgtgcgaggc cagaggccac ttgtgtagcg 1800
   ccaagtgccc agcggggctg ctaaagcgca tgctccagac tgccttggga aaagcgcctc 1860
   ccctacccgg tagaatttcg aggtcgacga attcggatcg cgccataact tcgtataatg 1920
   tatgctatac gaagttatct atggccagct acccctgtca ccagcacgcc agcgccttcg 1980
   accaggccgc cagaagcagg ggccacagca accggcggac cgccctgaga cccaggcggc 2040
   agcaggaagc caccgaagtc cggctggaac agaagatgcc caccctgtta agggtgtaca 2100
   tcgacggccc ccacggcatg ggcaagacca ccaccaccca gctgctggtg gccctgggca 2160
   gccgggacga catcgtgtac gtgcccgagc ccatgaccta ctggcaggtg ctgggcgcca 2220
   gcgagaccat cgccaacatc tacaccacac agcacaggct ggaccagggc gagatctctg 2280
   ccggcgacgc cgccgtggtg atgaccagcg cccagatcac aatgggcatg ccctacgccg 2340
   tgaccgacgc cgtgctggcc cctcacgtgg gcggcgaggc cggctctagc cacgcccctc 2400
   cccctgccct gaccctgatc ttcgaccggc accccatcgc ccacctgctg tgctaccctg 2460
   ccgccagata cctgatgggc agcatgaccc cccaggccgt gctggccttc gtggccctga 2520
   tcccccccac cctgcccggc accaacatcg tgctgggagc cctgcccgag gaccggcaca 2580
   tcgaccggct ggccaagcgg cagagacccg gcgagcggct ggacctggcc atgctggccg 2640
   ccatccggcg ggtgtacggc ctgctggcca acaccgtgag atacctgcag ggcggagggt 2700
   cttggtggga ggactggggc cagctgtccg gcaccgccgt gccacctcag ggcgccgagc 2760
   cccagagcaa tgccggccct cggccccaca tcggcgacac cctgtttacc ctgttcagag 2820
   cccccgagct gctggccccc aacggcgacc tgtacaacgt gttcgcctgg gccctggacg 2880
   tgctggccaa gaggctgcgg cccatgcacg tgttcatcct ggactacgac cagagccctg 2940
   ccggctgcag ggacgccctg ctgcagctga ccagcggcat ggtgcagacc cacgtgacca 3000
   cccccggcag catccccacc atctgcgacc tggcccggac cttcgcccgg gagatgggcg 3060
   aggccaacgg ctccggagag ggcagaggaa gtctgctaac atgcggtgac gtcgaggaga 3120
   atcctggccc actcgagatg gccacaacca tggtgagcaa gggcgaggag gataacatgg 3180
   ccatcatcaa ggagttcatg cgcttcaagg tgcacatgga gggctccgtg aacggccacg 3240
   agttcgagat cgagggcgag ggcgagggcc gcccctacga gggcacccag accgccaagc 3300
   tgaaggtgac caagggtggc cccctgccct tcgcctggga catcctgtcc cctcagttca 3360
   tgtacggctc caaggcctac gtgaagcacc ccgccgacat ccccgactac ttgaagctgt 3420
   ccttccccga gggcttcaag tgggagcgcg tgatgaactt cgaggacggc ggcgtggtga 3480
   ccgtgaccca ggactcctcc ctgcaggacg gcgagttcat ctacaaggtg aagctgcgcg 3540
   gcaccaactt cccctccgac ggccccgtaa tgcagaagaa gaccatgggc tgggaggcct 3600
   cctccgagcg gatgtacccc gaggacggcg ccctgaaggg cgagatcaag cagaggctga 3660
   agctgaagga cggcggccac tacgacgctg aggtcaagac cacctacaag gccaagaagc 3720
   ccgtgcagct gcccggcgcc tacaacgtca acatcaagtt ggacatcacc tcccacaacg 3780
   aggactacac catcgtggaa cagtacgaac gcgccgaggg ccgccactcc accggcggca 3840
   tggacgagct gtacaagtag 3860

<210> 20
<211> 4848
<212> DNA
<213> Artificial Sequence

<220>
<223> synthetic DNA

<400> 20
tccaccggcg gcatggacga gctgtacaag tagctttctg acaaaaagtt tccatatgtt 60

atgtcaacag atagttgtgt ttttattgtt aactcttgtc tatttttgtc ttatatatat 120

ttctttgtta tcaaacttca gctgtacttc gtcttctaat ttcaaaaata taacttaaaa 180

atgtaaatat tctatatgaa tttaaatata attctgtaaa tgtgtgtagg tctcactgta 240

acaactattt gttactataa taaaactata atattgatgt caggaatcag gaaaaaattt 300

gagttggctt aaatcatctc agtccttatg gcagttttat tttcctgtag ttggaactac 360

taaaatttag gaaaatgcta agttcaagtt tcgtaatgct ttgaagtatt tttatgctct 420

gaatgtttaa atgttctcat cagtttcttg ccatgttgtt aactatacaa cctggctaaa 480

gatgaatatt tttctactgg tattttaatt tttgacctaa atgtttaagc attcggaatg 540

agaaaactat acagatttga gaaatgatgc taaatttata ggagttttca gtaacttaaa 600

aagctaacat gagagcatgc caaaatttgc taagtcttac aaagatcaag ggctgtccgc 660

aacagggaag aacagttttg aaaatttatg aactatctta tttttaggta ggttttgaaa 720

gctttttgtc taagtgaatt cttatgcctt ggtcagagta ataactgaag gaggtgctta 780

tcttggcttt cgagtctgag tttaaaacta cacattttga catagtgttt attagcagcc 840

atctaaaaag gctctaatgt atatttaact aaaattacta gctttgggaa ttaaactgtt 900

taacaaataa tgctgctcat tgtgattctt acctataagc agcctaattt gaattatttg 960

ctgcaatcga attcccgcgg ccgccatggc ggccgggagc atgcgacgtc gggcccaatt 1020

cgccctatag tgagtcgtat tacaattcac tggccgtcgt tttacaacgt cgtgactggg 1080

aaaaccctgg cgttacccaa cttaatcgcc ttgcagcaca tccccctttc gccagctggc 1140

gtaatagcga agaggcccgc accgatcgcc cttcccaaca gttgcgcagc ctgaatggcg 1200

aatggacgcg ccctgtagcg gcgcattaag cgcggcgggt gtggtggtta cgcgcagcgt 1260

gaccgctaca cttgccagcg ccctagcgcc cgctcctttc gctttcttcc cttcctttct 1320

cgccacgttc gccggctttc cccgtcaagc tctaaatcgg gggctccctt tagggttccg 1380

atttagtgct ttacggcacc tcgaccccaa aaaacttgat tagggtgatg gttcacgtag 1440

tgggccatcg ccctgataga cggtttttcg ccctttgacg ttggagtcca cgttctttaa 1500

tagtggactc ttgttccaaa ctggaacaac actcaaccct atctcggtct attcttttga 1560

tttataaggg attttgccga tttcggccta ttggttaaaa aatgagctga tttaacaaaa 1620

atttaacgcg aattttaaca aaatattaac gcttacaatt tcctgatgcg gtattttctc 1680

cttacgcatc tgtgcggtat ttcacaccgc atcaggtggc acttttcggg gaaatgtgcg 1740

cggaacccct atttgtttat ttttctaaat acattcaaat atgtatccgc tcatgagaca 1800

ataaccctga taaatgcttc aataatattg aaaaaggaag agtatgagta ttcaacattt 1860

ccgtgtcgcc cttattccct tttttgcggc attttgcctt cctgtttttg ctcacccaga 1920

aacgctggtg aaagtaaaag atgctgaaga tcagttgggt gcacgagtgg gttacatcga 1980

actggatctc aacagcggta agatccttga gagttttcgc cccgaagaac gttttccaat 2040

gatgagcact tttaaagttc tgctatgtgg cgcggtatta tcccgtattg acgccgggca 2100

agagcaactc ggtcgccgca tacactattc tcagaatgac ttggttgagt actcaccagt 2160

cacagaaaag catcttacgg atggcatgac agtaagagaa ttatgcagtg ctgccataac 2220

catgagtgat aacactgcgg ccaacttact tctgacaacg atcggaggac cgaaggagct 2280

aaccgctttt ttgcacaaca tgggggatca tgtaactcgc cttgatcgtt gggaaccgga 2340

gctgaatgaa gccataccaa acgacgagcg tgacaccacg atgcctgtag caatggcaac 2400

aacgttgcgc aaactattaa ctggcgaact acttactcta gcttcccggc aacaattaat 2460

agactggatg gaggcggata aagttgcagg accacttctg cgctcggccc ttccggctgg 2520

ctggtttatt gctgataaat ctggagccgg tgagcgtggg tctcgcggta tcattgcagc 2580

actggggcca gatggtaagc cctcccgtat cgtagttatc tacacgacgg ggagtcaggc 2640

aactatggat gaacgaaata gacagatcgc tgagataggt gcctcactga ttaagcattg 2700

gtaactgtca gaccaagttt actcatatat actttagatt gatttaaaac ttcattttta 2760

atttaaaagg atctaggtga agatcctttt tgataatctc atgaccaaaa tcccttaacg 2820

tgagttttcg ttccactgag cgtcagaccc cgtagaaaag atcaaaggat cttcttgaga 2880

tccttttttt ctgcgcgtaa tctgctgctt gcaaacaaaa aaaccaccgc taccagcggt 2940

ggtttgtttg ccggatcaag agctaccaac tctttttccg aaggtaactg gcttcagcag 3000

agcgcagata ccaaatactg ttcttctagt gtagccgtag ttaggccacc acttcaagaa 3060

ctctgtagca ccgcctacat acctcgctct gctaatcctg ttaccagtgg ctgctgccag 3120

tggcgataag tcgtgtctta ccgggttgga ctcaagacga tagttaccgg ataaggcgca 3180

gcggtcgggc tgaacggggg gttcgtgcac acagcccagc ttggagcgaa cgacctacac 3240

cgaactgaga tacctacagc gtgagctatg agaaagcgcc acgcttcccg aagggagaaa 3300

ggcggacagg tatccggtaa gcggcagggt cggaacagga gagcgcacga gggagcttcc 3360

agggggaaac gcctggtatc tttatagtcc tgtcgggttt cgccacctct gacttgagcg 3420

tcgatttttg tgatgctcgt caggggggcg gagcctatgg aaaaacgcca gcaacgcggc 3480

ctttttacgg ttcctggcct tttgctggcc ttttgctcac atgttctttc ctgcgttatc 3540

ccctgattct gtggataacc gtattaccgc ctttgagtga gctgataccg ctcgccgcag 3600

ccgaacgacc gagcgcagcg agtcagtgag cgaggaagcg gaagagcgcc caatacgcaa 3660

accgcctctc cccgcgcgtt ggccgattca ttaatgcagc tggcacgaca ggtttcccga 3720

ctggaaagcg ggcagtgagc gcaacgcaat taatgtgagt tagctcactc attaggcacc 3780

ccaggcttta cactttatgc ttccggctcg tatgttgtgt ggaattgtga gcggataaca 3840

atttcacaca ggaaacagct atgaccatga ttacgccaag ctatttaggt gacactatag 3900

aatactcaag ctatgcatcc aacgcgttgg gagctctccc atatggtcga cctgcaggcg 3960

gccgcgaatt cactagtgat tgttacttcc cttcgagcct caatgtcctc atttgtaaaa 4020

tgacattaat acctactttt agctgtggga attgagtacc atgatttata caaagcagtt 4080

tgtatggtgc tggttacatg agagttcaga tggtaactag ttagtaaaaa atctctagtg 4140

tgcttgttga ttttatttta ttttagtatt tcttaaagat caaatttaac atcaatccta 4200

aactttattt agctttttct ggcgtgtaaa ctaacatact aagttgtgtg actataattc 4260

atttagtgac tcatttttag ctatttttat aacacattgt gctatggggg gttttggaac 4320

ttgctggaag ctacatcaga aactgccata gttaattgcc atttcaagaa tgttgtaaat 4380

aactcaggtg gccgtttaat tctcaatgta aatataatta actagacatc tttcctatat 4440

ttgtgtctca gttttaaagc tatttctgga tgcttgagtc ttaccgtaat tgataacaaa 4500

aagaggttat tgagaatatc tatgatttac agagtaagtt attctagacc tcaagagtga 4560

aatgtagggg aggagacatt tgtgtgttaa actaatggaa atgctcattt aatagatatt 4620

cactgaaagt attagttttg gtttattgct agaaaagttg aggttttatg gagatttttg 4680

taaaaaatgg tttatttcct aaataaatat ctctttttct tttttctccc agaaaatgtt 4740

aatctatgat ccagccaaac gaatttctgg caaaatggca ctgaatcatc catattttaa 4800

tgatttggac aatcagatta agaagatggg atccggttct ggcgtgaa 4848

<210> 21
<211> 8507
<212> DNA
<213> Artificial Sequence

<220>
<223> synthetic DNA

<400> 21
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg taagaagatg tagccctcgt tttagagcta gaaatagcaa gttaaaataa 300

ggctagtccg ttatcaactt gaaaaagtgg caccgagtcg gtgctttttt gttttagagc 360

tagaaatagc aagttaaaat aaggctagtc cgtttttagc gcgtgcgcca attctgcaga 420

caaatggctc tagaggtacc cgttacataa cttacggtaa atggcccgcc tggctgaccg 480

cccaacgacc cccgcccatt gacgtcaata gtaacgccaa tagggacttt ccattgacgt 540

caatgggtgg agtatttacg gtaaactgcc cacttggcag tacatcaagt gtatcatatg 600

ccaagtacgc cccctattga cgtcaatgac ggtaaatggc ccgcctggca ttgtgcccag 660

tacatgacct tatgggactt tcctacttgg cagtacatct acgtattagt catcgctatt 720

accatggtcg aggtgagccc cacgttctgc ttcactctcc ccatctcccc cccctcccca 780

cccccaattt tgtatttatt tattttttaa ttattttgtg cagcgatggg ggcggggggg 840

gggggggggc gcgcgccagg cggggcgggg cggggcgagg ggcggggcgg ggcgaggcgg 900

agaggtgcgg cggcagccaa tcagagcggc gcgctccgaa agtttccttt tatggcgagg 960

cggcggcggc ggcggcccta taaaaagcga agcgcgcggc gggcgggagt cgctgcgacg 1020

ctgccttcgc cccgtgcccc gctccgccgc cgcctcgcgc cgcccgcccc ggctctgact 1080

gaccgcgtta ctcccacagg tgagcgggcg ggacggccct tctcctccgg gctgtaatta 1140

gctgagcaag aggtaagggt ttaaggatgg gttggttggt ggggtattaa tgtttaatta 1200

cctggagcac ctgcctgaaa tcactttttt tcaggttgga ccggtgccac catggactat 1260

aaggaccacg acggagacta caaggatcat gatattgatt acaaagacga tgacgataag 1320

atggccccaa agaagaagcg gaaggtcggt atccacggag tcccagcagc cgacaagaag 1380

tacagcatcg gcctggacat cggcaccaac tctgtgggct gggccgtgat caccgacgag 1440

tacaaggtgc ccagcaagaa attcaaggtg ctgggcaaca ccgaccggca cagcatcaag 1500

aagaacctga tcggagccct gctgttcgac agcggcgaaa cagccgaggc cacccggctg 1560

aagagaaccg ccagaagaag atacaccaga cggaagaacc ggatctgcta tctgcaagag 1620

atcttcagca acgagatggc caaggtggac gacagcttct tccacagact ggaagagtcc 1680

ttcctggtgg aagaggataa gaagcacgag cggcacccca tcttcggcaa catcgtggac 1740

gaggtggcct accacgagaa gtaccccacc atctaccacc tgagaaagaa actggtggac 1800

agcaccgaca aggccgacct gcggctgatc tatctggccc tggcccacat gatcaagttc 1860

cggggccact tcctgatcga gggcgacctg aaccccgaca acagcgacgt ggacaagctg 1920

ttcatccagc tggtgcagac ctacaaccag ctgttcgagg aaaaccccat caacgccagc 1980

ggcgtggacg ccaaggccat cctgtctgcc agactgagca agagcagacg gctggaaaat 2040

ctgatcgccc agctgcccgg cgagaagaag aatggcctgt tcggaaacct gattgccctg 2100

agcctgggcc tgacccccaa cttcaagagc aacttcgacc tggccgagga tgccaaactg 2160

cagctgagca aggacaccta cgacgacgac ctggacaacc tgctggccca gatcggcgac 2220

cagtacgccg acctgtttct ggccgccaag aacctgtccg acgccatcct gctgagcgac 2280

atcctgagag tgaacaccga gatcaccaag gcccccctga gcgcctctat gatcaagaga 2340

tacgacgagc accaccagga cctgaccctg ctgaaagctc tcgtgcggca gcagctgcct 2400

gagaagtaca aagagatttt cttcgaccag agcaagaacg gctacgccgg ctacattgac 2460

ggcggagcca gccaggaaga gttctacaag ttcatcaagc ccatcctgga aaagatggac 2520

ggcaccgagg aactgctcgt gaagctgaac agagaggacc tgctgcggaa gcagcggacc 2580

ttcgacaacg gcagcatccc ccaccagatc cacctgggag agctgcacgc cattctgcgg 2640

cggcaggaag atttttaccc attcctgaag gacaaccggg aaaagatcga gaagatcctg 2700

accttccgca tcccctacta cgtgggccct ctggccaggg gaaacagcag attcgcctgg 2760

atgaccagaa agagcgagga aaccatcacc ccctggaact tcgaggaagt ggtggacaag 2820

ggcgcttccg cccagagctt catcgagcgg atgaccaact tcgataagaa cctgcccaac 2880

gagaaggtgc tgcccaagca cagcctgctg tacgagtact tcaccgtgta taacgagctg 2940

accaaagtga aatacgtgac cgagggaatg agaaagcccg ccttcctgag cggcgagcag 3000

aaaaaggcca tcgtggacct gctgttcaag accaaccgga aagtgaccgt gaagcagctg 3060

aaagaggact acttcaagaa aatcgagtgc ttcgactccg tggaaatctc cggcgtggaa 3120

gatcggttca acgcctccct gggcacatac cacgatctgc tgaaaattat caaggacaag 3180

gacttcctgg acaatgagga aaacgaggac attctggaag atatcgtgct gaccctgaca 3240

ctgtttgagg acagagagat gatcgaggaa cggctgaaaa cctatgccca cctgttcgac 3300

gacaaagtga tgaagcagct gaagcggcgg agatacaccg gctggggcag gctgagccgg 3360

aagctgatca acggcatccg ggacaagcag tccggcaaga caatcctgga tttcctgaag 3420

tccgacggct tcgccaacag aaacttcatg cagctgatcc acgacgacag cctgaccttt 3480

aaagaggaca tccagaaagc ccaggtgtcc ggccagggcg atagcctgca cgagcacatt 3540

gccaatctgg ccggcagccc cgccattaag aagggcatcc tgcagacagt gaaggtggtg 3600

gacgagctcg tgaaagtgat gggccggcac aagcccgaga acatcgtgat cgaaatggcc 3660

agagagaacc agaccaccca gaagggacag aagaacagcc gcgagagaat gaagcggatc 3720

gaagagggca tcaaagagct gggcagccag atcctgaaag aacaccccgt ggaaaacacc 3780

cagctgcaga acgagaagct gtacctgtac tacctgcaga atgggcggga tatgtacgtg 3840

gaccaggaac tggacatcaa ccggctgtcc gactacgatg tggaccatat cgtgcctcag 3900

agctttctga aggacgactc catcgacaac aaggtgctga ccagaagcga caagaaccgg 3960

ggcaagagcg acaacgtgcc ctccgaagag gtcgtgaaga agatgaagaa ctactggcgg 4020

cagctgctga acgccaagct gattacccag agaaagttcg acaatctgac caaggccgag 4080

agaggcggcc tgagcgaact ggataaggcc ggcttcatca agagacagct ggtggaaacc 4140

cggcagatca caaagcacgt ggcacagatc ctggactccc ggatgaacac taagtacgac 4200

gagaatgaca agctgatccg ggaagtgaaa gtgatcaccc tgaagtccaa gctggtgtcc 4260

gatttccgga aggatttcca gttttacaaa gtgcgcgaga tcaacaacta ccaccacgcc 4320

cacgacgcct acctgaacgc cgtcgtggga accgccctga tcaaaaagta ccctaagctg 4380

gaaagcgagt tcgtgtacgg cgactacaag gtgtacgacg tgcggaagat gatcgccaag 4440

agcgagcagg aaatcggcaa ggctaccgcc aagtacttct tctacagcaa catcatgaac 4500

tttttcaaga ccgagattac cctggccaac ggcgagatcc ggaagcggcc tctgatcgag 4560

acaaacggcg aaaccgggga gatcgtgtgg gataagggcc gggattttgc caccgtgcgg 4620

aaagtgctga gcatgcccca agtgaatatc gtgaaaaaga ccgaggtgca gacaggcggc 4680

ttcagcaaag agtctatcct gcccaagagg aacagcgata agctgatcgc cagaaagaag 4740

gactgggacc ctaagaagta cggcggcttc gacagcccca ccgtggccta ttctgtgctg 4800

gtggtggcca aagtggaaaa gggcaagtcc aagaaactga agagtgtgaa agagctgctg 4860

gggatcacca tcatggaaag aagcagcttc gagaagaatc ccatcgactt tctggaagcc 4920

aagggctaca aagaagtgaa aaaggacctg atcatcaagc tgcctaagta ctccctgttc 4980

gagctggaaa acggccggaa gagaatgctg gcctctgccg gcgaactgca gaagggaaac 5040

gaactggccc tgccctccaa atatgtgaac ttcctgtacc tggccagcca ctatgagaag 5100

ctgaagggct cccccgagga taatgagcag aaacagctgt ttgtggaaca gcacaagcac 5160

tacctggacg agatcatcga gcagatcagc gagttctcca agagagtgat cctggccgac 5220

gctaatctgg acaaagtgct gtccgcctac aacaagcacc gggataagcc catcagagag 5280

caggccgaga atatcatcca cctgtttacc ctgaccaatc tgggagcccc tgccgccttc 5340

aagtactttg acaccaccat cgaccggaag aggtacacca gcaccaaaga ggtgctggac 5400

gccaccctga tccaccagag catcaccggc ctgtacgaga cacggatcga cctgtctcag 5460

ctgggaggcg acaaaaggcc ggcggccacg aaaaaggccg gccaggcaaa aaagaaaaag 5520

taagaattcc tagagctcgc tgatcagcct cgactgtgcc ttctagttgc cagccatctg 5580

ttgtttgccc ctcccccgtg ccttccttga ccctggaagg tgccactccc actgtccttt 5640

cctaataaaa tgaggaaatt gcatcgcatt gtctgagtag gtgtcattct attctggggg 5700

gtggggtggg gcaggacagc aagggggagg attgggaaga gaatagcagg catgctgggg 5760

agcggccgca ggaaccccta gtgatggagt tggccactcc ctctctgcgc gctcgctcgc 5820

tcactgaggc cgggcgacca aaggtcgccc gacgcccggg ctttgcccgg gcggcctcag 5880

tgagcgagcg agcgcgcagc tgcctgcagg ggcgcctgat gcggtatttt ctccttacgc 5940

atctgtgcgg tatttcacac cgcatacgtc aaagcaacca tagtacgcgc cctgtagcgg 6000

cgcattaagc gcggcgggtg tggtggttac gcgcagcgtg accgctacac ttgccagcgc 6060

cctagcgccc gctcctttcg ctttcttccc ttcctttctc gccacgttcg ccggctttcc 6120

ccgtcaagct ctaaatcggg ggctcccttt agggttccga tttagtgctt tacggcacct 6180

cgaccccaaa aaacttgatt tgggtgatgg ttcacgtagt gggccatcgc cctgatagac 6240

ggtttttcgc cctttgacgt tggagtccac gttctttaat agtggactct tgttccaaac 6300

tggaacaaca ctcaacccta tctcgggcta ttcttttgat ttataaggga ttttgccgat 6360

ttcggcctat tggttaaaaa atgagctgat ttaacaaaaa tttaacgcga attttaacaa 6420

aatattaacg tttacaattt tatggtgcac tctcagtaca atctgctctg atgccgcata 6480

gttaagccag ccccgacacc cgccaacacc cgctgacgcg ccctgacggg cttgtctgct 6540

cccggcatcc gcttacagac aagctgtgac cgtctccggg agctgcatgt gtcagaggtt 6600

ttcaccgtca tcaccgaaac gcgcgagacg aaagggcctc gtgatacgcc tatttttata 6660

ggttaatgtc atgataataa tggtttctta gacgtcaggt ggcacttttc ggggaaatgt 6720

gcgcggaacc cctatttgtt tatttttcta aatacattca aatatgtatc cgctcatgag 6780

acaataaccc tgataaatgc ttcaataata ttgaaaaagg aagagtatga gtattcaaca 6840

tttccgtgtc gcccttattc ccttttttgc ggcattttgc cttcctgttt ttgctcaccc 6900

agaaacgctg gtgaaagtaa aagatgctga agatcagttg ggtgcacgag tgggttacat 6960

cgaactggat ctcaacagcg gtaagatcct tgagagtttt cgccccgaag aacgttttcc 7020

aatgatgagc acttttaaag ttctgctatg tggcgcggta ttatcccgta ttgacgccgg 7080

gcaagagcaa ctcggtcgcc gcatacacta ttctcagaat gacttggttg agtactcacc 7140

agtcacagaa aagcatctta cggatggcat gacagtaaga gaattatgca gtgctgccat 7200

aaccatgagt gataacactg cggccaactt acttctgaca acgatcggag gaccgaagga 7260

gctaaccgct tttttgcaca acatggggga tcatgtaact cgccttgatc gttgggaacc 7320

ggagctgaat gaagccatac caaacgacga gcgtgacacc acgatgcctg tagcaatggc 7380

aacaacgttg cgcaaactat taactggcga actacttact ctagcttccc ggcaacaatt 7440

aatagactgg atggaggcgg ataaagttgc aggaccactt ctgcgctcgg cccttccggc 7500

tggctggttt attgctgata aatctggagc cggtgagcgt ggaagccgcg gtatcattgc 7560

agcactgggg ccagatggta agccctcccg tatcgtagtt atctacacga cggggagtca 7620

ggcaactatg gatgaacgaa atagacagat cgctgagata ggtgcctcac tgattaagca 7680

ttggtaactg tcagaccaag tttactcata tatactttag attgatttaa aacttcattt 7740

ttaatttaaa aggatctagg tgaagatcct ttttgataat ctcatgacca aaatccctta 7800

acgtgagttt tcgttccact gagcgtcaga ccccgtagaa aagatcaaag gatcttcttg 7860

agatcctttt tttctgcgcg taatctgctg cttgcaaaca aaaaaaccac cgctaccagc 7920

ggtggtttgt ttgccggatc aagagctacc aactcttttt ccgaaggtaa ctggcttcag 7980

cagagcgcag ataccaaata ctgtccttct agtgtagccg tagttaggcc accacttcaa 8040

gaactctgta gcaccgccta catacctcgc tctgctaatc ctgttaccag tggctgctgc 8100

cagtggcgat aagtcgtgtc ttaccgggtt ggactcaaga cgatagttac cggataaggc 8160

gcagcggtcg ggctgaacgg ggggttcgtg cacacagccc agcttggagc gaacgaccta 8220

caccgaactg agatacctac agcgtgagct atgagaaagc gccacgcttc ccgaagggag 8280

aaaggcggac aggtatccgg taagcggcag ggtcggaaca ggagagcgca cgagggagct 8340

tccaggggga aacgcctggt atctttatag tcctgtcggg tttcgccacc tctgacttga 8400

gcgtcgattt ttgtgatgct cgtcaggggg gcggagccta tggaaaaacg ccagcaacgc 8460

ggccttttta cggttcctgg ccttttgctg gccttttgct cacatgt 8507

<210> 22

<211>  8507
<212>  DNA
<213>  Artificial Sequence

<220>
<223>  synthetic DNA

<400>  22
catatacgat acaaggctgt tagagagata attggaatta atttgactgt aaacacaaag      60

atattagtac aaaatacgtg acgtagaaag taataatttc ttgggtagtt tgcagtttta     120

aaattatgtt ttaaaatgga ctatcatatg cttaccgtaa cttgaaagta tttcgatttc     180

ttggctttat atatcttgtg gaaaggacga aacaccgaca gggacatcca tccaggtttt     240

agagctagaa atagcaagtt aaaataaggc tagtccgtta tcaacttgaa aaagtggcac     300

cgagtcggtg cttttttgtt ttagagctag aaatagcaag ttaaaataag gctagtccgt     360

ttttagcgcg tgcgccaatt ctgcagacaa atggctctag aggtacccgt tacataactt     420

acggtaaatg gcccgcctgg ctgaccgccc aacgaccccc gcccattgac gtcaatagta     480

acgccaatag ggactttcca ttgacgtcaa tgggtggagt atttacggta aactgcccac     540

ttggcagtac atcaagtgta tcatatgcca agtacgcccc ctattgacgt caatgacggt     600

aaatggcccg cctggcattg tgcccagtac atgaccttat gggactttcc tacttggcag     660

tacatctacg tattagtcat cgctattacc atggtcgagg tgagccccac gttctgcttc     720

actctcccca tctccccccc ctccccaccc ccaattttgt atttatttat tttttaatta     780

ttttgtgcag cgatgggggc gggggggggg ggggggcgcg cgccaggcgg ggcggggcgg     840

ggcgaggggc ggggcggggc gaggcggaga ggtgcggcgg cagccaatca gagcggcgcg     900

ctccgaaagt ttccttttat ggcgaggcgg cggcggcggc ggccctataa aaagcgaagc     960

gcgcggcggg cgggagtcgc tgcgacgctg ccttcgcccc gtgccccgct ccgccgccgc    1020

ctcgcgccgc ccgccccggc tctgactgac cgcgttactc ccacaggtga gcgggcggga    1080

cggcccttct cctccgggct gtaattagct gagcaagagg taagggttta agggatggtt    1140

ggttggtggg gtattaatgt ttaattacct ggagcacctg cctgaaatca ctttttttca    1200

ggttggaccg gtgccaccat ggactataag gaccacgacg gagactacaa ggatcatgat    1260

attgattaca aagacgatga cgataagatg gccccaaaga agaagcggaa ggtcggtatc    1320

cacggagtcc cagcagccga caagaagtac agcatcggcc tggacatcgg caccaactct    1380

gtgggctggg ccgtgatcac cgacgagtac aaggtgccca gcaagaaatt caaggtgctg    1440

ggcaacaccg accggcacag catcaagaag aacctgatcg gagccctgct gttcgacagc    1500

ggcgaaacag ccgaggccac ccggctgaag agaaccgcca gaagaagata caccagacgg    1560

aagaaccgga tctgctatct gcaagagatc ttcagcaacg agatggccaa ggtggacgac    1620

agcttcttcc acagactgga agagtccttc ctggtggaag aggataagaa gcacgagcgg    1680

caccccatct tcggcaacat cgtggacgag gtggcctacc acgagaagta ccccaccatc 1740

taccacctga gaaagaaact ggtggacagc accgacaagg ccgacctgcg gctgatctat 1800

ctggccctgg cccacatgat caagttccgg ggccacttcc tgatcgaggg cgacctgaac 1860

cccgacaaca gcgacgtgga caagctgttc atccagctgg tgcagaccta caaccagctg 1920

ttcgaggaaa accccatcaa cgccagcggc gtggacgcca aggccatcct gtctgccaga 1980

ctgagcaaga gcagacggct ggaaaatctg atcgcccagc tgcccggcga gaagaagaat 2040

ggcctgttcg gaaacctgat tgccctgagc ctgggcctga cccccaactt caagagcaac 2100

ttcgacctgg ccgaggatgc caaactgcag ctgagcaagg acacctacga cgacgacctg 2160

gacaacctgc tggcccagat cggcgaccag tacgccgacc tgtttctggc cgccaagaac 2220

ctgtccgacg ccatcctgct gagcgacatc ctgagagtga acaccgagat caccaaggcc 2280

cccctgagcg cctctatgat caagagatac gacgagcacc accaggacct gaccctgctg 2340

aaagctctcg tgcggcagca gctgcctgag aagtacaaag agattttctt cgaccagagc 2400

aagaacggct acgccggcta cattgacggc ggagccagcc aggaagagtt ctacaagttc 2460

atcaagccca tcctggaaaa gatggacggc accgaggaac tgctcgtgaa gctgaacaga 2520

gaggacctgc tgcggaagca gcggaccttc gacaacggca gcatccccca ccagatccac 2580

ctgggagagc tgcacgccat tctgcggcgg caggaagatt tttacccatt cctgaaggac 2640

aaccgggaaa agatcgagaa gatcctgacc ttccgcatcc cctactacgt gggccctctg 2700

gccaggggaa acagcagatt cgcctggatg accagaaaga gcgaggaaac catcaccccc 2760

tggaacttcg aggaagtggt ggacaagggc gcttccgccc agagcttcat cgagcggatg 2820

accaacttcg ataagaacct gcccaacgag aaggtgctgc ccaagcacag cctgctgtac 2880

gagtacttca ccgtgtataa cgagctgacc aaagtgaaat acgtgaccga gggaatgaga 2940

aagcccgcct tcctgagcgg cgagcagaaa aaggccatcg tggacctgct gttcaagacc 3000

aaccggaaag tgaccgtgaa gcagctgaaa gaggactact tcaagaaaat cgagtgcttc 3060

gactccgtgg aaatctccgg cgtggaagat cggttcaacg cctccctggg cacataccac 3120

gatctgctga aaattatcaa ggacaaggac ttcctggaca atgaggaaaa cgaggacatt 3180

ctggaagata tcgtgctgac cctgacactg tttgaggaca gagagatgat cgaggaacgg 3240

ctgaaaacct atgcccacct gttcgacgac aaagtgatga agcagctgaa gcggcggaga 3300

tacaccggct ggggcaggct gagccggaag ctgatcaacg gcatccggga caagcagtcc 3360

ggcaagacaa tcctggattt cctgaagtcc gacggcttcg ccaacagaaa cttcatgcag 3420

ctgatccacg acgacagcct gacctttaaa gaggacatcc agaaagccca ggtgtccggc 3480

cagggcgata gcctgcacga gcacattgcc aatctggccg gcagccccgc cattaagaag 3540

ggcatcctgc agacagtgaa ggtggtggac gagctcgtga aagtgatggg ccggcacaag 3600

cccgagaaca tcgtgatcga aatggccaga gagaaccaga ccacccagaa gggacagaag 3660

aacagccgcg agagaatgaa gcggatcgaa gagggcatca aagagctggg cagccagatc 3720

ctgaaagaac accccgtgga aaacacccag ctgcagaacg agaagctgta cctgtactac 3780

ctgcagaatg ggcgggatat gtacgtggac caggaactgg acatcaaccg gctgtccgac 3840

tacgatgtgg accatatcgt gcctcagagc tttctgaagg acgactccat cgacaacaag 3900

gtgctgacca gaagcgacaa gaaccggggc aagagcgaca acgtgccctc cgaagaggtc 3960

gtgaagaaga tgaagaacta ctggcggcag ctgctgaacg ccaagctgat tacccagaga 4020

aagttcgaca atctgaccaa ggccgagaga ggcggcctga gcgaactgga taaggccggc 4080

ttcatcaaga gacagctggt ggaaacccgg cagatcacaa agcacgtggc acagatcctg 4140

gactcccgga tgaacactaa gtacgacgag aatgacaagc tgatccggga agtgaaagtg 4200

atcaccctga agtccaagct ggtgtccgat ttccggaagg atttccagtt ttacaaagtg 4260

cgcgagatca acaactacca ccacgcccac gacgcctacc tgaacgccgt cgtgggaacc 4320

gccctgatca aaaagtaccc taagctggaa agcgagttcg tgtacggcga ctacaaggtg 4380

tacgacgtgc ggaagatgat cgccaagagc gagcaggaaa tcggcaaggc taccgccaag 4440

tacttcttct acagcaacat catgaacttt ttcaagaccg agattaccct ggccaacggc 4500

gagatccgga agcggcctct gatcgagaca aacggcgaaa ccggggagat cgtgtgggat 4560

aagggccggg attttgccac cgtgcggaaa gtgctgagca tgccccaagt gaatatcgtg 4620

aaaaagaccg aggtgcagac aggcggcttc agcaaagagt ctatcctgcc caagaggaac 4680

agcgataagc tgatcgccag aaagaaggac tgggacccta agaagtacgg cggcttcgac 4740

agccccaccg tggcctattc tgtgctggtg gtggccaaag tggaaaaggg caagtccaag 4800

aaactgaaga gtgtgaaaga gctgctgggg atcaccatca tggaaagaag cagcttcgag 4860

aagaatccca tcgactttct ggaagccaag ggctacaaag aagtgaaaaa ggacctgatc 4920

atcaagctgc ctaagtactc cctgttcgag ctggaaaacg gccggaagag aatgctggcc 4980

tctgccggcg aactgcagaa gggaaacgaa ctggccctgc cctccaaata tgtgaacttc 5040

ctgtacctgg ccagccacta tgagaagctg aagggctccc ccgaggataa tgagcagaaa 5100

cagctgtttg tggaacagca caagcactac ctggacgaga tcatcgagca gatcagcgag 5160

ttctccaaga gagtgatcct ggccgacgct aatctggaca aagtgctgtc cgcctacaac 5220

aagcaccggg ataagcccat cagagagcag gccgagaata tcatccacct gtttaccctg 5280

accaatctgg gagcccctgc cgccttcaag tactttgaca ccaccatcga ccggaagagg 5340

tacaccagca ccaaagaggt gctggacgcc accctgatcc accagagcat caccggcctg 5400

tacgagacac ggatcgacct gtctcagctg ggaggcgaca aaaggccggc ggccacgaaa 5460

aaggccggcc aggcaaaaaa gaaaaagtaa gaattcctag agctcgctga tcagcctcga 5520

ctgtgccttc tagttgccag ccatctgttg tttgcccctc ccccgtgcct tccttgaccc 5580

tggaaggtgc cactcccact gtcctttcct aataaaatga ggaaattgca tcgcattgtc 5640

tgagtaggtg tcattctatt ctggggggtg gggtggggca ggacagcaag ggggaggatt 5700

gggaagagaa tagcaggcat gctggggagc ggccgcagga acccctagtg atggagttgg 5760

ccactccctc tctgcgcgct cgctcgctca ctgaggccgg gcgaccaaag gtcgcccgac 5820

gcccgggctt tgcccgggcg gcctcagtga gcgagcgagc gcgcagctgc ctgcaggggc 5880

gcctgatgcg gtattttctc cttacgcatc tgtgcggtat ttcacaccgc atacgtcaaa 5940

gcaaccatag tacgcgccct gtagcggcgc attaagcgcg gcgggtgtgg tggttacgcg 6000

cagcgtgacc gctacacttg ccagcgccct agcgcccgct cctttcgctt tcttcccttc 6060

ctttctcgcc acgttcgccg gctttccccg tcaagctcta aatcgggggc tccctttagg 6120

gttccgattt agtgctttac ggcacctcga ccccaaaaaa cttgatttgg gtgatggttc 6180

acgtagtggg ccatcgccct gatagacggt ttttcgccct ttgacgttgg agtccacgtt 6240

ctttaatagt ggactcttgt tccaaactgg aacaacactc aaccctatct cgggctattc 6300

ttttgattta taagggattt tgccgatttc ggcctattgg ttaaaaaatg agctgattta 6360

acaaaaattt aacgcgaatt ttaacaaaat attaacgttt acaattttat ggtgcactct 6420

cagtacaatc tgctctgatg ccgcatagtt aagccagccc cgacacccgc caacacccgc 6480

tgacgcgccc tgacgggctt gtctgctccc ggcatccgct tacagacaag ctgtgaccgt 6540

ctccgggagc tgcatgtgtc agaggttttc accgtcatca ccgaaacgcg cgagacgaaa 6600

gggcctcgtg atacgcctat ttttataggt taatgtcatg ataataatgg tttcttagac 6660

gtcaggtggc acttttcggg gaaatgtgcg cggaacccct atttgtttat ttttctaaat 6720

acattcaaat atgtatccgc tcatgagaca ataaccctga taaatgcttc aataatattg 6780

aaaaaggaag agtatgagta ttcaacattt ccgtgtcgcc cttattccct tttttgcggc 6840

attttgcctt cctgtttttg ctcacccaga aacgctggtg aaagtaaaag atgctgaaga 6900

tcagttgggt gcacgagtgg gttacatcga actggatctc aacagcggta agatccttga 6960

gagttttcgc cccgaagaac gttttccaat gatgagcact tttaaagttc tgctatgtgg 7020

cgcggtatta tcccgtattg acgccgggca agagcaactc ggtcgccgca tacactattc 7080

tcagaatgac ttggttgagt actcaccagt cacagaaaag catcttacgg atggcatgac 7140

agtaagagaa ttatgcagtg ctgccataac catgagtgat aacactgcgg ccaacttact 7200

tctgacaacg atcggaggac cgaaggagct aaccgctttt ttgcacaaca tgggggatca 7260

tgtaactcgc cttgatcgtt gggaaccgga gctgaatgaa gccataccaa acgacgagcg 7320

tgacaccacg atgcctgtag caatggcaac aacgttgcgc aaactattaa ctggcgaact 7380

acttactcta gcttcccggc aacaattaat agactggatg gaggcggata aagttgcagg 7440

accacttctg cgctcggccc ttccggctgg ctggtttatt gctgataaat ctggagccgg 7500

tgagcgtgga agccgcggta tcattgcagc actggggcca gatggtaagc cctcccgtat 7560

cgtagttatc tacacgacgg ggagtcaggc aactatggat gaacgaaata gacagatcgc 7620

tgagataggt gcctcactga ttaagcattg gtaactgtca gaccaagttt actcatatat 7680

actttagatt gatttaaaac ttcattttta atttaaaagg atctaggtga agatcctttt 7740

tgataatctc atgaccaaaa tcccttaacg tgagttttcg ttccactgag cgtcagaccc 7800

cgtagaaaag atcaaaggat cttcttgaga tccttttttt ctgcgcgtaa tctgctgctt 7860

gcaaacaaaa aaaccaccgc taccagcggt ggtttgtttg ccggatcaag agctaccaac 7920

tctttttccg aaggtaactg gcttcagcag agcgcagata ccaaatactg tccttctagt 7980

gtagccgtag ttaggccacc acttcaagaa ctctgtagca ccgcctacat acctcgctct 8040

gctaatcctg ttaccagtgg ctgctgccag tggcgataag tcgtgtctta ccgggttgga 8100

ctcaagacga tagttaccgg ataaggcgca gcggtcgggc tgaacggggg gttcgtgcac 8160

acagcccagc ttggagcgaa cgacctacac cgaactgaga tacctacagc gtgagctatg 8220

agaaagcgcc acgcttcccg aagggagaaa ggcggacagg tatccggtaa gcggcagggt 8280

cggaacagga gagcgcacga gggagcttcc agggggaaac gcctggtatc tttatagtcc 8340

tgtcgggttt cgccacctct gacttgagcg tcgatttttg tgatgctcgt caggggggcg 8400

gagcctatgg aaaaacgcca gcaacgcggc ctttttacgg ttcctggcct tttgctggcc 8460

ttttgctcac atgtgagggc ctatttccca tgattccttc atatttg 8507

<210> 23
<211> 35
<212> DNA
<213> Mus musculus

<400> 23
acagggacat ccatccagag ggctacatct tctta 35

<210> 24
<211> 8509
<212> DNA
<213> Artificial Sequence

<220>
<223> synthetic DNA

<400> 24
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg ctatctgttg acataacata gttttagagc tagaaatagc aagttaaaat 300

aaggctagtc cgttatcaac ttgaaaaagt ggcaccgagt cggtgctttt ttgttttaga 360

gctagaaata gcaagttaaa ataaggctag tccgttttta gcgcgtgcgc caattctgca 420

gacaaatggc tctagaggta cccgttacat aacttacggt aaatggcccg cctggctgac 480

cgcccaacga cccccgccca ttgacgtcaa tagtaacgcc aatagggact ttccattgac 540

gtcaatgggt ggagtattta cggtaaactg cccacttggc agtacatcaa gtgtatcata 600

tgccaagtac gccccctatt gacgtcaatg acggtaaatg gcccgcctgg cattgtgccc 660

agtacatgac cttatgggac tttcctactt ggcagtacat ctacgtatta gtcatcgcta 720

ttaccatggt cgaggtgagc cccacgttct gcttcactct ccccatctcc cccccctccc 780

cacccccaat tttgtattta tttatttttt aattattttg tgcagcgatg ggggcggggg 840

gggggggggg gcgcgcgcca ggcggggcgg ggcggggcga ggggcggggc ggggcgaggc 900

ggagaggtgc ggcggcagcc aatcagagcg gcgcgctccg aaagtttcct tttatggcga 960

ggcggcggcg gcggcggccc tataaaaagc gaagcgcgcg gcgggcggga gtcgctgcga 1020

cgctgccttc gccccgtgcc ccgctccgcc gccgcctcgc gccgcccgcc ccggctctga 1080

ctgaccgcgt tactcccaca ggtgagcggg cgggacggcc cttctcctcc gggctgtaat 1140

tagctgagca agaggtaagg gtttaaggga tggttggttg gtggggtatt aatgtttaat 1200

tacctggagc acctgcctga aatcactttt tttcaggttg gaccggtgcc accatggact 1260

ataaggacca cgacggagac tacaaggatc atgatattga ttacaaagac gatgacgata 1320

agatggcccc aaagaagaag cggaaggtcg gtatccacgg agtcccagca gccgacaaga 1380

agtacagcat cggcctggac atcggcacca actctgtggg ctgggccgtg atcaccgacg 1440

agtacaaggt gcccagcaag aaattcaagg tgctgggcaa caccgaccgg cacagcatca 1500

agaagaacct gatcggagcc ctgctgttcg acagcggcga aacagccgag gccacccggc 1560

tgaagagaac cgccagaaga agatacacca gacggaagaa ccggatctgc tatctgcaag 1620

agatcttcag caacgagatg gccaaggtgg acgacagctt cttccacaga ctggaagagt 1680

ccttcctggt ggaagaggat aagaagcacg agcggcaccc catcttcggc aacatcgtgg 1740

acgaggtggc ctaccacgag aagtacccca ccatctacca cctgagaaag aaactggtgg 1800

acagcaccga caaggccgac ctgcggctga tctatctggc cctggcccac atgatcaagt 1860

tccggggcca cttcctgatc gagggcgacc tgaaccccga caacagcgac gtggacaagc 1920

tgttcatcca gctggtgcag acctacaacc agctgttcga ggaaaacccc atcaacgcca 1980

gcggcgtgga cgccaaggcc atcctgtctg ccagactgag caagagcaga cggctggaaa 2040

atctgatcgc ccagctgccc ggcgagaaga agaatggcct gttcggaaac ctgattgccc 2100

tgagcctggg cctgaccccc aacttcaaga gcaacttcga cctggccgag gatgccaaac 2160

tgcagctgag caaggacacc tacgacgacg acctggacaa cctgctggcc cagatcggcg 2220

accagtacgc cgacctgttt ctggccgcca agaacctgtc cgacgccatc ctgctgagcg 2280

acatcctgag agtgaacacc gagatcacca aggcccccct gagcgcctct atgatcaaga 2340

gatacgacga gcaccaccag gacctgaccc tgctgaaagc tctcgtgcgg cagcagctgc 2400

ctgagaagta caaagagatt ttcttcgacc agagcaagaa cggctacgcc ggctacattg 2460

acggcggagc cagccaggaa gagttctaca agttcatcaa gcccatcctg gaaaagatgg 2520

acggcaccga ggaactgctc gtgaagctga acagagagga cctgctgcgg aagcagcgga 2580

ccttcgacaa cggcagcatc ccccaccaga tccacctggg agagctgcac gccattctgc 2640

ggcggcagga agatttttac ccattcctga aggacaaccg ggaaaagatc gagaagatcc 2700

tgaccttccg catcccctac tacgtgggcc ctctggccag gggaaacagc agattcgcct 2760

ggatgaccag aaagagcgag gaaaccatca ccccctggaa cttcgaggaa gtggtggaca 2820

agggcgcttc cgcccagagc ttcatcgagc ggatgaccaa cttcgataag aacctgccca 2880

acgagaaggt gctgcccaag cacagcctgc tgtacgagta cttcaccgtg tataacgagc 2940

tgaccaaagt gaaatacgtg accgagggaa tgagaaagcc cgccttcctg agcggcgagc 3000

agaaaaaggc catcgtggac ctgctgttca agaccaaccg gaaagtgacc gtgaagcagc 3060

tgaaagagga ctacttcaag aaaatcgagt gcttcgactc cgtggaaatc tccggcgtgg 3120

aagatcggtt caacgcctcc ctgggcacat accacgatct gctgaaaatt atcaaggaca 3180

aggacttcct ggacaatgag gaaaacgagg acattctgga agatatcgtg ctgaccctga 3240

cactgtttga ggacagagag atgatcgagg aacggctgaa aacctatgcc cacctgttcg 3300

acgacaaagt gatgaagcag ctgaagcggc ggagatacac cggctggggc aggctgagcc 3360

ggaagctgat caacggcatc cgggacaagc agtccggcaa gacaatcctg gatttcctga 3420

agtccgacgg cttcgccaac agaaacttca tgcagctgat ccacgacgac agcctgacct 3480

ttaaagagga catccagaaa gcccaggtgt ccggccaggg cgatagcctg cacgagcaca 3540

ttgccaatct ggccggcagc cccgccatta agaagggcat cctgcagaca gtgaaggtgg 3600

tggacgagct cgtgaaagtg atgggccggc acaagcccga gaacatcgtg atcgaaatgg 3660

ccagagagaa ccagaccacc cagaagggac agaagaacag ccgcgagaga atgaagcgga 3720

tcgaagaggg catcaaagag ctgggcagcc agatcctgaa agaacacccc gtggaaaaca 3780

cccagctgca gaacgagaag ctgtacctgt actacctgca gaatgggcgg gatatgtacg 3840

tggaccagga actggacatc aaccggctgt ccgactacga tgtggaccat atcgtgcctc 3900

agagctttct gaaggacgac tccatcgaca acaaggtgct gaccagaagc gacaagaacc 3960

ggggcaagag cgacaacgtg ccctccgaag aggtcgtgaa gaagatgaag aactactggc 4020

ggcagctgct gaacgccaag ctgattaccc agagaaagtt cgacaatctg accaaggccg 4080

agagaggcgg cctgagcgaa ctggataagg ccggcttcat caagagacag ctggtggaaa 4140

cccggcagat cacaaagcac gtggcacaga tcctggactc ccggatgaac actaagtacg 4200

acgagaatga caagctgatc cgggaagtga aagtgatcac cctgaagtcc aagctggtgt 4260

ccgatttccg gaaggatttc cagttttaca aagtgcgcga gatcaacaac taccaccacg 4320

cccacgacgc ctacctgaac gccgtcgtgg gaaccgccct gatcaaaaag taccctaagc 4380

tggaaagcga gttcgtgtac ggcgactaca aggtgtacga cgtgcggaag atgatcgcca 4440

agagcgagca ggaaatcggc aaggctaccg ccaagtactt cttctacagc aacatcatga 4500

actttttcaa gaccgagatt accctggcca acggcgagat ccggaagcgg cctctgatcg 4560

agacaaacgg cgaaaccggg gagatcgtgt gggataaggg ccgggatttt gccaccgtgc 4620

ggaaagtgct gagcatgccc caagtgaata tcgtgaaaaa gaccgaggtg cagacaggcg 4680

gcttcagcaa agagtctatc ctgcccaaga ggaacagcga taagctgatc gccagaaaga 4740

aggactggga ccctaagaag tacggcggct tcgacagccc caccgtggcc tattctgtgc 4800

tggtggtggc caaagtggaa aagggcaagt ccaagaaact gaagagtgtg aaagagctgc 4860

tggggatcac catcatggaa agaagcagct tcgagaagaa tcccatcgac tttctggaag 4920

ccaagggcta caaagaagtg aaaaaggacc tgatcatcaa gctgcctaag tactccctgt 4980

tcgagctgga aaacggccgg aagagaatgc tggcctctgc cggcgaactg cagaagggaa 5040

acgaactggc cctgccctcc aaatatgtga acttcctgta cctggccagc cactatgaga 5100

agctgaaggg ctcccccgag gataatgagc agaaacagct gtttgtggaa cagcacaagc 5160

actacctgga cgagatcatc gagcagatca gcgagttctc caagagagtg atcctggccg 5220

acgctaatct ggacaaagtg ctgtccgcct acaacaagca ccgggataag cccatcagag 5280

agcaggccga gaatatcatc cacctgttta ccctgaccaa tctgggagcc cctgccgcct 5340

tcaagtactt tgacaccacc atcgaccgga agaggtacac cagcaccaaa gaggtgctgg 5400

acgccaccct gatccaccag agcatcaccg gcctgtacga gacacggatc gacctgtctc 5460

agctgggagg cgacaaaagg ccggcggcca cgaaaaaggc cggccaggca aaaaagaaaa 5520

agtaagaatt cctagagctc gctgatcagc ctcgactgtg ccttctagtt gccagccatc 5580

tgttgtttgc ccctcccccg tgccttcctt gaccctggaa ggtgccactc ccactgtcct 5640

ttcctaataa aatgaggaaa ttgcatcgca ttgtctgagt aggtgtcatt ctattctggg 5700

gggtggggtg gggcaggaca gcaaggggga ggattgggaa gagaatagca ggcatgctgg 5760

ggagcggccg caggaacccc tagtgatgga gttggccact ccctctctgc gcgctcgctc 5820

gctcactgag gccgggcgac caaaggtcgc ccgacgcccg ggctttgccc gggcggcctc 5880

agtgagcgag cgagcgcgca gctgcctgca ggggcgcctg atgcggtatt ttctccttac 5940

gcatctgtgc ggtatttcac accgcatacg tcaaagcaac catagtacgc gccctgtagc 6000

ggcgcattaa gcgcggcggg tgtggtggtt acgcgcagcg tgaccgctac acttgccagc 6060

gccctagcgc ccgctccttt cgctttcttc ccttcctttc tcgccacgtt cgccggcttt 6120

ccccgtcaag ctctaaatcg ggggctccct ttagggttcc gatttagtgc tttacggcac 6180

ctcgacccca aaaaacttga tttgggtgat ggttcacgta gtgggccatc gccctgatag 6240

acggtttttc gccctttgac gttggagtcc acgttcttta atagtggact cttgttccaa 6300

actggaacaa cactcaaccc tatctcgggc tattcttttg atttataagg gattttgccg 6360

atttcggcct attggttaaa aaatgagctg atttaacaaa aatttaacgc gaattttaac 6420

aaaatattaa cgtttacaat tttatggtgc actctcagta caatctgctc tgatgccgca 6480

tagttaagcc agccccgaca cccgccaaca cccgctgacg cgccctgacg ggcttgtctg 6540

ctcccggcat ccgcttacag acaagctgtg accgtctccg ggagctgcat gtgtcagagg 6600

ttttcaccgt catcaccgaa acgcgcgaga cgaaagggcc tcgtgatacg cctattttta 6660

taggttaatg tcatgataat aatggtttct tagacgtcag gtggcacttt tcggggaaat 6720

gtgcgcggaa cccctatttg tttatttttc taaatacatt caaatatgta tccgctcatg 6780

agacaataac cctgataaat gcttcaataa tattgaaaaa ggaagagtat gagtattcaa 6840

catttccgtg tcgcccttat tccctttttt gcggcatttt gccttcctgt ttttgctcac 6900

ccagaaacgc tggtgaaagt aaaagatgct gaagatcagt tgggtgcacg agtgggttac 6960

atcgaactgg atctcaacag cggtaagatc cttgagagtt ttcgccccga agaacgtttt 7020

ccaatgatga gcacttttaa agttctgcta tgtggcgcgg tattatcccg tattgacgcc 7080

gggcaagagc aactcggtcg ccgcatacac tattctcaga atgacttggt tgagtactca 7140

ccagtcacag aaaagcatct tacggatggc atgacagtaa gagaattatg cagtgctgcc 7200

ataaccatga gtgataacac tgcggccaac ttacttctga caacgatcgg aggaccgaag 7260

gagctaaccg cttttttgca caacatgggg gatcatgtaa ctcgccttga tcgttgggaa 7320

ccggagctga atgaagccat accaaacgac gagcgtgaca ccacgatgcc tgtagcaatg 7380

gcaacaacgt tgcgcaaact attaactggc gaactactta ctctagcttc ccggcaacaa 7440

ttaatagact ggatggaggc ggataaagtt gcaggaccac ttctgcgctc ggcccttccg 7500

gctggctggt ttattgctga taaatctgga gccggtgagc gtggaagccg cggtatcatt 7560

gcagcactgg ggccagatgg taagccctcc cgtatcgtag ttatctacac gacggggagt 7620

caggcaacta tggatgaacg aaatagacag atcgctgaga taggtgcctc actgattaag 7680

cattggtaac tgtcagacca agtttactca tatatacttt agattgattt aaaacttcat 7740

ttttaattta aaaggatcta ggtgaagatc ctttttgata atctcatgac caaaatccct 7800

taacgtgagt tttcgttcca ctgagcgtca gaccccgtag aaaagatcaa aggatcttct 7860

tgagatcctt tttttctgcg cgtaatctgc tgcttgcaaa caaaaaaacc accgctacca 7920

gcggtggttt gtttgccgga tcaagagcta ccaactcttt ttccgaaggt aactggcttc 7980

agcagagcgc agataccaaa tactgtcctt ctagtgtagc cgtagttagg ccaccacttc 8040

aagaactctg tagcaccgcc tacatacctc gctctgctaa tcctgttacc agtggctgct 8100

gccagtggcg ataagtcgtg tcttaccggg ttggactcaa gacgatagtt accggataag 8160

gcgcagcggt cgggctgaac ggggggttcg tgcacacagc ccagcttgga gcgaacgacc 8220

tacaccgaac tgagatacct acagcgtgag ctatgagaaa gcgccacgct tcccgaaggg 8280

agaaaggcgg acaggtatcc ggtaagcggc agggtcggaa caggagagcg cacgagggag 8340

cttccagggg gaaacgcctg gtatctttat agtcctgtcg ggtttcgcca cctctgactt 8400

gagcgtcgat ttttgtgatg ctcgtcaggg gggcggagcc tatggaaaaa cgccagcaac 8460

gcggcctttt tacggttcct ggccttttgc tggccttttg ctcacatgt 8509

<210> 25
<211> 9177
<212> DNA
<213> Artificial Sequence

<220>
<223> synthetic DNA

<400> 25
   ctggcctttt gctcacatgt gagggcctat ttcccatgat tccttcatat ttgcatatac 60
   gatacaaggc tgttagagag ataattggaa ttaatttgac tgtaaacaca aagatattag 120
   tacaaaatac gtgacgtaga aagtaataat ttcttgggta gtttgcagtt ttaaaattat 180
   gttttaaaat ggactatcat atgcttaccg taacttgaaa gtatttcgat ttcttggctt 240
   tatatatctt gtggaaagga cgaaacaccg ctatctgttg acataacata gttttagagc 300
   tagaaatagc aagttaaaat aaggctagtc cgttatcaac ttgaaaaagt ggcaccgagt 360
   cggtgctttt ttgttttaga gctagaaata gcaagttaaa ataaggctag tccgttttta 420
   gcgcgtgcgc caattctgca gacaaatggc tctagaggta cccgttacat aacttacggt 480
   aaatggcccg cctggctgac cgcccaacga cccccgccca ttgacgtcaa tagtaacgcc 540
   aatagggact ttccattgac gtcaatgggt ggagtattta cggtaaactg cccacttggc 600
   agtacatcaa gtgtatcata tgccaagtac gccccctatt gacgtcaatg acggtaaatg 660
   gcccgcctgg cattgtgccc agtacatgac cttatgggac tttcctactt ggcagtacat 720
   ctacgtatta gtcatcgcta ttaccatggt cgaggtgagc cccacgttct gcttcactct 780
   ccccatctcc cccccctccc cacccccaat tttgtattta tttatttttt aattattttg 840
   tgcagcgatg ggggcggggg gggggggggg gcgcgcgcca ggcggggcgg ggcggggcga 900
   ggggcggggc ggggcgaggc ggagaggtgc ggcggcagcc aatcagagcg gcgcgctccg 960
   aaagtttcct tttatggcga ggcggcggcg gcggcggccc tataaaaagc gaagcgcgcg 1020
   gcgggcggga gtcgctgcga cgctgccttc gccccgtgcc ccgctccgcc gccgcctcgc 1080
   gccgcccgcc ccggctctga ctgaccgcgt tactcccaca ggtgagcggg cgggacggcc 1140
   cttctcctcc gggctgtaat tagctgagca agaggtaagg gtttaaggga tggttggttg 1200
   gtggggtatt aatgtttaat tacctggagc acctgcctga aatcactttt tttcaggttg 1260
   gaccggtgcc accatggact ataaggacca cgacggagac tacaaggatc atgatattga 1320
   ttacaaagac gatgacgata agatggcccc aaagaagaag cggaaggtcg gtatccacgg 1380
   agtcccagca gccgacaaga agtacagcat cggcctggac atcggcacca actctgtggg 1440
   ctgggccgtg atcaccgacg agtacaaggt gcccagcaag aaattcaagg tgctgggcaa 1500
   caccgaccgg cacagcatca agaagaacct gatcggagcc ctgctgttcg acagcggcga 1560
   aacagccgag gccacccggc tgaagagaac cgccagaaga agatacacca gacggaagaa 1620
   ccggatctgc tatctgcaag agatcttcag caacgagatg gccaaggtgg acgacagctt 1680
   cttccacaga ctggaagagt ccttcctggt ggaagaggat aagaagcacg agcggcaccc 1740
   catcttcggc aacatcgtgg acgaggtggc ctaccacgag aagtacccca ccatctacca 1800
   cctgagaaag aaactggtgg acagcaccga caaggccgac ctgcggctga tctatctggc 1860
   cctggcccac atgatcaagt tccggggcca cttcctgatc gagggcgacc tgaaccccga 1920
   caacagcgac gtggacaagc tgttcatcca gctggtgcag acctacaacc agctgttcga 1980
   ggaaaacccc atcaacgcca gcggcgtgga cgccaaggcc atcctgtctg ccagactgag 2040
   caagagcaga cggctggaaa atctgatcgc ccagctgccc ggcgagaaga agaatggcct 2100
   gttcggaaac ctgattgccc tgagcctggg cctgaccccc aacttcaaga gcaacttcga 2160
   cctggccgag gatgccaaac tgcagctgag caaggacacc tacgacgacg acctggacaa 2220
   cctgctggcc cagatcggcg accagtacgc cgacctgttt ctggccgcca agaacctgtc 2280
   cgacgccatc ctgctgagcg acatcctgag agtgaacacc gagatcacca aggcccccct 2340
   gagcgcctct atgatcaaga gatacgacga gcaccaccag gacctgaccc tgctgaaagc 2400
   tctcgtgcgg cagcagctgc ctgagaagta caaagagatt ttcttcgacc agagcaagaa 2460
   cggctacgcc ggctacattg acggcggagc cagccaggaa gagttctaca agttcatcaa 2520
   gcccatcctg gaaaagatgg acggcaccga ggaactgctc gtgaagctga acagagagga 2580
   cctgctgcgg aagcagcgga ccttcgacaa cggcagcatc ccccaccaga tccacctggg 2640
   agagctgcac gccattctgc ggcggcagga agatttttac ccattcctga aggacaaccg 2700
   ggaaaagatc gagaagatcc tgaccttccg catcccctac tacgtgggcc ctctggccag 2760
   gggaaacagc agattcgcct ggatgaccag aaagagcgag gaaaccatca ccccctggaa 2820
   cttcgaggaa gtggtggaca agggcgcttc cgcccagagc ttcatcgagc ggatgaccaa 2880
   cttcgataag aacctgccca acgagaaggt gctgcccaag cacagcctgc tgtacgagta 2940
   cttcaccgtg tataacgagc tgaccaaagt gaaatacgtg accgagggaa tgagaaagcc 3000
   cgccttcctg agcggcgagc agaaaaaggc catcgtggac ctgctgttca agaccaaccg 3060
   gaaagtgacc gtgaagcagc tgaaagagga ctacttcaag aaaatcgagt gcttcgactc 3120
   cgtggaaatc tccggcgtgg aagatcggtt caacgcctcc ctgggcacat accacgatct 3180
   gctgaaaatt atcaaggaca aggacttcct ggacaatgag gaaaacgagg acattctgga 3240
   agatatcgtg ctgaccctga cactgtttga ggacagagag atgatcgagg aacggctgaa 3300
   aacctatgcc cacctgttcg acgacaaagt gatgaagcag ctgaagcggc ggagatacac 3360
   cggctggggc aggctgagcc ggaagctgat caacggcatc cgggacaagc agtccggcaa 3420
   gacaatcctg gatttcctga agtccgacgg cttcgccaac agaaacttca tgcagctgat 3480
   ccacgacgac agcctgacct ttaaagagga catccagaaa gcccaggtgt ccggccaggg 3540
   cgatagcctg cacgagcaca ttgccaatct ggccggcagc cccgccatta agaagggcat 3600
   cctgcagaca gtgaaggtgg tggacgagct cgtgaaagtg atgggccggc acaagcccga 3660
   gaacatcgtg atcgaaatgg ccagagagaa ccagaccacc cagaagggac agaagaacag 3720
   ccgcgagaga atgaagcgga tcgaagaggg catcaaagag ctgggcagcc agatcctgaa 3780
   agaacacccc gtggaaaaca cccagctgca gaacgagaag ctgtacctgt actacctgca 3840
   gaatgggcgg gatatgtacg tggaccagga actggacatc aaccggctgt ccgactacga 3900
   tgtggaccat atcgtgcctc agagctttct gaaggacgac tccatcgaca acaaggtgct 3960
   gaccagaagc gacaagaacc ggggcaagag cgacaacgtg ccctccgaag aggtcgtgaa 4020
   gaagatgaag aactactggc ggcagctgct gaacgccaag ctgattaccc agagaaagtt 4080
   cgacaatctg accaaggccg agagaggcgg cctgagcgaa ctggataagg ccggcttcat 4140
   caagagacag ctggtggaaa cccggcagat cacaaagcac gtggcacaga tcctggactc 4200
   ccggatgaac actaagtacg acgagaatga caagctgatc cgggaagtga aagtgatcac 4260
   cctgaagtcc aagctggtgt ccgatttccg gaaggatttc cagttttaca aagtgcgcga 4320
   gatcaacaac taccaccacg cccacgacgc ctacctgaac gccgtcgtgg gaaccgccct 4380
   gatcaaaaag taccctaagc tggaaagcga gttcgtgtac ggcgactaca aggtgtacga 4440
   cgtgcggaag atgatcgcca agagcgagca ggaaatcggc aaggctaccg ccaagtactt 4500
   cttctacagc aacatcatga actttttcaa gaccgagatt accctggcca acggcgagat 4560
   ccggaagcgg cctctgatcg agacaaacgg cgaaaccggg gagatcgtgt gggataaggg 4620
   ccgggatttt gccaccgtgc ggaaagtgct gagcatgccc caagtgaata tcgtgaaaaa 4680
   gaccgaggtg cagacaggcg gcttcagcaa agagtctatc ctgcccaaga ggaacagcga 4740
   taagctgatc gccagaaaga aggactggga ccctaagaag tacggcggct tcgacagccc 4800
   caccgtggcc tattctgtgc tggtggtggc caaagtggaa aagggcaagt ccaagaaact 4860
   gaagagtgtg aaagagctgc tggggatcac catcatggaa agaagcagct tcgagaagaa 4920
   tcccatcgac tttctggaag ccaagggcta caaagaagtg aaaaaggacc tgatcatcaa 4980
   gctgcctaag tactccctgt tcgagctgga aaacggccgg aagagaatgc tggcctctgc 5040
   cggcgaactg cagaagggaa acgaactggc cctgccctcc aaatatgtga acttcctgta 5100
   cctggccagc cactatgaga agctgaaggg ctcccccgag gataatgagc agaaacagct 5160
   gtttgtggaa cagcacaagc actacctgga cgagatcatc gagcagatca gcgagttctc 5220
   caagagagtg atcctggccg acgctaatct ggacaaagtg ctgtccgcct acaacaagca 5280
   ccgggataag cccatcagag agcaggccga gaatatcatc cacctgttta ccctgaccaa 5340
   tctgggagcc cctgccgcct tcaagtactt tgacaccacc atcgaccgga agaggtacac 5400
   cagcaccaaa gaggtgctgg acgccaccct gatccaccag agcatcaccg gcctgtacga 5460
   gacacggatc gacctgtctc agctgggagg cgacaaaagg ccggcggcca cgaaaaaggc 5520
   cggccaggca aaaaagaaaa aggaattcgg cagtggagag ggcagaggaa gtctgctaac 5580
   atgcggtgac gtcgaggaga atcctggccc aatgaccgag tacaagccca cggtgcgcct 5640
   cgccacccgc gacgacgtcc ccagggccgt acgcaccctc gccgccgcgt tcgccgacta 5700
   ccccgccacg cgccacaccg tcgatccgga ccgccacatc gagcgggtca ccgagctgca 5760
   agaactcttc ctcacgcgcg tcgggctcga catcggcaag gtgtgggtcg cggacgacgg 5820
   cgccgcggtg gcggtctgga ccacgccgga gagcgtcgaa gcgggggcgg tgttcgccga 5880
   gatcggcccg cgcatggccg agttgagcgg ttcccggctg gccgcgcagc aacagatgga 5940
   aggcctcctg gcgccgcacc ggcccaagga gcccgcgtgg ttcctggcca ccgtcggagt 6000
   ctcgcccgac caccagggca agggtctggg cagcgccgtc gtgctccccg gagtggaggc 6060
   ggccgagcgc gccggggtgc ccgccttcct ggagacctcc gcgccccgca acctcccctt 6120
   ctacgagcgg ctcggcttca ccgtcaccgc cgacgtcgag gtgcccgaag gaccgcgcac 6180
   ctggtgcatg acccgcaagc ccggtgcctg agaattctaa ctagagctcg ctgatcagcc 6240
   tcgactgtgc cttctagttg ccagccatct gttgtttgcc cctcccccgt gccttccttg 6300
   accctggaag gtgccactcc cactgtcctt tcctaataaa atgaggaaat tgcatcgcat 6360
   tgtctgagta ggtgtcattc tattctgggg ggtggggtgg ggcaggacag caagggggag 6420
   gattgggaag agaatagcag gcatgctggg gagcggccgc aggaacccct agtgatggag 6480
   ttggccactc cctctctgcg cgctcgctcg ctcactgagg ccgggcgacc aaaggtcgcc 6540
   cgacgcccgg gctttgcccg ggcggcctca gtgagcgagc gagcgcgcag ctgcctgcag 6600
   gggcgcctga tgcggtattt tctccttacg catctgtgcg gtatttcaca ccgcatacgt 6660
   caaagcaacc atagtacgcg ccctgtagcg gcgcattaag cgcggcgggt gtggtggtta 6720
   cgcgcagcgt gaccgctaca cttgccagcg ccctagcgcc cgctcctttc gctttcttcc 6780
   cttcctttct cgccacgttc gccggctttc cccgtcaagc tctaaatcgg gggctccctt 6840
   tagggttccg atttagtgct ttacggcacc tcgaccccaa aaaacttgat ttgggtgatg 6900
   gttcacgtag tgggccatcg ccctgataga cggtttttcg ccctttgacg ttggagtcca 6960
   cgttctttaa tagtggactc ttgttccaaa ctggaacaac actcaaccct atctcgggct 7020
   attcttttga tttataaggg attttgccga tttcggccta ttggttaaaa aatgagctga 7080
   tttaacaaaa atttaacgcg aattttaaca aaatattaac gtttacaatt ttatggtgca 7140
   ctctcagtac aatctgctct gatgccgcat agttaagcca gccccgacac ccgccaacac 7200
   ccgctgacgc gccctgacgg gcttgtctgc tcccggcatc cgcttacaga caagctgtga 7260
   ccgtctccgg gagctgcatg tgtcagaggt tttcaccgtc atcaccgaaa cgcgcgagac 7320
   gaaagggcct cgtgatacgc ctatttttat aggttaatgt catgataata atggtttctt 7380
   agacgtcagg tggcactttt cggggaaatg tgcgcggaac ccctatttgt ttatttttct 7440
   aaatacattc aaatatgtat ccgctcatga gacaataacc ctgataaatg cttcaataat 7500
   attgaaaaag gaagagtatg agtattcaac atttccgtgt cgcccttatt cccttttttg 7560
   cggcattttg ccttcctgtt tttgctcacc cagaaacgct ggtgaaagta aaagatgctg 7620
   aagatcagtt gggtgcacga gtgggttaca tcgaactgga tctcaacagc ggtaagatcc 7680
   ttgagagttt tcgccccgaa gaacgttttc caatgatgag cacttttaaa gttctgctat 7740
   gtggcgcggt attatcccgt attgacgccg ggcaagagca actcggtcgc cgcatacact 7800
   attctcagaa tgacttggtt gagtactcac cagtcacaga aaagcatctt acggatggca 7860
   tgacagtaag agaattatgc agtgctgcca taaccatgag tgataacact gcggccaact 7920
   tacttctgac aacgatcgga ggaccgaagg agctaaccgc ttttttgcac aacatggggg 7980
   atcatgtaac tcgccttgat cgttgggaac cggagctgaa tgaagccata ccaaacgacg 8040
   agcgtgacac cacgatgcct gtagcaatgg caacaacgtt gcgcaaacta ttaactggcg 8100
   aactacttac tctagcttcc cggcaacaat taatagactg gatggaggcg gataaagttg 8160
   caggaccact tctgcgctcg gcccttccgg ctggctggtt tattgctgat aaatctggag 8220
   ccggtgagcg tggaagccgc ggtatcattg cagcactggg gccagatggt aagccctccc 8280
   gtatcgtagt tatctacacg acggggagtc aggcaactat ggatgaacga aatagacaga 8340
   tcgctgagat aggtgcctca ctgattaagc attggtaact gtcagaccaa gtttactcat 8400
   atatacttta gattgattta aaacttcatt tttaatttaa aaggatctag gtgaagatcc 8460
   tttttgataa tctcatgacc aaaatccctt aacgtgagtt ttcgttccac tgagcgtcag 8520
   accccgtaga aaagatcaaa ggatcttctt gagatccttt ttttctgcgc gtaatctgct 8580
   gcttgcaaac aaaaaaacca ccgctaccag cggtggtttg tttgccggat caagagctac 8640
   caactctttt tccgaaggta actggcttca gcagagcgca gataccaaat actgtccttc 8700
   tagtgtagcc gtagttaggc caccacttca agaactctgt agcaccgcct acatacctcg 8760
   ctctgctaat cctgttacca gtggctgctg ccagtggcga taagtcgtgt cttaccgggt 8820
   tggactcaag acgatagtta ccggataagg cgcagcggtc gggctgaacg gggggttcgt 8880
   gcacacagcc cagcttggag cgaacgacct acaccgaact gagataccta cagcgtgagc 8940
   tatgagaaag cgccacgctt cccgaaggga gaaaggcgga caggtatccg gtaagcggca 9000
   gggtcggaac aggagagcgc acgagggagc ttccaggggg aaacgcctgg tatctttata 9060
   gtcctgtcgg gtttcgccac ctctgacttg agcgtcgatt tttgtgatgc tcgtcagggg 9120
   ggcggagcct atggaaaaac gccagcaacg cggccttttt acggttcctg gcctttt 9177

<210> 26
<211> 21
<212> DNA
<213> Homo sapiens

<400> 26
ccatatgtta tatcaacaga t 21

<210> 27
<211> 22
<212> DNA
<213> Artificial Sequence

<220>
<223> oligonucleotide

<400> 27
ggttggtcat tgactgttga gg 22

<210> 28
<211> 20
<212> DNA
<213> Artificial Sequence

<220>
<223> oligonucleotide

<400> 28
gtgctggtga caggggtagc 20

<210>  29
<211>  24
<212>  DNA
<213>  Artificial Sequence

<220>
<223>  oligonucleotide

<400>  29
atggccacaa ccatggtgag caag                                              24


<210>  30
<211>  25
<212>  DNA
<213>  Artificial Sequence

<220>
<223>  oligonucleotide

<400>  30
cctcctgcat ttgctggagt caggg                                             25


<210>  31
<211>  22
<212>  DNA
<213>  Artificial Sequence

<220>
<223>  oligonucleotide

<400>  31
tgatggattc gggcaagttt ct                                                22


<210>  32
<211>  20
<212>  DNA
<213>  Artificial Sequence

<220>
<223>  oligonucleotide

<400>  32
acatcggcaa ggtgtgggtc                                                   20


<210>  33
<211>  25
<212>  DNA
<213>  Artificial Sequence

<220>
<223>  oligonucleotide

<400>  33
accgagatcc tccaaacagt agaca                                             25


<210>  34
<211>  14437
<212>  DNA

<213>  Artificial Sequence

<220>
<223>  synthetic DNA


<220>
<221>  misc_feature
<222>  (8819)..(8821)
<223>  n is a, c, g, or t

<400>  34
gtaaaaaggc cgcgttgctg gcgtttttcc ataggctccg cccccctgac gagcatcaca      60

aaaatcgacg ctcaagtcag aggtggcgaa acccgacagg actataaaga taccaggcgt     120

ttccccctgg aagctccctc gtgcgctctc ctgttccgac cctgccgctt accggatacc     180

tgtccgcctt tctcccttcg ggaagcgtgg cgctttctca tagctcacgc tgtaggtatc     240

tcagttcggt gtaggtcgtt cgctccaagc tgggctgtgt gcacgaaccc cccgttcagc     300

ccgaccgctg cgccttatcc ggtaactatc gtcttgagtc caacccggta agacacgact     360

tatcgccact ggcagcagcc actggtaaca ggattagcag agcgaggtat gtaggcggtg     420

ctacagagtt cttgaagtgg tggcctaact acggctacac tagaaggaca gtatttggta     480

tctgcgctct gctgaagcca gttaccttcg gaaaaagagt tggtagctct tgatccggca     540

aacaaaccac cgctggtagc ggtggttttt ttgtttgcaa gcagcagatt acgcgcagaa     600

aaaaaggatc tcaagaagat cctttgatct tttctacggg gtctgacgct cagtggaacg     660

aaaactcacg ttaagggatt ttggtcatga gattatcaaa aaggatcttc acctagatcc     720

ttttaaatta aaaatgaagt tttaaatcaa tctaaagtat atatgagtaa acttggtctg     780

acagttacca atgcttaatc agtgaggcac ctatctcagc gatctgtcta tttcgttcat     840

ccatagttgc ctgactcccc gtcgtgtaga taactacgat acgggagggc ttaccatctg     900

gccccagtgc tgcaatgata ccgcgagacc cacgctcacc ggctccagat ttatcagcaa     960

taaaccagcc agccggaagg gccgagcgca gaagtggtcc tgcaacttta tccgcctcca    1020

tccagtctat taattgttgc cgggaagcta gagtaagtag ttcgccagtt aatagtttgc    1080

gcaacgttgt tgccattgct gcaggcatcg tggtgtcacg ctcgtcgttt ggtatggctt    1140

cattcagctc cggttcccaa cgatcaaggc gagttacatg atcccccatg ttgtgcaaaa    1200

aagcggttag ctccttcggt cctccgatcg ttgtcagaag taagttggcc gcagtgttat    1260

cactcatggt tatggcagca ctgcataatt ctcttactgt catgccatcc gtaagatgct    1320

tttctgtgac tggtgagtac tcaaccaagt cattctgaga atagtgtatg cggcgaccga    1380

gttgctcttg cccggcgtca acacgggata ataccgcgcc acatagcaga actttaaaag    1440

tgctcatcat tggaaaacgt tcttcggggc gaaaactctc aaggatctta ccgctgttga    1500

gatccagttc gatgtaaccc actcgtgcac ccaactgatc ttcagcatct tttactttca    1560

ccagcgtttc tgggtgagca aaaacaggaa ggcaaaatgc cgcaaaaaag ggaataaggg 1620

cgacacggaa atgttgaata ctcatactct tcctttttca atattattga agcatttatc 1680

agggttattg tctcatgagc ggatacatat ttgaatgtat ttagaaaaat aaacaaatag 1740

gggttccgcg cacatttccc cgaaaagtgc cacctgacgt ctaagaaacc attattatca 1800

tgacattaac ctataaaaat aggcgtatca cgaggccctt tcgtcttcaa gaattctcat 1860

gtttgacagc ttatcatcga taagctgatc ctcacaggcc gcacccagct tttcttccgt 1920

tgccccagta gcatctctgt ctggtgacct tgaagaggaa gaggaggggt cccgagaatc 1980

cccatcccta ccgtccagca aaaaggggga cgaggaattt gaggcctggc ttgaggctca 2040

ggacgcaaat cttgaggatg ttcagcggga gttttccggg ctgcgagtaa ttggtgatga 2100

ggacgaggat ggttcggagg atggggaatt ttcagacctg gatctgtctg acagcgacca 2160

tgaaggggat gagggtgggg gggctgttgg agggggcagg agtctgcact ccctgtattc 2220

actgagcgtc gtctaataaa gatgtctatt gatctctttt agtgtgaatc atgtctgacg 2280

aggggccagg tacaggacct ggaaatggcc taggagagaa gggagacaca tctggaccag 2340

aaggctccgg cggcagtgga cctcaaagaa gagggggtga taaccatgga cgaggacggg 2400

gaagaggacg aggacgagga ggcggaagac caggagcccc gggcggctca ggatcagggc 2460

caagacatag agatggtgtc cggagacccc aaaaacgtcc aagttgcatt ggctgcaaag 2520

ggacccacgg tggaacagga gcaggagcag gagcgggagg ggcaggagca ggaggggcag 2580

gagcaggagg aggggcagga gcaggaggag gggcaggagg ggcaggaggg gcaggagggg 2640

caggagcagg aggaggggca ggagcaggag gaggggcagg aggggcagga ggggcaggag 2700

caggaggagg ggcaggagca ggaggagggg caggaggggc aggagcagga ggaggggcag 2760

gaggggcagg aggggcagga gcaggaggag gggcaggagc aggaggaggg gcaggagggg 2820

caggagcagg aggaggggca ggaggggcag gaggggcagg agcaggagga ggggcaggag 2880

caggaggggc aggaggggca ggaggggcag gagcaggagg ggcaggagca ggaggagggg 2940

caggaggggc aggaggggca ggagcaggag gggcaggagc aggaggggca ggagcaggag 3000

gggcaggagc aggaggggca ggaggggcag gagcaggagg ggcaggaggg gcaggagcag 3060

gaggggcagg aggggcagga gcaggaggag gggcaggagg ggcaggagca ggaggagggg 3120

caggaggggc aggagcagga ggggcaggag gggcaggagc aggaggggca ggaggggcag 3180

gagcaggagg ggcaggaggg gcaggagcag gaggaggggc aggagcagga ggggcaggag 3240

caggaggtgg aggccggggt cgaggaggca gtggaggccg gggtcgagga ggtagtggag 3300

gccggggtcg aggaggtagt ggaggccgcc ggggtagagg acgtgaaaga gccagggggg 3360

gaagtcgtga aagagccagg gggagaggtc gtggacgtgg agaaaagagg cccaggagtc 3420

ccagtagtca gtcatcatca tccgggtctc caccgcgcag gccccctcca ggtagaaggc 3480

catttttcca ccctgtaggg gaagccgatt attttgaata ccaccaagaa ggtggcccag 3540

atggtgagcc tgacgtgccc ccgggagcga tagagcaggg ccccgcagat gacccaggag 3600

aaggcccaag cactggaccc cggggtcagg gtgatggagg caggcgcaaa aaaggagggt 3660

ggtttggaaa gcatcgtggt caaggaggtt ccaacccgaa atttgagaac attgcagaag 3720

gtttaagagc tctcctggct aggagtcacg tagaaaggac taccgacgaa ggaacttggg 3780

tcgccggtgt gttcgtatat ggaggtagta agacctccct ttacaaccta aggcgaggaa 3840

ctgcccttgc tattccacaa tgtcgtctta caccattgag tcgtctcccc tttggaatgg 3900

cccctggacc cggcccacaa cctggcccgc taagggagtc cattgtctgt tatttcatgg 3960

tctttttaca aactcatata tttgctgagg ttttgaagga tgcgattaag gaccttgtta 4020

tgacaaagcc cgctcctacc tgcaatatca gggtgactgt gtgcagcttt gacgatggag 4080

tagatttgcc tccctggttt ccacctatgg tggaaggggc tgccgcggag ggtgatgacg 4140

gagatgacgg agatgaagga ggtgatggag atgagggtga ggaagggcag gagtgatgta 4200

acttgttagg agacgccctc aatcgtatta aaagccgtgt attcccccgc actaaagaat 4260

aaatccccag tagacatcat gcgtgctgtt ggtgtatttc tggccatctg tcttgtcacc 4320

attttcgtcc tcccaacatg gggcaattgg gcatacccat gttgtcacgt cactcagctc 4380

cgcgctcaac accttctcgc gttggaaaac attagcgaca tttacctggt gagcaatcag 4440

acatgcgacg gctttagcct ggcctcctta aattcaccta agaatgggag caaccagcag 4500

gaaaaggaca agcagcgaaa attcacgccc ccttgggagg tggcggcata tgcaaaggat 4560

agcactccca ctctactact gggtatcata tgctgactgt atatgcatga ggatagcata 4620

tgctacccgg atacagatta ggatagcata tactacccag atatagatta ggatagcata 4680

tgctacccag atatagatta ggatagccta tgctacccag atataaatta ggatagcata 4740

tactacccag atatagatta ggatagcata tgctacccag atatagatta ggatagccta 4800

tgctacccag atatagatta ggatagcata tgctacccag atatagatta ggatagcata 4860

tgctatccag atatttgggt agtatatgct acccagatat aaattaggat agcatatact 4920

accctaatct ctattaggat agcatatgct acccggatac agattaggat agcatatact 4980

acccagatat agattaggat agcatatgct acccagatat agattaggat agcctatgct 5040

acccagatat aaattaggat agcatatact acccagatat agattaggat agcatatgct 5100

acccagatat agattaggat agcctatgct acccagatat agattaggat agcatatgct 5160

atccagatat ttgggtagta tatgctaccc atggcaacat tagcccaccg tgctctcagc 5220

gacctcgtga atatgaggac caacaaccct gtgcttggcg ctcaggcgca agtgtgtgta 5280

atttgtcctc cagatcgcag caatcgcgcc cctatcttgg cccgcccacc tacttatgca 5340

ggtattcccc ggggtgccat tagtggtttt gtgggcaagt ggtttgaccg cagtggttag 5400

cggggttaca atcagccaag ttattacacc cttattttac agtccaaaac cgcagggcgg 5460

cgtgtggggg ctgacgcgtg cccccactcc acaatttcaa aaaaaagagt ggccacttgt 5520

ctttgtttat gggccccatt ggcgtggagc cccgtttaat tttcgggggt gttagagaca 5580

accagtggag tccgctgctg tcggcgtcca ctctctttcc ccttgttaca aatagagtgt 5640

aacaacatgg ttcacctgtc ttggtccctg cctgggacac atcttaataa ccccagtatc 5700

atattgcact aggattatgt gttgcccata gccataaatt cgtgtgagat ggacatccag 5760

tctttacggc ttgtccccac cccatggatt tctattgtta aagatattca gaatgtttca 5820

ttcctacact agtatttatt gcccaagggg tttgtgaggg ttatattggt gtcatagcac 5880

aatgccacca ctgaaccccc cgtccaaatt ttattctggg ggcgtcacct gaaaccttgt 5940

tttcgagcac ctcacataca ccttactgtt cacaactcag cagttattct attagctaaa 6000

cgaaggagaa tgaagaagca ggcgaagatt caggagagtt cactgcccgc tccttgatct 6060

tcagccactg cccttgtgac taaaatggtt cactaccctc gtggaatcct gaccccatgt 6120

aaataaaacc gtgacagctc atggggtggg agatatcgct gttccttagg acccttttac 6180

taaccctaat tcgatagcat atgcttcccg ttgggtaaca tatgctattg aattagggtt 6240

agtctggata gtatatacta ctacccggga agcatatgct acccgtttag ggttaacaag 6300

ggggccttat aaacactatt gctaatgccc tcttgagggt ccgcttatcg gtagctacac 6360

aggcccctct gattgacgtt ggtgtagcct cccgtagtct tcctgggccc ctgggaggta 6420

catgtccccc agcattggtg taagagcttc agccaagagt tacacataaa ggcaatgttg 6480

tgttgcagtc cacagactgc aaagtctgct ccaggatgaa agccactcag tgttggcaaa 6540

tgtgcacatc catttataag gatgtcaact acagtcagag aacccctttg tgtttggtcc 6600

ccccccgtgt cacatgtgga acagggccca gttggcaagt tgtaccaacc aactgaaggg 6660

attacatgca ctgccccgcg aagaaggggc agagatgtcg tagtcaggtt tagttcgtcc 6720

ggggcgggga tcgatcctct agagtcgact agtaacggcc gccagtgtgc tggaattcgg 6780

cttacaagtt tgtacaaaaa agcaggctag ctcactcatt aggcacccca ggctttacac 6840

tttatgcttc cggctcgtat gttgtgtgga attgtgagcg gataacaatt tcacacagga 6900

aacagctatg accatgatta cgccaagctt gcatgcctgc aggtcgacat tgattattga 6960

ctagttatta atagtaatca attacggggt cattagttca tagcccatat atggagttcc 7020

gcgttacata acttacggta aatggcccgc ctggctgacc gcccaacgac ccccgcccat 7080

tgacgtcaat aatgacgtat gttcccatag taacgccaat agggactttc cattgacgtc 7140

aatgggtgga ctatttacgg taaactgccc acttggcagt acatcaagtg tatcatatgc 7200

caagtacgcc ccctattgac gtcaatgacg gtaaatggcc cgcctggcat tatgcccagt 7260

acatgacctt atgggacttt cctacttggc agtacatcta cgtattagtc atcgctatta 7320

ccatgggtcg aggtgagccc cacgttctgc ttcactctcc ccatctcccc cccctcccca 7380

cccccaattt tgtatttatt tattttttaa ttattttgtg cagcgatggg ggcggggggg 7440

gggggggcgc gcgccaggcg gggcggggcg gggcgagggg cggggcgggg cgaggcggag 7500

aggtgcggcg gcagccaatc agagcggcgc gctccgaaag tttcctttta tggcgaggcg 7560

gcggcggcgg cggccctata aaaagcgaag cgcgcggcgg gcgggagtcg ctgcgttgcc 7620

ttcgccccgt gccccgctcc gcgccgcctc gcgccgcccg ccccggctct gactgaccgc 7680

gttactccca caggtgagcg ggcgggacgg cccttctcct ccgggctgta attagcgctt 7740

ggtttaatga cggctcgttt cttttctgtg gctgcgtgaa agccttaaag ggctccggga 7800

gggccctttg tgcggggggg agcggctcgg ggggtgcgtg cgtgtgtgtg tgcgtgggga 7860

gcgccgcgtg cggcccgcgc tgcccggcgg ctgtgagcgc tgcgggcgcg gcgcggggct 7920

ttgtgcgctc cgcgtgtgcg cgaggggagc gcggccgggg gcggtgcccc gcggtgcggg 7980

ggggctgcga ggggaacaaa ggctgcgtgc ggggtgtgtg cgtggggggg tgagcagggg 8040

gtgtgggcgc ggcggtcggg ctgtaacccc cccctgcacc cccctccccg agttgctgag 8100

cacggcccgg cttcgggtgc ggggctccgt gcggggcgtg gcgcggggct cgccgtgccg 8160

ggcggggggt ggcggcaggt gggggtgccg ggcggggcgg ggccgcctcg ggccggggag 8220

ggctcggggg aggggcgcgg cggccccgga gcgccggcgg ctgtcgaggc gcggcgagcc 8280

gcagccattg ccttttatgg taatcgtgcg agagggcgca gggacttcct ttgtcccaaa 8340

tctggcggag ccgaaatctg ggaggcgccg ccgcaccccc tctagcgggc gcgggcgaag 8400

cggtgcggcg ccggcaggaa ggaaatgggc ggggagggcc ttcgtgcgtc gccgcgccgc 8460

cgtccccttc tccatctcca gcctcggggc tgccgcaggg ggacggctgc cttcgggggg 8520

gacggggcag ggcggggttc ggcttctggc gtgtgaccgg cggctctaga gcctctgcta 8580

accatgttca tgccttcttc tttttcctac agctcctggg caacgtgctg gttgttgtgc 8640

tgtctcatca ttttggcaaa gaattccttc tagaggatcc ccgggtaccg agctcgaccc 8700

aacttttcta tactctatac aaagttggtc agtcagaaac aactttggca catatcaata 8760

ttatgctctc gttaattaac tcgaggtaaa acttaagctt ggtaccgagc tcggatccnn 8820

natgggtagt tctttagacg atgagcatat cctctctgct cttctgcaaa gcgatgacga 8880

gcttgttggt gaggattctg acagtgaaat atcagatcac gtaagtgaag atgacgtcca 8940

gagcgataca gaagaagcgt ttatagatga ggtacatgaa gtgcagccaa cgtcaagcgg 9000

tagtgaaata ttagacgaac aaaatgttat tgaacaacca ggttcttcat tggcttctaa 9060

cagaatcttg accttgccac agaggactat tagaggtaag aataaacatt gttggtcaac 9120

ttcaaagtcc acgaggcgta gccgagtctc tgcactgaac attgtcagat ctcaaagagg 9180

tccgacgcgt atgtgccgca atatatatga cccactttta tgcttcaaac tattttttac 9240

tgatgagata atttcggaaa ttgtaaaatg gacaaatgct gagatatcat tgaaacgtcg 9300

ggaatctatg acaggtgcta catttcgtga cacgaatgaa gatgaaatct atgctttctt 9360

tggtattctg gtaatgacag cagtgagaaa agataaccac atgtccacag atgacctctt 9420

tgatcgatct ttgtcaatgg tgtacgtctc tgtaatgagt cgtgatcgtt ttgatttttt 9480

gatacgatgt cttagaatgg atgacaaaag tatacggccc acacttcgag aaaacgatgt 9540

atttactcct gttagaaaaa tatgggatct ctttatccat cagtgcatac aaaattacac 9600

tccaggggct catttgacca tagatgaaca gttacttggt tttagaggac ggtgtccgtt 9660

taggatgtat atcccaaaca agccaagtaa gtatggaata aaaatcctca tgatgtgtga 9720

cagtggtacg aagtatatga taaatggaat gccttatttg ggaagaggaa cacagaccaa 9780

cggagtacca ctcggtgaat actacgtgaa ggagttatca aagcctgtgc acggtagttg 9840

tcgtaatatt acgtgtgaca attggttcac ctcaatccct ttggcaaaaa acttactaca 9900

agaaccgtat aagttaacca ttgtgggaac cgtgcgatca aacaaacgcg agataccgga 9960

agtactgaaa aacagtcgct ccaggccagt gggaacatcg atgttttgtt ttgacggacc 10020

ccttactctc gtctcatata aaccgaagcc agctaagatg gtatacttat tatcatcttg 10080

tgatgaggat gcttctatca acgaaagtac cggtaaaccg caaatggtta tgtattataa 10140

tcaaactaaa ggcggagtgg acacgctaga ccaaatgtgt tctgtgatga cctgcagtag 10200

gaagacgaat aggtggccta tggcattatt gtacggaatg ataaacattg cctgcataaa 10260

ttcttttatt atatacagcc ataatgtcag tagcaaggga gaaaaggttc aaagtcgcaa 10320

aaaatttatg agaaaccttt acatgagcct gacgtcatcg tttatgcgta agcgtttaga 10380

agctcctact ttgaagagat atttgcgcga taatatctct aatattttgc caaatgaagt 10440

gcctggtaca tcagatgaca gtactgaaga gccagtaatg aaaaaacgta cttactgtac 10500

ttactgcccc tctaaaataa ggcgaaaggc aaatgcatcg tgcaaaaaat gcaaaaaagt 10560

tatttgtcga gagcataata ttgatatgtg ccaaagttgt ttctgactgc ggccgcacta 10620

gtctagagcg atcgcccggg tttaaacgct agcaacaagc tcgtcatcgc tttgcagaag 10680

agcacaactt tgtataataa agttgagcta aaacgcggcc tcgaatcgaa ttccgccccc 10740

ccccctctcc ctcccccccc cctaacgtta ctggccgaag ccgcttggaa taaggccggt 10800

gtgcgtttgt ctatatgtta ttttccacca tattgccgtc ttttggcaat gtgagggccc 10860

ggaaacctgg ccctgtcttc ttgacgagca ttcctagggg tctttcccct ctcgccaaag 10920

gaatgcaagg tctgttgaat gtcgtgaagg aagcagttcc tctggaagct tcttgaagac 10980

aaacaacgtc tgtagcgacc ctttgcaggc agcggaaccc cccacctggc gacaggtgcc 11040

tctgcggcca aaagccacgt gtataagata cacctgcaaa ggcggcacaa ccccagtgcc 11100

acgttgtgag ttggatagtt gtggaaagag tcaaatggct ctcctcaagc gtattcaaca 11160

aggggctgaa ggatgcccag aaggtacccc attgtatggg atctgatctg gggcctcggt 11220

gcacatgctt tacatgtgtt tagtcgaggt taaaaaaacg tctaggcccc ccgaaccacg 11280

gggacgtggt tttcctttga aaaacacgat gataatatgg ccacaaccat gaccgagtac 11340

aagcccacgg tgcgcctcgc cacccgcgac gacgtcccca gggccgtacg caccctcgcc 11400

gccgcgttcg ccgactaccc cgccacgcgc cacaccgtcg atccggaccg ccacatcgag 11460

cgggtcaccg agctgcaaga actcttcctc acgcgcgtcg ggctcgacat cggcaaggtg 11520

tgggtcgcgg acgacggcgc cgcggtggcg gtctggacca cgccggagag cgtcgaagcg 11580

ggggcggtgt tcgccgagat cggcccgcgc atggccgagt tgagcggttc ccggctggcc 11640

gcgcagcaac agatggaagg cctcctggcg ccgcaccggc ccaaggagcc cgcgtggttc 11700

ctggccaccg tcggcgtctc gcccgaccac cagggcaagg gtctgggcag cgccgtcgtg 11760

ctccccggag tggaggcggc cgagcgcgcc ggggtgcccg ccttcctgga gacctccgcg 11820

ccccgcaacc tccccttcta cgagcggctc ggcttcaccg tcaccgccga cgtcgaggtg 11880

cccgaaggac cgcgcacctg gtgcatgacc cgcaagcccg gtgcctgacg cccgccccac 11940

gacccgcagc gcccgaccga aaggagcgca cgaccccatg catcgatgat ctagagctcg 12000

ctgatcagcc tcgactgtgc cttctagttg ccagccatct gttgtttgcc cctcccccgt 12060

gccttccttg accctggaag gtgccactcc cactgtcctt tcctaataaa atgaggaaat 12120

tgcatcgcat tgtctgagta ggtgtcattc tattctgggg ggtggggtgg ggcaggacag 12180

caagggggag gattgggaag acaatagcag gcatgctggg gatgcggtgg gctctatggc 12240

ttctgaggcg gaaagaatac ccagctttct tgtacaaagt ggtaagccga attctgcaga 12300

ttcgactagc ttggcacgcc agaaatccgc gcggtggttt ttgggggtcg ggggtgtttg 12360

gcagccacag acgcccggtg ttcgtgtcgc gccagtacat gcggtccatg cccaggccat 12420

ccaaaaacca tgggtctgtc tgctcagtcc agtcgtggac ctgaccccac gcaacgccca 12480

aaataataac ccccacgaac cataaaccat tccccatggg ggaccccgtc cctaacccac 12540

ggggccagtg gctatggcag ggcctgccgc cccgacgttg gctgcgagcc ctgggccttc 12600

acccgaactt gggggtgggg gtggggaaaa ggaagaaacg cgggcgtatt ggccccaatg 12660

gggtctcggt ggggtatcga cagagtgcca gccctgggac cgaaccccgc gtttatgaac 12720

aaacgaccca acacccgtgc gttttattct gtctttttat tgccgtcata gcgcgggttc 12780

cttccggtat tgtctccttc cgtgtttcag ttagcctccc ccatctcccg atccggacga 12840

gtgctggggc gtcggtttcc actatcggcg agtacttcta cacagccatc ggtccagacg 12900

gccgcgcttc tgcgggcgat ttgtgtacgc ccgacagtcc cggctccgga tcggacgatt 12960

gcgtcgcatc gaccctgcgc ccaagctgca tcatcgaaat tgccgtcaac caagctctga 13020

tagagttggt caagaccaat gcggagcata tacgcccgga gccgcggcga tcctgcaagc 13080

tccggatgcc tccgctcgaa gtagcgcgtc tgctgctcca tacaagccaa ccacggcctc 13140

cagaagaaga tgttggcgac ctcgtattgg gaatccccga acatcgcctc gctccagtca 13200

atgaccgctg ttatgcggcc attgtccgtc aggacattgt tggagccgaa atccgcgtgc 13260

acgaggtgcc ggacttcggg gcagtcctcg gcccaaagca tcagctcatc gagagcctgc 13320

gcgacggacg cactgacggt gtcgtccatc acagtttgcc agtgatacac atggggatca 13380

gcaatcgcgc atatgaaatc acgccatgta gtgtattgac cgattccttg cggtccgaat 13440

gggccgaacc cgctcgtctg gctaagatcg gccgcagcga tcgcatccat ggcctccgcg 13500

accggctgca gaacagcggg cagttcggtt tcaggcaggt cttgcaacgt gacaccctgt 13560

gcacggcggg agatgcaata ggtcaggctc tcgctgaatt ccccaatgtc aagcacttcc 13620

ggaatcggga gcgcggccga tgcaaagtgc cgataaacat aacgatcttt gtagaaacca 13680

tcggcgcagc tatttacccg caggacatat ccacgccctc ctacatcgaa gctgaaagca 13740

cgagattctt cgccctccga gagctgcatc aggtcggaga cgctgtcgaa cttttcgatc 13800

agaaacttct cgacagacgt cgcggtgagt tcaggctttt tcatatctca ttgccccccg 13860

ggatctgcgg cacgctgttg acgctgttaa gcgggtcgct gcagggtcgc tcggtgttcg 13920

aggccacacg cgtcacctta atatgcgaag tggacctcgg accgcgccgc cccgactgca 13980

tctgcgtgtt cgaattcgcc aatgacaaga cgctgggcgg ggtttgtgtc atcatagaac 14040

taaagacatg caaatatatt tcttccgggg acaccgccag caaacgcgag caacgggcca 14100

cggggatgaa gcagggcggc acctcgctaa cggattcacc actccaagaa ttggagccaa 14160

tcaattcttg cggagaactg tgaatgcgca aaccaaccct tggcagaaca tatccatcgc 14220

gtccgccatc tccagcagcc gcacgcggcg catctcgggg ccgacgcgct gggctacgtc 14280

ttgctggcgt tcgcgacgcg aggctggatg gccttcccca ttatgattct tctcgcttcc 14340

ggcggcatcg ggatgcccgc gttgcaggcc atgctgtcca ggcaggtaga tgacgaccat 14400

cagggacagc ttcaaggcca gcaaaaggcc aggaacc 14437

<210> 35
<211> 7602
<212> DNA
<213> Artificial Sequence

<220>
<223> synthetic DNA

<400> 35

actcttcctt tttcaatgac cgctttggaa aaacaaagac tgtatttcct ggaaattaat 60

gtttattcaa taaactgtgt attcagctat atcacatagt ggtgaggctg aaatgaggcg 120

ggaagaggcg gttggggctt aattatatca atttgggtgg ccccacagcg cctccaaggc 180

gccagtcctg ttttgacaag ttgcctctgg aagcctctac aatgcctctc ttctttttct 240

ccagagtaag cggaggccag gggcccccgg cctctgctta atactaaaaa aaacagctgt 300

tgtcatagta atgattgggt ggaaacattc caggcctggg tggagaggct ttttgcttcc 360

tcttgcaaaa ccacactgac attccaggcc tgggtggaga ggctttttgc ttcctcttgc 420

aaaaccacac tgccctctgg agggcagttg cctagcaact aactaaaaga ggatgtcgca 480

cggccagctg cggtcagtta gtcacttcct gcttaactga cttgacattt tctattttaa 540

gagtcgggag gaaaattact gtgttggagg ccctccgcca tcttctgaag ctgaatcgaa 600

ttaacttgtt tattgcagct tataatggtt acaaataaag caatagcatc acaaatttca 660

caaataaagc atttttttca ctgcattcta gttgtggttt gtccaaactc atcaatgtat 720

cttatcatgt ctggatctga tatcatcgtc gacattgatt attgactagt tattaatagt 780

aatcaattac ggggtcatta gttcatagcc catatatgga gttccgcgtt acataactta 840

cggtaaatgg cccgcctggc tgaccgccca acgacccccg cccattgacg tcaataatga 900

cgtatgttcc catagtaacg ccaataggga ctttccattg acgtcaatgg gtggagtatt 960

tacggtaaac tgcccacttg gcagtacatc aagtgtatca tatgccaagt acgcccccta 1020

ttgacgtcaa tgacggtaaa tggcccgcct ggcattatgc ccagtacatg accttatggg 1080

actttcctac ttggcagtac atctacgtat tagtcatcgc tattaccatg gtcgaggtga 1140

gccccacgtt ctgcttcact ctccccatct cccccccctc cccaccccca attttgtatt 1200

tatttatttt ttaattattt tgtgcagcga tgggggcggg gggggggggg gggcgcgcgc 1260

caggcggggc ggggcggggc gaggggcggg gcggggcgag gcggagaggt gcggcggcag 1320

ccaatcagag cggcgcgctc cgaaagtttc cttttatggc gaggcggcgg cggcggcggc 1380

cctataaaaa gcgaagcgcg cggcgggcgg gagtcgctgc gcgctgcctt cgccccgtgc 1440

cccgctccgc gccgcctcgc gccgcccgcc ccggctctga ctgaccgcgt tactcccaca 1500

ggtgagcggg cgggacggcc cttctcctcc gggctgtaat tagcgcttgg tttaatgacg 1560

gcttgtttct tttctgtggc tgcgtgaaag ccttgagggg ctccgggagg gccctttgtg 1620

cggggggagc ggctcggggg gtgcgtgcgt gtgtgtgtgc gtggggagcg ccgcgtgcgg 1680

ctccgcgctg cccggcggct gtgagcgctg cgggcgcggc gcggggcttt gtgcgctccg 1740

cagtgtgcgc gaggggagcg cggccggggg cggtgccccg cggtgcgggg ggggctgcga 1800

ggggaacaaa ggctgcgtgc ggggtgtgtg cgtggggggg tgagcagggg gtgtgggcgc 1860

gtcggtcggg ctgcaacccc ccctgcaccc ccctccccga gttgctgagc acggcccggc 1920

ttcgggtgcg gggctccgta cggggcgtgg cgcggggctc gccgtgccgg gcggggggtg 1980

gcggcaggtg ggggtgccgg gcggggcggg gccgcctcgg gccggggagg gctcggggga 2040

ggggcgcggc ggcccccgga gcgccggcgg ctgtcgaggc gcggcgagcc gcagccattg 2100

ccttttatgg taatcgtgcg agagggcgca gggacttcct ttgtcccaaa tctgtgcgga 2160

gccgaaatct gggaggcgcc gccgcacccc ctctagcggg cgcggggcga agcggtgcgg 2220

cgccggcagg aaggaaatgg gcggggaggg ccttcgtgcg tcgccgcgcc gccgtcccct 2280

tctccctctc cagcctcggg gctgtccgcg gggggacggc tgccttcggg ggggacgggg 2340

cagggcgggg ttcggcttct ggcgtgtgac cggcggctct agagcctctg ctaaccatgt 2400

tcatgccttc ttctttttcc tacagctcct gggcaacgtg ctggttattg tgctgtctca 2460

tcattttggc aaagaatttc gacctgcagc ccaagctgta ccccctcgaa actgacagga 2520

gaaccaccat gggcccaaag aagaagagaa aggtttcgaa tttactgacc gtacaccaaa 2580

atttgcctgc attaccggtc gatgcaacga gtgatgaggt tcgcaagaac ctgatggaca 2640

tgttcaggga tcgccaggcg ttttctgagc atacctggaa aatgcttctg tccgtttgcc 2700

ggtcgtgggc ggcatggtgc aagttgaata accggaaatg gtttcccgca gaacctgaag 2760

atgttcgcga ttatcttcta tatcttcagg cgcgcggtct ggcagtaaaa actatccagc 2820

aacatttggg ccagctaaac atgcttcatc gtcggtccgg gctgccacga ccaagtgaca 2880

gcaatgctgt ttcactggtt atgcggcgga tccgaaaaga aaacgttgat gccggtgaac 2940

gtgcaaaaca ggctctagcg ttcgaacgca ctgatttcga ccaggttcgt tcactcatgg 3000

aaaatagcga tcgctgccag gatatacgta atctggcatt tctggggatt gcttataaca 3060

ccctgttacg tatagccgaa attgccagga tcagggttaa agatatctca cgtactgacg 3120

gtgggagaat gttaatccat attggcagaa cgaaaacgct ggttagcacc gcaggtgtag 3180

agaaggcact tagcctgggg gtaactaaac tggtcgagcg atggatttcc gtctctggtg 3240

tagctgatga tccgaataac tacctgtttt gccgggtcag aaaaaatggt gttgccgcgc 3300

catctgccac cagccagcta tcaactcgcg ccctggaagg gatttttgaa gcaactcatc 3360

gattgattta cggcgctaag gatgactctg gtcagagata cctggcctgg tctggacaca 3420

gtgcccgtgt cggagccgcg cgagatatgg cccgcgctgg agtttcaata ccggagatca 3480

tgcaagctgg tggctggacc aatgtaaata ttgtcatgaa ctatatccgt aacctggata 3540

gtgaaacagg ggcaatggtg cgcctgctgg aagatggcga ttagccatta acgcgcgacc 3600

agcttgatat cgaattaatt cactcctcag gtgcaggctg cctatcagaa ggtggtggct 3660

ggtgtggcca atgccctggc tcacaaatac cactgaatta agaattccgc ccctctccct 3720

cccccccccc taacgttact ggccgaagcc gcttggaata aggccggtgt gcgtttgtct 3780

atatgttatt ttccaccata ttgccgtctt ttggcaatgt gagggcccgg aaacctggcc 3840

ctgtcttctt gacgagcatt cctaggggtc tttcccctct cgccaaagga atgcaaggtc 3900

tgttgaatgt cgtgaaggaa gcagttcctc tggaagcttc ttgaagacaa acaacgtctg 3960

tagcgaccct ttgcaggcag cggaaccccc cacctggcga caggtgcctc tgcggccaaa 4020

agccacgtgt ataagataca cctgcaaagg cggcacaacc ccagtgccac gttgtgagtt 4080

ggatagttgt ggaaagagtc aaatggctct cctcaagcgt attcaacaag gggctgaagg 4140

atgcccagaa ggtaccccat tgtatgggat ctgatctggg gcctcggtgc acatgcttta 4200

catgtgttta gtcgaggtta aaaaacgtct aggccccccg aaccacgggg acgtggtttt 4260

cctttgaaaa acacgatgat aatatggcca caaccatgac cgagtacaag cccacggtgc 4320

gcctcgccac ccgcgacgac gtcccccggg ccgtacgcac cctcgccgcc gcgttcgccg 4380

actaccccgc cacgcgccac accgtcgacc cggaccgcca catcgagcgg gtcaccgagc 4440

tgcaagaact cttcctcacg cgcgtcgggc tcgacatcgg caaggtgtgg gtcgcggacg 4500

acggcgccgc ggtggcggtc tggaccacgc cggagagcgt cgaagcgggg gcggtgttcg 4560

ccgagatcgg cccgcgcatg gccgagttga gcggttcccg gctggccgcg cagcaacaga 4620

tggaaggcct cctggcgccg caccggccca aggagcccgc gtggttcctg gccaccgtcg 4680

gcgtctcgcc cgaccaccag ggcaagggtc tgggcagcgc cgtcgtgctc cccggagtgg 4740

aggcggccga gcgcgccggg gtgcccgcct tcctggagac ctccgcgccc cgcaacctcc 4800

ccttctacga gcggctcggc ttcaccgtca ccgccgacgt cgaggtgccc gaaggaccgc 4860

gcacctggtg catgacccgc aagcccggtg cctgacgccc gccccacgac ccgcagcgcc 4920

cgaccgaaag gagcgcacga ccccatgcat ggatcgatag cctcgactgt gccttctagt 4980

tgccagccat ctgttgtttg cccctccccc gtgccttcct tgaccctgga aggtgccact 5040

cccactgtcc tttcctaata aaatgaggaa attgcatcgc attgtctgag taggtgtcat 5100

tctattctgg ggggtggggt ggggcaggac agcaaggggg aggattggga agacaatagc 5160

aggcatgctg gggatgcggt gggctctatc ttctgaggcg gaaagaacct gcagcccaag 5220

cttggcgtaa tcatggtcat agctgtttcc tgtgtgaaat tgttatccgc tcacaattcc 5280

acacaacata cgagccggaa gcataaagtg taaagcctgg ggtgcctaat gagtgagcta 5340

actcacatta attgcgttgc gctcactgcc cgctttccag tcgggaaacc tgtcgtgcca 5400

gcggatccgc atctcaatta gtcagcaacc atagtcccgc ccctaactcc gcccatcccg 5460

cccctaactc cgcccagttc cgcccattct ccgccccatg gctgactaat tttttttatt 5520

tatgcagagg ccgaggccgc ctcggcctct gagctattcc agaagtagtg aggaggcttt 5580

tttggaggcc taggcttttg caaaaagcta acttgtttat tgcagcttat aatggttaca 5640

aataaagcaa tagcatcaca aatttcacaa ataaagcatt tttttcactg cattctagtt 5700

gtggtttgtc caaactcatc aatgtatctt atcatgtctg gatccgctgc attaatgaat 5760

cggccaacgc gcggggagag gcggtttgcg tattgggcgc tcttccgctt cctcgctcac 5820

tgactcgctg cgctcggtcg ttcggctgcg gcgagcggta tcagctcact caaaggcggt 5880

aatacggtta tccacagaat caggggataa cgcaggaaag aacatgtgag caaaaggcca 5940

gcaaaaggcc aggaaccgta aaaaggccgc gttgctggcg tttttccata ggctccgccc 6000

ccctgacgag catcacaaaa atcgacgctc aagtcagagg tggcgaaacc cgacaggact 6060

ataaagatac caggcgtttc cccctggaag ctccctcgtg cgctctcctg ttccgaccct 6120

gccgcttacc ggatacctgt ccgcctttct cccttcggga agcgtggcgc tttctcaatg 6180

ctcacgctgt aggtatctca gttcggtgta ggtcgttcgc tccaagctgg gctgtgtgca 6240

cgaacccccc gttcagcccg accgctgcgc cttatccggt aactatcgtc ttgagtccaa 6300

cccggtaaga cacgacttat cgccactggc agcagccact ggtaacagga ttagcagagc 6360

gaggtatgta ggcggtgcta cagagttctt gaagtggtgg cctaactacg gctacactag 6420

aaggacagta tttggtatct gcgctctgct gaagccagtt accttcggaa aaagagttgg 6480

tagctcttga tccggcaaac aaaccaccgc tggtagcggt ggtttttttg tttgcaagca 6540

gcagattacg cgcagaaaaa aaggatctca agaagatcct ttgatctttt ctacggggtc 6600

tgacgctcag tggaacgaaa actcacgtta agggattttg gtcatgagat tatcaaaaag 6660

gatcttcacc tagatccttt taaattaaaa atgaagtttt aaatcaatct aaagtatata 6720

tgagtaaact tggtctgaca gttaccaatg cttaatcagt gaggcaccta tctcagcgat 6780

ctgtctattt cgttcatcca tagttgcctg actccccgtc gtgtagataa ctacgatacg 6840

ggagggctta ccatctggcc ccagtgctgc aatgataccg cgagacccac gctcaccggc 6900

tccagattta tcagcaataa accagccagc cggaagggcc gagcgcagaa gtggtcctgc 6960

aactttatcc gcctccatcc agtctattaa ttgttgccgg gaagctagag taagtagttc 7020

gccagttaat agtttgcgca acgttgttgc cattgctaca ggcatcgtgg tgtcacgctc 7080

gtcgtttggt atggcttcat tcagctccgg ttcccaacga tcaaggcgag ttacatgatc 7140

ccccatgttg tgcaaaaaag cggttagctc cttcggtcct ccgatcgttg tcagaagtaa 7200

gttggccgca gtgttatcac tcatggttat ggcagcactg cataattctc ttactgtcat 7260

gccatccgta agatgctttt ctgtgactgg tgagtactca accaagtcat tctgagaata 7320

gtgtatgcgg cgaccgagtt gctcttgccc ggcgtcaata cgggataata ccgcgccaca 7380

tagcagaact ttaaaagtgc tcatcattgg aaaacgttct tcggggcgaa aactctcaag 7440

gatcttaccg ctgttgagat ccagttcgat gtaacccact cgtgcaccca actgatcttc 7500

agcatctttt actttcacca gcgtttctgg gtgagcaaaa acaggaaggc aaaatgccgc 7560

aaaaaaggga ataagggcga cacggaaatg ttgaatactc at                    7602

<210>  36
<211>  20
<212>  DNA
<213>  Artificial Sequence

<220>
<223>  oligonucleotide

<400>  36
gtatctggcg gcagggtagc                                                20

<210>  37
<211>  21
<212>  DNA
<213>  Artificial Sequence

<220>
<223>  oligonucleotide

<400>  37
tttgttgctg gcattgttct g                                              21

<210>  38
<211>  24
<212>  DNA
<213>  Artificial Sequence

<220>
<223>  oligonucleotide

<400>  38
tggacgtaaa ctcctcttca gacc                                           24

<210>  39
<211>  24
<212>  DNA
<213>  Artificial Sequence

<220>
<223>  oligonucleotide

<400>  39
gaggactaca ccatcgtgga acag                                           24

<210>  40
<211>  21
<212>  DNA
<213>  Artificial Sequence

<220>
<223>  oligonucleotide

<400>  40
ggcttccacc aaagaccaca g                                              21

<210>  41
<211>  2041
<212>  DNA
<213>  Artificial Sequence

<220>
<223>  synthetic DNA

<400>  41
taccgttcgt atagcataca ttatacgaag ttataccatg tctagactgg acaagagcaa      60

agtcataaac ggcgctctgg aattactcaa tggagtcggt atcgaaggcc tgacgacaag     120

gaaactcgct caaaagctgg gagttgagca gcctaccctg tactggcacg tgaagaacaa     180

gcgggccctg ctcgatgccc tgccaatcga gatgctggac aggcatcata cccacttctg     240

ccccctggaa ggcgagtcat ggcaagactt tctgcggaac aacgccaagt cattccgctg     300

tgctctcctc tcacatcgcg acggggctaa agtgcatctc ggcacccgcc caacagagaa     360

acagtacgaa accctggaaa atcagctcgc gttcctgtgt cagcaaggct tctccctgga     420

gaacgcactg tacgctctgt ccgccgtggg ccactttaca ctgggctgcg tattggagga     480

acaggagcat caagtagcaa aagaggaaag agagacacct accaccgatt ctatgccccc     540

acttctgaga caagcaattg agctgttcga ccggcaggga gccgaacctg ccttcctttt     600

cggcctggaa ctaatcatat gtggcctgga gaaacagcta aagtgcgaaa gcggcgggcc     660

ggccgacgcc cttgacgatt ttgacttaga catgctccca gccgatgccc ttgacgactt     720

tgaccttgat atgctgcctg ctgacgctct tgacgatttt gaccttgaca tgctccccgg     780

gtaactaagt aaggatccag acatgataag atacattgat gagtttggac aaaccacaac     840

tagaatgcag tgaaaaaaat gctttatttg tgaaatttgt gatgctattg ctttatttgt     900

aaccattata agctgcaata aacaagttaa caacaacaat tgcattcatt ttatgtttca     960

ggttcagggg gaggtgtggg aggtttttta aagcaagtaa aacctctaca aatgtggtat    1020

ggctgattat gatcctctag agtcgcagat ccagacatga taagatacat tgatgagttt    1080

ggacaaacca caactagaat gcagtgaaaa aaatgcttta tttgtgaaat ttgtgatgct    1140

attgctttat ttgtaaccat tataagctgc aataaacaag ttaacaacaa caattgcatt    1200

cattttatgt ttcaggttca gggggaggtg tgggaggttt tttaaagcaa gtaaaacctc    1260

tacaaatgtg gtatggctga ttatgatcct ctagagtcgc agatccagac atgataagat    1320

acattgatga gtttggacaa accacaacta gaatgcagtg aaaaaaatgc tttatttgtg    1380

aaatttgtga tgctattgct ttatttgtaa ccattataag ctgcaataaa caagttaaca    1440

acaacaattg cattcatttt atgtttcagg ttcaggggga ggtgtgggag gttttttaaa    1500

gcaagtaaaa cctctacaaa tgtggtatgg ctgattatga tcctctagag tcgcagatcc    1560

tctagagtcg cagatctttt tccctctgcc aaaaattatg gggacatcat gaagcccctt    1620

gagcatctga cttctggcta ataaaggaaa tttattttca ttgcaatagt gtgttggaat 1680

tttttgtgtc tctcactcgg aaggacatat gggagggcaa atcatttaaa acatcagaat 1740

gagtatttgg tttagagttt ggcaacatat gccatatgct ggctgccatg aacaaaggtg 1800

gctataaaga ggtcatcagt atatgaaaca gccccctgct gtccattcct tattccatag 1860

aaaagccttg acttgaggtt agattttttt tatattttgt tttgtgttat ttttttcttt 1920

aacatcccta aaattttcct tacatgtttt actagccaga tttttcctcc tctcctgact 1980

actcccagtc atagctgtcc ctcttctctt atgaagatcc ctcgacctgc agcccaagct 2040

t 2041

<210> 42
<211> 413
<212> DNA
<213> Artificial Sequence

<220>
<223> synthetic DNA

<400> 42
ccgcggtggc ggccgcacgt ctccctatca gtgatagaga agtcgacacg tctcgagctc 60

cctatcagtg atagagaagg tacgtctaga acgtctccct atcagtgata gagaagtcga 120

cacgtctcga gctccctatc agtgatagag aaggtacgtc tagaacgtct ccctatcagt 180

gatagagaag tcgacacgtc tcgagctccc tatcagtgat agagaaggta cgtctagaac 240

gtctccctat cagtgataga gaagtcgaca cgtctcgagc tccctatcag tgatagagaa 300

ggtaccccct atataagcag agctcgttta gtgaaccgtc agatcgcctg gagacgccat 360

ccacgctgtt ttgacctcca tagaagacac cgggaccgat ccagcctccg cgg 413

<210> 43
<211> 1224
<212> DNA
<213> Artificial Sequence

<220>
<223> synthetic DNA

<400> 43
ggatcccaaa taccactgaa ttaagaattc cgcccctctc cctccccccc ccctaacgtt 60

actggccgaa gccgcttgga ataaggccgg tgtgcgtttg tctatatgtt attttccacc 120

atattgccgt cttttggcaa tgtgagggcc cggaaacctg gccctgtctt cttgacgagc 180

attcctaggg gtctttcccc tctcgccaaa ggaatgcaag gtctgttgaa tgtcgtgaag 240

gaagcagttc ctctggaagc ttcttgaaga caaacaacgt ctgtagcgac cctttgcagg 300

cagcggaacc ccccacctgg cgacaggtgc ctctgcggcc aaaagccacg tgtataagat 360

acacctgcaa aggcggcaca accccagtgc cacgttgtga gttggatagt tgtggaaaga 420

gtcaaatggc tctcctcaag cgtattcaac aaggggctga aggatgccca gaaggtaccc 480

cattgtatgg gatctgatct ggggcctcgg tgcacatgct ttacatgtgt ttagtcgagg 540

ttaaaaaacg tctaggcccc ccgaaccacg gggacgtggt tttcctttga aaaacacgat 600

gataatatgg ccacaaccat gaccgagtac aagcccacgg tgcgcctcgc cacccgcgac 660

gacgtccccc gggccgtacg caccctcgcc gccgcgttcg ccgactaccc cgccacgcgc 720

cacaccgtcg acccggaccg ccacatcgag cgggtcaccg agctgcaaga actcttcctc 780

acgcgcgtcg ggctcgacat cggcaaggtg tgggtcgcgg acgacggcgc cgcggtggcg 840

gtctggacca cgccggagag cgtcgaagcg ggggcggtgt tcgccgagat cggcccgcgc 900

atggccgagt tgagcggttc ccggctggcc gcgcagcaac agatggaagg cctcctggcg 960

ccgcaccggc ccaaggagcc cgcgtggttc ctggccaccg tcggcgtctc gcccgaccac 1020

cagggcaagg gtctgggcag cgccgtcgtg ctccccggag tggaggcggc cgagcgcgcc 1080

ggggtgcccg ccttcctgga gacctccgcg ccccgcaacc tccccttcta cgagcggctc 1140

ggcttcaccg tcaccgccga cgtcgaggtg cccgaaggac cgcgcacctg gtgcatgacc 1200

cgcaagcccg gtgcctgagg atcc 1224

<210> 44
<211> 1044
<212> DNA
<213> Artificial Sequence

<220>
<223> synthetic DNA

<400> 44
actagttaat taagaaactg acctaaagac ttgcctccag ccccatccta tgggagcatt 60

ttcttaactg aggttctctc ctctcaagat gacttcagct tgtgacaatg tgacctggcc 120

taaagctagc cagcacagca caacaccttc cattctttct tctaaggtgc ttatggacat 180

ttctttctct ttgagtacct tgagtgggat tgtctgtggc tgcttttggg aagcattcaa 240

ttgaatgatt tttgtttttt gtttatttcc cccgagacag ggtttctctg tgtagccctg 300

gctgtcctgg aactcacttt gtagacctcg aactcagaaa tccgcctgcc tctgcctccc 360

aagtgctggg attaatggca tgtgccacca ctgcctggag aattgaatgc ttataaggta 420

aacagatgct tttattaaaa acaacaacaa caactgttat tcctagtggt tggactttac 480

agttgtactt tgtttccttt tgaaacagtg ttgcagcgaa cgaggccaca ggttggcctt 540

gaacttgttc ttccctgtgc taggattaca tcgtgcacaa cttccttctc gcagactctt 600

attattttaa gaaaaactaa aacagtacac cattttagta cggtagtagt gtgctgaaat 660

tcagccctgg ggatcagggc cttttgcttt ttttggtaaa ttttgagaca tagtgtcact 720

aagttgcttt agctagctgt gtatcagtct ccacagtggc cggcttgaca ggcctgcttg 780

ctttgtgcat gtgaaggggc taagcccttg gctgtgtaga atatacaaac gcagtgtctg 840

agaaggctca gaatggctca cttgcaagaa cagctaaacc ctaacaagac catctgacca 900

tctgggatga ctgacgtttg ccggctacgc gggaggccct ggaaaagatg tcttccagat 960

gatgcttaga ctgtttttga gttagtagca cggaatcaca gcactgtttc ccctcatttt 1020

agggtccgtc gtaacctgac tagt 1044

<210> 45
<211> 36
<212> DNA
<213> Artificial Sequence

<220>
<223> oligonucleotide

<400> 45
actagttaat taagaaactg acctaaagac ttgcct 36

<210> 46
<211> 35
<212> DNA
<213> Artificial Sequence

<220>
<223> oligonucleotide

<400> 46
actagtcagg ttacgacgga ccctaaaatg agggg 35

<210> 47
<211> 682
<212> DNA
<213> Artificial Sequence

<220>
<223> synthetic DNA

<400> 47
ccatggtacc gttcgtatag catacattat acgaagttat ttgagtaact atggaagact 60

atatcaaaat agagaaaatt ggagaaggta agtggtttta gcagaatgtt ttttgaaaag 120

aagataacaa tgctactgtg gaacacagaa acctttactg agtgcagtcg tagatgcatc 180

tgtacgcgtg cgcatgagtg tgctgtgaga gttggtgttt gtggcctcct agacaggatc 240

tgggatcact ctgagcacac ctgtagggga ttgtctttga ctctattaat tgacgtgggc 300

aggaacctcc cccagtgtgg gtgccttcct tgcctgagat cctggactgt gtgactggag 360

aagggtgtca agccacagtg aacctcactc tcccactgcc tgtgctcttg ctgctgctgc 420

tgctggactc taaccccgat cctgagtgca aacaaaccct tccttgggct acttttgtct 480

gggtgtttct cacagcacca gcaaaaggaa ggcacgcagc tgggcgtggt ggcacacacc 540

tttaatccca gcacttggga ggcagaggca ggcggatttc tgagttcgag gccagcctgg 600

tctacaaagt tccaggatag ccagggctat acagagaaac cctgtctcga aaaacagaaa 660

agaaaaaaa aagaaagcat gc 682

<210> 48
<211> 51
<212> DNA
<213> Artificial Sequence

<220>
<223> oligonucleotide

<400> 48
gcatgctttc tttttttttt cttttctgtt tttcgagaca gggtttctct g 51

<210> 49
<211> 72
<212> DNA
<213> Artificial Sequence

<220>
<223> oligonucleotide

<400> 49
ccatggtacc gttcgtatag catacattat acgaagttat ttgagtaact atggaagact 60

atatcaaaat ag 72

<210> 50
<211> 24
<212> DNA
<213> Artificial Sequence

<220>
<223> oligonucleotide

<400> 50
caccgacgac ggaccctaaa atga 24

<210> 51
<211> 24
<212> DNA
<213> Artificial Sequence

<220>
<223> oligonucleotide

<400> 51
aaactcattt tagggtccgt cgtc 24

<210> 52
<211> 24
<212> DNA
<213> Artificial Sequence

<220>
<223>  oligonucleotide

<400>  52
caccgatatc aaaatagaga aaat                                              24


<210>  53
<211>  24
<212>  DNA
<213>  Artificial Sequence

<220>
<223>  oligonucleotide

<400>  53
aaacatttc tctattttga tatc                                               24


<210>  54
<211>  23
<212>  DNA
<213>  Artificial Sequence

<220>
<223>  oligonucleotide

<400>  54
ccaggccaca tatgattagt tcc                                               23


<210>  55
<211>  23
<212>  DNA
<213>  Artificial Sequence

<220>
<223>  oligonucleotide

<400>  55
gtgagccaag aaattgtttc tcc                                               23


<210>  56
<211>  22
<212>  DNA
<213>  Artificial Sequence

<220>
<223>  oligonucleotide

<400>  56
ttgacctcca tagaagacac cg                                                22


<210>  57
<211>  19
<212>  DNA
<213>  Artificial Sequence

<220>
<223>  oligonucleotide

<400>  57
gctaagtccc ctgccttgg                                                    19


<210>  58
<211>  1978
<212>  DNA
<213>  Artificial Sequence

<220>
<223>  synthetic DNA

<400>  58
ggatccggtt ctggcgtgaa acagactttg aattttgacc ttctcaagtt ggcgggagac        60

gtggagtcca acccagggcc catggccagc tacccctgtc accagcacgc cagcgccttc       120

gaccaggccg ccagaagcag gggccacagc aaccggcgga ccgccctgag acccaggcgg        180

cagcaggaag ccaccgaagt ccggctggaa cagaagatgc ccaccctgtt aagggtgtac        240

atcgacggcc cccacggcat gggcaagacc accaccaccc agctgctggt ggccctgggc        300

agccgggacg acatcgtgta cgtgcccgag cccatgacct actggcaggt gctgggcgcc        360

agcgagacca tcgccaacat ctacaccaca cagcacaggc tggaccaggg cgagatctct        420

gccggcgacg ccgccgtggt gatgaccagc gcccagatca caatgggcat gccctacgcc        480

gtgaccgacg ccgtgctggc ccctcacgtg ggcggcgagg ccggctctag ccacgcccct        540

ccccctgccc tgaccctgat cttcgaccgg caccccatcg cccacctgct gtgctaccct        600

gccgccagat acctgatggg cagcatgacc ccccaggccg tgctggcctt cgtggccctg        660

atccccccca ccctgcccgg caccaacatc gtgctgggag ccctgcccga ggaccggcac        720

atcgaccggc tggccaagcg gcagagaccc ggcgagcggc tggacctggc catgctggcc        780

gccatccggc gggtgtacgg cctgctggcc aacaccgtga gatacctgca gggcggaggg        840

tcttggtggg aggactgggg ccagctgtcc ggcaccgccg tgccacctca gggcgccgag        900

ccccagagca atgccggccc tcggccccac atcggcgaca ccctgtttac cctgttcaga        960

gcccccgagc tgctggcccc caacggcgac ctgtacaacg tgttcgcctg ggccctggac       1020

gtgctggcca agaggctgcg gcccatgcac gtgttcatcc tggactacga ccagagccct       1080

gccggctgca gggacgccct gctgcagctg accagcggca tggtgcagac ccacgtgacc       1140

acccccggca gcatccccac catctgcgac ctggcccgga ccttcgcccg ggagatgggc       1200

gaggccaacg gctccggaga gggcagagga agtctgctaa catgcggtga cgtcgaggag       1260

aatcctggcc cactcgagat ggccacaacc atggtgagca agggcgagga ggataacatg       1320

gccatcatca aggagttcat gcgcttcaag gtgcacatgg agggctccgt gaacggccac       1380

gagttcgaga tcgagggcga gggcgagggc cgcccctacg agggcaccca gaccgccaag       1440

ctgaaggtga ccaagggtgg ccccctgccc ttcgcctggg acatcctgtc ccctcagttc       1500

atgtacggct ccaaggccta cgtgaagcac cccgccgaca tccccgacta cttgaagctg 1560

tccttccccg agggcttcaa gtgggagcgc gtgatgaact tcgaggacgg cggcgtggtg 1620

accgtgaccc aggactcctc cctgcaggac ggcgagttca tctacaaggt gaagctgcgc 1680

ggcaccaact tcccctccga cggccccgta atgcagaaga agaccatggg ctgggaggcc 1740

tcctccgagc ggatgtaccc cgaggacggc gccctgaagg gcgagatcaa gcagaggctg 1800

aagctgaagg acggcggcca ctacgacgct gaggtcaaga ccacctacaa ggccaagaag 1860

cccgtgcagc tgcccggcgc ctacaacgtc aacatcaagt tggacatcac ctcccacaac 1920

gaggactaca ccatcgtgga acagtacgaa cgcgccgagg gccgccactc caccggcg 1978

<210> 59
<211> 42
<212> DNA
<213> Artificial Sequence

<220>
<223> oligonucleotide

<400> 59
gctttctgac aaaaagtttc aatatgttat gtcaacagat ag 42

<210> 60
<211> 42
<212> DNA
<213> Artificial Sequence

<220>
<223> oligonucleotide

<400> 60
ctatctgttg acataacata ttgaaacttt ttgtcagaaa gc 42

<210> 61
<211> 758
<212> DNA
<213> Artificial Sequence

<220>
<223> synthetic DNA

<400> 61
ggagaatcct ggcccactcg aggtgagcaa gggcgaggag ctgttcaccg gggtggtgcc 60

catcctggtc gagctggacg gcgacgtaaa cggccacaag ttcagcgtgt ccggcgaggg 120

cgagggcgat gccacctacg gcaagctgac cctgaagttc atctgcacca ccggcaagct 180

gcccgtgccc tggcccaccc tcgtgaccac cctgacctac ggcgtgcagt gcttcagccg 240

ctaccccgac cacatgaagc agcacgactt cttcaagtcc gccatgcccg aaggctacgt 300

ccaggagcgc accatcttct tcaaggacga cggcaactac aagacccgcg ccgaggtgaa 360

gttcgagggc gacaccctgg tgaaccgcat cgagctgaag ggcatcgact tcaaggagga 420

cggcaacatc ctggggcaca agctggagta caactacaac agccacaacg tctatatcat 480

ggccgacaag cagaagaacg gcatcaaggt gaacttcaag atccgccaca acatcgagga 540

cggcagcgtg cagctcgccg accactacca gcagaacacc cccatcggcg acggccccgt 600

gctgctgccc gacaaccact acctgagcac ccagtccgcc ctgagcaaag accccaacga 660

gaagcgcgat cacatggtcc tgctggagtt cgtgaccgcc gccgggatca ctctcggcat 720

ggacgagctg tacaagtagc tttctgacaa aaagtttc 758

<210> 62
<211> 6080
<212> DNA
<213> Artificial Sequence

<220>
<223> synthetic DNA

<400> 62
tagctttctg acaaaaagtt tcaatatgtt atgtcaacag atagttgtgt ttttattgtt 60

aactcttgtc tatttttgtc ttatatatat ttctttgtta tcaaacttca gctgtacttc 120

gtcttctaat ttcaaaaata taacttaaaa atgtaaatat tctatatgaa tttaaatata 180

attctgtaaa tgtgtgtagg tctcactgta acaactattt gttactataa taaaactata 240

atattgatgt caggaatcag gaaaaaattt gagttggctt aaatcatctc agtccttatg 300

gcagttttat tttcctgtag ttggaactac taaaatttag gaaaatgcta agttcaagtt 360

tcgtaatgct ttgaagtatt tttatgctct gaatgtttaa atgttctcat cagtttcttg 420

ccatgttgtt aactatacaa cctggctaaa gatgaatatt tttctactgg tattttaatt 480

tttgacctaa atgtttaagc attcggaatg agaaaactat acagatttga gaaatgatgc 540

taaatttata ggagttttca gtaacttaaa aagctaacat gagagcatgc caaaatttgc 600

taagtcttac aaagatcaag ggctgtccgc aacagggaag aacagttttg aaaatttatg 660

aactatctta tttttaggta ggttttgaaa gctttttgtc taagtgaatt cttatgcctt 720

ggtcagagta ataactgaag gaggtgctta tcttggcttt cgagtctgag tttaaaacta 780

cacattttga catagtgttt attagcagcc atctaaaaag gctctaatgt atatttaact 840

aaaattacta gctttgggaa ttaaactgtt taacaaataa tgctgctcat tgtgattctt 900

acctataagc agcctaattt gaattatttg ctgcaatcga attcccgcgg ccgccatggc 960

ggccgggagc atgcgacgtc gggcccaatt cgccctatag tgagtcgtat tacaattcac 1020

tggccgtcgt tttacaacgt cgtgactggg aaaaccctgg cgttacccaa cttaatcgcc 1080

ttgcagcaca tccccctttc gccagctggc gtaatagcga agaggcccgc accgatcgcc 1140

cttcccaaca gttgcgcagc ctgaatggcg aatggacgcg ccctgtagcg gcgcattaag 1200

cgcggcgggt gtggtggtta cgcgcagcgt gaccgctaca cttgccagcg ccctagcgcc 1260

cgctcctttc gctttcttcc cttcctttct cgccacgttc gccggctttc cccgtcaagc 1320

tctaaatcgg gggctccctt tagggttccg atttagtgct ttacggcacc tcgaccccaa 1380

aaaacttgat tagggtgatg gttcacgtag tgggccatcg ccctgataga cggtttttcg 1440

ccctttgacg ttggagtcca cgttctttaa tagtggactc ttgttccaaa ctggaacaac 1500

actcaaccct atctcggtct attcttttga tttataaggg attttgccga tttcggccta 1560

ttggttaaaa aatgagctga tttaacaaaa atttaacgcg aattttaaca aaatattaac 1620

gcttacaatt tcctgatgcg gtattttctc cttacgcatc tgtgcggtat ttcacaccgc 1680

atcaggtggc acttttcggg gaaatgtgcg cggaacccct atttgtttat ttttctaaat 1740

acattcaaat atgtatccgc tcatgagaca ataaccctga taaatgcttc aataatattg 1800

aaaaaggaag agtatgagta ttcaacattt ccgtgtcgcc cttattccct tttttgcggc 1860

attttgcctt cctgtttttg ctcacccaga aacgctggtg aaagtaaaag atgctgaaga 1920

tcagttgggt gcacgagtgg gttacatcga actggatctc aacagcggta agatccttga 1980

gagttttcgc cccgaagaac gttttccaat gatgagcact tttaaagttc tgctatgtgg 2040

cgcggtatta tcccgtattg acgccgggca agagcaactc ggtcgccgca tacactattc 2100

tcagaatgac ttggttgagt actcaccagt cacagaaaag catcttacgg atggcatgac 2160

agtaagagaa ttatgcagtg ctgccataac catgagtgat aacactgcgg ccaacttact 2220

tctgacaacg atcggaggac cgaaggagct aaccgctttt ttgcacaaca tgggggatca 2280

tgtaactcgc cttgatcgtt gggaaccgga gctgaatgaa gccataccaa acgacgagcg 2340

tgacaccacg atgcctgtag caatggcaac aacgttgcgc aaactattaa ctggcgaact 2400

acttactcta gcttcccggc aacaattaat agactggatg gaggcggata aagttgcagg 2460

accacttctg cgctcggccc ttccggctgg ctggtttatt gctgataaat ctggagccgg 2520

tgagcgtggg tctcgcggta tcattgcagc actggggcca gatggtaagc cctcccgtat 2580

cgtagttatc tacacgacgg ggagtcaggc aactatggat gaacgaaata gacagatcgc 2640

tgagataggt gcctcactga ttaagcattg gtaactgtca gaccaagttt actcatatat 2700

actttagatt gatttaaaac ttcattttta atttaaaagg atctaggtga agatcctttt 2760

tgataatctc atgaccaaaa tcccttaacg tgagttttcg ttccactgag cgtcagaccc 2820

cgtagaaaag atcaaaggat cttcttgaga tccttttttt ctgcgcgtaa tctgctgctt 2880

gcaaacaaaa aaaccaccgc taccagcggt ggtttgtttg ccggatcaag agctaccaac 2940

tctttttccg aaggtaactg gcttcagcag agcgcagata ccaaatactg ttcttctagt 3000

gtagccgtag ttaggccacc acttcaagaa ctctgtagca ccgcctacat acctcgctct 3060

gctaatcctg ttaccagtgg ctgctgccag tggcgataag tcgtgtctta ccgggttgga 3120

ctcaagacga tagttaccgg ataaggcgca gcggtcgggc tgaacggggg gttcgtgcac 3180

acagcccagc ttggagcgaa cgacctacac cgaactgaga tacctacagc gtgagctatg 3240

agaaagcgcc acgcttcccg aagggagaaa ggcggacagg tatccggtaa gcggcagggt 3300

cggaacagga gagcgcacga gggagcttcc agggggaaac gcctggtatc tttatagtcc 3360

tgtcgggttt cgccacctct gacttgagcg tcgatttttg tgatgctcgt caggggggcg 3420

gagcctatgg aaaaacgcca gcaacgcggc ctttttacgg ttcctggcct tttgctggcc 3480

ttttgctcac atgttctttc ctgcgttatc ccctgattct gtggataacc gtattaccgc 3540

ctttgagtga gctgataccg ctcgccgcag ccgaacgacc gagcgcagcg agtcagtgag 3600

cgaggaagcg gaagagcgcc caatacgcaa accgcctctc cccgcgcgtt ggccgattca 3660

ttaatgcagc tggcacgaca ggtttcccga ctggaaagcg ggcagtgagc gcaacgcaat 3720

taatgtgagt tagctcactc attaggcacc ccaggcttta cactttatgc ttccggctcg 3780

tatgttgtgt ggaattgtga gcggataaca atttcacaca ggaaacagct atgaccatga 3840

ttacgccaag ctatttaggt gacactatag aatactcaag ctatgcatcc aacgcgttgg 3900

gagctctccc atatggtcga cctgcaggcg gccgcgaatt cactagtgat tgttacttcc 3960

cttcgagcct caatgtcctc atttgtaaaa tgacattaat acctactttt agctgtggga 4020

attgagtacc atgatttata caaagcagtt tgtatggtgc tggttacatg agagttcaga 4080

tggtaactag ttagtaaaaa atctctagtg tgcttgttga ttttatttta ttttagtatt 4140

tcttaaagat caaatttaac atcaatccta aactttattt agctttttct ggcgtgtaaa 4200

ctaacatact aagttgtgtg actataattc atttagtgac tcatttttag ctatttttat 4260

aacacattgt gctatggggg gttttggaac ttgctggaag ctacatcaga aactgccata 4320

gttaattgcc atttcaagaa tgttgtaaat aactcaggtg gccgtttaat tctcaatgta 4380

aatataatta actagacatc tttcctatat ttgtgtctca gttttaaagc tatttctgga 4440

tgcttgagtc ttaccgtaat tgataacaaa aagaggttat tgagaatatc tatgatttac 4500

agagtaagtt attctagacc tcaagagtga aatgtagggg aggagacatt tgtgtgttaa 4560

actaatggaa atgctcattt aatagatatt cactgaaagt attagttttg gtttattgct 4620

agaaaagttg aggttttatg gagatttttg taaaaaatgg tttatttcct aaataaatat 4680

ctctttttct tttttctccc agaaaatgtt aatctatgat ccagccaaac gaatttctgg 4740

caaaatggca ctgaatcatc catattttaa tgatttggac aatcagatta agaagatggg 4800

atccggttct ggcgtgaaac agactttgaa ttttgacctt ctcaagttgg cgggagacgt 4860

ggagtccaac ccaggcccca tggccagcta cccctgtcac cagcacgcca gcgccttcga 4920

ccaggccgcc agaagcaggg gccacagcaa ccggcggacc gccctgagac ccaggcggca 4980

gcaggaagcc accgaagtcc ggctggaaca gaagatgccc accctgttaa gggtgtacat 5040

cgacggcccc cacggcatgg gcaagaccac caccacccag ctgctggtgg ccctgggcag 5100

ccgggacgac atcgtgtacg tgcccgagcc catgacctac tggcaggtgc tgggcgccag 5160

cgagaccatc gccaacatct acaccacaca gcacaggctg gaccagggcg agatctctgc 5220

cggcgacgcc gccgtggtga tgaccagcgc ccagatcaca atgggcatgc cctacgccgt 5280

gaccgacgcc gtgctggccc ctcacgtggg cggcgaggcc ggctctagcc acgcccctcc 5340

ccctgccctg accctgatct tcgaccggca ccccatcgcc cacctgctgt gctaccctgc 5400

cgccagatac ctgatgggca gcatgacccc ccaggccgtg ctggccttcg tggccctgat 5460

cccccccacc ctgcccggca ccaacatcgt gctgggagcc ctgcccgagg accggcacat 5520

cgaccggctg gccaagcggc agagacccgg cgagcggctg gacctggcca tgctggccgc 5580

catccggcgg gtgtacggcc tgctggccaa caccgtgaga tacctgcagg gcggagggtc 5640

ttggtgggag gactggggcc agctgtccgg caccgccgtg ccacctcagg gcgccgagcc 5700

ccagagcaat gccggccctc ggccccacat cggcgacacc ctgtttaccc tgttcagagc 5760

ccccgagctg ctggccccca acggcgacct gtacaacgtg ttcgcctggg ccctggacgt 5820

gctggccaag aggctgcggc ccatgcacgt gttcatcctg gactacgacc agagccctgc 5880

cggctgcagg gacgccctgc tgcagctgac cagcggcatg gtgcagaccc acgtgaccac 5940

ccccggcagc atccccacca tctgcgacct ggcccggacc ttcgcccggg agatggcgga 6000

ggccaacggc tccggagagg gcagaggaag tctgctaaca tgcggtgacg tcgaggagaa 6060

tcctggccca ctcgaggtga 6080

<210> 63
<211> 58
<212> DNA
<213> Artificial Sequence

<220>
<223> oligonucleotide

<400> 63
taccgttcgt atagcataca ttatacgaag ttataccatg tctagactgg acaagagc 58

<210> 64
<211> 31
<212> DNA
<213> Artificial Sequence

<220>
<223> oligonucleotide

<400> 64
gcagagggaa aaagatctgc gactctagag g 31

<210>  65
<211>  23
<212>  DNA
<213>  Artificial Sequence

<220>
<223>  oligonucleotide

<400>  65
acgagtgtac acacacgagg tag                                              23


<210>  66
<211>  22
<212>  DNA
<213>  Artificial Sequence

<220>
<223>  oligonucleotide

<400>  66
tgtcaaccgg agtggagtaa cg                                               22


<210>  67
<211>  19
<212>  DNA
<213>  Artificial Sequence

<220>
<223>  oligonucleotide

<400>  67
atggagggga atacagccc                                                   19


<210>  68
<211>  20
<212>  DNA
<213>  Artificial Sequence

<220>
<223>  oligonucleotide

<400>  68
ttctttgcag ctccttcgtt                                                  20


<210>  69
<211>  982
<212>  DNA
<213>  Artificial Sequence

<220>
<223>  synthetic DNA

<400>  69
aagatacatc tctgcttctt tctggattga tttttctttg aagatgaatg tgagaaatag      60

aacttaaagg tctattctga gtgtgcttta taaatgattt tatcatcagt gatgggtgtt     120

agaaaaatct aggtaattat gcctggagtt ctgacgtttc attggcaaac ttcagaggaa     180

gtcttgaact tctggttgtc agcttttttt tttaacccat tcattgtgat tcaagtatac 240

cgtttttctg cttagtcctt gacattgttt aggttataat tagaatttta tctgtggaat 300

tgcacttttc attcttttgt tttacggtgc ttaagtgata tttcctactc ttggaaaaag 360

taccttgaaa gtcttgagca tgtttgtctg tgacctcagc aagcagagga aaaagattaa 420

aattgtcacg tgggcaactg taaacactta aaatgttttt gtcgttttaa acaaaatttt 480

cctaatgaac agtctgaaca cattttagtt ctgtcctcat aactttaaaa aataatgttt 540

tgaataaaat taggagtatc ataaagcggt ataaatttgt tgaaatattt gggtataact 600

ttggcacacg caggtactac tttaaagtgt cacaaaaagg gaacataatt ctggtttttg 660

tttggaaaga atagacaagt tgatacctga atttgagggt tattttgatt ggaagaagct 720

aaatactaca ctagtataga atatacacgt ttccaatgtc tcaggagtca agaaggatgg 780

aaagttattg taggagtcct tacccttgta aatattgttt gaatgtaatt aatatttatg 840

gaatacttat gctgcagtag gcactgtgga aaatgcttta catatagtgt ttcattaatg 900

cttaaaacta ctcgagttag tggcatgtaa ttaagtcagt ttttccttca ctttaggatc 960

taccataccc attgactaac tc 982

<210> 70
<211> 41
<212> DNA
<213> Artificial Sequence

<220>
<223> oligonucleotide

<400> 70
aaatcctgca ggaagataca tctctgcttc tttctggatt g 41

<210> 71
<211> 45
<212> DNA
<213> Artificial Sequence

<220>
<223> oligonucleotide

<400> 71
aaatcctgca ggagttagtc aatgggtatg gtagatccta aagtg 45

<210> 72
<211> 943
<212> DNA
<213> Artificial Sequence

<220>
<223> synthetic DNA

<400> 72
aagattatac caaaatagag aaaattggag aaggtgagtg gttttagtaa aataaatttt 60

atggaatgat ttaacaatgc tacaacttct gtaatatgaa cacatttaaa tatttattaa 120

aattcaacca ttaagttgtc ttgtagtacc agtgtgcatt aacatatgtt ctttacttaa 180

taaacgcaaa tttgctcaca tttcaagaga attgaaatta ccgaatccct aatgaccgtt 240

cgcctcaccc ccatagtctt acagtcttct gatactaaac acttatagta gtacttgtat 300

tttgaagaca ctattgataa cttgcatgtg tgttgagggg cagaggttag ggctgcagaa 360

ttatgctaac ttcataatat atttttagtt ttagtctctt tcaattgttt ttaggatgat 420

aaatattttt atccagactt tatagactgg ttacaataat tgacttaatc taatttaaat 480

aaaatttcta tttagttacc ctaagtataa atgacctgtc atatagacat gtttaagcta 540

ttctaaaatt ataacttatg tatataatcc ttccctttct gtggtacttg aaggaatgtt 600

tagatctata tatggcacaa atgaggggta tatcccttcg cctgttcctc gactccagca 660

gaacattctc gattctccag gtacaaaata atttgctaat agaatccagt ttatataggt 720

tctgccagaa gaaaattccc aactatattt ttaatgtgat caaagacccc acttgacaag 780

aaatagtgaa aaacaagggg gaatactgga gggaatttag tggaatagga gtgacaagtt 840

agtgtggtgt tcgattggag gtgtgtgtgt gtatacatat atatatatat ttttttttta 900

aatcacttaa atacctcata cccattctac taaacaaatt ttg 943

<210> 73
<211> 38
<212> DNA
<213> Artificial Sequence

<220>
<223> oligonucleotide

<400> 73
ttaaccatgg aagattatac caaaatagag aaaattgg 38

<210> 74
<211> 40
<212> DNA
<213> Artificial Sequence

<220>
<223> oligonucleotide

<400> 74
tttagcatgc caaaatttgt ttagtagaat gggtatgagg 40

<210> 75
<211> 8604
<212> DNA
<213> Artificial Sequence

<220>
<223> synthetic DNA

<400> 75
tatagtgagt cgtattacaa ttcactggcc gtcgttttac aacgtcgtga ctgggaaaac 60

cctggcgtta cccaacttaa tcgccttgca gcacatcccc ctttcgccag ctggcgtaat 120

agcgaagagg cccgcaccga tcgcccttcc caacagttgc gcagcctgaa tggcgaatgg 180

acgcgccctg tagcggcgca ttaagcgcgg cgggtgtggt ggttacgcgc agcgtgaccg 240

ctacacttgc cagcgcccta gcgcccgctc ctttcgcttt cttcccttcc tttctcgcca 300

cgttcgccgg ctttccccgt caagctctaa atcgggggct ccctttaggg ttccgattta 360

gtgctttacg gcacctcgac cccaaaaaac ttgattaggg tgatggttca cgtagtgggc 420

catcgccctg atagacggtt tttcgccctt tgacgttgga gtccacgttc tttaatagtg 480

gactcttgtt ccaaactgga acaacactca accctatctc ggtctattct tttgatttat 540

aagggatttt gccgatttcg gcctattggt taaaaaatga gctgatttaa caaaaattta 600

acgcgaattt taacaaaata ttaacgctta caatttcctg atgcggtatt ttctccttac 660

gcatctgtgc ggtatttcac accgcatcag gtggcacttt tcggggaaat gtgcgcggaa 720

cccctatttg tttatttttc taaatacatt caaatatgta tccgctcatg agacaataac 780

cctgataaat gcttcaataa tattgaaaaa ggaagagtat gagtattcaa catttccgtg 840

tcgcccttat tccctttttt gcggcatttt gccttcctgt ttttgctcac ccagaaacgc 900

tggtgaaagt aaaagatgct gaagatcagt tgggtgcacg agtgggttac atcgaactgg 960

atctcaacag cggtaagatc cttgagagtt ttcgccccga agaacgtttt ccaatgatga 1020

gcacttttaa agttctgcta tgtggcgcgg tattatcccg tattgacgcc gggcaagagc 1080

aactcggtcg ccgcatacac tattctcaga atgacttggt tgagtactca ccagtcacag 1140

aaaagcatct tacggatggc atgacagtaa gagaattatg cagtgctgcc ataaccatga 1200

gtgataacac tgcggccaac ttacttctga caacgatcgg aggaccgaag gagctaaccg 1260

cttttttgca caacatgggg gatcatgtaa ctcgccttga tcgttgggaa ccggagctga 1320

atgaagccat accaaacgac gagcgtgaca ccacgatgcc tgtagcaatg gcaacaacgt 1380

tgcgcaaact attaactggc gaactactta ctctagcttc ccggcaacaa ttaatagact 1440

ggatggaggc ggataaagtt gcaggaccac ttctgcgctc ggcccttccg gctggctggt 1500

ttattgctga taaatctgga gccggtgagc gtgggtctcg cggtatcatt gcagcactgg 1560

ggccagatgg taagccctcc cgtatcgtag ttatctacac gacggggagt caggcaacta 1620

tggatgaacg aaatagacag atcgctgaga taggtgcctc actgattaag cattggtaac 1680

tgtcagacca agtttactca tatatacttt agattgattt aaaacttcat ttttaattta 1740

aaaggatcta ggtgaagatc ctttttgata atctcatgac caaaatccct taacgtgagt 1800

tttcgttcca ctgagcgtca gaccccgtag aaaagatcaa aggatcttct tgagatcctt 1860

tttttctgcg cgtaatctgc tgcttgcaaa caaaaaaacc accgctacca gcggtggttt 1920

gtttgccgga tcaagagcta ccaactcttt ttccgaaggt aactggcttc agcagagcgc 1980

agataccaaa tactgttctt ctagtgtagc cgtagttagg ccaccacttc aagaactctg 2040

tagcaccgcc tacatacctc gctctgctaa tcctgttacc agtggctgct gccagtggcg 2100

ataagtcgtg tcttaccggg ttggactcaa gacgatagtt accggataag gcgcagcggt 2160

cgggctgaac ggggggttcg tgcacacagc ccagcttgga gcgaacgacc tacaccgaac 2220

tgagatacct acagcgtgag ctatgagaaa gcgccacgct tcccgaaggg agaaaggcgg 2280

acaggtatcc ggtaagcggc agggtcggaa caggagagcg cacgagggag cttccagggg 2340

gaaacgcctg gtatctttat agtcctgtcg ggtttcgcca cctctgactt gagcgtcgat 2400

ttttgtgatg ctcgtcaggg gggcggagcc tatggaaaaa cgccagcaac gcggcctttt 2460

tacggttcct ggccttttgc tggccttttg ctcacatgtt ctttcctgcg ttatcccctg 2520

attctgtgga taaccgtatt accgcctttg agtgagctga taccgctcgc cgcagccgaa 2580

cgaccgagcg cagcgagtca gtgagcgagg aagcggaaga gcgcccaata cgcaaaccgc 2640

ctctccccgc gcgttggccg attcattaat gcagctggca cgacaggttt cccgactgga 2700

aagcgggcag tgagcgcaac gcaattaatg tgagttagct cactcattag gcaccccagg 2760

ctttacactt tatgcttccg gctcgtatgt tgtgtggaat tgtgagcgga taacaatttc 2820

acacaggaaa cagctatgac catgattacg ccaagctatt taggtgacac tatagaatac 2880

tcaagctatg catccaacgc gttgggagct ctcccatatg gtcgacctgc aggaagatac 2940

atctctgctt ctttctggat tgatttttct ttgaagatga atgtgagaaa tagaacttaa 3000

aggtctattc tgagtgtgct ttataaatga ttttatcatc agtgatgggt gttagaaaaa 3060

tctaggtaat tatgcctgga gttctgacgt ttcattggca aacttcagag gaagtcttga 3120

acttctggtt gtcagctttt ttttttaacc cattcattgt gattcaagta taccgttttt 3180

ctgcttagtc cttgacattg tttaggttat aattagaatt ttatctgtgg aattgcactt 3240

ttcattcttt tgttttacgg tgcttaagtg atatttccta ctcttggaaa aagtaccttg 3300

aaagtcttga gcatgtttgt ctgtgacctc agcaagcaga ggaaaaagat taaaattgtc 3360

acgtgggcaa ctgtaaacac ttaaaatgtt tttgtcgttt taaacaaaat tttcctaatg 3420

aacagtctga acacatttta gttctgtcct cataacttta aaaaataatg ttttgaataa 3480

aattaggagt atcataaagc ggtataaatt tgttgaaata tttgggtata actttggcac 3540

acgcaggtac tactttaaag tgtcacaaaa agggaacata attctggttt ttgtttggaa 3600

agaatagaca agttgatacc tgaatttgag ggttattttg attggaagaa gctaaatact 3660

acactagtat agaatataca cgtttccaat gtctcaggag tcaagaagga tggaaagtta 3720

ttgtaggagt ccttaccctt gtaaatattg tttgaatgta attaatattt atggaatact 3780

tatgctgcag taggcactgt ggaaaatgct ttacatatag tgtttcatta atgcttaaaa 3840

ctactcgagt tagtggcatg taattaagtc agtttttcct tcactttagg atctaccata 3900

cccattgact aactcctgca ggcggccgcg aattcactag tgattaccgt tcgtatagca 3960

tacattatac gaagttatac catgtctaga ctggacaaga gcaaagtcat aaacggcgct 4020

ctggaattac tcaatggagt cggtatcgaa ggcctgacga caaggaaact cgctcaaaag 4080

ctgggagttg agcagcctac cctgtactgg cacgtgaaga acaagcgggc cctgctcgat 4140

gccctgccaa tcgagatgct ggacaggcat catacccact tctgccccct ggaaggcgag 4200

tcatggcaag actttctgcg gaacaacgcc aagtcattcc gctgtgctct cctctcacat 4260

cgcgacgggg ctaaagtgca tctcggcacc cgcccaacag agaaacagta cgaaaccctg 4320

gaaaatcagc tcgcgttcct gtgtcagcaa ggcttctccc tggagaacgc actgtacgct 4380

ctgtccgccg tgggccactt tacactgggc tgcgtattgg aggaacagga gcatcaagta 4440

gcaaaagagg aaagagagac acctaccacc gattctatgc ccccacttct gagacaagca 4500

attgagctgt tcgaccggca gggagccgaa cctgccttcc ttttcggcct ggaactaatc 4560

atatgtggcc tggagaaaca gctaaagtgc gaaagcggcg ggccggccga cgcccttgac 4620

gattttgact tagacatgct cccagccgat gcccttgacg actttgacct tgatatgctg 4680

cctgctgacg ctcttgacga ttttgacctt gacatgctcc ccgggtaact aagtaaggat 4740

cccaaatacc actgaattaa gaattccgcc cctctccctc ccccccccct aacgttactg 4800

gccgaagccg cttggaataa ggccggtgtg cgtttgtcta tatgttattt tccaccatat 4860

tgccgtcttt tggcaatgtg agggcccgga aacctggccc tgtcttcttg acgagcattc 4920

ctaggggtct ttcccctctc gccaaaggaa tgcaaggtct gttgaatgtc gtgaaggaag 4980

cagttcctct ggaagcttct tgaagacaaa caacgtctgt agcgaccctt tgcaggcagc 5040

ggaacccccc acctggcgac aggtgcctct gcggccaaaa gccacgtgta taagatacac 5100

ctgcaaaggc ggcacaaccc cagtgccacg ttgtgagttg gatagttgtg gaaagagtca 5160

aatggctctc ctcaagcgta ttcaacaagg ggctgaagga tgcccagaag gtaccccatt 5220

gtatgggatc tgatctgggg cctcggtgca catgctttac atgtgtttag tcgaggttaa 5280

aaaacgtcta ggccccccga accacgggga cgtggttttc ctttgaaaaa cacgatgata 5340

atatggccac aaccatgacc gagtacaagc ccacggtgcg cctcgccacc cgcgacgacg 5400

tcccccgggc cgtacgcacc ctcgccgccg cgttcgccga ctaccccgcc acgcgccaca 5460

ccgtcgaccc ggaccgccac atcgagcggg tcaccgagct gcaagaactc ttcctcacgc 5520

gcgtcgggct cgacatcggc aaggtgtggg tcgcggacga cggcgccgcg gtggcggtct 5580

ggaccacgcc ggagagcgtc gaagcggggg cggtgttcgc cgagatcggc ccgcgcatgg 5640

ccgagttgag cggttcccgg ctggccgcgc agcaacagat ggaaggcctc ctggcgccgc 5700

accggcccaa ggagcccgcg tggttcctgg ccaccgtcgg cgtctcgccc gaccaccagg 5760

gcaagggtct gggcagcgcc gtcgtgctcc ccggagtgga ggcggccgag cgcgccgggg 5820

tgcccgcctt cctggagacc tccgcgcccc gcaacctccc cttctacgag cggctcggct 5880

tcaccgtcac cgccgacgtc gaggtgcccg aaggaccgcg cacctggtgc atgacccgca 5940

agcccggtgc ctgaggatcc agacatgata agatacattg atgagtttgg acaaaccaca 6000

actagaatgc agtgaaaaaa atgctttatt tgtgaaattt gtgatgctat tgctttattt 6060

gtaaccatta taagctgcaa taaacaagtt aacaacaaca attgcattca ttttatgttt 6120

caggttcagg gggaggtgtg ggaggttttt taaagcaagt aaaacctcta caaatgtggt 6180

atggctgatt atgatcctct agagtcgcag atccagacat gataagatac attgatgagt 6240

ttggacaaac cacaactaga atgcagtgaa aaaaatgctt tatttgtgaa atttgtgatg 6300

ctattgcttt atttgtaacc attataagct gcaataaaca agttaacaac aacaattgca 6360

ttcattttat gtttcaggtt cagggggagg tgtgggaggt tttttaaagc aagtaaaacc 6420

tctacaaatg tggtatggct gattatgatc ctctagagtc gcagatccag acatgataag 6480

atacattgat gagtttggac aaaccacaac tagaatgcag tgaaaaaaat gctttatttg 6540

tgaaatttgt gatgctattg ctttatttgt aaccattata agctgcaata aacaagttaa 6600

caacaacaat tgcattcatt ttatgtttca ggttcagggg gaggtgtggg aggtttttta 6660

aagcaagtaa aacctctaca aatgtggtat ggctgattat gatcctctag agtcgcagat 6720

cctctagagt cgcagatctt tttccctctg ccaaaaatta tggggacatc atgaagcccc 6780

ttgagcatct gacttctggc taataaagga aatttatttt cattgcaata gtgtgttgga 6840

attttttgtg tctctcactc ggaaggacat atgggagggc aaatcattta aaacatcaga 6900

atgagtattt ggtttagagt ttggcaacat atgccatatg ctggctgcca tgaacaaagg 6960

tggctataaa gaggtcatca gtatatgaaa cagccccctg ctgtccattc cttattccat 7020

agaaaagcct tgacttgagg ttagattttt tttatatttt gttttgtgtt atttttttct 7080

ttaacatccc taaaattttc cttacatgtt ttactagcca gatttttcct cctctcctga 7140

ctactcccag tcatagctgt ccctcttctc ttatgaagat ccctcgacct gcagcccaag 7200

cttatcgaat tcccgcggtg gcggccgcac gtctccctat cagtgataga gaagtcgaca 7260

cgtctcgagc tccctatcag tgatagagaa ggtacgtcta gaacgtctcc ctatcagtga 7320

tagagaagtc gacacgtctc gagctcccta tcagtgatag agaaggtacg tctagaacgt 7380

ctccctatca gtgatagaga agtcgacacg tctcgagctc cctatcagtg atagagaagg 7440

tacgtctaga acgtctccct atcagtgata gagaagtcga cacgtctcga gctccctatc 7500

agtgatagag aaggtacccc ctatataagc agagctcgtt tagtgaaccg tcagatcgcc 7560

tggagacgcc atccacgctg ttttgacctc catagaagac accgggaccg atccagcctc 7620

cgcggccgcc atggaagatt ataccaaaat agagaaaatt ggagaaggtg agtggtttta 7680

gtaaaataaa ttttatggaa tgatttaaca atgctacaac ttctgtaata tgaacacatt 7740

taaatattta ttaaaattca accattaagt tgtcttgtag taccagtgtg cattaacata 7800

tgttctttac ttaataaacg caaatttgct cacatttcaa gagaattgaa attaccgaat 7860

ccctaatgac cgttcgcctc acccccatag tcttacagtc ttctgatact aaacacttat 7920

agtagtactt gtattttgaa gacactattg ataacttgca tgtgtgttga ggggcagagg 7980

ttagggctgc agaattatgc taacttcata atatattttt agttttagtc tctttcaatt 8040

gtttttagga tgataaatat ttttatccag actttataga ctggttacaa taattgactt 8100

aatctaattt aaataaaatt tctatttagt taccctaagt ataaatgacc tgtcatatag 8160

acatgtttaa gctattctaa aattataact tatgtatata atccttccct ttctgtggta 8220

cttgaaggaa tgtttagatc tatatatggc acaaatgagg ggtatatccc ttcgcctgtt 8280

cctcgactcc agcagaacat tctcgattct ccaggtacaa aataatttgc taatagaatc 8340

cagtttatat aggttctgcc agaagaaaat tcccaactat atttttaatg tgatcaaaga 8400

ccccacttga caagaaatag tgaaaaacaa gggggaatac tggagggaat ttagtggaat 8460

aggagtgaca agttagtgtg gtgttcgatt ggaggtgtgt gtgtgtatac atatatatat 8520

atattttttt tttaaatcac ttaaatacct catacccatt ctactaaaca aattttggca 8580

tgcgacgtcg ggcccaattc gccc 8604

<210> 76
<211> 24
<212> DNA
<213> Artificial Sequence

<220>
<223> oligonucleotide

<400> 76
caccgatacc aaaatagaga aaat 24

<210> 77
<211> 23
<212> DNA
<213> Artificial Sequence

<220>
<223> oligonucleotide

<400> 77
   aaacattttc tctattttgg tac 23

<210>  78
<211>  24
<212>  DNA
<213>  Artificial Sequence

<220>
<223>  oligonucleotide

<400>  78
caccgcatag ttagtcaatg ggta                                              24


<210>  79
<211>  24
<212>  DNA
<213>  Artificial Sequence

<220>
<223>  oligonucleotide

<400>  79
aaactaccca ttgactaact atgc                                              24


<210>  80
<211>  8436
<212>  DNA
<213>  Artificial Sequence

<220>
<223>  synthetic DNA

<400>  80
gagggcctat ttcccatgat tccttcatat ttgcatatac gatacaaggc tgttagagag      60

ataattggaa ttaatttgac tgtaaacaca aagatattag tacaaaatac gtgacgtaga     120

aagtaataat ttcttgggta gtttgcagtt ttaaaattat gttttaaaat ggactatcat     180

atgcttaccg taacttgaaa gtatttcgat ttcttggctt tatatatctt gtggaaagga     240

cgaaacaccg ataccaaaat agagaaaatg ttttagagct agaaatagca agttaaaata     300

aggctagtcc gttatcaact tgaaaaagtg gcaccgagtc ggtgcttttt tgttttagag     360

ctagaaatag caagttaaaa taaggctagt ccgtttttag cgcgtgcgcc aattctgcag     420

acaaatggct ctagaggtac ccgttacata acttacggta aatggcccgc ctggctgacc     480

gcccaacgac ccccgcccat tgacgtcaat agtaacgcca atagggactt tccattgacg     540

tcaatgggtg gagtatttac ggtaaactgc ccacttggca gtacatcaag tgtatcatat     600

gccaagtacg ccccctattg acgtcaatga cggtaaatgg cccgcctggc attgtgccca     660

gtacatgacc ttatgggact ttcctacttg gcagtacatc tacgtattag tcatcgctat     720

taccatggtc gaggtgagcc ccacgttctg cttcactctc cccatctccc ccccctcccc     780

acccccaatt ttgtatttat ttatttttta attattttgt gcagcgatgg gggcgggggg     840

gggggggggg cgcgcgccag gcggggcggg gcggggcgag gggcggggcg gggcgaggcg     900

gagaggtgcg gcggcagcca atcagagcgg cgcgctccga aagtttcctt ttatggcgag 960
gcggcggcgg cggcggccct ataaaaagcg aagcgcgcgg cgggcgggag tcgctgcgac 1020
gctgccttcg ccccgtgccc cgctccgccg ccgcctcgcg ccgcccgccc cggctctgac 1080
tgaccgcgtt actcccacag gtgagcgggc gggacggccc ttctcctccg ggctgtaatt 1140
agctgagcaa gaggtaaggg tttaagggat ggttggttgg tggggtatta atgtttaatt 1200
acctggagca cctgcctgaa atcacttttt ttcaggttgg accggtgcca ccatgtaccc 1260
atacgatgtt ccagattacg cttcgccgaa gaaaaagcgc aaggtcgaag cgtccgacaa 1320
gaagtacagc atcggcctgg ccatcggcac caactctgtg ggctgggccg tgatcaccga 1380
cgagtacaag gtgcccagca agaaattcaa ggtgctgggc aacaccgacc ggcacagcat 1440
caagaagaac ctgatcggag ccctgctgtt cgacagcggc gaaacagccg aggccacccg 1500
gctgaagaga accgccagaa gaagatacac cagacggaag aaccggatct gctatctgca 1560
agagatcttc agcaacgaga tggccaaggt ggacgacagc ttcttccaca gactggaaga 1620
gtccttcctg gtggaagagg ataagaagca cgagcggcac cccatcttcg gcaacatcgt 1680
ggacgaggtg gcctaccacg agaagtaccc caccatctac cacctgagaa agaaactggt 1740
ggacagcacc gacaaggccg acctgcggct gatctatctg gccctggccc acatgatcaa 1800
gttccggggc cacttcctga tcgagggcga cctgaacccc gacaacagcg acgtggacaa 1860
gctgttcatc cagctggtgc agacctacaa ccagctgttc gaggaaaacc ccatcaacgc 1920
cagcggcgtg gacgccaagg ccatcctgtc tgccagactg agcaagagca gacggctgga 1980
aaatctgatc gcccagctgc ccggcgagaa gaagaatggc ctgttcggca acctgattgc 2040
cctgagcctg ggcctgaccc ccaacttcaa gagcaacttc gacctggccg aggatgccaa 2100
actgcagctg agcaaggaca cctacgacga cgacctggac aacctgctgg cccagatcgg 2160
cgaccagtac gccgacctgt ttctggccgc caagaacctg tccgacgcca tcctgctgag 2220
cgacatcctg agagtgaaca ccgagatcac caaggccccc ctgagcgcct ctatgatcaa 2280
gagatacgac gagcaccacc aggacctgac cctgctgaaa gctctcgtgc ggcagcagct 2340
gcctgagaag tacaaagaga ttttcttcga ccagagcaag aacggctacg ccggctacat 2400
tgacggcgga gccagccagg aagagttcta caagttcatc aagcccatcc tggaaaagat 2460
ggacggcacc gaggaactgc tcgtgaagct gaacagagag gacctgctgc ggaagcagcg 2520
gaccttcgac aacggcagca tcccccacca gatccacctg ggagagctgc acgccattct 2580
gcggcggcag gaagattttt acccattcct gaaggacaac cgggaaaaga tcgagaagat 2640
cctgaccttc cgcatcccct actacgtggg ccctctggcc aggggaaaca gcagattcgc 2700
ctggatgacc agaaagagcg aggaaaccat caccccctgg aacttcgagg aagtggtgga 2760
caagggcgct tccgcccaga gcttcatcga gcggatgacc aacttcgata agaacctgcc 2820

caacgagaag gtgctgccca agcacagcct gctgtacgag tacttcaccg tgtataacga 2880

gctgaccaaa gtgaaatacg tgaccgaggg aatgagaaag cccgccttcc tgagcggcga 2940

gcagaaaaag gccatcgtgg acctgctgtt caagaccaac cggaaagtga ccgtgaagca 3000

gctgaaagag gactacttca agaaaatcga gtgcttcgac tccgtggaaa tctccggcgt 3060

ggaagatcgg ttcaacgcct ccctgggcac ataccacgat ctgctgaaaa ttatcaagga 3120

caaggacttc ctggacaatg aggaaaacga ggacattctg gaagatatcg tgctgaccct 3180

gacactgttt gaggacagag agatgatcga ggaacggctg aaaacctatg cccacctgtt 3240

cgacgacaaa gtgatgaagc agctgaagcg gcggagatac accggctggg gcaggctgag 3300

ccggaagctg atcaacggca tccgggacaa gcagtccggc aagacaatcc tggatttcct 3360

gaagtccgac ggcttcgcca acagaaactt catgcagctg atccacgacg acagcctgac 3420

ctttaaagag gacatccaga aagcccaggt gtccggccag ggcgatagcc tgcacgagca 3480

cattgccaat ctggccggca gccccgccat taagaagggc atcctgcaga cagtgaaggt 3540

ggtggacgag ctcgtgaaag tgatgggccg gcacaagccc gagaacatcg tgatcgaaat 3600

ggccagagag aaccagacca cccagaaggg acagaagaac agccgcgaga gaatgaagcg 3660

gatcgaagag ggcatcaaag agctgggcag ccagatcctg aaagaacacc ccgtggaaaa 3720

cacccagctg cagaacgaga agctgtacct gtactacctg cagaatgggc gggatatgta 3780

cgtggaccag gaactggaca tcaaccggct gtccgactac gatgtggacc atatcgtgcc 3840

tcagagcttt ctgaaggacg actccatcga caacaaggtg ctgaccagaa gcgacaagaa 3900

ccggggcaag agcgacaacg tgccctccga agaggtcgtg aagaagatga agaactactg 3960

gcggcagctg ctgaacgcca agctgattac ccagagaaag ttcgacaatc tgaccaaggc 4020

cgagagaggc ggcctgagcg aactggataa ggccggcttc atcaagagac agctggtgga 4080

aacccggcag atcacaaagc acgtggcaca gatcctggac tcccggatga acactaagta 4140

cgacgagaat gacaagctga tccgggaagt gaaagtgatc accctgaagt ccaagctggt 4200

gtccgatttc cggaaggatt tccagtttta caaagtgcgc gagatcaaca actaccacca 4260

cgcccacgac gcctacctga acgccgtcgt gggaaccgcc ctgatcaaaa agtaccctaa 4320

gctggaaagc gagttcgtgt acggcgacta caaggtgtac gacgtgcgga agatgatcgc 4380

caagagcgag caggaaatcg gcaaggctac cgccaagtac ttcttctaca gcaacatcat 4440

gaactttttc aagaccgaga ttaccctggc caacggcgag atccggaagc ggcctctgat 4500

cgagacaaac ggcgaaaccg gggagatcgt gtgggataag ggccgggatt ttgccaccgt 4560

gcggaaagtg ctgagcatgc cccaagtgaa tatcgtgaaa aagaccgagg tgcagacagg 4620

cggcttcagc aaagagtcta tcctgcccaa gaggaacagc gataagctga tcgccagaaa 4680

gaaggactgg gaccctaaga agtacggcgg cttcgacagc cccaccgtgg cctattctgt 4740

gctggtggtg gccaaagtgg aaaagggcaa gtccaagaaa ctgaagagtg tgaaagagct 4800

gctggggatc accatcatgg aaagaagcag cttcgagaag aatcccatcg actttctgga 4860

agccaagggc tacaaagaag tgaaaaagga cctgatcatc aagctgccta agtactccct 4920

gttcgagctg gaaaacggcc ggaagagaat gctggcctct gccggcgaac tgcagaaggg 4980

aaacgaactg gccctgccct ccaaatatgt gaacttcctg tacctggcca gccactatga 5040

gaagctgaag ggctcccccg aggataatga gcagaaacag ctgtttgtgg aacagcacaa 5100

gcactacctg gacgagatca tcgagcagat cagcgagttc tccaagagag tgatcctggc 5160

cgacgctaat ctggacaaag tgctgtccgc ctacaacaag caccgggata agcccatcag 5220

agagcaggcc gagaatatca tccacctgtt taccctgacc aatctgggag cccctgccgc 5280

cttcaagtac tttgacacca ccatcgaccg gaagaggtac accagcacca aagaggtgct 5340

ggacgccacc ctgatccacc agagcatcac cggcctgtac gagacacgga tcgacctgtc 5400

tcagctggga ggcgacagcc ccaagaagaa gagaaaggtg gaggccagct aagaattcct 5460

agagctcgct gatcagcctc gactgtgcct tctagttgcc agccatctgt tgtttgcccc 5520

tcccccgtgc cttccttgac cctggaaggt gccactccca ctgtcctttc ctaataaaat 5580

gaggaaattg catcgcattg tctgagtagg tgtcattcta ttctgggggg tggggtgggg 5640

caggacagca agggggagga ttgggaagag aatagcaggc atgctgggga gcggccgcag 5700

gaacccctag tgatggagtt ggccactccc tctctgcgcg ctcgctcgct cactgaggcc 5760

gggcgaccaa aggtcgcccg acgcccgggc tttgcccggg cggcctcagt gagcgagcga 5820

gcgcgcagct gcctgcaggg gcgcctgatg cggtattttc tccttacgca tctgtgcggt 5880

atttcacacc gcatacgtca aagcaaccat agtacgcgcc ctgtagcggc gcattaagcg 5940

cggcgggtgt ggtggttacg cgcagcgtga ccgctacact tgccagcgcc ctagcgcccg 6000

ctcctttcgc tttcttccct tcctttctcg ccacgttcgc cggctttccc cgtcaagctc 6060

taaatcgggg gctcccttta gggttccgat ttagtgcttt acggcacctc gaccccaaaa 6120

aacttgattt gggtgatggt tcacgtagtg ggccatcgcc ctgatagacg gtttttcgcc 6180

ctttgacgtt ggagtccacg ttctttaata gtggactctt gttccaaact ggaacaacac 6240

tcaaccctat ctcgggctat tcttttgatt tataagggat tttgccgatt tcggcctatt 6300

ggttaaaaaa tgagctgatt taacaaaaat ttaacgcgaa ttttaacaaa atattaacgt 6360

ttacaatttt atggtgcact ctcagtacaa tctgctctga tgccgcatag ttaagccagc 6420

cccgacaccc gccaacaccc gctgacgcgc cctgacgggc ttgtctgctc ccggcatccg 6480

cttacagaca agctgtgacc gtctccggga gctgcatgtg tcagaggttt tcaccgtcat 6540

caccgaaacg cgcgagacga aagggcctcg tgatacgcct atttttatag gttaatgtca 6600

tgataataat ggtttcttag acgtcaggtg gcacttttcg gggaaatgtg cgcggaaccc 6660

ctatttgttt atttttctaa atacattcaa atatgtatcc gctcatgaga caataaccct 6720

gataaatgct tcaataatat tgaaaaagga agagtatgag tattcaacat ttccgtgtcg 6780

cccttattcc cttttttgcg gcattttgcc ttcctgtttt tgctcaccca gaaacgctgg 6840

tgaaagtaaa agatgctgaa gatcagttgg gtgcacgagt gggttacatc gaactggatc 6900

tcaacagcgg taagatcctt gagagttttc gccccgaaga acgttttcca atgatgagca 6960

cttttaaagt tctgctatgt ggcgcggtat tatcccgtat tgacgccggg caagagcaac 7020

tcggtcgccg catacactat tctcagaatg acttggttga gtactcacca gtcacagaaa 7080

agcatcttac ggatggcatg acagtaagag aattatgcag tgctgccata accatgagtg 7140

ataacactgc ggccaactta cttctgacaa cgatcggagg accgaaggag ctaaccgctt 7200

ttttgcacaa catgggggat catgtaactc gccttgatcg ttgggaaccg gagctgaatg 7260

aagccatacc aaacgacgag cgtgacacca cgatgcctgt agcaatggca acaacgttgc 7320

gcaaactatt aactggcgaa ctacttactc tagcttcccg gcaacaatta atagactgga 7380

tggaggcgga taaagttgca ggaccacttc tgcgctcggc ccttccggct ggctggttta 7440

ttgctgataa atctggagcc ggtgagcgtg ggtctcgcgg tatcattgca gcactggggc 7500

cagatggtaa gccctcccgt atcgtagtta tctacacgac ggggagtcag gcaactatgg 7560

atgaacgaaa tagacagatc gctgagatag gtgcctcact gattaagcat tggtaactgt 7620

cagaccaagt ttactcatat atactttaga ttgatttaaa acttcatttt taatttaaaa 7680

ggatctaggt gaagatcctt tttgataatc tcatgaccaa aatcccttaa cgtgagtttt 7740

cgttccactg agcgtcagac cccgtagaaa agatcaaagg atcttcttga gatccttttt 7800

ttctgcgcgt aatctgctgc ttgcaaacaa aaaaaccacc gctaccagcg gtggtttgtt 7860

tgccggatca agagctacca actctttttc cgaaggtaac tggcttcagc agagcgcaga 7920

taccaaatac tgtccttcta gtgtagccgt agttaggcca ccacttcaag aactctgtag 7980

caccgcctac atacctcgct ctgctaatcc tgttaccagt ggctgctgcc agtggcgata 8040

agtcgtgtct taccgggttg gactcaagac gatagttacc ggataaggcg cagcggtcgg 8100

gctgaacggg gggttcgtgc acacagccca gcttggagcg aacgacctac accgaactga 8160

gatacctaca gcgtgagcta tgagaaagcg ccacgcttcc cgaagggaga aaggcggaca 8220

ggtatccggt aagcggcagg gtcggaacag gagagcgcac gagggagctt ccagggggaa 8280

acgcctggta tctttatagt cctgtcgggt ttcgccacct ctgacttgag cgtcgatttt 8340

tgtgatgctc gtcagggggg cggagcctat ggaaaaacgc cagcaacgcg gcctttttac 8400

ggttcctggc cttttgctgg ccttttgctc acatgt 8436

<210>  81
<211>  8440
<212>  DNA
<213>  Artificial Sequence

<220>
<223>  synthetic DNA

<400>  81
gagggcctat ttcccatgat tccttcatat ttgcatatac gatacaaggc tgttagagag      60

ataattggaa ttaatttgac tgtaaacaca aagatattag tacaaaatac gtgacgtaga     120

aagtaataat ttcttgggta gtttgcagtt ttaaaattat gttttaaaat ggactatcat     180

atgcttaccg taacttgaaa gtatttcgat ttcttggctt tatatatctt gtggaaagga     240

cgaaacaccc accgcatagt tagtcaatgg gtagttttag agctagaaat agcaagttaa     300

aataaggcta gtccgttatc aacttgaaaa agtggcaccg agtcggtgct tttttgtttt     360

agagctagaa atagcaagtt aaaataaggc tagtccgttt ttagcgcgtg cgccaattct     420

gcagacaaat ggctctagag gtacccgtta cataacttac ggtaaatggc ccgcctggct     480

gaccgcccaa cgacccccgc ccattgacgt caatagtaac gccaataggg actttccatt     540

gacgtcaatg ggtggagtat ttacggtaaa ctgcccactt ggcagtacat caagtgtatc     600

atatgccaag tacgccccct attgacgtca atgacggtaa atggcccgcc tggcattgtg     660

cccagtacat gaccttatgg gactttccta cttggcagta catctacgta ttagtcatcg     720

ctattaccat ggtcgaggtg agccccacgt tctgcttcac tctccccatc tcccccccct     780

ccccaccccc aattttgtat ttatttattt tttaattatt ttgtgcagcg atgggggcgg     840

gggggggggg ggggcgcgcg ccaggcgggg cggggcgggg cgaggggcgg ggcggggcga     900

ggcggagagg tgcggcggca gccaatcaga gcggcgcgct ccgaaagttt ccttttatgg     960

cgaggcggcg gcggcggcgg ccctataaaa agcgaagcgc gcggcgggcg ggagtcgctg    1020

cgacgctgcc ttcgccccgt gccccgctcc gccgccgcct cgcgccgccc gccccggctc    1080

tgactgaccg cgttactccc acaggtgagc gggcgggacg gcccttctcc tccgggctgt    1140

aattagctga gcaagaggta agggtttaag ggatggttgg ttggtggggt attaatgttt    1200

aattacctgg agcacctgcc tgaaatcact ttttttcagg ttggaccggt gccaccatgt    1260

acccatacga tgttccagat tacgcttcgc cgaagaaaaa gcgcaaggtc gaagcgtccg    1320

acaagaagta cagcatcggc ctggccatcg gcaccaactc tgtgggctgg gccgtgatca    1380

ccgacgagta caaggtgccc agcaagaaat tcaaggtgct gggcaacacc gaccggcaca    1440

gcatcaagaa gaacctgatc ggagccctgc tgttcgacag cggcgaaaca gccgaggcca    1500

cccggctgaa gagaaccgcc agaagaagat acaccagacg gaagaaccgg atctgctatc    1560

tgcaagagat cttcagcaac gagatggcca aggtggacga cagcttcttc cacagactgg    1620

aagagtcctt cctggtggaa gaggataaga agcacgagcg gcaccccatc ttcggcaaca 1680

tcgtggacga ggtggcctac cacgagaagt accccaccat ctaccacctg agaaagaaac 1740

tggtggacag caccgacaag gccgacctgc ggctgatcta tctggccctg gcccacatga 1800

tcaagttccg gggccacttc ctgatcgagg gcgacctgaa ccccgacaac agcgacgtgg 1860

acaagctgtt catccagctg gtgcagacct acaaccagct gttcgaggaa aaccccatca 1920

acgccagcgg cgtggacgcc aaggccatcc tgtctgccag actgagcaag agcagacggc 1980

tggaaaatct gatcgcccag ctgcccggcg agaagaagaa tggcctgttc ggcaacctga 2040

ttgccctgag cctgggcctg acccccaact tcaagagcaa cttcgacctg gccgaggatg 2100

ccaaactgca gctgagcaag gacacctacg acgacgacct ggacaacctg ctggcccaga 2160

tcggcgacca gtacgccgac ctgtttctgg ccgccaagaa cctgtccgac gccatcctgc 2220

tgagcgacat cctgagagtg aacaccgaga tcaccaaggc ccccctgagc gcctctatga 2280

tcaagagata cgacgagcac caccaggacc tgaccctgct gaaagctctc gtgcggcagc 2340

agctgcctga gaagtacaaa gagattttct tcgaccagag caagaacggc tacgccggct 2400

acattgacgg cggagccagc caggaagagt tctacaagtt catcaagccc atcctggaaa 2460

agatggacgg caccgaggaa ctgctcgtga agctgaacag agaggacctg ctgcggaagc 2520

agcggacctt cgacaacggc agcatccccc accagatcca cctgggagag ctgcacgcca 2580

ttctgcggcg gcaggaagat ttttacccat tcctgaagga caaccgggaa aagatcgaga 2640

agatcctgac cttccgcatc ccctactacg tgggccctct ggccagggga aacagcagat 2700

tcgcctggat gaccagaaag agcgaggaaa ccatcacccc ctggaacttc gaggaagtgg 2760

tggacaaggg cgcttccgcc cagagcttca tcgagcggat gaccaacttc gataagaacc 2820

tgcccaacga gaaggtgctg cccaagcaca gcctgctgta cgagtacttc accgtgtata 2880

acgagctgac caaagtgaaa tacgtgaccg agggaatgag aaagcccgcc ttcctgagcg 2940

gcgagcagaa aaaggccatc gtggacctgc tgttcaagac caaccggaaa gtgaccgtga 3000

agcagctgaa agaggactac ttcaagaaaa tcgagtgctt cgactccgtg gaaatctccg 3060

gcgtggaaga tcggttcaac gcctccctgg gcacatacca cgatctgctg aaaattatca 3120

aggacaagga cttcctggac aatgaggaaa acgaggacat tctggaagat atcgtgctga 3180

ccctgacact gtttgaggac agagagatga tcgaggaacg gctgaaaacc tatgcccacc 3240

tgttcgacga caaagtgatg aagcagctga agcggcggag atacaccggc tggggcaggc 3300

tgagccggaa gctgatcaac ggcatccggg acaagcagtc cggcaagaca atcctggatt 3360

tcctgaagtc cgacggcttc gccaacagaa acttcatgca gctgatccac gacgacagcc 3420

tgacctttaa agaggacatc cagaaagccc aggtgtccgg ccagggcgat agcctgcacg 3480

agcacattgc caatctggcc ggcagccccg ccattaagaa gggcatcctg cagacagtga 3540

aggtggtgga cgagctcgtg aaagtgatgg gccggcacaa gcccgagaac atcgtgatcg 3600

aaatggccag agagaaccag accacccaga agggacagaa gaacagccgc gagagaatga 3660

agcggatcga agagggcatc aaagagctgg gcagccagat cctgaaagaa caccccgtgg 3720

aaaacaccca gctgcagaac gagaagctgt acctgtacta cctgcagaat gggcgggata 3780

tgtacgtgga ccaggaactg gacatcaacc ggctgtccga ctacgatgtg gaccatatcg 3840

tgcctcagag ctttctgaag gacgactcca tcgacaacaa ggtgctgacc agaagcgaca 3900

agaaccgggg caagagcgac aacgtgccct ccgaagaggt cgtgaagaag atgaagaact 3960

actggcggca gctgctgaac gccaagctga ttacccagag aaagttcgac aatctgacca 4020

aggccgagag aggcggcctg agcgaactgg ataaggccgg cttcatcaag agacagctgg 4080

tggaaacccg gcagatcaca aagcacgtgg cacagatcct ggactcccgg atgaacacta 4140

agtacgacga gaatgacaag ctgatccggg aagtgaaagt gatcaccctg aagtccaagc 4200

tggtgtccga tttccggaag gatttccagt tttacaaagt gcgcgagatc aacaactacc 4260

accacgccca cgacgcctac ctgaacgccg tcgtgggaac cgccctgatc aaaaagtacc 4320

ctaagctgga aagcgagttc gtgtacggcg actacaaggt gtacgacgtg cggaagatga 4380

tcgccaagag cgagcaggaa atcggcaagg ctaccgccaa gtacttcttc tacagcaaca 4440

tcatgaactt tttcaagacc gagattaccc tggccaacgg cgagatccgg aagcggcctc 4500

tgatcgagac aaacggcgaa accggggaga tcgtgtggga taagggccgg gattttgcca 4560

ccgtgcggaa agtgctgagc atgccccaag tgaatatcgt gaaaaagacc gaggtgcaga 4620

caggcggctt cagcaaagag tctatcctgc ccaagaggaa cagcgataag ctgatcgcca 4680

gaaagaagga ctgggaccct aagaagtacg gcggcttcga cagccccacc gtggcctatt 4740

ctgtgctggt ggtggccaaa gtggaaaagg gcaagtccaa gaaactgaag agtgtgaaag 4800

agctgctggg gatcaccatc atggaaagaa gcagcttcga gaagaatccc atcgactttc 4860

tggaagccaa gggctacaaa gaagtgaaaa aggacctgat catcaagctg cctaagtact 4920

ccctgttcga gctggaaaac ggccggaaga gaatgctggc ctctgccggc gaactgcaga 4980

agggaaacga actggccctg ccctccaaat atgtgaactt cctgtacctg gccagccact 5040

atgagaagct gaagggctcc cccgaggata atgagcagaa acagctgttt gtggaacagc 5100

acaagcacta cctggacgag atcatcgagc agatcagcga gttctccaag agagtgatcc 5160

tggccgacgc taatctggac aaagtgctgt ccgcctacaa caagcaccgg gataagccca 5220

tcagagagca ggccgagaat atcatccacc tgtttaccct gaccaatctg ggagcccctg 5280

ccgccttcaa gtactttgac accaccatcg accggaagag gtacaccagc accaaagagg 5340

tgctggacgc caccctgatc caccagagca tcaccggcct gtacgagaca cggatcgacc 5400

tgtctcagct gggaggcgac agccccaaga agaagagaaa ggtggaggcc agctaagaat 5460

tcctagagct cgctgatcag cctcgactgt gccttctagt tgccagccat ctgttgtttg 5520

cccctccccc gtgccttcct tgaccctgga aggtgccact cccactgtcc tttcctaata 5580

aaatgaggaa attgcatcgc attgtctgag taggtgtcat tctattctgg ggggtggggt 5640

ggggcaggac agcaaggggg aggattggga agagaatagc aggcatgctg gggagcggcc 5700

gcaggaaccc ctagtgatgg agttggccac tccctctctg cgcgctcgct cgctcactga 5760

ggccgggcga ccaaaggtcg cccgacgccc gggctttgcc cgggcggcct cagtgagcga 5820

gcgagcgcgc agctgcctgc aggggcgcct gatgcggtat tttctcctta cgcatctgtg 5880

cggtatttca caccgcatac gtcaaagcaa ccatagtacg cgccctgtag cggcgcatta 5940

agcgcggcgg gtgtggtggt tacgcgcagc gtgaccgcta cacttgccag cgccctagcg 6000

cccgctcctt tcgctttctt cccttccttt ctcgccacgt tcgccggctt tccccgtcaa 6060

gctctaaatc gggggctccc tttagggttc cgatttagtg ctttacggca cctcgacccc 6120

aaaaaacttg atttgggtga tggttcacgt agtgggccat cgccctgata gacggttttt 6180

cgccctttga cgttggagtc cacgttcttt aatagtggac tcttgttcca aactggaaca 6240

acactcaacc ctatctcggg ctattctttt gatttataag ggattttgcc gatttcggcc 6300

tattggttaa aaaatgagct gatttaacaa aaatttaacg cgaattttaa caaaatatta 6360

acgtttacaa ttttatggtg cactctcagt acaatctgct ctgatgccgc atagttaagc 6420

cagccccgac acccgccaac acccgctgac gcgccctgac gggcttgtct gctcccggca 6480

tccgcttaca gacaagctgt gaccgtctcc gggagctgca tgtgtcagag gttttcaccg 6540

tcatcaccga aacgcgcgag acgaaagggc ctcgtgatac gcctattttt ataggttaat 6600

gtcatgataa taatggtttc ttagacgtca ggtggcactt ttcggggaaa tgtgcgcgga 6660

acccctattt gtttattttt ctaaatacat tcaaatatgt atccgctcat gagacaataa 6720

ccctgataaa tgcttcaata atattgaaaa aggaagagta tgagtattca acatttccgt 6780

gtcgccctta ttcccttttt tgcggcattt tgccttcctg tttttgctca cccagaaacg 6840

ctggtgaaag taaaagatgc tgaagatcag ttgggtgcac gagtgggtta catcgaactg 6900

gatctcaaca gcggtaagat ccttgagagt tttcgccccg aagaacgttt tccaatgatg 6960

agcactttta aagttctgct atgtggcgcg gtattatccc gtattgacgc cgggcaagag 7020

caactcggtc gccgcataca ctattctcag aatgacttgg ttgagtactc accagtcaca 7080

gaaaagcatc ttacggatgg catgacagta agagaattat gcagtgctgc cataaccatg 7140

agtgataaca ctgcggccaa cttacttctg acaacgatcg gaggaccgaa ggagctaacc 7200

gcttttttgc acaacatggg ggatcatgta actcgccttg atcgttggga accggagctg 7260

aatgaagcca taccaaacga cgagcgtgac accacgatgc ctgtagcaat ggcaacaacg 7320

ttgcgcaaac tattaactgg cgaactactt actctagctt cccggcaaca attaatagac 7380

tggatggagg cggataaagt tgcaggacca cttctgcgct cggcccttcc ggctggctgg 7440

tttattgctg ataaatctgg agccggtgag cgtggaagcc gcggtatcat tgcagcactg 7500

gggccagatg gtaagccctc ccgtatcgta gttatctaca cgacggggag tcaggcaact 7560

atggatgaac gaaatagaca gatcgctgag ataggtgcct cactgattaa gcattggtaa 7620

ctgtcagacc aagtttactc atatatactt tagattgatt taaaacttca tttttaattt 7680

aaaaggatct aggtgaagat cctttttgat aatctcatga ccaaaatccc ttaacgtgag 7740

ttttcgttcc actgagcgtc agaccccgta gaaaagatca aaggatcttc ttgagatcct 7800

ttttttctgc gcgtaatctg ctgcttgcaa acaaaaaaac caccgctacc agcggtggtt 7860

tgtttgccgg atcaagagct accaactctt tttccgaagg taactggctt cagcagagcg 7920

cagataccaa atactgtcct tctagtgtag ccgtagttag gccaccactt caagaactct 7980

gtagcaccgc ctacatacct cgctctgcta atcctgttac cagtggctgc tgccagtggc 8040

gataagtcgt gtcttaccgg gttggactca agacgatagt taccggataa ggcgcagcgg 8100

tcgggctgaa cggggggttc gtgcacacag cccagcttgg agcgaacgac ctacaccgaa 8160

ctgagatacc tacagcgtga gctatgagaa agcgccacgc ttcccgaagg gagaaaggcg 8220

gacaggtatc cggtaagcgg cagggtcgga acaggagagc gcacgaggga gcttccaggg 8280

ggaaacgcct ggtatcttta tagtcctgtc gggtttcgcc acctctgact tgagcgtcga 8340

tttttgtgat gctcgtcagg ggggcggagc ctatggaaaa acgccagcaa cgcggccttt 8400

ttacggttcc tggccttttg ctggcctttt gctcacatgt 8440

<210> 82
<211> 8509
<212> DNA
<213> Artificial Sequence

<220>
<223> synthetic DNA

<400> 82
cgcctccctg ggcacatacc acgatctgct gaaaattatc aaggacaagg acttcctgga 60

caatgaggaa aacgaggaca ttctggaaga tatcgtgctg accctgacac tgtttgagga 120

cagagagatg atcgaggaac ggctgaaaac ctatgcccac ctgttcgacg acaaagtgat 180

gaagcagctg aagcggcgga gatacaccgg ctggggcagg ctgagccgga agctgatcaa 240

cggcatccgg gacaagcagt ccggcaagac aatcctggat ttcctgaagt ccgacggctt 300

cgccaacaga aacttcatgc agctgatcca cgacgacagc ctgaccttta aagaggacat 360

ccagaaagcc caggtgtccg gccagggcga tagcctgcac gagcacattg ccaatctggc 420

cggcagcccc gccattaaga agggcatcct gcagacagtg aaggtggtgg acgagctcgt 480

gaaagtgatg ggccggcaca agcccgagaa catcgtgatc gaaatggcca gagagaacca 540

gaccacccag aagggacaga agaacagccg cgagagaatg aagcggatcg aagagggcat 600

caaagagctg ggcagccaga tcctgaaaga acaccccgtg gaaaacaccc agctgcagaa 660

cgagaagctg tacctgtact acctgcagaa tgggcgggat atgtacgtgg accaggaact 720

ggacatcaac cggctgtccg actacgatgt ggaccatatc gtgcctcaga gctttctgaa 780

ggacgactcc atcgacaaca aggtgctgac cagaagcgac aagaaccggg gcaagagcga 840

caacgtgccc tccgaagagg tcgtgaagaa gatgaagaac tactggcggc agctgctgaa 900

cgccaagctg attacccaga gaaagttcga caatctgacc aaggccgaga gaggcggcct 960

gagcgaactg gataaggccg gcttcatcaa gagacagctg gtggaaaccc ggcagatcac 1020

aaagcacgtg gcacagatcc tggactcccg gatgaacact aagtacgacg agaatgacaa 1080

gctgatccgg gaagtgaaag tgatcaccct gaagtccaag ctggtgtccg atttccggaa 1140

ggatttccag ttttacaaag tgcgcgagat caacaactac caccacgccc acgacgccta 1200

cctgaacgcc gtcgtgggaa ccgccctgat caaaaagtac cctaagctgg aaagcgagtt 1260

cgtgtacggc gactacaagg tgtacgacgt gcggaagatg atcgccaaga gcgagcagga 1320

aatcggcaag gctaccgcca agtacttctt ctacagcaac atcatgaact ttttcaagac 1380

cgagattacc ctggccaacg gcgagatccg gaagcggcct ctgatcgaga caaacggcga 1440

aaccggggag atcgtgtggg ataagggccg ggattttgcc accgtgcgga aagtgctgag 1500

catgccccaa gtgaatatcg tgaaaaagac cgaggtgcag acaggcggct tcagcaaaga 1560

gtctatcctg cccaagagga acagcgataa gctgatcgcc agaaagaagg actgggaccc 1620

taagaagtac ggcggcttcg acagccccac cgtggcctat tctgtgctgg tggtggccaa 1680

agtggaaaag ggcaagtcca agaaactgaa gagtgtgaaa gagctgctgg ggatcaccat 1740

catggaaaga agcagcttcg agaagaatcc catcgacttt ctggaagcca agggctacaa 1800

agaagtgaaa aaggacctga tcatcaagct gcctaagtac tccctgttcg agctggaaaa 1860

cggccggaag agaatgctgg cctctgccgg cgaactgcag aagggaaacg aactggccct 1920

gccctccaaa tatgtgaact tcctgtacct ggccagccac tatgagaagc tgaagggctc 1980

ccccgaggat aatgagcaga aacagctgtt tgtggaacag cacaagcact acctggacga 2040

gatcatcgag cagatcagcg agttctccaa gagagtgatc ctggccgacg ctaatctgga 2100

caaagtgctg tccgcctaca acaagcaccg ggataagccc atcagagagc aggccgagaa 2160

tatcatccac ctgtttaccc tgaccaatct gggagcccct gccgccttca agtactttga 2220

caccaccatc gaccggaaga ggtacaccag caccaaagag gtgctggacg ccaccctgat 2280

ccaccagagc atcaccggcc tgtacgagac acggatcgac ctgtctcagc tgggaggcga 2340

caaaaggccg gcggccacga aaaaggccgg ccaggcaaaa aagaaaaagt aagaattcct 2400

agagctcgct gatcagcctc gactgtgcct tctagttgcc agccatctgt tgtttgcccc 2460

tcccccgtgc cttccttgac cctggaaggt gccactccca ctgtcctttc ctaataaaat 2520

gaggaaattg catcgcattg tctgagtagg tgtcattcta ttctgggggg tggggtgggg 2580

caggacagca agggggagga ttgggaagag aatagcaggc atgctgggga gcggccgcag 2640

gaacccctag tgatggagtt ggccactccc tctctgcgcg ctcgctcgct cactgaggcc 2700

gggcgaccaa aggtcgcccg acgcccgggc tttgcccggg cggcctcagt gagcgagcga 2760

gcgcgcagct gcctgcaggg gcgcctgatg cggtattttc tccttacgca tctgtgcggt 2820

atttcacacc gcatacgtca aagcaaccat agtacgcgcc ctgtagcggc gcattaagcg 2880

cggcgggtgt ggtggttacg cgcagcgtga ccgctacact tgccagcgcc ctagcgcccg 2940

ctcctttcgc tttcttccct tcctttctcg ccacgttcgc cggctttccc cgtcaagctc 3000

taaatcgggg gctcccttta gggttccgat ttagtgcttt acggcacctc gaccccaaaa 3060

aacttgattt gggtgatggt tcacgtagtg ggccatcgcc ctgatagacg gtttttcgcc 3120

ctttgacgtt ggagtccacg ttctttaata gtggactctt gttccaaact ggaacaacac 3180

tcaaccctat ctcgggctat tcttttgatt tataagggat tttgccgatt tcggcctatt 3240

ggttaaaaaa tgagctgatt taacaaaaat ttaacgcgaa ttttaacaaa atattaacgt 3300

ttacaatttt atggtgcact ctcagtacaa tctgctctga tgccgcatag ttaagccagc 3360

cccgacaccc gccaacaccc gctgacgcgc cctgacgggc ttgtctgctc ccggcatccg 3420

cttacagaca agctgtgacc gtctccggga gctgcatgtg tcagaggttt tcaccgtcat 3480

caccgaaacg cgcgagacga aagggcctcg tgatacgcct atttttatag gttaatgtca 3540

tgataataat ggtttcttag acgtcaggtg gcacttttcg gggaaatgtg cgcggaaccc 3600

ctatttgttt atttttctaa atacattcaa atatgtatcc gctcatgaga caataaccct 3660

gataaatgct tcaataatat tgaaaaagga agagtatgag tattcaacat ttccgtgtcg 3720

cccttattcc cttttttgcg gcattttgcc ttcctgtttt tgctcaccca gaaacgctgg 3780

tgaaagtaaa agatgctgaa gatcagttgg gtgcacgagt gggttacatc gaactggatc 3840

tcaacagcgg taagatcctt gagagttttc gccccgaaga acgttttcca atgatgagca 3900

cttttaaagt tctgctatgt ggcgcggtat tatcccgtat tgacgccggg caagagcaac 3960

tcggtcgccg catacactat tctcagaatg acttggttga gtactcacca gtcacagaaa 4020

agcatcttac ggatggcatg acagtaagag aattatgcag tgctgccata accatgagtg 4080

ataacactgc ggccaactta cttctgacaa cgatcggagg accgaaggag ctaaccgctt 4140

ttttgcacaa catgggggat catgtaactc gccttgatcg ttgggaaccg gagctgaatg 4200

aagccatacc aaacgacgag cgtgacacca cgatgcctgt agcaatggca acaacgttgc 4260

gcaaactatt aactggcgaa ctacttactc tagcttcccg gcaacaatta atagactgga 4320

tggaggcgga taaagttgca ggaccacttc tgcgctcggc ccttccggct ggctggttta 4380

ttgctgataa atctggagcc ggtgagcgtg gaagccgcgg tatcattgca gcactggggc 4440

cagatggtaa gccctcccgt atcgtagtta tctacacgac ggggagtcag gcaactatgg 4500

atgaacgaaa tagacagatc gctgagatag gtgcctcact gattaagcat tggtaactgt 4560

cagaccaagt ttactcatat atactttaga ttgatttaaa acttcatttt taatttaaaa 4620

ggatctaggt gaagatcctt tttgataatc tcatgaccaa aatcccttaa cgtgagtttt 4680

cgttccactg agcgtcagac cccgtagaaa agatcaaagg atcttcttga gatccttttt 4740

ttctgcgcgt aatctgctgc ttgcaaacaa aaaaaccacc gctaccagcg gtggtttgtt 4800

tgccggatca agagctacca actctttttc cgaaggtaac tggcttcagc agagcgcaga 4860

taccaaatac tgtccttcta gtgtagccgt agttaggcca ccacttcaag aactctgtag 4920

caccgcctac atacctcgct ctgctaatcc tgttaccagt ggctgctgcc agtggcgata 4980

agtcgtgtct taccgggttg gactcaagac gatagttacc ggataaggcg cagcggtcgg 5040

gctgaacggg gggttcgtgc acacagccca gcttggagcg aacgacctac accgaactga 5100

gatacctaca gcgtgagcta tgagaaagcg ccacgcttcc cgaagggaga aaggcggaca 5160

ggtatccggt aagcggcagg gtcggaacag gagagcgcac gagggagctt ccagggggaa 5220

acgcctggta tctttatagt cctgtcgggt ttcgccacct ctgacttgag cgtcgatttt 5280

tgtgatgctc gtcagggggg cggagcctat ggaaaaacgc cagcaacgcg gcctttttac 5340

ggttcctggc cttttgctgg ccttttgctc acatgtgagg gcctatttcc catgattcct 5400

tcatatttgc atatacgata caaggctgtt agagagataa ttggaattaa tttgactgta 5460

aacacaaaga tattagtaca aaatacgtga cgtagaaagt aataatttct tgggtagttt 5520

gcagttttaa aattatgttt taaaatggac tatcatatgc ttaccgtaac ttgaaagtat 5580

ttcgatttct tggctttata tatcttgtgg aaaggacgaa acaccgccat agttagtcaa 5640

tgggtagttt tagagctaga aatagcaagt taaaataagg ctagtccgtt atcaacttga 5700

aaaagtggca ccgagtcggt gcttttttgt tttagagcta gaaatagcaa gttaaaataa 5760

ggctagtccg tttttagcgc gtgcgccaat tctgcagaca aatggctcta gaggtacccg 5820

ttacataact tacggtaaat ggcccgcctg gctgaccgcc caacgacccc cgcccattga 5880

cgtcaatagt aacgccaata gggactttcc attgacgtca atgggtggag tatttacggt 5940

aaactgccca cttggcagta catcaagtgt atcatatgcc aagtacgccc cctattgacg 6000

tcaatgacgg taaatggccc gcctggcatt gtgcccagta catgacctta tgggactttc 6060

ctacttggca gtacatctac gtattagtca tcgctattac catggtcgag gtgagcccca 6120

cgttctgctt cactctcccc atctcccccc cctccccacc cccaattttg tatttattta 6180

ttttttaatt attttgtgca gcgatggggg cggggggggg gggggggcgc gcgccaggcg 6240

gggcggggcg gggcgagggg cggggcgggg cgaggcggag aggtgcggcg gcagccaatc 6300

agagcggcgc gctccgaaag tttcctttta tggcgaggcg gcggcggcgg cggccctata 6360

aaaagcgaag cgcgcggcgg gcgggagtcg ctgcgacgct gccttcgccc cgtgccccgc 6420

tccgccgccg cctcgcgccg cccgccccgg ctctgactga ccgcgttact cccacaggtg 6480

agcgggcggg acggcccttc tcctccgggc tgtaattagc tgagcaagag gtaagggttt 6540

aagggatggt tggttggtgg ggtattaatg tttaattacc tggagcacct gcctgaaatc 6600

actttttttc aggttggacc ggtgccacca tggactataa ggaccacgac ggagactaca 6660

aggatcatga tattgattac aaagacgatg acgataagat ggccccaaag aagaagcgga 6720

aggtcggtat ccacggagtc ccagcagccg acaagaagta cagcatcggc ctggacatcg 6780

gcaccaactc tgtgggctgg gccgtgatca ccgacgagta caaggtgccc agcaagaaat 6840

tcaaggtgct gggcaacacc gaccggcaca gcatcaagaa gaacctgatc ggagccctgc 6900

tgttcgacag cggcgaaaca gccgaggcca cccggctgaa gagaaccgcc agaagaagat 6960

acaccagacg gaagaaccgg atctgctatc tgcaagagat cttcagcaac gagatggcca 7020

aggtggacga cagcttcttc cacagactgg aagagtcctt cctggtggaa gaggataaga 7080

agcacgagcg gcaccccatc ttcggcaaca tcgtggacga ggtggcctac cacgagaagt 7140

accccaccat ctaccacctg agaaagaaac tggtggacag caccgacaag gccgacctgc 7200

ggctgatcta tctggccctg gcccacatga tcaagttccg gggccacttc ctgatcgagg 7260

gcgacctgaa ccccgacaac agcgacgtgg acaagctgtt catccagctg gtgcagacct 7320

acaaccagct gttcgaggaa aaccccatca acgccagcgg cgtggacgcc aaggccatcc 7380

tgtctgccag actgagcaag agcagacggc tggaaaatct gatcgcccag ctgcccggcg 7440

agaagaagaa tggcctgttc ggaaacctga ttgccctgag cctgggcctg acccccaact 7500

tcaagagcaa cttcgacctg gccgaggatg ccaaactgca gctgagcaag gacacctacg 7560

acgacgacct ggacaacctg ctggcccaga tcggcgacca gtacgccgac ctgtttctgg 7620

ccgccaagaa cctgtccgac gccatcctgc tgagcgacat cctgagagtg aacaccgaga 7680

tcaccaaggc ccccctgagc gcctctatga tcaagagata cgacgagcac caccaggacc 7740

tgaccctgct gaaagctctc gtgcggcagc agctgcctga gaagtacaaa gagattttct 7800

tcgaccagag caagaacggc tacgccggct acattgacgg cggagccagc caggaagagt 7860

tctacaagtt catcaagccc atcctggaaa agatggacgg caccgaggaa ctgctcgtga 7920

agctgaacag agaggacctg ctgcggaagc agcggacctt cgacaacggc agcatccccc 7980

accagatcca cctgggagag ctgcacgcca ttctgcggcg gcaggaagat ttttacccat 8040

tcctgaagga caaccgggaa aagatcgaga agatcctgac cttccgcatc ccctactacg 8100

tgggccctct ggccagggga aacagcagat tcgcctggat gaccagaaag agcgaggaaa 8160

ccatcacccc ctggaacttc gaggaagtgg tggacaaggg cgcttccgcc cagagcttca 8220

tcgagcggat gaccaacttc gataagaacc tgcccaacga gaaggtgctg cccaagcaca 8280

gcctgctgta cgagtacttc accgtgtata acgagctgac caaagtgaaa tacgtgaccg 8340

agggaatgag aaagcccgcc ttcctgagcg gcgagcagaa aaaggccatc gtggacctgc 8400

tgttcaagac caaccggaaa gtgaccgtga agcagctgaa agaggactac ttcaagaaaa 8460

tcgagtgctt cgactccgtg gaaatctccg gcgtggaaga tcggttcaa 8509

<210> 83
<211> 20
<212> DNA
<213> Artificial Sequence

<220>
<223> oligonucleotide

<400> 83
ggcccacctg gagctgttaa 20

<210> 84
<211> 22
<212> DNA
<213> Artificial Sequence

<220>
<223> oligonucleotide

<400> 84
cgccgtttat gactttgctc tt 22

<210> 85
<211> 20
<212> DNA
<213> Artificial Sequence

<220>
<223> oligonucleotide

<400> 85
agacgccatc cacgctgttt 20

<210> 86
<211> 20
<212> DNA
<213> Artificial Sequence

<220>
<223> oligonucleotide

<400>  86
gccaaaactc ataggccaaa                                                   20


<210>  87
<211>  870
<212>  DNA
<213>  Artificial Sequence

<220>
<223>  oligonucleotide

<400>  87
ccacagggag tgttagtgct gctcgcgatt ttgctggcat ctctatactc ttcgtgtgtg        60

gcagcctaag accgcaagca gaagaacttg ccagaacttt gccagcatta actcagcgct       120

tctcaacttc ctaaggttgc gacccttcaa catagcgtct catgttgtga tgccctgcta       180

ccataattat tccattgcta cttcataact gtaatttttt tgctgctgtt atgagttgta       240

aggtaaacta tctgatatgc gacccccaag agtcacgacc cacaggctga gaaccgctgc       300

cttggccagc cttgagggag ggatggcgtt gtttcctcgg tcaggaaaaa aaaaggaaaa       360

ctcatcgcag gattaagact ctgtaatctc aacagatctg aaataaagaa tgagcatccg       420

ttttctagtt atattcagaa ggacggctta gtcaaccact ttgcacttcc aaaatgcctt       480

ccctctcttc agagtgtctc tcagagcaca tggaccttcc tcattggtca gattttctgt       540

cagtaccacc aacaattgct attggtccat tttgaagatt ctcccgcctt cttttaccta       600

attggtttat tcgaaaaggc ggtcttttgt tcgaataaaa gacgatctac gattggttac       660

tgcaaacaga gccaatgaga gcgagtcggt gattggttcc tctgatttgg gcgggctaga       720

taaaggttca aatgaaacta gggaagctct cctaaccgac aagcgtctgt gaggggagcg       780

tccaggtccc gcgtcgcgcg tcgcgggtcg cctagcgttt cccgctggtt ttgtcgcttt       840

ccggattctc gagcctcttc aggaccgtca                                        870


<210>  88
<211>  37
<212>  DNA
<213>  Artificial Sequence

<220>
<223>  synthetic DNA

<400>  88
aattcctgca ggccacaggg agtgttagtg ctgctcg                                37


<210>  89
<211>  36
<212>  DNA
<213>  Artificial Sequence

<220>

<223>  synthetic DNA

<400>  89
ttactagttg acggtcctga agaggctcga gaatcc                                36


<210>  90
<211>  818
<212>  DNA
<213>  Artificial Sequence

<220>
<223>  synthetic DNA

<400>  90
agttgtcacc gctgcaggta cagctcgcgg atggggccgg gtagtagagc ctttacgtgg      60

cctctgcgtt catggctgac ggcgagagtt gaggttccag ggccgagcgc tggccggcga     120

ccggctggat ccggtcagcc ccatcttctg ggccttgagg ggtcgtggac tgggtgaagc     180

tctttccgaa atgatatcta taatctaatc tgggttcttg agtgttgtgt ttttattgtt     240

tgtgggagca ggttcaacta gtcctgcgat cggtctgtag tctaatggct tctgtgtgac     300

ttccagccgg cttatactcg aattcagtgg ccctcttccc ttccctgggt gatttgaata     360

actcatcacg gaaggtgcag agcatggcag ttcccattga ggagctggcc gaccgagtgg     420

ctcaaaaaaa gtccagtttc gggatgtgaa tgtagccaag ggtttgcatt gctagcatcc     480

caactgctac gggtggatac catctgtaat cggcacgagc ctatgtcggc actcactaaa     540

tattttccca ttagatggaa gaagttgagt gtagtttatc gatgacataa aaaatattac     600

tcaaggcaga aatgtccata cttgcagaac taaaatacct atttaaacgg gcatctgtag     660

ccagacctag tggggcaggc ctgaaatggc agctgctcca gagactgagg caagaggatg     720

ccttaagcta tttaatggga ctctcaaaaa cagcaacagg gggtgaaaaa aatggtctgg     780

gaattttgct cagtggtcag agcatttgcc tagcatgt                              818


<210>  91
<211>  27
<212>  DNA
<213>  Artificial Sequence

<220>
<223>  oligonucleotide

<400>  91
ccgtcaccat ggagttgtca ccgctgc                                          27


<210>  92
<211>  30
<212>  DNA
<213>  Artificial Sequence

<220>
<223>  oligonucleotide

<400>  92
cccttgcatg cacatgctag gcaaatgctc                                        30


<210>  93
<211>  8347
<212>  DNA
<213>  Artificial Sequence

<220>
<223>  synthetic DNA

<400>  93
tatagtgagt cgtattacaa ttcactggcc gtcgttttac aacgtcgtga ctgggaaaac        60

cctggcgtta cccaacttaa tcgccttgca gcacatcccc ctttcgccag ctggcgtaat       120

agcgaagagg cccgcaccga tcgcccttcc caacagttgc gcagcctgaa tggcgaatgg       180

acgcgccctg tagcggcgca ttaagcgcgg cgggtgtggt ggttacgcgc agcgtgaccg       240

ctacacttgc cagcgcccta gcgcccgctc ctttcgcttt cttcccttcc tttctcgcca       300

cgttcgccgg ctttccccgt caagctctaa atcgggggct ccctttaggg ttccgattta       360

gtgctttacg gcacctcgac cccaaaaaac ttgattaggg tgatggttca cgtagtgggc       420

catcgccctg atagacggtt tttcgccctt tgacgttgga gtccacgttc tttaatagtg       480

gactcttgtt ccaaactgga acaacactca accctatctc ggtctattct tttgatttat       540

aagggatttt gccgatttcg gcctattggt taaaaaatga gctgatttaa caaaaattta       600

acgcgaattt taacaaaata ttaacgctta caatttcctg atgcggtatt ttctccttac       660

gcatctgtgc ggtatttcac accgcatcag gtggcacttt tcggggaaat gtgcgcggaa       720

cccctatttg tttatttttc taaatacatt caaatatgta tccgctcatg agacaataac       780

cctgataaat gcttcaataa tattgaaaaa ggaagagtat gagtattcaa catttccgtg       840

tcgcccttat tccctttttt gcggcatttt gccttcctgt ttttgctcac ccagaaacgc       900

tggtgaaagt aaaagatgct gaagatcagt tgggtgcacg agtgggttac atcgaactgg       960

atctcaacag cggtaagatc cttgagagtt ttcgccccga agaacgtttt ccaatgatga      1020

gcacttttaa agttctgcta tgtggcgcgg tattatcccg tattgacgcc gggcaagagc      1080

aactcggtcg ccgcatacac tattctcaga atgacttggt tgagtactca ccagtcacag      1140

aaaagcatct tacggatggc atgacagtaa gagaattatg cagtgctgcc ataaccatga      1200

gtgataacac tgcggccaac ttacttctga caacgatcgg aggaccgaag gagctaaccg      1260

cttttttgca caacatgggg gatcatgtaa ctcgccttga tcgttgggaa ccggagctga      1320

atgaagccat accaaacgac gagcgtgaca ccacgatgcc tgtagcaatg gcaacaacgt      1380

tgcgcaaact attaactggc gaactactta ctctagcttc ccggcaacaa ttaatagact      1440

ggatggaggc ggataaagtt gcaggaccac ttctgcgctc ggcccttccg gctggctggt      1500

ttattgctga taaatctgga gccggtgagc gtgggtctcg cggtatcatt gcagcactgg 1560

ggccagatgg taagccctcc cgtatcgtag ttatctacac gacggggagt caggcaacta 1620

tggatgaacg aaatagacag atcgctgaga taggtgcctc actgattaag cattggtaac 1680

tgtcagacca agtttactca tatatacttt agattgattt aaaacttcat ttttaattta 1740

aaaggatcta ggtgaagatc ctttttgata atctcatgac caaaatccct taacgtgagt 1800

tttcgttcca ctgagcgtca gaccccgtag aaaagatcaa aggatcttct tgagatcctt 1860

tttttctgcg cgtaatctgc tgcttgcaaa caaaaaaacc accgctacca gcggtggttt 1920

gtttgccgga tcaagagcta ccaactcttt ttccgaaggt aactggcttc agcagagcgc 1980

agataccaaa tactgttctt ctagtgtagc cgtagttagg ccaccacttc aagaactctg 2040

tagcaccgcc tacatacctc gctctgctaa tcctgttacc agtggctgct gccagtggcg 2100

ataagtcgtg tcttaccggg ttggactcaa gacgatagtt accggataag gcgcagcggt 2160

cgggctgaac ggggggttcg tgcacacagc ccagcttgga gcgaacgacc tacaccgaac 2220

tgagatacct acagcgtgag ctatgagaaa gcgccacgct tcccgaaggg agaaaggcgg 2280

acaggtatcc ggtaagcggc agggtcggaa caggagagcg cacgagggag cttccagggg 2340

gaaacgcctg gtatctttat agtcctgtcg ggtttcgcca cctctgactt gagcgtcgat 2400

ttttgtgatg ctcgtcaggg gggcggagcc tatggaaaaa cgccagcaac gcggcctttt 2460

tacggttcct ggccttttgc tggccttttg ctcacatgtt ctttcctgcg ttatcccctg 2520

attctgtgga taaccgtatt accgcctttg agtgagctga taccgctcgc cgcagccgaa 2580

cgaccgagcg cagcgagtca gtgagcgagg aagcggaaga gcgcccaata cgcaaaccgc 2640

ctctccccgc gcgttggccg attcattaat gcagctggca cgacaggttt cccgactgga 2700

aagcgggcag tgagcgcaac gcaattaatg tgagttagct cactcattag gcaccccagg 2760

ctttacactt tatgcttccg gctcgtatgt tgtgtggaat tgtgagcgga taacaatttc 2820

acacaggaaa cagctatgac catgattacg ccaagctatt taggtgacac tatagaatac 2880

tcaagctatg catccaacgc gttgggagct ctcccatatg gtcgacctgc aggccacagg 2940

gagtgttagt gctgctcgcg attttgctgg catctctata ctcttcgtgt gtggcagcct 3000

aagaccgcaa gcagaagaac ttgccagaac tttgccagca ttaactcagc gcttctcaac 3060

ttcctaaggt tgcgaccctt caacatagcg tctcatgttg tgatgccctg ctaccataat 3120

tattccattg ctacttcata actgtaattt ttttgctgct gttatgagtt gtaaggtaaa 3180

ctatctgata tgcgaccccc aagagtcacg acccacaggc tgagaaccgc tgccttggcc 3240

agccttgagg gagggatggc gttgtttcct cggtcaggaa aaaaaaagga aaactcatcg 3300

caggattaag actctgtaat ctcaacagat ctgaaataaa gaatgagcat ccgttttcta 3360

gttatattca gaaggacggc ttagtcaacc actttgcact tccaaaatgc cttccctctc 3420

ttcagagtgt ctctcagagc acatggacct tcctcattgg tcagattttc tgtcagtacc 3480

accaacaatt gctattggtc cattttgaag attctcccgc cttcttttac ctaattggtt 3540

tattcgaaaa ggcggtcttt tgttcgaata aaagacgatc tacgattggt tactgcaaac 3600

agagccaatg agagcgagtc ggtgattggt tcctctgatt tgggcgggct agataaaggt 3660

tcaaatgaaa ctagggaagc tctcctaacc gacaagcgtc tgtgagggga gcgtccaggt 3720

cccgcgtcgc gcgtcgcggg tcgcctagcg tttcccgctg gttttgtcgc tttccggatt 3780

ctcgagcctc ttcaggaccg tcaactagtg attaccgttc gtatagcata cattatacga 3840

agttatacca tgtctagact ggacaagagc aaagtcataa acggcgctct ggaattactc 3900

aatggagtcg gtatcgaagg cctgacgaca aggaaactcg ctcaaaagct gggagttgag 3960

cagcctaccc tgtactggca cgtgaagaac aagcgggccc tgctcgatgc cctgccaatc 4020

gagatgctgg acaggcatca tacccacttc tgccccctgg aaggcgagtc atggcaagac 4080

tttctgcgga acaacgccaa gtcattccgc tgtgctctcc tctcacatcg cgacggggct 4140

aaagtgcatc tcggcacccg cccaacagag aaacagtacg aaaccctgga aaatcagctc 4200

gcgttcctgt gtcagcaagg cttctccctg gagaacgcac tgtacgctct gtccgccgtg 4260

ggccacttta cactgggctg cgtattggag gaacaggagc atcaagtagc aaaagaggaa 4320

agagagacac ctaccaccga ttctatgccc ccacttctga gacaagcaat tgagctgttc 4380

gaccggcagg gagccgaacc tgccttcctt ttcggcctgg aactaatcat atgtggcctg 4440

gagaaacagc taaagtgcga aagcggcggg ccggccgacg cccttgacga ttttgactta 4500

gacatgctcc cagccgatgc ccttgacgac tttgaccttg atatgctgcc tgctgacgct 4560

cttgacgatt ttgaccttga catgctcccc gggtaactaa gtaaggatcc caaataccac 4620

tgaattaaga attccgcccc tctccctccc ccccccctaa cgttactggc cgaagccgct 4680

tggaataagg ccggtgtgcg tttgtctata tgttattttc caccatattg ccgtcttttg 4740

gcaatgtgag ggcccggaaa cctggccctg tcttcttgac gagcattcct aggggtcttt 4800

cccctctcgc caaaggaatg caaggtctgt tgaatgtcgt gaaggaagca gttcctctgg 4860

aagcttcttg aagacaaaca acgtctgtag cgaccctttg caggcagcgg aaccccccac 4920

ctggcgacag gtgcctctgc ggccaaaagc cacgtgtata agatacacct gcaaaggcgg 4980

cacaacccca gtgccacgtt gtgagttgga tagttgtgga aagagtcaaa tggctctcct 5040

caagcgtatt caacaagggg ctgaaggatg cccagaaggt accccattgt atgggatctg 5100

atctggggcc tcggtgcaca tgctttacat gtgtttagtc gaggttaaaa aacgtctagg 5160

ccccccgaac cacggggacg tggttttcct ttgaaaaaca cgatgataat atggccacaa 5220

ccatgaccga gtacaagccc acggtgcgcc tcgccacccg cgacgacgtc ccccgggccg 5280

tacgcaccct cgccgccgcg ttcgccgact accccgccac gcgccacacc gtcgacccgg 5340

accgccacat cgagcgggtc accgagctgc aagaactctt cctcacgcgc gtcgggctcg 5400

acatcggcaa ggtgtgggtc gcggacgacg gcgccgcggt ggcggtctgg accacgccgg 5460

agagcgtcga agcgggggcg gtgttcgccg agatcggccc gcgcatggcc gagttgagcg 5520

gttcccggct ggccgcgcag caacagatgg aaggcctcct ggcgccgcac cggcccaagg 5580

agcccgcgtg gttcctggcc accgtcggcg tctcgcccga ccaccagggc aagggtctgg 5640

gcagcgccgt cgtgctcccc ggagtggagg cggccgagcg cgccggggtg cccgccttcc 5700

tggagacctc cgcgccccgc aacctcccct tctacgagcg gctcggcttc accgtcaccg 5760

ccgacgtcga ggtgcccgaa ggaccgcgca cctggtgcat gacccgcaag cccggtgcct 5820

gaggatccag acatgataag atacattgat gagtttggac aaaccacaac tagaatgcag 5880

tgaaaaaaat gctttatttg tgaaatttgt gatgctattg ctttatttgt aaccattata 5940

agctgcaata aacaagttaa caacaacaat tgcattcatt ttatgtttca ggttcagggg 6000

gaggtgtggg aggtttttta aagcaagtaa aacctctaca aatgtggtat ggctgattat 6060

gatcctctag agtcgcagat ccagacatga taagatacat tgatgagttt ggacaaacca 6120

caactagaat gcagtgaaaa aaatgcttta tttgtgaaat ttgtgatgct attgctttat 6180

ttgtaaccat tataagctgc aataaacaag ttaacaacaa caattgcatt cattttatgt 6240

ttcaggttca gggggaggtg tgggaggttt tttaaagcaa gtaaaacctc tacaaatgtg 6300

gtatggctga ttatgatcct ctagagtcgc agatccagac atgataagat acattgatga 6360

gtttggacaa accacaacta gaatgcagtg aaaaaaatgc tttatttgtg aaatttgtga 6420

tgctattgct ttatttgtaa ccattataag ctgcaataaa caagttaaca acaacaattg 6480

cattcatttt atgtttcagg ttcaggggga ggtgtgggag gttttttaaa gcaagtaaaa 6540

cctctacaaa tgtggtatgg ctgattatga tcctctagag tcgcagatcc tctagagtcg 6600

cagatctttt tccctctgcc aaaaattatg gggacatcat gaagcccctt gagcatctga 6660

cttctggcta ataaaggaaa tttattttca ttgcaatagt gtgttggaat tttttgtgtc 6720

tctcactcgg aaggacatat gggagggcaa atcatttaaa acatcagaat gagtatttgg 6780

tttagagttt ggcaacatat gccatatgct ggctgccatg aacaaaggtg gctataaaga 6840

ggtcatcagt atatgaaaca gccccctgct gtccattcct tattccatag aaaagccttg 6900

acttgaggtt agattttttt tatattttgt tttgtgttat ttttttcttt aacatcccta 6960

aaattttcct tacatgtttt actagccaga tttttcctcc tctcctgact actcccagtc 7020

atagctgtcc ctcttctctt atgaagatcc ctcgacctgc agcccaagct tatcgaattc 7080

ccgcggtggc ggccgcacgt ctccctatca gtgatagaga agtcgacacg tctcgagctc 7140

cctatcagtg atagagaagg tacgtctaga acgtctccct atcagtgata gagaagtcga 7200

cacgtctcga gctccctatc agtgatagag aaggtacgtc tagaacgtct ccctatcagt 7260

gatagagaag tcgacacgtc tcgagctccc tatcagtgat agagaaggta cgtctagaac 7320

gtctccctat cagtgataga gaagtcgaca cgtctcgagc tccctatcag tgatagagaa 7380

ggtaccccct atataagcag agctcgttta gtgaaccgtc agatcgcctg gagacgccat 7440

ccacgctgtt ttgacctcca tagaagacac cgggaccgat ccagcctccg cggccgccat 7500

ggagttgtca ccgctgcagg tacagctcgc ggatggggcc gggtagtaga gcctttacgt 7560

ggcctctgcg ttcatggctg acggcgagag ttgaggttcc agggccgagc gctggccggc 7620

gaccggctgg atccggtcag ccccatcttc tgggccttga ggggtcgtgg actgggtgaa 7680

gctctttccg aaatgatatc tataatctaa tctgggttct tgagtgttgt gtttttattg 7740

tttgtgggag caggttcaac tagtcctgcg atcggtctgt agtctaatgg cttctgtgtg 7800

acttccagcc ggcttatact cgaattcagt ggccctcttc ccttccctgg gtgatttgaa 7860

taactcatca cggaaggtgc agagcatggc agttcccatt gaggagctgg ccgaccgagt 7920

ggctcaaaaa aagtccagtt tcgggatgtg aatgtagcca agggtttgca ttgctagcat 7980

cccaactgct acgggtggat accatctgta atcggcacga gcctatgtcg gcactcacta 8040

aatattttcc cattagatgg aagaagttga gtgtagttta tcgatgacat aaaaaatatt 8100

actcaaggca gaaatgtcca tacttgcaga actaaaatac ctatttaaac gggcatctgt 8160

agccagacct agtggggcag gcctgaaatg gcagctgctc cagagactga ggcaagagga 8220

tgccttaagc tatttaatgg gactctcaaa aacagcaaca gggggtgaaa aaaatggtct 8280

gggaattttg ctcagtggtc agagcatttg cctagcatgt gcatgcgacg tcgggcccaa 8340

ttcgccc 8347

<210> 94
<211> 24
<212> DNA
<213> Artificial Sequence

<220>
<223> oligonucleotide

<400> 94
caccgcatgg tgacggtcct gaag 24

<210> 95
<211> 24
<212> DNA
<213> Artificial Sequence

<220>
<223> oligonucleotide

<400>  95
aaaccttcag gaccgtcacc atgc                                              24


<210>  96
<211>  24
<212>  DNA
<213>  Artificial Sequence

<220>
<223>  oligonucleotide

<400>  96
caccgccgct gcaggtacag ctcg                                              24


<210>  97
<211>  24
<212>  DNA
<213>  Artificial Sequence

<220>
<223>  oligonucleotide

<400>  97
aaaccgagct gtacctgcag cggc                                              24


<210>  98
<211>  8436
<212>  DNA
<213>  Artificial Sequence

<220>
<223>  synthetic DNA

<400>  98
gagggcctat ttcccatgat tccttcatat ttgcatatac gatacaaggc tgttagagag      60

ataattggaa ttaatttgac tgtaaacaca aagatattag tacaaaatac gtgacgtaga     120

aagtaataat ttcttgggta gtttgcagtt ttaaaattat gttttaaaat ggactatcat     180

atgcttaccg taacttgaaa gtatttcgat ttcttggctt tatatatctt gtggaaagga     240

cgaaacaccg ccgctgcagg tacagctcgg ttttagagct agaaatagca agttaaaata     300

aggctagtcc gttatcaact tgaaaaagtg gcaccgagtc ggtgcttttt tgttttagag     360

ctagaaatag caagttaaaa taaggctagt ccgtttttag cgcgtgcgcc aattctgcag     420

acaaatggct ctagaggtac ccgttacata acttacggta aatggcccgc ctggctgacc     480

gcccaacgac ccccgcccat tgacgtcaat agtaacgcca atagggactt tccattgacg     540

tcaatgggtg gagtatttac ggtaaactgc ccacttggca gtacatcaag tgtatcatat     600

gccaagtacg ccccctattg acgtcaatga cggtaaatgg cccgcctggc attgtgccca     660

gtacatgacc ttatgggact ttcctacttg gcagtacatc tacgtattag tcatcgctat     720

taccatggtc gaggtgagcc ccacgttctg cttcactctc cccatctccc ccccctcccc     780

acccccaatt ttgtatttat ttatttttta attattttgt gcagcgatgg gggcgggggg 840

gggggggggg cgcgcgccag gcggggcggg gcggggcgag gggcggggcg gggcgaggcg 900

gagaggtgcg gcggcagcca atcagagcgg cgcgctccga aagtttcctt ttatggcgag 960

gcggcggcgg cggcggccct ataaaaagcg aagcgcgcgg cgggcgggag tcgctgcgac 1020

gctgccttcg ccccgtgccc cgctccgccg ccgcctcgcg ccgcccgccc cggctctgac 1080

tgaccgcgtt actcccacag gtgagcgggc gggacggccc ttctcctccg ggctgtaatt 1140

agctgagcaa gaggtaaggg tttaagggat ggttggttgg tggggtatta atgtttaatt 1200

acctggagca cctgcctgaa atcacttttt ttcaggttgg accggtgcca ccatgtaccc 1260

atacgatgtt ccagattacg cttcgccgaa gaaaaagcgc aaggtcgaag cgtccgacaa 1320

gaagtacagc atcggcctgg ccatcggcac caactctgtg ggctgggccg tgatcaccga 1380

cgagtacaag gtgcccagca agaaattcaa ggtgctgggc aacaccgacc ggcacagcat 1440

caagaagaac ctgatcggag ccctgctgtt cgacagcggc gaaacagccg aggccacccg 1500

gctgaagaga accgccagaa gaagatacac cagacggaag aaccggatct gctatctgca 1560

agagatcttc agcaacgaga tggccaaggt ggacgacagc ttcttccaca gactggaaga 1620

gtccttcctg gtggaagagg ataagaagca cgagcggcac cccatcttcg gcaacatcgt 1680

ggacgaggtg gcctaccacg agaagtaccc caccatctac cacctgagaa agaaactggt 1740

ggacagcacc gacaaggccg acctgcggct gatctatctg gccctggccc acatgatcaa 1800

gttccggggc cacttcctga tcgagggcga cctgaacccc gacaacagcg acgtggacaa 1860

gctgttcatc cagctggtgc agacctacaa ccagctgttc gaggaaaacc ccatcaacgc 1920

cagcggcgtg gacgccaagg ccatcctgtc tgccagactg agcaagagca gacggctgga 1980

aaatctgatc gcccagctgc ccggcgagaa gaagaatggc ctgttcggca acctgattgc 2040

cctgagcctg ggcctgaccc ccaacttcaa gagcaacttc gacctggccg aggatgccaa 2100

actgcagctg agcaaggaca cctacgacga cgacctggac aacctgctgg cccagatcgg 2160

cgaccagtac gccgacctgt ttctggccgc caagaacctg tccgacgcca tcctgctgag 2220

cgacatcctg agagtgaaca ccgagatcac caaggccccc ctgagcgcct ctatgatcaa 2280

gagatacgac gagcaccacc aggacctgac cctgctgaaa gctctcgtgc ggcagcagct 2340

gcctgagaag tacaaagaga ttttcttcga ccagagcaag aacggctacg ccggctacat 2400

tgacggcgga gccagccagg aagagttcta caagttcatc aagcccatcc tggaaaagat 2460

ggacggcacc gaggaactgc tcgtgaagct gaacagagag gacctgctgc ggaagcagcg 2520

gaccttcgac aacggcagca tcccccacca gatccacctg ggagagctgc acgccattct 2580

gcggcggcag gaagattttt acccattcct gaaggacaac cgggaaaaga tcgagaagat 2640

cctgaccttc cgcatcccct actacgtggg ccctctggcc aggggaaaca gcagattcgc 2700

ctggatgacc agaaagagcg aggaaaccat caccccctgg aacttcgagg aagtggtgga 2760

caagggcgct tccgcccaga gcttcatcga gcggatgacc aacttcgata agaacctgcc 2820

caacgagaag gtgctgccca agcacagcct gctgtacgag tacttcaccg tgtataacga 2880

gctgaccaaa gtgaaatacg tgaccgaggg aatgagaaag cccgccttcc tgagcggcga 2940

gcagaaaaag gccatcgtgg acctgctgtt caagaccaac cggaaagtga ccgtgaagca 3000

gctgaaagag gactacttca agaaaatcga gtgcttcgac tccgtggaaa tctccggcgt 3060

ggaagatcgg ttcaacgcct ccctgggcac ataccacgat ctgctgaaaa ttatcaagga 3120

caaggacttc ctggacaatg aggaaaacga ggacattctg gaagatatcg tgctgaccct 3180

gacactgttt gaggacagag agatgatcga ggaacggctg aaaacctatg cccacctgtt 3240

cgacgacaaa gtgatgaagc agctgaagcg gcggagatac accggctggg gcaggctgag 3300

ccggaagctg atcaacggca tccgggacaa gcagtccggc aagacaatcc tggatttcct 3360

gaagtccgac ggcttcgcca acagaaactt catgcagctg atccacgacg acagcctgac 3420

ctttaaagag gacatccaga aagcccaggt gtccggccag ggcgatagcc tgcacgagca 3480

cattgccaat ctggccggca gccccgccat taagaagggc atcctgcaga cagtgaaggt 3540

ggtggacgag ctcgtgaaag tgatgggccg gcacaagccc gagaacatcg tgatcgaaat 3600

ggccagagag aaccagacca cccagaaggg acagaagaac agccgcgaga gaatgaagcg 3660

gatcgaagag ggcatcaaag agctgggcag ccagatcctg aaagaacacc ccgtggaaaa 3720

cacccagctg cagaacgaga agctgtacct gtactacctg cagaatgggc gggatatgta 3780

cgtggaccag gaactggaca tcaaccggct gtccgactac gatgtggacc atatcgtgcc 3840

tcagagcttt ctgaaggacg actccatcga caacaaggtg ctgaccagaa gcgacaagaa 3900

ccggggcaag agcgacaacg tgccctccga agaggtcgtg aagaagatga agaactactg 3960

gcggcagctg ctgaacgcca agctgattac ccagagaaag ttcgacaatc tgaccaaggc 4020

cgagagaggc ggcctgagcg aactggataa ggccggcttc atcaagagac agctggtgga 4080

aacccggcag atcacaaagc acgtggcaca gatcctggac tcccggatga acactaagta 4140

cgacgagaat gacaagctga tccgggaagt gaaagtgatc accctgaagt ccaagctggt 4200

gtccgatttc cggaaggatt tccagtttta caaagtgcgc gagatcaaca actaccacca 4260

cgcccacgac gcctacctga acgccgtcgt gggaaccgcc ctgatcaaaa agtaccctaa 4320

gctggaaagc gagttcgtgt acggcgacta caaggtgtac gacgtgcgga agatgatcgc 4380

caagagcgag caggaaatcg gcaaggctac cgccaagtac ttcttctaca gcaacatcat 4440

gaactttttc aagaccgaga ttaccctggc caacggcgag atccggaagc ggcctctgat 4500

cgagacaaac ggcgaaaccg gggagatcgt gtgggataag ggccgggatt ttgccaccgt 4560

gcggaaagtg ctgagcatgc cccaagtgaa tatcgtgaaa aagaccgagg tgcagacagg 4620

cggcttcagc aaagagtcta tcctgcccaa gaggaacagc gataagctga tcgccagaaa 4680

gaaggactgg gaccctaaga agtacggcgg cttcgacagc cccaccgtgg cctattctgt 4740

gctggtggtg gccaaagtgg aaaagggcaa gtccaagaaa ctgaagagtg tgaaagagct 4800

gctggggatc accatcatgg aaagaagcag cttcgagaag aatcccatcg actttctgga 4860

agccaagggc tacaaagaag tgaaaaagga cctgatcatc aagctgccta agtactccct 4920

gttcgagctg gaaaacggcc ggaagagaat gctggcctct gccggcgaac tgcagaaggg 4980

aaacgaactg gccctgccct ccaaatatgt gaacttcctg tacctggcca gccactatga 5040

gaagctgaag ggctcccccg aggataatga gcagaaacag ctgtttgtgg aacagcacaa 5100

gcactacctg gacgagatca tcgagcagat cagcgagttc tccaagagag tgatcctggc 5160

cgacgctaat ctggacaaag tgctgtccgc ctacaacaag caccgggata agcccatcag 5220

agagcaggcc gagaatatca tccacctgtt taccctgacc aatctgggag cccctgccgc 5280

cttcaagtac tttgacacca ccatcgaccg gaagaggtac accagcacca aagaggtgct 5340

ggacgccacc ctgatccacc agagcatcac cggcctgtac gagacacgga tcgacctgtc 5400

tcagctggga ggcgacagcc ccaagaagaa gagaaaggtg gaggccagct aagaattcct 5460

agagctcgct gatcagcctc gactgtgcct tctagttgcc agccatctgt tgtttgcccc 5520

tcccccgtgc cttccttgac cctggaaggt gccactccca ctgtcctttc ctaataaaat 5580

gaggaaattg catcgcattg tctgagtagg tgtcattcta ttctgggggg tggggtgggg 5640

caggacagca agggggagga ttgggaagag aatagcaggc atgctgggga gcggccgcag 5700

gaacccctag tgatggagtt ggccactccc tctctgcgcg ctcgctcgct cactgaggcc 5760

gggcgaccaa aggtcgcccg acgcccgggc tttgcccggg cggcctcagt gagcgagcga 5820

gcgcgcagct gcctgcaggg gcgcctgatg cggtattttc tccttacgca tctgtgcggt 5880

atttcacacc gcatacgtca aagcaaccat agtacgcgcc ctgtagcggc gcattaagcg 5940

cggcgggtgt ggtggttacg cgcagcgtga ccgctacact tgccagcgcc ctagcgcccg 6000

ctcctttcgc tttcttccct tcctttctcg ccacgttcgc cggctttccc cgtcaagctc 6060

taaatcgggg gctcccttta gggttccgat ttagtgcttt acggcacctc gaccccaaaa 6120

aacttgattt gggtgatggt tcacgtagtg ggccatcgcc ctgatagacg gtttttcgcc 6180

ctttgacgtt ggagtccacg ttctttaata gtggactctt gttccaaact ggaacaacac 6240

tcaaccctat ctcgggctat tcttttgatt tataagggat tttgccgatt tcggcctatt 6300

ggttaaaaaa tgagctgatt taacaaaaat ttaacgcgaa ttttaacaaa atattaacgt 6360

ttacaatttt atggtgcact ctcagtacaa tctgctctga tgccgcatag ttaagccagc 6420

cccgacaccc gccaacaccc gctgacgcgc cctgacgggc ttgtctgctc ccggcatccg 6480

cttacagaca agctgtgacc gtctccggga gctgcatgtg tcagaggttt tcaccgtcat 6540

caccgaaacg cgcgagacga aagggcctcg tgatacgcct atttttatag gttaatgtca 6600

tgataataat ggtttcttag acgtcaggtg gcacttttcg gggaaatgtg cgcggaaccc 6660

ctatttgttt atttttctaa atacattcaa atatgtatcc gctcatgaga caataaccct 6720

gataaatgct tcaataatat tgaaaaagga agagtatgag tattcaacat ttccgtgtcg 6780

cccttattcc cttttttgcg gcattttgcc ttcctgtttt tgctcaccca gaaacgctgg 6840

tgaaagtaaa agatgctgaa gatcagttgg gtgcacgagt gggttacatc gaactggatc 6900

tcaacagcgg taagatcctt gagagttttc gccccgaaga acgttttcca atgatgagca 6960

cttttaaagt tctgctatgt ggcgcggtat tatcccgtat tgacgccggg caagagcaac 7020

tcggtcgccg catacactat tctcagaatg acttggttga gtactcacca gtcacagaaa 7080

agcatcttac ggatggcatg acagtaagag aattatgcag tgctgccata accatgagtg 7140

ataacactgc ggccaactta cttctgacaa cgatcggagg accgaaggag ctaaccgctt 7200

ttttgcacaa catgggggat catgtaactc gccttgatcg ttgggaaccg gagctgaatg 7260

aagccatacc aaacgacgag cgtgacacca cgatgcctgt agcaatggca acaacgttgc 7320

gcaaactatt aactggcgaa ctacttactc tagcttcccg gcaacaatta atagactgga 7380

tggaggcgga taaagttgca ggaccacttc tgcgctcggc ccttccggct ggctggttta 7440

ttgctgataa atctggagcc ggtgagcgtg gaagccgcgg tatcattgca gcactggggc 7500

cagatggtaa gccctcccgt atcgtagtta tctacacgac ggggagtcag gcaactatgg 7560

atgaacgaaa tagacagatc gctgagatag gtgcctcact gattaagcat tggtaactgt 7620

cagaccaagt ttactcatat atactttaga ttgatttaaa acttcatttt taatttaaaa 7680

ggatctaggt gaagatcctt tttgataatc tcatgaccaa aatcccttaa cgtgagtttt 7740

cgttccactg agcgtcagac cccgtagaaa agatcaaagg atcttcttga gatccttttt 7800

ttctgcgcgt aatctgctgc ttgcaaacaa aaaaaccacc gctaccagcg gtggtttgtt 7860

tgccggatca agagctacca actctttttc cgaaggtaac tggcttcagc agagcgcaga 7920

taccaaatac tgtccttcta gtgtagccgt agttaggcca ccacttcaag aactctgtag 7980

caccgcctac atacctcgct ctgctaatcc tgttaccagt ggctgctgcc agtggcgata 8040

agtcgtgtct taccgggttg gactcaagac gatagttacc ggataaggcg cagcggtcgg 8100

gctgaacggg gggttcgtgc acacagccca gcttggagcg aacgacctac accgaactga 8160

gatacctaca gcgtgagcta tgagaaagcg ccacgcttcc cgaagggaga aaggcggaca 8220

ggtatccggt aagcggcagg gtcggaacag gagagcgcac gagggagctt ccagggggaa 8280

acgcctggta tctttatagt cctgtcgggt ttcgccacct ctgacttgag cgtcgatttt 8340

tgtgatgctc gtcagggggg cggagcctat ggaaaaacgc cagcaacgcg gcctttttac 8400

ggttcctggc cttttgctgg ccttttgctc acatgt 8436

<210> 99
<211> 8436
<212> DNA
<213> Artificial Sequence

<220>
<223> synthetic DNA

<400> 99
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg catggtgacg gtcctgaagg ttttagagct agaaatagca agttaaaata 300

aggctagtcc gttatcaact tgaaaaagtg gcaccgagtc ggtgcttttt tgttttagag 360

ctagaaatag caagttaaaa taaggctagt ccgtttttag cgcgtgcgcc aattctgcag 420

acaaatggct ctagaggtac ccgttacata acttacggta aatggcccgc ctggctgacc 480

gcccaacgac ccccgcccat tgacgtcaat agtaacgcca atagggactt tccattgacg 540

tcaatgggtg gagtatttac ggtaaactgc ccacttggca gtacatcaag tgtatcatat 600

gccaagtacg ccccctattg acgtcaatga cggtaaatgg cccgcctggc attgtgccca 660

gtacatgacc ttatgggact ttcctacttg gcagtacatc tacgtattag tcatcgctat 720

taccatggtc gaggtgagcc ccacgttctg cttcactctc cccatctccc ccccctcccc 780

acccccaatt ttgtatttat ttatttttta attattttgt gcagcgatgg gggcgggggg 840

gggggggggg cgcgcgccag gcggggcggg gcggggcgag gggcggggcg gggcgaggcg 900

gagaggtgcg gcggcagcca atcagagcgg cgcgctccga aagtttcctt ttatggcgag 960

gcggcggcgg cggcggccct ataaaaagcg aagcgcgcgg cgggcgggag tcgctgcgac 1020

gctgccttcg ccccgtgccc cgctccgccg ccgcctcgcg ccgcccgccc cggctctgac 1080

tgaccgcgtt actcccacag gtgagcgggc gggacggccc ttctcctccg ggctgtaatt 1140

agctgagcaa gaggtaaggg tttaagggat ggttggttgg tggggtatta atgtttaatt 1200

acctggagca cctgcctgaa atcacttttt ttcaggttgg accggtgcca ccatgtaccc 1260

atacgatgtt ccagattacg cttcgccgaa gaaaaagcgc aaggtcgaag cgtccgacaa 1320

gaagtacagc atcggcctgg ccatcggcac caactctgtg ggctgggccg tgatcaccga 1380

cgagtacaag gtgcccagca agaaattcaa ggtgctgggc aacaccgacc ggcacagcat 1440

caagaagaac ctgatcggag ccctgctgtt cgacagcggc gaaacagccg aggccacccg 1500

gctgaagaga accgccagaa gaagatacac cagacggaag aaccggatct gctatctgca 1560

agagatcttc agcaacgaga tggccaaggt ggacgacagc ttcttccaca gactggaaga 1620

gtccttcctg gtggaagagg ataagaagca cgagcggcac cccatcttcg gcaacatcgt 1680

ggacgaggtg gcctaccacg agaagtaccc caccatctac cacctgagaa agaaactggt 1740

ggacagcacc gacaaggccg acctgcggct gatctatctg gccctggccc acatgatcaa 1800

gttccggggc cacttcctga tcgagggcga cctgaacccc gacaacagcg acgtggacaa 1860

gctgttcatc cagctggtgc agacctacaa ccagctgttc gaggaaaacc ccatcaacgc 1920

cagcggcgtg gacgccaagg ccatcctgtc tgccagactg agcaagagca gacggctgga 1980

aaatctgatc gcccagctgc ccggcgagaa gaagaatggc ctgttcggca acctgattgc 2040

cctgagcctg ggcctgaccc ccaacttcaa gagcaacttc gacctggccg aggatgccaa 2100

actgcagctg agcaaggaca cctacgacga cgacctggac aacctgctgg cccagatcgg 2160

cgaccagtac gccgacctgt ttctggccgc caagaacctg tccgacgcca tcctgctgag 2220

cgacatcctg agagtgaaca ccgagatcac caaggccccc ctgagcgcct ctatgatcaa 2280

gagatacgac gagcaccacc aggacctgac cctgctgaaa gctctcgtgc ggcagcagct 2340

gcctgagaag tacaaagaga ttttcttcga ccagagcaag aacggctacg ccggctacat 2400

tgacggcgga gccagccagg aagagttcta caagttcatc aagcccatcc tggaaaagat 2460

ggacggcacc gaggaactgc tcgtgaagct gaacagagag gacctgctgc ggaagcagcg 2520

gaccttcgac aacggcagca tcccccacca gatccacctg ggagagctgc acgccattct 2580

gcggcggcag gaagattttt acccattcct gaaggacaac cgggaaaaga tcgagaagat 2640

cctgaccttc cgcatcccct actacgtggg ccctctggcc aggggaaaca gcagattcgc 2700

ctggatgacc agaaagagcg aggaaaccat caccccctgg aacttcgagg aagtggtgga 2760

caagggcgct tccgcccaga gcttcatcga gcggatgacc aacttcgata agaacctgcc 2820

caacgagaag gtgctgccca agcacagcct gctgtacgag tacttcaccg tgtataacga 2880

gctgaccaaa gtgaaatacg tgaccgaggg aatgagaaag cccgccttcc tgagcggcga 2940

gcagaaaaag gccatcgtgg acctgctgtt caagaccaac cggaaagtga ccgtgaagca 3000

gctgaaagag gactacttca agaaaatcga gtgcttcgac tccgtggaaa tctccggcgt 3060

ggaagatcgg ttcaacgcct ccctgggcac ataccacgat ctgctgaaaa ttatcaagga 3120

caaggacttc ctggacaatg aggaaaacga ggacattctg gaagatatcg tgctgaccct 3180

gacactgttt gaggacagag agatgatcga ggaacggctg aaaacctatg cccacctgtt 3240

cgacgacaaa gtgatgaagc agctgaagcg gcggagatac accggctggg gcaggctgag 3300

ccggaagctg atcaacggca tccgggacaa gcagtccggc aagacaatcc tggatttcct 3360

gaagtccgac ggcttcgcca acagaaactt catgcagctg atccacgacg acagcctgac 3420

ctttaaagag gacatccaga aagcccaggt gtccggccag ggcgatagcc tgcacgagca 3480

cattgccaat ctggccggca gccccgccat taagaagggc atcctgcaga cagtgaaggt 3540

ggtggacgag ctcgtgaaag tgatgggccg gcacaagccc gagaacatcg tgatcgaaat 3600

ggccagagag aaccagacca cccagaaggg acagaagaac agccgcgaga gaatgaagcg 3660

gatcgaagag ggcatcaaag agctgggcag ccagatcctg aaagaacacc ccgtggaaaa 3720

cacccagctg cagaacgaga agctgtacct gtactacctg cagaatgggc gggatatgta 3780

cgtggaccag gaactggaca tcaaccggct gtccgactac gatgtggacc atatcgtgcc 3840

tcagagcttt ctgaaggacg actccatcga caacaaggtg ctgaccagaa gcgacaagaa 3900

ccggggcaag agcgacaacg tgccctccga agaggtcgtg aagaagatga agaactactg 3960

gcggcagctg ctgaacgcca agctgattac ccagagaaag ttcgacaatc tgaccaaggc 4020

cgagagaggc ggcctgagcg aactggataa ggccggcttc atcaagagac agctggtgga 4080

aacccggcag atcacaaagc acgtggcaca gatcctggac tcccggatga acactaagta 4140

cgacgagaat gacaagctga tccgggaagt gaaagtgatc accctgaagt ccaagctggt 4200

gtccgatttc cggaaggatt tccagtttta caaagtgcgc gagatcaaca actaccacca 4260

cgcccacgac gcctacctga acgccgtcgt gggaaccgcc ctgatcaaaa agtaccctaa 4320

gctggaaagc gagttcgtgt acggcgacta caaggtgtac gacgtgcgga agatgatcgc 4380

caagagcgag caggaaatcg gcaaggctac cgccaagtac ttcttctaca gcaacatcat 4440

gaactttttc aagaccgaga ttaccctggc caacggcgag atccggaagc ggcctctgat 4500

cgagacaaac ggcgaaaccg gggagatcgt gtgggataag ggccgggatt ttgccaccgt 4560

gcggaaagtg ctgagcatgc cccaagtgaa tatcgtgaaa aagaccgagg tgcagacagg 4620

cggcttcagc aaagagtcta tcctgcccaa gaggaacagc gataagctga tcgccagaaa 4680

gaaggactgg gaccctaaga agtacggcgg cttcgacagc cccaccgtgg cctattctgt 4740

gctggtggtg gccaaagtgg aaaagggcaa gtccaagaaa ctgaagagtg tgaaagagct 4800

gctggggatc accatcatgg aaagaagcag cttcgagaag aatcccatcg actttctgga 4860

agccaagggc tacaaagaag tgaaaaagga cctgatcatc aagctgccta agtactccct 4920

gttcgagctg gaaaacggcc ggaagagaat gctggcctct gccggcgaac tgcagaaggg 4980

aaacgaactg gccctgccct ccaaatatgt gaacttcctg tacctggcca gccactatga 5040

gaagctgaag ggctcccccg aggataatga gcagaaacag ctgtttgtgg aacagcacaa 5100

gcactacctg gacgagatca tcgagcagat cagcgagttc tccaagagag tgatcctggc 5160

cgacgctaat ctggacaaag tgctgtccgc ctacaacaag caccgggata agcccatcag 5220

agagcaggcc gagaatatca tccacctgtt taccctgacc aatctgggag cccctgccgc 5280

cttcaagtac tttgacacca ccatcgaccg gaagaggtac accagcacca aagaggtgct 5340

ggacgccacc ctgatccacc agagcatcac cggcctgtac gagacacgga tcgacctgtc 5400

tcagctggga ggcgacagcc ccaagaagaa gagaaaggtg gaggccagct aagaattcct 5460

agagctcgct gatcagcctc gactgtgcct tctagttgcc agccatctgt tgtttgcccc 5520

tcccccgtgc cttccttgac cctggaaggt gccactccca ctgtcctttc ctaataaaat 5580

gaggaaattg catcgcattg tctgagtagg tgtcattcta ttctgggggg tggggtgggg 5640

caggacagca agggggagga ttgggaagag aatagcaggc atgctgggga gcggccgcag 5700

gaacccctag tgatggagtt ggccactccc tctctgcgcg ctcgctcgct cactgaggcc 5760

gggcgaccaa aggtcgcccg acgcccgggc tttgcccggg cggcctcagt gagcgagcga 5820

gcgcgcagct gcctgcaggg gcgcctgatg cggtattttc tccttacgca tctgtgcggt 5880

atttcacacc gcatacgtca aagcaaccat agtacgcgcc ctgtagcggc gcattaagcg 5940

cggcgggtgt ggtggttacg cgcagcgtga ccgctacact tgccagcgcc ctagcgcccg 6000

ctcctttcgc tttcttccct tcctttctcg ccacgttcgc cggctttccc cgtcaagctc 6060

taaatcgggg gctcccttta gggttccgat ttagtgcttt acggcacctc gaccccaaaa 6120

aacttgattt gggtgatggt tcacgtagtg ggccatcgcc ctgatagacg gtttttcgcc 6180

ctttgacgtt ggagtccacg ttctttaata gtggactctt gttccaaact ggaacaacac 6240

tcaaccctat ctcgggctat tcttttgatt tataagggat tttgccgatt tcggcctatt 6300

ggttaaaaaa tgagctgatt taacaaaaat ttaacgcgaa ttttaacaaa atattaacgt 6360

ttacaatttt atggtgcact ctcagtacaa tctgctctga tgccgcatag ttaagccagc 6420

cccgacaccc gccaacaccc gctgacgcgc cctgacgggc ttgtctgctc ccggcatccg 6480

cttacagaca agctgtgacc gtctccggga gctgcatgtg tcagaggttt tcaccgtcat 6540

caccgaaacg cgcgagacga aagggcctcg tgatacgcct atttttatag gttaatgtca 6600

tgataataat ggtttcttag acgtcaggtg gcacttttcg gggaaatgtg cgcggaaccc 6660

ctatttgttt atttttctaa atacattcaa atatgtatcc gctcatgaga caataaccct 6720

gataaatgct tcaataatat tgaaaaagga agagtatgag tattcaacat ttccgtgtcg 6780

cccttattcc cttttttgcg gcattttgcc ttcctgtttt tgctcaccca gaaacgctgg 6840

tgaaagtaaa agatgctgaa gatcagttgg gtgcacgagt gggttacatc gaactggatc 6900

tcaacagcgg taagatcctt gagagttttc gccccgaaga acgttttcca atgatgagca 6960

cttttaaagt tctgctatgt ggcgcggtat tatcccgtat tgacgccggg caagagcaac 7020

tcggtcgccg catacactat tctcagaatg acttggttga gtactcacca gtcacagaaa 7080

agcatcttac ggatggcatg acagtaagag aattatgcag tgctgccata accatgagtg 7140

ataacactgc ggccaactta cttctgacaa cgatcggagg accgaaggag ctaaccgctt 7200

ttttgcacaa catgggggat catgtaactc gccttgatcg ttgggaaccg gagctgaatg 7260

aagccatacc aaacgacgag cgtgacacca cgatgcctgt agcaatggca acaacgttgc 7320

gcaaactatt aactggcgaa ctacttactc tagcttcccg gcaacaatta atagactgga 7380

tggaggcgga taaagttgca ggaccacttc tgcgctcggc ccttccggct ggctggttta 7440

ttgctgataa atctggagcc ggtgagcgtg gaagccgcgg tatcattgca gcactggggc 7500

cagatggtaa gccctcccgt atcgtagtta tctacacgac ggggagtcag gcaactatgg 7560

atgaacgaaa tagacagatc gctgagatag gtgcctcact gattaagcat tggtaactgt 7620

cagaccaagt ttactcatat atactttaga ttgatttaaa acttcatttt taatttaaaa 7680

ggatctaggt gaagatcctt tttgataatc tcatgaccaa aatcccttaa cgtgagtttt 7740

cgttccactg agcgtcagac cccgtagaaa agatcaaagg atcttcttga gatccttttt 7800

ttctgcgcgt aatctgctgc ttgcaaacaa aaaaaccacc gctaccagcg gtggtttgtt 7860

tgccggatca agagctacca actctttttc cgaaggtaac tggcttcagc agagcgcaga 7920

taccaaatac tgtccttcta gtgtagccgt agttaggcca ccacttcaag aactctgtag 7980

caccgcctac atacctcgct ctgctaatcc tgttaccagt ggctgctgcc agtggcgata 8040

agtcgtgtct taccgggttg gactcaagac gatagttacc ggataaggcg cagcggtcgg 8100

gctgaacggg gggttcgtgc acacagccca gcttggagcg aacgacctac accgaactga 8160

gatacctaca gcgtgagcta tgagaaagcg ccacgcttcc cgaagggaga aaggcggaca 8220

ggtatccggt aagcggcagg gtcggaacag gagagcgcac gagggagctt ccagggggaa 8280

acgcctggta tctttatagt cctgtcgggt ttcgccacct ctgacttgag cgtcgatttt 8340

tgtgatgctc gtcagggggg cggagcctat ggaaaaacgc cagcaacgcg gcctttttac 8400

ggttcctggc cttttgctgg ccttttgctc acatgt 8436

<210> 100
<211> 874
<212> DNA
<213> Artificial Sequence

<220>
<223> synthetic DNA

<400> 100
catttcagat tcgaaccatg acacatttcc atagcagctg tgccatttta caccacccac 60

cccaatgttc caccaggctt ctatgaaacc actgattata taagtatttt cctgattaga 120

gtacacgttc atgatagatc atttagaaaa ctcaaaaaag caaaatgacg aaattctctt 180

tattctaatt acggagccat gacctgaatt catattactc ccgggagaaa aggaaaagcc 240

ggtttttttc tcaggcaatt ctgtcattgt gtttgatagc atggtccaat aatagtgctg 300

gcgcctgccc tgcctggacc ttgtcctcag attcctccgt cctggctgtc cccacgaccc 360

tatcttctga aaaggtcatg cagttaacca ggcttctgaa tcatatttgc tggttgcttt 420

caggagtgtc acgggtgctg tgtggagctg tcacattctt gggcatctgt ttccgtattt 480

ttaaagccac ctcaacgttt gatagaaaag cataaaactt ttgaggaagt gagctctaca 540

aacccaccac ccacgtaaga actgagccgt cctgcccaga gggcatctga aaaagagatc 600

aacaggaaaa gggtggggaa atcctgcctt ccttcccgtc ttcaagtacc cattggcttc 660

agacctttat tctcattggt ttatctggat gtcgctccac gctggctttc cagaaccagc 720

cactgagagt cacaaagttg ggatttaaaa gatgcggtgg gaggggcggg tgattttttg 780

aactgctggc gaccgagttc tggcggtcct gacgcgcacc ttgggcttgc tcgcgttcgg 840

tttcggcagc aggaccaaga aggcttaggg aagg 874

<210> 101
<211> 42
<212> DNA
<213> Artificial Sequence

<220>
<223> oligonucleotide

<400> 101
aattcctgca ggcatttcag attcgaacca tgacacattt cc 42

<210> 102
<211> 37
<212> DNA
<213> Artificial Sequence

<220>
<223> oligonucleotide

<400> 102
ttactagtcc ttccctaagc cttcttggtc ctgctgc 37

<210> 103
<211> 825
<212> DNA
<213> Artificial Sequence

<220>
<223> synthetic DNA

<400> 103
gcccgcgtcg caaaccgcag accccaagga ggagaccctc cagcccggcg cctggaccct 60

cgcgacagag ctccagtgta ggtaagcgag cctgccctgc acctccgagg ccggtgcggt 120

catcctgtgg ggccgggcct gtagagggac ggttcctggt cacggcttgc cgctaacctc 180

gcgtaggggt aatggttttt ttgtttattt gtttgtttgt ttgcttgttt gttcctgacc 240

ggtttacagc agggagaaaa acgaaatgca gacaagacaa ggagttggag caaagacaaa 300

ctcattctga cggacacggg gtcttttttt gcatttattt actttgcatg tattttgcat 360

ttattttgtg ttcgtgctct ggcgacggcc agaagagagc gtcggaaccc cggggccgga 420

gttacagttg tgagctgccc ttttttttag agtgctagga agagaactcg gcccccctgg 480

aagagcagcc tgggctatta acagctgaac cagctttcta actcctactc tttattattt 540

atttgcagca ctagagatgg aacccaggac tccgtgtatg ttaggcaagg gttctactac 600

agaactatat ccccaaccct ctccccgacc cttttttttt taagacggga actcggtatg 660

tatcggtgtg tagccttggc ttgctgacct gaaacgtgct atgtagacta ggctagccta 720

gagctcacag atctctgcct gtgttgggga tggttgtatg cagtgtgtat tcaggaacta 780

tttctgagat ctggttacaa tccagacact gcctcaatgt gatgt 825

<210> 104
<211> 23
<212> DNA
<213> Artificial Sequence

<220>
<223> oligonucleotide

<400> 104
agccatgggc ccgcgtcgca aac 23

<210> 105
<211> 34
<212> DNA
<213> Artificial Sequence

<220>
<223> oligonucleotide

<400> 105
aattgcatgc acatcacatt gaggcagtgt ctgg 34

<210> 106
<211> 8358
<212> DNA
<213> Artificial Sequence

<220>
<223> synthetic DNA

<400> 106
tatagtgagt cgtattacaa ttcactggcc gtcgttttac aacgtcgtga ctgggaaaac 60

cctggcgtta cccaacttaa tcgccttgca gcacatcccc ctttcgccag ctggcgtaat 120

agcgaagagg cccgcaccga tcgcccttcc caacagttgc gcagcctgaa tggcgaatgg 180

acgcgccctg tagcggcgca ttaagcgcgg cgggtgtggt ggttacgcgc agcgtgaccg 240

ctacacttgc cagcgcccta gcgcccgctc ctttcgcttt cttcccttcc tttctcgcca 300

cgttcgccgg ctttccccgt caagctctaa atcgggggct ccctttaggg ttccgattta 360

gtgctttacg gcacctcgac cccaaaaaac ttgattaggg tgatggttca cgtagtgggc 420

catcgccctg atagacggtt tttcgccctt tgacgttgga gtccacgttc tttaatagtg 480

gactcttgtt ccaaactgga acaacactca accctatctc ggtctattct tttgatttat 540

aagggatttt gccgatttcg gcctattggt taaaaaatga gctgatttaa caaaaattta 600

acgcgaattt taacaaaata ttaacgctta caatttcctg atgcggtatt ttctccttac 660

gcatctgtgc ggtatttcac accgcatcag gtggcacttt tcggggaaat gtgcgcggaa 720

cccctatttg tttatttttc taaatacatt caaatatgta tccgctcatg agacaataac 780

cctgataaat gcttcaataa tattgaaaaa ggaagagtat gagtattcaa catttccgtg 840

tcgcccttat tccctttttt gcggcatttt gccttcctgt ttttgctcac ccagaaacgc 900

tggtgaaagt aaaagatgct gaagatcagt tgggtgcacg agtgggttac atcgaactgg 960

atctcaacag cggtaagatc cttgagagtt ttcgccccga agaacgtttt ccaatgatga 1020

gcacttttaa agttctgcta tgtggcgcgg tattatcccg tattgacgcc gggcaagagc 1080

aactcggtcg ccgcatacac tattctcaga atgacttggt tgagtactca ccagtcacag 1140

aaaagcatct tacggatggc atgacagtaa gagaattatg cagtgctgcc ataaccatga 1200

gtgataacac tgcggccaac ttacttctga caacgatcgg aggaccgaag gagctaaccg 1260

cttttttgca caacatgggg gatcatgtaa ctcgccttga tcgttgggaa ccggagctga 1320

atgaagccat accaaacgac gagcgtgaca ccacgatgcc tgtagcaatg gcaacaacgt 1380

tgcgcaaact attaactggc gaactactta ctctagcttc ccggcaacaa ttaatagact 1440

ggatggaggc ggataaagtt gcaggaccac ttctgcgctc ggcccttccg gctggctggt 1500

ttattgctga taaatctgga gccggtgagc gtgggtctcg cggtatcatt gcagcactgg 1560

ggccagatgg taagccctcc cgtatcgtag ttatctacac gacggggagt caggcaacta 1620

tggatgaacg aaatagacag atcgctgaga taggtgcctc actgattaag cattggtaac 1680

tgtcagacca agtttactca tatatacttt agattgattt aaaacttcat ttttaattta 1740

aaaggatcta ggtgaagatc ctttttgata atctcatgac caaaatccct taacgtgagt 1800

tttcgttcca ctgagcgtca gaccccgtag aaaagatcaa aggatcttct tgagatcctt 1860

tttttctgcg cgtaatctgc tgcttgcaaa caaaaaaacc accgctacca gcggtggttt 1920

gtttgccgga tcaagagcta ccaactcttt ttccgaaggt aactggcttc agcagagcgc 1980

agataccaaa tactgttctt ctagtgtagc cgtagttagg ccaccacttc aagaactctg 2040

tagcaccgcc tacatacctc gctctgctaa tcctgttacc agtggctgct gccagtggcg 2100

ataagtcgtg tcttaccggg ttggactcaa gacgatagtt accggataag gcgcagcggt 2160

cgggctgaac ggggggttcg tgcacacagc ccagcttgga gcgaacgacc tacaccgaac 2220

tgagatacct acagcgtgag ctatgagaaa gcgccacgct tcccgaaggg agaaaggcgg 2280

acaggtatcc ggtaagcggc agggtcggaa caggagagcg cacgagggag cttccagggg 2340

gaaacgcctg gtatctttat agtcctgtcg ggtttcgcca cctctgactt gagcgtcgat 2400

ttttgtgatg ctcgtcaggg gggcggagcc tatggaaaaa cgccagcaac gcggcctttt 2460

tacggttcct ggccttttgc tggccttttg ctcacatgtt ctttcctgcg ttatcccctg 2520

attctgtgga taaccgtatt accgcctttg agtgagctga taccgctcgc cgcagccgaa 2580

cgaccgagcg cagcgagtca gtgagcgagg aagcggaaga gcgcccaata cgcaaaccgc 2640

ctctccccgc gcgttggccg attcattaat gcagctggca cgacaggttt cccgactgga 2700

aagcgggcag tgagcgcaac gcaattaatg tgagttagct cactcattag gcaccccagg 2760

ctttacactt tatgcttccg gctcgtatgt tgtgtggaat tgtgagcgga taacaatttc 2820

acacaggaaa cagctatgac catgattacg ccaagctatt taggtgacac tatagaatac 2880

tcaagctatg catccaacgc gttgggagct ctcccatatg gtcgacctgc aggcatttca 2940

gattcgaacc atgacacatt tccatagcag ctgtgccatt ttacaccacc caccccaatg 3000

ttccaccagg cttctatgaa accactgatt atataagtat tttcctgatt agagtacacg 3060

ttcatgatag atcatttaga aaactcaaaa aagcaaaatg acgaaattct ctttattcta 3120

attacggagc catgacctga attcatatta ctcccgggag aaaaggaaaa gccggttttt 3180

ttctcaggca attctgtcat tgtgtttgat agcatggtcc aataatagtg ctggcgcctg 3240

ccctgcctgg accttgtcct cagattcctc cgtcctggct gtccccacga ccctatcttc 3300

tgaaaaggtc atgcagttaa ccaggcttct gaatcatatt tgctggttgc tttcaggagt 3360

gtcacgggtg ctgtgtggag ctgtcacatt cttgggcatc tgtttccgta tttttaaagc 3420

cacctcaacg tttgatagaa aagcataaaa cttttgagga agtgagctct acaaacccac 3480

cacccacgta agaactgagc cgtcctgccc agagggcatc tgaaaaagag atcaacagga 3540

aaagggtggg gaaatcctgc cttccttccc gtcttcaagt acccattggc ttcagacctt 3600

tattctcatt ggtttatctg gatgtcgctc cacgctggct ttccagaacc agccactgag 3660

agtcacaaag ttgggattta aaagatgcgg tgggaggggc gggtgatttt ttgaactgct 3720

ggcgaccgag ttctggcggt cctgacgcgc accttgggct tgctcgcgtt cggtttcggc 3780

agcaggacca agaaggctta gggaaggact agtgattacc gttcgtatag catacattat 3840

acgaagttat accatgtcta gactggacaa gagcaaagtc ataaacggcg ctctggaatt 3900

actcaatgga gtcggtatcg aaggcctgac gacaaggaaa ctcgctcaaa agctgggagt 3960

tgagcagcct accctgtact ggcacgtgaa gaacaagcgg gccctgctcg atgccctgcc 4020

aatcgagatg ctggacaggc atcataccca cttctgcccc ctggaaggcg agtcatggca 4080

agactttctg cggaacaacg ccaagtcatt ccgctgtgct ctcctctcac atcgcgacgg 4140

ggctaaagtg catctcggca cccgcccaac agagaaacag tacgaaaccc tggaaaatca 4200

gctcgcgttc ctgtgtcagc aaggcttctc cctggagaac gcactgtacg ctctgtccgc 4260

cgtgggccac tttacactgg gctgcgtatt ggaggaacag gagcatcaag tagcaaaaga 4320

ggaaagagag acacctacca ccgattctat gcccccactt ctgagacaag caattgagct 4380

gttcgaccgg cagggagccg aacctgcctt ccttttcggc ctggaactaa tcatatgtgg 4440

cctggagaaa cagctaaagt gcgaaagcgg cgggccggcc gacgcccttg acgattttga 4500

cttagacatg ctcccagccg atgcccttga cgactttgac cttgatatgc tgcctgctga 4560

cgctcttgac gattttgacc ttgacatgct ccccgggtaa ctaagtaagg atcccaaata 4620

ccactgaatt aagaattccg cccctctccc tccccccccc ctaacgttac tggccgaagc 4680

cgcttggaat aaggccggtg tgcgtttgtc tatatgttat tttccaccat attgccgtct 4740

tttggcaatg tgagggcccg gaaacctggc cctgtcttct tgacgagcat tcctaggggt 4800

ctttcccctc tcgccaaagg aatgcaaggt ctgttgaatg tcgtgaagga agcagttcct 4860

ctggaagctt cttgaagaca aacaacgtct gtagcgaccc tttgcaggca gcggaacccc 4920

ccacctggcg acaggtgcct ctgcggccaa aagccacgtg tataagatac acctgcaaag 4980

gcggcacaac cccagtgcca cgttgtgagt tggatagttg tggaaagagt caaatggctc 5040

tcctcaagcg tattcaacaa ggggctgaag gatgcccaga aggtacccca ttgtatggga 5100

tctgatctgg ggcctcggtg cacatgcttt acatgtgttt agtcgaggtt aaaaaacgtc 5160

taggcccccc gaaccacggg gacgtggttt tcctttgaaa aacacgatga taatatggcc 5220

acaaccatga ccgagtacaa gcccacggtg cgcctcgcca cccgcgacga cgtccccggg 5280

gccgtacgca ccctcgccgc cgcgttcgcc gactaccccg ccacgcgcca caccgtcgac 5340

ccggaccgcc acatcgagcg ggtcaccgag ctgcaagaac tcttcctcac gcgcgtcggg 5400

ctcgacatcg gcaaggtgtg ggtcgcggac gacggcgccg cggtggcggt ctggaccacg 5460

ccggagagcg tcgaagcggg ggcggtgttc gccgagatcg gcccgcgcat ggccgagttg 5520

agcggttccc ggctggccgc gcagcaacag atggaaggcc tcctggcgcc gcaccggccc 5580

aaggagcccg cgtggttcct ggccaccgtc ggcgtctcgc ccgaccacca gggcaagggt 5640

ctgggcagcg ccgtcgtgct ccccggagtg gaggcggccg agcgcgccgg ggtgcccgcc 5700

ttcctggaga cctccgcgcc ccgcaacctc cccttctacg agcggctcgg cttcaccgtc 5760

accgccgacg tcgaggtgcc cgaaggaccg cgcacctggt gcatgacccg caagcccggt 5820

gcctgaggat ccagacatga taagatacat tgatgagttt ggacaaacca caactagaat 5880

gcagtgaaaa aaatgcttta tttgtgaaat ttgtgatgct attgctttat ttgtaaccat 5940

tataagctgc aataaacaag ttaacaacaa caattgcatt cattttatgt ttcaggttca 6000

gggggaggtg tgggaggttt tttaaagcaa gtaaaacctc tacaaatgtg gtatggctga 6060

ttatgatcct ctagagtcgc agatccagac atgataagat acattgatga gtttggacaa 6120

accacaacta gaatgcagtg aaaaaaatgc tttatttgtg aaatttgtga tgctattgct 6180

ttatttgtaa ccattataag ctgcaataaa caagttaaca acaacaattg cattcatttt 6240

atgtttcagg ttcaggggga ggtgtgggag gttttttaaa gcaagtaaaa cctctacaaa 6300

tgtggtatgg ctgattatga tcctctagag tcgcagatcc agacatgata agatacattg 6360

atgagtttgg acaaaccaca actagaatgc agtgaaaaaa atgctttatt tgtgaaattt 6420

gtgatgctat tgctttattt gtaaccatta taagctgcaa taaacaagtt aacaacaaca 6480

attgcattca ttttatgttt caggttcagg gggaggtgtg ggaggttttt taaagcaagt 6540

aaaacctcta caaatgtggt atggctgatt atgatcctct agagtcgcag atcctctaga 6600

gtcgcagatc tttttccctc tgccaaaaat tatggggaca tcatgaagcc ccttgagcat 6660

ctgacttctg gctaataaag gaaatttatt ttcattgcaa tagtgtgttg gaattttttg 6720

tgtctctcac tcggaaggac atatgggagg gcaaatcatt taaaacatca gaatgagtat 6780

ttggtttaga gtttggcaac atatgccata tgctggctgc catgaacaaa ggtggctata 6840

aagaggtcat cagtatatga aacagccccc tgctgtccat tccttattcc atagaaaagc 6900

cttgacttga ggttagattt tttttatatt ttgttttgtg ttattttttt ctttaacatc 6960

cctaaaattt tccttacatg ttttactagc cagatttttc ctcctctcct gactactccc 7020

agtcatagct gtccctcttc tcttatgaag atccctcgac ctgcagccca agcttatcga 7080

attcccgcgg tggcggccgc acgtctccct atcagtgata gagaagtcga cacgtctcga 7140

gctccctatc agtgatagag aaggtacgtc tagaacgtct ccctatcagt gatagagaag 7200

tcgacacgtc tcgagctccc tatcagtgat agagaaggta cgtctagaac gtctccctat 7260

cagtgataga gaagtcgaca cgtctcgagc tccctatcag tgatagagaa ggtacgtcta 7320

gaacgtctcc ctatcagtga tagagaagtc gacacgtctc gagctcccta tcagtgatag 7380

agaaggtacc ccctatataa gcagagctcg tttagtgaac cgtcagatcg cctggagacg 7440

ccatccacgc tgttttgacc tccatagaag acaccgggac cgatccagcc tccgcggccg 7500

ccatgggccc gcgtcgcaaa ccgcagaccc caaggaggag accctccagc ccggcgcctg 7560

gaccctcgcg acagagctcc agtgtaggta agcgagcctg ccctgcacct ccgaggccgg 7620

tgcggtcatc ctgtggggcc gggcctgtag agggacggtt cctggtcacg gcttgccgct 7680

aacctcgcgt aggggtaatg gtttttttgt ttatttgttt gtttgtttgc ttgtttgttc 7740

ctgaccggtt tacagcaggg agaaaaacga aatgcagaca agacaaggag ttggagcaaa 7800

gacaaactca ttctgacgga cacggggtct ttttttgcat ttatttactt tgcatgtatt 7860

ttgcatttat tttgtgttcg tgctctggcg acggccagaa gagagcgtcg gaaccccggg 7920

gccggagtta cagttgtgag ctgccctttt ttttagagtg ctaggaagag aactcggccc 7980

ccctggaaga gcagcctggg ctattaacag ctgaaccagc tttctaactc ctactcttta 8040

ttatttattt gcagcactag agatggaacc caggactccg tgtatgttag gcaagggttc 8100

tactacagaa ctatatcccc aaccctctcc ccgacccttt tttttttaag acgggaactc 8160

ggtatgtatc ggtgtgtagc cttggcttgc tgacctgaaa cgtgctatgt agactaggct 8220

agcctagagc tcacagatct ctgcctgtgt tggggatggt tgtatgcagt gtgtattcag 8280

gaactatttc tgagatctgg ttacaatcca gacactgcct caatgtgatg tgcatgcgac 8340

gtcgggccca attcgccc 8358

<210> 107
<211> 24
<212> DNA
<213> Artificial Sequence

<220>
<223> oligonucleotide

<400> 107
caccgtcgca aaccgcagac ccca 24

<210> 108
<211> 24
<212> DNA
<213> Artificial Sequence

<220>
<223> oligonucleotide

<400> 108
aaactggggt ctgcggtttg cgac 24

<210> 109
<211> 24
<212> DNA
<213> Artificial Sequence

<220>
<223> oligonucleotide

<400> 109
caccgtggct ggcctcgagg ggcc 24

<210> 110
<211> 24
<212> DNA
<213> Artificial Sequence

<220>
<223> oligonucleotide

<400>  110
aaacggcccc tcgaggccag ccac                                              24


<210>  111
<211>  8436
<212>  DNA
<213>  Artificial Sequence

<220>
<223>  synthetic DNA

<400>  111
gagggcctat ttcccatgat tccttcatat ttgcatatac gatacaaggc tgttagagag        60

ataattggaa ttaatttgac tgtaaacaca aagatattag tacaaaatac gtgacgtaga       120

aagtaataat ttcttgggta gtttgcagtt ttaaaattat gttttaaaat ggactatcat       180

atgcttaccg taacttgaaa gtatttcgat ttcttggctt tatatatctt gtggaaagga       240

cgaaacaccg tcgcaaaccg cagaccccag ttttagagct agaaatagca agttaaaata       300

aggctagtcc gttatcaact tgaaaaagtg gcaccgagtc ggtgcttttt tgttttagag       360

ctagaaatag caagttaaaa taaggctagt ccgtttttag cgcgtgcgcc aattctgcag       420

acaaatggct ctagaggtac ccgttacata acttacggta aatggcccgc ctggctgacc       480

gcccaacgac ccccgcccat tgacgtcaat agtaacgcca atagggactt tccattgacg       540

tcaatgggtg gagtatttac ggtaaactgc ccacttggca gtacatcaag tgtatcatat       600

gccaagtacg ccccctattg acgtcaatga cggtaaatgg cccgcctggc attgtgccca       660

gtacatgacc ttatgggact ttcctacttg gcagtacatc tacgtattag tcatcgctat       720

taccatggtc gaggtgagcc ccacgttctg cttcactctc cccatctccc ccccctcccc       780

acccccaatt ttgtatttat ttatttttta attattttgt gcagcgatgg gggcgggggg       840

gggggggggg cgcgcgccag gcggggcggg gcggggcgag gggcggggcg gggcgaggcg       900

gagaggtgcg gcggcagcca atcagagcgg cgcgctccga aagtttcctt ttatggcgag       960

gcggcggcgg cggcggccct ataaaaagcg aagcgcgcgg cgggcgggag tcgctgcgac      1020

gctgccttcg ccccgtgccc cgctccgccg ccgcctcgcg ccgcccgccc cggctctgac      1080

tgaccgcgtt actcccacag gtgagcgggc gggacggccc ttctcctccg ggctgtaatt      1140

agctgagcaa gaggtaaggg tttaagggat ggttggttgg tggggtatta atgtttaatt      1200

acctggagca cctgcctgaa atcacttttt ttcaggttgg accggtgcca ccatgtaccc      1260

atacgatgtt ccagattacg cttcgccgaa gaaaaagcgc aaggtcgaag cgtccgacaa      1320

gaagtacagc atcggcctgg ccatcggcac caactctgtg ggctgggccg tgatcaccga      1380

cgagtacaag gtgcccagca agaaattcaa ggtgctgggc aacaccgacc ggcacagcat      1440

caagaagaac ctgatcggag ccctgctgtt cgacagcggc gaaacagccg aggccacccg      1500

gctgaagaga accgccagaa gaagatacac cagacggaag aaccggatct gctatctgca 1560

agagatcttc agcaacgaga tggccaaggt ggacgacagc ttcttccaca gactggaaga 1620

gtccttcctg gtggaagagg ataagaagca cgagcggcac cccatcttcg gcaacatcgt 1680

ggacgaggtg gcctaccacg agaagtaccc caccatctac cacctgagaa agaaactggt 1740

ggacagcacc gacaaggccg acctgcggct gatctatctg gccctggccc acatgatcaa 1800

gttccggggc cacttcctga tcgagggcga cctgaacccc gacaacagcg acgtggacaa 1860

gctgttcatc cagctggtgc agacctacaa ccagctgttc gaggaaaacc ccatcaacgc 1920

cagcggcgtg gacgccaagg ccatcctgtc tgccagactg agcaagagca gacggctgga 1980

aaatctgatc gcccagctgc ccggcgagaa gaagaatggc ctgttcggca acctgattgc 2040

cctgagcctg ggcctgaccc ccaacttcaa gagcaacttc gacctggccg aggatgccaa 2100

actgcagctg agcaaggaca cctacgacga cgacctggac aacctgctgg cccagatcgg 2160

cgaccagtac gccgacctgt ttctggccgc caagaacctg tccgacgcca tcctgctgag 2220

cgacatcctg agagtgaaca ccgagatcac caaggccccc ctgagcgcct ctatgatcaa 2280

gagatacgac gagcaccacc aggacctgac cctgctgaaa gctctcgtgc ggcagcagct 2340

gcctgagaag tacaaagaga ttttcttcga ccagagcaag aacggctacg ccggctacat 2400

tgacggcgga gccagccagg aagagttcta caagttcatc aagcccatcc tggaaaagat 2460

ggacggcacc gaggaactgc tcgtgaagct gaacagagag gacctgctgc ggaagcagcg 2520

gaccttcgac aacggcagca tcccccacca gatccacctg ggagagctgc acgccattct 2580

gcggcggcag gaagattttt acccattcct gaaggacaac cgggaaaaga tcgagaagat 2640

cctgaccttc cgcatcccct actacgtggg ccctctggcc aggggaaaca gcagattcgc 2700

ctggatgacc agaaagagcg aggaaaccat caccccctgg aacttcgagg aagtggtgga 2760

caagggcgct tccgcccaga gcttcatcga gcggatgacc aacttcgata agaacctgcc 2820

caacgagaag gtgctgccca agcacagcct gctgtacgag tacttcaccg tgtataacga 2880

gctgaccaaa gtgaaatacg tgaccgaggg aatgagaaag cccgccttcc tgagcggcga 2940

gcagaaaaag gccatcgtgg acctgctgtt caagaccaac cggaaagtga ccgtgaagca 3000

gctgaaagag gactacttca agaaaatcga gtgcttcgac tccgtggaaa tctccggcgt 3060

ggaagatcgg ttcaacgcct ccctgggcac ataccacgat ctgctgaaaa ttatcaagga 3120

caaggacttc ctggacaatg aggaaaacga ggacattctg gaagatatcg tgctgaccct 3180

gacactgttt gaggacagag agatgatcga ggaacggctg aaaacctatg cccacctgtt 3240

cgacgacaaa gtgatgaagc agctgaagcg gcggagatac accggctggg gcaggctgag 3300

ccggaagctg atcaacggca tccgggacaa gcagtccggc aagacaatcc tggatttcct 3360

gaagtccgac ggcttcgcca acagaaactt catgcagctg atccacgacg acagcctgac 3420

ctttaaagag gacatccaga aagcccaggt gtccggccag ggcgatagcc tgcacgagca 3480

cattgccaat ctggccggca gccccgccat taagaagggc atcctgcaga cagtgaaggt 3540

ggtggacgag ctcgtgaaag tgatgggccg gcacaagccc gagaacatcg tgatcgaaat 3600

ggccagagag aaccagacca cccagaaggg acagaagaac agccgcgaga gaatgaagcg 3660

gatcgaagag ggcatcaaag agctgggcag ccagatcctg aaagaacacc ccgtggaaaa 3720

cacccagctg cagaacgaga agctgtacct gtactacctg cagaatgggc gggatatgta 3780

cgtggaccag gaactggaca tcaaccggct gtccgactac gatgtggacc atatcgtgcc 3840

tcagagcttt ctgaaggacg actccatcga caacaaggtg ctgaccagaa gcgacaagaa 3900

ccggggcaag agcgacaacg tgccctccga agaggtcgtg aagaagatga agaactactg 3960

gcggcagctg ctgaacgcca agctgattac ccagagaaag ttcgacaatc tgaccaaggc 4020

cgagagaggc ggcctgagcg aactggataa ggccggcttc atcaagagac agctggtgga 4080

aacccggcag atcacaaagc acgtggcaca gatcctggac tcccggatga acactaagta 4140

cgacgagaat gacaagctga tccgggaagt gaaagtgatc accctgaagt ccaagctggt 4200

gtccgatttc cggaaggatt tccagtttta caaagtgcgc gagatcaaca actaccacca 4260

cgcccacgac gcctacctga acgccgtcgt gggaaccgcc ctgatcaaaa agtaccctaa 4320

gctggaaagc gagttcgtgt acggcgacta caaggtgtac gacgtgcgga agatgatcgc 4380

caagagcgag caggaaatcg gcaaggctac cgccaagtac ttcttctaca gcaacatcat 4440

gaactttttc aagaccgaga ttaccctggc caacggcgag atccggaagc ggcctctgat 4500

cgagacaaac ggcgaaaccg gggagatcgt gtgggataag ggccgggatt ttgccaccgt 4560

gcggaaagtg ctgagcatgc cccaagtgaa tatcgtgaaa aagaccgagg tgcagacagg 4620

cggcttcagc aaagagtcta tcctgcccaa gaggaacagc gataagctga tcgccagaaa 4680

gaaggactgg gaccctaaga agtacggcgg cttcgacagc cccaccgtgg cctattctgt 4740

gctggtggtg gccaaagtgg aaaagggcaa gtccaagaaa ctgaagagtg tgaaagagct 4800

gctggggatc accatcatgg aaagaagcag cttcgagaag aatcccatcg actttctgga 4860

agccaagggc tacaaagaag tgaaaaagga cctgatcatc aagctgccta agtactccct 4920

gttcgagctg gaaaacggcc ggaagagaat gctggcctct gccggcgaac tgcagaaggg 4980

aaacgaactg gccctgccct ccaaatatgt gaacttcctg tacctggcca gccactatga 5040

gaagctgaag ggctcccccg aggataatga gcagaaacag ctgtttgtgg aacagcacaa 5100

gcactacctg gacgagatca tcgagcagat cagcgagttc tccaagagag tgatcctggc 5160

cgacgctaat ctggacaaag tgctgtccgc ctacaacaag caccgggata agcccatcag 5220

agagcaggcc gagaatatca tccacctgtt taccctgacc aatctgggag cccctgccgc 5280

cttcaagtac tttgacacca ccatcgaccg gaagaggtac accagcacca aagaggtgct 5340

ggacgccacc ctgatccacc agagcatcac cggcctgtac gagacacgga tcgacctgtc 5400

tcagctggga ggcgacagcc ccaagaagaa gagaaaggtg gaggccagct aagaattcct 5460

agagctcgct gatcagcctc gactgtgcct tctagttgcc agccatctgt tgtttgcccc 5520

tcccccgtgc cttccttgac cctggaaggt gccactccca ctgtcctttc ctaataaaat 5580

gaggaaattg catcgcattg tctgagtagg tgtcattcta ttctgggggg tggggtgggg 5640

caggacagca agggggagga ttgggaagag aatagcaggc atgctgggga gcggccgcag 5700

gaacccctag tgatggagtt ggccactccc tctctgcgcg ctcgctcgct cactgaggcc 5760

gggcgaccaa aggtcgcccg acgcccgggc tttgcccggg cggcctcagt gagcgagcga 5820

gcgcgcagct gcctgcaggg gcgcctgatg cggtattttc tccttacgca tctgtgcggt 5880

atttcacacc gcatacgtca aagcaaccat agtacgcgcc ctgtagcggc gcattaagcg 5940

cggcgggtgt ggtggttacg cgcagcgtga ccgctacact tgccagcgcc ctagcgcccg 6000

ctcctttcgc tttcttccct tcctttctcg ccacgttcgc cggctttccc cgtcaagctc 6060

taaatcgggg gctcccttta gggttccgat ttagtgcttt acggcacctc gaccccaaaa 6120

aacttgattt gggtgatggt tcacgtagtg ggccatcgcc ctgatagacg gtttttcgcc 6180

ctttgacgtt ggagtccacg ttctttaata gtggactctt gttccaaact ggaacaacac 6240

tcaaccctat ctcgggctat tcttttgatt tataagggat tttgccgatt tcggcctatt 6300

ggttaaaaaa tgagctgatt taacaaaaat ttaacgcgaa ttttaacaaa atattaacgt 6360

ttacaatttt atggtgcact ctcagtacaa tctgctctga tgccgcatag ttaagccagc 6420

cccgacaccc gccaacaccc gctgacgcgc cctgacgggc ttgtctgctc ccggcatccg 6480

cttacagaca agctgtgacc gtctccggga gctgcatgtg tcagaggttt tcaccgtcat 6540

caccgaaacg cgcgagacga aagggcctcg tgatacgcct atttttatag gttaatgtca 6600

tgataataat ggtttcttag acgtcaggtg gcacttttcg gggaaatgtg cgcggaaccc 6660

ctatttgttt atttttctaa atacattcaa atatgtatcc gctcatgaga caataaccct 6720

gataaatgct tcaataatat tgaaaaagga agagtatgag tattcaacat ttccgtgtcg 6780

cccttattcc cttttttgcg gcattttgcc ttcctgtttt tgctcaccca gaaacgctgg 6840

tgaaagtaaa agatgctgaa gatcagttgg gtgcacgagt gggttacatc gaactggatc 6900

tcaacagcgg taagatcctt gagagttttc gccccgaaga acgttttcca atgatgagca 6960

cttttaaagt tctgctatgt ggcgcggtat tatcccgtat tgacgccggg caagagcaac 7020

tcggtcgccg catacactat tctcagaatg acttggttga gtactcacca gtcacagaaa 7080

agcatcttac ggatggcatg acagtaagag aattatgcag tgctgccata accatgagtg 7140

ataacactgc ggccaactta cttctgacaa cgatcggagg accgaaggag ctaaccgctt 7200

ttttgcacaa catgggggat catgtaactc gccttgatcg ttgggaaccg gagctgaatg 7260

aagccatacc aaacgacgag cgtgacacca cgatgcctgt agcaatggca acaacgttgc 7320

gcaaactatt aactggcgaa ctacttactc tagcttcccg gcaacaatta atagactgga 7380

tggaggcgga taaagttgca ggaccacttc tgcgctcggc ccttccggct ggctggttta 7440

ttgctgataa atctggagcc ggtgagcgtg gaagccgcgg tatcattgca gcactggggc 7500

cagatggtaa gccctcccgt atcgtagtta tctacacgac ggggagtcag gcaactatgg 7560

atgaacgaaa tagacagatc gctgagatag gtgcctcact gattaagcat tggtaactgt 7620

cagaccaagt ttactcatat atactttaga ttgatttaaa acttcatttt taatttaaaa 7680

ggatctaggt gaagatcctt tttgataatc tcatgaccaa aatcccttaa cgtgagtttt 7740

cgttccactg agcgtcagac cccgtagaaa agatcaaagg atcttcttga gatccttttt 7800

ttctgcgcgt aatctgctgc ttgcaaacaa aaaaaccacc gctaccagcg gtggtttgtt 7860

tgccggatca agagctacca actctttttc cgaaggtaac tggcttcagc agagcgcaga 7920

taccaaatac tgtccttcta gtgtagccgt agttaggcca ccacttcaag aactctgtag 7980

caccgcctac atacctcgct ctgctaatcc tgttaccagt ggctgctgcc agtggcgata 8040

agtcgtgtct taccgggttg gactcaagac gatagttacc ggataaggcg cagcggtcgg 8100

gctgaacggg gggttcgtgc acacagccca gcttggagcg aacgacctac accgaactga 8160

gatacctaca gcgtgagcta tgagaaagcg ccacgcttcc cgaagggaga aaggcggaca 8220

ggtatccggt aagcggcagg gtcggaacag gagagcgcac gagggagctt ccagggggaa 8280

acgcctggta tctttatagt cctgtcgggt ttcgccacct ctgacttgag cgtcgatttt 8340

tgtgatgctc gtcagggggg cggagcctat ggaaaaacgc cagcaacgcg gcctttttac 8400

ggttcctggc cttttgctgg ccttttgctc acatgt 8436

<210> 112
<211> 8436
<212> DNA
<213> Artificial Sequence

<220>
<223> synthetic DNA

<400> 112
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg tggctggcct cgaggggccg ttttagagct agaaatagca agttaaaata 300

aggctagtcc gttatcaact tgaaaaagtg gcaccgagtc ggtgcttttt tgttttagag 360

ctagaaatag caagttaaaa taaggctagt ccgtttttag cgcgtgcgcc aattctgcag 420

acaaatggct ctagaggtac ccgttacata acttacggta aatggcccgc ctggctgacc 480

gcccaacgac ccccgcccat tgacgtcaat agtaacgcca atagggactt tccattgacg 540

tcaatgggtg gagtatttac ggtaaactgc ccacttggca gtacatcaag tgtatcatat 600

gccaagtacg ccccctattg acgtcaatga cggtaaatgg cccgcctggc attgtgccca 660

gtacatgacc ttatgggact ttcctacttg gcagtacatc tacgtattag tcatcgctat 720

taccatggtc gaggtgagcc ccacgttctg cttcactctc cccatctccc ccccctcccc 780

acccccaatt ttgtatttat ttatttttta attattttgt gcagcgatgg gggcgggggg 840

gggggggggg cgcgcgccag gcggggcggg gcggggcgag gggcggggcg gggcgaggcg 900

gagaggtgcg gcggcagcca atcagagcgg cgcgctccga aagtttcctt ttatggcgag 960

gcggcggcgg cggcggccct ataaaaagcg aagcgcgcgg cgggcgggag tcgctgcgac 1020

gctgccttcg ccccgtgccc cgctccgccg ccgcctcgcg ccgcccgccc cggctctgac 1080

tgaccgcgtt actcccacag gtgagcgggc gggacggccc ttctcctccg ggctgtaatt 1140

agctgagcaa gaggtaaggg tttaagggat ggttggttgg tggggtatta atgtttaatt 1200

acctggagca cctgcctgaa atcacttttt ttcaggttgg accggtgcca ccatgtaccc 1260

atacgatgtt ccagattacg cttcgccgaa gaaaaagcgc aaggtcgaag cgtccgacaa 1320

gaagtacagc atcggcctgg ccatcggcac caactctgtg ggctgggccg tgatcaccga 1380

cgagtacaag gtgcccagca agaaattcaa ggtgctgggc aacaccgacc ggcacagcat 1440

caagaagaac ctgatcggag ccctgctgtt cgacagcggc gaaacagccg aggccacccg 1500

gctgaagaga accgccagaa gaagatacac cagacggaag aaccggatct gctatctgca 1560

agagatcttc agcaacgaga tggccaaggt ggacgacagc ttcttccaca gactggaaga 1620

gtccttcctg gtggaagagg ataagaagca cgagcggcac cccatcttcg gcaacatcgt 1680

ggacgaggtg gcctaccacg agaagtaccc caccatctac cacctgagaa agaaactggt 1740

ggacagcacc gacaaggccg acctgcggct gatctatctg gccctggccc acatgatcaa 1800

gttccggggc cacttcctga tcgagggcga cctgaacccc gacaacagcg acgtggacaa 1860

gctgttcatc cagctggtgc agacctacaa ccagctgttc gaggaaaacc ccatcaacgc 1920

cagcggcgtg gacgccaagg ccatcctgtc tgccagactg agcaagagca gacggctgga 1980

aaatctgatc gcccagctgc ccggcgagaa gaagaatggc ctgttcggca acctgattgc 2040

cctgagcctg ggcctgaccc ccaacttcaa gagcaacttc gacctggccg aggatgccaa 2100

actgcagctg agcaaggaca cctacgacga cgacctggac aacctgctgg cccagatcgg 2160

cgaccagtac gccgacctgt ttctggccgc caagaacctg tccgacgcca tcctgctgag 2220

cgacatcctg agagtgaaca ccgagatcac caaggccccc ctgagcgcct ctatgatcaa 2280

gagatacgac gagcaccacc aggacctgac cctgctgaaa gctctcgtgc ggcagcagct 2340

gcctgagaag tacaaagaga ttttcttcga ccagagcaag aacggctacg ccggctacat 2400

tgacggcgga gccagccagg aagagttcta caagttcatc aagcccatcc tggaaaagat 2460

ggacggcacc gaggaactgc tcgtgaagct gaacagagag gacctgctgc ggaagcagcg 2520

gaccttcgac aacggcagca tcccccacca gatccacctg ggagagctgc acgccattct 2580

gcggcggcag gaagattttt acccattcct gaaggacaac cgggaaaaga tcgagaagat 2640

cctgaccttc cgcatcccct actacgtggg ccctctggcc aggggaaaca gcagattcgc 2700

ctggatgacc agaaagagcg aggaaaccat caccccctgg aacttcgagg aagtggtgga 2760

caagggcgct tccgcccaga gcttcatcga gcggatgacc aacttcgata agaacctgcc 2820

caacgagaag gtgctgccca agcacagcct gctgtacgag tacttcaccg tgtataacga 2880

gctgaccaaa gtgaaatacg tgaccgaggg aatgagaaag cccgccttcc tgagcggcga 2940

gcagaaaaag gccatcgtgg acctgctgtt caagaccaac cggaaagtga ccgtgaagca 3000

gctgaaagag gactacttca agaaaatcga gtgcttcgac tccgtggaaa tctccggcgt 3060

ggaagatcgg ttcaacgcct ccctgggcac ataccacgat ctgctgaaaa ttatcaagga 3120

caaggacttc ctggacaatg aggaaaacga ggacattctg gaagatatcg tgctgaccct 3180

gacactgttt gaggacagag agatgatcga ggaacggctg aaaacctatg cccacctgtt 3240

cgacgacaaa gtgatgaagc agctgaagcg gcggagatac accggctggg gcaggctgag 3300

ccggaagctg atcaacggca tccgggacaa gcagtccggc aagacaatcc tggatttcct 3360

gaagtccgac ggcttcgcca acagaaactt catgcagctg atccacgacg acagcctgac 3420

ctttaaagag gacatccaga aagcccaggt gtccggccag ggcgatagcc tgcacgagca 3480

cattgccaat ctggccggca gccccgccat taagaagggc atcctgcaga cagtgaaggt 3540

ggtggacgag ctcgtgaaag tgatgggccg gcacaagccc gagaacatcg tgatcgaaat 3600

ggccagagag aaccagacca cccagaaggg acagaagaac agccgcgaga gaatgaagcg 3660

gatcgaagag ggcatcaaag agctgggcag ccagatcctg aaagaacacc ccgtggaaaa 3720

cacccagctg cagaacgaga agctgtacct gtactacctg cagaatgggc gggatatgta 3780

cgtggaccag gaactggaca tcaaccggct gtccgactac gatgtggacc atatcgtgcc 3840

tcagagcttt ctgaaggacg actccatcga caacaaggtg ctgaccagaa gcgacaagaa 3900

ccggggcaag agcgacaacg tgccctccga agaggtcgtg aagaagatga agaactactg 3960

gcggcagctg ctgaacgcca agctgattac ccagagaaag ttcgacaatc tgaccaaggc 4020

cgagagaggc ggcctgagcg aactggataa ggccggcttc atcaagagac agctggtgga 4080

aacccggcag atcacaaagc acgtggcaca gatcctggac tcccggatga acactaagta 4140

cgacgagaat gacaagctga tccgggaagt gaaagtgatc accctgaagt ccaagctggt 4200

gtccgatttc cggaaggatt tccagtttta caaagtgcgc gagatcaaca actaccacca 4260

cgcccacgac gcctacctga acgccgtcgt gggaaccgcc ctgatcaaaa agtaccctaa 4320

gctggaaagc gagttcgtgt acggcgacta caaggtgtac gacgtgcgga agatgatcgc 4380

caagagcgag caggaaatcg gcaaggctac cgccaagtac ttcttctaca gcaacatcat 4440

gaactttttc aagaccgaga ttaccctggc caacggcgag atccggaagc ggcctctgat 4500

cgagacaaac ggcgaaaccg gggagatcgt gtgggataag ggccgggatt ttgccaccgt 4560

gcggaaagtg ctgagcatgc cccaagtgaa tatcgtgaaa aagaccgagg tgcagacagg 4620

cggcttcagc aaagagtcta tcctgcccaa gaggaacagc gataagctga tcgccagaaa 4680

gaaggactgg gaccctaaga agtacggcgg cttcgacagc cccaccgtgg cctattctgt 4740

gctggtggtg gccaaagtgg aaaagggcaa gtccaagaaa ctgaagagtg tgaaagagct 4800

gctggggatc accatcatgg aaagaagcag cttcgagaag aatcccatcg actttctgga 4860

agccaagggc tacaaagaag tgaaaaagga cctgatcatc aagctgccta agtactccct 4920

gttcgagctg gaaaacggcc ggaagagaat gctggcctct gccggcgaac tgcagaaggg 4980

aaacgaactg gccctgccct ccaaatatgt gaacttcctg tacctggcca gccactatga 5040

gaagctgaag ggctcccccg aggataatga gcagaaacag ctgtttgtgg aacagcacaa 5100

gcactacctg gacgagatca tcgagcagat cagcgagttc tccaagagag tgatcctggc 5160

cgacgctaat ctggacaaag tgctgtccgc ctacaacaag caccgggata agcccatcag 5220

agagcaggcc gagaatatca tccacctgtt taccctgacc aatctgggag cccctgccgc 5280

cttcaagtac tttgacacca ccatcgaccg gaagaggtac accagcacca aagaggtgct 5340

ggacgccacc ctgatccacc agagcatcac cggcctgtac gagacacgga tcgacctgtc 5400

tcagctggga ggcgacagcc ccaagaagaa gagaaaggtg gaggccagct aagaattcct 5460

agagctcgct gatcagcctc gactgtgcct tctagttgcc agccatctgt tgtttgcccc 5520

tcccccgtgc cttccttgac cctggaaggt gccactccca ctgtcctttc ctaataaaat 5580

gaggaaattg catcgcattg tctgagtagg tgtcattcta ttctgggggg tggggtgggg 5640

caggacagca agggggagga ttgggaagag aatagcaggc atgctgggga gcggccgcag 5700

gaacccctag tgatggagtt ggccactccc tctctgcgcg ctcgctcgct cactgaggcc 5760

gggcgaccaa aggtcgcccg acgcccgggc tttgcccggg cggcctcagt gagcgagcga 5820

gcgcgcagct gcctgcaggg gcgcctgatg cggtattttc tccttacgca tctgtgcggt 5880

atttcacacc gcatacgtca aagcaaccat agtacgcgcc ctgtagcggc gcattaagcg 5940

cggcgggtgt ggtggttacg cgcagcgtga ccgctacact tgccagcgcc ctagcgcccg 6000

ctcctttcgc tttcttccct tcctttctcg ccacgttcgc cggctttccc cgtcaagctc 6060

taaatcgggg gctcccttta gggttccgat ttagtgcttt acggcacctc gaccccaaaa 6120

aacttgattt gggtgatggt tcacgtagtg ggccatcgcc ctgatagacg gtttttcgcc 6180

ctttgacgtt ggagtccacg ttctttaata gtggactctt gttccaaact ggaacaacac 6240

tcaaccctat ctcgggctat tcttttgatt tataagggat tttgccgatt tcggcctatt 6300

ggttaaaaaa tgagctgatt taacaaaaat ttaacgcgaa ttttaacaaa atattaacgt 6360

ttacaatttt atggtgcact ctcagtacaa tctgctctga tgccgcatag ttaagccagc 6420

cccgacaccc gccaacaccc gctgacgcgc cctgacgggc ttgtctgctc ccggcatccg 6480

cttacagaca agctgtgacc gtctccggga gctgcatgtg tcagaggttt tcaccgtcat 6540

caccgaaacg cgcgagacga aagggcctcg tgatacgcct atttttatag gttaatgtca 6600

tgataataat ggtttcttag acgtcaggtg gcacttttcg gggaaatgtg cgcggaaccc 6660

ctatttgttt atttttctaa atacattcaa atatgtatcc gctcatgaga caataaccct 6720

gataaatgct tcaataatat tgaaaaagga agagtatgag tattcaacat ttccgtgtcg 6780

cccttattcc cttttttgcg gcattttgcc ttcctgtttt tgctcaccca gaaacgctgg 6840

tgaaagtaaa agatgctgaa gatcagttgg gtgcacgagt gggttacatc gaactggatc 6900

tcaacagcgg taagatcctt gagagttttc gccccgaaga acgttttcca atgatgagca 6960

cttttaaagt tctgctatgt ggcgcggtat tatcccgtat tgacgccggg caagagcaac 7020

tcggtcgccg catacactat tctcagaatg acttggttga gtactcacca gtcacagaaa 7080

agcatcttac ggatggcatg acagtaagag aattatgcag tgctgccata accatgagtg 7140

ataacactgc ggccaactta cttctgacaa cgatcggagg accgaaggag ctaaccgctt 7200

ttttgcacaa catgggggat catgtaactc gccttgatcg ttgggaaccg gagctgaatg 7260

aagccatacc aaacgacgag cgtgacacca cgatgcctgt agcaatggca acaacgttgc 7320

gcaaactatt aactggcgaa ctacttactc tagcttcccg gcaacaatta atagactgga 7380

tggaggcgga taaagttgca ggaccacttc tgcgctcggc ccttccggct ggctggttta 7440

ttgctgataa atctggagcc ggtgagcgtg gaagccgcgg tatcattgca gcactggggc 7500

cagatggtaa gccctcccgt atcgtagtta tctacacgac ggggagtcag gcaactatgg 7560

atgaacgaaa tagacagatc gctgagatag gtgcctcact gattaagcat tggtaactgt 7620

cagaccaagt ttactcatat atactttaga ttgatttaaa acttcatttt taatttaaaa 7680

ggatctaggt gaagatcctt tttgataatc tcatgaccaa aatcccttaa cgtgagtttt 7740

cgttccactg agcgtcagac cccgtagaaa agatcaaagg atcttcttga gatccttttt 7800

ttctgcgcgt aatctgctgc ttgcaaacaa aaaaaccacc gctaccagcg gtggtttgtt 7860

tgccggatca agagctacca actctttttc cgaaggtaac tggcttcagc agagcgcaga 7920

taccaaatac tgtccttcta gtgtagccgt agttaggcca ccacttcaag aactctgtag 7980

caccgcctac atacctcgct ctgctaatcc tgttaccagt ggctgctgcc agtggcgata 8040

agtcgtgtct taccgggttg gactcaagac gatagttacc ggataaggcg cagcggtcgg 8100

gctgaacggg gggttcgtgc acacagccca gcttggagcg aacgacctac accgaactga 8160

gatacctaca gcgtgagcta tgagaaagcg ccacgcttcc cgaagggaga aaggcggaca 8220

ggtatccggt aagcggcagg gtcggaacag gagagcgcac gagggagctt ccagggggaa 8280

acgcctggta tctttatagt cctgtcgggt ttcgccacct ctgacttgag cgtcgatttt 8340

tgtgatgctc gtcagggggg cggagcctat ggaaaaacgc cagcaacgcg gcctttttac 8400

ggttcctggc cttttgctgg ccttttgctc acatgt 8436

<210> 113
<211> 29
<212> DNA
<213> Artificial Sequence

<220>
<223> oligonucleotide

<400> 113
tctggaactc actatgtagg tctactggc 29

<210> 114
<211> 22
<212> DNA
<213> Artificial Sequence

<220>
<223> oligonucleotide

<400> 114
ttgacctcca tagaagacac cg 22

<210> 115
<211> 30
<212> DNA
<213> Artificial Sequence

<220>
<223> oligonucleotide

<400> 115
ctcttcttct cacctggtct cttcaggacc 30

<210> 116
<211> 24
<212> DNA
<213> Artificial Sequence

<220>
<223>  oligonucleotide

<400>  116
acctcctctc cttacatgct ggtc                                              24


<210>  117
<211>  23
<212>  DNA
<213>  Artificial Sequence

<220>
<223>  oligonucleotide

<400>  117
tacattccac tggcagctta ggg                                               23


<210>  118
<211>  617
<212>  DNA
<213>  Artificial Sequence

<220>
<223>  synthetic DNA

<400>  118
ggagctccgg cgctgcccca gatctggcag ctgtacctca agaactaccg catcgccacc      60

ttcaagaact ggcccttcct ggaggactgc gcctgcaccc cagagcgagt gagtcccagc     120

ttccagcgac tccaccctgg gcccgacggt gccttagttg tgtgacccgg gatcagaacc     180

cgctatcccc accctcgctt cgtactgtta gccaggctgc tttgctctgc tctgtcctcc     240

acgaggccac cccccccccc cgctctcccc accctctgcc tgaaccactg ttagcctccg     300

ggcctcttcc cactgtcctc agttctcggc ccggaacgat ctctgggaat catgggataa     360

ggtcagactg ttgatcccgc tcacgagctg tcattttcag atggcggagg ctggcttcat     420

ccactgccct accgagaacg agcctgattt ggcccagtgt tttttctgct ttaaggaatt     480

ggaaggctgg gaacccgatg acaacccgat gtaagtccca caggctactc tcggtgggtt     540

tcctggtgtt ccccttccat tgagagcctt ttgggttgaa ctggagttgg gacaaaccat     600

ttgtatttag tagaaga                                                     617


<210>  119
<211>  31
<212>  DNA
<213>  Artificial Sequence

<220>
<223>  oligonucleotide

<400>  119
caccatggga gctccggcgc tgccccagat c                                      31

<210>  120
<211>  34
<212>  DNA
<213>  Artificial Sequence

<220>
<223>  oligonucleotide

<400>  120
taactggaac acttgagcaa gaagcatgct cttc                                    34


<210>  121
<211>  775
<212>  DNA
<213>  Artificial Sequence

<220>
<223>  synthetic DNA

<400>  121
cagtgtgaag ctctttagaa gccactgcaa ccacaggccg cccgacagga acagagacac        60

tgaaaacggg cccgcagcaa ggcaggctca gcagccaaca gtcacaccca ggaagcagta       120

tttttcttct gctcctggac tctcttgcgg tgtatggctg cttccctttg gtctgagcca       180

ggccgatggt ctcagaaata gacacccatt gactttcttt tccagcgctg ggacatacag       240

accccgcctc catcccaggg tgtctatagg aaggatggcg gctgctgcag ggaggagggt       300

ctcctgtctt cctaagggcg cccctccacc agcctgtggg tgggtccgag gcacttccat       360

tccgatatct agctggccaa atcctgcaaa ccttgaggca ggaagaacct gcagagcaca       420

tgggacttgc agcggacatg ctttaaagag gtgccccagg cccgtccacc gccctcggcc       480

accctccgtg tcctctgggg agcagctgcg gaagattcga gtcagaatag caagaaggaa       540

ccgcagcaga aggtacaact cccagcatgc cctgcgcccg ccacgcccac aaggccaggc       600

gcagatgggc gtggggcggg actttcccgg ctcgcctcgc gccgtccact cccagaaggc       660

agcgggcgag ggcgtggggc cggggctctc ccggcatgct ctgcggcgcg cctccgcccg       720

cgcgatttga atcctgcgtt tgagtcgtct tggcggaggt tgtggtgacg ccatc            775


<210>  122
<211>  39
<212>  DNA
<213>  Artificial Sequence

<220>
<223>  oligonucleotide

<400>  122
aattcctgca ggcagtgtga agctctttag aagccactg                               39


<210>  123
<211>  35
<212>  DNA

<213>  Artificial Sequence

<220>
<223>  oligonucleotide

<400>  123
ttactagtga tggcgtcacc acaacctccg ccaag                                35


<210>  124
<211>  8050
<212>  DNA
<213>  Artificial Sequence

<220>
<223>  synthetic DNA

<400>  124
tatagtgagt cgtattacaa ttcactggcc gtcgttttac aacgtcgtga ctgggaaaac      60

cctggcgtta cccaacttaa tcgccttgca gcacatcccc ctttcgccag ctggcgtaat     120

agcgaagagg cccgcaccga tcgcccttcc caacagttgc gcagcctgaa tggcgaatgg     180

acgcgccctg tagcggcgca ttaagcgcgg cgggtgtggt ggttacgcgc agcgtgaccg     240

ctacacttgc cagcgcccta gcgcccgctc ctttcgcttt cttcccttcc tttctcgcca     300

cgttcgccgg ctttccccgt caagctctaa atcgggggct ccctttaggg ttccgattta     360

gtgctttacg gcacctcgac cccaaaaaac ttgattaggg tgatggttca cgtagtgggc     420

catcgccctg atagacggtt tttcgccctt tgacgttgga gtccacgttc tttaatagtg     480

gactcttgtt ccaaactgga acaacactca accctatctc ggtctattct tttgatttat     540

aagggatttt gccgatttcg gcctattggt taaaaaatga gctgatttaa caaaaattta     600

acgcgaattt taacaaaata ttaacgctta caatttcctg atgcggtatt ttctccttac     660

gcatctgtgc ggtatttcac accgcatcag gtggcacttt tcggggaaat gtgcgcggaa     720

cccctatttg tttatttttc taaatacatt caaatatgta tccgctcatg agacaataac     780

cctgataaat gcttcaataa tattgaaaaa ggaagagtat gagtattcaa catttccgtg     840

tcgcccttat tccctttttt gcggcatttt gccttcctgt ttttgctcac ccagaaacgc     900

tggtgaaagt aaaagatgct gaagatcagt tgggtgcacg agtgggttac atcgaactgg     960

atctcaacag cggtaagatc cttgagagtt ttcgccccga agaacgtttt ccaatgatga    1020

gcacttttaa agttctgcta tgtggcgcgg tattatcccg tattgacgcc gggcaagagc    1080

aactcggtcg ccgcatacac tattctcaga atgacttggt tgagtactca ccagtcacag    1140

aaaagcatct tacggatggc atgacagtaa gagaattatg cagtgctgcc ataaccatga    1200

gtgataacac tgcggccaac ttacttctga caacgatcgg aggaccgaag gagctaaccg    1260

cttttttgca caacatgggg gatcatgtaa ctcgccttga tcgttgggaa ccggagctga    1320

atgaagccat accaaacgac gagcgtgaca ccacgatgcc tgtagcaatg gcaacaacgt    1380

tgcgcaaact attaactggc gaactactta ctctagcttc ccggcaacaa ttaatagact 1440

ggatggaggc ggataaagtt gcaggaccac ttctgcgctc ggcccttccg gctggctggt 1500

ttattgctga taaatctgga gccggtgagc gtgggtctcg cggtatcatt gcagcactgg 1560

ggccagatgg taagccctcc cgtatcgtag ttatctacac gacggggagt caggcaacta 1620

tggatgaacg aaatagacag atcgctgaga taggtgcctc actgattaag cattggtaac 1680

tgtcagacca agtttactca tatatacttt agattgattt aaaacttcat ttttaattta 1740

aaaggatcta ggtgaagatc ctttttgata atctcatgac caaaatccct taacgtgagt 1800

tttcgttcca ctgagcgtca gaccccgtag aaaagatcaa aggatcttct tgagatcctt 1860

tttttctgcg cgtaatctgc tgcttgcaaa caaaaaaacc accgctacca gcggtggttt 1920

gtttgccgga tcaagagcta ccaactcttt ttccgaaggt aactggcttc agcagagcgc 1980

agataccaaa tactgttctt ctagtgtagc cgtagttagg ccaccacttc aagaactctg 2040

tagcaccgcc tacatacctc gctctgctaa tcctgttacc agtggctgct gccagtggcg 2100

ataagtcgtg tcttaccggg ttggactcaa gacgatagtt accggataag gcgcagcggt 2160

cgggctgaac ggggggttcg tgcacacagc ccagcttgga gcgaacgacc tacaccgaac 2220

tgagatacct acagcgtgag ctatgagaaa gcgccacgct tcccgaaggg agaaaggcgg 2280

acaggtatcc ggtaagcggc agggtcggaa caggagagcg cacgagggag cttccagggg 2340

gaaacgcctg gtatctttat agtcctgtcg ggtttcgcca cctctgactt gagcgtcgat 2400

ttttgtgatg ctcgtcaggg gggcggagcc tatggaaaaa cgccagcaac gcggcctttt 2460

tacggttcct ggccttttgc tggccttttg ctcacatgtt ctttcctgcg ttatcccctg 2520

attctgtgga taaccgtatt accgcctttg agtgagctga taccgctcgc cgcagccgaa 2580

cgaccgagcg cagcgagtca gtgagcgagg aagcggaaga gcgcccaata cgcaaaccgc 2640

ctctccccgc gcgttggccg attcattaat gcagctggca cgacaggttt cccgactgga 2700

aagcgggcag tgagcgcaac gcaattaatg tgagttagct cactcattag gcaccccagg 2760

ctttacactt tatgcttccg gctcgtatgt tgtgtggaat tgtgagcgga taacaatttc 2820

acacaggaaa cagctatgac catgattacg ccaagctatt taggtgacac tatagaatac 2880

tcaagctatg catccaacgc gttgggagct ctcccatatg gtcgacctgc aggcagtgtg 2940

aagctcttta gaagccactg caaccacagg ccgcccgaca ggaacagaga cactgaaaac 3000

gggcccgcag caaggcaggc tcagcagcca acagtcacac ccaggaagca gtatttttct 3060

tctgctcctg gactctcttg cggtgtatgg ctgcttccct ttggtctgag ccaggccgat 3120

ggtctcagaa atagacaccc attgactttc ttttccagcg ctgggacata cagaccccgc 3180

ctccatccca gggtgtctat aggaaggatg gcggctgctg cagggaggag ggtctcctgt 3240

cttcctaagg gcgcccctcc accagcctgt gggtgggtcc gaggcacttc cattccgata 3300

tctagctggc caaatcctgc aaaccttgag gcaggaagaa cctgcagagc acatgggact 3360

tgcagcggac atgctttaaa gaggtgcccc aggcccgtcc accgccctcg gccaccctcc 3420

gtgtcctctg gggagcagct gcggaagatt cgagtcagaa tagcaagaag gaaccgcagc 3480

agaaggtaca actcccagca tgccctgcgc ccgccacgcc cacaaggcca ggcgcagatg 3540

ggcgtggggc gggactttcc cggctcgcct cgcgccgtcc actcccagaa ggcagcgggc 3600

gagggcgtgg ggccggggct ctcccggcat gctctgcggc gcgcctccgc ccgcgcgatt 3660

tgaatcctgc gtttgagtcg tcttggcgga ggttgtggtg acgccatcac tagtgattac 3720

cgttcgtata gcatacatta tacgaagtta taccatgtct agactggaca agagcaaagt 3780

cataaacggc gctctggaat tactcaatgg agtcggtatc gaaggcctga cgacaaggaa 3840

actcgctcaa aagctgggag ttgagcagcc taccctgtac tggcacgtga agaacaagcg 3900

ggccctgctc gatgccctgc caatcgagat gctggacagg catcataccc acttctgccc 3960

cctggaaggc gagtcatggc aagactttct gcggaacaac gccaagtcat tccgctgtgc 4020

tctcctctca catcgcgacg gggctaaagt gcatctcggc acccgcccaa cagagaaaca 4080

gtacgaaacc ctggaaaatc agctcgcgtt cctgtgtcag caaggcttct ccctggagaa 4140

cgcactgtac gctctgtccg ccgtgggcca ctttacactg ggctgcgtat tggaggaaca 4200

ggagcatcaa gtagcaaaag aggaaagaga gacacctacc accgattcta tgcccccact 4260

tctgagacaa gcaattgagc tgttcgaccg gcagggagcc gaacctgcct tccttttcgg 4320

cctggaacta atcatatgtg gcctggagaa acagctaaag tgcgaaagcg gcgggccggc 4380

cgacgccctt gacgattttg acttagacat gctcccagcc gatgcccttg acgactttga 4440

ccttgatatg ctgcctgctg acgctcttga cgattttgac cttgacatgc tccccgggta 4500

actaagtaag gatcccaaat accactgaat taagaattcc gcccctctcc ctcccccccc 4560

cctaacgtta ctggccgaag ccgcttggaa taaggccggt gtgcgtttgt ctatatgtta 4620

ttttccacca tattgccgtc ttttggcaat gtgagggccc ggaaacctgg ccctgtcttc 4680

ttgacgagca ttcctagggg tctttcccct ctcgccaaag gaatgcaagg tctgttgaat 4740

gtcgtgaagg aagcagttcc tctggaagct tcttgaagac aaacaacgtc tgtagcgacc 4800

ctttgcaggc agcggaaccc cccacctggc gacaggtgcc tctgcggcca aaagccacgt 4860

gtataagata cacctgcaaa ggcggcacaa ccccagtgcc acgttgtgag ttggatagtt 4920

gtggaaagag tcaaatggct ctcctcaagc gtattcaaca aggggctgaa ggatgcccag 4980

aaggtacccc attgtatggg atctgatctg gggcctcggt gcacatgctt tacatgtgtt 5040

tagtcgaggt taaaaaacgt ctaggccccc cgaaccacgg ggacgtggtt ttcctttgaa 5100

aaacacgatg ataatatggc cacaaccatg accgagtaca agcccacggt gcgcctcgcc 5160

acccgcgacg acgtcccccg ggccgtacgc accctcgccg ccgcgttcgc cgactacccc 5220

gccacgcgcc acaccgtcga cccggaccgc cacatcgagc gggtcaccga gctgcaagaa 5280

ctcttcctca cgcgcgtcgg gctcgacatc ggcaaggtgt gggtcgcgga cgacggcgcc 5340

gcggtggcgg tctggaccac gccggagagc gtcgaagcgg gggcggtgtt cgccgagatc 5400

ggcccgcgca tggccgagtt gagcggttcc cggctggccg cgcagcaaca gatggaaggc 5460

ctcctggcgc cgcaccggcc caaggagccc gcgtggttcc tggccaccgt cggcgtctcg 5520

cccgaccacc agggcaaggg tctgggcagc gccgtcgtgc tccccggagt ggaggcggcc 5580

gagcgcgccg gggtgcccgc cttcctggag acctccgcgc cccgcaacct ccccttctac 5640

gagcggctcg gcttcaccgt caccgccgac gtcgaggtgc ccgaaggacc gcgcacctgg 5700

tgcatgaccc gcaagcccgg tgcctgagga tccagacatg ataagataca ttgatgagtt 5760

tggacaaacc acaactagaa tgcagtgaaa aaaatgcttt atttgtgaaa tttgtgatgc 5820

tattgcttta tttgtaacca ttataagctg caataaacaa gttaacaaca acaattgcat 5880

tcattttatg tttcaggttc agggggaggt gtgggaggtt ttttaaagca agtaaaacct 5940

ctacaaatgt ggtatggctg attatgatcc tctagagtcg cagatccaga catgataaga 6000

tacattgatg agtttggaca aaccacaact agaatgcagt gaaaaaaatg ctttatttgt 6060

gaaatttgtg atgctattgc tttatttgta accattataa gctgcaataa acaagttaac 6120

aacaacaatt gcattcattt tatgtttcag gttcaggggg aggtgtggga ggttttttaa 6180

agcaagtaaa acctctacaa atgtggtatg gctgattatg atcctctaga gtcgcagatc 6240

cagacatgat aagatacatt gatgagtttg gacaaaccac aactagaatg cagtgaaaaa 6300

aatgctttat ttgtgaaatt tgtgatgcta ttgctttatt tgtaaccatt ataagctgca 6360

ataaacaagt taacaacaac aattgcattc attttatgtt tcaggttcag ggggaggtgt 6420

gggaggtttt ttaaagcaag taaaacctct acaaatgtgg tatggctgat tatgatcctc 6480

tagagtcgca gatcctctag agtcgcagat ctttttccct ctgccaaaaa ttatggggac 6540

atcatgaagc cccttgagca tctgacttct ggctaataaa ggaaatttat tttcattgca 6600

atagtgtgtt ggaatttttt gtgtctctca ctcggaagga catatgggag ggcaaatcat 6660

ttaaaacatc agaatgagta tttggtttag agtttggcaa catatgccat atgctggctg 6720

ccatgaacaa aggtggctat aaagaggtca tcagtatatg aaacagcccc ctgctgtcca 6780

ttccttattc catagaaaag ccttgacttg aggttagatt ttttttatat tttgttttgt 6840

gttatttttt tctttaacat ccctaaaatt ttccttacat gttttactag ccagattttt 6900

cctcctctcc tgactactcc cagtcatagc tgtccctctt ctcttatgaa gatccctcga 6960

cctgcagccc aagcttatcg aattcccgcg gtggcggccg cacgtctccc tatcagtgat 7020

agagaagtcg acacgtctcg agctccctat cagtgataga gaaggtacgt ctagaacgtc 7080

tccctatcag tgatagagaa gtcgacacgt ctcgagctcc ctatcagtga tagagaaggt 7140

acgtctagaa cgtctcccta tcagtgatag agaagtcgac acgtctcgag ctccctatca 7200

gtgatagaga aggtacgtct agaacgtctc cctatcagtg atagagaagt cgacacgtct 7260

cgagctccct atcagtgata gagaaggtac cccctatata agcagagctc gtttagtgaa 7320

ccgtcagatc gcctggagac gccatccacg ctgttttgac ctccatagaa gacaccggga 7380

ccgatccagc ctccgcggcc gccatgggag ctccggcgct gccccagatc tggcagctgt 7440

acctcaagaa ctaccgcatc gccaccttca agaactggcc cttcctggag gactgcgcct 7500

gcaccccaga gcgagtgagt cccagcttcc agcgactcca ccctgggccc gacggtgcct 7560

tagttgtgtg acccgggatc agaacccgct atccccaccc tcgcttcgta ctgttagcca 7620

ggctgctttg ctctgctctg tcctccacga ggccaccccc cccccccgct ctccccaccc 7680

tctgcctgaa ccactgttag cctccgggcc tcttcccact gtcctcagtt ctcggcccgg 7740

aacgatctct gggaatcatg ggataaggtc agactgttga tcccgctcac gagctgtcat 7800

tttcagatgg cggaggctgg cttcatccac tgccctaccg agaacgagcc tgatttggcc 7860

cagtgttttt tctgctttaa ggaattggaa ggctgggaac ccgatgacaa cccgatgtaa 7920

gtcccacagg ctactctcgg tgggtttcct ggtgttcccc ttccattgag agccttttgg 7980

gttgaactgg agttgggaca aaccatttgt atttagtaga agagcatgcg acgtcgggcc 8040

caattcgccc 8050

<210> 125
<211> 24
<212> DNA
<213> Artificial Sequence

<220>
<223> oligonucleotide

<400> 125
caccggacgc catcatggga gctc 24

<210> 126
<211> 24
<212> DNA
<213> Artificial Sequence

<220>
<223> oligonucleotide

<400> 126
aaacgagctc ccatgatggc gtcc 24

<210> 127
<211> 24

<212>  DNA
<213>  Artificial Sequence

<220>
<223>  oligonucleotide

<400>  127
caccggccaa gacgactcaa acgc                                              24


<210>  128
<211>  24
<212>  DNA
<213>  Artificial Sequence

<220>
<223>  oligonucleotide

<400>  128
aaacgcgttt gagtcgtctt ggcc                                              24


<210>  129
<211>  8436
<212>  DNA
<213>  Artificial Sequence

<220>
<223>  synthetic DNA

<400>  129
gagggcctat ttcccatgat tccttcatat ttgcatatac gatacaaggc tgttagagag      60

ataattggaa ttaatttgac tgtaaacaca aagatattag tacaaaatac gtgacgtaga     120

aagtaataat ttcttgggta gtttgcagtt ttaaaattat gttttaaaat ggactatcat     180

atgcttaccg taacttgaaa gtatttcgat ttcttggctt tatatatctt gtggaaagga     240

cgaaacaccg gacgccatca tgggagctcg ttttagagct agaaatagca agttaaaata     300

aggctagtcc gttatcaact tgaaaaagtg gcaccgagtc ggtgcttttt tgttttagag     360

ctagaaatag caagttaaaa taaggctagt ccgtttttag cgcgtgcgcc aattctgcag     420

acaaatggct ctagaggtac ccgttacata acttacggta aatggcccgc ctggctgacc     480

gcccaacgac ccccgcccat tgacgtcaat agtaacgcca atagggactt tccattgacg     540

tcaatgggtg gagtatttac ggtaaactgc ccacttggca gtacatcaag tgtatcatat     600

gccaagtacg ccccctattg acgtcaatga cggtaaatgg cccgcctggc attgtgccca     660

gtacatgacc ttatgggact ttcctacttg gcagtacatc tacgtattag tcatcgctat     720

taccatggtc gaggtgagcc ccacgttctg cttcactctc cccatctccc ccccctcccc     780

acccccaatt ttgtatttat ttatttttta attattttgt gcagcgatgg gggcgggggg     840

gggggggggg cgcgcgccag gcggggcggg gcggggcgag gggcggggcg gggcgaggcg     900

gagaggtgcg gcggcagcca atcagagcgg cgcgctccga aagtttcctt ttatggcgag     960

gcggcggcgg cggcggccct ataaaaagcg aagcgcgcgg cgggcgggag tcgctgcgac 1020

gctgccttcg ccccgtgccc cgctccgccg ccgcctcgcg ccgcccgccc cggctctgac 1080

tgaccgcgtt actcccacag gtgagcgggc gggacggccc ttctcctccg ggctgtaatt 1140

agctgagcaa gaggtaaggg tttaagggat ggttggttgg tggggtatta atgtttaatt 1200

acctggagca cctgcctgaa atcacttttt ttcaggttgg accggtgcca ccatgtaccc 1260

atacgatgtt ccagattacg cttcgccgaa gaaaaagcgc aaggtcgaag cgtccgacaa 1320

gaagtacagc atcggcctgg ccatcggcac caactctgtg ggctgggccg tgatcaccga 1380

cgagtacaag gtgcccagca agaaattcaa ggtgctgggc aacaccgacc ggcacagcat 1440

caagaagaac ctgatcggag ccctgctgtt cgacagcggc gaaacagccg aggccacccg 1500

gctgaagaga accgccagaa gaagatacac cagacggaag aaccggatct gctatctgca 1560

agagatcttc agcaacgaga tggccaaggt ggacgacagc ttcttccaca gactggaaga 1620

gtccttcctg gtggaagagg ataagaagca cgagcggcac cccatcttcg gcaacatcgt 1680

ggacgaggtg gcctaccacg agaagtaccc caccatctac cacctgagaa agaaactggt 1740

ggacagcacc gacaaggccg acctgcggct gatctatctg gccctggccc acatgatcaa 1800

gttccggggc cacttcctga tcgagggcga cctgaacccc gacaacagcg acgtggacaa 1860

gctgttcatc cagctggtgc agacctacaa ccagctgttc gaggaaaacc ccatcaacgc 1920

cagcggcgtg gacgccaagg ccatcctgtc tgccagactg agcaagagca gacggctgga 1980

aaatctgatc gcccagctgc ccggcgagaa gaagaatggc ctgttcggca acctgattgc 2040

cctgagcctg ggcctgaccc ccaacttcaa gagcaacttc gacctggccg aggatgccaa 2100

actgcagctg agcaaggaca cctacgacga cgacctggac aacctgctgg cccagatcgg 2160

cgaccagtac gccgacctgt ttctggccgc caagaacctg tccgacgcca tcctgctgag 2220

cgacatcctg agagtgaaca ccgagatcac caaggccccc ctgagcgcct ctatgatcaa 2280

gagatacgac gagcaccacc aggacctgac cctgctgaaa gctctcgtgc ggcagcagct 2340

gcctgagaag tacaaagaga ttttcttcga ccagagcaag aacggctacg ccggctacat 2400

tgacggcgga gccagccagg aagagttcta caagttcatc aagcccatcc tggaaaagat 2460

ggacggcacc gaggaactgc tcgtgaagct gaacagagag gacctgctgc ggaagcagcg 2520

gaccttcgac aacggcagca tcccccacca gatccacctg ggagagctgc acgccattct 2580

gcggcggcag gaagattttt acccattcct gaaggacaac cgggaaaaga tcgagaagat 2640

cctgaccttc cgcatcccct actacgtggg ccctctggcc aggggaaaca gcagattcgc 2700

ctggatgacc agaaagagcg aggaaaccat caccccctgg aacttcgagg aagtggtgga 2760

caagggcgct tccgcccaga gcttcatcga gcggatgacc aacttcgata agaacctgcc 2820

caacgagaag gtgctgccca agcacagcct gctgtacgag tacttcaccg tgtataacga 2880

gctgaccaaa gtgaaatacg tgaccgaggg aatgagaaag cccgccttcc tgagcggcga 2940

gcagaaaaag gccatcgtgg acctgctgtt caagaccaac cggaaagtga ccgtgaagca 3000

gctgaaagag gactacttca agaaaatcga gtgcttcgac tccgtggaaa tctccggcgt 3060

ggaagatcgg ttcaacgcct ccctgggcac ataccacgat ctgctgaaaa ttatcaagga 3120

caaggacttc ctggacaatg aggaaaacga ggacattctg gaagatatcg tgctgaccct 3180

gacactgttt gaggacagag agatgatcga ggaacggctg aaaacctatg cccacctgtt 3240

cgacgacaaa gtgatgaagc agctgaagcg gcggagatac accggctggg gcaggctgag 3300

ccggaagctg atcaacggca tccgggacaa gcagtccggc aagacaatcc tggatttcct 3360

gaagtccgac ggcttcgcca acagaaactt catgcagctg atccacgacg acagcctgac 3420

ctttaaagag gacatccaga aagcccaggt gtccggccag ggcgatagcc tgcacgagca 3480

cattgccaat ctggccggca gccccgccat taagaagggc atcctgcaga cagtgaaggt 3540

ggtggacgag ctcgtgaaag tgatgggccg gcacaagccc gagaacatcg tgatcgaaat 3600

ggccagagag aaccagacca cccagaaggg acagaagaac agccgcgaga gaatgaagcg 3660

gatcgaagag ggcatcaaag agctgggcag ccagatcctg aaagaacacc ccgtggaaaa 3720

cacccagctg cagaacgaga agctgtacct gtactacctg cagaatgggc gggatatgta 3780

cgtggaccag gaactggaca tcaaccggct gtccgactac gatgtggacc atatcgtgcc 3840

tcagagcttt ctgaaggacg actccatcga caacaaggtg ctgaccagaa gcgacaagaa 3900

ccggggcaag agcgacaacg tgccctccga agaggtcgtg aagaagatga agaactactg 3960

gcggcagctg ctgaacgcca agctgattac ccagagaaag ttcgacaatc tgaccaaggc 4020

cgagagaggc ggcctgagcg aactggataa ggccggcttc atcaagagac agctggtgga 4080

aacccggcag atcacaaagc acgtggcaca gatcctggac tcccggatga acactaagta 4140

cgacgagaat gacaagctga tccgggaagt gaaagtgatc accctgaagt ccaagctggt 4200

gtccgatttc cggaaggatt tccagtttta caaagtgcgc gagatcaaca actaccacca 4260

cgcccacgac gcctacctga acgccgtcgt gggaaccgcc ctgatcaaaa agtaccctaa 4320

gctggaaagc gagttcgtgt acggcgacta caaggtgtac gacgtgcgga agatgatcgc 4380

caagagcgag caggaaatcg gcaaggctac cgccaagtac ttcttctaca gcaacatcat 4440

gaactttttc aagaccgaga ttaccctggc caacggcgag atccggaagc ggcctctgat 4500

cgagacaaac ggcgaaaccg gggagatcgt gtgggataag ggccgggatt ttgccaccgt 4560

gcggaaagtg ctgagcatgc cccaagtgaa tatcgtgaaa aagaccgagg tgcagacagg 4620

cggcttcagc aaagagtcta tcctgcccaa gaggaacagc gataagctga tcgccagaaa 4680

gaaggactgg gaccctaaga agtacggcgg cttcgacagc cccaccgtgg cctattctgt 4740

gctggtggtg gccaaagtgg aaaagggcaa gtccaagaaa ctgaagagtg tgaaagagct 4800

gctggggatc accatcatgg aaagaagcag cttcgagaag aatcccatcg actttctgga 4860

agccaagggc tacaaagaag tgaaaaagga cctgatcatc aagctgccta agtactccct 4920

gttcgagctg gaaaacggcc ggaagagaat gctggcctct gccggcgaac tgcagaaggg 4980

aaacgaactg gccctgccct ccaaatatgt gaacttcctg tacctggcca gccactatga 5040

gaagctgaag ggctcccccg aggataatga gcagaaacag ctgtttgtgg aacagcacaa 5100

gcactacctg gacgagatca tcgagcagat cagcgagttc tccaagagag tgatcctggc 5160

cgacgctaat ctggacaaag tgctgtccgc ctacaacaag caccgggata agcccatcag 5220

agagcaggcc gagaatatca tccacctgtt taccctgacc aatctgggag cccctgccgc 5280

cttcaagtac tttgacacca ccatcgaccg gaagaggtac accagcacca aagaggtgct 5340

ggacgccacc ctgatccacc agagcatcac cggcctgtac gagacacgga tcgacctgtc 5400

tcagctggga ggcgacagcc ccaagaagaa gagaaaggtg gaggccagct aagaattcct 5460

agagctcgct gatcagcctc gactgtgcct tctagttgcc agccatctgt tgtttgcccc 5520

tcccccgtgc cttccttgac cctggaaggt gccactccca ctgtcctttc ctaataaaat 5580

gaggaaattg catcgcattg tctgagtagg tgtcattcta ttctgggggg tggggtgggg 5640

caggacagca agggggagga ttgggaagag aatagcaggc atgctgggga gcggccgcag 5700

gaacccctag tgatggagtt ggccactccc tctctgcgcg ctcgctcgct cactgaggcc 5760

gggcgaccaa aggtcgcccg acgcccgggc tttgcccggg cggcctcagt gagcgagcga 5820

gcgcgcagct gcctgcaggg gcgcctgatg cggtattttc tccttacgca tctgtgcggt 5880

atttcacacc gcatacgtca aagcaaccat agtacgcgcc ctgtagcggc gcattaagcg 5940

cggcgggtgt ggtggttacg cgcagcgtga ccgctacact tgccagcgcc ctagcgcccg 6000

ctcctttcgc tttcttccct tcctttctcg ccacgttcgc cggctttccc cgtcaagctc 6060

taaatcgggg gctcccttta gggttccgat ttagtgcttt acggcacctc gaccccaaaa 6120

aacttgattt gggtgatggt tcacgtagtg ggccatcgcc ctgatagacg gtttttcgcc 6180

ctttgacgtt ggagtccacg ttctttaata gtggactctt gttccaaact ggaacaacac 6240

tcaaccctat ctcgggctat tcttttgatt tataagggat tttgccgatt tcggcctatt 6300

ggttaaaaaa tgagctgatt taacaaaaat ttaacgcgaa ttttaacaaa atattaacgt 6360

ttacaatttt atggtgcact ctcagtacaa tctgctctga tgccgcatag ttaagccagc 6420

cccgacaccc gccaacaccc gctgacgcgc cctgacgggc ttgtctgctc ccggcatccg 6480

cttacagaca agctgtgacc gtctccggga gctgcatgtg tcagaggttt tcaccgtcat 6540

caccgaaacg cgcgagacga aagggcctcg tgatacgcct atttttatag gttaatgtca 6600

tgataataat ggtttcttag acgtcaggtg gcacttttcg gggaaatgtg cgcggaaccc 6660

ctatttgttt atttttctaa atacattcaa atatgtatcc gctcatgaga caataaccct 6720

gataaatgct tcaataatat tgaaaaagga agagtatgag tattcaacat ttccgtgtcg 6780

cccttattcc cttttttgcg gcattttgcc ttcctgtttt tgctcaccca gaaacgctgg 6840

tgaaagtaaa agatgctgaa gatcagttgg gtgcacgagt gggttacatc gaactggatc 6900

tcaacagcgg taagatcctt gagagttttc gccccgaaga acgttttcca atgatgagca 6960

cttttaaagt tctgctatgt ggcgcggtat tatcccgtat tgacgccggg caagagcaac 7020

tcggtcgccg catacactat tctcagaatg acttggttga gtactcacca gtcacagaaa 7080

agcatcttac ggatggcatg acagtaagag aattatgcag tgctgccata accatgagtg 7140

ataacactgc ggccaactta cttctgacaa cgatcggagg accgaaggag ctaaccgctt 7200

ttttgcacaa catgggggat catgtaactc gccttgatcg ttgggaaccg gagctgaatg 7260

aagccatacc aaacgacgag cgtgacacca cgatgcctgt agcaatggca acaacgttgc 7320

gcaaactatt aactggcgaa ctacttactc tagcttcccg gcaacaatta atagactgga 7380

tggaggcgga taaagttgca ggaccacttc tgcgctcggc ccttccggct ggctggttta 7440

ttgctgataa atctggagcc ggtgagcgtg gaagccgcgg tatcattgca gcactggggc 7500

cagatggtaa gccctcccgt atcgtagtta tctacacgac ggggagtcag gcaactatgg 7560

atgaacgaaa tagacagatc gctgagatag gtgcctcact gattaagcat tggtaactgt 7620

cagaccaagt ttactcatat atactttaga ttgatttaaa acttcatttt taatttaaaa 7680

ggatctaggt gaagatcctt tttgataatc tcatgaccaa aatcccttaa cgtgagtttt 7740

cgttccactg agcgtcagac cccgtagaaa agatcaaagg atcttcttga gatccttttt 7800

ttctgcgcgt aatctgctgc ttgcaaacaa aaaaaccacc gctaccagcg gtggtttgtt 7860

tgccggatca agagctacca actctttttc cgaaggtaac tggcttcagc agagcgcaga 7920

taccaaatac tgtccttcta gtgtagccgt agttaggcca ccacttcaag aactctgtag 7980

caccgcctac atacctcgct ctgctaatcc tgttaccagt ggctgctgcc agtggcgata 8040

agtcgtgtct taccgggttg gactcaagac gatagttacc ggataaggcg cagcggtcgg 8100

gctgaacggg gggttcgtgc acacagccca gcttggagcg aacgacctac accgaactga 8160

gatacctaca gcgtgagcta tgagaaagcg ccacgcttcc cgaagggaga aaggcggaca 8220

ggtatccggt aagcggcagg gtcggaacag gagagcgcac gagggagctt ccagggggaa 8280

acgcctggta tctttatagt cctgtcgggt ttcgccacct ctgacttgag cgtcgatttt 8340

tgtgatgctc gtcagggggg cggagcctat ggaaaaacgc cagcaacgcg gcctttttac 8400

ggttcctggc cttttgctgg ccttttgctc acatgt 8436

<210> 130

<211>  8436
<212>  DNA
<213>  Artificial Sequence

<220>
<223>  synthetic DNA

<400>  130
gagggcctat ttcccatgat tccttcatat ttgcatatac gatacaaggc tgttagagag      60

ataattggaa ttaatttgac tgtaaacaca aagatattag tacaaaatac gtgacgtaga     120

aagtaataat ttcttgggta gtttgcagtt ttaaaattat gttttaaaat ggactatcat     180

atgcttaccg taacttgaaa gtatttcgat ttcttggctt tatatatctt gtggaaagga     240

cgaaacaccg gccaagacga ctcaaacgcg ttttagagct agaaatagca agttaaaata     300

aggctagtcc gttatcaact tgaaaaagtg gcaccgagtc ggtgcttttt tgttttagag     360

ctagaaatag caagttaaaa taaggctagt ccgtttttag cgcgtgcgcc aattctgcag     420

acaaatggct ctagaggtac ccgttacata acttacggta aatggcccgc ctggctgacc     480

gcccaacgac ccccgcccat tgacgtcaat agtaacgcca atagggactt tccattgacg     540

tcaatgggtg gagtatttac ggtaaactgc ccacttggca gtacatcaag tgtatcatat     600

gccaagtacg ccccctattg acgtcaatga cggtaaatgg cccgcctggc attgtgccca     660

gtacatgacc ttatgggact ttcctacttg gcagtacatc tacgtattag tcatcgctat     720

taccatggtc gaggtgagcc ccacgttctg cttcactctc cccatctccc ccccctcccc     780

acccccaatt ttgtatttat ttatttttta attattttgt gcagcgatgg gggcgggggg     840

gggggggggg cgcgcgccag gcggggcggg gcggggcgag gggcggggcg gggcgaggcg     900

gagaggtgcg gcggcagcca atcagagcgg cgcgctccga aagtttcctt ttatggcgag     960

gcggcggcgg cggcggccct ataaaaagcg aagcgcgcgg cgggcgggag tcgctgcgac    1020

gctgccttcg ccccgtgccc cgctccgccg ccgcctcgcg ccgcccgccc cggctctgac    1080

tgaccgcgtt actcccacag gtgagcgggc gggacggccc ttctcctccg ggctgtaatt    1140

agctgagcaa gaggtaaggg tttaagggat ggttggttgg tggggtatta atgtttaatt    1200

acctggagca cctgcctgaa atcacttttt ttcaggttgg accggtgcca ccatgtaccc    1260

atacgatgtt ccagattacg cttcgccgaa gaaaaagcgc aaggtcgaag cgtccgacaa    1320

gaagtacagc atcggcctgg ccatcggcac caactctgtg ggctgggccg tgatcaccga    1380

cgagtacaag gtgcccagca agaaattcaa ggtgctgggc aacaccgacc ggcacagcat    1440

caagaagaac ctgatcggag ccctgctgtt cgacagcggc gaaacagccg aggccacccg    1500

gctgaagaga accgccagaa gaagatacac cagacggaag aaccggatct gctatctgca    1560

agagatcttc agcaacgaga tggccaaggt ggacgacagc ttcttccaca gactggaaga    1620

gtccttcctg gtggaagagg ataagaagca cgagcggcac cccatcttcg gcaacatcgt    1680

ggacgaggtg gcctaccacg agaagtaccc caccatctac cacctgagaa agaaactggt 1740

ggacagcacc gacaaggccg acctgcggct gatctatctg gccctggccc acatgatcaa 1800

gttccggggc cacttcctga tcgagggcga cctgaacccc gacaacagcg acgtggacaa 1860

gctgttcatc cagctggtgc agacctacaa ccagctgttc gaggaaaacc ccatcaacgc 1920

cagcggcgtg gacgccaagg ccatcctgtc tgccagactg agcaagagca gacggctgga 1980

aaatctgatc gcccagctgc ccggcgagaa gaagaatggc ctgttcggca acctgattgc 2040

cctgagcctg ggcctgaccc ccaacttcaa gagcaacttc gacctggccg aggatgccaa 2100

actgcagctg agcaaggaca cctacgacga cgacctggac aacctgctgg cccagatcgg 2160

cgaccagtac gccgacctgt ttctggccgc caagaacctg tccgacgcca tcctgctgag 2220

cgacatcctg agagtgaaca ccgagatcac caaggccccc ctgagcgcct ctatgatcaa 2280

gagatacgac gagcaccacc aggacctgac cctgctgaaa gctctcgtgc ggcagcagct 2340

gcctgagaag tacaaagaga ttttcttcga ccagagcaag aacggctacg ccggctacat 2400

tgacggcgga gccagccagg aagagttcta caagttcatc aagcccatcc tggaaaagat 2460

ggacggcacc gaggaactgc tcgtgaagct gaacagagag gacctgctgc ggaagcagcg 2520

gaccttcgac aacggcagca tcccccacca gatccacctg ggagagctgc acgccattct 2580

gcggcggcag gaagattttt acccattcct gaaggacaac cgggaaaaga tcgagaagat 2640

cctgaccttc cgcatcccct actacgtggg ccctctggcc aggggaaaca gcagattcgc 2700

ctggatgacc agaaagagcg aggaaaccat caccccctgg aacttcgagg aagtggtgga 2760

caagggcgct tccgcccaga gcttcatcga gcggatgacc aacttcgata agaacctgcc 2820

caacgagaag gtgctgccca agcacagcct gctgtacgag tacttcaccg tgtataacga 2880

gctgaccaaa gtgaaatacg tgaccgaggg aatgagaaag cccgccttcc tgagcggcga 2940

gcagaaaaag gccatcgtgg acctgctgtt caagaccaac cggaaagtga ccgtgaagca 3000

gctgaaagag gactacttca agaaaatcga gtgcttcgac tccgtggaaa tctccggcgt 3060

ggaagatcgg ttcaacgcct ccctgggcac ataccacgat ctgctgaaaa ttatcaagga 3120

caaggacttc ctggacaatg aggaaaacga ggacattctg gaagatatcg tgctgaccct 3180

gacactgttt gaggacagag agatgatcga ggaacggctg aaaacctatg cccacctgtt 3240

cgacgacaaa gtgatgaagc agctgaagcg gcggagatac accggctggg gcaggctgag 3300

ccggaagctg atcaacggca tccgggacaa gcagtccggc aagacaatcc tggatttcct 3360

gaagtccgac ggcttcgcca acagaaactt catgcagctg atccacgacg acagcctgac 3420

ctttaaagag gacatccaga aagcccaggt gtccggccag ggcgatagcc tgcacgagca 3480

cattgccaat ctggccggca gccccgccat taagaagggc atcctgcaga cagtgaaggt 3540

ggtggacgag ctcgtgaaag tgatgggccg gcacaagccc gagaacatcg tgatcgaaat 3600

ggccagagag aaccagacca cccagaaggg acagaagaac agccgcgaga gaatgaagcg 3660

gatcgaagag ggcatcaaag agctgggcag ccagatcctg aaagaacacc ccgtggaaaa 3720

cacccagctg cagaacgaga agctgtacct gtactacctg cagaatgggc gggatatgta 3780

cgtggaccag gaactggaca tcaaccggct gtccgactac gatgtggacc atatcgtgcc 3840

tcagagcttt ctgaaggacg actccatcga caacaaggtg ctgaccagaa gcgacaagaa 3900

ccggggcaag agcgacaacg tgccctccga agaggtcgtg aagaagatga agaactactg 3960

gcggcagctg ctgaacgcca agctgattac ccagagaaag ttcgacaatc tgaccaaggc 4020

cgagagaggc ggcctgagcg aactggataa ggccggcttc atcaagagac agctggtgga 4080

aacccggcag atcacaaagc acgtggcaca gatcctggac tcccggatga acactaagta 4140

cgacgagaat gacaagctga tccgggaagt gaaagtgatc accctgaagt ccaagctggt 4200

gtccgatttc cggaaggatt tccagtttta caaagtgcgc gagatcaaca actaccacca 4260

cgcccacgac gcctacctga acgccgtcgt gggaaccgcc ctgatcaaaa agtaccctaa 4320

gctggaaagc gagttcgtgt acggcgacta caaggtgtac gacgtgcgga agatgatcgc 4380

caagagcgag caggaaatcg gcaaggctac cgccaagtac ttcttctaca gcaacatcat 4440

gaactttttc aagaccgaga ttaccctggc caacggcgag atccggaagc ggcctctgat 4500

cgagacaaac ggcgaaaccg gggagatcgt gtgggataag ggccgggatt ttgccaccgt 4560

gcggaaagtg ctgagcatgc cccaagtgaa tatcgtgaaa aagaccgagg tgcagacagg 4620

cggcttcagc aaagagtcta tcctgcccaa gaggaacagc gataagctga tcgccagaaa 4680

gaaggactgg gaccctaaga agtacggcgg cttcgacagc cccaccgtgg cctattctgt 4740

gctggtggtg gccaaagtgg aaaagggcaa gtccaagaaa ctgaagagtg tgaaagagct 4800

gctggggatc accatcatgg aaagaagcag cttcgagaag aatcccatcg actttctgga 4860

agccaagggc tacaaagaag tgaaaaagga cctgatcatc aagctgccta agtactccct 4920

gttcgagctg gaaaacggcc ggaagagaat gctggcctct gccggcgaac tgcagaaggg 4980

aaacgaactg gccctgccct ccaaatatgt gaacttcctg tacctggcca gccactatga 5040

gaagctgaag ggctcccccg aggataatga gcagaaacag ctgtttgtgg aacagcacaa 5100

gcactacctg gacgagatca tcgagcagat cagcgagttc tccaagagag tgatcctggc 5160

cgacgctaat ctggacaaag tgctgtccgc ctacaacaag caccgggata agcccatcag 5220

agagcaggcc gagaatatca tccacctgtt taccctgacc aatctgggag cccctgccgc 5280

cttcaagtac tttgacacca ccatcgaccg gaagaggtac accagcacca aagaggtgct 5340

ggacgccacc ctgatccacc agagcatcac cggcctgtac gagacacgga tcgacctgtc 5400

tcagctggga ggcgacagcc ccaagaagaa gagaaaggtg gaggccagct aagaattcct 5460

agagctcgct gatcagcctc gactgtgcct tctagttgcc agccatctgt tgtttgcccc 5520

tcccccgtgc cttccttgac cctggaaggt gccactccca ctgtcctttc ctaataaaat 5580

gaggaaattg catcgcattg tctgagtagg tgtcattcta ttctgggggg tggggtgggg 5640

caggacagca agggggagga ttgggaagag aatagcaggc atgctgggga gcggccgcag 5700

gaacccctag tgatggagtt ggccactccc tctctgcgcg ctcgctcgct cactgaggcc 5760

gggcgaccaa aggtcgcccg acgcccgggc tttgcccggg cggcctcagt gagcgagcga 5820

gcgcgcagct gcctgcaggg gcgcctgatg cggtattttc tccttacgca tctgtgcggt 5880

atttcacacc gcatacgtca aagcaaccat agtacgcgcc ctgtagcggc gcattaagcg 5940

cggcgggtgt ggtggttacg cgcagcgtga ccgctacact tgccagcgcc ctagcgcccg 6000

ctcctttcgc tttcttccct tcctttctcg ccacgttcgc cggctttccc cgtcaagctc 6060

taaatcgggg gctcccttta gggttccgat ttagtgcttt acggcacctc gaccccaaaa 6120

aacttgattt gggtgatggt tcacgtagtg ggccatcgcc ctgatagacg gtttttcgcc 6180

ctttgacgtt ggagtccacg ttctttaata gtggactctt gttccaaact ggaacaacac 6240

tcaaccctat ctcgggctat tcttttgatt tataagggat tttgccgatt tcggcctatt 6300

ggttaaaaaa tgagctgatt taacaaaaat ttaacgcgaa ttttaacaaa atattaacgt 6360

ttacaatttt atggtgcact ctcagtacaa tctgctctga tgccgcatag ttaagccagc 6420

cccgacaccc gccaacaccc gctgacgcgc cctgacgggc ttgtctgctc ccggcatccg 6480

cttacagaca agctgtgacc gtctccggga gctgcatgtg tcagaggttt tcaccgtcat 6540

caccgaaacg cgcgagacga aagggcctcg tgatacgcct atttttatag gttaatgtca 6600

tgataataat ggtttcttag acgtcaggtg gcacttttcg gggaaatgtg cgcggaaccc 6660

ctatttgttt atttttctaa atacattcaa atatgtatcc gctcatgaga caataaccct 6720

gataaatgct tcaataatat tgaaaaagga agagtatgag tattcaacat ttccgtgtcg 6780

cccttattcc cttttttgcg gcattttgcc ttcctgtttt tgctcaccca gaaacgctgg 6840

tgaaagtaaa agatgctgaa gatcagttgg gtgcacgagt gggttacatc gaactggatc 6900

tcaacagcgg taagatcctt gagagttttc gccccgaaga acgttttcca atgatgagca 6960

cttttaaagt tctgctatgt ggcgcggtat tatcccgtat tgacgccggg caagagcaac 7020

tcggtcgccg catacactat tctcagaatg acttggttga gtactcacca gtcacagaaa 7080

agcatcttac ggatggcatg acagtaagag aattatgcag tgctgccata accatgagtg 7140

ataacactgc ggccaactta cttctgacaa cgatcggagg accgaaggag ctaaccgctt 7200

ttttgcacaa catgggggat catgtaactc gccttgatcg ttgggaaccg gagctgaatg 7260

aagccatacc aaacgacgag cgtgacacca cgatgcctgt agcaatggca acaacgttgc 7320

gcaaactatt aactggcgaa ctacttactc tagcttcccg gcaacaatta atagactgga 7380

tggaggcgga taaagttgca ggaccacttc tgcgctcggc ccttccggct ggctggttta 7440

ttgctgataa atctggagcc ggtgagcgtg gaagccgcgg tatcattgca gcactggggc 7500

cagatggtaa gccctcccgt atcgtagtta tctacacgac ggggagtcag gcaactatgg 7560

atgaacgaaa tagacagatc gctgagatag gtgcctcact gattaagcat tggtaactgt 7620

cagaccaagt ttactcatat atactttaga ttgatttaaa acttcatttt taatttaaaa 7680

ggatctaggt gaagatcctt tttgataatc tcatgaccaa aatcccttaa cgtgagtttt 7740

cgttccactg agcgtcagac cccgtagaaa agatcaaagg atcttcttga gatccttttt 7800

ttctgcgcgt aatctgctgc ttgcaaacaa aaaaaccacc gctaccagcg gtggtttgtt 7860

tgccggatca agagctacca actctttttc cgaaggtaac tggcttcagc agagcgcaga 7920

taccaaatac tgtccttcta gtgtagccgt agttaggcca ccacttcaag aactctgtag 7980

caccgcctac atacctcgct ctgctaatcc tgttaccagt ggctgctgcc agtggcgata 8040

agtcgtgtct taccgggttg gactcaagac gatagttacc ggataaggcg cagcggtcgg 8100

gctgaacggg gggttcgtgc acacagccca gcttggagcg aacgacctac accgaactga 8160

gatacctaca gcgtgagcta tgagaaagcg ccacgcttcc cgaagggaga aaggcggaca 8220

ggtatccggt aagcggcagg gtcggaacag gagagcgcac gagggagctt ccagggggaa 8280

acgcctggta tctttatagt cctgtcgggt ttcgccacct ctgacttgag cgtcgatttt 8340

tgtgatgctc gtcagggggg cggagcctat ggaaaaacgc cagcaacgcg gcctttttac 8400

ggttcctggc cttttgctgg ccttttgctc acatgt 8436

<210> 131
<211> 24
<212> DNA
<213> Artificial Sequence

<220>
<223> oligonucleotide

<400> 131
cactcgtttg caacgccaac ctgg 24

<210> 132
<211> 22
<212> DNA
<213> Artificial Sequence

<220>
<223> oligonucleotide

<400> 132
gaacccgatg acaacccgat ag 22

<210>  133
<211>  24
<212>  DNA
<213>  Artificial Sequence

<220>
<223>  oligonucleotide

<400>  133
tccatctgct tcttgacagt gagg                                              24


<210>  134
<211>  817
<212>  DNA
<213>  Artificial Sequence

<220>
<223>  synthetic DNA

<400>  134
gattaaaggc gtgcaccact actgtctgcc ttttgttttg gttttatcat aattgttgca      60

tttttgcccg caggaggatc aggaattcaa ggacaacctc tacagccaac ccagttgagg     120

gccagcctgt tctatcgaga tcctgtctcc ggacaaaaaa agtttcatga gagcgtggtg     180

tgttggcaca gtcttttagt ccgagcactg acgcagtaga ttaaggcaga tcttcatgat     240

ttggtgcggt cctgggctgc atccaaagac cttgtttcca aaacaaacaa acgaaaaagt     300

gtaataaaaa tacaagcaag gttttgcaga aataaatctg tggcctcaag ggtccctttc     360

tccagctttt cccagaggtg tcaaattaca taacagtctc aaacccagga aattgaactt     420

tggcacaacc cactgaccgg tccgaatttc aaagtctttt tcctattgac ctacaaggtt     480

ttcaagaatc atgttgtaag caactgtgtt ctgaggaatc tatgtttaaa aacccatccg     540

tggatcttgg cccagggtcc agagactgag ctagccacgc cccggccgcg ccgcagccac     600

tcccacggca gttcaagtgt taagtcccaa agaccgcgct ctgtgcatgc gcagacccgt     660

ccacagctgg ctcctagcca acccggccgg acgagcaccc ggcgccgtca cgtgacgcac     720

ccaaccggcg tcgacctata aaaggccggg cgttgacgtc agcggtctct tccgccgcag     780

ccgccgccat cgtcggcgcg cttccctgtt cacctct                              817


<210>  135
<211>  37
<212>  DNA
<213>  Artificial Sequence

<220>
<223>  oligonucleotide

<400>  135
tttaacctgc agggattaaa ggcgtgcacc actactg                                37


<210>  136

<211>  36
<212>  DNA
<213>  Artificial Sequence

<220>
<223>  oligonucleotide

<400>  136
tatataacta gtagaggtga acagggaagc gcgccg                                36


<210>  137
<211>  826
<212>  DNA
<213>  Artificial Sequence

<220>
<223>  synthetic DNA

<400>  137
gactctgaga atccgtcgcc atccgccacc atggtgagtc tcctcggctc cgctagactc      60

ggggaccgag acgaatctct ggggcagcgg gacgtggctg tagcgggacg ctgagaggga     120

cgggaggaag agacatggct gccctggccc gggcggcagg acgtggtcgg gccgcggcgc     180

catatctgcg cgtccctgag ggccttggga gtgtcaactg ccgaggtcgg ggtgttttct     240

tgaagtcctt caactccccg cggccgccgg ggtgactgcg ggaggggttg tgcttggtga     300

tgtggcagcg ggcaaagcgc cgtccccgcg cccctggtga cgggcggagg gtgtcctcgg     360

gaggtgacag cctgtagggc tggcttcctt ggacacctcc agtgggctga acgccttccg     420

ggccctttcc ggtagccccc gtctctgttt tctatctgag ttcacacgtg agcaccggtc     480

cccataatct aagaaagtgg ctcactgggc ctagtggcgc attgtggcct ttgatccggg     540

ctttgacctt ggcgcacagc acccagtggt ttggggaaga ggtgtgtgta gcagaggagg     600

ttttttcgtg ctttggtccc aatcaatccg gcatctttgc agtgccgagg tggccgtgca     660

ccttggcttt gaattcttgt gctgaggtta tgtgacttga gcctcaagat agggtcttct     720

agcacaggct tgctcttaag tgtcgcagtt gtcggtttcg gcgtttgttt agagctgtgg     780

acacatctgt gaacttttga tgcttatttc agaggtcctg ggttgt                    826


<210>  138
<211>  36
<212>  DNA
<213>  Artificial Sequence

<220>
<223>  oligonucleotide

<400>  138
ttatagcatg cgactctgag aatccgtcgc catccg                                36


<210>  139
<211>  36

<212>  DNA
<213>  Artificial Sequence

<220>
<223>  oligonucleotide

<400>  139
ttatagcatg cacaacccag gacctctgaa ataagc                                36


<210>  140
<211>  8317
<212>  DNA
<213>  Artificial Sequence

<220>
<223>  synthetic DNA

<400>  140
tatagtgagt cgtattacaa ttcactggcc gtcgttttac aacgtcgtga ctgggaaaac      60

cctggcgtta cccaacttaa tcgccttgca gcacatcccc ctttcgccag ctggcgtaat     120

agcgaagagg cccgcaccga tcgcccttcc caacagttgc gcagcctgaa tggcgaatgg     180

acgcgccctg tagcggcgca ttaagcgcgg cgggtgtggt ggttacgcgc agcgtgaccg     240

ctacacttgc cagcgcccta gcgcccgctc ctttcgcttt cttcccttcc tttctcgcca     300

cgttcgccgg ctttccccgt caagctctaa atcgggggct ccctttaggg ttccgattta     360

gtgctttacg gcacctcgac cccaaaaaac ttgattaggg tgatggttca cgtagtgggc     420

catcgccctg atagacggtt tttcgccctt tgacgttgga gtccacgttc tttaatagtg     480

gactcttgtt ccaaactgga acaacactca accctatctc ggtctattct tttgatttat     540

aagggatttt gccgatttcg gcctattggt taaaaaatga gctgatttaa caaaaattta     600

acgcgaattt taacaaaata ttaacgctta caatttcctg atgcggtatt ttctccttac     660

gcatctgtgc ggtatttcac accgcatcag gtggcacttt tcggggaaat gtgcgcggaa     720

cccctatttg tttatttttc taaatacatt caaatatgta tccgctcatg agacaataac     780

cctgataaat gcttcaataa tattgaaaaa ggaagagtat gagtattcaa catttccgtg     840

tcgcccttat tccctttttt gcggcatttt gccttcctgt ttttgctcac ccagaaacgc     900

tggtgaaagt aaaagatgct gaagatcagt tgggtgcacg agtgggttac atcgaactgg     960

atctcaacag cggtaagatc cttgagagtt ttcgccccga agaacgtttt ccaatgatga    1020

gcacttttaa agttctgcta tgtggcgcgg tattatcccg tattgacgcc gggcaagagc    1080

aactcggtcg ccgcatacac tattctcaga atgacttggt tgagtactca ccagtcacag    1140

aaaagcatct tacggatggc atgacagtaa gagaattatg cagtgctgcc ataaccatga    1200

gtgataacac tgcggccaac ttacttctga caacgatcgg aggaccgaag gagctaaccg    1260

cttttttgca caacatgggg gatcatgtaa ctcgccttga tcgttgggaa ccggagctga    1320

atgaagccat accaaacgac gagcgtgaca ccacgatgcc tgtagcaatg gcaacaacgt 1380

tgcgcaaact attaactggc gaactactta ctctagcttc ccggcaacaa ttaatagact 1440

ggatggaggc ggataaagtt gcaggaccac ttctgcgctc ggcccttccg gctggctggt 1500

ttattgctga taaatctgga gccggtgagc gtgggtctcg cggtatcatt gcagcactgg 1560

ggccagatgg taagccctcc cgtatcgtag ttatctacac gacggggagt caggcaacta 1620

tggatgaacg aaatagacag atcgctgaga taggtgcctc actgattaag cattggtaac 1680

tgtcagacca agtttactca tatatacttt agattgattt aaaacttcat ttttaattta 1740

aaaggatcta ggtgaagatc ctttttgata atctcatgac caaaatccct taacgtgagt 1800

tttcgttcca ctgagcgtca gaccccgtag aaaagatcaa aggatcttct tgagatcctt 1860

tttttctgcg cgtaatctgc tgcttgcaaa caaaaaaacc accgctacca gcggtggttt 1920

gtttgccgga tcaagagcta ccaactcttt ttccgaaggt aactggcttc agcagagcgc 1980

agataccaaa tactgttctt ctagtgtagc cgtagttagg ccaccacttc aagaactctg 2040

tagcaccgcc tacatacctc gctctgctaa tcctgttacc agtggctgct gccagtggcg 2100

ataagtcgtg tcttaccggg ttggactcaa gacgatagtt accggataag gcgcagcggt 2160

cgggctgaac ggggggttcg tgcacacagc ccagcttgga gcgaacgacc tacaccgaac 2220

tgagatacct acagcgtgag ctatgagaaa gcgccacgct tcccgaaggg agaaaggcgg 2280

acaggtatcc ggtaagcggc agggtcggaa caggagagcg cacgagggag cttccagggg 2340

gaaacgcctg gtatctttat agtcctgtcg ggtttcgcca cctctgactt gagcgtcgat 2400

ttttgtgatg ctcgtcaggg gggcggagcc tatggaaaaa cgccagcaac gcggcctttt 2460

tacggttcct ggccttttgc tggccttttg ctcacatgtt ctttcctgcg ttatcccctg 2520

attctgtgga taaccgtatt accgcctttg agtgagctga taccgctcgc cgcagccgaa 2580

cgaccgagcg cagcgagtca gtgagcgagg aagcggaaga gcgcccaata cgcaaaccgc 2640

ctctccccgc gcgttggccg attcattaat gcagctggca cgacaggttt cccgactgga 2700

aagcgggcag tgagcgcaac gcaattaatg tgagttagct cactcattag gcaccccagg 2760

ctttacactt tatgcttccg gctcgtatgt tgtgtggaat tgtgagcgga taacaatttc 2820

acacaggaaa cagctatgac catgattacg ccaagctatt taggtgacac tatagaatac 2880

tcaagctatg catccaacgc gttgggagct ctcccatatg gtcgacctgc agggattaaa 2940

ggcgtgcacc actactgtct gccttttgtt ttggttttat cataattgtt gcatttttgc 3000

ccgcaggagg atcaggaatt caaggacaac ctctacagcc aacccagttg agggccagcc 3060

tgttctatcg agatcctgtc tccggacaaa aaaagtttca tgagagcgtg gtgtgttggc 3120

acagtctttt agtccgagca ctgacgcagt agattaaggc agatcttcat gatttggtgc 3180

ggtcctgggc tgcatccaaa gaccttgttt ccaaaacaaa caaacgaaaa agtgtaataa 3240

aaatacaagc aaggttttgc agaaataaat ctgtggcctc aagggtccct ttctccagct 3300

tttcccagag gtgtcaaatt acataacagt ctcaaaccca ggaaattgaa ctttggcaca 3360

acccactgac cggtccgaat ttcaaagtct ttttcctatt gacctacaag gttttcaaga 3420

atcatgttgt aagcaactgt gttctgagga atctatgttt aaaaacccat ccgtggatct 3480

tggcccaggg tccagagact gagctagcca cgccccggcc gcgccgcagc cactcccacg 3540

gcagttcaag tgttaagtcc caaagaccgc gctctgtgca tgcgcagacc cgtccacagc 3600

tggctcctag ccaacccggc cggacgagca cccggcgccg tcacgtgacg cacccaaccg 3660

gcgtcgacct ataaaaggcc gggcgttgac gtcagcggtc tcttccgccg cagccgccgc 3720

catcgtcggc gcgcttccct gttcacctct actagtgatt accgttcgta tagcatacat 3780

tatacgaagt tataccatgt ctagactgga caagagcaaa gtcataaacg gcgctctgga 3840

attactcaat ggagtcggta tcgaaggcct gacgacaagg aaactcgctc aaaagctggg 3900

agttgagcag cctaccctgt actggcacgt gaagaacaag cgggccctgc tcgatgccct 3960

gccaatcgag atgctggaca ggcatcatac ccacttctgc cccctggaag gcgagtcatg 4020

gcaagacttt ctgcggaaca acgccaagtc attccgctgt gctctcctct cacatcgcga 4080

cggggctaaa gtgcatctcg gcacccgccc aacagagaaa cagtacgaaa ccctggaaaa 4140

tcagctcgcg ttcctgtgtc agcaaggctt ctccctggag aacgcactgt acgctctgtc 4200

cgccgtgggc cactttacac tgggctgcgt attggaggaa caggagcatc aagtagcaaa 4260

agaggaaaga gagacaccta ccaccgattc tatgccccca cttctgagac aagcaattga 4320

gctgttcgac cggcagggag ccgaacctgc cttccttttc ggcctggaac taatcatatg 4380

tggcctggag aaacagctaa agtgcgaaag cggcgggccg gccgacgccc ttgacgattt 4440

tgacttagac atgctcccag ccgatgccct tgacgacttt gaccttgata tgctgcctgc 4500

tgacgctctt gacgattttg accttgacat gctccccggg taactaagta aggatcccaa 4560

ataccactga attaagaatt ccgcccctct ccctcccccc cccctaacgt tactggccga 4620

agccgcttgg aataaggccg gtgtgcgttt gtctatatgt tattttccac catattgccg 4680

tcttttggca atgtgagggc ccggaaacct ggccctgtct tcttgacgag cattcctagg 4740

ggtctttccc ctctcgccaa aggaatgcaa ggtctgttga atgtcgtgaa ggaagcagtt 4800

cctctggaag cttcttgaag acaaacaacg tctgtagcga ccctttgcag gcagcggaac 4860

cccccacctg gcgacaggtg cctctgcggc caaaagccac gtgtataaga tacacctgca 4920

aaggcggcac aaccccagtg ccacgttgtg agttggatag ttgtggaaag agtcaaatgg 4980

ctctcctcaa gcgtattcaa caaggggctg aaggatgccc agaaggtacc ccattgtatg 5040

ggatctgatc tggggcctcg gtgcacatgc tttacatgtg tttagtcgag gttaaaaaac 5100

gtctaggccc cccgaaccac ggggacgtgg ttttcctttg aaaaacacga tgataatatg 5160

gccacaacca tgaccgagta caagcccacg gtgcgcctcg ccacccgcga cgacgtcccc 5220

cgggccgtac gcaccctcgc cgccgcgttc gccgactacc ccgccacgcg ccacaccgtc 5280

gacccggacc gccacatcga gcgggtcacc gagctgcaag aactcttcct cacgcgcgtc 5340

gggctcgaca tcggcaaggt gtgggtcgcg gacgacggcg ccgcggtggc ggtctggacc 5400

acgccggaga gcgtcgaagc gggggcggtg ttcgccgaga tcggcccgcg catggccgag 5460

ttgagcggtt cccggctggc cgcgcagcaa cagatggaag gcctcctggc gccgcaccgg 5520

cccaaggagc ccgcgtggtt cctggccacc gtcggcgtct cgcccgacca ccagggcaag 5580

ggtctgggca gcgccgtcgt gctccccgga gtggaggcgg ccgagcgcgc cggggtgccc 5640

gccttcctgg agacctccgc gccccgcaac ctccccttct acgagcggct cggcttcacc 5700

gtcaccgccg acgtcgaggt gcccgaagga ccgcgcacct ggtgcatgac ccgcaagccc 5760

ggtgcctgag gatccagaca tgataagata cattgatgag tttggacaaa ccacaactag 5820

aatgcagtga aaaaaatgct ttatttgtga aatttgtgat gctattgctt tatttgtaac 5880

cattataagc tgcaataaac aagttaacaa caacaattgc attcatttta tgtttcaggt 5940

tcagggggag gtgtgggagg ttttttaaag caagtaaaac ctctacaaat gtggtatggc 6000

tgattatgat cctctagagt cgcagatcca gacatgataa gatacattga tgagtttgga 6060

caaaccacaa ctagaatgca gtgaaaaaaa tgctttattt gtgaaatttg tgatgctatt 6120

gctttatttg taaccattat aagctgcaat aaacaagtta acaacaacaa ttgcattcat 6180

tttatgtttc aggttcaggg ggaggtgtgg gaggtttttt aaagcaagta aaacctctac 6240

aaatgtggta tggctgatta tgatcctcta gagtcgcaga tccagacatg ataagataca 6300

ttgatgagtt tggacaaacc acaactagaa tgcagtgaaa aaaatgcttt atttgtgaaa 6360

tttgtgatgc tattgcttta tttgtaacca ttataagctg caataaacaa gttaacaaca 6420

acaattgcat tcattttatg tttcaggttc agggggaggt gtgggaggtt ttttaaagca 6480

agtaaaacct ctacaaatgt ggtatggctg attatgatcc tctagagtcg cagatcctct 6540

agagtcgcag atcttttccc ctctgccaaa aattatgggg acatcatgaa gccccttgag 6600

catctgactt ctggctaata aaggaaattt attttcattg caatagtgtg ttggaatttt 6660

ttgtgtctct cactcggaag gacatatggg agggcaaatc atttaaaaca tcagaatgag 6720

tatttggttt agagtttggc aacatatgcc atatgctggc tgccatgaac aaaggtggct 6780

ataaagaggt catcagtata tgaaacagcc cctgctgtcc attccttatt ccatagaaaa 6840

agccttgact tgaggttaga ttttttttat attttgtttt gtgttatttt tttctttaac 6900

atccctaaaa ttttccttac atgttttact agccagattt ttcctcctct cctgactact 6960

cccagtcata gctgtccctc ttctcttatg aagatccctc gacctgcagc ccaagcttat 7020

cgaattcccg cggtggcggc cgcacgtctc cctatcagtg atagagaagt cgacacgtct 7080

cgagctccct atcagtgata gagaaggtac gtctagaacg tctccctatc agtgatagag 7140

aagtcgacac gtctcgagct ccctatcagt gatagagaag gtacgtctag aacgtctccc 7200

tatcagtgat agagaagtcg acacgtctcg agctccctat cagtgataga gaaggtacgt 7260

ctagaacgtc tccctatcag tgatagagaa gtcgacacgt ctcgagctcc ctatcagtga 7320

tagagaaggt accccctata taagcagagc tcgtttagtg aaccgtcaga tcgcctggag 7380

acgccatcca cgctgttttg acctccatag aagacaccgg gaccgatcca gcctccgcgg 7440

ccgccatggc ggccgggagc atgcgactct gagaatccgt cgccatccgc caccatggtg 7500

agtctcctcg gctccgctag actcggggac cgagacgaat ctctggggca gcgggacgtg 7560

gctgtagcgg gacgctgaga gggacgggag gaagagacat ggctgccctg gcccgggcgg 7620

caggacgtgg tcgggccgcg gcgccatatc tgcgcgtccc tgagggcctt gggagtgtca 7680

actgccgagg tcggggtgtt ttcttgaagt ccttcaactc cccgcggccg ccggggtgac 7740

tgcgggaggg gttgtgcttg gtgatgtggc agcgggcaaa gcgccgtccc cgcgcccctg 7800

gtgacgggcg gagggtgtcc tcgggaggtg acagcctgta gggctggctt ccttggacac 7860

ctccagtggg ctgaacgcct tccgggccct ttccggtagc ccccgtctct gttttctatc 7920

tgagttcaca cgtgagcacc ggtccccata atctaagaaa gtggctcact gggcctagtg 7980

gcgcattgtg gcctttgatc cgggctttga ccttggcgca cagcacccag tggtttgggg 8040

aagaggtgtg tgtagcagag gaggtttttt cgtgctttgg tcccaatcaa tccggcatct 8100

ttgcagtgcc gaggtggccg tgcaccttgg ctttgaattc ttgtgctgag gttatgtgac 8160

ttgagcctca agatagggtc ttctagcaca ggcttgctct taagtgtcgc agttgtcggt 8220

ttcggcgttt gtttagagct gtggacacat ctgtgaactt ttgatgctta tttcagaggt 8280

cctgggttgt gcatgcgacg tcgggcccaa ttcgccc 8317

<210> 141
<211> 23
<212> DNA
<213> Artificial Sequence

<220>
<223> oligonucleotide

<400> 141
caccgtcggc tccgctagac tcg 23

<210> 142
<211> 23
<212> DNA
<213> Artificial Sequence

<220>
<223>  oligonucleotide

<400>  142
aaaccgagtc tagcggagcc gac                                              23


<210>  143
<211>  23
<212>  DNA
<213>  Artificial Sequence

<220>
<223>  oligonucleotide

<400>  143
caccgactca ccatggtggc gga                                              23


<210>  144
<211>  23
<212>  DNA
<213>  Artificial Sequence

<220>
<223>  oligonucleotide

<400>  144
aaactccgcc accatggtga gtc                                              23


<210>  145
<211>  8435
<212>  DNA
<213>  Artificial Sequence

<220>
<223>  synthetic DNA

<400>  145
gagggcctat ttcccatgat tccttcatat ttgcatatac gatacaaggc tgttagagag      60

ataattggaa ttaatttgac tgtaaacaca aagatattag tacaaaatac gtgacgtaga     120

aagtaataat ttcttgggta gtttgcagtt ttaaaattat gttttaaaat ggactatcat     180

atgcttaccg taacttgaaa gtatttcgat ttcttggctt tatatatctt gtggaaagga     240

cgaaacaccg tcggctccgc tagactcggt tttagagcta gaaatagcaa gttaaaataa     300

ggctagtccg ttatcaactt gaaaaagtgg caccgagtcg gtgctttttt gttttagagc     360

tagaaatagc aagttaaaat aaggctagtc cgtttttagc gcgtgcgcca attctgcaga     420

caaatggctc tagaggtacc cgttacataa cttacggtaa atggcccgcc tggctgaccg     480

cccaacgacc cccgcccatt gacgtcaata gtaacgccaa tagggacttt ccattgacgt     540

caatgggtgg agtatttacg gtaaactgcc cacttggcag tacatcaagt gtatcatatg     600

ccaagtacgc cccctattga cgtcaatgac ggtaaatggc ccgcctggca ttgtgcccag     660

tacatgacct tatgggactt tcctacttgg cagtacatct acgtattagt catcgctatt     720

accatggtcg aggtgagccc cacgttctgc ttcactctcc ccatctcccc cccctcccca 780

cccccaattt tgtatttatt tattttttaa ttattttgtg cagcgatggg ggcggggggg 840

gggggggggc gcgcgccagg cggggcgggg cggggcgagg ggcggggcgg ggcgaggcgg 900

agaggtgcgg cggcagccaa tcagagcggc gcgctccgaa agtttccttt tatggcgagg 960

cggcggcggc ggcggcccta taaaaagcga agcgcgcggc gggcgggagt cgctgcgacg 1020

ctgccttcgc cccgtgcccc gctccgccgc cgcctcgcgc cgcccgcccc ggctctgact 1080

gaccgcgtta ctcccacagg tgagcgggcg ggacggccct tctcctccgg gctgtaatta 1140

gctgagcaag aggtaagggt ttaagggatg gttggttggt ggggtattaa tgtttaatta 1200

cctggagcac ctgcctgaaa tcactttttt tcaggttgga ccggtgccac catgtaccca 1260

tacgatgttc cagattacgc ttcgccgaag aaaaagcgca aggtcgaagc gtccgacaag 1320

aagtacagca tcggcctggc catcggcacc aactctgtgg gctgggccgt gatcaccgac 1380

gagtacaagg tgcccagcaa gaaattcaag gtgctgggca acaccgaccg gcacagcatc 1440

aagaagaacc tgatcggagc cctgctgttc gacagcggcg aaacagccga ggccacccgg 1500

ctgaagagaa ccgccagaag aagatacacc agacggaaga accggatctg ctatctgcaa 1560

gagatcttca gcaacgagat ggccaaggtg gacgacagct tcttccacag actggaagag 1620

tccttcctgg tggaagagga taagaagcac gagcggcacc ccatcttcgg caacatcgtg 1680

gacgaggtgg cctaccacga gaagtacccc accatctacc acctgagaaa gaaactggtg 1740

gacagcaccg acaaggccga cctgcggctg atctatctgg ccctggccca catgatcaag 1800

ttccggggcc acttcctgat cgagggcgac ctgaaccccg acaacagcga cgtggacaag 1860

ctgttcatcc agctggtgca gacctacaac cagctgttcg aggaaaaccc catcaacgcc 1920

agcggcgtgg acgccaaggc catcctgtct gccagactga gcaagagcag acggctggaa 1980

aatctgatcg cccagctgcc cggcgagaag aagaatggcc tgttcggcaa cctgattgcc 2040

ctgagcctgg gcctgacccc caacttcaag agcaacttcg acctggccga ggatgccaaa 2100

ctgcagctga gcaaggacac ctacgacgac gacctggaca acctgctggc ccagatcggc 2160

gaccagtacg ccgacctgtt tctggccgcc aagaacctgt ccgacgccat cctgctgagc 2220

gacatcctga gagtgaacac cgagatcacc aaggcccccc tgagcgcctc tatgatcaag 2280

agatacgacg agcaccacca ggacctgacc ctgctgaaag ctctcgtgcg gcagcagctg 2340

cctgagaagt acaaagagat tttcttcgac cagagcaaga acggctacgc cggctacatt 2400

gacggcggag ccagccagga agagttctac aagttcatca agcccatcct ggaaaagatg 2460

gacggcaccg aggaactgct cgtgaagctg aacagagagg acctgctgcg gaagcagcgg 2520

accttcgaca acggcagcat cccccaccag atccacctgg gagagctgca cgccattctg 2580

cggcggcagg aagattttta cccattcctg aaggacaacc gggaaaagat cgagaagatc 2640

ctgaccttcc gcatccccta ctacgtgggc cctctggcca ggggaaacag cagattcgcc 2700

tggatgacca gaaagagcga ggaaaccatc accccctgga acttcgagga agtggtggac 2760

aagggcgctt ccgcccagag cttcatcgag cggatgacca acttcgataa gaacctgccc 2820

aacgagaagg tgctgcccaa gcacagcctg ctgtacgagt acttcaccgt gtataacgag 2880

ctgaccaaag tgaaatacgt gaccgaggga atgagaaagc ccgccttcct gagcggcgag 2940

cagaaaaagg ccatcgtgga cctgctgttc aagaccaacc ggaaagtgac cgtgaagcag 3000

ctgaaagagg actacttcaa gaaaatcgag tgcttcgact ccgtggaaat ctccggcgtg 3060

gaagatcggt tcaacgcctc cctgggcaca taccacgatc tgctgaaaat tatcaaggac 3120

aaggacttcc tggacaatga ggaaaacgag gacattctgg aagatatcgt gctgaccctg 3180

acactgtttg aggacagaga gatgatcgag gaacggctga aaacctatgc ccacctgttc 3240

gacgacaaag tgatgaagca gctgaagcgg cggagataca ccggctgggg caggctgagc 3300

cggaagctga tcaacggcat ccgggacaag cagtccggca agacaatcct ggatttcctg 3360

aagtccgacg gcttcgccaa cagaaacttc atgcagctga tccacgacga cagcctgacc 3420

tttaaagagg acatccagaa agcccaggtg tccggccagg gcgatagcct gcacgagcac 3480

attgccaatc tggccggcag ccccgccatt aagaagggca tcctgcagac agtgaaggtg 3540

gtggacgagc tcgtgaaagt gatgggccgg cacaagcccg agaacatcgt gatcgaaatg 3600

gccagagaga accagaccac ccagaaggga cagaagaaca gccgcgagag aatgaagcgg 3660

atcgaagagg gcatcaaaga gctgggcagc cagatcctga aagaacaccc cgtggaaaac 3720

acccagctgc agaacgagaa gctgtacctg tactacctgc agaatgggcg ggatatgtac 3780

gtggaccagg aactggacat caaccggctg tccgactacg atgtggacca tatcgtgcct 3840

cagagctttc tgaaggacga ctccatcgac aacaaggtgc tgaccagaag cgacaagaac 3900

cggggcaaga gcgacaacgt gccctccgaa gaggtcgtga agaagatgaa gaactactgg 3960

cggcagctgc tgaacgccaa gctgattacc cagagaaagt tcgacaatct gaccaaggcc 4020

gagagaggcg gcctgagcga actggataag gccggcttca tcaagagaca gctggtggaa 4080

acccggcaga tcacaaagca cgtggcacag atcctggact cccggatgaa cactaagtac 4140

gacgagaatg acaagctgat ccgggaagtg aaagtgatca ccctgaagtc caagctggtg 4200

tccgatttcc ggaaggattt ccagttttac aaagtgcgcg agatcaacaa ctaccaccac 4260

gcccacgacg cctacctgaa cgccgtcgtg ggaaccgccc tgatcaaaaa gtaccctaag 4320

ctggaaagcg agttcgtgta cggcgactac aaggtgtacg acgtgcggaa gatgatcgcc 4380

aagagcgagc aggaaatcgg caaggctacc gccaagtact tcttctacag caacatcatg 4440

aactttttca agaccgagat taccctggcc aacggcgaga tccggaagcg gcctctgatc 4500

gagacaaacg gcgaaaccgg ggagatcgtg tgggataagg gccgggattt tgccaccgtg 4560

cggaaagtgc tgagcatgcc ccaagtgaat atcgtgaaaa agaccgaggt gcagacaggc 4620

ggcttcagca aagagtctat cctgcccaag aggaacagcg ataagctgat cgccagaaag 4680

aaggactggg accctaagaa gtacggcggc ttcgacagcc ccaccgtggc ctattctgtg 4740

ctggtggtgg ccaaagtgga aaagggcaag tccaagaaac tgaagagtgt gaaagagctg 4800

ctggggatca ccatcatgga aagaagcagc ttcgagaaga atcccatcga ctttctggaa 4860

gccaagggct acaaagaagt gaaaaaggac ctgatcatca agctgcctaa gtactccctg 4920

ttcgagctgg aaaacggccg gaagagaatg ctggcctctg ccggcgaact gcagaaggga 4980

aacgaactgg ccctgccctc caaatatgtg aacttcctgt acctggccag ccactatgag 5040

aagctgaagg gctcccccga ggataatgag cagaaacagc tgtttgtgga acagcacaag 5100

cactacctgg acgagatcat cgagcagatc agcgagttct ccaagagagt gatcctggcc 5160

gacgctaatc tggacaaagt gctgtccgcc tacaacaagc accgggataa gcccatcaga 5220

gagcaggccg agaatatcat ccacctgttt accctgacca atctgggagc ccctgccgcc 5280

ttcaagtact ttgacaccac catcgaccgg aagaggtaca ccagcaccaa agaggtgctg 5340

gacgccaccc tgatccacca gagcatcacc ggcctgtacg agacacggat cgacctgtct 5400

cagctgggag gcgacagccc caagaagaag agaaaggtgg aggccagcta agaattccta 5460

gagctcgctg atcagcctcg actgtgcctt ctagttgcca gccatctgtt gtttgcccct 5520

cccccgtgcc ttccttgacc ctggaaggtg ccactcccac tgtcctttcc taataaaatg 5580

aggaaattgc atcgcattgt ctgagtaggt gtcattctat tctggggggt ggggtggggc 5640

aggacagcaa gggggaggat tgggaagaga atagcaggca tgctggggag cggccgcagg 5700

aacccctagt gatggagttg gccactccct ctctgcgcgc tcgctcgctc actgaggccg 5760

ggcgaccaaa ggtcgcccga cgcccgggct ttgcccgggc ggcctcagtg agcgagcgag 5820

cgcgcagctg cctgcagggg cgcctgatgc ggtattttct ccttacgcat ctgtgcggta 5880

tttcacaccg catacgtcaa agcaaccata gtacgcgccc tgtagcggcg cattaagcgc 5940

ggcgggtgtg gtggttacgc gcagcgtgac cgctacactt gccagcgccc tagcgcccgc 6000

tcctttcgct ttcttccctt cctttctcgc cacgttcgcc ggctttcccc gtcaagctct 6060

aaatcggggg ctccctttag ggttccgatt tagtgcttta cggcacctcg accccaaaaa 6120

acttgatttg ggtgatggtt cacgtagtgg gccatcgccc tgatagacgg tttttcgccc 6180

tttgacgttg gagtccacgt tctttaatag tggactcttg ttccaaactg gaacaacact 6240

caaccctatc tcgggctatt cttttgattt ataagggatt ttgccgattt cggcctattg 6300

gttaaaaaat gagctgattt aacaaaaatt taacgcgaat tttaacaaaa tattaacgtt 6360

tacaatttta tggtgcactc tcagtacaat ctgctctgat gccgcatagt taagccagcc 6420

ccgacacccg ccaacacccg ctgacgcgcc ctgacgggct tgtctgctcc cggcatccgc 6480

ttacagacaa gctgtgaccg tctccgggag ctgcatgtgt cagaggtttt caccgtcatc 6540

accgaaacgc gcgagacgaa agggcctcgt gatacgccta tttttatagg ttaatgtcat 6600

gataataatg gtttcttaga cgtcaggtgg cacttttcgg ggaaatgtgc gcggaacccc 6660

tatttgttta tttttctaaa tacattcaaa tatgtatccg ctcatgagac aataaccctg 6720

ataaatgctt caataatatt gaaaaaggaa gagtatgagt attcaacatt tccgtgtcgc 6780

ccttattccc ttttttgcgg cattttgcct tcctgttttt gctcacccag aaacgctggt 6840

gaaagtaaaa gatgctgaag atcagttggg tgcacgagtg ggttacatcg aactggatct 6900

caacagcggt aagatccttg agagttttcg ccccgaagaa cgttttccaa tgatgagcac 6960

ttttaaagtt ctgctatgtg gcgcggtatt atcccgtatt gacgccgggc aagagcaact 7020

cggtcgccgc atacactatt ctcagaatga cttggttgag tactcaccag tcacagaaaa 7080

gcatcttacg gatggcatga cagtaagaga attatgcagt gctgccataa ccatgagtga 7140

taacactgcg gccaacttac ttctgacaac gatcggagga ccgaaggagc taaccgcttt 7200

tttgcacaac atgggggatc atgtaactcg ccttgatcgt tgggaaccgg agctgaatga 7260

agccatacca aacgacgagc gtgacaccac gatgcctgta gcaatggcaa caacgttgcg 7320

caaactatta actggcgaac tacttactct agcttcccgg caacaattaa tagactggat 7380

ggaggcggat aaagttgcag gaccacttct gcgctcggcc cttccggctg gctggtttat 7440

tgctgataaa tctggagccg gtgagcgtgg aagccgcggt atcattgcag cactggggcc 7500

agatggtaag ccctcccgta tcgtagttat ctacacgacg gggagtcagg caactatgga 7560

tgaacgaaat agacagatcg ctgagatagg tgcctcactg attaagcatt ggtaactgtc 7620

agaccaagtt tactcatata tactttagat tgatttaaaa cttcattttt aatttaaaag 7680

gatctaggtg aagatccttt ttgataatct catgaccaaa atcccttaac gtgagttttc 7740

gttccactga gcgtcagacc ccgtagaaaa gatcaaagga tcttcttgag atcctttttt 7800

tctgcgcgta atctgctgct tgcaaacaaa aaaaccaccg ctaccagcgg tggtttgttt 7860

gccggatcaa gagctaccaa ctctttttcc gaaggtaact ggcttcagca gagcgcagat 7920

accaaatact gtccttctag tgtagccgta gttaggccac cacttcaaga actctgtagc 7980

accgcctaca tacctcgctc tgctaatcct gttaccagtg gctgctgcca gtggcgataa 8040

gtcgtgtctt accgggttgg actcaagacg atagttaccg gataaggcgc agcggtcggg 8100

ctgaacgggg ggttcgtgca cacagcccag cttggagcga acgacctaca ccgaactgag 8160

atacctacag cgtgagctat gagaaagcgc cacgcttccc gaagggagaa aggcggacag 8220

gtatccggta agcggcaggg tcggaacagg agagcgcacg agggagcttc cagggggaaa 8280

cgcctggtat ctttatagtc ctgtcgggtt tcgccacctc tgacttgagc gtcgattttt 8340

gtgatgctcg tcaggggggc ggagcctatg gaaaaacgcc agcaacgcgg cctttttacg 8400

gttcctggcc ttttgctggc cttttgctca catgt 8435

<210> 146
<211> 8435
<212> DNA
<213> Artificial Sequence

<220>
<223> synthetic DNA

<400> 146
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg actcaccatg gtggcggagt tttagagcta gaaatagcaa gttaaaataa 300

ggctagtccg ttatcaactt gaaaaagtgg caccgagtcg gtgctttttt gttttagagc 360

tagaaatagc aagttaaaat aaggctagtc cgtttttagc gcgtgcgcca attctgcaga 420

caaatggctc tagaggtacc cgttacataa cttacggtaa atggcccgcc tggctgaccg 480

cccaacgacc cccgcccatt gacgtcaata gtaacgccaa tagggacttt ccattgacgt 540

caatgggtgg agtatttacg gtaaactgcc cacttggcag tacatcaagt gtatcatatg 600

ccaagtacgc cccctattga cgtcaatgac ggtaaatggc ccgcctggca ttgtgcccag 660

tacatgacct tatgggactt tcctacttgg cagtacatct acgtattagt catcgctatt 720

accatggtcg aggtgagccc cacgttctgc ttcactctcc ccatctcccc cccctcccca 780

cccccaattt tgtatttatt tattttttaa ttattttgtg cagcgatggg ggcggggggg 840

gggggggggc gcgcgccagg cggggcgggg cggggcgagg ggcggggcgg ggcgaggcgg 900

agaggtgcgg cggcagccaa tcagagcggc gcgctccgaa agtttccttt tatggcgagg 960

cggcggcggc ggcggcccta taaaaagcga agcgcgcggc gggcgggagt cgctgcgacg 1020

ctgccttcgc cccgtgcccc gctccgccgc cgcctcgcgc cgcccgcccc ggctctgact 1080

gaccgcgtta ctcccacagg tgagcgggcg ggacggccct tctcctccgg gctgtaatta 1140

gctgagcaag aggtaagggt ttaagggatg gttggttggt ggggtattaa tgtttaatta 1200

cctggagcac ctgcctgaaa tcactttttt tcaggttgga ccggtgccac catgtaccca 1260

tacgatgttc cagattacgc ttcgccgaag aaaaagcgca aggtcgaagc gtccgacaag 1320

aagtacagca tcggcctggc catcggcacc aactctgtgg gctgggccgt gatcaccgac 1380

gagtacaagg tgcccagcaa gaaattcaag gtgctgggca acaccgaccg gcacagcatc 1440

aagaagaacc tgatcggagc cctgctgttc gacagcggcg aaacagccga ggccacccgg 1500

ctgaagagaa ccgccagaag aagatacacc agacggaaga accggatctg ctatctgcaa 1560

gagatcttca gcaacgagat ggccaaggtg gacgacagct tcttccacag actggaagag 1620

tccttcctgg tggaagagga taagaagcac gagcggcacc ccatcttcgg caacatcgtg 1680

gacgaggtgg cctaccacga gaagtacccc accatctacc acctgagaaa gaaactggtg 1740

gacagcaccg acaaggccga cctgcggctg atctatctgg ccctggccca catgatcaag 1800

ttccggggcc acttcctgat cgagggcgac ctgaaccccg acaacagcga cgtggacaag 1860

ctgttcatcc agctggtgca gacctacaac cagctgttcg aggaaaaccc catcaacgcc 1920

agcggcgtgg acgccaaggc catcctgtct gccagactga gcaagagcag acggctggaa 1980

aatctgatcg cccagctgcc cggcgagaag aagaatggcc tgttcggcaa cctgattgcc 2040

ctgagcctgg gcctgacccc caacttcaag agcaacttcg acctggccga ggatgccaaa 2100

ctgcagctga gcaaggacac ctacgacgac gacctggaca acctgctggc ccagatcggc 2160

gaccagtacg ccgacctgtt tctggccgcc aagaacctgt ccgacgccat cctgctgagc 2220

gacatcctga gagtgaacac cgagatcacc aaggcccccc tgagcgcctc tatgatcaag 2280

agatacgacg agcaccacca ggacctgacc ctgctgaaag ctctcgtgcg gcagcagctg 2340

cctgagaagt acaaagagat tttcttcgac cagagcaaga acggctacgc cggctacatt 2400

gacggcggag ccagccagga agagttctac aagttcatca agcccatcct ggaaaagatg 2460

gacggcaccg aggaactgct cgtgaagctg aacagagagg acctgctgcg gaagcagcgg 2520

accttcgaca acggcagcat cccccaccag atccacctgg gagagctgca cgccattctg 2580

cggcggcagg aagattttta cccattcctg aaggacaacc gggaaaagat cgagaagatc 2640

ctgaccttcc gcatccccta ctacgtgggc cctctggcca ggggaaacag cagattcgcc 2700

tggatgacca gaaagagcga ggaaaccatc accccctgga acttcgagga agtggtggac 2760

aagggcgctt ccgcccagag cttcatcgag cggatgacca acttcgataa gaacctgccc 2820

aacgagaagg tgctgcccaa gcacagcctg ctgtacgagt acttcaccgt gtataacgag 2880

ctgaccaaag tgaaatacgt gaccgaggga atgagaaagc ccgccttcct gagcggcgag 2940

cagaaaaagg ccatcgtgga cctgctgttc aagaccaacc ggaaagtgac cgtgaagcag 3000

ctgaaagagg actacttcaa gaaaatcgag tgcttcgact ccgtggaaat ctccggcgtg 3060

gaagatcggt tcaacgcctc cctgggcaca taccacgatc tgctgaaaat tatcaaggac 3120

aaggacttcc tggacaatga ggaaaacgag gacattctgg aagatatcgt gctgaccctg 3180

acactgtttg aggacagaga gatgatcgag gaacggctga aaacctatgc ccacctgttc 3240

gacgacaaag tgatgaagca gctgaagcgg cggagataca ccggctgggg caggctgagc 3300

cggaagctga tcaacggcat ccgggacaag cagtccggca agacaatcct ggatttcctg 3360

aagtccgacg gcttcgccaa cagaaacttc atgcagctga tccacgacga cagcctgacc 3420

tttaaagagg acatccagaa agcccaggtg tccggccagg gcgatagcct gcacgagcac 3480

attgccaatc tggccggcag ccccgccatt aagaagggca tcctgcagac agtgaaggtg 3540

gtggacgagc tcgtgaaagt gatgggccgg cacaagcccg agaacatcgt gatcgaaatg 3600

gccagagaga accagaccac ccagaaggga cagaagaaca gccgcgagag aatgaagcgg 3660

atcgaagagg gcatcaaaga gctgggcagc cagatcctga aagaacaccc cgtggaaaac 3720

acccagctgc agaacgagaa gctgtacctg tactacctgc agaatgggcg ggatatgtac 3780

gtggaccagg aactggacat caaccggctg tccgactacg atgtggacca tatcgtgcct 3840

cagagctttc tgaaggacga ctccatcgac aacaaggtgc tgaccagaag cgacaagaac 3900

cggggcaaga gcgacaacgt gccctccgaa gaggtcgtga agaagatgaa gaactactgg 3960

cggcagctgc tgaacgccaa gctgattacc cagagaaagt tcgacaatct gaccaaggcc 4020

gagagaggcg gcctgagcga actggataag gccggcttca tcaagagaca gctggtggaa 4080

acccggcaga tcacaaagca cgtggcacag atcctggact cccggatgaa cactaagtac 4140

gacgagaatg acaagctgat ccgggaagtg aaagtgatca ccctgaagtc caagctggtg 4200

tccgatttcc ggaaggattt ccagttttac aaagtgcgcg agatcaacaa ctaccaccac 4260

gcccacgacg cctacctgaa cgccgtcgtg ggaaccgccc tgatcaaaaa gtaccctaag 4320

ctggaaagcg agttcgtgta cggcgactac aaggtgtacg acgtgcggaa gatgatcgcc 4380

aagagcgagc aggaaatcgg caaggctacc gccaagtact tcttctacag caacatcatg 4440

aactttttca agaccgagat taccctggcc aacggcgaga tccggaagcg gcctctgatc 4500

gagacaaacg gcgaaaccgg ggagatcgtg tgggataagg gccgggattt tgccaccgtg 4560

cggaaagtgc tgagcatgcc ccaagtgaat atcgtgaaaa agaccgaggt gcagacaggc 4620

ggcttcagca aagagtctat cctgcccaag aggaacagcg ataagctgat cgccagaaag 4680

aaggactggg accctaagaa gtacggcggc ttcgacagcc ccaccgtggc ctattctgtg 4740

ctggtggtgg ccaaagtgga aaagggcaag tccaagaaac tgaagagtgt gaaagagctg 4800

ctggggatca ccatcatgga aagaagcagc ttcgagaaga atcccatcga ctttctggaa 4860

gccaagggct acaaagaagt gaaaaaggac ctgatcatca agctgcctaa gtactccctg 4920

ttcgagctgg aaaacggccg gaagagaatg ctggcctctg ccggcgaact gcagaaggga 4980

aacgaactgg ccctgccctc caaatatgtg aacttcctgt acctggccag ccactatgag 5040

aagctgaagg gctcccccga ggataatgag cagaaacagc tgtttgtgga acagcacaag 5100

cactacctgg acgagatcat cgagcagatc agcgagttct ccaagagagt gatcctggcc 5160

gacgctaatc tggacaaagt gctgtccgcc tacaacaagc accgggataa gcccatcaga 5220

gagcaggccg agaatatcat ccacctgttt accctgacca atctgggagc ccctgccgcc 5280

ttcaagtact ttgacaccac catcgaccgg aagaggtaca ccagcaccaa agaggtgctg 5340

gacgccaccc tgatccacca gagcatcacc ggcctgtacg agacacggat cgacctgtct 5400

cagctgggag gcgacagccc caagaagaag agaaaggtgg aggccagcta agaattccta 5460

gagctcgctg atcagcctcg actgtgcctt ctagttgcca gccatctgtt gtttgcccct 5520

cccccgtgcc ttccttgacc ctggaaggtg ccactcccac tgtcctttcc taataaaatg 5580

aggaaattgc atcgcattgt ctgagtaggt gtcattctat tctggggggt ggggtggggc 5640

aggacagcaa gggggaggat tgggaagaga atagcaggca tgctggggag cggccgcagg 5700

aacccctagt gatggagttg gccactccct ctctgcgcgc tcgctcgctc actgaggccg 5760

ggcgaccaaa ggtcgcccga cgcccgggct ttgcccgggc ggcctcagtg agcgagcgag 5820

cgcgcagctg cctgcagggg cgcctgatgc ggtattttct ccttacgcat ctgtgcggta 5880

tttcacaccg catacgtcaa agcaaccata gtacgcgccc tgtagcggcg cattaagcgc 5940

ggcgggtgtg gtggttacgc gcagcgtgac cgctacactt gccagcgccc tagcgcccgc 6000

tcctttcgct ttcttccctt cctttctcgc cacgttcgcc ggctttcccc gtcaagctct 6060

aaatcggggg ctccctttag ggttccgatt tagtgcttta cggcacctcg accccaaaaa 6120

acttgatttg ggtgatggtt cacgtagtgg gccatcgccc tgatagacgg tttttcgccc 6180

tttgacgttg gagtccacgt tctttaatag tggactcttg ttccaaactg gaacaacact 6240

caaccctatc tcgggctatt cttttgattt ataagggatt ttgccgattt cggcctattg 6300

gttaaaaaat gagctgattt aacaaaaatt taacgcgaat tttaacaaaa tattaacgtt 6360

tacaatttta tggtgcactc tcagtacaat ctgctctgat gccgcatagt taagccagcc 6420

ccgacacccg ccaacacccg ctgacgcgcc ctgacgggct tgtctgctcc cggcatccgc 6480

ttacagacaa gctgtgaccg tctccgggag ctgcatgtgt cagaggtttt caccgtcatc 6540

accgaaacgc gcgagacgaa agggcctcgt gatacgccta tttttatagg ttaatgtcat 6600

gataataatg gtttcttaga cgtcaggtgg cacttttcgg ggaaatgtgc gcggaacccc 6660

tatttgttta tttttctaaa tacattcaaa tatgtatccg ctcatgagac aataaccctg 6720

ataaatgctt caataatatt gaaaaaggaa gagtatgagt attcaacatt tccgtgtcgc 6780

ccttattccc ttttttgcgg cattttgcct tcctgttttt gctcacccag aaacgctggt 6840

gaaagtaaaa gatgctgaag atcagttggg tgcacgagtg ggttacatcg aactggatct 6900

caacagcggt aagatccttg agagttttcg ccccgaagaa cgttttccaa tgatgagcac 6960

ttttaaagtt ctgctatgtg gcgcggtatt atcccgtatt gacgccgggc aagagcaact 7020

cggtcgccgc atacactatt ctcagaatga cttggttgag tactcaccag tcacagaaaa 7080

gcatcttacg gatggcatga cagtaagaga attatgcagt gctgccataa ccatgagtga 7140

taacactgcg gccaacttac ttctgacaac gatcggagga ccgaaggagc taaccgcttt 7200

tttgcacaac atgggggatc atgtaactcg ccttgatcgt tgggaaccgg agctgaatga 7260

agccatacca aacgacgagc gtgacaccac gatgcctgta gcaatggcaa caacgttgcg 7320

caaactatta actggcgaac tacttactct agcttcccgg caacaattaa tagactggat 7380

ggaggcggat aaagttgcag gaccacttct gcgctcggcc cttccggctg gctggtttat 7440

tgctgataaa tctggagccg gtgagcgtgg aagccgcggt atcattgcag cactggggcc 7500

agatggtaag ccctcccgta tcgtagttat ctacacgacg gggagtcagg caactatgga 7560

tgaacgaaat agacagatcg ctgagatagg tgcctcactg attaagcatt ggtaactgtc 7620

agaccaagtt tactcatata tactttagat tgatttaaaa cttcattttt aatttaaaag 7680

gatctaggtg aagatccttt ttgataatct catgaccaaa atcccttaac gtgagttttc 7740

gttccactga gcgtcagacc ccgtagaaaa gatcaaagga tcttcttgag atcctttttt 7800

tctgcgcgta atctgctgct tgcaaacaaa aaaaccaccg ctaccagcgg tggtttgttt 7860

gccggatcaa gagctaccaa ctctttttcc gaaggtaact ggcttcagca gagcgcagat 7920

accaaatact gtccttctag tgtagccgta gttaggccac cacttcaaga actctgtagc 7980

accgcctaca tacctcgctc tgctaatcct gttaccagtg gctgctgcca gtggcgataa 8040

gtcgtgtctt accgggttgg actcaagacg atagttaccg gataaggcgc agcggtcggg 8100

ctgaacgggg ggttcgtgca cacagcccag cttggagcga acgacctaca ccgaactgag 8160

atacctacag cgtgagctat gagaaagcgc cacgcttccc gaagggagaa aggcggacag 8220

gtatccggta agcggcaggg tcggaacagg agagcgcacg agggagcttc cagggggaaa 8280

cgcctggtat ctttatagtc ctgtcgggtt tcgccacctc tgacttgagc gtcgattttt 8340

gtgatgctcg tcaggggggc ggagcctatg gaaaaacgcc agcaacgcgg cctttttacg 8400

gttcctggcc ttttgctggc cttttgctca catgt 8435

<210> 147
<211> 30
<212> DNA
<213> Artificial Sequence

<220>
<223> oligonucleotide

<400> 147
agaccaggtt agccttgaac aaagatctcc 30

<210> 148
<211> 6681
<212> DNA

<213>  Artificial Sequence

<220>
<223>  synthetic DNA

<400>  148
tatagtgagt cgtattacaa ttcactggcc gtcgttttac aacgtcgtga ctgggaaaac      60

cctggcgtta cccaacttaa tcgccttgca gcacatcccc ctttcgccag ctggcgtaat     120

agcgaagagg cccgcaccga tcgcccttcc caacagttgc gcagcctgaa tggcgaatgg     180

acgcgccctg tagcggcgca ttaagcgcgg cgggtgtggt ggttacgcgc agcgtgaccg     240

ctacacttgc cagcgcccta gcgcccgctc ctttcgcttt cttcccttcc tttctcgcca     300

cgttcgccgg ctttccccgt caagctctaa atcgggggct ccctttaggg ttccgattta     360

gtgctttacg gcacctcgac cccaaaaaac ttgattaggg tgatggttca cgtagtgggc     420

catcgccctg atagacggtt tttcgccctt tgacgttgga gtccacgttc tttaatagtg     480

gactcttgtt ccaaactgga acaacactca accctatctc ggtctattct tttgatttat     540

aagggatttt gccgatttcg gcctattggt taaaaaatga gctgatttaa caaaaattta     600

acgcgaattt taacaaaata ttaacgctta caatttcctg atgcggtatt ttctccttac     660

gcatctgtgc ggtatttcac accgcatcag gtggcacttt tcggggaaat gtgcgcggaa     720

cccctatttg tttatttttc taaatacatt caaatatgta tccgctcatg agacaataac     780

cctgataaat gcttcaataa tattgaaaaa ggaagagtat gagtattcaa catttccgtg     840

tcgcccttat tccctttttt gcggcatttt gccttcctgt ttttgctcac ccagaaacgc     900

tggtgaaagt aaaagatgct gaagatcagt tgggtgcacg agtgggttac atcgaactgg     960

atctcaacag cggtaagatc cttgagagtt ttcgccccga agaacgtttt ccaatgatga    1020

gcacttttaa agttctgcta tgtggcgcgg tattatcccg tattgacgcc gggcaagagc    1080

aactcggtcg ccgcatacac tattctcaga atgacttggt tgagtactca ccagtcacag    1140

aaaagcatct tacggatggc atgacagtaa gagaattatg cagtgctgcc ataaccatga    1200

gtgataacac tgcggccaac ttacttctga caacgatcgg aggaccgaag gagctaaccg    1260

cttttttgca caacatgggg gatcatgtaa ctcgccttga tcgttgggaa ccggagctga    1320

atgaagccat accaaacgac gagcgtgaca ccacgatgcc tgtagcaatg gcaacaacgt    1380

tgcgcaaact attaactggc gaactactta ctctagcttc ccggcaacaa ttaatagact    1440

ggatggaggc ggataaagtt gcaggaccac ttctgcgctc ggcccttccg gctggctggt    1500

ttattgctga taaatctgga gccggtgagc gtgggtctcg cggtatcatt gcagcactgg    1560

ggccagatgg taagccctcc cgtatcgtag ttatctacac gacggggagt caggcaacta    1620

tggatgaacg aaatagacag atcgctgaga taggtgcctc actgattaag cattggtaac    1680

tgtcagacca agtttactca tatatacttt agattgattt aaaacttcat ttttaattta    1740

aaaggatcta ggtgaagatc ctttttgata atctcatgac caaaatccct taacgtgagt 1800

tttcgttcca ctgagcgtca gaccccgtag aaaagatcaa aggatcttct tgagatcctt 1860

tttttctgcg cgtaatctgc tgcttgcaaa caaaaaaacc accgctacca gcggtggttt 1920

gtttgccgga tcaagagcta ccaactcttt ttccgaaggt aactggcttc agcagagcgc 1980

agataccaaa tactgttctt ctagtgtagc cgtagttagg ccaccacttc aagaactctg 2040

tagcaccgcc tacatacctc gctctgctaa tcctgttacc agtggctgct gccagtggcg 2100

ataagtcgtg tcttaccggg ttggactcaa gacgatagtt accggataag gcgcagcggt 2160

cgggctgaac ggggggttcg tgcacacagc ccagcttgga gcgaacgacc tacaccgaac 2220

tgagatacct acagcgtgag ctatgagaaa gcgccacgct tcccgaaggg agaaaggcgg 2280

acaggtatcc ggtaagcggc agggtcggaa caggagagcg cacgagggag cttccagggg 2340

gaaacgcctg gtatctttat agtcctgtcg ggtttcgcca cctctgactt gagcgtcgat 2400

ttttgtgatg ctcgtcaggg gggcggagcc tatggaaaaa cgccagcaac gcggcctttt 2460

tacggttcct ggccttttgc tggccttttg ctcacatgtt ctttcctgcg ttatcccctg 2520

attctgtgga taaccgtatt accgcctttg agtgagctga taccgctcgc cgcagccgaa 2580

cgaccgagcg cagcgagtca gtgagcgagg aagcggaaga gcgcccaata cgcaaaccgc 2640

ctctccccgc gcgttggccg attcattaat gcagctggca cgacaggttt cccgactgga 2700

aagcgggcag tgagcgcaac gcaattaatg tgagttagct cactcattag gcaccccagg 2760

ctttacactt tatgcttccg gctcgtatgt tgtgtggaat tgtgagcgga taacaatttc 2820

acacaggaaa cagctatgac catgattacg ccaagctatt taggtgacac tatagaatac 2880

tcaagctatg catccaacgc gttgggagct ctcccatatg gtcgacctgc aggcggccgc 2940

gaattcacta gtgattaccg ttcgtatagc atacattata cgaagttata ccatgtctag 3000

actggacaag agcaaagtca taaacggcgc tctggaatta ctcaatggag tcggtatcga 3060

aggcctgacg acaaggaaac tcgctcaaaa gctgggagtt gagcagccta ccctgtactg 3120

gcacgtgaag aacaagcggg ccctgctcga tgccctgcca atcgagatgc tggacaggca 3180

tcatacccac ttctgccccc tggaaggcga gtcatggcaa gactttctgc ggaacaacgc 3240

caagtcattc cgctgtgctc tcctctcaca tcgcgacggg gctaaagtgc atctcggcac 3300

ccgcccaaca gagaaacagt acgaaaccct ggaaaatcag ctcgcgttcc tgtgtcagca 3360

aggcttctcc ctggagaacg cactgtacgc tctgtccgcc gtgggccact ttacactggg 3420

ctgcgtattg gaggaacagg agcatcaagt agcaaaagag gaaagagaga cacctaccac 3480

cgattctatg cccccacttc tgagacaagc aattgagctg ttcgaccggc agggagccga 3540

acctgccttc cttttcggcc tggaactaat catatgtggc ctggagaaac agctaaagtg 3600

cgaaagcggc gggccggccg acgcccttga cgattttgac ttagacatgc tcccagccga 3660

tgcccttgac gactttgacc ttgatatgct gcctgctgac gctcttgacg attttgacct 3720

tgacatgctc cccgggtaac taagtaagga tcccaaatac cactgaatta agaattccgc 3780

ccctctccct cccccccccc taacgttact ggccgaagcc gcttggaata aggccggtgt 3840

gcgtttgtct atatgttatt ttccaccata ttgccgtctt ttggcaatgt gagggcccgg 3900

aaacctggcc ctgtcttctt gacgagcatt cctaggggtc tttcccctct cgccaaagga 3960

atgcaaggtc tgttgaatgt cgtgaaggaa gcagttcctc tggaagcttc ttgaagacaa 4020

acaacgtctg tagcgaccct ttgcaggcag cggaaccccc cacctggcga caggtgcctc 4080

tgcggccaaa agccacgtgt ataagataca cctgcaaagg cggcacaacc ccagtgccac 4140

gttgtgagtt ggatagttgt ggaaagagtc aaatggctct cctcaagcgt attcaacaag 4200

gggctgaagg atgcccagaa ggtaccccat tgtatgggat ctgatctggg gcctcggtgc 4260

acatgcttta catgtgttta gtcgaggtta aaaaacgtct aggccccccg aaccacgggg 4320

acgtggtttt cctttgaaaa acacgatgat aatatggcca caaccatgac cgagtacaag 4380

cccacggtgc gcctcgccac ccgcgacgac gtcccccggg ccgtacgcac cctcgccgcc 4440

gcgttcgccg actaccccgc cacgcgccac accgtcgacc cggaccgcca catcgagcgg 4500

gtcaccgagc tgcaagaact cttcctcacg cgcgtcgggc tcgacatcgg caaggtgtgg 4560

gtcgcggacg acggcgccgc ggtggcggtc tggaccacgc cggagagcgt cgaagcgggg 4620

gcggtgttcg ccgagatcgg cccgcgcatg gccgagttga gcggttcccg gctggccgcg 4680

cagcaacaga tggaaggcct cctggcgccg caccggccca aggagcccgc gtggttcctg 4740

gccaccgtcg gcgtctcgcc cgaccaccag ggcaagggtc tgggcagcgc cgtcgtgctc 4800

cccggagtgg aggcggccga gcgcgccggg gtgcccgcct tcctggagac ctccgcgccc 4860

cgcaacctcc ccttctacga gcggctcggc ttcaccgtca ccgccgacgt cgaggtgccc 4920

gaaggaccgc gcacctggtg catgacccgc aagcccggtg cctgaggatc cagacatgat 4980

aagatacatt gatgagtttg gacaaaccac aactagaatg cagtgaaaaa aatgctttat 5040

ttgtgaaatt tgtgatgcta ttgctttatt tgtaaccatt ataagctgca ataaacaagt 5100

taacaacaac aattgcattc attttatgtt tcaggttcag ggggaggtgt gggaggtttt 5160

ttaaagcaag taaaacctct acaaatgtgg tatggctgat tatgatcctc tagagtcgca 5220

gatccagaca tgataagata cattgatgag tttggacaaa ccacaactag aatgcagtga 5280

aaaaaatgct ttatttgtga aatttgtgat gctattgctt tatttgtaac cattataagc 5340

tgcaataaac aagttaacaa caacaattgc attcatttta tgtttcaggt tcagggggag 5400

gtgtgggagg ttttttaaag caagtaaaac ctctacaaat gtggtatggc tgattatgat 5460

cctctagagt cgcagatcca gacatgataa gatacattga tgagtttgga caaaccacaa 5520

ctagaatgca gtgaaaaaaa tgctttattt gtgaaatttg tgatgctatt gctttatttg 5580

taaccattat aagctgcaat aaacaagtta acaacaacaa ttgcattcat tttatgtttc 5640

aggttcaggg ggaggtgtgg gaggtttttt aaagcaagta aaacctctac aaatgtggta 5700

tggctgatta tgatcctcta gagtcgcaga tcctctagag tcgcagatct ttttccctct 5760

gccaaaaatt atggggacat catgaagccc cttgagcatc tgacttctgg ctaataaagg 5820

aaatttattt tcattgcaat agtgtgttgg aattttttgt gtctctcact cggaaggaca 5880

tatgggaggg caaatcattt aaaacatcag aatgagtatt tggtttagag tttggcaaca 5940

tatgccatat gctggctgcc atgaacaaag gtggctataa agaggtcatc agtatatgaa 6000

acagccccct gctgtccatt ccttattcca tagaaaagcc ttgacttgag gttagatttt 6060

ttttatattt tgttttgtgt tatttttttc tttaacatcc ctaaaatttt ccttacatgt 6120

tttactagcc agatttttcc tcctctcctg actactccca gtcatagctg tccctcttct 6180

cttatgaaga tccctcgacc tgcagcccaa gcttatcgaa ttcccgcggt ggcggccgca 6240

cgtctcccta tcagtgatag agaagtcgac acgtctcgag ctccctatca gtgatagaga 6300

aggtacgtct agaacgtctc cctatcagtg atagagaagt cgacacgtct cgagctccct 6360

atcagtgata gagaaggtac gtctagaacg tctccctatc agtgatagag aagtcgacac 6420

gtctcgagct ccctatcagt gatagagaag gtacgtctag aacgtctccc tatcagtgat 6480

agagaagtcg acacgtctcg agctccctat cagtgataga gaaggtaccc cctatataag 6540

cagagctcgt ttagtgaacc gtcagatcgc ctggagacgc catccacgct gttttgacct 6600

ccatagaaga caccgggacc gatccagcct ccgcggccgc catggcggcc gggagcatgc 6660

gacgtcgggc ccaattcgcc c 6681

<210> 149
<211> 8434
<212> DNA
<213> Artificial Sequence

<220>
<223> synthetic DNA

<400> 149
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg ggtcttcgag aagacctgtt ttagagctag aaatagcaag ttaaaataag 300

gctagtccgt tatcaacttg aaaaagtggc accgagtcgg tgcttttttg ttttagagct 360

agaaatagca agttaaaata aggctagtcc gtttttagcg cgtgcgccaa ttctgcagac 420

aaatggctct agaggtaccc gttacataac ttacggtaaa tggcccgcct ggctgaccgc 480

ccaacgaccc ccgcccattg acgtcaatag taacgccaat agggactttc cattgacgtc 540

aatgggtgga gtatttacgg taaactgccc acttggcagt acatcaagtg tatcatatgc 600

caagtacgcc ccctattgac gtcaatgacg gtaaatggcc cgcctggcat tgtgcccagt 660

acatgacctt atgggacttt cctacttggc agtacatcta cgtattagtc atcgctatta 720

ccatggtcga ggtgagcccc acgttctgct tcactctccc catctccccc ccctccccac 780

ccccaatttt gtatttattt attttttaat tattttgtgc agcgatgggg gcgggggggg 840

ggggggggcg cgcgccaggc ggggcggggc ggggcgaggg gcggggcggg gcgaggcgga 900

gaggtgcggc ggcagccaat cagagcggcg cgctccgaaa gtttcctttt atggcgaggc 960

ggcggcggcg gcggccctat aaaaagcgaa gcgcgcggcg ggcgggagtc gctgcgacgc 1020

tgccttcgcc ccgtgccccg ctccgccgcc gcctcgcgcc gcccgccccg gctctgactg 1080

accgcgttac tcccacaggt gagcgggcgg gacggccctt ctcctccggg ctgtaattag 1140

ctgagcaaga ggtaagggtt taagggatgg ttggttggtg gggtattaat gtttaattac 1200

ctggagcacc tgcctgaaat cacttttttt caggttggac cggtgccacc atgtacccat 1260

acgatgttcc agattacgct tcgccgaaga aaaagcgcaa ggtcgaagcg tccgacaaga 1320

agtacagcat cggcctggcc atcggcacca actctgtggg ctgggccgtg atcaccgacg 1380

agtacaaggt gcccagcaag aaattcaagg tgctgggcaa caccgaccgg cacagcatca 1440

agaagaacct gatcggagcc ctgctgttcg acagcggcga aacagccgag gccacccggc 1500

tgaagagaac cgccagaaga agatacacca gacggaagaa ccggatctgc tatctgcaag 1560

agatcttcag caacgagatg gccaaggtgg acgacagctt cttccacaga ctggaagagt 1620

ccttcctggt ggaagaggat aagaagcacg agcggcaccc catcttcggc aacatcgtgg 1680

acgaggtggc ctaccacgag aagtacccca ccatctacca cctgagaaag aaactggtgg 1740

acagcaccga caaggccgac ctgcggctga tctatctggc cctggcccac atgatcaagt 1800

tccggggcca cttcctgatc gagggcgacc tgaaccccga caacagcgac gtggacaagc 1860

tgttcatcca gctggtgcag acctacaacc agctgttcga ggaaaacccc atcaacgcca 1920

gcggcgtgga cgccaaggcc atcctgtctg ccagactgag caagagcaga cggctggaaa 1980

atctgatcgc ccagctgccc ggcgagaaga agaatggcct gttcggcaac ctgattgccc 2040

tgagcctggg cctgaccccc aacttcaaga gcaacttcga cctggccgag gatgccaaac 2100

tgcagctgag caaggacacc tacgacgacg acctggacaa cctgctggcc cagatcggcg 2160

accagtacgc cgacctgttt ctggccgcca agaacctgtc cgacgccatc ctgctgagcg 2220

acatcctgag agtgaacacc gagatcacca aggcccccct gagcgcctct atgatcaaga 2280

gatacgacga gcaccaccag gacctgaccc tgctgaaagc tctcgtgcgg cagcagctgc 2340

ctgagaagta caaagagatt ttcttcgacc agagcaagaa cggctacgcc ggctacattg 2400

acggcggagc cagccaggaa gagttctaca agttcatcaa gcccatcctg gaaaagatgg 2460

acggcaccga ggaactgctc gtgaagctga acagagagga cctgctgcgg aagcagcgga 2520

ccttcgacaa cggcagcatc ccccaccaga tccacctggg agagctgcac gccattctgc 2580

ggcggcagga agatttttac ccattcctga aggacaaccg ggaaaagatc gagaagatcc 2640

tgaccttccg catcccctac tacgtgggcc ctctggccag gggaaacagc agattcgcct 2700

ggatgaccag aaagagcgag gaaaccatca ccccctggaa cttcgaggaa gtggtggaca 2760

agggcgcttc cgcccagagc ttcatcgagc ggatgaccaa cttcgataag aacctgccca 2820

acgagaaggt gctgcccaag cacagcctgc tgtacgagta cttcaccgtg tataacgagc 2880

tgaccaaagt gaaatacgtg accgagggaa tgagaaagcc cgccttcctg agcggcgagc 2940

agaaaaaggc catcgtggac ctgctgttca agaccaaccg gaaagtgacc gtgaagcagc 3000

tgaaagagga ctacttcaag aaaatcgagt gcttcgactc cgtggaaatc tccggcgtgg 3060

aagatcggtt caacgcctcc ctgggcacat accacgatct gctgaaaatt atcaaggaca 3120

aggacttcct ggacaatgag gaaaacgagg acattctgga agatatcgtg ctgaccctga 3180

cactgtttga ggacagagag atgatcgagg aacggctgaa aacctatgcc cacctgttcg 3240

acgacaaagt gatgaagcag ctgaagcggc ggagatacac cggctggggc aggctgagcc 3300

ggaagctgat caacggcatc cgggacaagc agtccggcaa gacaatcctg gatttcctga 3360

agtccgacgg cttcgccaac agaaacttca tgcagctgat ccacgacgac agcctgacct 3420

ttaaagagga catccagaaa gcccaggtgt ccggccaggg cgatagcctg cacgagcaca 3480

ttgccaatct ggccggcagc cccgccatta agaagggcat cctgcagaca gtgaaggtgg 3540

tggacgagct cgtgaaagtg atgggccggc acaagcccga gaacatcgtg atcgaaatgg 3600

ccagagagaa ccagaccacc cagaagggac agaagaacag ccgcgagaga atgaagcgga 3660

tcgaagaggg catcaaagag ctgggcagcc agatcctgaa agaacacccc gtggaaaaca 3720

cccagctgca gaacgagaag ctgtacctgt actacctgca gaatgggcgg gatatgtacg 3780

tggaccagga actggacatc aaccggctgt ccgactacga tgtggaccat atcgtgcctc 3840

agagctttct gaaggacgac tccatcgaca acaaggtgct gaccagaagc gacaagaacc 3900

ggggcaagag cgacaacgtg ccctccgaag aggtcgtgaa gaagatgaag aactactggc 3960

ggcagctgct gaacgccaag ctgattaccc agagaaagtt cgacaatctg accaaggccg 4020

agagaggcgg cctgagcgaa ctggataagg ccggcttcat caagagacag ctggtggaaa 4080

cccggcagat cacaaagcac gtggcacaga tcctggactc ccggatgaac actaagtacg 4140

acgagaatga caagctgatc cgggaagtga aagtgatcac cctgaagtcc aagctggtgt 4200

ccgatttccg gaaggatttc cagttttaca aagtgcgcga gatcaacaac taccaccacg 4260

cccacgacgc ctacctgaac gccgtcgtgg gaaccgccct gatcaaaaag taccctaagc 4320

tggaaagcga gttcgtgtac ggcgactaca aggtgtacga cgtgcggaag atgatcgcca 4380

agagcgagca ggaaatcggc aaggctaccg ccaagtactt cttctacagc aacatcatga 4440

actttttcaa gaccgagatt accctggcca acggcgagat ccggaagcgg cctctgatcg 4500

agacaaacgg cgaaaccggg gagatcgtgt gggataaggg ccgggatttt gccaccgtgc 4560

ggaaagtgct gagcatgccc caagtgaata tcgtgaaaaa gaccgaggtg cagacaggcg 4620

gcttcagcaa agagtctatc ctgcccaaga ggaacagcga taagctgatc gccagaaaga 4680

aggactggga ccctaagaag tacggcggct tcgacagccc caccgtggcc tattctgtgc 4740

tggtggtggc caaagtggaa aagggcaagt ccaagaaact gaagagtgtg aaagagctgc 4800

tggggatcac catcatggaa agaagcagct tcgagaagaa tcccatcgac tttctggaag 4860

ccaagggcta caaagaagtg aaaaaggacc tgatcatcaa gctgcctaag tactccctgt 4920

tcgagctgga aaacggccgg aagagaatgc tggcctctgc cggcgaactg cagaagggaa 4980

acgaactggc cctgccctcc aaatatgtga acttcctgta cctggccagc cactatgaga 5040

agctgaaggg ctcccccgag gataatgagc agaaacagct gtttgtggaa cagcacaagc 5100

actacctgga cgagatcatc gagcagatca gcgagttctc caagagagtg atcctggccg 5160

acgctaatct ggacaaagtg ctgtccgcct acaacaagca ccgggataag cccatcagag 5220

agcaggccga gaatatcatc cacctgttta ccctgaccaa tctgggagcc cctgccgcct 5280

tcaagtactt tgacaccacc atcgaccgga agaggtacac cagcaccaaa gaggtgctgg 5340

acgccaccct gatccaccag agcatcaccg gcctgtacga gacacggatc gacctgtctc 5400

agctgggagg cgacagcccc aagaagaaga gaaaggtgga ggccagctaa gaattcctag 5460

agctcgctga tcagcctcga ctgtgccttc tagttgccag ccatctgttg tttgcccctc 5520

ccccgtgcct tccttgaccc tggaaggtgc cactcccact gtcctttcct aataaaatga 5580

ggaaattgca tcgcattgtc tgagtaggtg tcattctatt ctggggggtg gggtggggca 5640

ggacagcaag ggggaggatt gggaagagaa tagcaggcat gctggggagc ggccgcagga 5700

acccctagtg atggagttgg ccactccctc tctgcgcgct cgctcgctca ctgaggccgg 5760

gcgaccaaag gtcgcccgac gcccgggctt tgcccgggcg gcctcagtga gcgagcgagc 5820

gcgcagctgc ctgcaggggc gcctgatgcg gtattttctc cttacgcatc tgtgcggtat 5880

ttcacaccgc atacgtcaaa gcaaccatag tacgcgccct gtagcggcgc attaagcgcg 5940

gcgggtgtgg tggttacgcg cagcgtgacc gctacacttg ccagcgccct agcgcccgct 6000

cctttcgctt tcttcccttc ctttctcgcc acgttcgccg gctttccccg tcaagctcta 6060

aatcgggggc tccctttagg gttccgattt agtgctttac ggcacctcga ccccaaaaaa 6120

cttgatttgg gtgatggttc acgtagtggg ccatcgccct gatagacggt ttttcgccct 6180

ttgacgttgg agtccacgtt ctttaatagt ggactcttgt tccaaactgg aacaacactc 6240

aaccctatct cgggctattc ttttgattta taagggattt tgccgatttc ggcctattgg 6300

ttaaaaaatg agctgattta acaaaaattt aacgcgaatt ttaacaaaat attaacgttt 6360

acaattttat ggtgcactct cagtacaatc tgctctgatg ccgcatagtt aagccagccc 6420

cgacacccgc caacacccgc tgacgcgccc tgacgggctt gtctgctccc ggcatccgct 6480

tacagacaag ctgtgaccgt ctccgggagc tgcatgtgtc agaggttttc accgtcatca 6540

ccgaaacgcg cgagacgaaa gggcctcgtg atacgcctat ttttataggt taatgtcatg 6600

ataataatgg tttcttagac gtcaggtggc acttttcggg gaaatgtgcg cggaacccct 6660

atttgtttat ttttctaaat acattcaaat atgtatccgc tcatgagaca ataaccctga 6720

taaatgcttc aataatattg aaaaaggaag agtatgagta ttcaacattt ccgtgtcgcc 6780

cttattccct tttttgcggc attttgcctt cctgtttttg ctcacccaga aacgctggtg 6840

aaagtaaaag atgctgaaga tcagttgggt gcacgagtgg gttacatcga actggatctc 6900

aacagcggta agatccttga gagttttcgc cccgaagaac gttttccaat gatgagcact 6960

tttaaagttc tgctatgtgg cgcggtatta tcccgtattg acgccgggca agagcaactc 7020

ggtcgccgca tacactattc tcagaatgac ttggttgagt actcaccagt cacagaaaag 7080

catcttacgg atggcatgac agtaagagaa ttatgcagtg ctgccataac catgagtgat 7140

aacactgcgg ccaacttact tctgacaacg atcggaggac cgaaggagct aaccgctttt 7200

ttgcacaaca tgggggatca tgtaactcgc cttgatcgtt gggaaccgga gctgaatgaa 7260

gccataccaa acgacgagcg tgacaccacg atgcctgtag caatggcaac aacgttgcgc 7320

aaactattaa ctggcgaact acttactcta gcttcccggc aacaattaat agactggatg 7380

gaggcggata aagttgcagg accacttctg cgctcggccc ttccggctgg ctggtttatt 7440

gctgataaat ctggagccgg tgagcgtgga agccgcggta tcattgcagc actggggcca 7500

gatggtaagc cctcccgtat cgtagttatc tacacgacgg ggagtcaggc aactatggat 7560

gaacgaaata gacagatcgc tgagataggt gcctcactga ttaagcattg gtaactgtca 7620

gaccaagttt actcatatat actttagatt gatttaaaac ttcattttta atttaaaagg 7680

atctaggtga agatcctttt tgataatctc atgaccaaaa tcccttaacg tgagttttcg 7740

ttccactgag cgtcagaccc cgtagaaaag atcaaaggat cttcttgaga tccttttttt 7800

ctgcgcgtaa tctgctgctt gcaaacaaaa aaaccaccgc taccagcggt ggtttgtttg 7860

ccggatcaag agctaccaac tctttttccg aaggtaactg gcttcagcag agcgcagata 7920

ccaaatactg tccttctagt gtagccgtag ttaggccacc acttcaagaa ctctgtagca 7980

ccgcctacat acctcgctct gctaatcctg ttaccagtgg ctgctgccag tggcgataag 8040

tcgtgtctta ccgggttgga ctcaagacga tagttaccgg ataaggcgca gcggtcgggc 8100

tgaacggggg gttcgtgcac acagcccagc ttggagcgaa cgacctacac cgaactgaga 8160

tacctacagc gtgagctatg agaaagcgcc acgcttcccg aagggagaaa ggcggacagg 8220

tatccggtaa gcggcagggt cggaacagga gagcgcacga gggagcttcc agggggaaac 8280

gcctggtatc tttatagtcc tgtcgggttt cgccacctct gacttgagcg tcgatttttg 8340

tgatgctcgt caggggggcg gagcctatgg aaaaacgcca gcaacgcggc ctttttacgg 8400

ttcctggcct tttgctggcc ttttgctcac atgt 8434

<210> 150
<211> 8506
<212> DNA
<213> Artificial Sequence

<220>
<223> synthetic DNA

<400> 150
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccg ggtcttcgag aagacctgtt ttagagctag aaatagcaag ttaaaataag 300

gctagtccgt tatcaacttg aaaaagtggc accgagtcgg tgcttttttg ttttagagct 360

agaaatagca agttaaaata aggctagtcc gtttttagcg cgtgcgccaa ttctgcagac 420

aaatggctct agaggtaccc gttacataac ttacggtaaa tggcccgcct ggctgaccgc 480

ccaacgaccc ccgcccattg acgtcaatag taacgccaat agggactttc cattgacgtc 540

aatgggtgga gtatttacgg taaactgccc acttggcagt acatcaagtg tatcatatgc 600

caagtacgcc ccctattgac gtcaatgacg gtaaatggcc cgcctggcat tgtgcccagt 660

acatgacctt atgggacttt cctacttggc agtacatcta cgtattagtc atcgctatta 720

ccatggtcga ggtgagcccc acgttctgct tcactctccc catctccccc ccctccccac 780

ccccaatttt gtatttattt attttttaat tattttgtgc agcgatgggg gcgggggggg 840

ggggggggcg cgcgccaggc ggggcggggc ggggcgaggg gcggggcggg gcgaggcgga 900

gaggtgcggc ggcagccaat cagagcggcg cgctccgaaa gtttcctttt atggcgaggc 960

ggcggcggcg gcggccctat aaaaagcgaa gcgcgcggcg ggcgggagtc gctgcgacgc 1020

tgccttcgcc ccgtgccccg ctccgccgcc gcctcgcgcc gcccgccccg gctctgactg 1080

accgcgttac tcccacaggt gagcgggcgg gacggccctt ctcctccggg ctgtaattag 1140

ctgagcaaga ggtaagggtt taagggatgg ttggttggtg gggtattaat gtttaattac 1200

ctggagcacc tgcctgaaat cacttttttt caggttggac cggtgccacc atggactata 1260

aggaccacga cggagactac aaggatcatg atattgatta caaagacgat gacgataaga 1320

tggccccaaa gaagaagcgg aaggtcggta tccacggagt cccagcagcc gacaagaagt 1380

acagcatcgg cctggacatc ggcaccaact ctgtgggctg ggccgtgatc accgacgagt 1440

acaaggtgcc cagcaagaaa ttcaaggtgc tgggcaacac cgaccggcac agcatcaaga 1500

agaacctgat cggagccctg ctgttcgaca gcggcgaaac agccgaggcc acccggctga 1560

agagaaccgc cagaagaaga tacaccagac ggaagaaccg gatctgctat ctgcaagaga 1620

tcttcagcaa cgagatggcc aaggtggacg acagcttctt ccacagactg gaagagtcct 1680

tcctggtgga agaggataag aagcacgagc ggcaccccat cttcggcaac atcgtggacg 1740

aggtggccta ccacgagaag taccccacca tctaccacct gagaaagaaa ctggtggaca 1800

gcaccgacaa ggccgacctg cggctgatct atctggccct ggcccacatg atcaagttcc 1860

ggggccactt cctgatcgag ggcgacctga accccgacaa cagcgacgtg gacaagctgt 1920

tcatccagct ggtgcagacc tacaaccagc tgttcgagga aaaccccatc aacgccagcg 1980

gcgtggacgc caaggccatc ctgtctgcca gactgagcaa gagcagacgg ctggaaaatc 2040

tgatcgccca gctgcccggc gagaagaaga atggcctgtt cggaaacctg attgccctga 2100

gcctgggcct gacccccaac ttcaagagca acttcgacct ggccgaggat gccaaactgc 2160

agctgagcaa ggacacctac gacgacgacc tggacaacct gctggcccag atcggcgacc 2220

agtacgccga cctgtttctg gccgccaaga acctgtccga cgccatcctg ctgagcgaca 2280

tcctgagagt gaacaccgag atcaccaagg cccccctgag cgcctctatg atcaagagat 2340

acgacgagca ccaccaggac ctgaccctgc tgaaagctct cgtgcggcag cagctgcctg 2400

agaagtacaa agagattttc ttcgaccaga gcaagaacgg ctacgccggc tacattgacg 2460

gcggagccag ccaggaagag ttctacaagt tcatcaagcc catcctggaa aagatggacg 2520

gcaccgagga actgctcgtg aagctgaaca gagaggacct gctgcggaag cagcggacct 2580

tcgacaacgg cagcatcccc caccagatcc acctgggaga gctgcacgcc attctgcggc 2640

ggcaggaaga tttttaccca ttcctgaagg acaaccggga aaagatcgag aagatcctga 2700

ccttccgcat cccctactac gtgggccctc tggccagggg aaacagcaga ttcgcctgga 2760

tgaccagaaa gagcgaggaa accatcaccc cctggaactt cgaggaagtg gtggacaagg 2820

gcgcttccgc ccagagcttc atcgagcgga tgaccaactt cgataagaac ctgcccaacg 2880

agaaggtgct gcccaagcac agcctgctgt acgagtactt caccgtgtat aacgagctga 2940

ccaaagtgaa atacgtgacc gagggaatga gaaagcccgc cttcctgagc ggcgagcaga 3000

aaaaggccat cgtggacctg ctgttcaaga ccaaccggaa agtgaccgtg aagcagctga 3060

aagaggacta cttcaagaaa atcgagtgct tcgactccgt ggaaatctcc ggcgtggaag 3120

atcggttcaa cgcctccctg ggcacatacc acgatctgct gaaaattatc aaggacaagg 3180

acttcctgga caatgaggaa aacgaggaca ttctggaaga tatcgtgctg accctgacac 3240

tgtttgagga cagagagatg atcgaggaac ggctgaaaac ctatgcccac ctgttcgacg 3300

acaaagtgat gaagcagctg aagcggcgga gatacaccgg ctggggcagg ctgagccgga 3360

agctgatcaa cggcatccgg gacaagcagt ccggcaagac aatcctggat ttcctgaagt 3420

ccgacggctt cgccaacaga aacttcatgc agctgatcca cgacgacagc ctgaccttta 3480

aagaggacat ccagaaagcc caggtgtccg gccagggcga tagcctgcac gagcacattg 3540

ccaatctggc cggcagcccc gccattaaga agggcatcct gcagacagtg aaggtggtgg 3600

acgagctcgt gaaagtgatg ggccggcaca agcccgagaa catcgtgatc gaaatggcca 3660

gagagaacca gaccacccag aagggacaga agaacagccg cgagagaatg aagcggatcg 3720

aagagggcat caaagagctg ggcagccaga tcctgaaaga acaccccgtg gaaaacaccc 3780

agctgcagaa cgagaagctg tacctgtact acctgcagaa tgggcgggat atgtacgtgg 3840

accaggaact ggacatcaac cggctgtccg actacgatgt ggaccatatc gtgcctcaga 3900

gctttctgaa ggacgactcc atcgacaaca aggtgctgac cagaagcgac aagaaccggg 3960

gcaagagcga caacgtgccc tccgaagagg tcgtgaagaa gatgaagaac tactggcggc 4020

agctgctgaa cgccaagctg attacccaga gaaagttcga caatctgacc aaggccgaga 4080

gaggcggcct gagcgaactg gataaggccg gcttcatcaa gagacagctg gtggaaaccc 4140

ggcagatcac aaagcacgtg gcacagatcc tggactcccg gatgaacact aagtacgacg 4200

agaatgacaa gctgatccgg gaagtgaaag tgatcaccct gaagtccaag ctggtgtccg 4260

atttccggaa ggatttccag ttttacaaag tgcgcgagat caacaactac caccacgccc 4320

acgacgccta cctgaacgcc gtcgtgggaa ccgccctgat caaaaagtac cctaagctgg 4380

aaagcgagtt cgtgtacggc gactacaagg tgtacgacgt gcggaagatg atcgccaaga 4440

gcgagcagga aatcggcaag gctaccgcca agtacttctt ctacagcaac atcatgaact 4500

ttttcaagac cgagattacc ctggccaacg gcgagatccg gaagcggcct ctgatcgaga 4560

caaacggcga aaccggggag atcgtgtggg ataagggccg ggattttgcc accgtgcgga 4620

aagtgctgag catgccccaa gtgaatatcg tgaaaaagac cgaggtgcag acaggcggct 4680

tcagcaaaga gtctatcctg cccaagagga acagcgataa gctgatcgcc agaaagaagg 4740

actgggaccc taagaagtac ggcggcttcg acagccccac cgtggcctat tctgtgctgg 4800

tggtggccaa agtggaaaag ggcaagtcca agaaactgaa gagtgtgaaa gagctgctgg 4860

ggatcaccat catggaaaga agcagcttcg agaagaatcc catcgacttt ctggaagcca 4920

agggctacaa agaagtgaaa aaggacctga tcatcaagct gcctaagtac tccctgttcg 4980

agctggaaaa cggccggaag agaatgctgg cctctgccgg cgaactgcag aagggaaacg 5040

aactggccct gccctccaaa tatgtgaact tcctgtacct ggccagccac tatgagaagc 5100

tgaagggctc ccccgaggat aatgagcaga aacagctgtt tgtggaacag cacaagcact 5160

acctggacga gatcatcgag cagatcagcg agttctccaa gagagtgatc ctggccgacg 5220

ctaatctgga caaagtgctg tccgcctaca acaagcaccg ggataagccc atcagagagc 5280

aggccgagaa tatcatccac ctgtttaccc tgaccaatct gggagcccct gccgccttca 5340

agtactttga caccaccatc gaccggaaga ggtacaccag caccaaagag gtgctggacg 5400

ccaccctgat ccaccagagc atcaccggcc tgtacgagac acggatcgac ctgtctcagc 5460

tgggaggcga caaaaggccg gcggccacga aaaaggccgg ccaggcaaaa aagaaaaagt 5520

aagaattcct agagctcgct gatcagcctc gactgtgcct tctagttgcc agccatctgt 5580

tgtttgcccc tcccccgtgc cttccttgac cctggaaggt gccactccca ctgtcctttc 5640

ctaataaaat gaggaaattg catcgcattg tctgagtagg tgtcattcta ttctgggggg 5700

tggggtgggg caggacagca agggggagga ttgggaagag aatagcaggc atgctgggga 5760

gcggccgcag gaacccctag tgatggagtt ggccactccc tctctgcgcg ctcgctcgct 5820

cactgaggcc gggcgaccaa aggtcgcccg acgcccgggc tttgcccggg cggcctcagt 5880

gagcgagcga gcgcgcagct gcctgcaggg gcgcctgatg cggtattttc tccttacgca 5940

tctgtgcggt atttcacacc gcatacgtca aagcaaccat agtacgcgcc ctgtagcggc 6000

gcattaagcg cggcgggtgt ggtggttacg cgcagcgtga ccgctacact tgccagcgcc 6060

ctagcgcccg ctcctttcgc tttcttccct tcctttctcg ccacgttcgc cggctttccc 6120

cgtcaagctc taaatcgggg gctcccttta gggttccgat ttagtgcttt acggcacctc 6180

gaccccaaaa aacttgattt gggtgatggt tcacgtagtg ggccatcgcc ctgatagacg 6240

gtttttcgcc ctttgacgtt ggagtccacg ttctttaata gtggactctt gttccaaact 6300

ggaacaacac tcaaccctat ctcgggctat tcttttgatt tataagggat tttgccgatt 6360

tcggcctatt ggttaaaaaa tgagctgatt taacaaaaat ttaacgcgaa ttttaacaaa 6420

atattaacgt ttacaatttt atggtgcact ctcagtacaa tctgctctga tgccgcatag 6480

ttaagccagc cccgacaccc gccaacaccc gctgacgcgc cctgacgggc ttgtctgctc 6540

ccggcatccg cttacagaca agctgtgacc gtctccggga gctgcatgtg tcagaggttt 6600

tcaccgtcat caccgaaacg cgcgagacga aagggcctcg tgatacgcct atttttatag 6660

gttaatgtca tgataataat ggtttcttag acgtcaggtg gcacttttcg gggaaatgtg 6720

cgcggaaccc ctatttgttt atttttctaa atacattcaa atatgtatcc gctcatgaga 6780

caataaccct gataaatgct tcaataatat tgaaaaagga agagtatgag tattcaacat 6840

ttccgtgtcg cccttattcc cttttttgcg gcattttgcc ttcctgtttt tgctcaccca 6900

gaaacgctgg tgaaagtaaa agatgctgaa gatcagttgg gtgcacgagt gggttacatc 6960

gaactggatc tcaacagcgg taagatcctt gagagttttc gccccgaaga acgttttcca 7020

atgatgagca cttttaaagt tctgctatgt ggcgcggtat tatcccgtat tgacgccggg 7080

caagagcaac tcggtcgccg catacactat tctcagaatg acttggttga gtactcacca 7140

gtcacagaaa agcatcttac ggatggcatg acagtaagag aattatgcag tgctgccata 7200

accatgagtg ataacactgc ggccaactta cttctgacaa cgatcggagg accgaaggag 7260

ctaaccgctt ttttgcacaa catgggggat catgtaactc gccttgatcg ttgggaaccg 7320

gagctgaatg aagccatacc aaacgacgag cgtgacacca cgatgcctgt agcaatggca 7380

acaacgttgc gcaaactatt aactggcgaa ctacttactc tagcttcccg gcaacaatta 7440

atagactgga tggaggcgga taaagttgca ggaccacttc tgcgctcggc ccttccggct 7500

ggctggttta ttgctgataa atctggagcc ggtgagcgtg gaagccgcgg tatcattgca 7560

gcactggggc cagatggtaa gccctcccgt atcgtagtta tctacacgac ggggagtcag 7620

gcaactatgg atgaacgaaa tagacagatc gctgagatag gtgcctcact gattaagcat 7680

tggtaactgt cagaccaagt ttactcatat atactttaga ttgatttaaa acttcatttt 7740

taatttaaaa ggatctaggt gaagatcctt tttgataatc tcatgaccaa aatcccttaa 7800

cgtgagtttt cgttccactg agcgtcagac cccgtagaaa agatcaaagg atcttcttga 7860

gatccttttt ttctgcgcgt aatctgctgc ttgcaaacaa aaaaaccacc gctaccagcg 7920

gtggtttgtt tgccggatca agagctacca actctttttc cgaaggtaac tggcttcagc 7980

agagcgcaga taccaaatac tgtccttcta gtgtagccgt agttaggcca ccacttcaag 8040

aactctgtag caccgcctac atacctcgct ctgctaatcc tgttaccagt ggctgctgcc 8100

agtggcgata agtcgtgtct taccgggttg gactcaagac gatagttacc ggataaggcg 8160

cagcggtcgg gctgaacggg gggttcgtgc acacagccca gcttggagcg aacgacctac 8220

accgaactga gatacctaca gcgtgagcta tgagaaagcg ccacgcttcc cgaagggaga 8280

aaggcggaca ggtatccggt aagcggcagg gtcggaacag gagagcgcac gagggagctt 8340

ccagggggaa acgcctggta tctttatagt cctgtcgggt ttcgccacct ctgacttgag 8400

cgtcgatttt tgtgatgctc gtcagggggg cggagcctat ggaaaaacgc cagcaacgcg 8460

gcctttttac ggttcctggc cttttgctgg ccttttgctc acatgt 8506

<210> 151
<211> 8436

<212>   DNA
<213>   Artificial Sequence

<220>
<223>   synthetic DNA

<400>   151
gagggcctat ttcccatgat tccttcatat ttgcatatac gatacaaggc tgttagagag      60

ataattggaa ttaatttgac tgtaaacaca aagatattag tacaaaatac gtgacgtaga     120

aagtaataat ttcttgggta gtttgcagtt ttaaaattat gttttaaaat ggactatcat     180

atgcttaccg taacttgaaa gtatttcgat ttcttggctt tatatatctt gtggaaagga     240

cgaaacaccg gatcccggga gctttaccgg ttttagagct agaaatagca agttaaaata     300

aggctagtcc gttatcaact tgaaaaagtg gcaccgagtc ggtgcttttt tgttttagag     360

ctagaaatag caagttaaaa taaggctagt ccgtttttag cgcgtgcgcc aattctgcag     420

acaaatggct ctagaggtac ccgttacata acttacggta aatggcccgc ctggctgacc     480

gcccaacgac ccccgcccat tgacgtcaat agtaacgcca atagggactt tccattgacg     540

tcaatgggtg gagtatttac ggtaaactgc ccacttggca gtacatcaag tgtatcatat     600

gccaagtacg ccccctattg acgtcaatga cggtaaatgg cccgcctggc attgtgccca     660

gtacatgacc ttatgggact ttcctacttg gcagtacatc tacgtattag tcatcgctat     720

taccatggtc gaggtgagcc ccacgttctg cttcactctc cccatctccc ccccctcccc     780

acccccaatt ttgtatttat ttatttttta attattttgt gcagcgatgg gggcgggggg     840

gggggggggg cgcgcgccag gcggggcggg gcggggcgag gggcggggcg gggcgaggcg     900

gagaggtgcg gcggcagcca atcagagcgg cgcgctccga aagtttcctt ttatggcgag     960

gcggcggcgg cggcggccct ataaaaagcg aagcgcgcgg cgggcgggag tcgctgcgac    1020

gctgccttcg ccccgtgccc cgctccgccg ccgcctcgcg ccgcccgccc cggctctgac    1080

tgaccgcgtt actcccacag gtgagcgggc gggacggccc ttctcctccg ggctgtaatt    1140

agctgagcaa gaggtaaggg tttaagggat ggttggttgg tggggtatta atgtttaatt    1200

acctggagca cctgcctgaa atcacttttt ttcaggttgg accggtgcca ccatgtaccc    1260

atacgatgtt ccagattacg cttcgccgaa gaaaaagcgc aaggtcgaag cgtccgacaa    1320

gaagtacagc atcggcctgg ccatcggcac caactctgtg ggctgggccg tgatcaccga    1380

cgagtacaag gtgcccagca agaaattcaa ggtgctgggc aacaccgacc ggcacagcat    1440

caagaagaac ctgatcggag ccctgctgtt cgacagcggc gaaacagccg aggccacccg    1500

gctgaagaga accgccagaa gaagatacac cagacggaag aaccggatct gctatctgca    1560

agagatcttc agcaacgaga tggccaaggt ggacgacagc ttcttccaca gactggaaga    1620

gtccttcctg gtggaagagg ataagaagca cgagcggcac cccatcttcg gcaacatcgt    1680

ggacgaggtg gcctaccacg agaagtaccc caccatctac cacctgagaa agaaactggt 1740

ggacagcacc gacaaggccg acctgcggct gatctatctg gccctggccc acatgatcaa 1800

gttccggggc cacttcctga tcgagggcga cctgaacccc gacaacagcg acgtggacaa 1860

gctgttcatc cagctggtgc agacctacaa ccagctgttc gaggaaaacc ccatcaacgc 1920

cagcggcgtg gacgccaagg ccatcctgtc tgccagactg agcaagagca gacggctgga 1980

aaatctgatc gcccagctgc ccggcgagaa gaagaatggc ctgttcggca acctgattgc 2040

cctgagcctg ggcctgaccc ccaacttcaa gagcaacttc gacctggccg aggatgccaa 2100

actgcagctg agcaaggaca cctacgacga cgacctggac aacctgctgg cccagatcgg 2160

cgaccagtac gccgacctgt ttctggccgc caagaacctg tccgacgcca tcctgctgag 2220

cgacatcctg agagtgaaca ccgagatcac caaggccccc ctgagcgcct ctatgatcaa 2280

gagatacgac gagcaccacc aggacctgac cctgctgaaa gctctcgtgc ggcagcagct 2340

gcctgagaag tacaaagaga ttttcttcga ccagagcaag aacggctacg ccggctacat 2400

tgacggcgga gccagccagg aagagttcta caagttcatc aagcccatcc tggaaaagat 2460

ggacggcacc gaggaactgc tcgtgaagct gaacagagag gacctgctgc ggaagcagcg 2520

gaccttcgac aacggcagca tcccccacca gatccacctg ggagagctgc acgccattct 2580

gcggcggcag gaagattttt acccattcct gaaggacaac cgggaaaaga tcgagaagat 2640

cctgaccttc cgcatcccct actacgtggg ccctctggcc aggggaaaca gcagattcgc 2700

ctggatgacc agaaagagcg aggaaaccat caccccctgg aacttcgagg aagtggtgga 2760

caagggcgct tccgcccaga gcttcatcga gcggatgacc aacttcgata agaacctgcc 2820

caacgagaag gtgctgccca agcacagcct gctgtacgag tacttcaccg tgtataacga 2880

gctgaccaaa gtgaaatacg tgaccgaggg aatgagaaag cccgccttcc tgagcggcga 2940

gcagaaaaag gccatcgtgg acctgctgtt caagaccaac cggaaagtga ccgtgaagca 3000

gctgaaagag gactacttca agaaaatcga gtgcttcgac tccgtggaaa tctccggcgt 3060

ggaagatcgg ttcaacgcct ccctgggcac ataccacgat ctgctgaaaa ttatcaagga 3120

caaggacttc ctggacaatg aggaaaacga ggacattctg gaagatatcg tgctgaccct 3180

gacactgttt gaggacagag agatgatcga ggaacggctg aaaacctatg cccacctgtt 3240

cgacgacaaa gtgatgaagc agctgaagcg gcggagatac accggctggg gcaggctgag 3300

ccggaagctg atcaacggca tccgggacaa gcagtccggc aagacaatcc tggatttcct 3360

gaagtccgac ggcttcgcca acagaaactt catgcagctg atccacgacg acagcctgac 3420

ctttaaagag gacatccaga aagcccaggt gtccggccag ggcgatagcc tgcacgagca 3480

cattgccaat ctggccggca gccccgccat taagaagggc atcctgcaga cagtgaaggt 3540

ggtggacgag ctcgtgaaag tgatgggccg gcacaagccc gagaacatcg tgatcgaaat 3600

ggccagagag aaccagacca cccagaaggg acagaagaac agccgcgaga gaatgaagcg 3660

gatcgaagag ggcatcaaag agctgggcag ccagatcctg aaagaacacc ccgtggaaaa 3720

cacccagctg cagaacgaga agctgtacct gtactacctg cagaatgggc gggatatgta 3780

cgtggaccag gaactggaca tcaaccggct gtccgactac gatgtggacc atatcgtgcc 3840

tcagagcttt ctgaaggacg actccatcga caacaaggtg ctgaccagaa gcgacaagaa 3900

ccggggcaag agcgacaacg tgccctccga agaggtcgtg aagaagatga agaactactg 3960

gcggcagctg ctgaacgcca agctgattac ccagagaaag ttcgacaatc tgaccaaggc 4020

cgagagaggc ggcctgagcg aactggataa ggccggcttc atcaagagac agctggtgga 4080

aacccggcag atcacaaagc acgtggcaca gatcctggac tcccggatga acactaagta 4140

cgacgagaat gacaagctga tccgggaagt gaaagtgatc accctgaagt ccaagctggt 4200

gtccgatttc cggaaggatt tccagtttta caaagtgcgc gagatcaaca actaccacca 4260

cgcccacgac gcctacctga acgccgtcgt gggaaccgcc ctgatcaaaa agtaccctaa 4320

gctggaaagc gagttcgtgt acggcgacta caaggtgtac gacgtgcgga agatgatcgc 4380

caagagcgag caggaaatcg gcaaggctac cgccaagtac ttcttctaca gcaacatcat 4440

gaactttttc aagaccgaga ttaccctggc caacggcgag atccggaagc ggcctctgat 4500

cgagacaaac ggcgaaaccg gggagatcgt gtgggataag ggccgggatt ttgccaccgt 4560

gcggaaagtg ctgagcatgc cccaagtgaa tatcgtgaaa aagaccgagg tgcagacagg 4620

cggcttcagc aaagagtcta tcctgcccaa gaggaacagc gataagctga tcgccagaaa 4680

gaaggactgg gaccctaaga agtacggcgg cttcgacagc cccaccgtgg cctattctgt 4740

gctggtggtg gccaaagtgg aaaagggcaa gtccaagaaa ctgaagagtg tgaaagagct 4800

gctggggatc accatcatgg aaagaagcag cttcgagaag aatcccatcg actttctgga 4860

agccaagggc tacaaagaag tgaaaaagga cctgatcatc aagctgccta agtactccct 4920

gttcgagctg gaaaacggcc ggaagagaat gctggcctct gccggcgaac tgcagaaggg 4980

aaacgaactg gccctgccct ccaaatatgt gaacttcctg tacctggcca gccactatga 5040

gaagctgaag ggctcccccg aggataatga gcagaaacag ctgtttgtgg aacagcacaa 5100

gcactacctg gacgagatca tcgagcagat cagcgagttc tccaagagag tgatcctggc 5160

cgacgctaat ctggacaaag tgctgtccgc ctacaacaag caccgggata agcccatcag 5220

agagcaggcc gagaatatca tccacctgtt taccctgacc aatctgggag cccctgccgc 5280

cttcaagtac tttgacacca ccatcgaccg gaagaggtac accagcacca aagaggtgct 5340

ggacgccacc ctgatccacc agagcatcac cggcctgtac gagacacgga tcgacctgtc 5400

tcagctggga ggcgacagcc ccaagaagaa gagaaaggtg gaggccagct aagaattcct 5460

agagctcgct gatcagcctc gactgtgcct tctagttgcc agccatctgt tgtttgcccc 5520

tcccccgtgc cttccttgac cctggaaggt gccactccca ctgtcctttc ctaataaaat 5580

gaggaaattg catcgcattg tctgagtagg tgtcattcta ttctgggggg tggggtgggg 5640

caggacagca agggggagga ttgggaagag aatagcaggc atgctgggga gcggccgcag 5700

gaacccctag tgatggagtt ggccactccc tctctgcgcg ctcgctcgct cactgaggcc 5760

gggcgaccaa aggtcgcccg acgcccgggc tttgcccggg cggcctcagt gagcgagcga 5820

gcgcgcagct gcctgcaggg gcgcctgatg cggtattttc tccttacgca tctgtgcggt 5880

atttcacacc gcatacgtca aagcaaccat agtacgcgcc ctgtagcggc gcattaagcg 5940

cggcgggtgt ggtggttacg cgcagcgtga ccgctacact tgccagcgcc ctagcgcccg 6000

ctcctttcgc tttcttccct tcctttctcg ccacgttcgc cggctttccc cgtcaagctc 6060

taaatcgggg gctcccttta gggttccgat ttagtgcttt acggcacctc gaccccaaaa 6120

aacttgattt gggtgatggt tcacgtagtg ggccatcgcc ctgatagacg gtttttcgcc 6180

ctttgacgtt ggagtccacg ttctttaata gtggactctt gttccaaact ggaacaacac 6240

tcaaccctat ctcgggctat tcttttgatt tataagggat tttgccgatt tcggcctatt 6300

ggttaaaaaa tgagctgatt taacaaaaat ttaacgcgaa ttttaacaaa atattaacgt 6360

ttacaatttt atggtgcact ctcagtacaa tctgctctga tgccgcatag ttaagccagc 6420

cccgacaccc gccaacaccc gctgacgcgc cctgacgggc ttgtctgctc ccggcatccg 6480

cttacagaca agctgtgacc gtctccggga gctgcatgtg tcagaggttt tcaccgtcat 6540

caccgaaacg cgcgagacga aagggcctcg tgatacgcct atttttatag gttaatgtca 6600

tgataataat ggtttcttag acgtcaggtg gcacttttcg gggaaatgtg cgcggaaccc 6660

ctatttgttt atttttctaa atacattcaa atatgtatcc gctcatgaga caataaccct 6720

gataaatgct tcaataatat tgaaaaagga agagtatgag tattcaacat ttccgtgtcg 6780

cccttattcc cttttttgcg gcattttgcc ttcctgtttt tgctcaccca gaaacgctgg 6840

tgaaagtaaa agatgctgaa gatcagttgg gtgcacgagt gggttacatc gaactggatc 6900

tcaacagcgg taagatcctt gagagttttc gccccgaaga acgttttcca atgatgagca 6960

cttttaaagt tctgctatgt ggcgcggtat tatcccgtat tgacgccggg caagagcaac 7020

tcggtcgccg catacactat tctcagaatg acttggttga gtactcacca gtcacagaaa 7080

agcatcttac ggatggcatg acagtaagag aattatgcag tgctgccata accatgagtg 7140

ataacactgc ggccaactta cttctgacaa cgatcggagg accgaaggag ctaaccgctt 7200

ttttgcacaa catgggggat catgtaactc gccttgatcg ttgggaaccg gagctgaatg 7260

aagccatacc aaacgacgag cgtgacacca cgatgcctgt agcaatggca acaacgttgc 7320

gcaaactatt aactggcgaa ctacttactc tagcttcccg gcaacaatta atagactgga 7380

tggaggcgga taaagttgca ggaccacttc tgcgctcggc ccttccggct ggctggttta 7440

ttgctgataa atctggagcc ggtgagcgtg gaagccgcgg tatcattgca gcactggggc 7500

cagatggtaa gccctcccgt atcgtagtta tctacacgac ggggagtcag gcaactatgg 7560

atgaacgaaa tagacagatc gctgagatag gtgcctcact gattaagcat tggtaactgt 7620

cagaccaagt ttactcatat atactttaga ttgatttaaa acttcatttt taatttaaaa 7680

ggatctaggt gaagatcctt tttgataatc tcatgaccaa aatcccttaa cgtgagtttt 7740

cgttccactg agcgtcagac cccgtagaaa agatcaaagg atcttcttga gatccttttt 7800

ttctgcgcgt aatctgctgc ttgcaaacaa aaaaaccacc gctaccagcg gtggtttgtt 7860

tgccggatca agagctacca actctttttc cgaaggtaac tggcttcagc agagcgcaga 7920

taccaaatac tgtccttcta gtgtagccgt agttaggcca ccacttcaag aactctgtag 7980

caccgcctac atacctcgct ctgctaatcc tgttaccagt ggctgctgcc agtggcgata 8040

agtcgtgtct taccgggttg gactcaagac gatagttacc ggataaggcg cagcggtcgg 8100

gctgaacggg gggttcgtgc acacagccca gcttggagcg aacgacctac accgaactga 8160

gatacctaca gcgtgagcta tgagaaagcg ccacgcttcc cgaagggaga aaggcggaca 8220

ggtatccggt aagcggcagg gtcggaacag gagagcgcac gagggagctt ccagggggaa 8280

acgcctggta tctttatagt cctgtcgggt ttcgccacct ctgacttgag cgtcgatttt 8340

tgtgatgctc gtcagggggg cggagcctat ggaaaaacgc cagcaacgcg gcctttttac 8400

ggttcctggc cttttgctgg ccttttgctc acatgt 8436

<210> 152
<211> 8435
<212> DNA
<213> Artificial Sequence

<220>
<223> synthetic DNA

<400> 152
gagggcctat ttcccatgat tccttcatat ttgcatatac gatacaaggc tgttagagag 60

ataattggaa ttaatttgac tgtaaacaca aagatattag tacaaaatac gtgacgtaga 120

aagtaataat ttcttgggta gtttgcagtt ttaaaattat gttttaaaat ggactatcat 180

atgcttaccg taacttgaaa gtatttcgat ttcttggctt tatatatctt gtggaaagga 240

cgaaacaccc tcgccgcggt aaagctccgt tttagagcta gaaatagcaa gttaaaataa 300

ggctagtccg ttatcaactt gaaaaagtgg caccgagtcg gtgctttttt gttttagagc 360

tagaaatagc aagttaaaat aaggctagtc cgtttttagc gcgtgcgcca attctgcaga 420

caaatggctc tagaggtacc cgttacataa cttacggtaa atggcccgcc tggctgaccg 480

cccaacgacc cccgcccatt gacgtcaata gtaacgccaa tagggacttt ccattgacgt 540

caatgggtgg agtatttacg gtaaactgcc cacttggcag tacatcaagt gtatcatatg 600

ccaagtacgc cccctattga cgtcaatgac ggtaaatggc ccgcctggca ttgtgcccag 660

tacatgacct tatgggactt tcctacttgg cagtacatct acgtattagt catcgctatt 720

accatggtcg aggtgagccc cacgttctgc ttcactctcc ccatctcccc cccctcccca 780

cccccaattt tgtatttatt tattttttaa ttattttgtg cagcgatggg ggcggggggg 840

gggggggggc gcgcgccagg cggggcgggg cggggcgagg ggcggggcgg ggcgaggcgg 900

agaggtgcgg cggcagccaa tcagagcggc gcgctccgaa agtttccttt tatggcgagg 960

cggcggcggc ggcggcccta taaaaagcga agcgcgcggc gggcgggagt cgctgcgacg 1020

ctgccttcgc cccgtgcccc gctccgccgc cgcctcgcgc cgcccgcccc ggctctgact 1080

gaccgcgtta ctcccacagg tgagcgggcg ggacggccct tctcctccgg gctgtaatta 1140

gctgagcaag aggtaagggt ttaagggatg gttggttggt ggggtattaa tgtttaatta 1200

cctggagcac ctgcctgaaa tcactttttt tcaggttgga ccggtgccac catgtaccca 1260

tacgatgttc cagattacgc ttcgccgaag aaaaagcgca aggtcgaagc gtccgacaag 1320

aagtacagca tcggcctggc catcggcacc aactctgtgg gctgggccgt gatcaccgac 1380

gagtacaagg tgcccagcaa gaaattcaag gtgctgggca acaccgaccg gcacagcatc 1440

aagaagaacc tgatcggagc cctgctgttc gacagcggcg aaacagccga ggccacccgg 1500

ctgaagagaa ccgccagaag aagatacacc agacggaaga accggatctg ctatctgcaa 1560

gagatcttca gcaacgagat ggccaaggtg gacgacagct tcttccacag actggaagag 1620

tccttcctgg tggaagagga taagaagcac gagcggcacc ccatcttcgg caacatcgtg 1680

gacgaggtgg cctaccacga gaagtacccc accatctacc acctgagaaa gaaactggtg 1740

gacagcaccg acaaggccga cctgcggctg atctatctgg ccctggccca catgatcaag 1800

ttccggggcc acttcctgat cgagggcgac ctgaaccccg acaacagcga cgtggacaag 1860

ctgttcatcc agctggtgca gacctacaac cagctgttcg aggaaaaccc catcaacgcc 1920

agcggcgtgg acgccaaggc catcctgtct gccagactga gcaagagcag acggctggaa 1980

aatctgatcg cccagctgcc cggcgagaag aagaatggcc tgttcggcaa cctgattgcc 2040

ctgagcctgg gcctgacccc caacttcaag agcaacttcg acctggccga ggatgccaaa 2100

ctgcagctga gcaaggacac ctacgacgac gacctggaca acctgctggc ccagatcggc 2160

gaccagtacg ccgacctgtt tctggccgcc aagaacctgt ccgacgccat cctgctgagc 2220

gacatcctga gagtgaacac cgagatcacc aaggcccccc tgagcgcctc tatgatcaag 2280

agatacgacg agcaccacca ggacctgacc ctgctgaaag ctctcgtgcg gcagcagctg 2340

cctgagaagt acaaagagat tttcttcgac cagagcaaga acggctacgc cggctacatt 2400

gacggcggag ccagccagga agagttctac aagttcatca agcccatcct ggaaaagatg 2460

gacggcaccg aggaactgct cgtgaagctg aacagagagg acctgctgcg gaagcagcgg 2520

accttcgaca acggcagcat cccccaccag atccacctgg gagagctgca cgccattctg 2580

cggcggcagg aagattttta cccattcctg aaggacaacc gggaaaagat cgagaagatc 2640

ctgaccttcc gcatccccta ctacgtgggc cctctggcca ggggaaacag cagattcgcc 2700

tggatgacca gaaagagcga ggaaaccatc accccctgga acttcgagga agtggtggac 2760

aagggcgctt ccgcccagag cttcatcgag cggatgacca acttcgataa gaacctgccc 2820

aacgagaagg tgctgcccaa gcacagcctg ctgtacgagt acttcaccgt gtataacgag 2880

ctgaccaaag tgaaatacgt gaccgaggga atgagaaagc ccgccttcct gagcggcgag 2940

cagaaaaagg ccatcgtgga cctgctgttc aagaccaacc ggaaagtgac cgtgaagcag 3000

ctgaaagagg actacttcaa gaaaatcgag tgcttcgact ccgtggaaat ctccggcgtg 3060

gaagatcggt tcaacgcctc cctgggcaca taccacgatc tgctgaaaat tatcaaggac 3120

aaggacttcc tggacaatga ggaaaacgag gacattctgg aagatatcgt gctgaccctg 3180

acactgtttg aggacagaga gatgatcgag gaacggctga aaacctatgc ccacctgttc 3240

gacgacaaag tgatgaagca gctgaagcgg cggagataca ccggctgggg caggctgagc 3300

cggaagctga tcaacggcat ccgggacaag cagtccggca agacaatcct ggatttcctg 3360

aagtccgacg gcttcgccaa cagaaacttc atgcagctga tccacgacga cagcctgacc 3420

tttaaagagg acatccagaa agcccaggtg tccggccagg gcgatagcct gcacgagcac 3480

attgccaatc tggccggcag ccccgccatt aagaagggca tcctgcagac agtgaaggtg 3540

gtggacgagc tcgtgaaagt gatgggccgg cacaagcccg agaacatcgt gatcgaaatg 3600

gccagagaga accagaccac ccagaaggga cagaagaaca gccgcgagag aatgaagcgg 3660

atcgaagagg gcatcaaaga gctgggcagc cagatcctga aagaacaccc cgtggaaaac 3720

acccagctgc agaacgagaa gctgtacctg tactacctgc agaatgggcg ggatatgtac 3780

gtggaccagg aactggacat caaccggctg tccgactacg atgtggacca tatcgtgcct 3840

cagagctttc tgaaggacga ctccatcgac aacaaggtgc tgaccagaag cgacaagaac 3900

cggggcaaga gcgacaacgt gccctccgaa gaggtcgtga agaagatgaa gaactactgg 3960

cggcagctgc tgaacgccaa gctgattacc cagagaaagt tcgacaatct gaccaaggcc 4020

gagagaggcg gcctgagcga actggataag gccggcttca tcaagagaca gctggtggaa 4080

acccggcaga tcacaaagca cgtggcacag atcctggact cccggatgaa cactaagtac 4140

gacgagaatg acaagctgat ccgggaagtg aaagtgatca ccctgaagtc caagctggtg 4200

tccgatttcc ggaaggattt ccagttttac aaagtgcgcg agatcaacaa ctaccaccac 4260

gcccacgacg cctacctgaa cgccgtcgtg ggaaccgccc tgatcaaaaa gtaccctaag 4320

ctggaaagcg agttcgtgta cggcgactac aaggtgtacg acgtgcggaa gatgatcgcc 4380

aagagcgagc aggaaatcgg caaggctacc gccaagtact tcttctacag caacatcatg 4440

aactttttca agaccgagat taccctggcc aacggcgaga tccggaagcg gcctctgatc 4500

gagacaaacg gcgaaaccgg ggagatcgtg tgggataagg gccgggattt tgccaccgtg 4560

cggaaagtgc tgagcatgcc ccaagtgaat atcgtgaaaa agaccgaggt gcagacaggc 4620

ggcttcagca aagagtctat cctgcccaag aggaacagcg ataagctgat cgccagaaag 4680

aaggactggg accctaagaa gtacggcggc ttcgacagcc ccaccgtggc ctattctgtg 4740

ctggtggtgg ccaaagtgga aaagggcaag tccaagaaac tgaagagtgt gaaagagctg 4800

ctggggatca ccatcatgga aagaagcagc ttcgagaaga atcccatcga ctttctggaa 4860

gccaagggct acaaagaagt gaaaaaggac ctgatcatca agctgcctaa gtactccctg 4920

ttcgagctgg aaaacggccg gaagagaatg ctggcctctg ccggcgaact gcagaaggga 4980

aacgaactgg ccctgccctc caaatatgtg aacttcctgt acctggccag ccactatgag 5040

aagctgaagg gctcccccga ggataatgag cagaaacagc tgtttgtgga acagcacaag 5100

cactacctgg acgagatcat cgagcagatc agcgagttct ccaagagagt gatcctggcc 5160

gacgctaatc tggacaaagt gctgtccgcc tacaacaagc accgggataa gcccatcaga 5220

gagcaggccg agaatatcat ccacctgttt accctgacca atctgggagc ccctgccgcc 5280

ttcaagtact ttgacaccac catcgaccgg aagaggtaca ccagcaccaa agaggtgctg 5340

gacgccaccc tgatccacca gagcatcacc ggcctgtacg agacacggat cgacctgtct 5400

cagctgggag gcgacagccc caagaagaag agaaaggtgg aggccagcta agaattccta 5460

gagctcgctg atcagcctcg actgtgcctt ctagttgcca gccatctgtt gtttgcccct 5520

cccccgtgcc ttccttgacc ctggaaggtg ccactcccac tgtcctttcc taataaaatg 5580

aggaaattgc atcgcattgt ctgagtaggt gtcattctat tctggggggt ggggtggggc 5640

aggacagcaa gggggaggat tgggaagaga atagcaggca tgctggggag cggccgcagg 5700

aacccctagt gatggagttg gccactccct ctctgcgcgc tcgctcgctc actgaggccg 5760

ggcgaccaaa ggtcgcccga cgcccgggct ttgcccgggc ggcctcagtg agcgagcgag 5820

cgcgcagctg cctgcagggg cgcctgatgc ggtattttct ccttacgcat ctgtgcggta 5880

tttcacaccg catacgtcaa agcaaccata gtacgcgccc tgtagcggcg cattaagcgc 5940

ggcgggtgtg gtggttacgc gcagcgtgac cgctacactt gccagcgccc tagcgcccgc 6000

tcctttcgct ttcttccctt cctttctcgc cacgttcgcc ggctttcccc gtcaagctct 6060

aaatcggggg ctccctttag ggttccgatt tagtgcttta cggcacctcg accccaaaaa 6120

acttgatttg ggtgatggtt cacgtagtgg gccatcgccc tgatagacgg tttttcgccc 6180

tttgacgttg gagtccacgt tctttaatag tggactcttg ttccaaactg gaacaacact 6240

caaccctatc tcgggctatt cttttgattt ataagggatt ttgccgattt cggcctattg 6300

gttaaaaaat gagctgattt aacaaaaatt taacgcgaat tttaacaaaa tattaacgtt 6360

tacaatttta tggtgcactc tcagtacaat ctgctctgat gccgcatagt taagccagcc 6420

ccgacacccg ccaacacccg ctgacgcgcc ctgacgggct tgtctgctcc cggcatccgc 6480

ttacagacaa gctgtgaccg tctccgggag ctgcatgtgt cagaggtttt caccgtcatc 6540

accgaaacgc gcgagacgaa agggcctcgt gatacgccta tttttatagg ttaatgtcat 6600

gataataatg gtttcttaga cgtcaggtgg cacttttcgg ggaaatgtgc gcggaacccc 6660

tatttgttta tttttctaaa tacattcaaa tatgtatccg ctcatgagac aataaccctg 6720

ataaatgctt caataatatt gaaaaaggaa gagtatgagt attcaacatt tccgtgtcgc 6780

ccttattccc ttttttgcgg cattttgcct tcctgttttt gctcacccag aaacgctggt 6840

gaaagtaaaa gatgctgaag atcagttggg tgcacgagtg ggttacatcg aactggatct 6900

caacagcggt aagatccttg agagttttcg ccccgaagaa cgttttccaa tgatgagcac 6960

ttttaaagtt ctgctatgtg gcgcggtatt atcccgtatt gacgccgggc aagagcaact 7020

cggtcgccgc atacactatt ctcagaatga cttggttgag tactcaccag tcacagaaaa 7080

gcatcttacg gatggcatga cagtaagaga attatgcagt gctgccataa ccatgagtga 7140

taacactgcg gccaacttac ttctgacaac gatcggagga ccgaaggagc taaccgcttt 7200

tttgcacaac atgggggatc atgtaactcg ccttgatcgt tgggaaccgg agctgaatga 7260

agccatacca aacgacgagc gtgacaccac gatgcctgta gcaatggcaa caacgttgcg 7320

caaactatta actggcgaac tacttactct agcttcccgg caacaattaa tagactggat 7380

ggaggcggat aaagttgcag gaccacttct gcgctcggcc cttccggctg gctggtttat 7440

tgctgataaa tctggagccg gtgagcgtgg aagccgcggt atcattgcag cactggggcc 7500

agatggtaag ccctcccgta tcgtagttat ctacacgacg gggagtcagg caactatgga 7560

tgaacgaaat agacagatcg ctgagatagg tgcctcactg attaagcatt ggtaactgtc 7620

agaccaagtt tactcatata tactttagat tgatttaaaa cttcattttt aatttaaaag 7680

gatctaggtg aagatccttt ttgataatct catgaccaaa atcccttaac gtgagttttc 7740

gttccactga gcgtcagacc ccgtagaaaa gatcaaagga tcttcttgag atcctttttt 7800

tctgcgcgta atctgctgct tgcaaacaaa aaaaccaccg ctaccagcgg tggtttgttt 7860

gccggatcaa gagctaccaa ctctttttcc gaaggtaact ggcttcagca gagcgcagat 7920

accaaatact gtccttctag tgtagccgta gttaggccac cacttcaaga actctgtagc 7980

accgcctaca tacctcgctc tgctaatcct gttaccagtg gctgctgcca gtggcgataa 8040

gtcgtgtctt accgggttgg actcaagacg atagttaccg gataaggcgc agcggtcggg 8100

ctgaacgggg ggttcgtgca cacagcccag cttggagcga acgacctaca ccgaactgag 8160

atacctacag cgtgagctat gagaaagcgc cacgcttccc gaagggagaa aggcggacag 8220

gtatccggta agcggcaggg tcggaacagg agagcgcacg agggagcttc cagggggaaa 8280

cgcctggtat ctttatagtc ctgtcgggtt tcgccacctc tgacttgagc gtcgattttt 8340

gtgatgctcg tcaggggggc ggagcctatg gaaaaacgcc agcaacgcgg cctttttacg 8400

gttcctggcc ttttgctggc cttttgctca catgt 8435

## Description

### CROSS REFERENCE TO PRIOR APPLICATIONS

This application claims priority under the Paris Convention to US Provisional Patent Application 62/130,258, filed March 9, 2015, and US Provisional Patent Application 62/130,270, filed March 9, 2015, each of which are incorporated herein by reference as if set forth in their entirety.

### FIELD OF THE DISCLOSURE

The present description relates generally to the fields of cell and molecular biology. More particularly, the description relates to molecular tools, methods and kits for controlling division of animal cells and genetically modified cells related to same.

### BACKGROUND OF THE DISCLOSURE

Human pluripotent stem (hPS) cells, may be used as tools for understanding normal cellular development, disease development and for use in cellular therapeutics for treating currently incurable disorders, such as, for example, genetic disorders, degenerative diseases and/or various injuries. The pluripotent nature of these cells renders them able to differentiate into any cell type after a period of self-renewal in the stem cell state (Rossant and Nagy, 1999). The gold standard of hPS cells are the human embryonic stem (hES) cells reported in 1998 (Thomson et al., 1998). In 2006 and 2007 a method for reprogramming differentiated somatic cells, such as skin fibroblasts, into ES cell-like "induced pluripotent stem" (iPS) cells was reported and expanded the types of pluripotent cells (Takahashi and Yamanaka, 2006; Takahashi et al., 2007). The methods of generation of iPS cells and their applications toward many directions including cell-based therapies for treating diseases and aberrant physiological conditions have been developed further in the years since.

One concern regarding pluripotent cell-based therapies is safety. For example, malignant growth originating from a cell graft is of concern. The process of reprogramming differentiated cells into iPS cells is also relevant to safety, as it has been reported that reprogramming methods can cause genome damage and aberrant epigenetic changes (Hussein et al., 2011; Laurent et al., 2011; Lister et al., 2011), which may pose a risk for malignant transformation of iPS cell-derived cells.

One challenge with cell-based therapies involving pluripotent cells expanded *in vitro* is the pluripotent nature of the cells themselves. For example, if pluripotent cells remain among differentiated therapeutic cells, the pluripotent cells may develop into teratomas (Yoshida and Yamanaka, 2010). Attempts to increase the safety of pluripotent cell-derived products and therapies have included efforts to eliminate pluripotent cells from cell cultures after *in vitro* differentiation. For example: cytotoxic antibodies have been used to eliminate cells having pluripotent-specific antigens (Choo et al., 2008; Tan et al., 2009); cells have been sorted based on pluripotency cell surface markers (Ben-David et al., 2013a; Fong et al., 2009; Tang et al., 2011); tumour progression genes have been genetically altered in cells (Blum et al., 2009; Menendez et al., 2012); transgenes for assisting with separation of differentiated cells have been introduced into cells (Chung et al., 2006; Eiges et al., 2001; Huber et al., 2007); suicide genes have been introduced into cells and used to eliminate residual pluripotent stem cells after differentiation (Rong et al., 2012; Schuldiner et al., 2003); and undesired pluripotent cells have been ablated using chemicals (Ben-David et al., 2013b; Dabir et al., 2013; Tohyama et al., 2013). It is possible that even if residual pluripotent cells are eliminated from differentiated cultures, the differentiated derivatives of pluripotent cells may have oncogenic properties (Ghosh et al., 2011). Related oncogenic events could occur in therapeutic cells i) during *in vitro* preparation of cells; or ii) following grafting of cells into a host.

Most current strategies for eliminating or preventing unwanted cell growth and/or differentiation are based on the herpes simplex virus - thymidine kinase (HSV-TK) / ganciclovir (GCV) negatively selectable system, which may be used to eliminate a graft entirely, if malignancy develops (Schuldiner et al., 2003) or to eliminate only the pluripotent cells 'contaminating' the intended differentiated derivatives (Ben-David and Benvenisty, 2014; Lim et al., 2013). The mechanism of GCV-induced cell killing and apoptosis is wall understood. It creates a replication-dependent formation of DNA double-strand breaks (Halloran and Fenton, 1998), which leads to apoptosis (Tomicic et al., 2002). However, many HSV-TK/GCV-based systems are unreliably expressed, at least because they rely on random integration or transient expression of HSV-TK. Strategies involving negative selectable markers with different killing mechanisms, such as, for example, Caspase 9 (Di Stasi et al., 2011) have been tested, but reliable expression of the negative selectable marker has not been shown. Cell-based therapies may require millions or billions of cells, which may amplify any issues caused by unwanted cell growth and/or differentiation.

It is an object of the present disclosure to mitigate and/or obviate one or more of the above deficiencies.

### SUMMARY OF THE DISCLOSURE

In an aspect, a method of controlling proliferation of an animal cell is provided. The method comprises: providing an animal cell; genetically modifying in the animal cell a cell division locus (CDL), the CDL being one or more loci whose transcription product(s) is expressed by dividing cells; the genetic modification of the CDL comprising one or more of: a) an ablation link (ALINK) system, the ALINK system comprising a DNA sequence encoding a negative selectable marker that is transcriptionally linked to a DNA sequence encoding the CDL; and b) an inducible exogenous activator of regulation of a CDL (EARC) system, the EARC system comprising an inducible activator-based gene expression system that is operably linked to the CDL; controlling proliferation of the genetically modified animal cell comprising the ALINK system with an inducer of the negative selectable marker; and/or controlling proliferation of the genetically modified animal cell comprising the EARC system with an inducer of the inducible activator-based gene expression system.

In an embodiment of the method of controlling proliferation of an animal cell provided herein, the controlling of the ALINK-modified animal cell comprises one or more of: permitting proliferation of the genetically modified animal cell comprising the ALINKsystem by maintaining the genetically modified animal cell comprising the ALINK system in the absence of an inducer of the negative selectable marker; and ablating or inhibiting proliferation of the genetically modified animal cell comprising the ALINK system by exposing the animal cell comprising the ALINK system to the inducer of the negative selectable marker.

In an embodiment of the method of controlling proliferation of an animal cell provided herein, the controlling of the EARC-modified animal cell comprises one or more of: permitting proliferation of the genetically modified animal cell comprising the EARC system by exposing the genetically modified animal cell comprising the EARC system to an inducer of the inducible activator-based gene expression system; and preventing or inhibiting proliferation of the genetically modified animal cell comprising the EARC system by maintaining the animal cell comprising the EARC system in the absence of the inducer of the inducible activator-based gene expression system.

In various embodiments of the method of controlling proliferation of an animal cell provided herein, the genetic modification of the CDL comprises preforming targeted replacement of the CDL with one or more of: a) a DNA vector comprising the ALINK system; b) a DNA vector comprising the EARC system; and c) a DNA vector comprising the ALINK system and the EARC system.

In various embodiments of the method of controlling proliferation of an animal cell provided herein, the ALINK genetic modification of the CDL is homozygous, heterozygous, hemizygous or compound heterozygous and/or wherein the EARC genetic modification ensures that functional CDL modification can only be generated through EARC-modified alleles.

In various embodiments of the method of controlling proliferation of an animal cell provided herein, the CDL is one or more loci recited in Table 2. In various embodiments, the CDL encodes a gene product whose function is involved with one or more of: cell cycle, DNA replication, RNA transcription, protein translation, and metabolism. In various embodiments the CDL is one or more of Cdk1/CDK1,Top2A/TOP2A, Cenpa/CENPA, Birc5/BIRC5, and Eef2/EEF2, preferably the CDL is Cdk1 or CDK1.

In various embodiments of the method of controlling proliferation of an animal cell provided herein, the ALINK system comprises a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.

In various embodiments of the method of controlling proliferation of an animal cell provided herein, the EARC system is a dox-bridge system, a cumate switch inducible system, an ecdysone inducible system, a radio wave inducible system, or a ligand-reversible dimerization system, preferably the EARC system is a dox-bridge system.

In various embodiments of the method of controlling proliferation of an animal cell provided herein, the animal cell is a mammalian cell or an avian cell. In various embodiment, the mammalian cell is a human, mouse, rat, hamster, guinea pig, cat, dog, cow, horse, deer, elk, bison, oxen, camel, llama, rabbit, pig, goat, sheep, or non-human primate cell, preferably the mammalian cell is a human cell.

In various embodiments of the method of controlling proliferation of an animal cell provided herein, the animal cell is a pluripotent stem cell a multipotent cell, a monopotent progenitor cell, or a terminally differentiated cell.

In various embodiments of the method of controlling proliferation of an animal cell provided herein, the animal cell is derived from a pluripotent stem cell, a multipotent cell, a monopotent progenitor cell, or a terminally differentiated cell.

In an aspect, an animal cell genetically modified to comprise at least one mechanism for controlling cell proliferation is provided. The genetically modified animal cell comprises: a genetic modification of one or more cell division locus (CDL), the CDL being one or more loci whose transcription product(s) is expressed by dividing cells. The genetic modification being one or more of: a) an ablation link (ALINK) system, the ALINK system comprising a DNA sequence encoding a negative selectable marker that is transcriptionally linked to a DNA sequence encoding the CDL; and b) an exogenous activator of regulation of a CEDL (EARC) system, the EARC system comprising an inducible activator-based gene expression system that is operably linked to the CDL.

In an embodiment of the animal cell genetically modified to comprise at least one mechanism for controlling cell proliferation provided herein, the genetic modification of the CDL comprises preforming targeted replacement of the CDL with one or more of: a) a DNA vector comprising the ALINK system; b) a DNA vector comprising the EARC system; and c) a DNA vector comprising the ALINK system and the EARC system.

In various embodiments of the animal cell genetically modified to comprise at least one mechanism for controlling cell proliferation provided herein, the ALINK genetic modification of the CDL is homozygous, heterozygous, hemizygous or compound heterozygous and/or wherein the EARC genetic modification ensures that functional CDL modification can only be generated through EARC-modified alleles.

In various embodiments of the animal cell genetically modified to comprise at least one mechanism for controlling cell proliferation provided herein, the CDL is one or more loci recited in Table 2. In various embodiments, the CDL encodes a gene product whose function is involved with one or more of: cell cycle, DNA replication, RNA transcription, protein translation, and metabolism. In various embodiments, the CDL is one or more of Cdk1/CDK1, Top2A/TOP2A, Cenpa/CENPA, Birc5/BIRC5, and Eef2/EEF2, preferably the CDL is Cdk1 or CDK1.

In various embodiments of the animal cell genetically modified to comprise at least one mechanism for controlling cell proliferation provided herein, the ALINK system comprises a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.

In various embodiments of the animal cell genetically modified to comprise at least one mechanism for controlling cell proliferation provided herein, the EARC system is a dox-bridge system, a cumate switch inducible system, an ecdysone inducible system, a radio wave inducible system, or a ligand-reversible dimerization system, preferably the EARC system is a dox-bridge system.

In various embodiments of the animal cell genetically modified to comprise at least one mechanism for controlling cell proliferation provided herein, the animal cell is a mammalian cell or an avian cell. In various embodiments, the mammalian cell is a human, mouse, rat, hamster, guinea pig, cat, dog, cow, horse, deer, elk, bison, oxen, camel, llama, rabbit, pig, goat, sheep, or non-human primate cell, preferably the mammalian cell is a human cell.

In various embodiments of the animal cell genetically modified to comprise at least one mechanism for controlling cell proliferation provided herein, the animal cell is a pluripotent stem cell a multipotent cell, a monopotent progenitor cell, or a terminally differentiated cell.

In various embodiments of the animal cell genetically modified to comprise at least one mechanism for controlling cell proliferation provided herein, the animal cell is derived from a pluripotent stem cell, a multipotent cell, a monopotent progenitor cell, or a terminally differentiated cell.

In an aspect, a DNA vector for modifying expression of a cell division locus (CDL), the CDL being one or more loci whose transcription product(s) is expressed by dividing cells is provided. The DNA vector comprises: an ablation link (ALINK) system, the ALINK system comprising a DNA sequence encoding a negative selectable marker that is transcriptionally linked to the CDL, wherein if the DNA vector is inserted into one or more host cells, proliferating host cells comprising the DNA vector will be killed if the proliferating host cells comprising the DNA vector are exposed to an inducer of the negative selectable marker.

In an aspect, DNA vector for modifying expression of a cell division essential locus (CDL), the CDL being one or more loci whose transcription product(s) is expressed by dividing cells is provided. The DNA vector comprises: an exogenous activator of regulation of a CDL (EARC) system, the EARC system comprising an inducible activator-based gene expression system that is operably linked to the CDL, wherein if the DNA vector is inserted into one or more host cells, proliferating host cells comprising the DNA vector will be killed if the proliferating host cells comprising the DNA vector are not exposed to an inducer of the inducible activator-based gene expression system.

In an aspect, a DNA vector for modifying expression of a cell division essential locus (CDL), the CDL being one or more loci whose transcription product(s) is expressed by dividing cells is provided. The DNA vector comprises: an ablation link (ALINK) system, the ALINK system being a DNA sequence encoding a negative selectable marker that is transcriptionally linked to the CDL; and an exogenous activator of regulation of CDL (EARC) system, the EARC system comprising an inducible activator-based gene expression system that is operably linked to the CDL, wherein if the DNA vector is inserted into one or more host cells, proliferating host cells comprising the DNA vector will be killed if the proliferating host cells comprising the DNA vector are exposed to an inducer of the negative selectable marker and if the proliferating host cells comprising the DNA vector are not exposed to an inducer of the inducible activator-based gene expression system.

In various embodiments of the DNA vectors provided herein, the CDL is one or more loci recited in Table 2. In various embodiments, the CDL encodes a gene product whose function is involved with one or more of: cell cycle, DNA replication, RNA transcription, protein translation, and metabolism. In various embodiments, the CDL is one or more of Cdk1/CDK1 ,Top2A/TOP2A, Cenpa/CENPA, Birc5/BIRC5, and Eef2/EEF2, preferably the CDL is Cdk1 or CDK1.

In various embodiments of the DNA vectors provided herein, the ALINK system comprises a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.

In various embodiments of the DNA vectors provided herein, the EARC system is a dox-bridge system, a cumate switch inducible system, an ecdysone inducible system, a radio wave inducible system, or a ligand-reversible dimerization system, preferably the EARC system is a dox-bridge system.

In an aspect, a kit for controlling proliferation of an animal cell by genetically modifying one or more cell division essential locus/loci (CDL), the CDL being one or more loci whose transcription product(s) is expressed by dividing cells is provided. The kit comprises: a DNA vector comprising an ablation link (ALINK) system, the ALINK system comprising a DNA sequence encoding a negative selectable marker that is transcriptionally linked to a DNA sequence encoding the CDL; and/or a DNA vector comprising an exogenous activator of regulation of a CDL (EARC) system, the EARC system comprising an inducible activator-based gene expression system that is operably linked to the CDL; and/or a DNA vector comprising an ALINK system and an EARC system, the ALINK and EARC systems each being operably linked to the CDL; and instructions for targeted replacement of the CDL in an animal cell using one or more of the DNA vectors.

In an embodiment of the kit provided herein, the CDL is one or more loci recited in Table 2. In various embodiments, the CDL encodes a gene product whose function is involved with one or more of: cell cycle, DNA replication, RNA transcription, protein translation, and metabolism. In various embodiments, the CDL is one or more of Cdkl/CDK1,Top2A/TOP2A, Cenpa/CENPA, Birc5/BIRC5, and Eef2/EEF2, preferably the CDL is Cdk1 or CDK1.

In various embodiments of the kit provided herein, the ALINK system comprises a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.

In various embodiments of the kit provided herein, the EARC system is a dox-bridge system, a cumate switch inducible system, an ecdysone inducible system, a radio wave inducible system, or a ligand-reversible dimerization system, preferably the EARC system is a dox-bridge system.

Aspects or embodiments of the invention may also be provided according to the following paragraphs:
1. A method of controlling proliferation of an animal cell, the method comprising:
   - providing an animal cell;
   - genetically modifying in the animal cell a cell division locus (CDL), the CDL being one or more loci whose transcription product(s) is expressed by dividing cells, the genetic modification of the CDL comprising one or more of:
      a) an ablation link (ALINK) system, the ALINK system comprising a DNA sequence encoding a negative selectable marker that is transcriptionally linked to a DNA sequence encoding the CDL; and
      b) an inducible exogenous activator of regulation of a CDL (EARC) system, the EARC system comprising an inducible activator-based gene expression system that is operably linked to the CDL;
   - controlling proliferation of the genetically modified animal cell comprising the ALINK system with an inducer of the negative selectable marker; and/or
   - controlling proliferation of the genetically modified animal cell comprising the EARC system with an inducer of the inducible activator-based gene expression system.
2. The method of paragraph 1, wherein the controlling of the ALINK-modified animal cell comprises one or more of:
   - permitting proliferation of the genetically modified animal cell comprising the ALINK system by maintaining the genetically modified animal cell comprising the ALINK system in the absence of an inducer of the negative selectable marker; and
   - ablating or inhibiting proliferation of the genetically modified animal cell comprising the ALINK system by exposing the animal cell comprising the ALINK system to the inducer of the negative selectable marker.
3. The method of paragraph 1 or 2, wherein the controlling of the EARC-modified animal cell comprises one or more of:
   - permitting proliferation of the genetically modified animal cell comprising the EARC system by exposing the genetically modified animal cell comprising the EARC system to an inducer of the inducible activator-based gene expression system; and
   - preventing or inhibiting proliferation of the genetically modified animal cell comprising the EARC system by maintaining the animal cell comprising the EARC system in the absence of the inducer of the inducible activator-based gene expression system.
4. The method of any one of paragraphs 1 to 3, wherein the genetic modification of the CDL comprises preforming targeted replacement of the CDL with one or more of:
   a) a DNA vector comprising the ALINK system;
   b) a DNA vector comprising the EARC system; and
   c) a DNA vector comprising the ALINK system and the EARC system.
5. The method of any one of paragraphs 1 to 4, wherein the ALINK genetic modification of the CDL is homozygous, heterozygous, hemizygous or compound heterozygous and/or wherein the EARC genetic modification ensures that functional CDL modification can only be generated through EARC-modified alleles.
6. The method of any one of paragraphs 1 to 5 wherein the CDL is one or more loci recited in Table 2.
7. The method of paragraph 6, wherein the CDL encodes a gene product whose function is involved with one or more of: cell cycle, DNA replication, RNA transcription, protein translation, and metabolism.
8. The method of any one of paragraph 7, wherein the CDL is one or more of Cdk1/CDK1,Top2A/TOP2A, Cenpa/CENPA, Birc5/BIRC5, and Eef2/EEF2, preferably the CDL is Cdk1 or CDK1.
9. The method of any one of paragraphs 1 to 8, wherein the ALINK system comprises a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.
10. The method of any one of paragraphs 1 to 8, wherein the EARC system is a dox-bridge system, a cumate switch inducible system, an ecdysone inducible system, a radio wave inducible system, or a ligand-reversible dimerization system, preferably the EARC system is a dox-bridge system.
11. The method of any one of paragraphs 1 to 10, wherein the animal cell is a mammalian cell or an avian cell.
12. The method of paragraph 11, wherein the mammalian cell is a human, mouse, rat, hamster, guinea pig, cat, dog, cow, horse, deer, elk, bison, oxen, camel, llama, rabbit, pig, goat, sheep, or non-human primate cell, preferably the mammalian cell is a human cell.
13. The method of any one of paragraphs 1 to 12, wherein the animal cell is a pluripotent stem cell a multipotent cell, a monopotent progenitor cell, or a terminally differentiated cell.
14. The method of any one of paragraphs 1 to 12, wherein the animal cell is derived from a pluripotent stem cell, a multipotent cell, a monopotent progenitor cell, or a terminally differentiated cell.
15. A method of controlling proliferation of an animal cell population according to the method of any one of paragraphs 1 to 14.
16 An animal cell genetically modified to comprise at least one mechanism for controlling cell proliferation, the genetically modified animal cell comprising:
   - a genetic modification of one or more cell division locus (CDL), the CDL being one or more loci whose transcription product(s) is expressed by dividing cells, the genetic modification being one or more of:
      a) an ablation link (ALINK) system, the ALINK system comprising a DNA sequence encoding a negative selectable marker that is transcriptionally linked to a DNA sequence encoding the CDL; and
      b) an exogenous activator of regulation of a CEDL (EARC) system, the EARC system comprising an inducible activator-based gene expression system that is operably linked to the CDL.
17. The genetically modified animal cell of paragraph 16, wherein the genetic modification of the CDL comprises preforming targeted replacement of the CDL with one or more of:
   a) a DNA vector comprising the ALINK system;
   b) a DNA vector comprising the EARC system;
   c) a DNA vector comprising the ALINK system and the EARC system.
18. The genetically modified animal cell of paragraph 16 or 17, wherein the ALINK genetic modification of the CDL is homozygous, heterozygous, hemizygous or compound heterozygous and/or wherein the EARC genetic modification ensures that functional CDL modification can only be generated through EARC-modified alleles.
19. The genetically modified animal cell of any one of paragraphs 16 to 18, wherein the CDL is one or more of the loci recited in Table 2.
20. The genetically modified animal cell of paragraph 19, wherein the CDL encodes a gene product whose function is involved with one or more of: cell cycle, DNA replication, RNA transcription, protein translation, and metabolism.
21. The genetically modified animal cell of paragraph 20, wherein the CDL is one or more of Cdk1/CDK1,Top2A/TOP2A, Cenpa/CENPA, Birc5/BIRC5, and Eef2/EEF2, preferably the CDL is Cdk1 or CDK1.
22. The genetically modified animal cell of any one of paragraphs 16 to 21, wherein the ALINK system comprises a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.
23. The genetically modified animal cell of any one of paragraphs 16 to 21, wherein the EARC system is a dox-bridge system, a cumate switch inducible system, an ecdysone inducible system, a radio wave inducible system, or a ligand-reversible dimerization system, preferably the EARC system is a dox-bridge system.
24. The genetically modified animal cell of any one of paragraphs 16 to 23, wherein the animal cell is a mammalian cell or an avian cell.
25. The genetically modified animal cell of paragraph 24, wherein the mammalian cell is a human, mouse, rat, hamster, guinea pig, cat, dog, cow, horse, deer, elk, bison, oxen, camel, llama, rabbit, pig, goat, sheep, or non-human primate cell, preferably the mammalian cell is a human cell.
26. The genetically modified animal cell of any one of paragraphs 16 to 25, wherein the animal cell is a pluripotent stem cell a multipotent cell, a monopotent progenitor cell, or a terminally differentiated cell.
27. The genetically modified animal cell of any one of paragraphs 16 to 25, wherein the animal cell is derived from a pluripotent stem cell, a multipotent cell, a monopotent progenitor cell, or a terminally differentiated cell.
28. A population of genetically modified animals cells according to the cell of any one of paragraphs 16 to 27.
29. A DNA vector for modifying expression of a cell division locus (CDL), the CDL being one or more loci whose transcription product(s) is expressed by dividing cells, the DNA vector comprising:
   - an ablation link (ALINK) system, the ALINK system comprising a DNA sequence encoding a negative selectable marker that is transcriptionally linked to the CDL,
   wherein if the DNA vector is inserted into one or more host cells, proliferating host cells comprising the DNA vector will be killed if the proliferating host cells comprising the DNA vector are exposed to an inducer of the negative selectable marker.
30. A DNA vector for modifying expression of a cell division essential locus (CDL), the CDL being one or more loci whose transcription product(s) is expressed by dividing cells, the DNA vector comprising:
   - an exogenous activator of regulation of a CDL (EARC) system, the EARC system comprising an inducible activator-based gene expression system that is operably linked to the CDL,
   wherein if the DNA vector is inserted into one or more host cells, proliferating host cells comprising the DNA vector will be killed if the proliferating host cells comprising the DNA vector are not exposed to an inducer of the inducible activator-based gene expression system.
31. A DNA vector for modifying expression of a cell division essential locus (CDL), the CDL being one or more loci whose transcription product(s) is expressed by dividing cells, the DNA vector comprising:
   - an ablation link (ALINK) system, the ALINK system being a DNA sequence encoding a negative selectable marker that is transcriptionally linked to the CDL; and
   - an exogenous activator of regulation of CDL (EARC) system, the EARC system comprising an inducible activator-based gene expression system that is operably linked to the CDL,
   wherein if the DNA vector is inserted into one or more host cells, proliferating host cells comprising the DNA vector will be killed if the proliferating host cells comprising the DNA vector are exposed to an inducer of the negative selectable marker and if the proliferating host cells comprising the DNA vector are not exposed to an inducer of the inducible activator-based gene expression system.
32. The DNA vector of any one of paragraphs 29 to 31, wherein the CDL is one or more of the loci recited in Table 2.
33. The DNA vector of paragraph 32, wherein the CDL encodes a gene product whose function is involved with one or more of: cell cycle, DNA replication, RNA transcription, protein translation, and metabolism.
34. The DNA vector of paragraph 33, wherein the CDL is one or more of Cdk1/CDK1,Top2A/TOP2A, Cenpa/CENPA, Birc5/BIRC5, and Eef2/EEF2, preferably the CDL
   is Cdk1 or CDK1.
35. The DNA vector of paragraph 29 or 31, wherein the ALINK system comprises a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.
36. The DNA vector of paragraph 30 or 31, wherein the EARC system is a dox-bridge system, a cumate switch inducible system, an ecdysone inducible system, a radio wave inducible system, or a ligand-reversible dimerization system, preferably the EARC system is a dox-bridge system.
37. A kit for controlling proliferation of an animal cell by genetically modifying one or more cell division essential locus/loci (CDL), the CDL being one or more loci whose transcription product(s) is expressed by dividing cells, the kit comprising:
   - a DNA vector comprising an ablation link (ALINK) system, the ALINK system comprising a DNA sequence encoding a negative selectable marker that is transcriptionally linked to a DNA sequence encoding the CDL; and/or
   - a DNA vector comprising an exogenous activator of regulation of a CDL (EARC) system, the EARC system comprising an inducible activator-based gene expression system that is operably linked to the CDL; and/or
   - a DNA vector comprising an ALINK system and an EARC system, the ALINK and EARC systems each being operably linked to the CDL; and
   - instructions for targeted replacement of the CDL in an animal cell using one or more of the DNA vectors.
38. The kit of paragraph 37, wherein the CDL is one or more loci recited in Table 2.
39. The kit of paragraph 38, wherein the CDL encodes a gene product whose function is involved with one or more of: cell cycle, DNA replication, RNA transcription, protein translation, and metabolism.
40. The kit of paragraph 39, wherein the CDL is one or more of Cdk1/CDK1,Top2A/TOP2A, Cenpa/CENPA, Birc5/BIRC5, and Eef2/EEF2, preferably the CDL is Cdk1 or CDK1.
41. The kit of any one of paragraphs 37 to 40, wherein the ALINK system comprises a herpes simplex virus-thymidine kinase/ganciclovir system, a cytosine deaminase/5-fluorocytosine system, a carboxyl esterase/irinotecan system or an iCasp9/AP1903 system, preferably the ALINK system is a herpes simplex virus-thymidine kinase/ganciclovir system.
42. The kit of any one of paragraphs 37 to 40, wherein the EARC system is a dox-bridge system, a cumate switch inducible system, an ecdysone inducible system, a radio wave inducible system, or a ligand-reversible dimerization system, preferably the EARC system is a dox-bridge system.

### DESCRIPTION OF THE DRAWINGS

The patent or application file contains at least one drawing in color. Copies of this patent or patent application publication with color drawings will be provided by the Office upon request and payment of the necessary fee.

These and other features of the disclosure will become more apparent in the following detailed description in which reference is made to the appended drawings wherein:

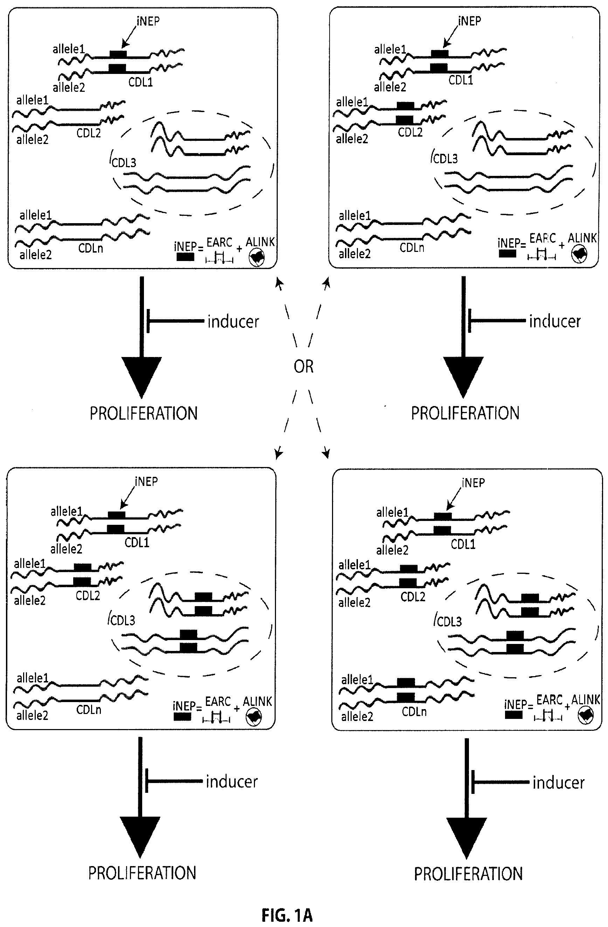

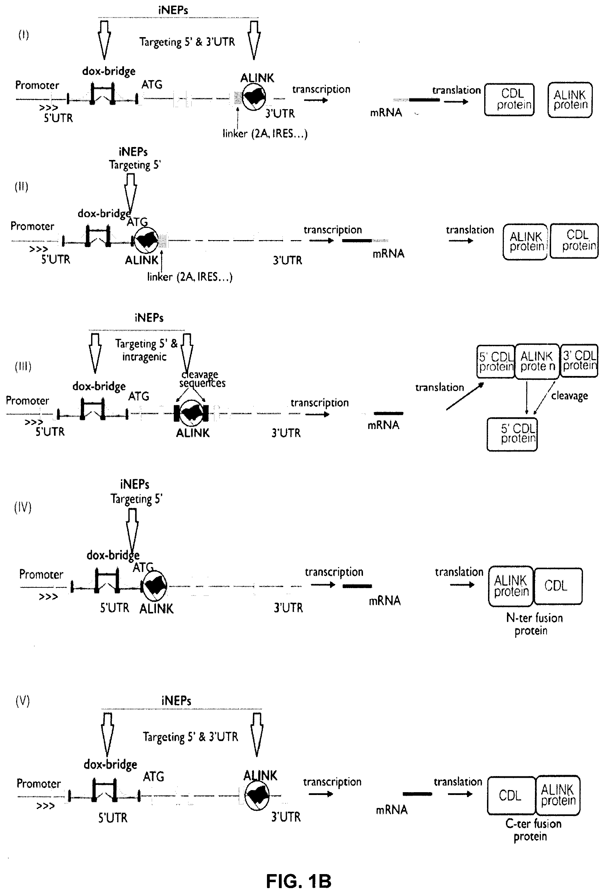

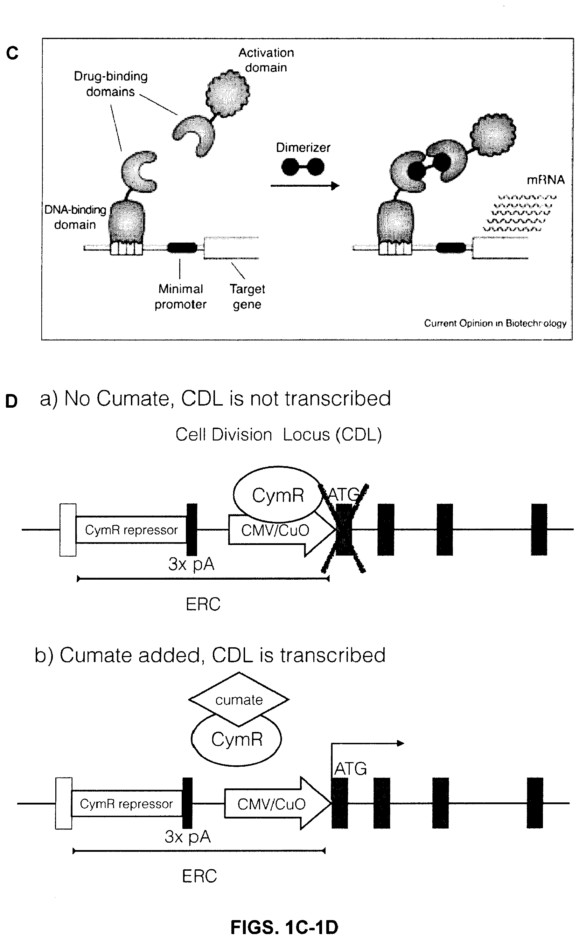

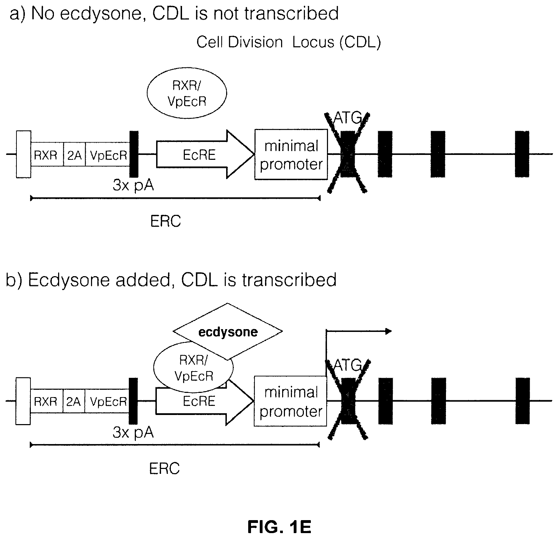

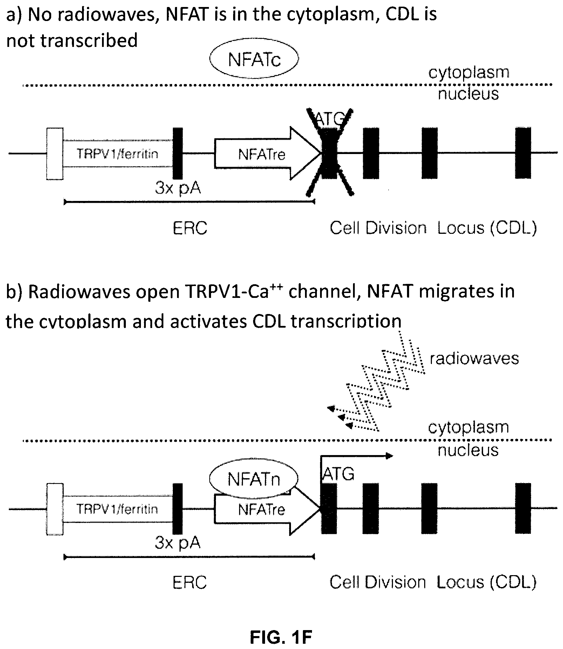

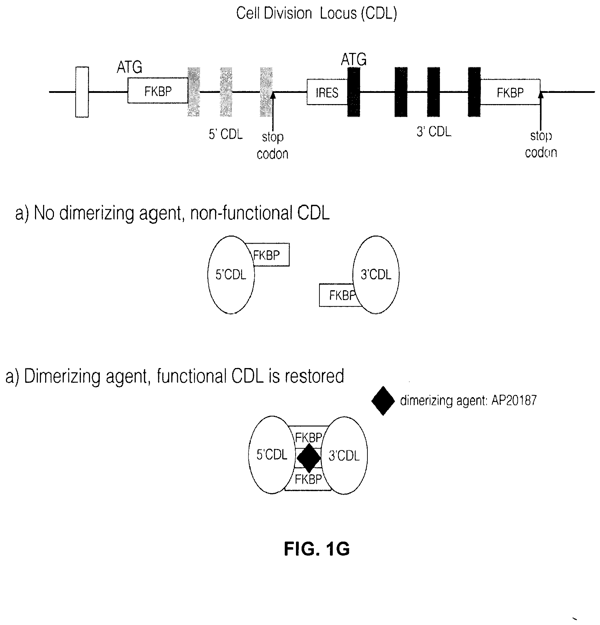

Figures 1A-1G depict schematics illustrating the concept of induced negative effectors of proliferation (iNEPs) and examples of iNEP systems contemplated for use in the methods and tools provided herein. FIG. 1A depicts a schematic representing different examples of iNEP-modified CDLs, including a homozygous modification in CDL1, homozygous insertions in CDL1 and CDL2, CDL comprising two separate loci that together are essential for cell division (CDL3). FIG. 1B depicts schematics representing examples of iNEP comprising an ablation link (ALINK) and an exogenous activator of regulation of a CDL (EARC) in different configurations. FIG. 1C depicts a schematic illustrating transcription activator-like effector (TALE) technology combined with dimerizer-regulated expression induction. FIG. 1D depicts a schematic illustrating a reverse-cumate-Trans-Activator (rcTA) system. FIG. 1E depicts a schematic illustrating a retinoid X receptor (RXR) and an N-terminal truncation of ecdysone receptor (EcR) fused to the activation domain of Vp16 (VpEcR). FIG. 1F depicts a schematic illustrating a transient receptor potential vanilloid-1 (TRPV1), together with ferritin, which is one example of an iNEP system, as set forth herein. FIG. 1G depicts a schematic illustrating how an IRES and a dimerization agent may be used as an iNEP.

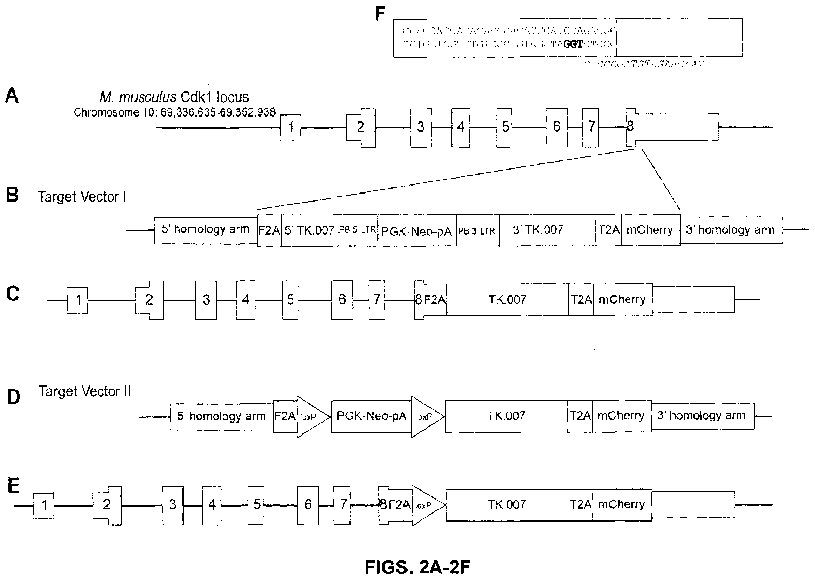

Figures 2A-2F depict schematics illustrating targeting HSV-TK into the 3'UTR of the Cdk1 locus to generate an ALINK, which enables elimination of dividing modified CDK1-expressing cells. FIG. 2A shows a schematic of the mouse *Cdk1* locus. FIG. 2B shows a schematic of mouse target vector I. FIG. 2C shows a schematic of a Cdk1^{TC} allele. FIG. 2D shows a schematic of mouse target vector II. FIG. 2E shows a schematic of a Cdk1^{TClox} allele. FIG. 2F depicts the position of the CRISPR guide RNA; the sequence in the yellow box is the 8th exon of Cdk1.

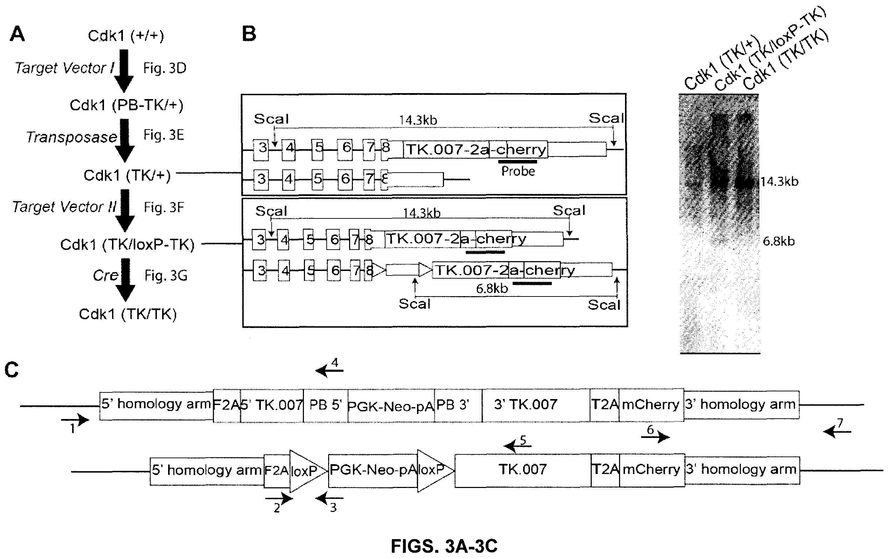

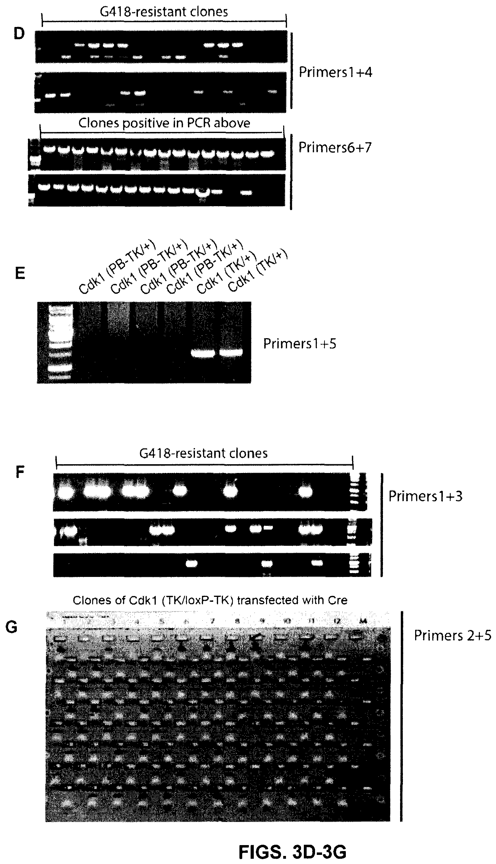

Figures 3A-3G depict generation of ALINK example, HSV-TK-mCherry into the 3'UTR of the CDK1 locus to generate ALINK in mouse ES cell lines. Fig. 3A shows the overall steps of generating ALINK in mouse C2 ES cells. Fig. 3B shows southern blotting result of correct genotyping of Cdk1(TK/+), Cdk1(TK, loxP-TK), and Cdk1 (TK/TK). Fig. 3C shows the locations of the primers used in ALINK genotyping in mouse cells. Fig. 3D includes PCR results illustrating targeting of Targeting Vector I into the 3'UTR of the CDK1 locus. Fig. 3E shows PCR results illustrating the excision event of selection marker in a mouse ES cell line already correctly targeted with Targeting Vector I to activate the expression of HSV-TK-mCherry. Fig. 3F shows PCR results illustrating targeting of Targeting Vector II into Cdk1(TK/+) cells. Fig. 3G shows PCR results illustrating the excision event of selection marker in Cdk1(TK, loxP-TK) to activate the 2^{nd} allele expression of HSV-TK-mCherry, thus generating Cdk1(TK/TK).

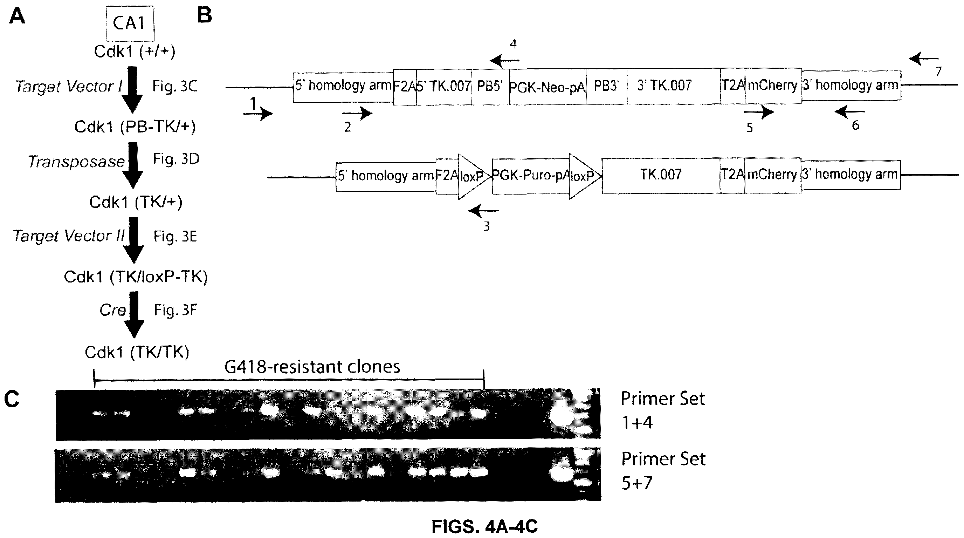

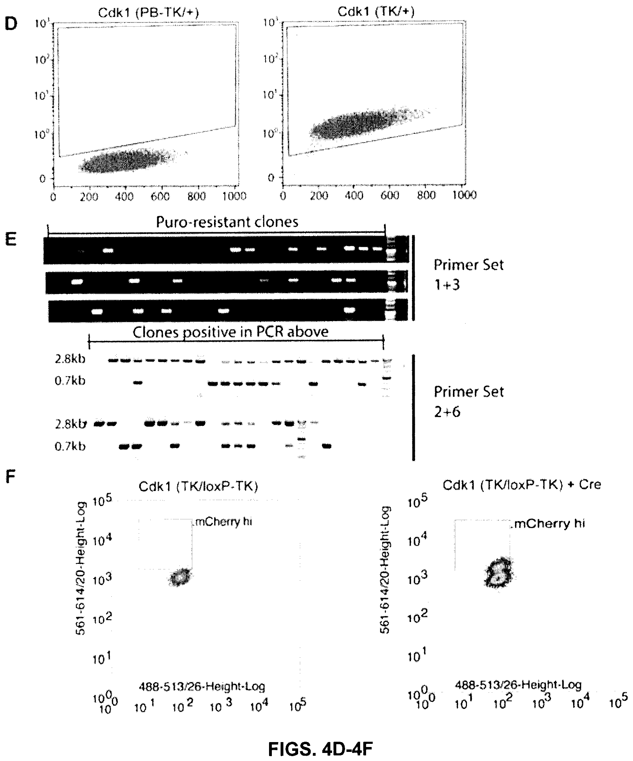

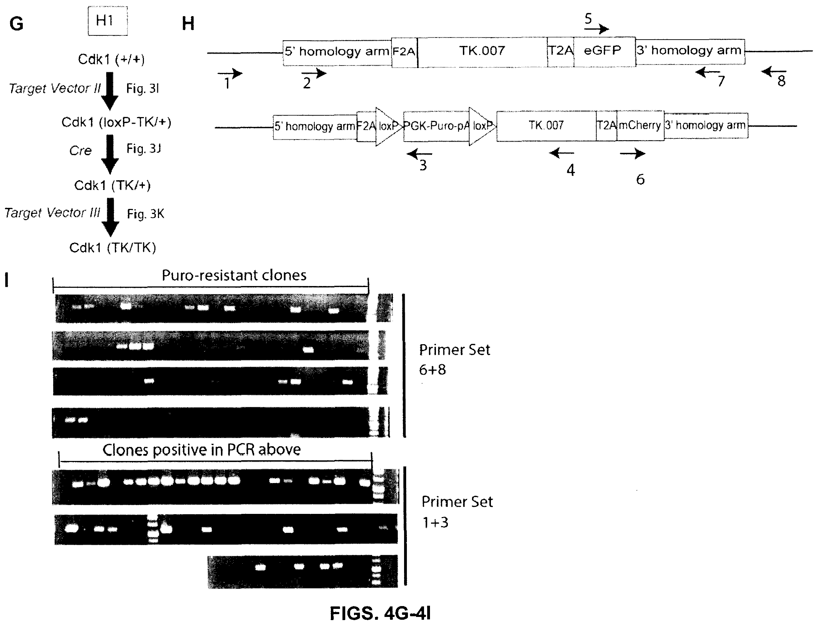

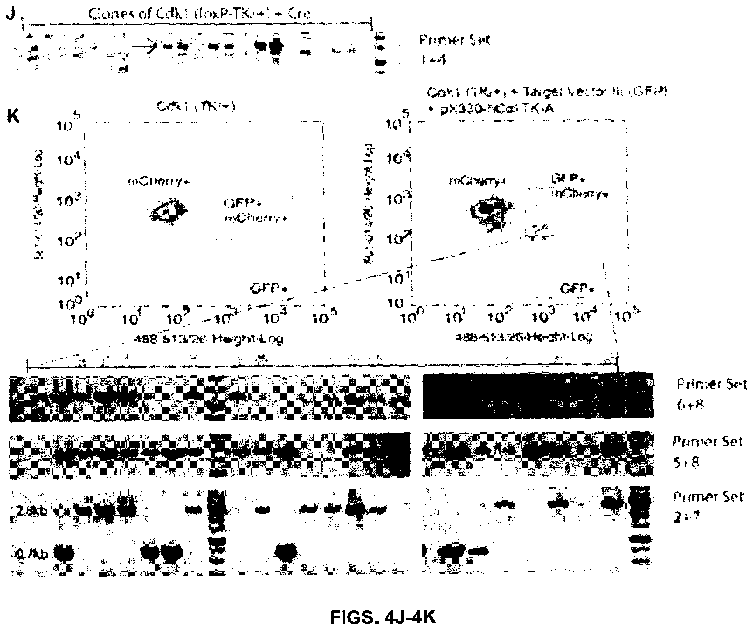

Figures 4A-4K depict generation of an ALINK modification, HSV-TK-mCherry into the 3'UTR of the CDK1 locus, in human ES cell lines. Fig. 4A shows the overall steps of generating ALINK in human CA1 ES cells. Fig. 4B shows the locations of the primers used in ALINK genotyping in human CA1 cells. Fig. 4C shows PCR results illustrating targeting of Targeting Vector I into the 3'UTR of the CDK1 locus. Fig. 4D shows flow cytometry illustrating the excision event of selection marker in human Cdk1 (PB-TK/+) ES cell line to activate the expression of HSV-TK-mCherry; the Y-axis shows the mCherry expression level, while the X-axis is an autofluorescence channel. Fig. 4E shows PCR results illustrating targeting of Targeting Vector II (puro-version) into Cdk1(TK/+) cells; the upper panel is PCR using primers flanking the 5'homology arm; the lower panel is PCR using primers inside 5' and 3' homology arm, so absence of 0.7kb band and presence of 2.8kb band means that the clone is homozygous in ALINK, and presence of 0.7kb band means that the clone is heterozygous in ALINK or the population is not clonal. Fig. 4F shows flow cytometry analysis illustrating the excision event of selection marker in Cdk1(TK, loxP-TK) to activate the 2nd allele expression of HSV-TK-mCherry; the Y-axis shows the mCherry expression level, while the X-axis is an autofluorescence channel. Fig. 4G shows the overall steps of generating ALINK in human H1 ES cells. Fig. 4H shows the locations of the primers used in ALINK genotyping in human H1 cells. Fig. 4I shows PCR results illustrating targeting of Targeting Vector II into the 3'UTR of the CDK1 locus. Fig. 4J shows PCR results illustrating the excision event of selection marker in human H1 Cdk1 (loxP-TK/+) to activate the expression of HSV-TK-mCherry; the Y-axis shows the mCherry expression level, while the X-axis is an autofluorescence channel. Fig. 4K shows fluorescence-activated cell sorting (FACS) of targeting of Targeting Vector III (GFP-version) into Cdk1 (TK/+) cells. After FACS sorting, clones picked from sparse plating were genotyped with mCherry-allele-specific primers, eGFP-allele-specific primers and primers in 5' and 3' homology arms; clones labeled with orange star sign are homozygous ALINK with one allele of mCherry and one allele of eGFP; the one clone labeled with green star sign is homozygous ALINK with two alleles of eGFP.

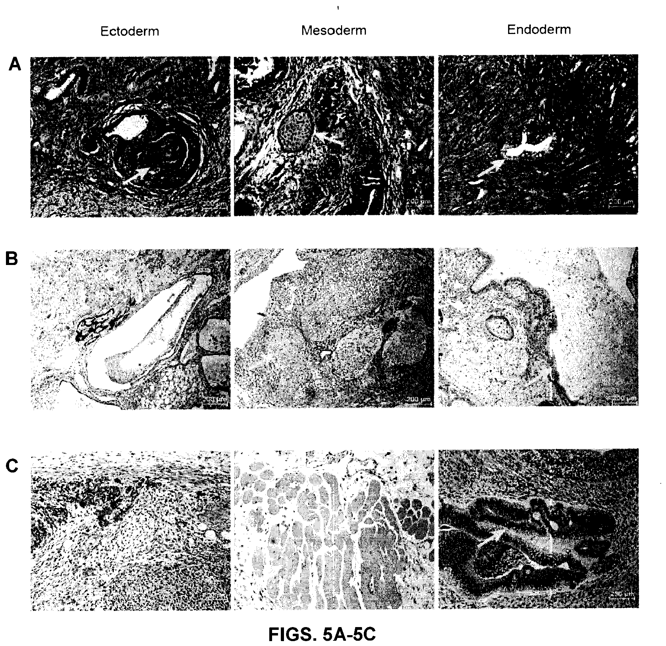

Figures 5A-C depict teratoma histology (endoderm, mesoderm and ectoderm portions of the teratoma are shown from left to right, respectively). FIG. 5A depicts photomicrographs of a teratoma derived from a mouse ES Cdk1^{+/+,alink/alink} cell. FIG 5B depicts photomicrographs of a teratoma derived from a mouse ES Cdk1^{earc/earc, alink/alink} cell. FIG. 5C depicts photomicrographs of a teratoma derived from a human ES Cdk1^{+/+, alink/alink} cell.

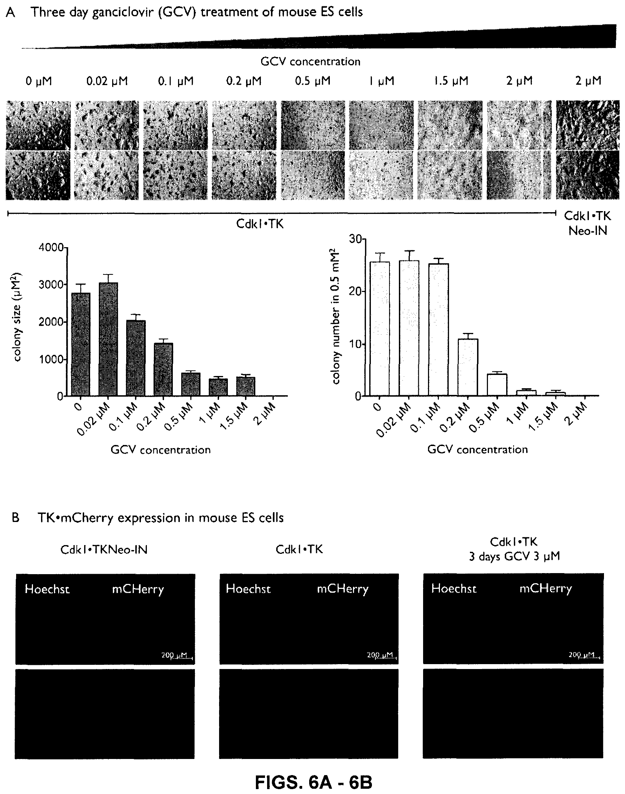

Figures 6A-6B depict *in vitro* functional analysis of mouse ES cells with an HSV-TK- mCherry knock-in into the 3'UTR of the CDK1 locus. FIG. 6A illustrates killing efficiency provided by the TK.007 gene after cells were exposed to different concentrations of GCV for 3 days. Colony size and number are directly proportional to GCV concentration. The second lowest concentration of 0.01 µM did not affect the colony number but slowed down cell growth as evidenced by the reduced colony size (n=5). FIG. 6B illustrates expression of mCherry before (Cdk1•HSV-TK•NeolN) and after (Cdk1•HSV-TK) PB-mediated removal of the neo-cassette.

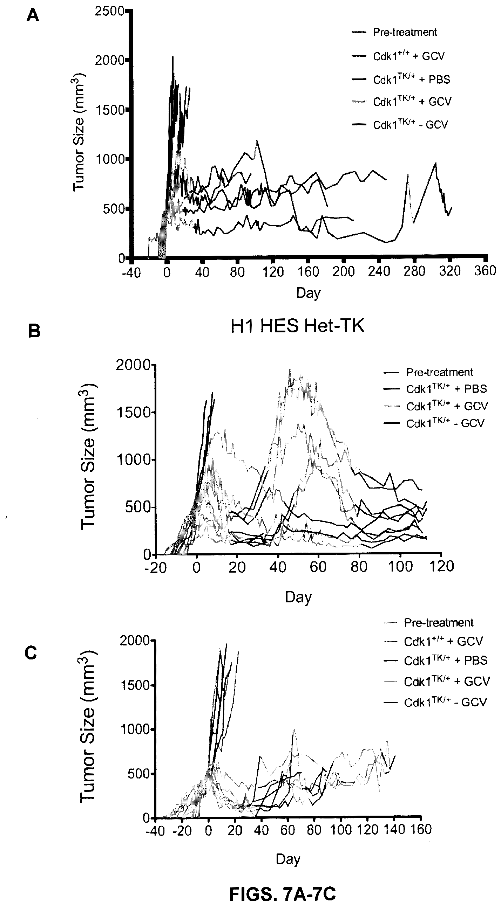

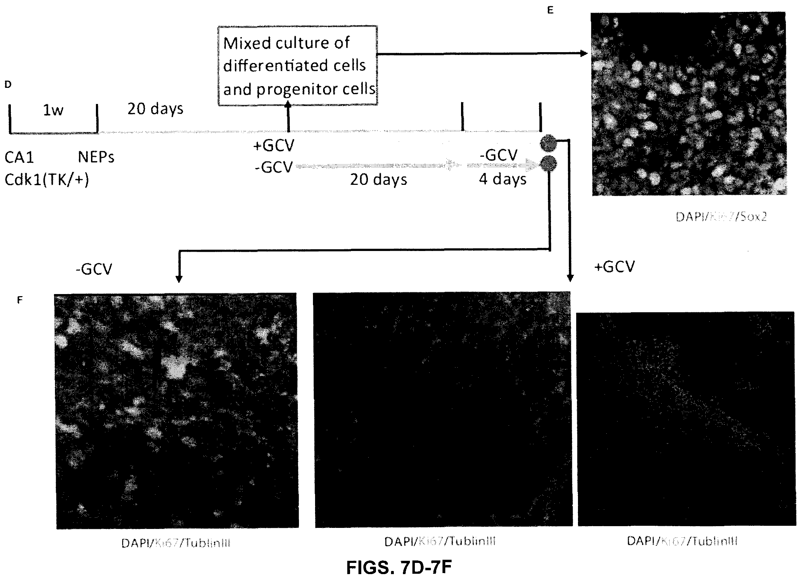

Figures 7A-F depict results of cellular experiments using ALINK-modified cells. FIG. 7A graphically depicts results of GCV treatment of subcutaneous teratomas comprising ALINK-modified mouse C2 cells. FIG. 7B graphically depicts results of GCV treatment of subcutaneous teratomas comprising ALINK-modified H1 ES cells. FIG. 7C graphically depicts results of GCV treatment of mammary gland tumors comprising ALINK-modified cells. FIG. 7D schematically depicts experimental design of neural assay. FIG. 7E is a microscopic image of Neural Epithelial Progenitor (NEP) cells derived from Cdk1^{+/+, +/alink} human CA1 ES cells. FIG. 7F depicts microscopic images illustrating GCV-induced killing of dividing ALINK-modified NEPs and non-killing of non-dividing neurons.

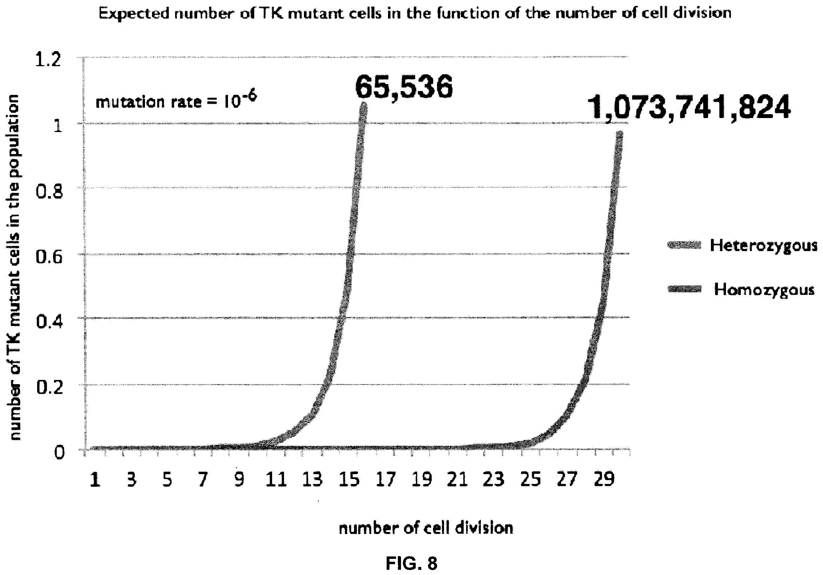

Figure 8 depicts a graph showing the expected number of cells comprising spontaneous mutations in the HSV-TK gene as a population is expanded from heterozygous (blue line) and homozygous (red line) ALINKcells.

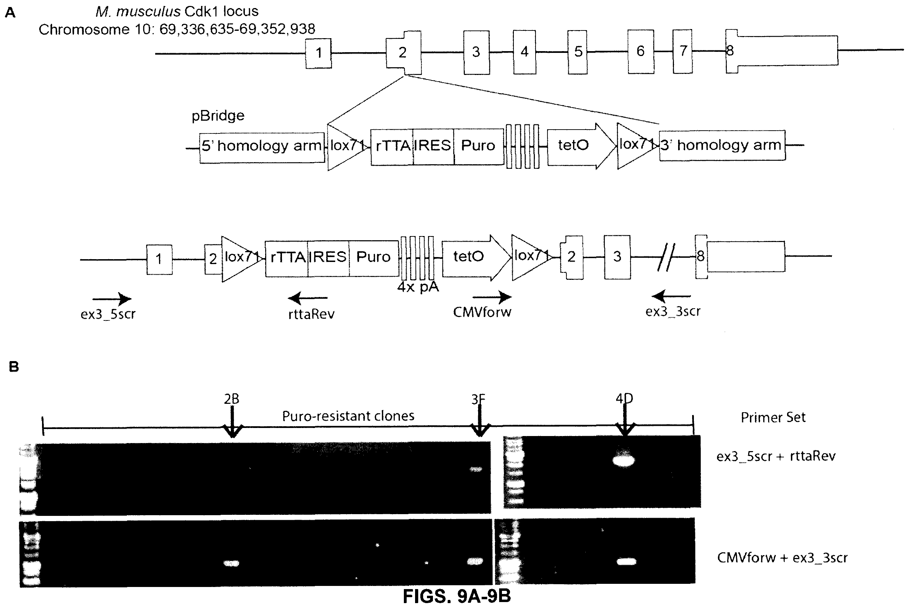

Figures 9A-9B depict targeting of a dox-bridge into the 5'UTR of the mouse Cdk1 locus to generate EARC and behavior of the bridge after insertion into Cdk1. FIG. 9A is a schematic illustrating the structure of the mouse Cdk1 locus, the target vector, and the position of the primers used for genotyping for homologous recombination events. FIG. 9B depicts PCR results showing the genotyping of the puromycin resistant colonies to identify those that integrated the dox-bridge to the Cdk1 5'UTR.

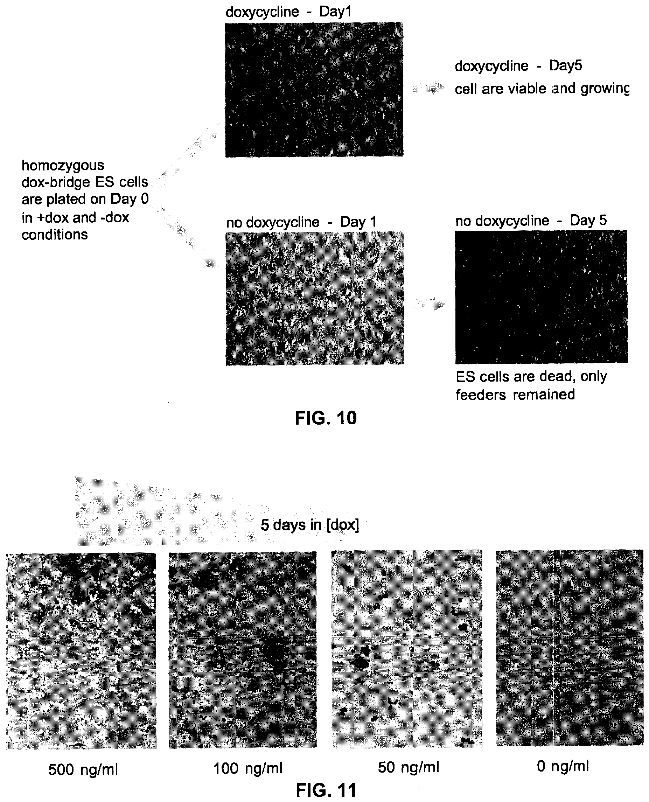

Figure 10 depicts a flowchart illustrating that ES cells having a homozygous dox-bridge knock-in survive and divide only in the presence of doxycycline (or drug with doxycycline overlapping function).

Figure 11 depicts representative photomicrographs illustrating that homozygous dox-bridge knock-in ES cells show doxycycline concentration dependent survival and growth.

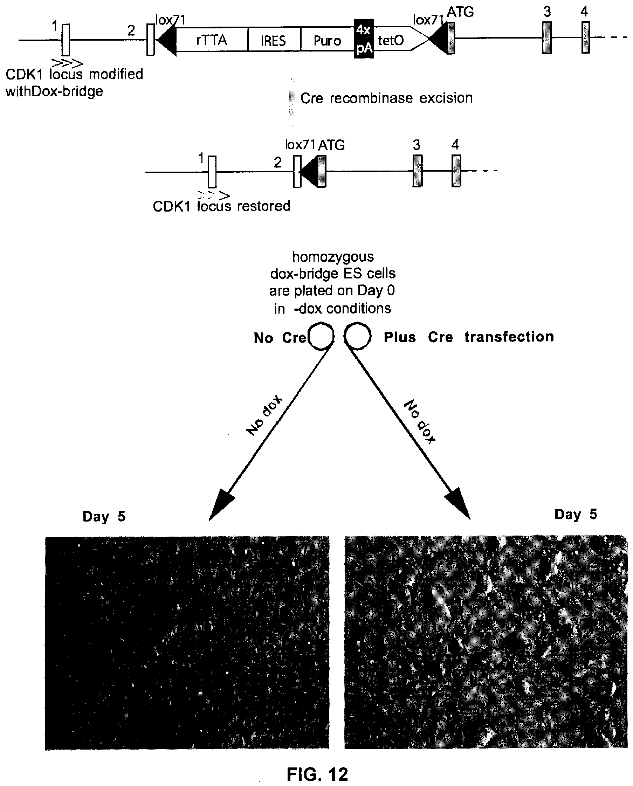

Figure 12 depicts dox-bridge removal with Cre recombinase-mediated excision, which rescues the doxycycline dependent survival of the ES cells.

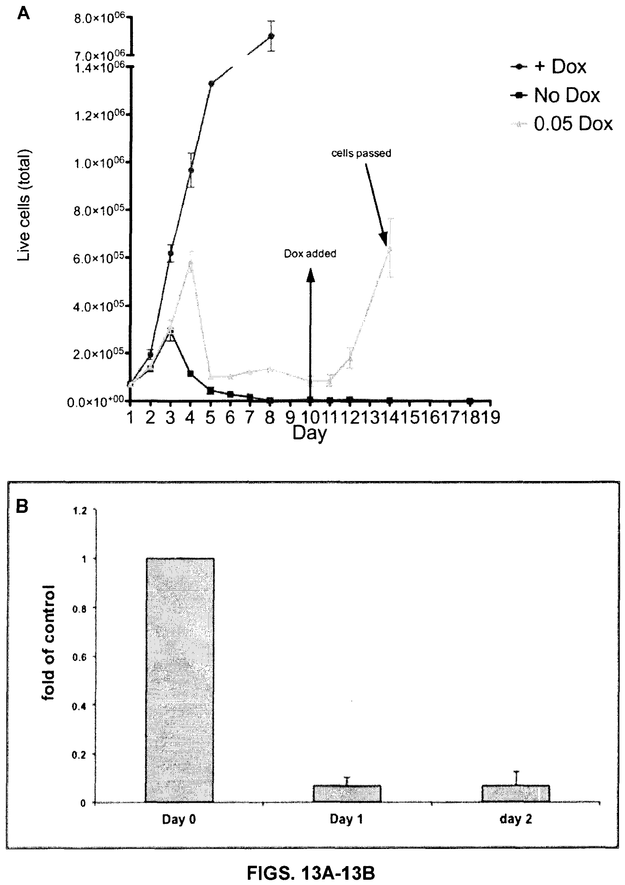

Figures 13A-13B depict the effect of doxycycline withdrawal on the growth of dox-bridged ES cells. FIG. 13A depicts a graph showing that in the presence of doxycycline the cells grew exponentially (red line with circle), indicating their normal growth. Upon doxycycline withdrawal on Day 1, the cells grew only for two days and then they started disappearing from the plates until no cell left on Day 9 on (dark blue line with square). The 20x lower doxycycline concentration (50ng/ml) after an initial 3 days of growth kept a constant number of cells on the plate for at least five days (Fig. 13, light blue line with triangle). On Day 10 the normal concentration of doxycycline was added back to the plates and the cells started growing again as normal ES cells. FIG. 13B depicts a bar graph showing the level of Cdk1 mRNA (as measured by quantitative-PCR) after 0, 1 and 2 days of Dox removal. Expression levels are normalized to beta-actin.

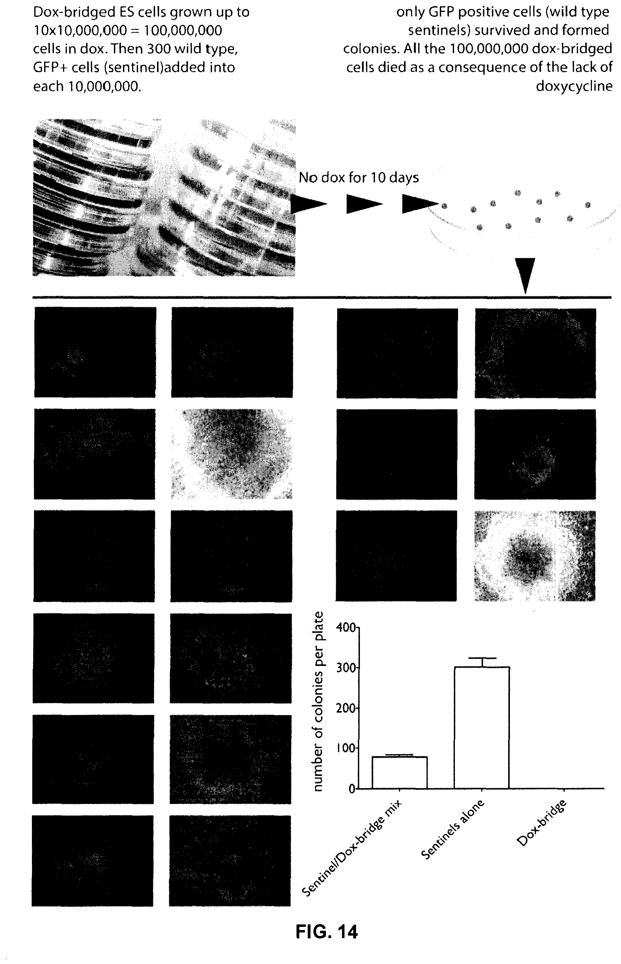

Figure 14 depicts the process of growing dox-bridged ES cells and illustrates that no escaper cells were found among 100,000,000 dox-bridged ES cells when doxycycline was withdrawn from the media, but the sentinel (wild type, GFP positive) cells survived with high efficiency.

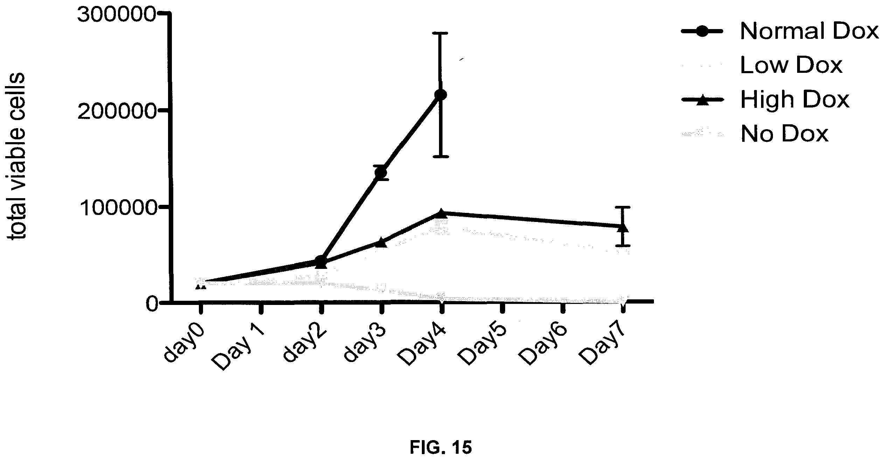

Figure 15 depicts a graph showing the effect of high doxycycline concentration (10 µg/ml) on dox-bridged ES cells: in the presence of high doxycycline, the cells slow down their growth rate similarly to when in low-doxycycline (high dox was 10 µg/ml, normal dox was 1 µg/ml, low dox was 0.05 µg/ml), indicating that there is a window for Dox concentration defining optimal level of CDK1 expression for cell proliferation.

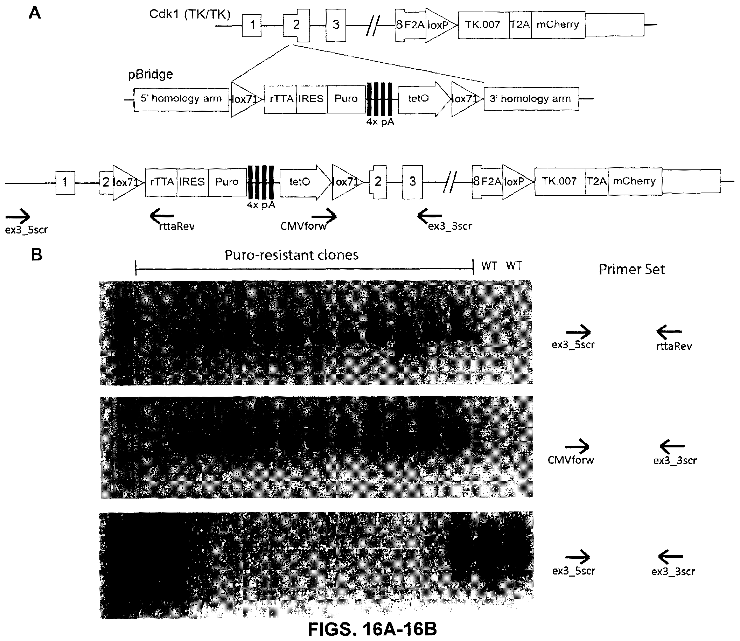

Figures 16A-16B depict targeting of dox-bridge into the 5'UTR of the Cdk1 locus of mouse cells comprising ALINK modifications (i.e., Cdk1 (TK/TK) cells; the cell product described in FIGS. 3A-3G). FIG. 16A is a schematic illustrating the structure of the Cdk1 locus in Cdk1(TK/TK) cells, the bridge target vector, and the location of genotyping primers. FIG. 16B depicts PCR results showing the genotyping of the puromycin resistant colonies to identify those that integrated the dox-bridge to the Cdk1 5'UTR in mouse Cdk1(TK/TK) cells, thus generating mouse cell product Cdk1^{earc/earc, alink/alink}.

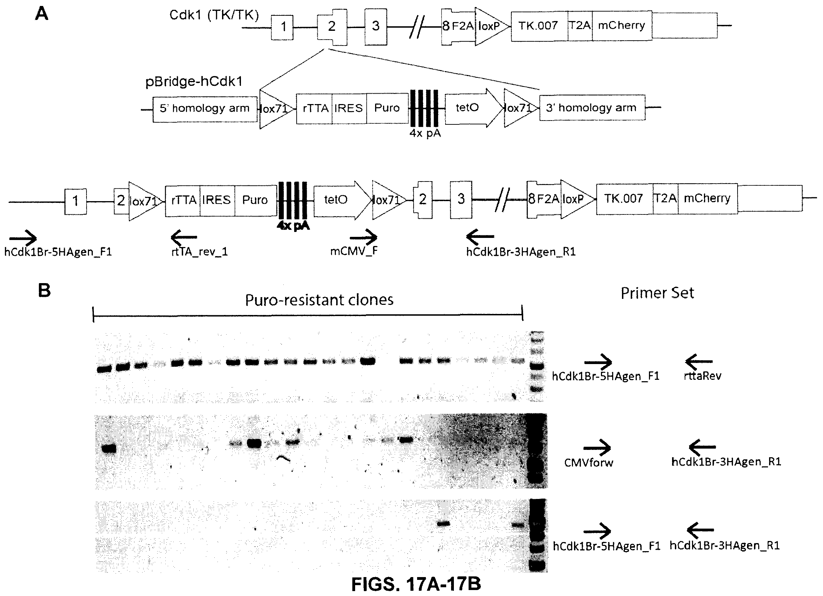

Figures 17A-17B depict targeting of dox-bridge into the 5'UTR of the Cdk1 locus of human cells comprising ALINK modifications (i.e., Cdk1 (TK/TK) cells; the cell product described in FIGS. 4A-4F). FIG. 17A is a schematic illustrating the structure of the Cdk1 locus in Cdk1(TK/TK) cells, the bridge target vector, and the location of genotyping primers. Fig. 17B depicts PCR results showing the genotyping of the puromycin resistant colonies to identify those that integrated the dox-bridge to the Cdk1 5'UTR in human Cdk1(TK/TK) cells, thus generating human cell product Cdk1^{earc/earc,alink/alink}.

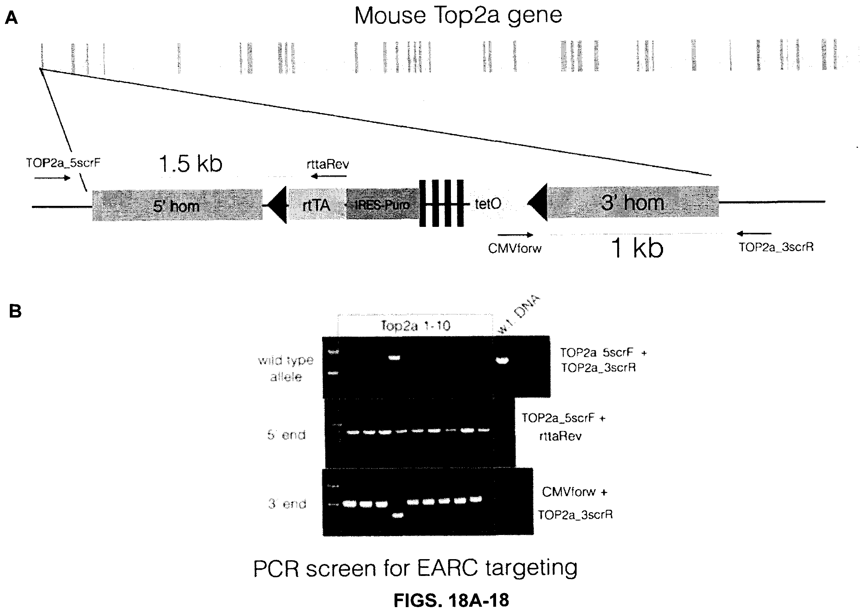

Figures 18A-18B depict targeting of a dox-bridge into the 5'UTR of the Top2 locus to generate EARC insertion into Top2a. FIG. 18A is a schematic illustrating the structure of the Top2a locus and the target vector. TOP2a_5scrF, rttaRev, CMVforw and TOP2a_3scrR indicate the position of the primers used for genotyping for homologous recombination events. FIG. 18B depicts PCR results showing the genotyping of the puro resistant colonies to identify those that integrated the dox-bridge to the Top2a 5'UTR. Nine of these cell lines was found to be homozygous targeted comprising a dox-bridge inserted by homologous recombination into the 5'UTR of both alleles of Top2a.

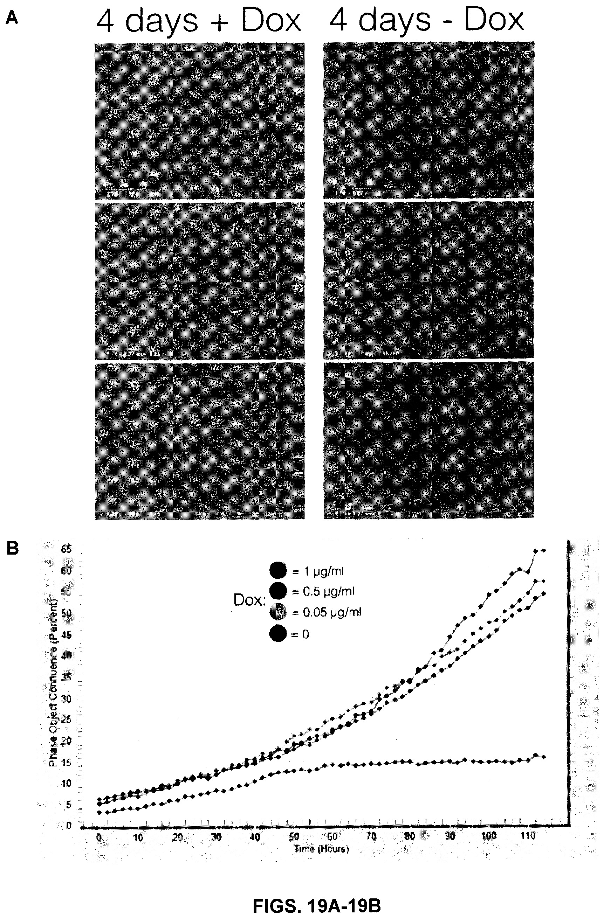

Figures 19A-19B depict the effect of doxycycline withdrawal on the growth of Top2a-EARC ES cells. FIG. 19A shows that withdrawal of doxycycline results in complete elimination of mitotically active ES cells within 4 days. FIG. 19B depicts how different concentrations of doxycycline affected proliferation of the dox-bridge ES cells by measuring cell growth for 4 days. ES cells in the presence of doxycycline grew exponentially, indicating their normal growth. In contrast, two days after doxycycline removal, cells growth was completely arrested.

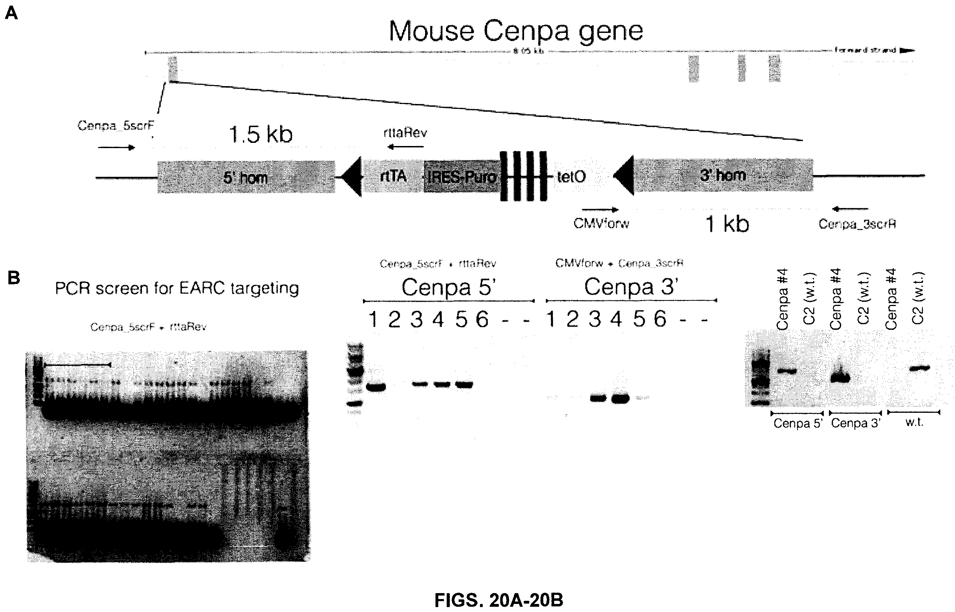

Figures 20A-20B depict targeting of a dox-bridge into the 5'UTR of the Cenpa locus to generate EARC insertion into Cenpa. FIG. 20A is a schematic illustrating the structure of the Cenpa locus and the target vector. Cenpa_5scrF, rttaRev, CMVforw and Cenpa_3scrR indicate the position of the primers used for genotyping for homologous recombination events. FIG. 20B depicts PCR results showing the genotyping of the puro resistant colonies to identify those that integrated the dox-bridge to the Cenpa 5'UTR. Six of these cells were found to have a correct insertion at the 5' and 3', and at least one clone (Cenpa#4), was found to have homozygous targeting comprising a dox-bridge inserted by homologous recombination into the 5'UTR of both alleles of Cenpa.

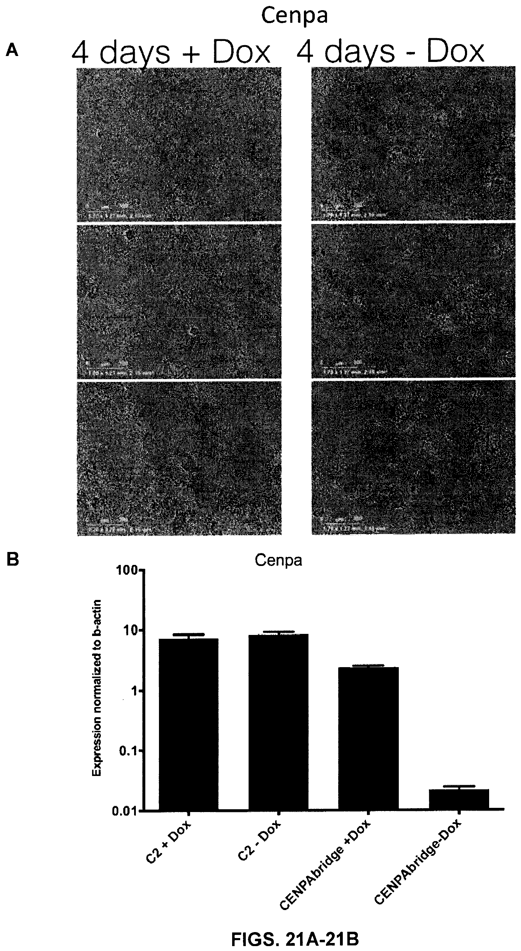

Figures 21A-21B depict the effect of doxycycline withdrawal on the growth of Cenpa-EARC ES cells. FIG. 21A depicts that withdrawal of doxycycline results in complete elimination of mitotically active ES cells within 4 days. FIG. 21B is the Cenpa gene expression level (determined by q-PCR) in Cenpa-EARC cells with Dox and after 2 days of Dox removal, and compared it to the expression level in wild type mouse ES cells (C2). As expected Cenpa expression level is greatly reduced in Cenpa-EARC cells without Dox for 2 days.

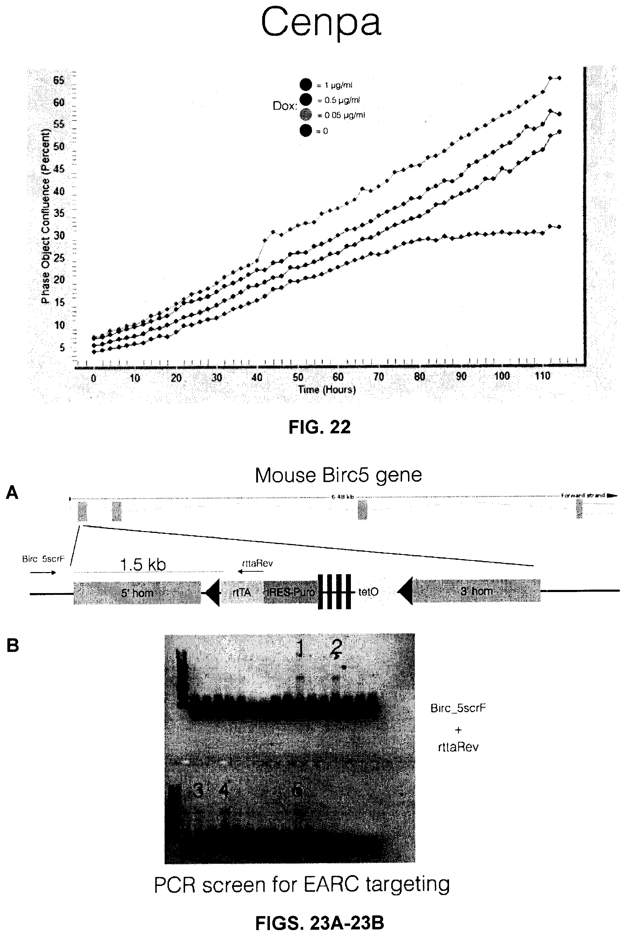

Figure 22 depicts how different concentrations of doxycycline affected proliferation of the Cenpa-EARC ES cells by measuring cell growth for 4 days. ES cells in the presence of doxycycline grew exponentially, indicating their normal growth. In contrast, 80 hours after doxycycline removal, cells growth was completely arrested.

Figures 23A-23B depict targeting of a dox-bridge into the 5'UTR of the Birc5 locus to generate EARC insertion into Birc5. FIG. 23A is a schematic illustrating the structure of the Birc5 locus and the target vector. Birc_5scrF and rttaRev indicate the position of the primers used for genotyping for homologous recombination events. FIG. 23B depicts PCR results showing the genotyping of the puro resistant colonies to identify those that integrated the dox-bridge to the Birc5 5'UTR. Five clones were found to be correctly targeted comprising a dox-bridge inserted by recombination into the 5'UTR of both alleles of Birc5. One of these clones was Birc#3, was found to stop growing or die in the absence of Dox.

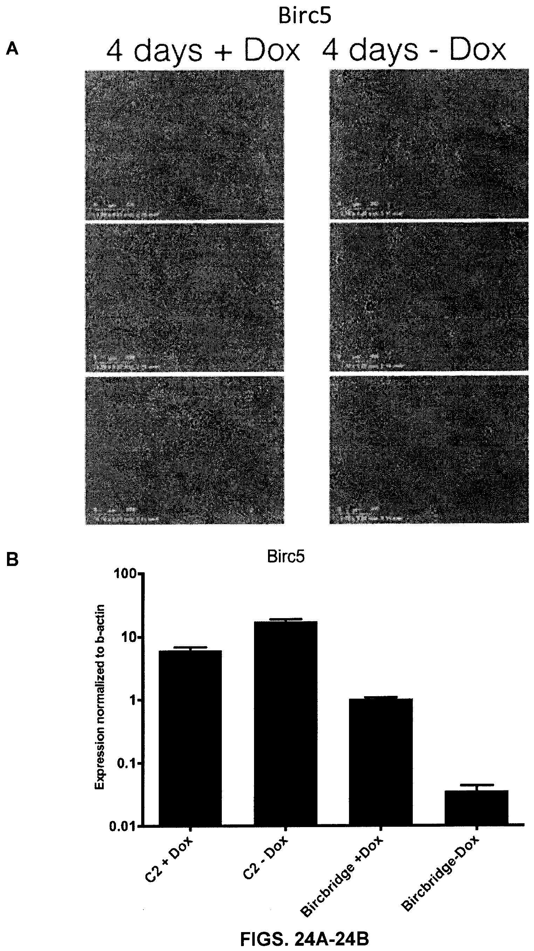

Figures 24A-24B depict the effect of doxycycline withdrawal on the growth of Birc5-EARC ES cells. FIG. 24A depicts that withdrawal of doxycycline results in complete elimination of mitotically active ES cells within 4 days. FIG. 24B is the Birc5 gene expression level (determined by q-PCR) in Birc5-EARC cells with Dox and after 2 days of Dox removal, and compared it to the expression level in wild type mouse ES cells (C2). As expected Birc5 expression level is greatly reduced in Birc5-EARC cells without Dox for 2 days.

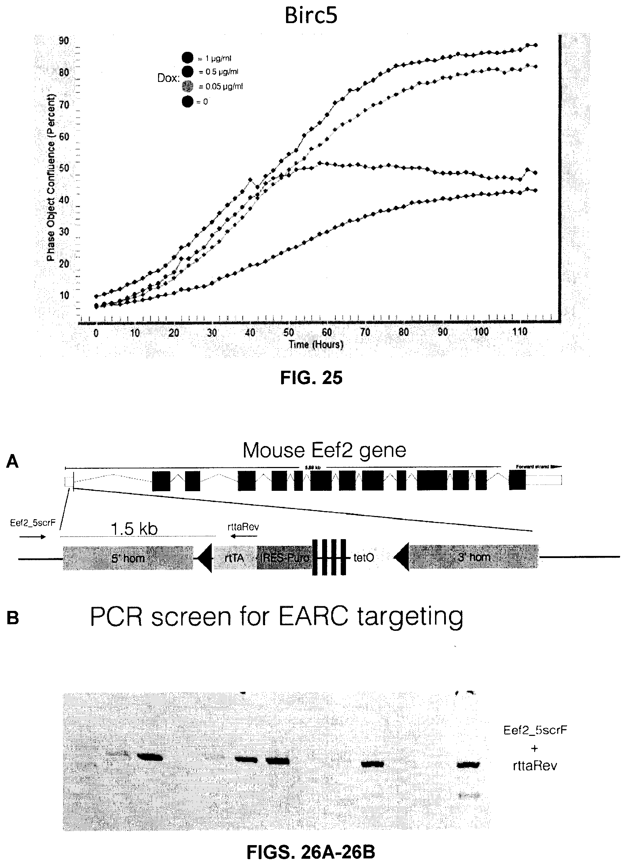

Figure 25 depicts how different concentrations of doxycycline affected proliferation of the Birc5-EARC ES cells by measuring cell growth for 4 days. ES cells in the presence of doxycycline grew exponentially, indicating their normal growth. In contrast, 50 hours after doxycycline removal, cells growth was completely arrested. Interestingly, it appears that lower Dox concentrations (0.5 and 0.05 µg/ml) promote better cell growth than a higher concentration (1 µg/ml).

Figures 26A-26B depict targeting of a dox-bridge into the 5'UTR of the Eef2 locus to generate EARC insertion into Eef2. FIG. 26A is a schematic illustrating the structure of the Eef2 locus and the target vector. Eef2_5scrF and rttaRev indicate the position of the primers used for genotyping for homologous recombination events. FIG. 26B depicts PCR results showing the genotyping of the puro resistant colonies to identify those that integrated the dox-bridge to the Eef2 5'UTR. Nine of these cell lines was found to be correctly targeted with at least one clone growing only in Dox-media.

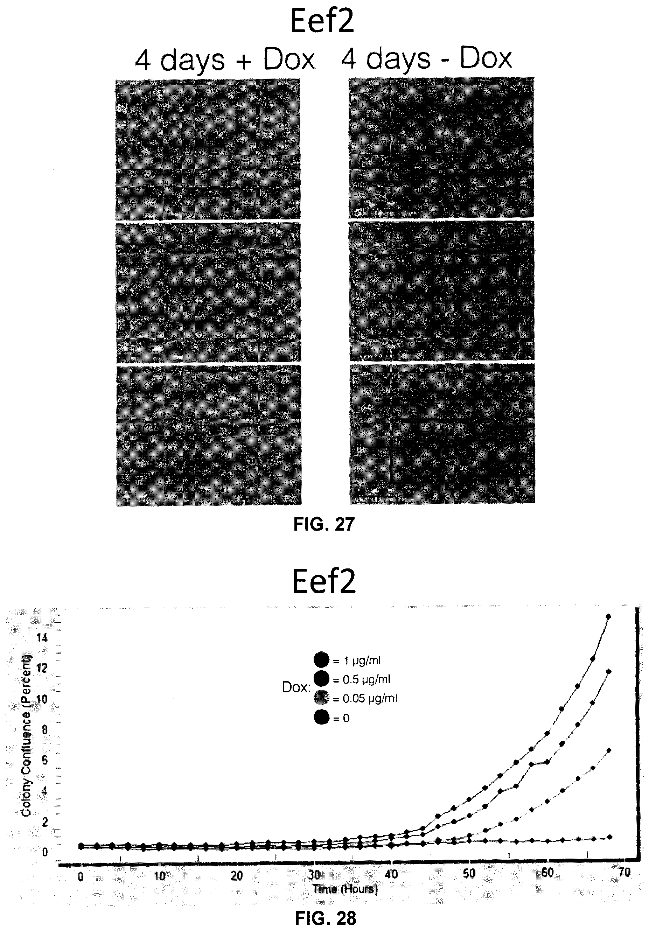

Figures 27 depict the effect of doxycycline withdrawal on the growth of Eef2-EARC ES cells. Withdrawal of doxycycline results in complete elimination of mitotically active ES cells within 4 days.

Figure 28 depicts how different concentrations of doxycycline affected proliferation of the Eef2-EARC ES cells by measuring cell growth for 4 days. ES cells in the presence of doxycycline grew exponentially, indicating their normal growth. In contrast, without doxycycline cells completely fail to grow.

### DETAILED DESCRIPTION OF THE DISCLOSURE

Unless defined otherwise, all technical and scientific terms used herein have the same meaning as commonly understood by one of ordinary skill in the art to which this disclosure belongs.

### Definitions

The terms "cell division locus", "cell division loci", and "CDL" as used herein, refer to a genomic locus (or loci) whose transcription product(s) is expressed by dividing cells. When a CDL comprises a single locus, absence of CDL expression in a cell (or its derivatives) means that tumour initiation and/or formation is prohibited either because the cell(s) will be ablated in the absence of CDL expression or because proliferation of the cell(s) will be blocked or compromised in the absence of CDL expression. When a CDL comprises multiple loci, absence of expression by all or subsets of the loci in a cell (or its derivatives) means that tumour initiation and/or formation is prohibited either because the cell(s) will be ablated in the absence of CDL expression or because proliferation of the cell(s) will be blocked or compromised in the absence of CDL expression. A CDL may or may not be expressed in non-dividing and/or non-proliferating cells. A CDL may be endogenous to a host cell or it may be a transgene. If a CDL is a transgene, it may be from the same or different species as a host cell or it may be of synthetic origin. In an embodiment, a CDL is a single locus that is transcribed during cell division. For example, in an embodiment, a single locus CDL is CDK1. In an embodiment, a CDL comprises two or more loci that are transcribed during cell division. For example, in an embodiment, a multi-locus CDL comprises two MYC genes (c-Myc and N-myc) (Scognamiglio et al., 2016). In an embodiment, a multi-locus CDL comprises AURORA B and C kinases, which may have overlapping functions (Fernandez-Miranda et al., 2011). Cell division and cell proliferation are terms that may be used interchangeably herein.

The terms "normal rate of cell division", "normal cell division rate", "normal rate of cell proliferation", and "normal cell proliferation rate" as used herein, refer to a rate of cell division and/or proliferation that is typical of a non-cancerous healthy cell. A normal rate of cell division and/or proliferation may be specific to cell type. For example, it is widely accepted that the number of cells in the epidermis, intestine, lung, blood, bone marrow, thymus, testis, uterus and mammary gland is maintained by a high rate of cell division and a high rate of cell death. In contrast, the number of cells in the pancreas, kidney, cornea, prostate, bone, heart and brain is maintained by a low rate of cell division and a low rate of cell death (Pellettieri and Sánchez Alvarado, 2007).

The terms "inducible negative effector of proliferation" and "iNEP" as used herein, refer to a genetic modification that facilitates use of CDL expression to control cell division and/or proliferation by: i) inducibly stopping or blocking CDL expression, thereby prohibiting cell division and proliferation; ii) inducibly ablating at least a portion of CDL-expressing cells (i.e., killing at least a portion of proliferating cells); or iii) inducibly slowing the rate of cell division relative to a cell's normal cell division rate, such that the rate of cell division would not be fast enough to contribute to tumor formation.

The terms "ablation link" and "ALINK" as used herein, refer to an example of an iNEP, which comprises a transcriptional link between a CDL and a sequence encoding a negative selectable marker. The ALINK modification allows a user to inducibly kill proliferating host cells comprising the ALINKor inhibit the host cell's proliferation by killing at least a portion of proliferating cells by exposing the ALINK-modified cells to an inducer of the negative selectable marker. For example, a cell modified to comprise an ALINK at a CDL may be treated with an inducer (e.g., a prodrug) of the negative selectable marker in order to ablate proliferating cells or to inhibit cell proliferation by killing at least a portion of proliferating cells (Figure 1B).

The terms "exogenous activator of regulation of CDL" and "EARC" as used herein, refer to an example of an iNEP, which comprises a mechanism or system that facilitates exogenous alteration of non-coding or coding DNA transcription or corresponding translation via an activator. An EARC modification allows a user to inducibly stop or inhibit division of cells comprising the EARC by removing from the EARC-modified cells an inducer that permits transcription and/or translation of the EARC-modified CDL. For example, an inducible activator-based gene expression system may be operably linked to a CDL and used to exogenously control expression of a CDL or CDL translation, such that the presence of a drug inducible activator and corresponding inducer drug are required for CDL transcription and/or translation. In the absence of the inducer drug, cell division and/or proliferation would be stopped or inhibited (e.g., slowed to a normal cell division rate). For example, the CDL Cdk1/CDK1 may be modified to comprise a dox-bridge (Figure 1B), such that expression of Cdk1/CDK1 and cell division and proliferation are only possible in the presence of an inducer (e.g., doxycycline).

The term "proliferation antagonist system" as used herein, refers to a natural or engineered compound(s) whose presence inhibits (completely or partially) proliferation of a cell.

### General Description of Tools and Methods

As described herein, the inventors have provided molecular tools, methods and kits for using one or more cell division loci (CDL) in an animal cell to generate genetically modified cells in which cell division and/or proliferation can be controlled by a user through one or more iNEPs (FIG. 1A). For example, division of cells generated using one or more tools and/or methods provided herein could be stopped, blocked or inhibited by a user such that a cell's division rate would not be fast enough to contribute to tumor formation. For example, proliferation of cells generated using one or more tools and/or methods provided herein could be stopped, blocked or inhibited by a user, by killing or stopping at least a portion of proliferating cells, such that a cell's proliferation rate or volume may be maintained at a rate or size, respectively, desired by the user.

Tools and methods for controlling cell division and/or proliferation are desirable, for example, in instances wherein faster cell division rates (relative to normal cell division rates) are undesirable. For example, cells that divide at faster than normal rates may form tumors *in situ,* which may be harmful to a host. In an embodiment, the genetically modified animal cells provided herein comprise one or more mechanisms for allowing normal cell division and/or proliferation and for stopping, ablating, blocking and/or slowing cell division and/or proliferation, such that undesirable cell division and/or proliferation may be controlled by a user (FIG. 1B). Referring to FIG. 1B, in example (I) EARC is inserted at the 5' UTR of the CDL and ALINK is inserted at the 3' UTR, the product of transcription is a bi-cistronic mRNA that get processed in two proteins. In example (II) both EARC and ALINK are inserted at the 5' UTR of the CDL, the product of transcription is a bi-cistronic mRNA that get processed in two proteins. In example (III) EARC is inserted at the 5' UTR of the CDL and ALINK is inserted within the CDL coding sequence, the product of transcription is a mRNA that get processed in a precursor protein that will generate two separate protein upon cleavage of specifically designed cleavage sequences. In example (IV) both EARC and ALINK are inserted at the 5' UTR of the CDL, the product of transcription is a mRNA that get processed into a fusion protein that maintains both CDL and ALINK functions. In example (V) EARC is inserted at the 5' UTR of the CDL and ALINK is inserted at the 3' UTR, the product of transcription is a mRNA that get processed into a fusion protein that maintains both CDL and ALINKfunctions.

For example, the genetically modified animal cells provided herein may be used in a cell therapeutic treatment applied to a subject. If one or more of the genetically modified animal cells provided to the subject were to begin dividing at an undesirable rate (e.g., faster than normal), then a user could stop or slow division of cells dividing at the undesirable rate or block, slow or stop cells proliferating at the undesirable rate by i) applying to the cells dividing at the undesirable rate an inducer corresponding to the genetic modification in the cells; or ii) restricting access of the cells dividing at the undesirable rate to an inducer corresponding to the genetic modification in the cells, i) or ii) being determined based on the type of iNEP(s) provided in the genetically modified animal cells.

In an embodiment, the genetically modified animal cells provided herein may be referred to as "fail-safe cells". A fail-safe cell contains one or more homozygous, heterozygous, hemizygous or compound heterozygous ALINKs in one or more CDLs. In an embodiment, a fail-safe cell further comprises one or more EARCs in one or more CDL. In an embodiment, a fail-safe cell comprises a CDL comprising both ALINK and EARC modifications.

As used herein, the term "fail-safe", refers to the probability (designated as pFS) defining a cell number. For example, the number of cells that can be grown from a single fail-safe cell (clone volume) where the probability of obtaining a clone containing cells, which have lost all ALINKs is less than an arbitrary value (pFS). For example, a pFS = 0.01 refers to a scenario wherein if clones were grown from a single cell comprising an ALINK-modified CDL 100 times, only one clone expected to have cells, which lost ALINKfunction (the expression of the negative selectable marker) while still capable of cell division. The fail-safe volume will depend on the number of ALINKs and the number of ALINK-targeted CDLs. The fail-safe property is further described in Table 1.

**Table 1. Fail-safe cell volumes and their relationship to a human body were calculated using mathematical modelling. The model did not take into a count the events when CDL expression was co-lost with the loss of negative selectable marker activity,compromising cell proliferation. Therefore the values are underestimates and were calculated assuming 10-6 forward mutation rate for the negative selectable marker. The estimated number of cells in a human body as 3.72x10¹³ was taken from (Bianconi et al., 2013).**

| CDL # | ALINK# | Genotype in CDLs | Fail-safe volume (#cells) | Relative (x) to a human body=3.72x10¹³ cells | Estimated weight of clones |
|---|---|---|---|---|---|
| 1 | 1 | het | 512 | 0.0000000000137 | 1µg |
| 1 | 2 | hom | 16777216 | 0.000000451 | 31 mg |
| 2 | 3 | het, hom | 1.374E+11 | 0.004 | 0.26 kg |
| 2 | 4 | hom, hom | 1.13E+15 | 30 | 2100 kg |

It is contemplated herein that fail-safe cells may be of use in cell-based therapies wherein it may be desirable to eliminate cells exhibiting undesirable growth rates, irrespective of whether such cells are generated before or after grafting the cells into a host.

### Cell Division Loci (CDLs)

The systems, methods and compositions provided herein are based on the identification of one or more CDLs, such as, for example, the CDLs set forth in Table 2. It is contemplated herein that various CDLs could be targeted using the methods provided herein.

In various embodiments, a CDL is a locus identified as an "essential gene" as set forth in Wang et al., 2015, which is incorporated herein by reference as if set forth in its entirety. Essential genes in Wang et al., 2015, were identified by computing a score (i.e., a CRISPR score) for each gene that reflects the fitness cost imposed by inactivation of the gene. In an embodiment, a CDL has a CRISPR score of less than about-1.0 (Table 2, column 5).

In various embodiments, a CDL is a locus/loci that encodes a gene product that is relevant to cell division and/or replication (Table 2, column 6). For example, in various embodiments, a CDL is a locus/loci that encodes a gene product that is relevant to one or more of: i) cell cycle; ii) DNA replication; iii) RNA transcription and/or protein translation; and iv) metabolism (Table 2, column 7).

In an embodiment, a CDL is one or more cyclin-dependent kinases that are involved with regulating progression of the cell cycle (e.g., control of G1/S G2/M and metaphase-to-anaphase transition), such as CDK1, CDK2, CDK3, CDK4, CDK5, CDK6, CDK7, CDK8, CDK9 and/or CDK11 (Morgan, 2007). In an embodiment, a CDL is one or more cyclins that are involved with controlling progression of the cell cycle by activating one or more CDK, such as, for example, cyclinB, cyclinE, cyclinA, cyclinC, cyclinD, cyclinH, cyclinC, cyclinT, cyclinL and/or cyclinF (FUNG and POON, 2005). In an embodiment, a CDL is one or more loci involved in the anaphase-promoting complex that controls the progression of metaphase to anaphase transition in the M phase of the cell cycle (Peters, 2002). In an embodiment, a CDL is one or more loci involved with kinetochore components that control the progression of metaphase to anaphase transition in the M phase of the cell cycle (Fukagawa, 2007). In an embodiment, a CDL is one or more loci involved with microtuble components that control microtubule dynamics required for the cell cycle (Cassimeris, 1999).

In various embodiments, a CDL is a locus/loci involved with housekeeping. As used herein, the term "housekeeping gene" or "housekeeping locus" refers to one or more genes that are required for the maintenance of basic cellular function. Housekeeping genes are expressed in all cells of an organism under normal and patho-physiological conditions.

In various embodiments, a CDL is a locus/loci that encodes a gene product that is relevant to cell division and/or proliferation and has a CRISPR score of less than about-1.0. For example, in an embodiment, a CDL is a locus/loci that encodes a gene product that is relevant to one or more of: i) cell cycle; ii) DNA replication; iii) RNA transcription and/or protein translation; and iv) metabolism, and has a CRISPR score of less than about -1.0. In an embodiment, the CDL may also be a housekeeping gene.

In an embodiment, to identify potential CDLs, the inventors examined early mouse embryonic lethal phenotypes of gene knockouts (KOs; Table 2, column 8). For example, the inventors found that mouse embryos homozygous null for *Cdk1* (cyclin-dependent kinase 1, also referred to as cell division cycle protein 2 homolog (CDC2)) null mutation die at the 2-cell stage (E1.5) (Santamaría et al., 2007). Cdk1 (referred to as CDK1 in humans) is a highly conserved serine/threonine kinase whose function is critical in regulating the cell cycle. Protein complexes of Cdk1 phosphorylate a large number of target substrates, which leads to cell cycle progression. In the absence of Cdk1 expression, a cell cannot transition through the G2 to M phase of the cell cycle.

Cdk1/CDK1 is one example of a single locus CDL. Genetic modifications of Cdk1/CDK1, in which transcription of the locus is ablated by insertion of an ALINK modification and/or exogenously controlled by insertion of an EARC modification, are examined herein as set forth in Examples 1, 2 and 3. Top2A/TOP2A is one example of a CDL. Cenpa/CEPNA is one example of a CDL. Birc5/BIRC5 is one example of a CDL. Eef2/EEF2 is one example of a CDL. Genetic modifications of Top2a, Cenpa, Birc5, and Eef2 in which transcription of the locus can be exogenously controlled by insertion of an EARC modification are examined herein as set forth in Examples 4-7, respectively.

It an embodiment, is contemplated herein that alternative and/or additional loci are CDLs that could be targeted using the method provided herein.

For example, RNAi screening of human cell lines identified a plurality of genes essential for cell proliferation (Harborth et al., 2001; Kittler et al., 2004). The inventors predicted that a subset of these loci were CDLs after confirming the loci's early embryonic lethal phenotype of mouse deficient of the orthologues and/or analyzing the Loci's GO term and/or genecards (Table 2, column 8).

### Targeting a CDL with an Ablation Link (ALINK) Genetic Modification

In one aspect, the disclosure provides molecular tools, methods and kits for modifying a CDL by linking the expression of a CDL with that of a DNA sequence encoding a negative selectable marker, thereby allowing drug-induced ablation of mitotically active cells consequently expressing the CDL and the negative selectable marker. Ablation of proliferating cells may be desirable, for example, when cell proliferation is uncontrolled and/or accelerated relative to a cell's normal division rate (e.g., uncontrolled cell division exhibited by cancerous cells). Ablation of proliferating cells may be achieved via a genetic modification to the cell, referred to herein as an "ablation link" (ALINK), which links the expression of a DNA sequence encoding a negative selectable marker to that of a CDL, thereby allowing elimination or sufficient inhibition of ALINK-modified proliferating cells consequently expressing the CDL locus (sufficient inhibition being inhibition of cell expansion rate to a rate that is too low to contribute to tumour formation). In the presence of a pro-drug or other inducer of the negatively selectable system, cells expressing the negative selectable marker will stop proliferating or die, depending on the mechanism of action of the selectable marker. Cells may be modified to comprise homozygous, heterozygous, hemizygous or compound heterozygous ALINKS. In one embodiment, to improve fidelity of ablation, a negative selectable marker may be introduced into all alleles functional of a CDL. In one preferred embodiment, a negative selectable marker may be introduced into all functional alleles of a CDL.

An ALINK may be inserted in any position of CDL, which allows co-expression of the CDL and the negative selectable marker.

As discussed further below in Example 1, DNA encoding a negatively selectable marker (e.g., HSV-TK), may be inserted into a CDL (e.g., CDK1) in a host cell, such that expression of the negative selectable marker causes host cells expressing the negative selectable marker and, necessarily, the CDL, to be killed in the presence of an inducer (e.g., prodrug) of the negative selectable marker (e.g., ganciclovir (GCV)). In this example, host cells modified with the ALINK will produce thymidine kinase (TK) and the TK protein will convert GCV into GCV monophosphate, which is then converted into GCV triphosphate by cellular kinases. GCV triphosphate incorporates into the replicating DNA during S phase, which leads to the termination of DNA elongation and cell apoptosis (Halloran and Fenton, 1998).

A modified HSV-TK gene (Preuß et al., 2010) is disclosed herein as one example of DNA encoding a negative selectable marker that may be used in an ALINK genetic modification to selectively ablate cells comprising undesirable cell division rate.

It is contemplated herein that alternative and/or additional negative selectable systems could be used in the tools and/or methods provided herein. Various negative selectable marker systems are known in the art (e.g., dCK.DM (Neschadim et al., 2012)).

For example, various negative selectable system having clinical relevance have been under active development in the field of "gene-direct enzyme/prodrug therapy" (GEPT), which aims to improve therapeutic efficacy of conventional cancer therapy with no or minimal side-effects (Hedley et al., 2007; Nawa et al., 2008). Frequently, GEPT involves the use of viral vectors to deliver a gene into cancer cells or into the vicinity of cancer cells in an area of the cancer cells that is not found in mammalian cells and that produces enzymes, which can convert a relatively non-toxic prodrug into a toxic agent.

HSV-TK/GCV, cytosine deaminase/5-fluorocytosine (CD/5-FC), and carboxyl esterase/irinotecan (CE/CPT-11) are examples of negative selectable marker systems being evaluated in GEPT pre- and clinical trials (Danks et al., 2007; Shah, 2012).

To overcome the potential immunogenicity issue of Herpes Simplex Virus type 1 thymidine kinase/ganciclovir (TK/GCV) system, a "humanized" suicide system has been developed by engineering the human deoxycytidine kinase enzyme to become thymidine-active and to work as a negative selectable (suicide) system with non-toxic prodrugs: bromovinyl-deoxyuridine (BVdU), L-deoxythymidine (LdT) or L-deoxyuridine (LdU) (Neschadim et al., 2012).

The CD/5-FC negative selectable marker system is a widely used "suicide gene" system. Cytosine deaminase (CD) is a non-mammalian enzyme that may be obtained from bacteria or yeast (e.g., from *Escherichia coli or Saccharomyces cerevisiae,* respectively) (Ramnaraine et al., 2003). CD catalyzes conversion of cytosine into uracil and is an important member of the pyrimidine salvage pathway in prokaryotes and fungi, but it does not exist in mammalian cells. 5-fluorocytosine (5-FC) is an antifungal prodrug that causes a low level of cytotoxicity in humans (Denny, 2003). CD catalyzes conversion of 5-FC into the genotoxic agent 5-FU, which has a high level of toxicity in humans (Ireton et al., 2002).

The CE/CPT-11 system is based on the carboxyl esterase enzyme, which is a serine esterase found in a different tissues of mammalian species (Humerickhouse et al., 2000). The anti-cancer agent CPT-11 is a prodrug that is activated by CE to generate an active referred to as 7-ethyl-10-hydroxycamptothecin (SN-38), which is a strong mammalian topoisomerase I inhibitor (Wierdl et al., 2001). SN-38 induces accumulation of double-strand DNA breaks in dividing cells (Kojima et al., 1998).

Another example of a negative selectable marker system is the iCasp9/AP1903 suicide system, which is based on a modified human caspase 9 fused to a human FK506 binding protein (FKBP) to allow chemical dimerization using a small molecule AP1903, which has tested safely in humans. Administration of the dimerizing drug induces apoptosis of cells expressing the engineered caspase 9 components. This system has several advantages, such as, for example, including low potential immunogenicity, since it consists of human gene products, the dimerizer drug only effects the cells expressing the engineered caspase 9 components (Straathof et al., 2005). The iCasp/AP1903 suicide system is being tested in clinical settings (Di Stasi et al., 2011).

It is contemplated herein that the negative selectable marker system of the ALINK system could be replaced with a proliferation antagonist system. The term "proliferation antagonist" as used herein, refers to a natural or engineered compound(s) whose presence inhibits (completely or partially) division of a cell. For example, Omomyc^{ER} is the fusion protein of MYC dominant negative Omomyc with mutant murine estrogen receptor (ER) domain. When induced with tamoxifen (TAM), the fusion protein Omomyc^{ER} localizes to the nucleus, where the dominant negative Omomyc dimerizes with C-Myc, L-Myc and N-Myc, sequestering them in complexes that are unable to bind the Myc DNA binding consensus sequences (Soucek et al., 2002). As a consequence of the lack of Myc activity, cells are unable to divide (Oricchio et al., 2014). Another example of a proliferation antagonist is A-Fos, a dominant negative to activation protein-1 (AP1) (a heterodimer of the oncogenes Fos and Jun) that inhibits DNA binding in an equimolar competition (Olive et al., 1997). A-Fos can also be fused to ER domain, rendering its nuclear localization to be induced by TAM. Omomyc^{ER} / tamoxifen or A-Fos^{ER} / tamoxifen could be a replacement for TK/GCV to be an ALINK.

### Targeting a CDL with an EARC Genetic Modification

In an aspect, the disclosure provides molecular tools, methods and kits for exogenously controlling a CDL by operably linking the CDL with an EARC, such as an inducible activator-based gene expression system. Under these conditions, the CDL will only be expressed (and the cell can only divide) in the presence of the inducer of the inducible activator-based gene expression system. Under these conditions, EARC-modified cells stop dividing, significantly slowdown, or die in the absence of the inducer, depending on the mechanism of action of the inducible activator-based gene expression system and CDL function. Cells may be modified to comprise homozygous or compound heterozygous EARCs or may be altered such that only EARC-modified alleles could produce functional CDLs. In an embodiment, an EARC modification may be introduced into all alleles of a CDL, for example, to provide a mechanism for cell division control.

An EARC may be inserted in any position of CDL that permits co-expression of the CDL and the activator component of the inducible system in the presence of the inducer.

In an embodiment, an "activator" based gene expression system is preferable to a "repressor" based gene expression system. For example, if a repressor is used to suppress a CDL a loss of function mutation of the repressor could release CDL expression, thereby allowing cell proliferation. In a case of an activation-based suppression of cell division, the loss of activator function (mutation) would shut down CDL expression, thereby disallowing cell proliferation.

As discussed further below in Examples 2-6, a dox-bridge may be inserted into a CDL (e.g., CDK1) in a host cell, such that in the presence of an inducer (e.g., doxycycline or "DOX") the dox-bridge permits CDL expression, thereby allowing cell division and proliferation. Host cells modified with a dox-bridge EARC may comprise a reverse tetracycline Trans-Activator (rtTA) gene (Urlinger et al., 2000) under the transcriptional control of a promoter, which is active in dividing cells (e.g., in the CDL). This targeted insertion makes the CDL promoter no longer available for CDL transcription. To regain CDL transcription, a tetracycline responder element promoter (for example TRE (Agha-Mohammadi et al., 2004)) is inserted in front of the CDL transcript, which will express the CDL gene only in a situation when rtTA is expressed and doxycycline is present. When the only source of CDL expression is dox-bridged alleles, there is no CDL gene expression in the absence of doxycycline. The lack of CDL expression causes the EARC-modified cells to be compromised in their proliferation, either by death, stopping cell division, or by rendering the cell mitotic rate so slow that the EARC-modified cell could not contribute to tumor formation.

The term "dox-bridge" as used herein, refers to a mechanism for separating activity of a promoter from a target transcribed region by expressing rtTA (Gossen et al., 1995) by the endogenous or exogenous promoter and rendering the transcription of target region under the control of TRE. As used herein, "rtTA" refers to the reverse tetracycline transactivator elements of the tetracycline inducible system (Gossen et al., 1995) and "TRE" refers to a promoter consisting of *TetO* operator sequences upstream of a minimal promoter. Upon binding of rtTA to the TRE promoter in the presence of doxycycline, transcription of loci downstream of the TRE promoter increases. The rtTA sequence may be inserted in the same transcriptional unit as the CDL or in a different location of the genome, so long as the transcriptional expression's permissive or non-permissive status of the target region is controlled by doxycycline. A dox-bridge is an example of an EARC.

Introduction of an EARC system into the 5' regulatory region of a CDL is also contemplated herein.

It is contemplated herein that alternative and/or additional inducible activator-based gene expression systems could be used in the tools and or methods provided herein to produce EARC modifications. Various inducible activator-based gene expression systems are known in the art.

For example, destabilizing protein domains (Banaszynski et al., 2006) fused with an acting protein product of a coding CDL could be used in conjunction with a small molecule synthetic ligand to stabilize a CDL fusion protein when cell division and/or proliferation is desirable. In the absence of a stabilizer, destabilized-CDL-protein will be degraded by the cell, which in turn would stop proliferation. When the stabilizer compound is added, it would bind to the destabilized-CDL-protein, which would not be degraded, thereby allowing the cell to proliferate.

For example, transcription activator-like effector (TALE) technology (Maeder et al., 2013) could be combined with dimerizer-regulated expression induction (Pollock and Clackson, 2002). The TALE technology could be used to generate a DNA binding domain designed to be specific to a sequence, placed together with a minimal promoter replacing the promoter of a CDL. The TALE DNA binding domain also extended with a drug dimerizing domain. The latter can bind to another engineered protein having corresponding dimerizing domain and a transcriptional activation domain. (FIG. 1C)

For example, referring to FIG. 1D, a reverse-cumate-Trans-Activator (rcTA) may be inserted in the 5' untranslated region of the CDL, such that it will be expressed by the endogenous CDL promoter. A 6-times repeat of a Cumate Operator (6xCuO) may be inserted just before the translational start (ATG) of CDL. In the absence of cumate in the system, rcTA cannot bind to the 6xCuO, so the CDL will not be transcribed because the 6xCuO is not active. When cumate is added, it will form a complex with rcTA, enabling binding to 6xCuO and enabling CDL transcription (Mullick et al., 2006).

For example, referring to FIG. 1E, a retinoid X receptor (RXR) and an N-terminal truncation of ecdysone receptor (EcR) fused to the activation domain of Vp16 (VpEcR) may be inserted in the 5' untranslated region of a CDL such that they are co-expressed by an endogenous CDL promoter. Ecdysone responsive element (EcRE), with a downstream minimal promoter, may also be inserted in the CDL, just upstream of the starting codon. Co-expressed RXR and VpEcR can heterodimerize with each other. In the absence of ecdysone or a synthetic drug analog muristerone A, dimerized RXR/VpEcR cannot bind to EcRE, so the CDL is not transcribed. In the presence of ecdysone or muristerone A, dimerized RXR/VpEcR can bind to EcRE, such that the CDL is transcribed (No et al., 1996).

For example, referring to FIG. 1F, a transient receptor potential vanilloid-1 (TRPV1), together with ferritin, may be inserted in the 5' untranslated region of a CDL and co-expressed by an endogenous CDL promoter. A promoter inducible by NFAT (NFATre) may also be inserted in the CDL, just upstream of the starting codon. In a normal environment, the NFAT promoter is not active. However, upon exposure to low-frequency radio waves, TRPV1 and ferritin create a wave of Ca⁺⁺ entering the cell, which in turn converts cytoplasmatic-NFAT (NFATc) to nuclear-NFAT (NFATn), that ultimately will activate the NFATre and transcribe the CDL (Stanley et al., 2015).

For example, referring to FIG. 1G, a CDL may be functionally divided in to parts/domains: 5'-CDL and 3'CDL, and a FKBP peptide sequence may be inserted into each domain. An IRES (internal ribosomal entry site) sequence may be placed between the two domains, which will be transcribed simultaneously by a CDL promoter but will generate two separate proteins. Without the presence of an inducer, the two separate CDL domains will be functionally inactive. Upon introduction of a dimerization agent, such as rapamycin or AP20187, the FKBP peptides will dimerize, bringing together the 5' and 3' CDL parts and reconstituting an active protein (Rollins et al., 2000).

### Methods of Controlling Division of an Animal Cell

In an aspect, a method of controlling division of an animal cell is provided herein.

The method comprises providing an animal cell. For example, the animal cell may be an avian or mammalian cell. For example, the mammalian cell may be an isolated human or non-human cell that is pluripotent (e.g., embryonic stem cell or iPS cell), multipotent, monopotent progenitor, or terminally differentiated. The mammalian cell may be derived from a pluripotent, multipotent, monopotent progenitor, or terminally differentiated cell. The mammalian cell may be a somatic stem cell, a multipotent or monopotent progenitor cell, a multipotent somatic cell or a cell derived from a somatic stem cell, a multipotent progenitor cell or a somatic cell. Preferably, the animal cell is amenable to genetic modification. Preferably, the animal cell is deemed by a user to have therapeutic value, meaning that the cell may be used to treat a disease, disorder, defect or injury in a subject in need of treatment for same. In various embodiments, the non-human mammalian cell may be a mouse, rat, hamster, guinea pig, cat, dog, cow, horse, deer, elk, bison, oxen, camel, llama, rabbit, pig, goat, sheep, or non-human primate cell. In a preferred embodiment, the animal cell is a human cell.

The method further comprises genetically modifying in the animal cell a CDL. The step of genetically modifying the CDL comprises introducing into the host animal cell an iNEP, such as one or more ALINK systems or one or more of an ALINK system and an EARC system. Techniques for introducing into animal cells various genetic modifications, such as negative selectable marker systems and inducible activator-based gene expression systems, are known in the art, including techniques for targeted (i.e., non-random), compound heterozygous and homozygous introduction of same. In cases involving use of EARC modifications, the modification should ensure that functional CDL expression can only be generated through EARC-modified alleles. For example, targeted replacement of a CDL or a CDL with a DNA vector comprising one or more of an ALINKalone or together with one or more EARC systems may be carried out to genetically modify the host animal cell.

The method further comprises permitting division of the genetically modified animal cell(s) comprising the iNEP system.

For example, permitting division of ALINK-modified cells by maintaining the genetically modified animal cells comprising the ALINK system in the absence of an inducer of the corresponding ALINK negative selectable marker. Cell division and proliferation may be carried out *in vitro* and/or *in vivo.* For example, genetically modified cells may be allowed to proliferate and expand *in vitro* until a population of cells that is large enough for therapeutic use has been generated. For example, one or more of the genetically modified animal cell(s) cells that have been proliferated and expanded may be introduced into a host (e.g., by grafting) and allowed to proliferate further *in vivo.* In various embodiment, ablating and/or inhibiting division of the genetically modified animal cell(s) comprising an ALINK system, may be done, *in vitro* and/or *in vivo,* by exposing the genetically modified animal cell(s) comprising the ALINK system to the inducer of the corresponding negative selectable marker. Such exposure will ablate proliferating cells and/or inhibit the genetically modified animal cell's rate of proliferation by killing at least a portion of proliferating cells. Ablation of genetically modified cells and/or inhibition of cell proliferation of the genetically modified animal cells may be desirable if, for example, the cells begin dividing at a rate that is faster than normal *in vitro or in vivo,* which could lead to tumor formation and/or undesirable cell growth.

For example, permitting division of EARC-modified cells by maintaining the genetically modified animal cell comprising the EARC system in the presence of an inducer of the inducible activator-based gene expression system. Cell division and proliferation may be carried out *in vitro* and/or *in vivo.* For example, genetically modified cells may be allowed to proliferate and expand *in vitro* until a population of cells that is large enough for therapeutic use has been generated. For example, one or more of the genetically modified animal cell(s) cells that have been proliferated and expanded may be introduced into a host (e.g., by grafting) and allowed to proliferate further *in vivo.* In various embodiment, ablating and/or inhibiting division of the genetically modified animal cell(s) comprising the EARC system, may be done, *in vitro* and/or *in vivo,* by preventing or inhibiting exposure the genetically modified animal cell(s) comprising the EARC system to the inducer of the inducible activator-based gene expression system. The absence of the inducer will ablate proliferating cells and/or inhibit the genetically modified animal cell's expansion by proliferation such that it is too slow to contribute to tumor formation. Ablation and/or inhibition of cell division of the genetically modified animal cells may be desirable if, for example, the cells begin dividing at a rate that is faster than normal *in vitro* or *in vivo,* which could lead to tumor formation and/or undesirable cell growth.

For example, in various embodiments of the method provided herein, set forth in various Examples below, the inducers are doxycycline and ganciclovir.

In an embodiment, doxycycline may be delivered to cells *in vitro* by adding to cell growth media a concentrated solution of the inducer, such as, for example, about 1 mg/ml of Dox dissolved in H₂O to a final concentration in growth media of about 1 µg/ml. *In vivo,* doxycycline may be administered to a subject orally, for example through drinking water (e.g., at a dosage of about 5-10 mg/kg) or eating food (e.g., at a dosage of about 100 mg/kg), by injection (e.g., I.V. or I.P. at a dosage of about 50 mg/kg) or by way of tablets (e.g., at a dosage of about 1-4 mg/kg).

In an embodiment, ganciclovir may be delivered to cells *in vitro* by adding to cell growth media a concentrated solution of the inducer, such as, for example, about 10 mg/ml of GCV dissolved in H₂O to a final concentration in growth media of about 0.25-25 µg/ml. *In vivo,* GCV may be administered to a subject orally, for example through drinking water (e.g., at a dosage of about 4-20 mg/kg) or eating food (e.g., at a dosage of about 4-20 mg/kg), by injection (e.g., at a dosage of about I.V. or I.P. 50 mg/kg) or by way of tablets (e.g., at a dosage of about 4-20 mg/kg).

In an embodiment, to assess whether the inducers are working *in vitro,* cell growth and cell death may be measured (e.g., by cell counting and viability assay), for example every 24 hours after treatment begins. To assess whether the inducers are working *in vivo,* the size of teratomas generated from genetically modified pluripotent cells may be measured, for example, every 1-2 days after treatment begins.

In a particularly preferred embodiment of the method provided herein, an animal cell may be genetically modified to comprise both ALINK and EARC systems. The ALINK and EARC systems may target the same or different CDLs. Such cells may be desirable for certain applications, for example, because they provide a user with at least two mechanisms for ablating and/or inhibiting cell division and/or ablating and/or inhibiting proliferation by killing at least a portion of proliferating cells.

It is contemplated herein that the method provided herein may be used to control division and/or proliferation of an avian cell, such as, for example, a chicken cell.

### Cells Engineered to Comprise at Least One Mechanism for Controlling Cell Division

In an aspect, an animal cell genetically modified to comprise at least one mechanism for controlling cell division and/or proliferation, and populations of same, are provided herein. For example, the mammalian cell may be an isolated human or non-human cell that is pluripotent (e.g., embryonic stem cell or iPS cell), multipotent, monopotent progenitor, or terminally differentiated. The mammalian cell may be derived from a pluripotent, multipotent, monopotent progenitor, or terminally differentiated cell. The mammalian cell may be a somatic stem cell, a multipotent, monopotent progenitor, progenitor cell or a somatic cell or a cell derived from a somatic stem cell, a multipotent or monopotent progenitor cell or a somatic cell. Preferably, the animal cell is amenable to genetic modification. Preferably, the animal cell is deemed by a user to have therapeutic value, meaning that the cell may be used to treat a disease, disorder, defect or injury in a subject in need of treatment for same. In some embodiments, the non-human mammalian cell may be a mouse, rat, hamster, guinea pig, cat, dog, cow, horse, deer, elk, bison, oxen, camel, llama, rabbit, pig, goat, sheep, or non-human primate cell.

The genetically modified cells provided herein comprise one or more genetic modification of one or more CDL. The genetic modification of a CDL being an ALINK system and, in the case of CDLs, one or more of an ALINK system and an EARC system, such as, for example, one or more of the ALINK and/or EARC systems described herein. For example, a genetically modified animal cell provided herein may comprise: an ALINK system in one or more CDLs; an EARC system in one or more CDLs; or ALINK and EARC systems in one or more CDLS, wherein the ALINK and EARC systems correspond to the same or different CDLs. The genetically modified cells may comprise homozygous, heterozygous, hemizygous or compound heterozygous ALINK genetic modifications. In the case of EARC modifications, the modification should ensure that functional CDL expression can only be generated through EARC-modified alleles.

It is contemplated that the genetically modified cells provided herein may be useful in cellular therapies directed to treat a disease, disorder or injury and/or in cellular therapeutics that comprise controlled cellular delivery of compounds and/or compositions (e.g., natural or engineered biologics). As indicated above, patient safety is a concern in cellular therapeutics, particularly with respect to the possibility of malignant growth arising from therapeutic cell grafts. For cell-based therapies where intensive proliferation of the therapeutic cell graft is not required, it is contemplated that the genetically modified cells comprising one or more iNEP modifications, as described herein, would be suitable for addressing therapeutic and safety needs. For cell-based therapies where intensive proliferation of the therapeutic cell graft is required, it is contemplated that the genetically modified cells comprising two or more iNEP modifications, as described herein, would be suitable for addressing therapeutic and safety needs.

It is contemplated herein that avian cells, such as chicken cells, may be provided, wherein the avian cells comprise the above genetic modifications.

### Molecular Tools for Targeting CDLs

In an aspect, various DNA vectors for modifying expression of a CDL are provided herein.

In one embodiment, the DNA vector comprises an ALINK system, the ALINK system comprising a DNA sequence encoding a negative selectable marker. The expression of the negative selectable marker is linked to that of a CDL.

In one embodiment, the DNA vector comprises an EARC system, the EARC system comprising an inducible activator-based gene expression system that is operably linked to a CDL, wherein expression of the CDL is inducible by an inducer of the inducible activator-based gene expression system.

In one embodiment, the DNA vector comprises an ALINK system, as described herein, and an EARC system, as described herein. When such a cassette is inserted into a host cell, CDL transcription product expression may be prevented and/or inhibited by an inducer of the negative selectable marker of the ALINK system and expression of the CDL is inducible by an inducer of the inducible activator-based gene expression system of the EARC system.

In various embodiments, the CDL in the DNA vector is a CDL listed in Table 2.

In various embodiments, the ALINK system in the DNA vector is a herpes simplex virus-thymidine kinase/ ganciclovir system, a cytosine deaminase/5-fluorocytosine system, a carboxyl esterase/irinotecan system or an iCasp9/AP1903 system.

In various embodiments, the EARC system in the DNA vector is a dox-bridge system, a cumate switch inducible system, an ecdysone inducible system, a radio wave inducible system, or a ligand-reversible dimerization system.

### Kits

The present disclosure contemplates kits for carrying out the methods disclosed herein. Such kits typically comprise two or more components required for using CDLs and/or CDLs to control cell proliferation. Components of the kit include, but are not limited to, one or more of compounds, reagents, containers, equipment and instructions for using the kit. Accordingly, the methods described herein may be performed by utilizing pre-packaged kits provided herein. In one embodiment, the kit comprises one or more DNA vectors and instructions. In some embodiments, the instructions comprise one or more protocols for introducing the one or more DNA vectors into host cells. In some embodiments, the kit comprises one or more controls.

In one embodiment, the kit comprises one or more DNA vector for modifying expression of a CDL, as described herein. By way of example, the kit may contain a DNA vector comprising an ALINK system; and/or a DNA vector comprising an EARC system; and/or a DNA vector comprising an ALINK system and an EARC system; and instructions for targeted replacement of a CDL and/or CDL in an animal cell using one or more of the DNA vectors. In preferred embodiments, the kit may further comprise one or more inducers (e.g., drug inducer) that correspond with the ALINK and/or EARC systems provided in the DNA vector(s) of the kit.

The following non-limiting examples illustrative of the disclosure are provided.

### Example 1: Generation of ALINK-Modified Cells (Mouse and Human)

In Example 1, construction of ALINK (HSV-TK) vectors targeting Cdk1/CDK1 and use of same to control cell proliferation in mouse and human ES cells, by way of killing at least a portion of proliferating cells, is described. In this example, Cdk1/CDK1 is the CDL and HSV-TK is the negative selectable marker.

Cdk1/CDK1 is expressed in all mitotically active (i.e., dividing) cells. In cells modified to comprise a homozygous ALINK between the CDK1 locus and HSV-TK, all mitotically active cells express CDK1 and HSV-TK. Thus, the ALINK-modified mitotically active cells can be eliminated by treatment with GCV (the pro-drug of HSV-TK). If all the functional CDK1 expressing allele is ALINK modified and the cells were to silence HSV-TK expression then likely CDK1 expression would also be silenced and the cells would no longer be able to divide. Quiescent (i.e., non-dividing) cells do not express Cdk1/CDK1. Thus, ALINK-modified quiescent cells would not express the Cdk1/CDK1-HSV-TK link.

In Example 1, the transcriptional link between Cdk1/CDK1 and HSV-TK was achieved by homologous recombination-based knock-ins.

### METHODS

### Generation of Target Vectors

*Mouse Target Vector* I: The mouse *Cdk1* genomic locus is shown in FIG. 2A. Referring to FIG. 2B, two DNA fragments: 5TK (SEQ ID NO: 1) and 3TK (SEQ ID NO: 2) (Sall-F2A-5'TK.007-PB 5'LTR-Notl-Sacll and Sall-Sacll-3'TK.007-PB 3'LTR-3'TK.007-T2A-Xhol-mCherry-Nhel) were obtained by gene synthesis in a pUC57 vector (GenScript). Fragment 5TK was digested with Sall + Sacll and cloned into 3TK with the same digestion to generate pUC57-5TK-3TK. A PGK-Neomycin cassette was obtained by cutting the plasmid pBluescript-M214 (SEQ ID NO: 3) with Notl + HindIII and it was ligated into the Notl + Sacll site of pUC57-5TK-3TK to generate the ALINK cassette to be inserted at the 3' end of Cdk1 (i.e., the CDL).

*Homology arms for the insertion* ALINK *at the 3' of the CDL:* Cdk1 DNA coding sequences were cloned by recombineering: DH10B *E*. *coli* cell strain containing bacterial artificial chromosomes (BACs) with the genomic sequences of Cdk1 (SEQ ID NO: 4), which were purchased from The Center for Applied Genomics (TCAG). The recombineering process was mediated by the plasmid pSC101-BAD-γβα Red/ET (pRET) (GeneBridges, Heidelberg Germany). pRET was first electroporated into BAC-containing DH10B *E*. *coli* at 1.8kV, 25µF, 4000hms (BioRad GenePulserl/II system, BioRad, ON, CA) and then selected for choloramphenicol and tetracycline resistance. Short homology arms (50bp) (SEQ ID NOs: 5 and 6 respectively) spanning the ALINK insertion point (5' and 3' of the Cdk1 stop codon) were added by PCR to the cassette, F2A-5'TK-PB-PGKneo-PB-3'TK-T2A-cherry. This PCR product was then electroporated into Bac+pRET DH10B *E*. *coli* under the conditions described above and then selected for kanamycin resistance. The final targeting cassette, consisting of 755bp and 842 base pair (bp) homology arms (SEQ ID NOs: 7 and 8, respectively), was retrieved by PCR with primers (SEQ ID NOs: 9 and 10, respectively) and cloned into a pGemT-Easy vector to generate mouse Target Vector I. The critical junction regions of the vector were sequenced at TCAG and confirmed.

*Mouse Target Vector II:* referring to FIG. 2D, F2A-loxP-PGK-neo-pA-loxP-Ascl (SEQ ID NO: 11) was PCR amplified from pLoxPNeo1 vector and TA cloned into a pDrive vector (Qiagen). Ascl-TK-T2A-mCherry-EcoRI (SEQ ID NO: 12) was PCR amplified from excised TC allele I, and TA cloned into the pDrive vector. The latter fragment was then cloned into the former vector by BamHI + Ascl restriction sites. This F2A-loxP-PGK-neo-pA-loxP-TK-T2A-mCherry cassette was inserted between mouse Cdk1 homology arms by GeneArt^{®} Seamless Cloning and Assembly Kit (Life Technologies). To generate the puromycin (puro) version vector, PGK-puro-pA fragment (SEQ ID NO: 13) was cut from pNewDockZ with BamHI + Notl and T4 blunted. The neo version vector was cut with Ascl+Clal, T4 blunted and ligated with PGK-puro-pA.

*Human Target Vector I:* Similar to mouse Target Vector I, 847 bp upstream of human CDK1 stop codon (SEQ ID NO: 14) + F2A-5'TK-PB-PGKneo-PB-3'TK-T2A-cherry (SEQ ID NO: 15) + 831 bp downstream of human CDK1 stop codon (SEQ ID NO: 16) was generated by recombineering technology. A different version of the vector containing a puromycin resistant cassette for selection, was generated to facilitate one-shot generation of homozygous targeting: Agel-PGK-puro-pA-Fsel (SEQ ID NO: 17) was amplified from pNewDockZ vector, digested and cloned into neo version vector cut by Agel+Fsel.

*Human Target Vector II:* BamHI-F2A-loxP-PGK-neo-pA-loxP-TK-T2A-mCherry (SEQ ID NO: 18) and BamHI-F2A-loxP-PGK-puro-pA-loxP-TK-T2A-mCherry (SEQ ID NO: 19) were amplified from the corresponding mouse Target Vector II, and digested with BamHI+SgrAI. The mCherry (3' 30bp)-hCDK13'HA-pGemEasy-hCDK15'HA-BamHI (SEQ ID NO: 20) was PCR-amplified and also digested with BamHI+SgrAI. The neo and puromycin version of human Target Vector II were generated by ligation of the homology arm backbone and the neo or puromycin version ALINK cassette.

*Human Target Vector III: Target vectors with no selection cassette were made for targeting with fluorescent marker (mCherry or eGFP) by FACS and avoiding the step of excision of selection cassette.* BamHI-F2A-TK-T2A-mCherry-SgrAI (SEQ ID NO: 58) was PCR amplified from excised TC allele I, digested with BamHI+SgrAI, and ligated with digested mCherry (3' 30bp)-hCDK13'HA-pGemEasy-hCDK15'HA-BamHI (SEQ ID NO: 20). The CRISPR PAM site in the target vector was mutagenized with primers PAM_fwd (SEQ ID NO: 59) and PAM_rev (SEQ ID NO: 60) using site-directed PCR-based mutagenesis protocol. The GFP version vector was generated by fusion of PCR-amplified Xhol-GFP (SEQ ID NO: 61) and pGemT-hCdk1-TK-PAMmut (SEQ ID NO: 62) with NEBuiler HiFi DNA Assembly Cloning Kit (New England Biolabs Inc.).

### Generation of CRISPR/Cas9 plasmids

CRISPR/Cas9-assisted gene targeting was used to achieve high targeting efficiency (Cong et al., 2013). Guide sequences for CRISPR/Cas9 were analyzed using the online CRISPR design tool (http://crispr.mit.edu) (Hsu et al., 2013).

CRISPR/Cas9 plasmids pX335-mCdkTK-A (SEQ ID NO: 21) and pX335-mCdkTK-B (SEQ ID NO: 22) were designed to target mouse Cdk1 at SEQ ID NO: 23.

CRISPR/Cas9 plasmids pX330-hCdkTK-A (SEQ ID NO: 24) and pX459-hCdkTK-A (SEQ ID NO: 25) were designed to target the human Cdk1 at SEQ ID NO: 26.

CRISPRs were generated according to the suggested protocol with backbone plasmids purchased from Addgene. (Ran et al., 2013).

### Generation of ALINK-Modified Mouse ES Cells

*Mouse ES Cell Culture:* Mouse ES cells are cultured in Dulbecco's modified Eagle's medium (DMEM) (high glucose, 4500 mg/liter) (Invitrogen), supplemented with 15% Fetal Bovine Serum (Invitrogen), 1 mM Sodium pyruvate (Invitrogen), 0.1 mM MEM Non-essential Amino-acids (Invitrogen), 2mM GlutaMAX (Invitrogen), 0.1 mM 2-mEARCaptoethanol (Sigma), 50U/ml each Penicillin/ Streptomycin (Invitrogen) and 1000U/ml Leukemia-inhibiting factor (LIF) (Chemicon). Mouse ES cells are passed with 0.25% trypsin and 0.1% EDTA.

*Targeting:* 5x10⁵ mouse C57BL/6 C2 ES cells (Gertsenstein et al., 2010) were transfected with 2ug DNA (Target Vector:0.5 µg, CRISPR vector: 1.5 µg) by JetPrime transfection (Polyplus). 48h after transfection cells were selected for G418 or/and puromycin-resistant. Resistant clones were picked independently and transferred to 96-well plates. 96-well plates were replicated for freezing and genotyping (SEQ ID NOs: 27, 28, 29 and 30). PCR-positive clones were expanded, frozen to multiple vials, and genotyped by southern blotting.

*Excision of the selection cassette:* correctly targeted ES clones were transfected with Episomal-hyPBase (for Target Vector I) (SEQ ID NO: 34) or pCAGGs-NLS-Cre-Ires-Puromycin(for Target Vector II) (SEQ ID NO: 35). 2-3 days following transfection, cells were trypsinized and plated clonally (1000-2000 cells per 10cm plate). mCherry-positive clones were picked and transferred to 96-well plates independently and genotyped by PCR (SEQ ID NOs: 31 and 36) and Southern blots to confirm the excision event. The junctions of the removal region were PCR-amplified, sequenced and confirmed to be intact and seamless without frame shift.

*Homozygous targeting:* ES clones that had already been correctly targeted with a neo version target vector and excised of selection cassette were transfected again with a puromycin-resistant version of the target vector. Selection of puromycin was added after 48 hours of transfection, then colonies were picked and analyzed, as described above (SEQ ID NOs: 31 and 32). Independent puro-resistant clones were grown on gelatin, then DNA was extracted for PCR to confirm the absence of a wild-type allele band (SEQ ID NOs: 31, 33).

### Generation of ALINK-Modified Human ES Cells

*Human ES Cell Culture:* Human CA1 or H1 (Adewumi et al., 2007) ES cells were cultured with mTeSR1 media (STEMCELL Technologies) plus penicillin-streptomycin (Gibco by Life Technologies) on Geltrex (Life Technologies) feeder-free condition. Cells were passed by TrypIE Express (Life Technologies) or Accutase (STEMCELL Technologies) and plated on mTeSR media plus ROCK inhibitor (STEMCELL Technologies) for the first 24h, then changed to mTeSR media. Half of cells from a fully confluent 6-well plate were frozen in 1 ml 90% FBS (Life Technologies) + 10%DMSO (Sigma).

*Targeting:* 6x10⁶ CA1 hES cells were transfected by Neon protocol 14 with 24ug DNA (Target Vector: pX330-hCdkTK-A = 18ug:6ug). After transfection, cells were plated on four 10-cm plates. G418 and/or puromycin selection was started 48h after transfection. Independent colonies were picked to 96-well plates. Each plate was duplicated for further growth and genotyping (SEQ ID NOs: 37, 38, 39 and 40). PCR-positive clones were expanded, frozen to multiple vials and genotyped with southern blotting.

*Excision of the selection cassette:* ALINK-targeted ES clones were transfected with hyPBase or pCAGGs-NLS-Cre-IRES-Puromycin and plated in a 6-well plate. When cells reached confluence in 6-well plates, cells were suspended in Hanks Balanced Salt Solution (HBSS) (Ca2+/ Mg2+ Free) (25 mM HEPES pH7.0, 1% Fetal Calf Serum), and mCherry-positive cells were sorted to a 96-well plate using an ASTRIOS EQ cell sorter (Beckman Coulter).

*Homozygous Targeting:* Homozygous targeting can be achieved by the same way as in the mouse system or by transfecting mCherry and eGFP human target vector III plus pX330-hCdkTK-A or pX459-hCdkTK-A followed by FACS sorting for mCherry-and-eGFP double-positive cells.

### Teratoma Assay

Matrigel Matrix High Concentration (Corning) was diluted 1:3 with cold DMEM media on ice. 5-10x10⁶ cells were suspended into 100ul of 66% DMEM + 33% Matrigel media and injected subcutaneously into either or both dorsal flanks of B6N mice (for mouse C2 ES cells) and NOD-SCID mice (for human ES cells). Teratomas formed 2-4 weeks after injection. Teratoma size was measured by caliper, and teratoma volume was calculated using the formula V= (LxWxH)π/6. GCV/PBS treatment was performed by daily injection with 50mg/kg into the peritoneal cavity with different treatment durations. At the end of treatment, mice were sacrificed and tumors were dissected and fixed in 4% paraformaldehyde for histology analysis.

### Mammary aland tumor assay

Chimeras of Cdk1^{+/+ +/loxp-alink} mouse C2 ES and CD-1 backgrounds were generated through diploid aggregation, and then were bred with B6N WT mice to generate Cdk1^{+/+,+/loxp-alink} mice through germline transmission. Cdk1^{+/+}, ^{+/loxp-alink} mice were bred with Ella-Cre mice to generate Cdk1^{+/+, +/alink} mice. Cdk1^{+/+,+/alink} mice were then bred with MMTV-PyMT mice (Guy et al., 1992) to get double-positive pups with mammary gland tumors and ALINK modification. Mammary gland tumors with fail-safe modification were isolated, cut into 1mm³ pieces, and transplanted into the 4^{th} mammary gland of wild-type B6N females. GCV/PBS treatment was injected every other day at the dosage of 50mg/kg into the peritoneal cavity with different treatment durations. Mammary gland tumor size was measured by calipers and calculated with the formula V=Length*Width*Height* π/6.

### Neuronal progenitor vs. neuron killing assay

Cdk1^{+/+, +/alink} human CA1 ES cells were differentiated to neural epithelial progenitor cells (NEPs). NEPs were subsequently cultured under conditions for differentiation into neurons, thereby generating a mixed culture of non-dividing neurons and dividing NEPs, which were characterized by immunostaining of DAPI, Ki67 and Sox2. GCV (10uM) was provided to the mixed culture every other day for 20 days. Then, GCV was withdrawn from culture for 4 days before cells were fixed by 4% PFA. Fixed cells were immunostained for proliferation marker Ki67 to check whether all the leftover cells have exited cell cycle, and mature neutron marker beta-Tublinlll.

### RESULTS

The mouse *Cdk1* genomic locus is shown in FIG. 2a. Two vectors targeting murine *Cdk1* were generated (FIGs. 2B and D), each configured to modify the 3'UTR of the *Cdk1* gene (FIG. 2A) by replacing the STOP codon of the last exon with an F2A (Szymczak et al., 2004) sequence followed by an enhanced *HSV-TK* (TK.007 (Preuß et al., 2010)) gene connected to an mCherry reporter with a T2A (Szymczak et al., 2004) sequence.

Referring to FIG. 2B and mouse target vector I, the PGK-neo-pA selectable marker (necessary for targeting) was inserted into the TK.007 open-reading-frame with a *piggyBac* transposon, interrupting TK expression. The *piggyBac* transposon insertion was designed such that transposon removal restored the normal ORF of TK.007, resulting in expression of functional thymidine kinase (FIG. 2C).

Referring to FIG. 2D and mouse target vector II, the neo cassette was loxP-flanked and inserted between the F2A and TK.007.

Target vectors I and II had short (~800 bp) homology arms, which were sufficient for CRISPRs assisted homologous recombination targeting and made the PCR genotyping for identifying targeting events easy and reliable. The CRISPRs facilitated high targeting frequency at 40% PCR-positive of drug-resistant clones (FIG. 3D).

Both the *piggyBac*-inserted and the loxP-flanked neo cassettes were removed by transient expression of the piggyBac transposase and Cre recombinase, respectively, resulting in cell lines comprising alleles shown in FIGs. 2C and 2E, respectively. Referring to FIG. 2E, the remaining loxP site was in frame with TK and added 13 amino acids to the N-terminus of TK. The TK functionality test (GCV killing) proved that this N-terminus insertion did not interfere with TK function.

Referring to FIG. 4, assisted with CRISPR-Cas9 technology, homozygous ALINK can also be generated efficiently in two different human ES cell lines, CA1 and H1 (Adewumi et al., 2007).

Referring to FIG. 5A and 5C, the data indicate that: i) the TK.007 insertion into the 3'UTR of Cdk1 does not interfere with Cdk1 expression; ii) the ALINK-modified homozygous mouse C2 ES cells properly self-renew under ES cell conditions and differentiate in vivo and form complex teratomas; iii) the ALINK-modified homozygous human CA1 ES cells properly self-renew under ES cell conditions and differentiate in vivo and form complex teratomas.

Referring to FIG. 6, the data indicate that: i) TK.007 is properly expressed; GCV treatment of undifferentiated ES cells ablates both homozygously- and heterozygously-modified cells (FIG. 6A); and ii) the T2A-linked mCherry is constitutively expressed in ES cells (FIG. 6B).

Referring to FIG. 7A, the data indicate that in hosts comprising ALINK-modified cell grafts, GCV treatment of subcutaneous teratomas comprising the ALINK-modified ES cells stops teratoma growth by ablating dividing cells. GCV treatment did not affect quiescent cells of the teratoma. A brief (3 week) GCV treatment period of the recipient was sufficient to render the teratomas dormant. Referring to FIG. 7B, in NOD scid gamma mouse hosts comprising ALINK-modified human cell grafts, two rounds of GCV treatment (1st round 15 days + 2^{nd} round 40 days) rendered the teratomas to dormancy.

Referring to FIG. 7C, in B6N hosts comprising ALINK-modified MMTV-PyMT-transformed mammary epithelial tumorigenic cell grafts, GCV treatment was able to render the mammary gland tumors to dormancy.

Referring to FIGS. 7D-F, in a mixed culture of non-dividing neurons and dividing NEPs, all cells having been derived from Cdk1^{+/+, +/alink} human CA1 ES cells, GCV killed the dividing NEPs but did not kill the non-dividing neurons.

In an embodiment, it is contemplated that one or more dividing cells could escape GCV-mediated ablation if an inactivating mutation were to occur in the HSV-TK component of the CDL-HSV-TK transcriptional link. To address the probability of cell escape, the inventors considered the general mutation rate per cell division (i.e., 10⁻⁶) and determined that the expected number of cell divisions required to create 1 mutant cell would be 16 in cells comprising a heterozygous Cdk1 - HSV-TK transcriptional link, and 30 cell divisions in cells comprising a homozygous Cdk1 - HSV-TK transcriptional link. This means that if a single heterozygous ALINK-modified cell is expanded to 2¹⁶ (i.e., 65,000 cells) and a single homozygous ALINK-modified cell is expanded to 2³⁰ (i.e., 1 billion cells), then an average of one mutant cell comprising lost HSV-TK activity per heterozygous and homozygous cell population would be generated (FIG. 8). Accordingly, the inventors have determined that homozygous ALINK-modified cells would be very safe for use in cell-based therapies. Another way of calculating the level of safety of cell therapy was presented above.

### Example 2: Generation of EARC-Modified Mouse ES Cells in the Cdk1 Locus

In Example 2, construction of EARC (dox-bridge) vectors targeting Cdk1 and use of same to control cell division in mouse ES cells is described. In this example, Cdk1/CDK1 is the CDL, which is targeted with an inducible gene expression system, wherein a dox-bridge is inserted and doxycycline induces expression of the CDL.

As described above, Cdk1/CDK1 is expressed in all mitotically active (i.e., dividing) cells. In cells modified to comprise an EARC (dox-bridge) insertion at the Cdk1 locus, cell division is only possible in the presence of the inducer (doxycycline), which permits expression of Cdk1. Thus, cell division of EARC-modified mitotically active cells can be eliminated in the absence of doxycycline.

In Example 2, dox-bridge insertion into the 5'UTR of the *Cdk1* gene was achieved by homologous recombination knock-in technology.

### METHODS

### Construction of EARC Targeting Vector Comprising a Dox-bridae

A fragment containing an rTTA coding sequence (SEQ ID NO: 41) followed by a 3x SV40 pA signal was amplified by PCR from a pPB-CAGG-rtta plasmid, using primers containing a lox71 site added at the 5' of the rTTA (rtta3xpaFrw1 (SEQ ID NO: 63), rtta3xpaRev1(SEQ ID NO: 64)). This fragment was subcloned into a pGemT plasmid, to generate pGem-bridge-step1. Subsequently, a Sacll fragment containing a TetO promoter (SEQ ID NO: 42) (derived from pPB-TetO-IRES-mCherry) was cloned into the Sacll site of the pGem-bridge-step1, generating a pGem-bridge-step2. The final element of the bridge was cloned by inserting a BamHI IRES-Puromycin fragment (SEQ ID NO: 43) into the BamHI site of the pGem-bridge-step2, generating a pGem-bridge-step3. The 5' homology arm was cloned by PCR-amplifying a 900 bp fragment (SEQ ID NO: 44) from C57/B6 genomic DNA (primers cdk5FrwPst (SEQ ID NO: 45) and cdk5RevSpe (SEQ ID NO: 46) and cloning it into Sbfl and Spel of the pGem-bridge-step3. Similarly, the 3' homology arm (900 bp) (SEQ ID NO: 47) was amplified by PCR using primers dkex3_5'FSpe (SEQ ID NO: 48), cdkex3_3lox (SEQ ID NO: 49) and cloned into Sphl and Ncol to generate a final targeting vector, referred to as pBridge (SEQ ID NO: 148).

### Construction of CRISPR/Cas9 Plasmids

A double-nickase strategy was chosen to minimize the possibility of off-target mutations. Guide RNA sequences (SEQ ID NOs: 50, 51, 52 and 53) were cloned into pX335 (obtained from Addgene, according to the suggested protocol) (Ran et al., 2013).

### Generation of EARC-modified Mouse ES cells

*Mouse ES cell culture:* All genetic manipulations were performed on a C57BL/6N mouse ES cell line previously characterized (C2) (Gertsenstein etal., 2010). Mouse ES cells were grown in media based on high-glucose DMEM (Invitrogen), supplemented with 15% ES cell-grade FBS (Gibco), 0.1 mM 2-mEARCaptophenol, 2 mM L-glutamine, 1 mM sodium pyruvate, 0.1 mM non-essential amino acids, and 2,000 units/ml leukemia inhibitory factor (LIF). Cells were maintained at 37°C in 5% CO₂ on mitomycin C-treated mouse embryonic fibroblasts (MEFs).

*Targeting:* Plasmids containing the CRISPR/Cas9 components (pX335-cdk-ex3A (SEQ ID NO: 151) and px335-cdk-ex3B (SEQ ID NO: 152)) and the targeting plasmid (pBridge; SEQ ID NO: 148) were co-transfected in mouse ES cells using FuGENE HD (Clontech), according to the manufacturer's instructions, using a FuGENE:DNA ratio of 8:2, (2 µg total DNA: 250 ng for each pX330 and 1500 ng for pBridge). Typical transfection was performed on 3x10⁵cells, plated on 35 mm plates. Upon transfection, doxycycline was added to the media to a final concentration of 1 µg/ml. 2 days following transfection, cells were plated on a 100 mm plate and selection was applied with 1 µg/ml of puromycin. Puromycin-resistant colonies were picked 8-10 days after start of selection and maintained in 96 well plates until PCR-screening.

*Genotyping:* DNA was extracted from ES cells directly in 96 well plates according to (Nagy et al., 2003). Clones positive for correct insertion by homologous recombination of pBridge in the 5' of the Cdk1 gene were screened by PCR using primers spanning the 5' and 3' homology arms (primers rttaRev (SEQ ID NO: 54), ex3_5scr (SEQ ID NO: 55) for the 5' arm, primers CMVforw (SEQ ID NO: 56), ex3_3scr (SEQ ID NO: 57) for the 3' arm).

*Targeted cell growth:* F3-bridge targeted cells were trypsinized and plated on gelatinized 24 well plates at a density of 5x10⁴ cells per well. Starting one day after plating, cell counting was performed by trypsinizing 3 wells for each condition and counting live cells using a Countess automated cell counter (Life Technologies). Doxycycline was removed or reduced to 0.05 ng/ml 2 days after plating and live cells were counted every day up to 18 days in the different conditions.

*Cre-excision:* F3-bridge cells (grown in Dox+ media) were trypsinized and transfected with 2 µg of a plasmid expressing Cre (pCAGG-NLS-Cre). Transfection was performed using JetPrime (Polyplus) according to the manufacturer's protocol. After transfection, doxycycline was removed and colonies were trypsinized and expanded as a pool.

*Quantitative PCR:* Total RNA was extracted from cells treated for 2 days with 1 µg/ml and 0 µg/ml of Dox using the GeneElute total RNA miniprep kit (Sigma) according to the manufacturer's protocol. cDNA was generated by reverse transcription of 1 µg of RNA using the QuantiTect reverse transcription kit (Qiagen), according to the manufacturer's protocol. Real-time qPCR were set up in a BioRad CFX thermocycler, using SensiFast-SYBR qPCR mix (Bioline). The primers used were: qpcrcdk1_F (SEQ ID NO: 65), qpcrcdk1_R (SEQ ID NO:66) and actBf (SEQ ID NO: 67), actBr (SEQ ID NO: 68). Results were analyzed with the ΔΔCT method and normalized for beta-actin.

### RESULTS

Referring to FIG. 9, the dox-bridge target vector, depicted in FIG. 9A, was used to generate three targeted C2 mouse ES cell lines (FIG. 9B). One of these cell lines was found to be a homozygous targeted line (3F in FIG. 9B) comprising a dox-bridge inserted by homologous recombination into the 5'UTR of both alleles of Cdk1.

As expected, this ES cell line grows only in the presence of doxycycline. In the presence of doxycycline, the *Cdk1* promoter activity produced rtTA binds to TRE and initiates transcription of the *Cdk1.* Similarly to the 3' modification, the dox-bridge may be inserted into the 5'UTR into both alleles of *Cdk1,* to ensure that the CDL expression could occur only through EARC. An alternative is to generate null mutations in all the remaining, non-EARC modified alleles of CDL.

Withdrawal of doxycycline resulted in complete elimination of mitotically active ES cells within 5 days (FIG. 10). Lowering the doxycycline concentration by 20x (50 ng/ml) compared to the concentration used for derivation and maintenance of the doc-bridged cell line, allowed some cells/colonies to survive the 5 days period (FIG. 11).

Referring to FIG. 12, the dox-bridge was removable with a Cre recombinase mediated excision of the segment between the two lox71 sites, which restore the original endogenous expression regulation of the allele and rescues the cell lethality from the lack of doxycycline. These data indicate that the dox-bridge was working in the cells as predicted.

Referring to FIG. 13, the inventors determined how doxycycline withdrawal affected elimination of the dox-bridge ES cells by measuring cell growth in the presence and absent of doxycycline. ES cells in the presence of doxycycline grew exponentially, indicating their normal growth. In contrast, upon withdrawal of doxycycline (Day 1) cells grew for only two days and then cells death began until no live cells were present on Day 9. A 20x lower doxycycline concentration (50ng/ml) provided after an initial 3 days of cell growth was sufficient to maintain a constant number of cells on the plates for at least five days (FIG. 13, light blue line). When the normal concentration of doxycycline was added back to the plate on day 10, cells started growing again as normal ES cells.

It is contemplated that dividing cells could escape EARC (dox-bridge)-modification of Cdk1 when grown in media lacking doxycycline. To address the probability of cell escape, EARC (dox-bridge)-modified mouse ES cells were grown up to 100,000,000 cells/plate on ten plates in medium containing doxycycline. 300 GFP-positive wild-type ES cells (sentinels) were then mixed into each 10 plate of modified ES cells and doxycycline was withdrawn from the culture medium. Only GFP positive colonies were recovered (FIG. 14) indicating that there were no escapee dox-bridged ES cells among the 100,000,000 cells in the culture. Accordingly, the inventors have determined that EARC (dox-bridge)-modified ES cells would add an additional level of safety to ALINK modification for certain cell therapy applications, because loss of the dox-bridge is unlikely to occur by mutation and cell division is not possible in the absence of the inducer (doxycycline) due to the block of CDL expression.

Referring to FIG. 15, the effect of high doxycycline concentration (10 µg/ml) on the growth of dox-bridged ES cells was examined. In the presence of high concentration doxycycline, the growth rate of dox-bridged ES cells stowed to a rate similar to that of cells grown in low concentration doxycycline. These data suggest that there is a range of doxycycline concentrations that may permit optimal Cdk1 expression for wild-type cell-like proliferation.

### Example 3: Generation of EARC-ALINK Modified Cells in the CDK1 locus (Mouse and Human)

In Example 3, construction of EARC (dox-bridge) vectors targeting CDK1 and use of same to control cell division in both mouse and human ALINK-modified ES cells is described. In this example, Cdk1/CDK1 is the CDL, the dox-bridge is the EARC, and HSV-TK is the ALINK. CDL Cdk1 is modified with both EARC and ALINK systems in the homozygous form, wherein doxycycline is required to induce expression of the CDL, and wherein doxycycline and GCV together provide a way of killing the modified proliferating cells.

In Example 3, dox-bridge insertion into the 5'UTR of the *CDK1* gene was achieved by homologous recombination knock-in technology.

### METHODS

Construction of mouse EARC targeting vector, CRISPR/Cas9 plasmids for mouse targeting are the same as in Example 2. Targeting and genotyping methods are also the same as described in Example 2 except that instead of C2 WT cells, Cdk1 (TK/TK) cells generated in Example 1 (FIG. 3A-3G) were used for transfection.

### Construction of EARC Targeting Vector Comprising a Dox-bridae for human CDK1

The 5' homology arm (SEQ ID NO: 69) was cloned by PCR-amplifying a 981 bp fragment from CA1 genomic DNA (primers hcdk5'F (SEQ ID NO: 70) and hcdk5'R (SEQ ID NO: 71) and cloning it into *Sbfl of the* pGem-bridge-step3. Similarly, the 3' homology arm (943 bp; SEQ ID NO: 72) was amplified by PCR using primers hcdk3'F (SEQ ID NO: 73) and hcdk3'R (SEQ ID NO: 74) and cloned into *Sphl* and *Ncol* to generate a final targeting vector, referred to as pBridge-hCdk1 (SEQ ID NO: 75).

### Construction of CRISPR/Cas9 Plasmids for human targeting

Guide RNA (hCdk1A_up (SEQ ID NO: 76), hCdk1A_low (SEQ ID NO: 77), hCdk1B_up (SEQ ID NO: 78), hCdk1B_low (SEQ ID NO: 79)) were cloned in to pX335 (SEQ ID NO: 149) and pX330 (SEQ ID NO: 150) to generate pX335-1A (SEQ ID NO: 80), pX335-1B (SEQ ID NO: 81) and pX330-1B (SEQ ID NO: 82).

### Generation of EARC-modified Human ES cells

*Targeting:* 2x10⁶ CA1 Cdk1 (TK/TK) (i.e., the cell product described in FIGS. 4A-4F) hES cells were transfected by Neon protocol 14 with 8ug DNA (Target Vector: pX330-hCdkTK-A = 6ug:2ug). After transfection, cells were plated on four 10-cm plates. Upon transfection, doxycycline was added to the media to a final concentration of 1 µg/ml. 2 days following transfection, selection was applied with 0.75 µg/ml of puromycin. Puromycin-resistant colonies were picked to 96-well plates, duplicated for further growth and genotyping with primers (hCdk1Br-5HAgen_F1 (SEQ ID NO: 83), rtTA_rev_1 (SEQ ID NO: 84), mCMV_F (SEQ ID NO:85), hCdk1Br-3HAgen_R1 (SEQ ID NO: 86)).

### RESULTS

Referring to FIG. 16A, the mouse dox-bridge target vector, pBridge was used to target mouse cell products generated in Example 1, Cdk1 (TK/TK), generating mouse Cdk1^{earc/earc,alink/alink} cells. Nine Cdk1^{earc/earc,alink/alink} clones were generated by one-shot transfection (FIG. 16B).

Referring to FIG. 5B, the data indicate that the EARC-and-ALINK-modified homozygous mouse C2 ES Cdk1^{earc/earc,alink/alink} cells properly self-renewed under ES cell conditions, differentiated in vivo, and formed complex teratomas.

Referring to FIG. 17A, the human dox-bridge target vector, pBridge-hCdk1 was used to target human CA1 cell products generated in Example 1, Cdk1(TKTK), generating human Cdk1 ^{earc/earc,alink/alink} cells. At least Cdk1 ^{earc/earc,alink/alink} CA1 clones were generated by one-shot transfection (FIG. 17B).

### Example 4: Generation of EARC-Modified Mouse ES Cells in the Top2a locus

In Example 4, construction of EARC (dox-bridge) vectors targeting Top2a and use of same to control cell division in mouse ES cells is described. In this example, Top2a/TOP2A is the CDL, which is targeted with an inducible gene expression system, wherein a dox-bridge is inserted and doxycycline induces expression of the CDL.

As described above, Top2a/TOP2A is expressed in all mitotically active (i.e., dividing) cells. In cells modified to comprise an EARC (dox-bridge) insertion at the Top2a locus, cell division is only possible in the presence of the inducer (doxycycline), which permits expression of Top2a. Thus, cell division of EARC-modified mitotically active cells can be eliminated in the absence of doxycycline.

In Example 4, dox-bridge insertion into the 5'UTR of the *Top2a* gene was achieved by homologous recombination knock-in technology.

### METHODS

### Construction of EARC Targeting Vector Comprising a Dox-bridae for Top2a

The 5' homology arm (SEQ ID NO: 87) was cloned by PCR-amplifying a 870 bp fragment from C57/B6 genomic DNA (primers Top5F (SEQ ID NO: 88) and Top5R (SEQ ID NO: 89) and cloning it into Sbfl and Spel of the pGem-bridge-step3. Similarly, the 3' homology arm (818 bp; SEQ ID NO: 90) was amplified by PCR using primers Top3F (SEQ ID NO: 91), Top3R (SEQ ID NO: 92) and cloned into Sphl and Ncol to generate a final targeting vector, referred to as pBridge-Top2a (SEQ ID NO: 93).

### Construction of CRISPR/Cas9 Plasmids

A double-nickase strategy was chosen to minimize the possibility of off-target mutations. Guide RNA sequences were cloned into pX335 (Addgene) using oligos:TOP2A1BF (SEQ ID NO: 94), TOP2A1BR (SEQ ID NO: 95), TOP2A1AF (SEQ ID NO: 96), TOP2A1AR (SEQ ID NO: 97), according to the suggested protocol (Ran et al., 2013), generating the CRISPR vectors pX335-Top2aA (SEQ ID NO: 98) and px335-Top2aB (SEQ ID NO: 99).

### Generation of EARC-modified Mouse ES cells

*Mouse ES cell culture:* All genetic manipulations were performed on a C57/B6 mouse ES cell line previously characterized (C2) (Gertsenstein etal., 2010). Mouse ES cells were grown in media based on high-glucose DMEM (Invitrogen), supplemented with 15% ES cell-grade FBS (Gibco), 0.1 mM 2-mEARCaptophenol, 2 mM L-glutamine, 1 mM sodium pyruvate, 0.1 mM non-essential amino acids, and 2,000 units/ml leukemia inhibitory factor (LIF). Cells were maintained at 37°C in 5% CO₂ on mitomycin C-treated mouse embryonic fibroblasts (MEFs).

*Targeting:* Plasmids containing the CRISPR/Cas9 components (pX335-Top2aA (SEQ ID NO: 98) and px335-Top2aB (SEQ ID NO: 99)) and the targeting plasmid (pBridge-Top2a (SEQ ID NO: 93)) were co-transfected in mouse ES cells using FuGENE HD (Clontech), according to the manufacturer's instructions, using a FuGENE:DNA ratio of 8:2, (2 µg total DNA: 250 ng for each pX335 and 1500 ng for pBridge-Top2a). Typical transfection was performed on 3x10⁵ cells, plated on 35 mm plates. Upon transfection, doxycycline was added to the media to a final concentration of 1 µg/ml. 2 days following transfection, cells were plated on a 100 mm plate and selection was applied with 1 µg/ml of puromycin. Puromycin-resistant colonies were picked 8-10 days after start of selection and maintained in 96 wall plates until PCR-screening.

*Genotyping:* DNA was extracted from ES cells directly in 96 wall plates according to (Nagy et al., 2003). Clones positive for correct insertion by homologous recombination of pBridge-Top2a in the 5' of the Top2a gene were screened by PCR using primers spanning the 5' and 3' homology arms (primers rttaRev (SEQ ID NO: 54), top2a_5scrF (SEQ ID NO: 55) for the 5' arm, primers CMVforw(SEQ ID NO: 56), top2a_3scrR (SEQ ID NO: 57) for the 3' arm).

*Targeted cell growth:* Top2a homozygously-targeted cells were trypsinized and plated on gelatinized 24 well plates at a density of 5x10⁴ cells per well. Starting one day after plating, cells were exposed to different Dox concentrations (1 µg/ml, 0.5 µg/ml, 0.05 µg/ml and 0 µg/ml), the plate was analyzed in a IncucyteZoom system (Essen Bioscience) by taking pictures every two hours for 3-4 days and measuring confluency.

### RESULTS

Referring to FIG. 18, the dox-bridge target vector, depicted in FIG. 18A, was used to generate several targeted C2 mouse ES cell lines (FIG. 18B). Nine of these cell lines were found to be homozygous targeted (FIG. 18B) comprising a dox-bridge inserted by homologous recombination into the 5'UTR of both alleles of Top2a.

As expected, this ES cell lines grows only in the presence of doxycycline. In the presence of doxycycline, the rtTA produced by *Top2a* promoter, binds to TRE and initiates transcription of the *Top2a* coding sequence. The dox-bridge may be inserted into the 5'UTR into both alleles of *Top2a* to ensure that the CDL expression could occur only through EARC. An alternative is to generate null mutations in all the remaining, non-EARC modified alleles of CDL.

Withdrawal of doxycycline resulted in complete elimination of mitotically active ES cells within 4 days (FIG. 19A).

Referring to FIG. 19B, the inventors determined how different concentrations of doxycycline affected proliferation of the dox-bridge ES cells by measuring cell growth for 4 days. ES cells in the presence of doxycycline grew exponentially, indicating their normal growth. In contrast, two days after doxycycline removal, cells growth of EARC-modified cells was completely arrested.

### Example 5: Generation of EARC-Modified Mouse ES Cells in the Cenpa locus

In Example 5, construction of EARC (dox-bridge) vectors targeting Cenpa and use of same to control cell division in mouse ES cells is described. In this example, Cenpa/CENPA is the CDL, which is targeted with an inducible gene expression system, wherein a dox-bridge is inserted and doxycycline induces expression of the CDL.

As described above, Cenpa/CENPA is expressed in all mitotically active (i.e., dividing) cells. In cells modified to comprise an EARC (dox-bridge) insertion at the Cenpa locus, cell division is only possible in the presence of the inducer (doxycycline), which permits expression of Cenpa. Thus, cell division of EARC-modified mitotically active cells can be eliminated in the absence of doxycycline.

In Example 5, dox-bridge insertion into the 5'UTR of the *Cenpa* gene was achieved by homologous recombination knock-in technology.

### METHODS

### Construction of EARC Targeting Vector Comprising a Dox-bridge

The 5' homology arm (SEQ ID NO: 100) was cloned by PCR-amplifying a 874 bp fragment fromC57/B6 genomic DNA (primers Cenpa5F (SEQ ID NO: 101) and Cenpa5R (SEQ ID NO: 102) and cloning it into Sbfl and Spel of the pGem-bridge-step3. Similarly, the 3' homology arm (825 bp; SEQ ID NO: 103) was amplified by PCR using primers Cenpa3F (SEQ ID NO: 104), Cenpa3R (SEQ ID NO: 105) and cloned into Sphl and Ncol to generate a final targeting vector, referred to as pBridge-Cenpa (SEQ ID NO: 106).

### Construction of CRISPR/Cas9 Plasmids

A double-nickase strategy was chosen to minimize the possibility of off-target mutations. Guide RNA sequences were cloned into pX335 (Addgene) using oligos CenpaAF (SEQ ID NO: 107), CenpaAR (SEQ ID NO: 108), CenpaBF (SEQ ID NO: 109), CenpaBR (SEQ ID NO: 110), according to the suggested protocol (Ran et al., 2013), generating the CRISPR vectors pX335-CenpaA (SEQ ID NO: 111) and px335-CenpaB (SEQ ID NO: 112).

### Generation of EARC-modified Mouse ES cells

*Mouse ES cell culture:* As in Example 4.

*Targeting:* Plasmids containing the CRISPR/Cas9 components (pX335-CenpaA; SEQ ID NO: 111, and px335-CenpaB; SEQ ID NO: 112) and the targeting plasmid (pBridge-Cenpa; SEQ ID NO: 106) were co-transfected in mouse ES cells using FuGENE HD (Clontech), as in Example 4.

*Genotyping:* DNA was extracted as in Example 4. Clones positive for correct insertion by homologous recombination of pBridge-Cenpa in the 5' of the Cenpa gene were screened by PCR using primers spanning the 5' and 3' homology arms (primers rttaRev (SEQ ID NO: 54), Cenpa_5scr (SEQ ID NO: 113) for the 5' arm, primers CMVforw(SEQ ID NO: 114), Cenpa_3scr (SEQ ID NO: 115) for the 3' arm).

*Targeted cell growth*: Cenpa homozygously-targeted cells were trypsinized and plated on gelatinized 24 well plates at a density of 5x10⁴ cells per well. Starting one day after plating, cells were exposed to different Dox concentrations (1 µg/ml, 0.5 µg/ml, 0.05 µg/ml and 0 µg/ml), the plate was analyzed in a IncucyteZoom system (Essen Bioscience) by taking pictures every two hours for 3-4 days and measuring confluency.

*Quantitative PCR:* Total RNA was extracted from cells treated for 2 days with 1 µg/ml and 0 µg/ml of Dox using the GeneElute total RNA miniprep kit (Sigma) according to the manufacturer's protocol. cDNA was generated by reverse transcription of 1 µg of RNA using the QuantiTect reverse transcription kit (Qiagen), according to the manufacturer's protocol. Real-time qPCR were set up in a BioRad CFX thermocycler, using SensiFast-SYBR qPCR mix (Bioline). The primers used were: qpcrcenpa_F (SEQ ID NO: 116), qpcrcenpa_R (SEQ ID NO: 117) and actBf (SEQ ID NO: 67), actBr (SEQ ID NO: 68). Results were analyzed with the ΔΔCT method and normalized for beta-actin.

### RESULTS

Referring to FIG. 20, the dox-bridge target vector, depicted in FIG. 20A, was used to generate several targeted C2 mouse ES cell lines (FIG. 20B). Six of these cells were found to have a correct insertion at the 5' and 3', and at least one clone (Cenpa#4), was found to have homozygous targeting (FIG. 20B) comprising a dox-bridge inserted by homologous recombination into the 5'UTR of both alleles of Cenpa.

As expected, this ES cell lines grows only in the presence of doxycycline. In the presence of doxycycline, the rtTA produced by *Cenpa* promoter, binds to TRE and initiates transcription of the *Cenpa* coding sequence. The dox-bridge may be inserted into the 5'UTR into both alleles of *Cenpa,* to ensure that the CDL expression could occur only through EARC. An alternative is to generate null mutations in all the remaining, non-EARC modified alleles of CDL.

Withdrawal of doxycycline resulted in complete elimination of mitotically active ES cells within 4 days (FIG. 21A).

Referring to FIG. 21B, the inventors determined by qPCR the Cenpa gene expression level in Cenpa-EARC cells with Dox and after 2 days of Dox removal, and compared it to the expression level in wild type mouse ES cells (C2). As expected Cenpa expression level is greatly reduced in Cenpa-EARC cells without Dox for 2 days.

Referring to FIG. 22, the inventors determined how different concentrations of doxycycline affected proliferation of the dox-bridge ES cells by measuring cell growth for 4 days. ES cells in the presence of doxycycline grew exponentially, indicating their normal growth. In contrast, 80 hours after doxycycline removal, cells growth was completely arrested.

### Example 6: Generation of EARC-Modified Mouse ES Cells in the Birc5 locus

In Example 6, construction of EARC (dox-bridge) vectors targeting Birc5 and use of same to control cell division in mouse ES cells is described. In this example, Birc5/BIRC5 is the CDL, which is targeted with an inducible gene expression system, wherein a dox-bridge is inserted and doxycycline induces expression of the CDL.

As described above, Birc5/BIRC5 is expressed in all mitotically active (i.e., dividing) cells. In cells modified to comprise an EARC (dox-bridge) insertion at the Birc5 locus, cell division is only possible in the presence of the inducer (doxycycline), which permits expression of Birc5. Thus, cell division of EARC-modified mitotically active cells can be eliminated in the absence of doxycycline.

In Example 6, dox-bridge insertion into the 5'UTR of the *Birc5* gene was achieved by homologous recombination knock-in technology.

### METHODS

### Construction of EARC Targeting Vector Comprising a Dox-bridge

The 3' homology arm (SEQ ID NO: 118) was cloned by PCR-amplifying a 775 bp fragment from C57/B6 genomic DNA (primers Birc3F (SEQ ID NO: 119), Birc3R (SEQ ID NO: 120)), and cloning it into Sbfl and Ncol of the pGem-bridge-step3. Similarly, the 5' homology arm (617 bp; SEQ ID NO: 121) was amplified by PCR using primers Birc5F (SEQ ID NO: 122) and Birc5R Pstl (SEQ ID NO: 123) and Spel and cloned into to generate a final targeting vector, referred to as pBridge-Birc5 (SEQ ID NO: 124).

### Construction of CRISPR/Cas9 Plasmids

A double-nickase strategy was chosen to minimize the possibility of off-target mutations. Guide RNA sequences were cloned into pX335 (Addgene) using oligos Birc5AF (SEQ ID NO: 125), Birc5AR (SEQ ID NO: 126), Birc5BF (SEQ ID NO: 127), Birc5BR (SEQ ID NO: 128), according to the suggested protocol (Ran et al., 2013), generating the CRISPR vectors pX335-Birc5A (SEQ ID NO: 129) and px335-Birc5B (SEQ ID NO: 130).

### Generation of EARC-modified Mouse ES cells

*Mouse ES cell culture:* As in Example 4.

*Targeting:* Plasmids containing the CRISPR/Cas9 components (pX335-Birc5A and px335-Birc5B) and the targeting plasmid (pBridge-Birc5) were co-transfected in mouse ES cells using FuGENE HD (Clontech), as in Example 4.

*Genotyping:* DNA was extracted as in Example 4. Clones positive for correct insertion by homologous recombination of pBridge-Birc5 in the 5' of the Birc5 gene were screened by PCR using primers spanning the 5' homology arm (primers rttaRev (SEQ ID NO: 54), Birc_5scrF (SEQ ID NO: 131)).

*Targeted cell growth*: Birc5 ho mozygously-targeted cells were trypsinized and plated on gelatinized 24 well plates at a density of 5x10⁴ cells per well. Starting one day after plating, cells were exposed to different Dox concentrations (1 µg/ml, 0.5 µg/ml, 0.05 µg/ml and 0 µg/ml), the plate was analyzed in a IncucyteZoom system (Essen Bioscience) by taking pictures every two hours for 3-4 days and measuring confluence.

*Quantitative PCR:* Total RNA was extracted from cells treated for 2 days with 1 µg/ml and 0 µg/ml of Dox using the GeneElute total RNA miniprep kit (Sigma) according to the manufacturer's protocol. cDNA was generated by reverse transcription of 1 µg of RNA using the QuantiTect reverse transcription kit (Qiagen), according to the manufacturer's protocol. Real-time qPCR were set up in a BioRad CFX thermocycler, using SensiFast-SYBR qPCR mix (Bioline). The primers used were: qpcrbirc_F (SEQ ID NO: 132), qpcrbirc_R (SEQ ID NO: 133) and actBf (SEQ ID NO: 67), actBr (SEQ ID NO: 68). Results were analyzed with the ΔΔCT method and normalized for beta-actin.

### RESULTS

Referring to FIG. 23, the dox-bridge target vector, depicted in FIG. 23A, was used to generate targeted C2 mouse ES cell lines (FIG. 23B). Five clones were found to be correctly targeted (FIG. 23B) comprising a dox-bridge inserted by recombination into the 5'UTR of both alleles of Birc5. One of these clones was Birc#3, was found to stop growing or die in the absence of Dox.

As expected, this ES cell lines grows only in the presence of doxycycline. In the presence of doxycycline, the rtTA produced by *Birc5* promoter, binds to TRE and initiates transcription of the *Birc5* coding sequence. The dox-bridge may be inserted into the 5'UTR into both alleles of *Birc5,* to ensure that the CDL expression could occur only through EARC. An alternative is to generate null mutations in all the remaining, non-EARC modified alleles of CDL.

Withdrawal of doxycycline resulted in complete elimination of mitotically active ES cells within 4 days (FIG. 24A).

Referring to FIG. 24B, the inventors determined by qPCR the Birc5 gene expression level in Birc5-EARC cells with Dox and after 2 days of Dox removal, and compared it to the expression level in wild type mouse ES cells (C2). As expected Birc5 expression level is greatly reduced in Birc5-EARC cells without Dox for 2 days.

Referring to FIG. 25, the inventors determined how different concentrations of doxycycline affected proliferation of the dox-bridge ES cells by measuring cell growth for 4 days. ES cells in the presence of doxycycline grew exponentially, indicating their normal growth. In contrast, 50 hours after doxycycline removal, cells growth was completely arrested. Interestingly, it appears that lower Dox concentrations (0.5 and 0.05 µg/ml) promote better cell growth than a higher concentration (1 µg/ml).

### Example 7: Generation of EARC-Modified Mouse ES Cells in the Eef2 locus

In Example 7, construction of EARC (dox-bridge) vectors targeting Eef2 and use of same to control cell division in mouse ES cells is described. In this example, Eef2/EEF2 is the CDL, which is targeted with an inducible gene expression system, wherein a dox-bridge is inserted and doxycycline induces expression of the CDL.

As described above, Eef2/EEF2 is expressed in all mitotically active (i.e., dividing) cells. In cells modified to comprise an EARC (dox-bridge) insertion at the Eef2 locus, cell division is only possible in the presence of the inducer (doxycycline), which permits expression of Eef2. Thus, cell division of EARC-modified mitotically active cells can be eliminated in the absence of doxycycline.

In Example 7, dox-bridge insertion into the 5'UTR of the *Eef2* gene was achieved by homologous recombination knock-in technology.

### METHODS

### Construction of EARC Targeting Vector Comprising a Dox-bridae

The 5' homology arm was cloned by PCR-amplifying a 817 bp fragment(SEQ ID NO: 134) from C57/B6 genomic DNA (primers Eef2_5F (SEQ ID NO: 135) and Eef2_5R (SEQ ID NO: 136) and cloning it into Sbfl and Spel of the pGem-bridge-step3. Similarly, the 3' homology arm (826 bp; SEQ ID NO: 137) was amplified by PCR using primers Eef2_3F (SEQ ID NO: 138), Eef2_3R (SEQ ID NO: 139) and cloned into Sphl to generate a final targeting vector, referred to as pBridge-Eef2 (SEQ ID NO: 140).

### Construction of CRISPR/Cas9 Plasmids

A double-nickase strategy was chosen to minimize the possibility of off-target mutations. Guide RNA sequences were cloned into pX335 (Addgene) using oligos Eef2aFWD (SEQ ID NO: 141), Eef2aREV (SEQ ID NO: 142), Eef2bFWD (SEQ ID NO: 143), Eef2bREV (SEQ ID NO: 144), according to the suggested protocol (Ran et al., 2013), generating the CRISPR vectors pX335-Eef2A (SEQ ID NO: 145) and px335-Eef2B (SEQ ID NO: 146).

### Generation of EARC-modified Mouse ES cells

*Mouse ES cell culture:* As in Example 4.

*Targeting:* Plasmids containing the CRISPR/Cas9 components (pX335-Eef2A and px335-Eef2B) and the targeting plasmid (pBridge-Eef2) were co-transfected in mouse ES cells using FuGENE HD (Clontech), as in Example 4.

*Genotyping:* DNA was extracted as in Example 4. Clones positive for correct insertion by homologous recombination of pBridge-Eef2 in the 5' of the Eef2 gene were screened by PCR using primers spanning the 5' homology arm (primers rttaRev (SEQ ID NO: 54), Eef2_5scrF (SEQ ID NO: 147)).

*Targeted cell growth*: Eef2 homozygously-targeted cells were trypsinized and plated on gelatinized 24 well plates at a density of 5x10⁴ cells per well. Starting one day after plating, cells were exposed to different Dox concentrations (1 µg/ml, 0.5 µg/ml, 0.05 µg/ml and 0 µg/ml), the plate was analyzed in a IncucyteZoom system (Essen Bioscience) by taking pictures every two hours for 3-4 days and measuring confluence.

### RESULTS

Referring to FIG. 26, the dox-bridge target vector, depicted in FIG. 26A, was used to generate several targeted C2 mouse ES cell lines (FIG. 26B). Nine of these cell lines was found to be correctly targeted (FIG. 26B) with at least one clone growing only in Dox-media.

As expected, this ES cell lines grows only in the presence of doxycycline. In the presence of doxycycline, the rtTA produced by *Eef2* promoter, binds to TRE and initiates transcription of the *Eef2* coding sequence. The dox-bridge may be inserted into the 5'UTR into both alleles of *Eef2,* to ensure that the CDL expression could occur only through EARC. An alternative is to generate null mutations in all the remaining, non-EARC modified alleles of CDL.

Withdrawal of doxycycline resulted in complete elimination of mitotically active ES cells within 4 days (FIG. 27).

Referring to FIG. 28, the inventors determined how different concentrations of doxycycline affected proliferation of the dox-bridge ES cells by measuring cell growth for 4 days. ES cells in the presence of doxycycline grew exponentially, indicating their normal growth. In contrast, without doxycycline cells completely failed to grow.

Although the disclosure has been described with reference to certain specific embodiments, various modifications thereof will be apparent to those skilled in the art. Any examples provided herein are included solely for the purpose of illustrating the disclosure and are not intended to limit the disclosure in any way. Any drawings provided herein are solely for the purpose of illustrating various aspects of the disclosure and are not intended to be drawn to scale or to limit the disclosure in any way. The scope of the claims appended hereto should not be limited by the preferred embodiments set forth in the above description, but should be given the broadest interpretation consistent with the present specification as a whole. The disclosures of all prior art recited herein are incorporated herein by reference in their entirety.

**Table 2: Predicted CDLs (ID refers to EntrezGene identification number CS score refers to the CRISPR score average provided in Wang et al., 2015; function refers to the known or predicted function of the locus, predictions being based on GO terms, as set forth in the Gene Ontology Consortium website http://geneontology.org/; functional category refers to 4 categories of cell functions based on the GO term-predicted function; CDL (basis) refers to information that the inventors used to predict that a gene is a CDL, predictions being based on CS score, available gene knockout (KO) data, gene function, and experimental data provided herein).**

| **Name (mouse)** | **ID (mouse)** | **Name (human)** | **ID (human)** | **CS score** | **Function (GO term)** | **Functional** | **CDL (basis)** | **Citation** |
|---|---|---|---|---|---|---|---|---|
| Actr8 | 56249 | ACTR8 | 93973 | -1.88 | chromatin remodeling | Cell cycle | CS score, function | |
| Alg11 | 207958 | ALG11 | 440138 | -127 | dolichol-linked oligosaccharide biosynthetic process | Cell cycle | CS score, function | |
| Anapc11 | 66156 | ANAPC1 1 | 51529 | -2.68 | protein ubiquitination involved in ubiquitindependent protein catabolic process | Cell cycle | CS score, function | |
| Anapc2 | 99152 | ANAPC2 | 29882 | -2.88 | mitotic cell cycle | Cell cycle | CS score, mouse K.O., function | Wirth KG, et al. Genes Dev. 2004 Jan 1;18.1):88-98 |
| Anapc4 | 52206 | ANAPC4 | 29945 | -1.79 | regulation of mitotic metaphase/anaphas e transition | Cell cycle | CS score, function | |
| Anapc5 | 59008 | ANAPC5 | 51433 | -1.66 | mitotic cell cycle | Cell cycle | CS score, function | |
| Aurka | 20878 | AURKA | 6790 | -2.26 | meiotic spindle organization | Cell cycle | CS score, mouse K.O., function | Sasai K, et al. Oncogere. 2008 Jul 3;27(29):4122-7 |
| Banf1 | 23825 | BANF1 | 8815 | -2.14 | mitotic cell cycle | Cell cycle | CS score, function | |
| Birc5 | 11799 | BIRC5 | 332 | -224 | regulation of signal transduction | Cell cycle | CS score, mouse K.O., function | Uren AG et al. Curr Biol. 2000 Nov 2;10(21):1319-28 |
| Bub3 | 12237 | BUB3 | 9184 | -315 | mitotic sister chromatid segregation | Cell cycle | CS score, mouse K.O., function | Kalitsis F, et al. Genes Dev. 2000 Sep 15,14(18:2277-82 |
| Casc5 | 76464 | CASC5 | 57082 | -1.16 | mitotic cell cycle | Cell cycle | CS score, mouse K.O., function | Overbeek PA, et al. MGI Direct Data Submiss. on. 2011 |
| Ccna2 | 12428 | CCNA2 | 890 | -1.59 | regulation of cyclindependent protein serine/threonine kinase activity | Cell cycle | CS score, mouse K.O., function | Kalaszczynska I, et al. Cell. 2009 Jul 23; 138(2):352-65 |
| Ccnh | 66671 | CCNH | 902 | -2.01 | regulation of cyclindependent protein serine/threonine kinase activity | Cell cycle | CS score. function | |
| Cdc123 | 98828 | CDC123 | 8872 | -2.45 | cell cycle | Cell cycle | CS score. function | |
| Cdc16 | 69957 | CDC16 | 8881 | -3.58 | cell division | Cell cycle | CS score, function | |
| Cdc20 | 107995 | CDC20 | 991 | -2.97 | mitotic cell cycle | Cell cycle | CS score, mouse K.O., function | Li M, et al. Mol Cell Biol. 2007 May;27(9):3481-8 |
| Cdc23 | 52563 | CDC23 | 8697 | -2.28 | mitotic cell cycle | Cell cycle | CS score. function | |
| Cdk1 | 12534 | CDK1 | 983 | -2.44 | cell cycle | Cell cycle | CS score, mouse K.O., function | Diril MK, et al. Proc Natl Acad Sci U S A. 2012 Mar 6;109(10):3826-31 |
| Cenpa | 12615 | CENPA | 1058 | -1.87 | cell cycle | Cell cycle | CS score, mouse K.O., function | Howman EV, et al. Proc Natl Acad Sci USA. 2000 Feb 1;97(3):1148-53 |
| Cenpm | 66570 | CENPM | 79019 | -2.53 | mitotic cell cycle | Cell cycle | CS score, function | |
| Chek1 | 12649 | CHEK1 | 1111 | -1.67 | protein phosphorylation | Cell cycle | CS score, mouse K.O., function | Takai H, et al. Genes Dev. 2000 Jun 15;14(12):1439-47 |
| Chmp2a | 68953 | CHMP2A | 27243 | -2.40 | vacuolar transport | Cell cycle | CS score, function | |
| Ckap5 | 75786 | CKAP5 | 9793 | -2.94 | G2/M transition of mitotic cell cycle | Cell cycle | CS score, mouse K.O., function | Barbarese E, et al. PLoS One. 2013;8(8):e69989 |
| Cltc | 67300 | CLTC | 1213 | -1.75 | intracellular protein transport | Cell cycle | CS score, function | |
| Cops5 | 26754 | COPS5 | 10987 | -1.75 | protein deneddylation | Cell cycle | CS score, mouse K.O., function | Tian L, et al. Oncogene. 2010 Nov 18;29(46):6125-37 |
| Dctn2 | 69654 | DCTN2 | 10540 | -1.48 | G2/M transition of mitotic cell cycle | Cell cycle | CS score, function | |
| Dctn3 | 53598 | DCTN3 | 11258 | -1.77 | G2/M transition of mitotic cell cycle | Cell cycle | CS score, function | |
| Dhfr | 13361 | DHFR | 1719 | -2.84 | G1/S transition of mitotic cell cycle | Cell cycle | CS score, function | |
| Dtl | 76843 | DTL | 51514 | -2.69 | protein polyubiquitination | Cell cycle | CS score, mouse K.O., function | Liu CL, et al. J Biol Chem. 2007 Jan 12;282(2):1109-18 |
| Dync1h1 | 13424 | DYNC1H 1 | 1778 | -3.44 | G2/M transition of mitotic cell cycle | Cell cycle | CS score, mouse K.O., function | Harada A, et al. J Cell Biol. 1998 Apr 6;141(1):51-9 |
| Ecd | 70601 | ECD | 11319 | -3.18 | regulation of glycolytic process | Cell cycle | CS score, function | |
| Ect2 | 13605 | ECT2 | 1894 | -1.80 | cell morphogenesis | Cell cycle | CS score, mouse K.O., function | Hansen J, et al. Proc Natl Acad Sci USA. 2003 Aug 19,100(17):9918-22 |
| Ep300 | 328572 | EP300 | 2033 | -2.04 | G2/M transition of mitotic cell cycle | Cell cycle | CS score, mouse K.O., function | Yao TP, et al. Cell. 1998 May 1;93(3):361-72 |
| Ercc3 | 13872 | ERCC3 | 2071 | -2.10 | nucleotide-excision repair | Cell cycle | CS score, mouse K.O., function | Andressoo JO, et al. Mol Cell Biol. 2009 Mar,29(5):1276-90 |
| Espl1 | 105988 | ESPL1 | 9700 | -3.24 | proteolysis | Cell cycle | CS score, mouse K.O., function | Wirth KG et al. J Cell Biol. 2006 Mar 13;172(6):847-60 |
| Fntb | 110606 | FNTB | 2342 | -2.42 | phototransduction, visible light | Cell cycle | CS score, mouse K.O., function | Mijimolle N, et al. Cancer Cell. 2005 Apr;7(4):313-24 |
| Gadd45gi p1 | 102060 | GADD45 GIP1 | 90480 | -1.81 | organelle organization | Cell cycle | CS score, mouse K.O., function | Kwon MC, et al. EMBO J. 2008 Feb 20;27(4):642-68 |
| Gins1 | 69270 | GINS1 | 9837 | -1.84 | mitotic cell cycle | Cell cycle | CS score, mouse K.O., function | Ueno M, et al. Mol Cell Biol. 2005 Dec;25(23):10528-32 |
| Gnb2l1 | 14694 | GNB2L1 | 10399 | -2.84 | osteoblast differentiation | Cell cycle | CS score, function | |
| Gspt1 | 14852 | GSPT1 | 2935 | -1.77 | G1/S transition of mitotic cell cycle | Cell cycle | CS score, function | |
| Haus1 | 225745 | HAUS1 | 115106 | -1.92 | spindle assembly | Cell cycle | CS score, function | |
| Haus3 | 231123 | HAUS3 | 79441 | -1.38 | mitotic nuclear division | Cell cycle | CS score, function | |
| Haus5 | 71909 | HAUS5 | 23354 | -2.55 | spindle assembly | Cell cycle | CS score, function | |
| Haus8 | 76478 | HAUS8 | 93323 | -1.73 | mitotic nuclear division | Cell cycle | CS score, function | |
| Hdac3 | 15183 | HDAC3 | 8841 | -2.12 | histone deacetylation | Cell cycle | CS score, mouse K.O., function | Bhaskara S, et al. Mol Cell. 2008 Apr 11;30(1):61-72 |
| Kif11 | 16551 | KIF11 | 3832 | -3.23 | microtubule-based movement | Cell cycle | CS score, mouse K.O., function | Castillo A, et al. Biochem Biophys Res Commun. 2007 Jun 8;357(3):694-9 |
| Kif23 | 71819 | KIF23 | 9493 | -1.59 | microtubule-based movement | Cell cycle | CS score, function | |
| Kpnb1 | 16211 | KPNB1 | 3837 | -3.19 | nucleocytoplasmic transport | Cell cycle | CS score, mouse K.O., function | Miura K, et al. Biochem Biophys Res Commun. 2006 Mar 3;34)(1):132-8 |
| Mastl | 67121 | MASTL | 84930 | -2.36 | protein phosphorylation | Cell cycle | CS score, mouse K.O., function | Alvarez-Fernandez M, et al. Proc Natl Acad Sci U S A. 2013 Oct 22;110(43):17374-9 |
| Mau2 | 74549 | MAU2 | 23383 | -2.71 | mitotic cell cycle | Cell cycle | CS score, mouse K.O., function | Smith TG, et al. Genesis. 2014 Jul;52(7)687-94 |
| Mcm3 | 17215 | MCM3 | 4172 | -2.52 | G1/S transition of mitotic cell cycle | Cell cycle | CS score, function | |
| Mcm4 | 17217 | MCM4 | 4173 | -1.87 | G1/S transition of mitotic cell cycle | Cell cycle | CS score, mouse K.O., function | Shima N, et al. Nat Genet. 2007 Jan;39(1):93-8 |
| Mcm7 | 17220 | MCM7 | 4176 | -2.39 | G1/S transition of mitotic cell cycle | Cell cycle | CS score, function | |
| Mnat1 | 17420 | MNAT1 | 4331 | -1.22 | regulation of cyclindependent protein serine/threonine kinase activity | Cell cycle | CS score, mouse K.O., function | Rossi DJ. et al. EMBO J. 2001 Jun 1;20(11):2844-56 |
| Mybbp1a | 18432 | MYBBP1 A | 10514 | -2.17 | osteoblast differentiation | Cell cycle | CS score, mouse K.O., function | Mori S, et al. PLoS One. 2012;7(17):e39723 |
| Ncapd2 | 68298 | NCAPD2 | 9918 | -2.03 | mitotic chromosome condensation | Cell cycle | CS score, function | |
| Ncaph | 215387 | NCAPH | 23397 | -2.33 | mitotic chromosome condensation | Cell cycle | CS score, mouse K.O., function | Nishide K, et al. PLoS Genet. 2014 Dec.10(12):e10048 47 |
| Ndc80 | 67052 | NDC80 | 10403 | -2.98 | attachment of mitotic spindle microtubules to kinetochore | Cell cycle | CS score, function | |
| Nle1 | 217011 | NLE1 | 54475 | -1.88 | somitogenesis | Cell cycle | CS score, mouse K.O., function | Hentges KE, et al. Gene Expr Patterns. 2006 Aug;6(6)653-65 |
| Nsl1 | 381318 | NSL1 | 25936 | -1.90 | mitotic cell cycle | Cell cycle | CS score, function | |
| Nude | 18221 | NUDC | 10726 | -1.93 | mitotic cell cycle | Cell cycle | CS score, function | |
| Nuf2 | 66977 | NUF2 | 83540 | -1.78 | mitotic nuclear division | Cell cycle | CS score, function | |
| Nup133 | 234865 | NUP133 | 55746 | -2.26 | mitotic cell cycle | Cell cycle | CS score, mouse K.O., function | Garcia-Garcia MJ, et al. Proc Natl Acad Sci USA. 2005 Apr 26;102(17):5913-9 |
| Nup160 | 59015 | NUP160 | 23279 | -2.64 | mitotic cell cycle | Cell cycle | CS score, function | |
| Nup188 | 227699 | NUP188 | 23511 | -1.16 | mitotic cell cycle | Cell cycle | CS score, function | |
| Nup214 | 227720 | NUP214 | 8021 | -2.70 | mitotic cell cycle | Cell cycle | CS score, mouse K.O., function | van Deursen J, et al. EMBO J 1996 Oct 15;15(20):5574-83 |
| n/a | n/a | NUP62 | 23636 | -2.35 | mitotic cell cycle | Cell cycle | CS score, function | |
| Nup85 | 445007 | NUP85 | 79902 | -2.47 | mitotic cell cycle | Cell cycle | CS score, function | |
| Orc3 | 50793 | ORC3 | 23595 | -1.67 | G1/S transition of mitotic cell cycle | Cell cycle | CS score, function | |
| Pafah1b1 | 18472 | PAFAH1 B1 | 5048 | -2.34 | G2/M transition of mitotic cell cycle | Cell cycle | CS score, mouse K.O., function | Cahana A, et al. Proc Natl Acad Sci USA. 2001 May 22;98(11):6429-84 |
| Pcid2 | 234069 | PCID2 | 55795 | -1.98 | negative regulation of apoptotic process | Cell cycle | CS score, function | |
| Pfas | 237823 | PFAS | 5198 | -2.58 | purine nucleotide biosynthetic process | Cell cycle | CS score, function | |
| Phb2 | 12034 | PHB2 | 11331 | -2.98 | protein import into nucleus, translocation | Cell cycle | CS score, mouse K.O., function | Park SE, et al. Mol Cell Biol. 2005 Mar;25(5):1989-99 |
| Pkmyt1 | 268930 | PKMYT1 | 9088 | -1.93 | regulation of cyclindependent protein serine/threonine kinase activity | Cell cycle | CS score, function | |
| Plk1 | 18817 | PLK1 | 5347 | -2.83 | protein phosphorylation | Cell cycle | CS score, mouse K.O., function | Lu LY, et al. Mol Cell Biol. 2008 Nov;28(22):6870-6 |
| Pmf1 | 67037 | PMF1 | 11243 | -2.15 | mitotic cell cycle | Cell cycle | CS score, function | |
| Pole2 | 18974 | POLE2 | 5427 | -3.08 | G1/S transition of mitotic cell cycle | Cell cycle | CS score, function | |
| Ppat | 231327 | PPAT | 5471 | -2.15 | G1/S transition of mitotic cell cycle | Cell cycle | CS score, function | |
| Psma6 | 26443 | PSMA6 | 5687 | -3.51 | G1/S transition of mitotic cell cycle | Cell cycle | CS score, function | |
| Psma7 | 26444 | PSMA7 | 5688 | -2.91 | G1/S transition of mitotic cell cycle | Cell cycle | CS score, function | |
| Psmb1 | 19170 | PSMB1 | 5689 | -1.63 | G1/S transition of mitotic cell cycle | Cell cycle | CS score, function | |
| Psmb4 | 19172 | PSMB4 | 5692 | -2.91 | G1/S transition of mitotic cell cycle | Cell cycle | CS score, function | |
| Psmd12 | 66997 | PSMD12 | 5718 | -1.69 | G1/S transition of mitotic cell cycle | Cell cycle | CS score, function | |
| Psmd13 | 23997 | PSMD13 | 5719 | -1.57 | G1/S transition of mitotic cell cycle | Cell cycle | CS score, function | |
| Psmd14 | 59029 | PSMD14 | 10213 | -3.01 | G1/S transition of mitotic cell cycle | Cell cycle | CS score, function | |
| Psmd7 | 17463 | PSMD7 | 5713 | -2.18 | G1/S transition of mitotic cell cycle | Cell cycle | CS score, mouse K.O., function | Soriano P, et al. Genes Dev. 1987 Jun;1(4):366-75 |
| Racgap1 | 26934 | RACGAP 1 | 29127 | -1.94 | mitotic spindle assembly | Cell cycle | CS score, mouse K.O., function | Van de Putte T, et al. Mech Dev. 2001 Apr;102(1-2):33-44 |
| Rad21 | 19357 | RAD21 | 5885 | -2.12 | mitotic cell cycle | Cell cycle | CS score, function | |
| Rae1 | 66679 | RAE1 | 8480 | -2.15 | mitotic cell cycle | Cell cycle | CS score, mouse K.O., function | Babu JR et al. J Cell Biol. 2003 Feb 3;160(3):341-53 |
| Rcc1 | 100088 | RCC1 | 1104 | -2.91 | G1/S transition of mitotic cell cycle | Cell cycle | CS score, function | |
| Rfc3 | 69263 | RFC3 | 5983 | -274 | mitotic cell cycle | Cell cycle | CS score, function | |
| Rps27a | 78294 | RPS27A | 6233 | -2.74 | G1/S transition of mitotic cell cycle | Cell cycle | CS score, function | |
| Rrm2 | 20135 | RRM2 | 6241 | -3.09 | G1/S transition of mitotic cell cycle | Cell cycle | CS score, function | |
| Sae1 | 56459 | SAE1 | 10055 | -2.08 | cellular protein modification process | Cell cycle | CS score, function | |
| Sec13 | 110379 | SEC13 | 6396 | -2.96 | mitotic cell cycle | Cell cycle | CS score, function | |
| Smarcb1 | 20587 | SMARCB 1 | 6598 | -1.98 | chromatin remodeling | Cell cycle | CS score, mouse K.O., function | Guidi CJ, et al. Mol Cell Biol. 2001 May 15;21(10):3598-603 |
| Smc2 | 14211 | SMC2 | 10592 | -2.13 | mitotic chromosome condensation | Cell cycle | CS score, mouse K.O., function | Nishide K, et al. PLoS Genet. 2014 Dec;10(12)e10048 47 |
| Smc4 | 70099 | SMC4 | 10051 | -1.47 | chromosome organization | Cell cycle | CS score, function | |
| Son | 20658 | SON | 6651 | -1.99 | microtubule cytoskeleton organization | Cell cycle | CS score, function | |
| Spc24 | 67629 | SPC24 | 147841 | -2.83 | mitotic cell cycle | Cell cycle | CS score, function | |
| Spc25 | 66442 | SPC25 | 57405 | -1.63 | mitotic cell cycle | Cell cycle | CS score, function | |
| Terf2 | 21750 | TERF2 | 7014 | -2.17 | telomere maintenance | Cell cycle | CS score, mouse K.O., function | Celli GB, et al. Nat Cell Biol. 2005 Jul;7(7):712-8 |
| Tpx2 | 72119 | TPX2 | 22974 | -2.08 | apoptotic process | Cell cycle | CS score, mouse K.O., function | Aguirre-Portoles C, et al. Cancer Res. 2012 Mar 15;72(6)1518-28 |
| Tubg1 | 103733 | TUBG1 | 7283 | -2.08 | microtubule nucleation | Cell cycle | CS score, mouse K.O., function | Yuba-Kubo A, et al. Dev Biol. 2005 Jun 15;282(2):361-73 |
| Tubgcp2 | 74237 | TUBGCP 2 | 10844 | -2.78 | microtubule cytoskeleton organization | Cell cycle | CS score, function | |
| Tubgcp5 | 233276 | TUBGCP 5 | 114791 | -1.76 | microtubule cytoskeleton organization | Cell cycle | CS score, function | |
| Tuhacp6 | 328580 | TUBGCP 6 | 85378 | -1.52 | microtubule cytoskeleton organization | Cell cycle | CS score, function | |
| Txnl4a | 27366 | TXNL4A | 10907 | -3.89 | mitotic nuclear division | Cell cycle | CS score, function | |
| Usp39 | 28035 | USP39 | 10713 | -2.85 | spliceosomal complex assembly | Cell cycle | CS score, function | |
| Wdr43 | 72515 | WDR43 | 23160 | -3.02 | reproduction | Cell cycle | CS score, function | |
| Zfp830 | 66983 | ZNF830 | 91603 | -1.52 | blastocyst growth | Cell cycle | CS score, mouse K.O., function | Houlard M, et al. Cell Cycle. 2011 Jan 1:10(1):108-17 |
| Aatf | 56321 | AATF | 26574 | -1.46 | cellular response to DNA damage stimulus | DNA replication , DNA repair | CS score, mouse K.O., function | Thomas T, et al. Dev Biol. 2000 Nov 15;227(2):324-42 |
| Alyref | 21681 | ALYREF | 10189 | -1.92 | regulation of DNA recombination | DNA replication , DNA repair | CS score, function | |
| Brf2 | 66653 | BRF2 | 55290 | -2.30 | DNA-templated transcription, initiation | DNA replication , DNA repair | CS score, function | |
| Cdc45 | 12544 | CDC45 | 8318 | -3.69 | DNA replication checkpoint | DNA replication , DNA repair | CS score, mouse K.O., function | Yoshida K, et al. Mol Cell Biol. 2001 Jul;21(14):4698-668 |
| Cdc6 | 23834 | CDC6 | 990 | -1.87 | DNA replication initiation | DNA replication, DNA repair | CS score, function | |
| Cdt1 | 67177 | CDT1 | 81620 | -2.74 | DNA replication checkpoint | DNA replication , DNA repair | CS score, function | |
| Cinp | 67236 | CINP | 51550 | -1.64 | DNA replication | DNA replication , DNA repair | CS score, function | |
| Cirh1a | 21771 | CIRH1A | 84916 | -2.62 | transcription, DNA-templated | DNA replication, DNA repair | CS score, function | |
| Ddb1 | 13194 | DDB1 | 1642 | -2.14 | nucleotide-excision repair, DNA damage removal | DNA replication , DNA repair | CS score, mouse K.O., function | Cang Y, et al. Cell. 2006 Dec 1;127(5)929-40 |
| Ercc2 | 13871 | ERCC2 | 2068 | -2.80 | DNA duplex unwinding | DNA replication , DNA repair | CS score, mouse K.O., function | de Boer J, et al. Cancer Res. 1998 Jan 1;58(1):89-94 |
| Gabpb1 | 14391 | GABPB1 | 2553 | -1.74 | transcription, DNA-templated | DNA replication , DNA repair | CS score, mouse K.O., function | Xue HH, et al. Mol Cell Biol. 2008 Jul;28(13):4300-9 |
| Gtf2b | 229906 | GTF2B | 2959 | -2.76 | regulation of transcription, DNA-templated | DNA replication , DNA repair | CS score, function | |
| Gtf2h4 | 14885 | GTF2H4 | 2968 | -1.93 | nucleotide-excision repair, DNA damage removal | DNA replication , DNA repair | CS score, function | |
| Gtf3a | 66596 | GTF3A | 2971 | -2.25 | regulation of transcription, DNA-templated | DNA replication , DNA repair | CS score, function | |
| Gtf3c1 | 233863 | GTF3C1 | 2975 | -2.45 | transcription, DNA-templated | DNA replication , DNA repair | CS score, function | |
| Gtf3c2 | 71752 | GTF3C2 | 2976 | -2.09 | transcription, DNA-templated | DNA replication , DNA repair | CS score, function | |
| Hinfp | 102423 | HINFP | 25988 | -2.35 | DNA damage checkpoint | DNA replication , DNA repair | CS score, mouse K.O., function | Xie R, et al. Proc Natl Acad Sci U S A. 2009 Jul 9 |
| n/a | n/a | HIST2H2 AA3 | 8337 | -1.71 | DNA repair | DNA replication , DNA repair | CS score, function | |
| Ints3 | 229543 | INTS3 | 65123 | -3.14 | DNA repair | DNA replication , DNA repair | CS score, function | |
| Kin | 16588 | KIN | 22944 | -1.99 | DNA replication | DNA replication , DNA repair | CS score, function | |
| Mcm2 | 17216 | MCM2 | 4171 | -2.86 | DNA replication initiation | DNA replication , DNA repair | CS score, function | |
| Mcm6 | 17219 | MCM6 | 4175 | -1.55 | DNA replication | DNA replication , DNA repair | CS score, function | |
| Mcrs1 | 51812 | MCRS1 | 10445 | -1.23 | DNA repair | DNA replication , DNA repair | CS score, function | |
| Med11 | 66172 | MED11 | 400569 | -2.39 | transcription, DNA-templated | DNA replication DNA repair | CS score, function | |
| Mtpap | 67440 | MTPAP | 55149 | -1.86 | transcription, DNA-templated | DNA replication , DNA repair | CS score, function | |
| Myc | 17869 | MYC | 4609 | -2.49 | regulation of transcription, DNA-templated | DNA replication , DNA repair | CS score, mouse K.O., function | Trumpp A, et al. Nature. 2001 Dec 13;414(6865):768-73 |
| Ndnl2 | 66647 | NDNL2 | 56160 | -2.03 | DNA repair | DNA replication , DNA repair | CS score, function | |
| Nol11 | 68979 | NOL11 | 25926 | -1.59 | transcription, DNA-templated | DNA replication , DNA repair | CS score, function | |
| Nol8 | 70930 | NOL8 | 55035 | -1.35 | DNA replication | DNA replication , DNA repair | CS score, function | |
| Pcna | 18538 | PCNA | 5111 | -3.60 | DNA replication | DNA replication , DNA repair | CS score, mouse K.O., function | Roa S, et al. Proc Natl Acad Sci U S A. 2008 Oct 21;105(42):16248-53 |
| Pola1 | 18968 | POLA1 | 5422 | -2.28 | DNA-dependent DNA replication | DNA replication , DNA repair | CS score, function | |
| Pold2 | 18972 | POLD2 | 5425 | -2.51 | DNA replication | DNA replication , DNA repair | CS score, function | |
| Pole | 18973 | POLE | 5426 | -2.90 | DNA replication | DNA replication , DNA repair | CS score, function | |
| Polr1a | 20019 | POLR1A | 25885 | -262 | transcription, DNA-templated | DNA replication , DNA repair | CS score, function | |
| n/a | n/a | POLR2J2 | 246721 | -3.08 | transcription, DNA-templated | DNA replication , DNA repair | CS score, function | |
| Polr3a | 218832 | POLR3A | 11128 | -2.43 | transcription, DNA-templated | DNA replication , DNA repair | CS score, function | |
| Polr3c | 74414 | POLR3C | 10623 | -2.02 | transcription, DNA-templated | DNA replication , DNA repair | CS score, function | |
| Polr3h | 78929 | POLR3H | 171568 | -2.66 | transcription, DNA-templated | DNA replication , DNA repair | CS score, function | |
| Prmt1 | 15469 | PRMT1 | 3276 | -2.40 | regulation of transcription, DNA-templated | DNA replication, DNA repair | CS score, mouse K.O., function | Pawlak MR, et al. Mol Cell Biol. 2000 Jul;20(13):4859-69 |
| Prmt5 | 27374 | PRMT5 | 10419 | -269 | regulation of transcription, DNA-templated | DNA replication , DNA repair | CS score, mouse K.O., function | Tee WW, et al. Genes Dev. 2010 Dec 15;24(24):2772-7 |
| Puf60 | 67959 | PUF60 | 22827 | -2.69 | transcription, DNA-templated | DNA replication , DNA repair | CS score, function | |
| Rad51 | 19361 | RAD51 | 5888 | -2.29 | DNA repair | DNA replication , DNA repair | CS score, mouse K.O., function | Tsuzuki T, et al. Proc Natl Acad Sci U S A. 1996 Jun 25;93(13):6236-40 |
| Rad51c | 114714 | RAD51C | 5889 | -1.62 | DNA repair | DNA replication , DNA repair | CS score, mouse K.O., function | Smeenk G, et al. Mutat Res. 2010 Jul 7;689(1-2):50-58 |
| Rbx1 | 56438 | RBX1 | 9978 | -2.19 | DNA repair | DNA replication , DNA repair | CS score, mouse K.O., function | Tan M, et al. Proc Natl Acad Sci U S A. 2009 Apr 14;106(15):6203-8 |
| Rfc2 | 19718 | RFC2 | 5982 | -2.88 | DNA-dependent DNA replication | DNA replication , DNA repair | CS score, function | |
| Rfc4 | 106344 | RFC4 | 5984 | -1.92 | DNA-dependent DNA replication | DNA replication , DNA repair | CS score, function | |
| Rfc5 | 72151 | RFC5 | 5985 | -2.78 | DNA-dependent DNA replication | DNA replication , DNA repair | CS score, function | |
| Rpa1 | 68275 | RPA1 | 6117 | -2.61 | DNA replication | DNA replication , DNA repair | CS score, mouse K.O., function | Wang Y, et al. Nat Genet. 2005 Jul;37(7):750-5 |
| Rps3 | 27050 | RPS3 | 6188 | -2.75 | DNA repair | DNA replication , DNA repair | CS score, function | |
| Rrm1 | 20133 | RRM1 | 6240 | -4.16 | DNA replication | DNA replication , DNA repair | CS score, function | |
| Ruvbl1 | 56505 | RUVBL1 | 8607 | -3.26 | DNA duplex unwinding | DNA replication , DNA repair | CS score, function | |
| Ruvbl2 | 20174 | RUVBL2 | 10856 | -3.91 | DNA repair | DNA replication , DNA repair | CS score, function | |
| Sap30bp | 57230 | SAP30BP | 29115 | -2.18 | regulation of transcription, DNA-templated | DNA replication , DNA repair | CS score, function | |
| Smc1a | 24061 | SMC1A | 8243 | -2.76 | DNA repair | DNA replication , DNA repair | CS score, function | |
| Smc3 | 13006 | SMC3 | 9126 | -3.22 | DNA repair | DNA replication , DNA repair | CS score, mouse K.O., function | White JK et al. Cell. 2013 Jul 18;154(2):452-64 |
| Snapc4 | 227644 | SNAPC4 | 6621 | -2.78 | regulation of transcription, DNA-templated | DNA replication DNA repair | CS score, function | |
| Snapc5 | 330959 | SNAPC5 | 10302 | -2.24 | regulation of transcription, DNA-templated | DNA replication , DNA repair | CS score, function | |
| Snip1 | 76793 | SNIP1 | 79753 | -1.78 | regulation of transcription, DNA-templated | DNA replication , DNA repair | CS score, function | |
| Srrt | 83701 | SRRT | 51593 | -2.18 | transcription, DNA-templated | DNA replication , DNA repair | CS score, mouse K.O., function | Wilson MD, et al. Mol Cell Biol. 2008 Mar;28(5):1503-14 |
| Ssrp1 | 20833 | SSRP1 | 6749 | -1.45 | DNA replication | DNA replication , DNA repair | CS score, mouse K.O., function | Cao S, et al 5 mouse embryos. Mol Cell Biol. 2003 Aug;23(15):5301-7 |
| Taf10 | 24075 | TAF10 | 6881 | -1.38 | DNA-templated transcription, initiation | DNA replication , DNA repair | CS score, mouse K.O., function | Mohan WS Jr, et al. Mol Cell Biol. 2003 Jun;23(12):4307-18 |
| Taf1c | 21341 | TAF1C | 9013 | -1.80 | chromatin silencing at rDNA | DNA replication , DNA repair | CS score, function | |
| Taf6 | 21343 | TAF6 | 6878 | -1.84 | DNA-templated transcription, initiation | DNA replication , DNA repair | CS score, function | |
| Taf6l | 67706 | TAF6L | 10629 | -1.53 | DNA-templated transcription, initiation | DNA replication , DNA repair | CS score, function | |
| Ticrr | 77011 | TICRR | 90381 | -2.03 | DNA replication | DNA replication , DNA repair | CS score, function | |
| Top1 | 21969 | TOP1 | 7150 | -2.02 | DNA topological change | DNA replication , DNA repair | CS score, mouse K.O., function | Morham SG, et al. Mol Cell Biol. 1996 Dec;16(12):6804-9 |
| Top2a | 21973 | TOP2A | 7153 | -1.50 | DNA replication | DNA replication , DNA repair | CS score, function | |
| Trrap | 100683 | TRRAP | 8295 | -2.36 | DNA repair | DNA replication , DNA repair | CS score, mouse K.O., function | Herceg Z et al. Nat Genet. 2001 Oct;29(2) 206-11 |
| Zbtb11 | 271377 | ZBTB11 | 27107 | -2.34 | transcription, DNA-templated | DNA replication , DNA repair | CS score, function | |
| Actl6a | 56456 | ACTL6A | 86 | -2.33 | neural retina development | DNA replication, DNA repair | CS score, mouse K.O., function | Krasteva V, et al. Blood. 2012 Dec 6;120(24:4720-32 |
| Atr | 245000 | ATR | 545 | -2.01 | double-strand break repair via homologous recombination | DNA replication, DNA repair | CS score, mouse K.O., function | de Klein A, et al. Curr Biol. 2000 Apr 20;10(8):479-82 |
| Chd4 | 107932 | CHD4 | 1108 | -1.71 | chromatin organization | DNA replication, DNA repair | CS score, function | |
| Ciao1 | 26371 | CIAO1 | 9391 | -1.94 | chromosome segregation | DNA replication, DNA repair | CS score, function | |
| Ddx21 | 56200 | DDX21 | 9188 | -2.84 | osteoblast differentiation | DNA replication, DNA repair | CS score, function | |
| Dnaja3 | 83945 | DNAJA3 | 9093 | -2.19 | mitochondrion organization | DNA replication, DNA repair | CS score, mouse K.O., function | Lo JF, et al. Mol Cell Biol. 2004 Mar;24(6):2226-36 |
| Dnmt1 | 13433 | DNMT1 | 1786 | -1.97 | methylation | DNA replication, DNA repair | CS score, mouse K.O., function | Lei H, et al Development. 1996 Oct;122(10):3195-205 |
| Gins2 | 272551 | GINS2 | 51659 | -3.32 | double-strand break repair via break-induced replication | DNA replication, DNA repair | CS score, function | |
| Gtf2h3 | 209357 | GTF2H3 | 2967 | -1.84 | nucleotide-excision repair | DNA replication, DNA repair | CS score, function | |
| n/a | n/a | HIST2H2 BF | 440689 | -1.70 | chromatin organization | DNA replication, DNA repair | CS score, function | |
| Mms221 | 212377 | MMS22L | 253714 | -1.38 | double-strand break repair via homologous recombination | DNA replication, DNA repair | CS score, function | |
| Mtor | 56717 | MTOR | 2475 | -1.98 | double-strand break repair via homologous recombination | DNA replication, DNA repair | CS score, mouse K.O., function | Murakam M, et al. Mol Cell Biol. 2004 Aug;24(15):6710-8 |
| Narfl | 67563 | NARFL | 64428 | -2.13 | response to hypoxia | DNA replication, DNA repair | CS score, mouse K.O., function | Song D, et al. J Biol Chem. 2011 Mar 2 |
| Ndufa13 | 67184 | NDUFA1 3 | 51079 | -1.31 | positive regulation of peptidase activity | DNA replication, DNA repair | CS score, mouse K.O., function | Huang G, et al Mol Cell Biol. 2004 Oct;24(191:8447-56 |
| Nol12 | 97961 | NOL12 | 79159 | -1.61 | poly(A) RNA binding | DNA replication, DNA repair | CS score, function | |
| Nup107 | 103468 | NUP107 | 57122 | -1.30 | transport | DNA replication, DNA repair | CS score, function | |
| Oraov1 | 72284 | ORAOV1 | 220064 | -2.26 | biological_process | DNA replication, DNA repair | CS score, function | |
| Pam16 | 66449 | PAM16 | 51025 | -2.13 | protein import into mitochondrial matrix | DNA replication, DNA repair | CS score, function | |
| Pola2 | 18969 | POLA2 | 23649 | -2.84 | protein import into nucleus, translocation | DNA replication, DNA repair | CS score, function | |
| Ppie | 56031 | PPIE | 10450 | -1.63 | protein peptidyl-prolyl isomerization | DNA replication, DNA repair | CS score, function | |
| Prpf19 | 28000 | PRPF19 | 27339 | -3.96 | generation of catalytic spliceosome for first transesterification step | DNA replication, DNA repair | CS score, mouse K.O., function | Fortschegger K, et al. Mol Cell Biol. 2007 Apr;27(8) : 3123-30 |
| Psmc5 | 19184 | PSMC5 | 5705 | -2.57 | ER-associated ubiquitin-dependent protein catabolic process | DNA replication, DNA repair | CS score, function | |
| Rbbp5 | 213464 | RBBP5 | 5929 | -1.70 | chromatin organization | DNA replication, DNA repair | CS score, function | |
| Rbbp6 | 19647 | RBBP6 | 5930 | -1.78 | in utero embryonic development | DNA replication, DNA repair | CS score, mouse K.O., function | Li L, et al Proc Natl Acad Sci USA. 2007 May 8;104(19):7951-6 |
| Rptor | 74370 | RPTOR | 57521 | -2.43 | TOR signaling | DNA replication, DNA repair | CS score, mouse K.O., function | Guertin CA, et al. Dev Cell. 2006 Dec;11(6):859-71 |
| Rrn3 | 106298 | RRN3 | 54700 | -1.85 | in utero embryonic development | DNA replication, DNA repair | CS score, mouse K.O., function | Yuan X, et al Mol Cell. 2005 Jul 1;19(1):77-87 |
| Smg1 | 233789 | SMG1 | 23049 | -1.94 | double-strand break repair via homologous recombination | DNA replication, DNA repair | CS score, mouse K.O., function | Roberts IL, et al. Proc Natl Acad Sci USA. 2013 Jan 22;110(4):E285-94 |
| Supt6 | 20926 | SUPT6H | 6830 | -1.78 | chromatin remodeling | DNA replication, DNA repair | CS score, mouse K.O., function | Dietrich JE, et al. EMBO Rep. 2015 Aug;16(8):1005-21 |
| Tada2b | 231151 | TADA2B | 93624 | -1.23 | chromatin organization | DNA replication, DNA repair | CS score, function | |
| Tfip11 | 54723 | TFIP11 | 24144 | -2.19 | spliceosomal complex disassembly | DNA replication, DNA repair | CS score, function | |
| Tonsl | 66914 | TONSL | 4796 | -3.03 | double-strand break repair via homologous recombination | DNA replication, DNA repair | CS score, function | |
| Tpt1 | 22070 | TPT1 | 7178 | -2.05 | calcium ion transport | DNA replication, DNA repair | CS score, mouse K.O., function | Susini L, et al. Cell Death Differ. 2008 Aug;15(8):1211-20 |
| Uba1 | 22201 | UBA1 | 7317 | -2.90 | protein ubiquitination | DNA replication, DNA repair | CS score, function | |
| Vps25 | 28084 | VPS25 | 84313 | -2.31 | protein targeting to vacuole involved in ubiquitin-dependent protein catabolic process via the multivesicular body sorting pathway | DNA replication, DNA repair | CS score, function | |
| Wbscr22 | 66138 | WBSCR2 2 | 114049 | -2.70 | methylation | DNA replication, DNA repair | CS score, function | |
| Wdr5 | 140858 | WDR5 | 11091 | -1.99 | skeletal system development | DNA replication, DNA repair | CS score, function | |
| Xab2 | 67439 | XAB2 | 56949 | -2.86 | generation of catalytic spliceosome for first transesterification step | DNA replication, DNA repair | CS score, mouse K.O., function | Yonemasu R, et al. DNA Repair (Amst). 2005 Apr 4;4(4):479-91 |
| Zmat2 | 66492 | ZMAT2 | 153527 | -2.17 | histidine-tRNA ligase activity | DNA replication, DNA repair | CS score, function | |
| Zfp335 | 329559 | ZNF335 | 63925 | -158 | in utero embryonic development | DNA replication, DNA repair | CS score, mouse K.O., function | Yang YJ, et al. Cell. 2012 Nov 21;151(5):1097-112 |
| Acly | 104112 | ACLY | 47 | -1.54 | acetyl-CoA metabolic process | Metabolism | CS score, mouse K.O., function | Beigneux AP, et al J Biol Chem 2004 Mar 5;279(10):9557-64 |
| Adsl | 11564 | ADSL | 158 | -2.39 | metabolic process | Metabolism | CS score, function | |
| Ahcy | 269378 | AHCY | 191 | -2.07 | sulfur amino acid metabolic process | Metabolism | CS score, function | |
| Arl2 | 56327 | ARL2 | 402 | -2.29 | energy reserve metabolic process | Metabolism | CS score, function | |
| Chka | 12660 | CHKA | 1119 | -1.64 | lipid metabolic process | Metabolism | CS score, mouse K.O., function | Wu G, et al. J Biol Chem. 2008 Jan 18;283(3):1456-62 |
| Coasy | 71743 | COASY | 80347 | -1.82 | vitamin metabolic process | Metabolism | CS score, function | |
| Cox4i1 | 12857 | COX4l1 | 1327 | -2.00 | generation of precursor metabolites and energy | Metabolism | CS score, function | |
| n/a | n/a | COX7C | 1350 | -1.59 | generation of precursor metabolites and energy | Metabolism | CS score, function | |
| n/a | n/a | CTPS1 | 1503 | -2.52 | nucleobase-containing compound metabolic process | Metabolism | CS score, function | |
| Ddx10 | 77591 | DDX10 | 1662 | -2.02 | metabolic process | Metabolism | CS score, function | |
| Ddx20 | 53975 | DDX20 | 11218 | -2.49 | metabolic process | Metabolism | CS score, mouse K.O., function | Mouillet JF, et al. Endocrinology. 2008 May;149(5)2168-75 |
| Dhdds | 67422 | DHDDS | 79947 | -2.86 | metabolic process | Metabolism | CS score, function | |
| Dhx30 | 72831 | DHX30 | 22907 | -1.93 | metabolic process | Metabolism | CS score, function | |
| Dhx8 | 217207 | DHX8 | 1659 | -2.61 | metabolic process | Metabolism | CS score, function | |
| Dhx9 | 13211 | DHX9 | 1660 | -1.73 | metabolic process | Metabolism | CS score, mouse K.O., function | Lee CG, et al. Proc Natl Acac Sci U S A. 1998 Nov 10;95(23):13709-13 |
| Dlst | 78920 | DLST | 1743 | -1.93 | metabolic process | Metabolism | CS score, function | |
| Dpagt1 | 13478 | DPAGT1 | 1798 | -2.80 | UDP-N-acetylglucosamine metabolic process | Metabolism | CS score, mouse K.O., function | Marek KW, et al. Glycobiology. 1999 Nov;9(11:1263-71 |
| Gfpt1 | 14583 | GFPT1 | 2673 | -1.81 | fructose 6-phosphate metabolic process | Metabolism | CS score, function | |
| Gmps | 229363 | GMPS | 8833 | -180 | purine nucleobase metabolic process | Metabolism | CS score, function | |
| Gpn1 | 74254 | GPN1 | 11321 | -1.79 | metabolic process | Metabolism | CS score, function | |
| Gpn3 | 68080 | GPN3 | 51184 | -3.12 | metabolic process | Metabolism | CS score, function | |
| Guk1 | 14923 | GUK1 | 2987 | -2.67 | purine nucleotide metabolic process | Metabolism | CS score, function | |
| Hsd17b10 | 15108 | HSD1781 0 | 3028 | -1.84 | lipid metabolic process | Metabolism | CS score, function | |
| Lrr1 | 69706 | LRR1 | 122769 | -3.44 | metabolic process | Metabolism | CS score, function | |
| Mta2 | 52856 | MTG2 | 26164 | -2.04 | metabolic process | Metabolism | CS score, function | |
| Myh9 | 17886 | MYH9 | 4627 | -1.70 | metabolic process | Metabolism | CS score, mouse K.O., function | Matsushita T, et al. Biochem Biophys Res Commun. 2004 Dec 24;325(4):1163-71 |
| Nampt | 59027 | NAMPT | 10135 | -2.40 | vitamin metabolic process | Metabolism | CS score, mouse K.O., function | Revollo JR, et al. Cell Metab. 2007 Nov:6(5):363-75 |
| Ncbp1 | 433702 | NCBP1 | 4686 | -1.62 | RNA metabolic process | Metabolism | CS score, function | |
| Nfs1 | 18041 | NFS1 | 9054 | -2.40 | metabolic process | Metabolism | CS score, function | |
| Ppcdc | 66812 | PPCDC | 60490 | -1.98 | metabolic process | Metabolism | CS score. function | |
| Qrsl1 | 76563 | QRSL1 | 55278 | -1.67 | metabolic process | Metabolism | CS score, function | |
| Rpp14 | 67053 | RPP14 | 11102 | -1.72 | fatty acid metabolic process | Metabolism | CS score, function | |
| Smarca4 | 20586 | SMARCA 4 | 6597 | -1.89 | metabolic process | Metabolism | CS score, mouse K.O., function | Bultman S, et al. Mol Cell. 2000 Dec;6(6) 1287-95 |
| Snrnp200 | 320632 | SNRNP2 00 | 23020 | -2.50 | metabolic process | Metabolism | CS score, function | |
| Srbd1 | 78586 | SRBD1 | 55133 | -235 | nucleobase-containing compound metabolic process | Metabolism | CS score, function | |
| Srcap | 100043 597 | SRCAP | 10847 | -1.43 | metabolic process | Metabolism | CS score, function | |
| Ube2i | 22196 | UBE2I | 7329 | -2.55 | metabolic process | Metabolism | CS score, mouse K.O., function | Nacerddine K, et al. Dev Cell. 2005 Dec;9(6):769-79 |
| Ube2m | 22192 | UBE2M | 9040 | -2.39 | metabolic process | Metabolism | CS score, function | |
| Vcp | 269523 | VCP | 7415 | -2.85 | metabolic process | Metabolism | CS score, mouse K.O., function | Muller JM, et al. Biochem Biophys Res Commun. 2007 Mar 9;354(2):459-465 |
| Aamp | 227290 | AAMP | 14 | -2.37 | angiogenesis | Metabolism | CS score, function | |
| Acin1 | 56215 | ACIN1 | 22985 | -1.53 | positive regulation of defense response to virus by host | Metabolism | CS score, function | |
| Aco2 | 11429 | ACO2 | 50 | -2.08 | tricarboxylic acid cycle | Metabolism | CS score, function | |
| Adss | 11566 | ADSS | 159 | -2.46 | purine nucleotide biosynthetic process | Metabolism | CS score, function | |
| Alg2 | 56737 | ALG2 | 85365 | -2.29 | biosynthetic process | Metabolism | CS score, function | |
| Ap2s1 | 232910 | AP2S1 | 1175 | -2.00 | intracellular protein transport | Metabolism | CS score, function | |
| Arcn1 | 213827 | ARCN1 | 372 | -191 | intracellular protein transport | Metabolism | CS score, function | |
| Armc7 | 276905 | ARMC7 | 79637 | -2.02 | molecular_function | Metabolism | CS score, function | |
| Atp2a2 | 11938 | ATP2A2 | 488 | -3.01 | calcium ion transmembrane transport | Metabolism | CS score, mouse K.O., function | Andersson KB, et al. Cell Calcium. 2009 Sep;46(3):219-25 |
| Atp5a1 | 11946 | ATP5A1 | 498 | -1.99 | negative regulation of endothelial cell proliferation | Metabolism | CS score, function | |
| Atp5d | 66043 | ATP5D | 513 | -2.21 | oxidative phosphorylation | Metabolism | CS score, function | |
| Atp5o | 28080 | ATP5O | 539 | -1.17 | ATP biosynthetic process | Metabolism | CS score, function | |
| At6v0b | 114143 | A TP6VOB | 533 | -3.01 | cellular iron ion homeostasis | Metabolism | CS score, function | |
| Atp6v0c | 11984 | ATP6V0C | 527 | -3.84 | cellular iron ion homeostasis | Metabolism | CS score, mouse K.O., function | Sun-Waca GH, et al. Dev Biol. 2000 Dec 15;228(2):315-25 |
| Atp6v1a | 11964 | ATP6V1A | 523 | -3.58 | proton transport | Metabolism | CS score, function | |
| Atp6v1b2 | 11966 | ATP6V1B 2 | 526 | -2.94 | cellular iron ion homeostasis | Metabolism | CS score, function | |
| Atp6v1d | 73834 | ATP6V1D | 51382 | -.258 | transmembrane transport | Metabolism | CS score, function | |
| Aurkaip1 | 66077 | AURKAIP 1 | 54998 | -1.56 | organelle organization | Metabolism | CS score, function | |
| n/a | n/a | C1orf109 | 54955 | -2.43 | molecular_function | Metabolism | CS score, function | |
| n/a | n/a | C21orf59 | 56683 | -2.77 | cell projection morph--ienesis | Metabolism | CS score, function | |
| Ccdc84 | 382073 | CCDC84 | 338657 | -1.86 | molecular_function | Metabolism | CS score, function | |
| Cct2 | 12461 | CCT2 | 10576 | -3.23 | protein folding | Metabolism | CS score, function | |
| Cct3 | 12462 | CCT3 | 7203 | -3.31 | protein folding | Metabolism | CS score, function | |
| Cct4 | 12464 | CCT4 | 10575 | -262 | protein folding | Metabolism | CS score, function | |
| Cct5 | 12465 | CCT5 | 22948 | -2.84 | protein folding | Metabolism | CS score, function | |
| Cct7 | 12468 | CCT7 | 10574 | -2.47 | protein folding | Metabolism | CS score, function | |
| Cct8 | 12469 | CCT8 | 10694 | -2.03 | protein folding | Metabolism | CS score, function | |
| Cdipt | 52858 | CDIPT | 10423 | -2.53 | phospholipid biosynthetic process | Metabolism | CS score, function | |
| Cenpi | 102920 | CENPI | 2491 | -1.81 | centromere complex assembly | Metabolism | CS score, function | |
| Chordc1 | 66917 | CHORDC 1 | 26973 | -1.52 | regulation of centrosome duplication | Metabolism | CS score, mouse K.O., function | Ferretti R, et al. Dev Cell. 2010 Mar 16; 18(3):486-95 |
| Coa5 | 76178 | COA5 | 493753 | -2.33 | mitochondrion | Metabolism | CS score, function | |
| Cog4 | 102339 | COG4 | 25839 | -1.39 | Golgi vesicle transport | Metabolism | CS score, function | |
| Copa | 12847 | COPA | 1314 | -1.63 | intracellular protein transport | Metabolism | CS score, function | |
| Copb1 | 70349 | COPB1 | 1315 | -2.30 | intracellular protein transport | Metabolism | CS score, function | |
| Copb2 | 50797 | COPB2 | 9276 | -2.65 | intracellular protein transport | Metabolism | CS score, function | |
| Cope | 59042 | COPE | 11316 | -2.93 | ER to Golgi vesicle-mediated transport | Metabolism | CS score, function | |
| Copz1 | 56447 | COPZ1 | 22818 | -1.87 | transport | Metabolism | CS score, function | |
| Coq4 | 227683 | COQ4 | 51117 | -1.29 | ubiquinone biosynthetic process | Metabolism | CS score, function | |
| Cox15 | 226139 | COX15 | 1355 | -2.14 | mitochondrial electron transport, cytochrome c to oxygen | Metabolism | CS score, mouse K.O., function | Viscomi C, et al. Cell Metab. 2011 Jul 6;14(1):80-90 |
| Cox17 | 12856 | COX17 | 10063 | -1.97 | copper ion transport | Metabolism | CS score, mouse K.O., function | Takahashi Y, et al. Mol Cell Biol. 2002 Nov;22(21):7614-21 |
| Cse11 | 110750 | CSE1L | 1434 | -2.31 | protein export from nucleus | Metabolism | CS score, mouse K.O., function | Bera TK, et al. Mol Cell Biol. 2001 Oct;21(20):7020-4 |
| Csnk2b | 13001 | CSNK2B | 1460 | -1.94 | regulation of protein kinase activity | Metabolism | CS score, mouse K.O., function | Buchou T, et al. Mol Cell Biol. 2003 Feb:23(3):908-15 |
| Cycs | 13063 | CYCS | 54205 | -2.36 | response to reactive oxygen species | Metabolism | CS score, mouse K.O., function | Li K, et al. Cell. 2000 May 12;101(4:389-99 |
| Dad1 | 13135 | DAD1 | 1603 | -2.21 | protein glycosylation | Metabolism | CS score, mouse K.O., function | Brewster JL, et al. Genesis. 2000 Apr;26(4)271-8 |
| Dap3 | 65111 | DAP3 | 7818 | -1.70 | apoptotic process | Metabolism | CS score, mouse K.O., function | Kim HR, et al. FASEB J 2007 Jan;21(1):188-96 |
| Dctn5 | 59288 | DCTN5 | 84516 | -2.39 | antigen processing and presentation of exogenous peptide antigen via MHC class II | Metabolism | CS score, function | |
| Ddost | 13200 | DDOST | 1650 | -2.38 | protein N-linked glycosylation via asparaqine | Metabolism | CS score, function | |
| Dgcr8 | 94223 | DGCR8 | 54487 | -2.10 | gene expression | Metabolism | CS score, mouse K.O., function | Ouchi Y, et al. J Neurosci 2013 May 29;33(22):9408-19 |
| Dhodh | 56749 | DHODH | 1723 | -2.57 | de novo' pyrimidine nucleobase biosynthetic process | Metabolism | CS score, function | |
| Dnlz | 52838 | DNLZ | 728489 | -1.92 | protein folding | Metabolism | CS score, function | |
| Dnm1l | 74006 | DNM1L | 10059 | -3.25 | mitochondrial fission | Metabolism | CS score, mouse K.O., function | Wakabayashi J, et al. J Cell Biol. 2009 Sep 21; 186(6):805-16 |
| Dnm2 | 13430 | DNM2 | 1785 | -3.98 | endocytosis | Metabolism | CS score, mouse K.O., function | Ferguson SM, et al. Dev Cell. 2009 Dec;17(6):811-22 |
| Dohh | 102115 | DOHH | 83475 | -1.76 | peptidyl-lysine modification to peptidyl-hypusine | Metabolism | CS score, function | |
| Dolk | 227697 | DOLK | 22845 | -2.38 | dolichol-linked oligosaccharide biosynthetic process | Metabolism | CS score, function | |
| Donson | 60364 | DONSON | 29980 | -2.30 | multicellular organismal development | Metabolism | CS score, function | |
| Dph3 | 105638 | DPH3 | 285381 | -1.62 | peptidyl-diphthamide biosynthetic process from peptidyl-histidine | Metabolism | CS score, mouse K.O., function | Liu S, et al. Mol Cell Biol. 2006 May;26(10):3835-41 |
| Dtymk | 21915 | DTYMK | 1841 | -3.54 | phosphorylation | Metabolism | CS score, function | |
| Eif2b2 | 217715 | EIF2B2 | 8892 | -2.24 | ovarian follicle development | Metabolism | CS score, function | |
| Eif2s2 | 67204 | EIF2S2 | 8894 | -2.33 | in utero embryonic development | Metabolism | CS score, mouse K.O., function | Heaney CD, et al. Hum Mol Genet. 2009 Apr 15;18(8):1395-404 |
| Emc1 | 230866 | EMC1 | 23065 | -1.34 | protein folding in endoplasmic reticulum | Metabolism | CS score, function | |
| Emc7 | 73024 | EMC7 | 56851 | -2.27 | biological_process | Metabolism | CS score, function | |
| Eno1 | 13806 | ENO1 | 2023 | -2.03 | glycolytic process | Metabolism | CS score, mouse K.O., function | Couldrey C, et al. Dev Dyn. 1998 Jun;212(2):284-92 |
| Fam50a | 108160 | FAM50A | 9130 | -3.16 | spermatogenesis | Metabolism | CS score, function | |
| Fam96b | 68523 | FAM96B | 51647 | -1.90 | iron-sulfur cluster assembly | Metabolism | CS score, function | |
| Fdps | 110196 | FDPS | 2224 | -2.41 | isoprenoid biosynthetic process | Metabolism | CS score, function | |
| Gapdh | 14433 | GAPDH | 2597 | -2.40 | oxidation-reduction process | Metabolism | CS score, function | |
| Gart | 14450 | GART | 2618 | -1.87 | purine nucleobase biosynthetic process | Metabolism | CS score, function | |
| Gemin4 | 276919 | GEMIN4 | 50628 | -1.56 | spliceosomal snRNP assembly | Metabolism | CS score, function | |
| Gemin5 | 216766 | GEMIN5 | 25929 | -2.51 | spliceosomal snRNP assembly | Metabolism | CS score, function | |
| Ggps1 | 14593 | GGPS1 | 9453 | -1.62 | cholesterol biosynthetic process | Metabolism | CS score, function | |
| Gmppb | 331026 | GMPPB | 29925 | -3.22 | biosynthetic process | Metabolism | CS score, function | |
| Gnb1l | 13972 | GNB1L | 54584 | -1.93 | G-protein coupled receptor signaling pathway | Metabolism | CS score, function | |
| n/a | n/a | GOLGA6 L1 | 283767 | -3.15 | | Metabolism | CS score, function | |
| Gosr2 | 56494 | GOSR2 | 9570 | -1.13 | protein targeting to vacuole | Metabolism | CS score, function | |
| Gpkow | 209416 | GPKOW | 27238 | -1.36 | biological_process | Metabolism | CS score, function | |
| Gpn2 | 100210 | GPN2 | 54707 | -3.71 | biological_process | Metabolism | CS score, function | |
| Gps1 | 209318 | GPS1 | 2873 | -2.11 | inactivation of MAPK activity | Metabolism | CS score, function | |
| Grpel1 | 17713 | GRPEL1 | 80273 | -2.61 | protein folding | Metabolism | CS score, function | |
| Grwd1 | 101612 | GRWD1 | 83743 | -1.90 | poly(A) RNA binding | Metabolism | CS score, function | |
| Hmgcr | 15357 | HMGCR | 3156 | -2.94 | cholesterol biosynthetic process | Metabolism | CS score, mouse K.O., function | Ohashi K et al. J Biol Chem. 2003 Oct 31;278(44):42936-41 |
| Hmgcs1 | 208715 | HMGCS1 | 3157 | -2.41 | liver development | Metabolism | CS score, function | |
| Hspa5 | 14828 | HSPA5 | 3309 | -3.86 | platelet degranulation | Metabolism | CS score, mouse K.O., function | Luo S, et al. Mol Cell Biol. 2006 Aug;26(15):5688-97 |
| Hspa9 | 15526 | HSPA9 | 3313 | -3.55 | protein folding | Metabolism | CS score, function | |
| Hspd1 | 15510 | HSPD1 | 3329 | -1.95 | response to hypoxia | Metabolism | CS score, mouse K.O., function | Christensen JH, et al. Cell Stress Chaperones. 2010 Nov;15(6):851-63 |
| Hspe1 | 15528 | HSPE1 | 3336 | -3.75 | osteoblast differentiation | Metabolism | CS score, function | |
| Hyou1 | 12282 | HYOU1 | 10525 | -2.06 | response to ischemia | Metabolism | CS score, function | |
| Ipo13 | 230673 | IPO13 | 9670 | -2.84 | intracellular protein transport | Metabolism | CS score, function | |
| Iscu | 66383 | ISCU | 23479 | -2.40 | cellular iron ion homeostasis | Metabolism | CS score, function | |
| Itpk1 | 217837 | ITPK1 | 3705 | -1.55 | phosphorylation | Metabolism | CS score, function | |
| Kansl2 | 69612 | KANSL2 | 54934 | -1.19 | chromatin organization | Metabolism | CS score, function | |
| Kansl3 | 226976 | KANSL3 | 55683 | -1.53 | chromatin organization | Metabolism | CS score, function | |
| Kri1 | 215194 | KRI1 | 65095 | -2.49 | poly(A) RNA binding | Metabolism | CS score, function | |
| Lamtor2 | 83409 | LAMTOR 2 | 28956 | -1.62 | activation of MAPKK activity | Metabolism | CS score, mouse K.O., function | Teis D, et al. J Cell Biol. 2006 Dec 18:175(6):861-8 |
| Leng8 | 232798 | LENG8 | 114823 | -1.75 | biological_process | Metabolism | CS score, function | |
| Ltv1 | 353258 | LTV1 | 84946 | -1.81 | nucleoplasm | Metabolism | CS score, function | |
| Mak16 | 67920 | MAK16 | 84549 | -2.30 | poly(A) RNA binding | Metabolism | CS score, function | |
| Mat2a | 232087 | MAT2A | 4144 | -2.34 | S-adenosylmethionine biosynthetic process | Metabolism | CS score, function | |
| Mcm3ap | 54387 | MCM3AP | 8888 | -1.58 | immune system process | Metabolism | CS score, mouse K.O., function | Yoshida M, et al. Genes Cells. 2007 Oct;12(10):1205-13 |
| Mdn1 | 100019 | MDN1 | 23195 | -1.68 | protein complex assembly | Metabolism | CS score, function | |
| n/a | n/a | MFAP1 | 4236 | -1.94 | biological_process | Metabolism | CS score, function | |
| Mmgt1 | 236792 | MMGT1 | 93380 | -1.55 | magnesium ion transport | Metabolism | CS score, function | |
| Mrpl16 | 94063 | MRPL16 | 54948 | -1.80 | organelle organization | Metabolism | CS score, function | |
| Mrpl17 | 27397 | MRPL17 | 63875 | -1.80 | mitochondrial genome maintenance | Metabolism | CS score, function | |
| Mrpl33 | 66845 | MRPL33 | 9553 | -1.62 | organelle organization | Metabolism | CS score, function | |
| Mrpl38 | 60441 | MRPL38 | 64978 | -1.95 | organelle organization | Metabolism | CS score, function | |
| Mrpl39 | 27393 | MRPL39 | 54148 | -1.71 | organelle organization | Metabolism | CS score, function | |
| Mrpl45 | 67036 | MRPL45 | 84311 | -1.75 | organelle organization | Metabolism | CS score, function | |
| Mrpl46 | 67308 | MRPL46 | 26589 | -1.83 | organelle organization | Metabolism | CS score, function | |
| Mrpl53 | 68499 | MRPL53 | 116540 | -1.84 | organelle organization | Metabolism | CS score, function | |
| Mrps22 | 64655 | MRPS22 | 56945 | -1.32 | organelle organization | Metabolism | CS score, function | |
| Mrps25 | 64658 | MRPS25 | 64432 | -1.63 | organelle organization | Metabolism | CS score, function | |
| Mrps35 | 232536 | MRPS35 | 60488 | -1.60 | organelle organization | Metabolism | CS score, function | |
| Mrps5 | 77721 | MRPS5 | 64969 | -1.65 | organelle organization | Metabolism | CS score, function | |
| Mvd | 192156 | MVD | 4597 | -3.24 | isoprenoid biosynthetic process | Metabolism | CS score, function | |
| Mvk | 17855 | MVK | 4598 | -1.73 | isoprenoid biosynthetic process | Metabolism | CS score, function | |
| Naa25 | 231713 | NAA25 | 80018 | -2.40 | peptide alpha-N-acetyltransferase activity | Metabolism | CS score, function | |
| Napa | 108124 | NAPA | 8775 | -2.31 | intracellular protein transport | Metabolism | CS score, function | |
| Nat10 | 98956 | NAT10 | 55226 | -2.16 | biological_process | Metabolism | CS score, function | |
| Ndor1 | 78797 | NDOR1 | 27158 | -2.10 | cell death | Metabolism | CS score, function | |
| Ndufab1 | 70316 | NDUFAB 1 | 4706 | -1.83 | fatty acid biosynthetic process | Metabolism | CS score, function | |
| Nol10 | 217431 | NOL10 | 79954 | -1.79 | poly(A) RNA binding | Metabolism | CS score, function | |
| Nop9 | 67842 | NOP9 | 161424 | -1.44 | biological_process | Metabolism | CS score, function | |
| Nrde2 | 217827 | NRDE2 | 55051 | -2.69 | biological_process | Metabolism | CS score, function | |
| Nsf | 18195 | NSF | 4905 | -2.76 | intra-Golgi vesicle-mediated transport | Metabolism | CS score, function | |
| Nubp1 | 26425 | NUBP1 | 4682 | -2.05 | cellular iron ion homeostasis | Metabolism | CS score, function | |
| Nudcd3 | 209586 | NUDCD3 | 23386 | -1.71 | molecular_function | Metabolism | CS score, function | |
| Nup155 | 170762 | NUP155 | 9631 | -1.59 | nucleocytoplasmic transport | Metabolism | CS score, mouse K.O., function | Zhang X, et al. Cell. 2008 Dec 12;135(6):1017-27 |
| Nup93 | 71805 | NUP93 | 9688 | -2.11 | protein import into nucleus | Metabolism | CS score, function | |
| Nus1 | 52014 | NUS1 | 116150 | -1.94 | angiogenesis | Metabolism | CS score, mouse K.O., function | Park EJ, et al. Cell Metab. 2014 Sep 2;20(3):448-57 |
| Nvl | 67459 | NVL | 4931 | -2.61 | positive regulation of telomerase activity | Metabolism | CS score, function | |
| Ogdh | 18293 | OGDH | 4967 | -2.98 | tricarboxylic acid cycle | Metabolism | CS score, function | |
| Osbp | 76303 | OSBP | 5007 | -2.06 | lipid transport | Metabolism | CS score, function | |
| Pak1ip1 | 68083 | PAK1IP1 | 55003 | -2.28 | cell proliferation | Metabolism | CS score, function | |
| Pfdn2 | 18637 | PFDN2 | 5202 | -1.32 | protein folding | Metabolism | CS score, function | |
| Pgam1 | 18648 | PGAM1 | 5223 | -2.37 | glycolytic process | Metabolism | CS score, function | |
| Pkm | 18746 | PKM | 5315 | -1.68 | glycolytic process | Metabolism | CS score, mouse K.O., function | Lewis SE, et al. 1983:267-78. Plenum Publ. Corp. |
| Pmpcb | 73078 | PMPCB | 9512 | -1.77 | proteolysis | Metabolism | CS score, function | |
| Ppil2 | 66053 | PPIL2 | 23759 | -3.01 | protein polyubiquitination | Metabolism | CS score, function | |
| Ppp4c | 56420 | PPP4C | 5531 | -2.89 | protein dephosphorylation | Metabolism | CS score, mouse K.O., function | Toyo-oka K, et al. J Cell Biol. 2008 Mar 24;180(6):1133-47 |
| Prelid1 | 66494 | PRELID1 | 27166 | -2.27 | apoptotic process | Metabolism | CS score, function | |
| Prpf31 | 68988 | PRPF31 | 26121 | -3.20 | spliceosomal trisnRNP complex assembly | Metabolism | CS score, mouse K.O., function | Bujakowska K, et al. Invest Ophthalmol Vis Sci. 2009 Dec;50(12):5927-33 |
| Prpf6 | 68879 | PRPF6 | 24148 | -2.96 | spliceosomal trisnRNP complex assembly | Metabolism | CS score, function | |
| Psma1 | 26440 | PSMA1 | 5682 | -2.39 | proteasomal ubiquitin-independent protein catabolic process | Metabolism | CS score, function | |
| Psma2 | 19166 | PSMA2 | 5683 | -2.23 | proteasomal ubiquitin-independent protein catabolic process | Metabolism | CS score, function | |
| Psma3 | 19167 | PSMA3 | 5684 | -2.30 | proteasomal ubiq uitin-independent protein catabolic process | Metabolism | CS score, function | |
| Psmb2 | 26445 | PSMB2 | 5690 | -2.12 | proteasomal ubiquitin-independent protein catabolic process | Metabolism | CS score, function | |
| Psmb3 | 26446 | PSMB3 | 5691 | -2.78 | proteolysis involved in cellular protein catabolic process | Metabolism | CS score, function | |
| Psmb5 | 19173 | PSMB5 | 5693 | -1.67 | proteasomal ubiquitin-independent protein catabolic process | Metabolism | CS score, function | |
| Psmb6 | 19175 | PSMB6 | 5694 | -2.42 | proteasomal ubiq uiti n-i ndepen dent protein catabolic process | Metabolism | CS score, function | |
| Psmb7 | 19177 | PSMB7 | 5695 | -2.69 | proteasomal ubiq uiti n-i ndepen dent protein catabolic process | Metabolism | CS score, function | |
| Psmc2 | 19181 | PSMC2 | 5701 | -2.35 | protein catabolic process | Metabolism | CS score, function | |
| Psmc3 | 19182 | PSMC3 | 5702 | -2.76 | ER-associated ubiquitin-dependent protein catabolic process | Metabolism | CS score, mouse K.O., function | Sakao Y, et al. Genomics. 2000 Jul 1;67(1):1-7 |
| Psmc4 | 23996 | PSMC4 | 5704 | -2.36 | blastocyst development | Metabolism | CS score, mouse K.O., function | Sakao Y, et al. Genomics. 2000 Jul 1;67(1):1-7 |
| Psmd1 | 70247 | PSMD1 | 5707 | -1.88 | regulation of protein catabolic process | Metabolism | CS score, function | |
| Psmd2 | 21762 | PSMD2 | 5708 | -2.16 | regulation of protein catabolic process | Metabolism | CS score, function | |
| Psmd3 | 22123 | PSMD3 | 5709 | -2.10 | regulation of protein catabolic process | Metabolism | CS score, function | |
| Psmd4 | 19185 | PSMD4 | 5710 | -1.77 | ubiq uitin-dependent protein catabolic process | Metabolism | CS score, mouse K.O., function | Soriano P, et al. Genes Dev. 1987 Jun;1(4):366-75 |
| Psmd6 | 66413 | PSMD6 | 9861 | -2.27 | proteasome-mediated ubiquitin-dependent protein catabolic process | Metabolism | CS score, function | |
| Psmg3 | 66506 | PSMG3 | 84262 | -2.57 | molecular_function | Metabolism | CS score, function | |
| Ptpmt1 | 66461 | PTPMT1 | 114971 | -2.89 | protein dephosphorylation | Metabolism | CS score, mouse K.O., function | Shen J, et al. Mol Cell Biol. 2011 Dec;31(24):4902-16 |
| Ptpn23 | 104831 | PTPN23 | 25930 | -1.59 | negative regulation of epithelial cell migration | Metabolism | CS score, mouse K.O., function | Gingras MC, et al. Int J Dev Biol. 2009;53(7):1069-74 |
| Rabggta | 56187 | RABGGT A | 5875 | -3.18 | protein prenylation | Metabolism | CS score, function | |
| Rabggtb | 19352 | RABGGT B | 5876 | -2.44 | protein geranylgeranylation | Metabolism | CS score, function | |
| Rbm19 | 74111 | RBM19 | 9904 | -2.03 | multicellular organismal development | Metabolism | CS score, mouse K.O., function | Zhang J, et al. BMC Dev Biol. 2008;8:115 |
| Rfk | 54391 | RFK | 55312 | -1.56 | riboflavin biosynthetic process | Metabolism | CS score, mouse K.O., function | Yazdanpanah B, et al. Nature. 2009 Aug 27;460(7259): 1159-63 |
| Rheb | 19744 | RHEB | 6009 | -1.38 | signal transduction | Metabolism | CS score, mouse K.O., function | Zou J, et al. Dev Cell. 2011 Jan 18;20(1)97-108 |
| Riok1 | 71340 | RIOK1 | 83732 | -1.27 | protein phosphorylation | Metabolism | CS score, function | |
| Rpn1 | 103963 | RPN1 | 6184 | -2.13 | protein glycosylation | Metabolism | CS score, function | |
| Rtfdc1 | 66404 | RTFDC1 | 51507 | -2.09 | biological_process | Metabolism | CS score, function | |
| Sacm1l | 83493 | SACM1L | 22908 | -1.80 | protein dephosphorylation | Metabolism | CS score, function | |
| Samm50 | 68653 | SAMM50 | 25813 | -1.62 | protein targeting to mitochondrion | Metabolism | CS score, function | |
| Sco2 | 100126 824 | SCO2 | 9997 | -1.60 | eye development | Metabolism | CS score, mouse K.O., function | Yang H, et al. Hum Mol Genet. 2010 Jan 1;19(1):170-80 |
| Sdha | 66945 | SDHA | 6389 | -2.20 | tricarboxylic acid cycle | Metabolism | CS score, function | |
| Sdhb | 67680 | SDHB | 6390 | -2.33 | tricarboxylic acid cycle | Metabolism | CS score, function | |
| Sec61a1 | 53421 | SEC61A1 | 29927 | -2.42 | protein transport | Metabolism | CS score, function | |
| Slc20a1 | 20515 | SLC20A1 | 6574 | -2.38 | sodium ion transport | Metabolism | CS score, mouse K.O., function | Festing MH, et al. Genesis. 2009 Dec,47(12):858-63 |
| Slc7a6os | 66432 | SLC7A6O S | 84138 | -2.30 | hematopoietic progenitor cell differentiation | Metabolism | CS score, function | |
| Smn1 | 20595 | SMN1 | 6606 | -1.58 | spliceosomal complex assembly | Metabolism | CS score, mouse K.O., function | Hsieh-Li HM, et al. Nat Genet. 2000 Jan;24(1):66-70 |
| Smu1 | 74255 | SMU1 | 55234 | -3.65 | molecular_function | Metabolism | CS score, function | |
| Snrpd1 | 20641 | SNRPD1 | 6632 | -2.79 | spliceosomal complex assembly | Metabolism | CS score, function | |
| Snrpd3 | 67332 | SNRPD3 | 6634 | -3.62 | spliceosomal complex assembly | Metabolism | CS score, function | |
| Snrpe | 20643 | SNRPE | 6635 | -2.74 | spliceosomal complex assembly | Metabolism | CS score, function | |
| Spata5 | 57815 | SPATA5 | 166378 | -1.50 | multicellular organismal development | Metabolism | CS score, function | |
| Spata5l1 | 214616 | SPATA5L 1 | 79029 | -2.70 | molecular_function | Metabolism | CS score, function | |
| Tango6 | 272538 | TANGO6 | 79613 | -2.29 | integral component of membrane | Metabolism | CS score, function | |
| n/a | n/a | TBC1D3B | 414059 | -1.67 | positive regulation of GTPase activity | Metabolism | CS score, function | |
| n/a | n/a | TBC1D3 C | 414060 | -2.01 | positive regulation of GTPase activity | Metabolism | CS score, function | |
| Tbcb | 66411 | TBCB | 1155 | -1.97 | nervous system development | Metabolism | CS score, function | |
| Tbcc | 72726 | TBCC | 6903 | -3.02 | cell morphogenesis | Metabolism | CS score, function | |
| Tbcd | 108903 | TBCD | 6904 | -1.82 | microtubule cytoskeleton organization | Metabolism | CS score, function | |
| Tcp1 | 21454 | TCP1 | 6950 | -2.34 | protein folding | Metabolism | CS score, function | |
| Telo2 | 71718 | TELO2 | 9894 | -2.34 | regulation of TOR signaling | Metabolism | CS score, mouse K.O., function | Takai H, et al. Cell. 2007 Dec 28;131(7):1248-59 |
| Tex10 | 269536 | TEX10 | 54881 | -1.26 | integral component of membrane | Metabolism | CS score, function | |
| Tfrc | 22042 | TFRC | 7037 | -3.40 | cellular iron ion homeostasis | Metabolism | CS score, mouse K.O., function | Levy JE, et al. Nat Genet. 1999 Apr;21(4):396-9 |
| Timm10 | 30059 | TIMM10 | 26519 | -1.99 | protein targeting to mitochondrion | Metabolism | CS score, function | |
| Timm13 | 30055 | TIMM13 | 26517 | -1.62 | protein targeting to mitochondrion | Metabolism | CS score, function | |
| Timm23 | 53600 | TIMM23 | 100287 932 | -2.00 | protein targeting to mitochondrion | Metabolism | CS score, mouse K.O., function | Ahting U, et al. Biochim Biophys Acta. 2009 May:1787(5):371-6 |
| Timm44 | 21856 | TIMM44 | 10469 | -1.73 | protein import into mitochondrial matrix | Metabolism | CS score, function | |
| Tmx2 | 66958 | TMX2 | 5107S | -229 | biological_process | Metabolism | CS score, function | |
| Tnpo3 | 320938 | TNPO3 | 23534 | -1.82 | splicing factor protein import into nucleus | Metabolism | CS score, function | |
| Trmt112 | 67674 | TRMT112 | 51504 | -3.70 | peptidyl-glutamine methylation | Metabolism | CS score, function | |
| Trnau1ap | 71787 | TRNAU1 AP | 54952 | -1.40 | selenocysteine incorporation | Metabolism | CS score, function | |
| Ttc1 | 66827 | TTC1 | 7265 | -1.74 | protein folding | Metabolism | CS score, function | |
| Ttc27 | 74196 | TTC27 | 55622 | -2.54 | biological_process | Metabolism | CS score, function | |
| Tti1 | 75425 | TTI1 | 9675 | -2.91 | regulation of TOR signaling | Metabolism | CS score, function | |
| Tti2 | 234138 | TTI2 | 80185 | -1.94 | molecular_function | Metabolism | CS score, function | |
| n/a | n/a | TUBB | 203068 | -3.40 | microtubule-based process | Metabolism | CS score, function | |
| Txn2 | 56551 | TXN2 | 25828 | -1.41 | sulfate assimilation | Metabolism | CS score, mouse K.O., function | Nonn L, et al. Mol Cell Biol. 2003 Feb;23(3):916-22 |
| Uqcrc1 | 22273 | UQCRC1 | 7384 | -1.29 | oxidative phosphorylation | Metabolism | CS score, function | |
| Uqcrh | 66576 | UQCRH | 7388 | -1.28 | oxidative phosphorylation | Metabolism | CS score, function | |
| Urb2 | 382038 | URB2 | 9816 | -2.25 | molecular function | Metabolism | CS score, function | |
| Vmp1 | 75909 | VMP1 | 81671 | -1.75 | exocytosis | Metabolism | CS score, function | |
| n/a | n/a | VPS28 | 51160 | -3.06 | protein targeting to vacuole involved in ubiquitin-dependent protein catabolic process via the multivesicular body sorting pathway | Metabolism | CS score, function | |
| Vps29 | 56433 | VPS29 | 51699 | -2.05 | intracellular protein transport | Metabolism | CS score, function | |
| Vps52 | 224705 | VPS52 | 6293 | -1.85 | ectodermal cell differentiation | Metabolism | CS score, mouse K.O., function | Sugimotc M, et al. Cell Rep. 2012 Nov 29;2(5):1363-74 |
| Wars2 | 70560 | WARS2 | 10352 | -1.16 | vasculogenesis | Metabolism | CS score, function | |
| Wdr7 | 104082 | WDR7 | 23335 | -1.47 | hematopoietic progenitor cell differentiation | Metabolism | CS score, function | |
| Wdr70 | 545085 | WDR70 | 55100 | -1.69 | enzyme binding | Metabolism | CS score, function | |
| Wdr74 | 107071 | WDR74 | 54663 | -2.84 | blastocyst formation | Metabolism | CS score, function | |
| Wdr77 | 70465 | WDR77 | 79084 | -2.19 | spliceosomal snRNP assembly | Metabolism | CS score, mouse K.O., function | Zhou L, et al. J Mol Endocrinol. 2006 Oct;37(2):283-300 |
| Yae1d1 | 67008 | YAE1D1 | 57002 | -1.71 | molecular function | Metabolism | CS score, function | |
| Yrdc | 230734 | YRDC | 79693 | -2.33 | negative regulation of transport | Metabolism | CS score, function | |
| Znhit2 | 29805 | ZNHIT2 | 741 | -2.70 | metal ion binding | Metabolism | CS score, function | |
| Aars | 234734 | AARS | 16 | -2.48 | alanyl-tRNA aminoacylation | RNA transcription, protein translation | CS score, function | |
| Bms1 | 213895 | BMS1 | 9790 | -1.36 | ribosome assembly | RNA transcription, protein translation | CS score, function | |
| Bud31 | 231889 | BUD31 | 8896 | -2.46 | mRNA splicing, via spliceosome | RNA transcription, protein translation | CS score, function | |
| Bysl | 53414 | BYSL | 705 | -2.24 | maturation of SSU-rRNA from tricistronic rRNA transcript (SSU-rRNA, 5.8S rRNA, LSU-rRNA) | RNA transcription, protein translation | CS score, mouse K.O., function | Aoki R, et al. FEBS Lett. 2006 Nov 13;580(26):6062-8 |
| Cars | 27267 | CARS | 833 | -2.45 | tRNA aminoacylation for protein translation | RNA transcription, protein translation | CS score, function | |
| Cdc5l | 71702 | CDC5L | 988 | -2.09 | mRNA splicing, via spliceosome | RNA transcription, protein translation | CS score, function | |
| Cdc73 | 214498 | CDC73 | 79577 | -258 | negative regulation of transcription from RNA polymerase II promoter | RNA transcription, protein translation | CS score, mouse K.O., function | Wang P, et al. Mol Cell Biol. 2008 May;28(9):2930-40 |
| Cebpz | 12607 | CEBPZ | 10153 | -2.11 | transcription from RNA polymerase II promoter | RNA transcription protein translation | CS score, function | |
| Clasrp | 53609 | CLASRP | 11129 | -1.30 | mRNA processing | RNA transcription, protein translation | CS score, function | |
| Clp1 | 98985 | CLP1 | 10978 | -3.47 | mRNA splicing, via spliceosome | RNA transcription, protein translation | CS score, mouse K.O., function | Hanada T, et al. Nature. 2013 Mar 28;495(7442):474-80 |
| Cox5b | 12859 | COX5B | 1329 | -1.50 | transcription initiation from RNA polymerase II promoter | RNA transcription, protein translation | CS score, function | |
| Cpsf1 | 94230 | CPSF1 | 29894 | -2.58 | mRNA splicing, via spliceosome | RNA transcription, protein translation | CS score, function | |
| Cpsf2 | 51786 | CPSF2 | 53981 | -2.55 | mRNA polyadenylation | RNA transcription, protein translation | CS score, function | |
| Cpsf3l | 71957 | CPSF3L | 54973 | -2.09 | snRNA processing | RNA transcription, protein translation | CS score, function | |
| Dars | 226414 | DARS | 1615 | -2.90 | translation | RNA transcription, protein translation | CS score, function | |
| Dbr1 | 83703 | DBR1 | 51163 | -3.75 | RNA splicing, via transesterification reactions | RNA transcription, protein translation | CS score, function | |
| Ddx18 | 66942 | DDX18 | 8886 | -2.33 | RNA secondary structure unwinding | RNA transcription, protein translation | CS score, function | |
| Ddx23 | 74351 | DDX23 | 9416 | -3.01 | RNA secondary structure unwinding | RNA transcription, protein translation | CS score, function | |
| Ddx24 | 27225 | DDX24 | 57062 | -1.40 | RNA secondary structure unwinding | RNA transcription, protein translation | CS score, function | |
| Ddx41 | 72935 | DDX41 | 51428 | -1.74 | RNA secondary structure unwinding | RNA transcription, protein translation | CS score, function | |
| Ddx46 | 212880 | DDX46 | 9879 | -2.79 | mRNA splicing, via spliceosome | RNA transcription, protein translation | CS score, function | |
| Ddx47 | 67755 | DDX47 | 51202 | -2.20 | RNA secondary structure unwinding | RNA transcription, protein translation | CS score, function | |
| Ddx49 | 234374 | DDX49 | 54555 | -3.20 | RNA secondary structure unwinding | RNA transcription, protein translation | CS score, function | |
| Ddx54 | 71990 | DDX54 | 79039 | -2.94 | RNA secondary structure unwinding | RNA transcription, protein translation | CS score, function | |
| Ddx56 | 52513 | DDX56 | 54606 | -2.85 | rRNA processing | RNA transcription, protein translation | CS score, function | |
| Dgcr14 | 27886 | DGCR14 | 8220 | -1.76 | mRNA splicing, via spliceosome | RNA transcription, protein translation | CS score, function | |
| Dhx15 | 13204 | DHX15 | 1665 | -2.58 | mRNA processing | RNA transcription, protein translation | CS score, function | |
| Dhx16 | 69192 | DHX16 | 8449 | -1.35 | mRNA processing | RNA transcription, protein translation | CS score, function | |
| Dhx38 | 64340 | DHX38 | 9785 | -1.76 | mRNA splicing, via spliceosome | RNA transcription, protein translation | CS score, function | |
| Diexf | 215193 | DIEXF | 27042 | -2.03 | maturation of SSU-rRNA from tricistronic rRNA transcript (SSU-rRNA, 5.8S rRNA, LSU-rRNA) | RNA transcription, protein translation | CS score, function | |
| Dimt1 | 66254 | DIMT1 | 27292 | -1.87 | rRNA methylation | RNA transcription, protein translation | CS score, function | |
| Dis3 | 72662 | DIS3 | 22894 | -1.77 | mRNA catabolic process | RNA transcription, protein translation | CS score, function | |
| Dkc1 | 245474 | DKC1 | 1736 | -2.37 | box H/ACA snoRNA 3'-end processing | RNA transcription, protein translation | CS score, mouse K.O., function | He J, et al. Oncogene.2002 Oct 31;21(50):7740-4 |
| Dnajc17 | 69408 | DNAJC17 | 55192 | -2.25 | negative regulation of transcription from RNA polymerase II promoter | RNA transcription, protein translation | CS score, mouse K.O., function | Amendola E, et al. Endocrinology. 2010 pr;151(4):1948-58 |
| Ears2 | 67417 | EARS2 | 124454 | -1.91 | tRNA aminoacylation for protein translation | RNA transcription, protein translation | CS score, function | |
| Ebna1bp2 | 69072 | EBNA1B P2 | 10969 | -1.52 | ribosome biogenesis | RNA transcription, protein translation | CS score, function | |
| Eef1a1 | 13627 | EEF1A1 | 1915 | -3.11 | translational elongation | RNA transcription, protein translation | CS score, function | |
| Eef1g | 67160 | EEF1G | 1937 | -1.42 | translation | RNA transcription, protein translation | CS score, function | |
| Eef2 | 13629 | EEF2 | 1938 | -3.53 | translation | RNA transcription, protein translation | CS score, function | |
| Eftud2 | 20624 | EFTUD2 | 9343 | -3.79 | mRNA splicing, via spliceosome | RNA transcription, protein translation | CS score, function | |
| Eiflad | 69860 | EIF1AD | 84285 | -2.26 | translational initiation | RNA transcription, protein translation | CS score, function | |
| Eif2b1 | 209354 | EIF2B1 | 1967 | -2.23 | regulation of translational initiation | RNA transcription, protein translation | CS score, function | |
| Eif2b3 | 108067 | EIF2B3 | 8891 | -3.00 | translational initiation | RNA transcription, protein translation | CS score, function | |
| Eif2s1 | 13665 | EIF2S1 | 1965 | -3.93 | translation | RNA transcription, protein translation | CS score, function | |
| Eif3c | 56347 | EIF3C | 8663 | -2.59 | formation of translation preinitiation complex | RNA transcription, protein translation | CS score, function | |
| n/a | n/a | EIF3CL | 728689 | -2.71 | formation of translation preinitiation complex | RNA transcription, protein translation | CS score, function | |
| Eif3d | 55944 | EIF3D | 8664 | -3.23 | formation of translation preinitiation complex | RNA transcription, protein translation | CS score, function | |
| Eif3f | 66085 | EIF3F | 8665 | -1.44 | formation of translation preinitiation complex | RNA transcription, protein translation | CS score, function | |
| Eif3g | 53356 | EIF3G | 8666 | -3.10 | translational initiation | RNA transcription, protein translation | CS score, function | |
| Eif3i | 54709 | EIF3I | 8668 | -2.24 | formation of translation preinitiation complex | RNA transcription, protein translation | CS score, function | |
| Eif3l | 223691 | EIF3L | 51386 | -1.28 | translational initiation | RNA transcription, protein translation | CS score, function | |
| Eif4a1 | 13681 | EIF4A1 | 1973 | -1.97 | translational initiation | RNA transcription, protein translation | CS score, function | |
| Eif4a3 | 192170 | EIF4A3 | 9775 | -4.32 | RNA splicing | RNA transcription, protein translation | CS score, function | |
| Eif4g1 | 208643 | EIF4G1 | 1981 | -1.79 | nuclear-transcribed mRNA catabolic process, nonsense-mediated decay | RNA transcription, protein translation | CS score, function | |
| Eif5b | 226982 | EIF5B | 9669 | -2.93 | translational initiation | RNA transcription, protein translation | CS score, function | |
| Eif6 | 16418 | EIF6 | 3692 | -2.75 | mature ribosome assembly | RNA transcription, protein translation | CS score, mouse K.O., function | Gandin V et al. Nature. 2008 Oct 2;455(7213):684-8 |
| Elac2 | 68626 | ELAC2 | 60528 | -2.06 | tRNA 3'-trailer cleavage, endonucleolytic | RNA transcription, protein translation | CS score, function | |
| EII | 13716 | ELL | 8178 | -2.23 | transcription elongation from RNA polymerase II promoter | RNA transcription, protein translation | CS score, mouse K.O., function | Mitani K, et al. Biochem Biophys Res Commun. 2000 Dec 20:279(2):563-7 |
| Etf1 | 225363 | ETF1 | 2107 | -2.44 | translational termination | RNA transcription, protein translation | CS score, function | |
| Exosc2 | 227715 | EXOSC2 | 23404 | -1.66 | exonucleolytic trimming to generate mature 3'-end of 5.8S rRNA from tricistronic rRNA transcript (SSU-rRNA, 5.8S rRNA, LSU-rRNA) | RNA transcription, protein translation | CS score, function | |
| Exosc4 | 109075 | EXOSC4 | 54512 | -3.21 | nuclear-transcribed mRNA catabolic process, deadenylation-dependent decay | RNA transcription, protein translation | CS score, function | |
| Exosc5 | 27998 | EXOSC5 | 56915 | -2.09 | rRNA catabolic process | RNA transcription, protein translation | CS score, function | |
| n/a | n/a | EXOSC6 | 118460 | -3.20 | nuclear-transcribed mRNA catabolic process, deadenylation-dependent decay | RNA transcription, protein translation | CS score, function | |
| Exosc7 | 66446 | EXOSC7 | 23016 | -2.17 | nuclear-transcribed mRNA catabolic process, deadenylation-dependent decay | RNA transcription, protein translation | CS score, function | |
| Exosc8 | 69639 | EXOSC8 | 11340 | -2.08 | nuclear-transcribed mRNA catabolic process, deadenylation-dependent decay | RNA transcription, protein translation | CS score, function | |
| Fars2 | 69955 | FARS2 | 10667 | -1.90 | tRNA aminoacylation for protein translation | RNA transcription, protein translation | CS score, function | |
| Farsa | 66590 | FARSA | 2193 | -3.30 | phenylalanyl-tRNA aminoacylation | RNA transcription, protein translation | CS score, function | |
| Farsb | 23874 | FARSB | 10056 | -2.49 | phenylalanyl-tRNA aminoacylation | RNA transcription, protein translation | CS score, function | |
| Fau | 14109 | FAU | 2197 | -2.64 | translation | RNA transcription, protein translation | CS score, function | |
| Fip1l1 | 66899 | FIP1L1 | 81608 | -1.93 | mRNA processing | RNA transcription, protein translation | CS score, function | |
| Ftsj3 | 56095 | FTSJ3 | 117246 | -1.50 | rRNA methylation | RNA transcription, protein translation | CS score, function | |
| Gle1 | 74412 | GLE1 | 2733 | -1.89 | mRNA export from nucleus | RNA transcription, protein translation | CS score, function | |
| Gnl3l | 237107 | GNL3L | 54552 | -1.35 | ribosome biogenesis | RNA transcription, protein translation | CS score, function | |
| Gtf2e1 | 74197 | GTF2E1 | 2960 | -1.22 | transcriptional open complex formation at RNA polymerase II promoter | RNA transcription, protein translation | CS score, function | |
| Gtpbp4 | 69237 | GTPBP4 | 23560 | -2.25 | ribosome biogenesis | RNA transcription, protein translation | CS score, function | |
| Hars | 15115 | HARS | 3035 | -3.49 | histidyl-tRNA aminoacylation | RNA transcription, protein translation | CS score, function | |
| Hars2 | 70791 | HARS2 | 23438 | -1.92 | histidyl-tRNA aminoacylation | RNA transcription, protein translation | CS score, function | |
| Heatr1 | 217995 | HEATR1 | 55127 | -2.58 | maturation of SSU-rRNA from tricistronic rRNA transcript (SSU-rRNA, 5.8S rRNA, LSU-rRNA) | RNA transcription, protein translation | CS score, function | |
| Hnrnpc | 15381 | HNRNPC | 3183 | -1.95 | mRNA splicing, via spliceosome | RNA transcription, protein translation | CS score, mouse K.O., function | Williamsc n DJ, et al. Mol Cell Biol. 2000 Jun;20(11):4094-105 |
| Hnrnpk | 15387 | HNRNPK | 3190 | -2.39 | mRNA splicing, via spliceosome | RNA transcription, protein translation | CS score, function | |
| Hnrnpl | 15388 | HNRNPL | 3191 | -1.88 | mRNA processing | RNA transcription, protein translation | CS score, mouse K.O., function | Gaudreau MC, et al. J Immunol. 2012 Jun 1;188(11):5377-88 |
| Hnrnpu | 51810 | HNRNPU | 3192 | -2.44 | mRNA splicing, via spliceosome | RNA transcription, protein translation | CS score, mouse K.O., function | Roshon MJ, et al. Transgenic Res 2005 Apr 14(2):179-92 |
| lars | 105148 | IARS | 3376 | -3.87 | isoleucyl-tRNA aminoacylation | RNA transcription, protein translation | CS score, function | |
| lars2 | 381314 | IARS2 | 55699 | -2.83 | tRNA aminoacylation for protein translation | RNA transcription, protein translation | CS score, function | |
| Imp3 | 102462 | IMP3 | 55272 | -3.46 | rRNA processing | RNA transcription, protein translation | CS score, function | |
| Imp4 | 27993 | IMP4 | 92856 | -2.01 | rRNA processing | RNA transcription, protein translation | CS score, function | |
| Ints1 | 68510 | INTS1 | 26173 | -1.93 | snRNA processing | RNA transcription, protein translation | CS score, mouse K.O., function | Nakayama M, et al. FASEB J 2006 Aug;20(10):1718-20 |
| Ints4 | 101861 | INTS4 | 92105 | -1.75 | snRNA processing | RNA transcription, protein translation | CS score, function | |
| Ints5 | 109077 | INTS5 | 80789 | -2.10 | snRNA processing | RNA transcription, protein translation | CS score, function | |
| Ints8 | 72656 | INTS8 | 55656 | -1.35 | snRNA processing | RNA transcription, protein translation | CS score, function | |
| Ints9 | 210925 | INTS9 | 55756 | -2.26 | snRNA processing | RNA transcription, protein translation | CS score, function | |
| Isg20I2 | 229504 | ISG20L2 | 81875 | -2.27 | ribosome biogenesis | RNA transcription, protein translation | CS score, function | |
| Kars | 85305 | KARS | 3735 | -2.76 | tRNA aminoacylation for protein translation | RNA transcription, protein translation | CS score, function | |
| n/a | n/a | KIAA0391 | 9692 | -1.56 | tRNA processing | RNA transcription, protein translation | CS score, function | |
| Lars | 107045 | LARS | 51520 | -1.83 | tRNA aminoacylation for protein translation | RNA transcription, protein translation | CS score, function | |
| Lars2 | 102436 | LARS2 | 23395 | -1.60 | tRNA aminoacylation for protein translation | RNA transcription, protein translation | CS score, function | |
| Las1l | 76130 | LAS1L | 81887 | -2.12 | rRNA processing | RNA transcription, protein translation | CS score, function | |
| Lrpprc | 72416 | LRPPRC | 10128 | -1.39 | negative regulation of mitochondrial RNA catabolic process | RNA transcription, protein translation | CS score, mouse K.O., function | Ruzzenente B, et al. EMBO J. 2012 Jan 18;31(2):443-56 |
| Lsm2 | 27756 | LSM2 | 57819 | -2.96 | nuclear-transcribed mRNA catabolic process, deadenylation-dependent decay | RNA transcription, protein translation | CS score, function | |
| Lsm3 | 67678 | LSM3 | 27258 | -1.66 | nuclear-transcribed mRNA catabolic process, deadenylation-dependent decay | RNA transcription, protein translation | CS score, function | |
| Lsm7 | 66094 | LSM7 | 51690 | -1.96 | nuclear-transcribed mRNA catabolic process, deadenylation-dependent decay | RNA transcription, protein translation | CS score, function | |
| Magoh | 17149 | MAGOH | 4116 | -1.78 | nuclear-transcribed mRNA catabolic process, nonsense-mediated decay | RNA transcription, protein translation | CS score, mouse K.O., function | Silver DL et al. Nat Neurosci 2010 May;13(5):551-8 |
| Mars | 216443 | MARS | 4141 | -3.24 | methionyl-tRNA aminoacylation | RNA transcription, protein translation | CS score, function | |
| Mars2 | 212679 | MARS2 | 92935 | -2.31 | tRNA aminoacylation for protein translation | RNA transcription, protein translation | CS score, function | |
| Med17 | 234959 | MED17 | 9440 | -1.78 | regulation of transcription from RNA polymerase II promoter | RNA transcription, protein translation | CS score, function | |
| Med20 | 56771 | MED20 | 9477 | -2.00 | regulation of transcription from RNA polymerase II promoter | RNA transcription, protein translation | CS score, function | |
| Med22 | 20933 | MED22 | 6837 | -1.86 | regulation of transcription from RNA polymerase II promoter | RNA transcription, protein translation | CS score, function | |
| Med27 | 68975 | MED27 | 9442 | -1.48 | regulation of transcription from RNA polymerase II promoter | RNA transcription, protein translation | CS score, function | |
| Med30 | 69790 | MED30 | 90390 | -2.21 | regulation of transcription from | RNA transcription, | CS score, function | |
| | | | | | RNA polymerase II promoter | protein translation | | |
| Med8 | 80509 | MED8 | 112950 | -1.64 | regulation of transcription from RNA polymerase II promoter | RNA transcription, protein translation | CS score, function | |
| Mepce | 231803 | MEPCE | 56257 | -2.08 | negative regulation of transcription from RNA polymerase II promoter | RNA transcription, protein translation | CS score, function | |
| Mettl16 | 67493 | METTL16 | 79066 | -2.10 | rRNA base methylation | RNA transcription, protein translation | CS score, function | |
| Mphosph 10 | 67973 | MPHOSP H10 | 10199 | -1.85 | RNA splicing, via transesterification reactions | RNA transcription, protein translation | CS score, function | |
| Mrpl10 | 107732 | MRPL10 | 124995 | -1.38 | translation | RNA transcription, protein translation | CS score, function | |
| Mrpl12 | 56282 | MRPL12 | 6182 | -1.56 | translation | RNA transcription, protein translation | CS score, function | |
| Mrpl21 | 353242 | MRPL21 | 219927 | -1.91 | translation | RNA transcription, protein translation | CS score, function | |
| Mrpl28 | 68611 | MRPL28 | 10573 | -1.50 | translation | RNA transcription, protein translation | CS score, function | |
| Mrpl3 | 94062 | MRPL3 | 11222 | -1.58 | translation | RNA transcription, protein translation | CS score, function | |
| Mrpl34 | 94065 | MRPL34 | 64981 | -1.66 | translation | RNA transcription, protein translation | CS score, function | |
| Mrpl4 | 66163 | MRPL4 | 51073 | -2.41 | translation | RNA transcription, protein translation | CS score, function | |
| Mrpl41 | 107733 | MRPL41 | 64975 | -2.15 | translation | RNA transcription, protein translation | CS score, function | |
| Mrpl51 | 66493 | MRPL51 | 51258 | -1.40 | translation | RNA transcription, protein translation | CS score, function | |
| Mrps14 | 64659 | MRPS14 | 63931 | -1.82 | translation | RNA transcription, protein translation | CS score, function | |
| Mrps15 | 66407 | MRPS15 | 64960 | -1.28 | translation | RNA transcription, protein translation | CS score, function | |
| Mrps15 Mrps16 | 66242 | MRPS16 | 51021 | -2.29 | translation | RNA transcription, protein translation | CS score, function | |
| Mrps18a | 68565 | MRPS18 A | 55168 | -1.55 | translation | RNA transcription, protein translation | CS score, function | |
| Mrps2 | 118451 | MRPS2 | 51116 | -1.59 | translation | RNA transcription, protein translation | CS score, function | |
| Mrps21 | 66292 | MRPS21 | 54460 | -1.51 | translation | RNA transcription, protein translation | CS score, function | |
| Mrps24 | 64660 | MRPS24 | 64951 | -1.71 | translation | RNA transcription, protein translation | CS score, function | |
| Mrps6 | 121022 | MRPS6 | 64968 | -1.65 | translation | RNA transcription, protein translation | CS score, function | |
| Nars | 70223 | NARS | 4677 | -3.31 | tRNA aminoacylation for protein translation | RNA transcription, protein translation | CS score, function | |
| Nars2 | 244141 | NARS2 | 79731 | -1.32 | tRNA aminoacylation for protein translation | RNA transcription, protein translation | CS score, function | |
| Ncbp2 | 68092 | NCBP2 | 22916 | -3.00 | mRNA cis splicing, via spliceosome | RNA transcription, protein translation | CS score, function | |
| Nedd8 | 18002 | NEDD8 | 4738 | -2.45 | regulation of transcription from RNA polymerase II promoter | RNA transcription, protein translation | CS score, function | |
| Ngdn | 68966 | NGDN | 25983 | -2.35 | maturation of SSU-rRNA from tricistronic rRNA transcript (SSU-rRNA, 5.8S rRNA, LSU-rRNA) | RNA transcription, protein translation | CS score, function | |
| Nhp2 | 52530 | NHP2 | 55651 | -1.74 | rRNA pseudouridine synthesis | RNA transcription, protein translation | CS score, function | |
| Nip7 | 66164 | NIP7 | 51388 | -2.03 | ribosome assembly | RNA transcription, protein translation | CS score, function | |
| Noc2l | 57741 | NOC2L | 26155 | -2.34 | negative regulation of transcription from RNA polymerase II promoter | RNA transcription, protein translation | CS score, function | |
| Noc4l | 100608 | NOC4L | 79050 | -2.11 | ribosome biogenesis | RNA transcription, protein translation | CS score, function | |
| Nol6 | 230082 | NOL6 | 65083 | -2.28 | rRNA processing | RNA transcription, protein translation | CS score, function | |
| Nol9 | 74035 | NOL9 | 79707 | -2.20 | cleavage in ITS2 between 5.8S rRNA and LSU-rRNA of tricistronic rRNA transcript (SSU-rRNA, 5.8S rRNA, LSU-rRNA) | RNA transcription, protein translation | CS score, function | |
| Nop16 | 28126 | NOP16 | 51491 | -2.10 | ribosomal large subunit biogenesis | RNA transcription, protein translation | CS score, function | |
| Nop2 | 110109 | NOP2 | 4839 | -2.14 | rRNA processing | RNA transcription, protein translation | CS score, function | |
| Nop58 | 55989 | NOP58 | 51602 | -2.54 | rRNA modification | RNA transcription, protein translation | CS score, function | |
| Nsa2 | 59050 | NSA2 | 10412 | -1.78 | rRNA processing | RNA transcription, protein translation | CS score, function | |
| Nudt21 | 68219 | NUDT21 | 11051 | -2.36 | mRNA polyadenylation | RNA transcription, protein translation | CS score, function | |
| Osgep | 66246 | OSGEP | 55644 | -1.98 | tRNA processing | RNA transcription, protein translation | CS score, function | |
| Pabpn1 | 54196 | PABPN1 | 8106 | -1.92 | mRNA splicing, via spliceosome | RNA transcription, protein translation | CS score, function | |
| Pdcd11 | 18572 | PDCD11 | 22984 | -1.47 | rRNA processing | RNA transcription, protein translation | CS score, function | |
| Pes1 | 64934 | PES1 | 23481 | -2.92 | maturation of LSU-rRNA from tricistronic rRNA transcript (SSU-rRNA, 5.8S rRNA, LSU-rRNA) | RNA transcription, protein translation | CS score, mouse K.O., function | Lerch-Gaggl A, et al. J Biol Chem. 2002 Nov 22;277(47):45347-55 |
| Phb | 18673 | PHB | 5245 | -2.26 | regulation of transcription from RNA polymerase II promoter | RNA transcription, protein translation | CS score, mouse K.O., function | He B, et al. Endocrinology. 2011 Mar;152(3):1047-56 |
| Phf5a | 68479 | PHF5A | 84844 | -3.52 | mRNA splicing, via spliceosome | RNA transcription, protein translation | CS score, function | |
| Pnn | 18949 | PNN | 5411 | -1.34 | mRNA splicing, via spliceosome | RNA transcription, protein translation | CS score, mouse K.O., function | Joo JH, et al. Dev Dyn 2007 Aug;236(3):2147-58 |
| Polr1b | 20017 | POLR1B | 84172 | -3.23 | transcription from RNA polymerase I promoter | RNA transcription, protein translation | CS score, mouse KO , function | Chen H, et al. Biochem Biophys Res Commun. 2008 Jan 25;365(4):636-42 |
| Polr1c | 20016 | POLR1C | 9533 | -2.79 | transcription from RNA polymerase I promoter | RNA transcription, protein translation | CS score, function | |
| Polr2a | 20020 | POLR2A | 5430 | -3.15 | transcription from RNA polymerase II promoter | RNA transcription, protein translation | CS score, function | |
| Polr2b | 231329 | POLR2B | 5431 | -3.09 | transcription *from* RNA polymerase II promoter | RNA transcription, protein translation | CS score, function | |
| Polr2c | 20021 | POLR2C | 5432 | -3.15 | mRNA splicing, via spliceosome | RNA transcription, protein translation | CS score, function | |
| Polr2d | 69241 | POLR2D | 5433 | -2.23 | nuclear-transcribed mRNA catabolic process, deadenylation-dependent decay | RNA transcription, protein translation | CS score, function | |
| Polr2f | 69833 | POLR2F | 5435 | -2.31 | transcription from RNA polymerase I promoter | RNA transcription, protein translation | CS score, function | |
| Polr2g | 67710 | POLR2G | 5436 | -2.78 | nuclear-transcribed mRNA catabolic process, exonucleolytic | RNA transcription, protein translation | CS score, function | |
| Polr2h | 245841 | POLR2H | 5437 | -1.83 | transcription from RNA polymerase I promoter | RNA transcription, protein translation | CS score, function | |
| Polr2i | 69920 | POLR2I | 5438 | -2.92 | maintenance of transcriptional fidelity during DNA-templated transcription elongation from RNA polymerase II promoter | RNA transcription, protein translation | CS score, function | |
| Polr2i | 20022 | POLR2J | 5439 | -3.31 | mRNA splicing, via spliceosome | RNA transcription, protein translation | CS score, function | |
| Polr2l | 66491 | POLR2L | 5441 | -3.55 | mRNA splicing, via spliceosome | RNA transcription, protein translation | CS score, function | |
| Polr3e | 26939 | POLR3E | 55718 | -2.33 | transcription from RNA polymerase III promoter | RNA transcription, protein translation | CS score, function | |
| Pop1 | 67724 | POP1 | 10940 | -1.79 | tRNA 5'-leader removal | RNA transcription, protein translation | CS score, function | |
| Pop4 | 66161 | POP4 | 10775 | -1.87 | RNA phosphodiester bond hydrolysis | RNA transcription, protein translation | CS score, function | |
| Ppa1 | 67895 | PPA1 | 5464 | -1.63 | tRNA aminoacylation for protein translation | RNA transcription, protein translation | CS score, function | |
| Ppan | 235036 | PPAN | 56342 | -1.62 | ribosomal large subunit assembly | RNA transcription, protein translation | CS score, function | |
| Ppp2ca | 19052 | PPP2CA | 5515 | -3.01 | nuclear-transcribed mRNA catabolic process, nonsense-mediated decay | RNA transcription, protein translation | CS score, mouse K.O., function | Gu P, et al. Genesis. 2012 May;50(5):429-36 |
| Prim1 | 19075 | PRIM1 | 5557 | -2.07 | DNA replication, synthesis of RNA primer | RNA transcription, protein translation | CS score, function | |
| Prpf38b | 66921 | PRPF38B | 55119 | -2.68 | mRNA processing | RNA transcription, protein translation | CS score, function | |
| Prpf4 | 70052 | PRPF4 | 9128 | -2.24 | RNA splicing | RNA transcription, protein translation | CS score, function | |
| Prpf8 | 192159 | PRPF8 | 10594 | -3.43 | mRNA splicing, via spliceosome | RNA transcription, protein translation | CS score, function | |
| Ptcd1 | 71799 | PTCD1 | 26024 | -1.77 | tRNA 3'-end processing | RNA transcription, protein translation | CS score, function | |
| Pwp2 | 110816 | PWP2 | 5822 | -2.52 | ribosomal small subunit assembly | RNA transcription, protein translation | CS score, function | |
| Qars | 97541 | QARS | 5859 | -3.35 | tRNA aminoacylation for protein translation | RNA transcription, protein translation | CS score, function | |
| Ran | 19384 | RAN | 5901 | -3.09 | ribosomal large subunit export from nucleus | RNA transcription, protein translation | CS score, function | |
| Rars | 104458 | RARS | 5917 | -2.30 | tRNA aminoacylation for protein translation | RNA transcription, protein translation | CS score, function | |
| Rars2 | 109093 | RARS2 | 57038 | -1.93 | arginyl-tRNA aminoacylation | RNA transcription, protein translation | CS score, function | |
| Rbm25 | 67039 | RBM25 | 58517 | -2.15 | regulation of alternative mRNA splicing, via spliceosome | RNA transcription, protein translation | CS score, function | |
| Rbm8a | 60365 | RBM8A | 9939 | -2.97 | nuclear-transcribed mRNA catabolic process, nonsense-mediated decay | RNA transcription, protein translation | CS score, function | |
| Rbmx | 19655 | RBMX | 27316 | -1.95 | regulation of alternative mRNA splicing, via spliceosome | RNA transcription, protein translation | CS score, function | |
| Rcl1 | 59028 | RCL1 | 10171 | -2.08 | endonucleolytic cleavage of tricistronic rRNA transcript (SSU-rRNA, 5.8S rRNA, LSU-rRNA) | RNA transcription, protein translation | CS score, function | |
| Rnqtt | 24018 | RNGTT | 8732 | -2.90 | transcription from RNA polymerase II promoter | RNA transcription, protein translation | CS score, function | |
| Rnmt | 67897 | RNMT | 8731 | -1.45 | 7-methylguanosine mRNA capping | RNA transcription, protein translation | CS score, function | |
| Rnpc3 | 67225 | RNPC3 | 55599 | -1.95 | mRNA splicing, via spliceosome | RNA transcription, protein translation | CS score, function | |
| Rpap1 | 68925 | RPAP1 | 26015 | -2.58 | transcription from RNA polymerase II promoter | RNA transcription, protein translation | CS score, function | |
| Rpl10 | 110954 | RPL10 | 6134 | -3.76 | translation | RNA transcription, protein translation | CS score, function | |
| Rpl10a | 19896 | RPL10A | 4736 | -2.15 | nuclear-transcri bed mRNA catabolic process, nonsense-mediated decay | RNA transcription, protein translation | CS score, function | |
| Rpl11 | 67025 | RPL11 | 6135 | -2.99 | translation | RNA transcription, protein translation | CS score, function | |
| Rpl12 | 269261 | RPL12 | 6136 | -2.64 | ribosomal large subunit assembly | RNA transcription, protein translation | CS score, function | |
| Rpl13 | 270106 | RPL13 | 6137 | -3.28 | translation | RNA transcription, protein translation | CS score, function | |
| Rpl14 | 67115 | RPL14 | 9045 | -2.92 | nuclear-transcribed mRNA catabolic process, nonsense-mediated decay | RNA transcription, protein translation | CS score, function | |
| Rpl15 | 66480 | RPL15 | 6138 | -3.50 | translation | RNA transcription, protein translation | CS score, function | |
| Rpl18 | 19899 | RPL18 | 6141 | -3.72 | translation | RNA transcription, protein translation | CS score, function | |
| Rpl18a | 76808 | RPL18A | 6142 | -3.37 | translation | RNA transcription, protein translation | CS score, function | |
| Rpl23 | 65019 | RPL23 | 9349 | -3.02 | translation | RNA transcription, protein translation | CS score, function | |
| n/a | n/a | RPL23A | 6147 | -4.25 | translation | RNA transcription, protein translation | CS score, function | |
| Rpl24 | 68193 | RPL24 | 6152 | -2.55 | ribosomal large subunit assembly | RNA transcription, protein translation | CS score, mouse K.O., function | Oliver ER, et al. Development. 2004 Aug;131(16):3907-20 |
| Rpl26 | 19941 | RPL26 | 6154 | -2.88 | translation | RNA transcription, protein translation | CS score, function | |
| Rpl27 | 19942 | RPL27 | 6155 | -2.25 | translation | RNA transcription, protein translation | CS score, function | |
| Rpl27a | 26451 | RPL27A | 6157 | -2.87 | translation | RNA transcription, protein translation | CS score, mouse K.O., function | Terzian T et al. J Pathol. 2011 Aug;224(4):540-52 |
| Rpl3 | 27367 | RPL3 | 6122 | -3.27 | ribosomal large subunit assembly | RNA transcription, protein translation | CS score, function | |
| Rpl30 | 19946 | RPL30 | 6156 | -2.53 | nuclear-transcribed mRNA catabolic process, nonsense-mediated decay | RNA transcription, protein translation | CS score, function | |
| Rpl31 | 114641 | RPL31 | 6160 | -1.92 | translation | RNA transcription, protein translation | CS score, function | |
| Rpl32 | 19951 | RPL32 | 6161 | -3.70 | nuclear-transcri bed mRNA catabolic process, nonsense-mediated decay | RNA transcription, protein translation | CS score, function | |
| n/a | n/a | RPL34 | 6164 | -2.37 | nuclear-transcribed mRNA catabolic process, nonsense-mediated decay | RNA transcription, protein translation | CS score, function | |
| Rpl35 | 66489 | RPL35 | 11224 | -2.25 | nuclear-transcribed mRNA catabolic process, nonsense-mediated decay | RNA transcription, protein translation | CS score, function | |
| Rpl35a | 57808 | RPL35A | 6165 | -3.20 | translation | RNA transcription, protein translation | CS score, function | |
| Rpl36 | 54217 | RPL36 | 25873 | -3.44 | nuclear-transcribed mRNA catabolic process, nonsense-mediated decay | RNA transcription, protein translation | CS score, function | |
| Rpl37 | 67281 | RPL37 | 6167 | -3.02 | translation | RNA transcription, protein translation | CS score, function | |
| Rpl37a | 19981 | RPL37A | 6168 | -2.62 | nuclear-transcribed mRNA catabolic process, nonsense-mediated decay | RNA transcription, protein translation | CS score, function | |
| Rpl38 | 67671 | RPL38 | 6169 | -2.57 | translation | RNA transcription, protein translation | CS score, mouse K.O., function | MORGAN WC, et al. J Hered. 1950 Aug;41(8):208-15 |
| Rpl4 | 67891 | RPL4 | 6124 | -2.67 | nuclear-transcribed mRNA catabolic process, nonsense-mediated decay | RNA transcription, protein translation | CS score, function | |
| Rpl5 | 100503 670 | RPL5 | 6125 | -3.20 | translation | RNA transcription, protein translation | CS score, function | |
| Rpl6 | 19988 | RPL6 | 6128 | -3.07 | translation | RNA transcription, protein translation | CS score, function | |
| Rpl7 | 19989 | RPL7 | 6129 | -2.15 | nuclear-transcribed mRNA catabolic process, nonsense-mediated decay | RNA transcription, protein translation | CS score, function | |
| Rpl7a | 27176 | RPL7A | 6130 | -3.45 | ribosome biogenesis | RNA transcription, protein translation | CS score, function | |
| Rpl7l1 | 66229 | RPL7L1 | 285855 | -1.86 | maturation of LSU-rRNA from tricistronic rRNA transcript (SSU-rRNA, 5.8S rRNA, LSU-rRNA) | RNA transcription, protein translation | CS score, function | |
| Rpl8 | 26961 | RPL8 | 6132 | -4.00 | translation | RNA transcription, protein translation | CS score, function | |
| Rpl9 | 20005 | RPL9 | 6133 | -3.57 | translation | RNA transcription, protein translation | CS score, function | |
| Rplp0 | 11837 | RPLP0 | 6175 | -2.61 | nuclear-transcribed mRNA catabolic process, nonsense-mediated decay | RNA transcription, protein translation | CS score, function | |
| Rpp21 | 67676 | RPP21 | 79897 | -2.96 | tRNA processing | RNA transcription, protein translation | CS score, function | |
| Rpp30 | 54364 | RPP30 | 10556 | -1.79 | tRNA processing | RNA transcription, protein translation | CS score, function | |
| Rps10 | 67097 | RPS10 | 6204 | -2.88 | ribosomal small subunit assembly | RNA transcription, protein translation | CS score, function | |
| Rps11 | 27207 | RPS11 | 6205 | -2.93 | translation | RNA transcription, protein translation | CS score, function | |
| Rps12 | 20042 | RPS12 | 6206 | -3.33 | nuclear-transcribed mRNA catabolic process, nonsense-mediated decay | RNA transcription, protein translation | CS score, function | |
| Rps13 | 68052 | RPS13 | 6207 | -3.13 | translation | RNA transcription, protein translation | CS score, function | |
| n/a | n/a | RPS14 | 6208 | -3.18 | translation | RNA transcription, protein translation | CS score, function | |
| Rps15 | 20054 | RPS15 | 6209 | -3.20 | ribosomal small subunit assembly | RNA transcription, protein translation | CS score, function | |
| Rps15a | 267019 | RPS15A | 6210 | -3.18 | translation | RNA transcription, protein translation | CS score, function | |
| Rps16 | 20055 | RPS16 | 6217 | -2.35 | translation | RNA transcription, protein translation | CS score, function | |
| Rps17 | 20068 | RPS17 | 6218 | -2.69 | ribosomal small subunit assembly | RNA transcription, protein translation | CS score, function | |
| Rps19 | 20085 | RPS19 | 6223 | -3.49 | translation | RNA transcription, protein translation | CS score, mouse K.O., function | Matsson H, et al. Mol Cell Biol. 2004 May;24(9):4032-7 |
| Rps2 | 16898 | RPS2 | 6187 | -2.50 | translation | RNA transcription, protein translation | CS score, function | |
| Rps21 | 66481 | RPS21 | 6227 | -1.84 | nuclear -transcribed mRNA catabolic process, nonsense-mediated decay | RNA transcription, protein translation | CS score, function | |
| Rps23 | 66475 | RPS23 | 6228 | -2.86 | translation | RNA transcription, protein translation | CS score, function | |
| Rps25 | 75617 | RPS25 | 6230 | -2.38 | nuclear-transcribed mRNA catabolic process, nonsense-mediated decay | RNA transcription, protein translation | CS score, function | |
| n/a | n/a | RPS3A | 6189 | -3.72 | translation | RNA transcription, protein translation | CS score, function | |
| Rps4x | 20102 | RPS4X | 6191 | -3.04 | translation | RNA transcription, protein translation | CS score, function | |
| Rps5 | 20103 | RPS5 | 6193 | -2.61 | translation | RNA transcription, protein translation | CS score, function | |
| Rps6 | 20104 | RPS6 | 6194 | -3.31 | translation | RNA transcription, protein translation | CS score, function | |
| Rps7 | 20115 | RPS7 | 6201 | -2.97 | nuclear -transcribed mRNA catabolic process, nonsense-mediated decay | RNA transcription, protein translation | CS score, function | |
| Rps8 | 20116 | RPS8 | 6202 | -3.44 | nuclear -transcribed mRNA catabolic process, nonsense-mediated decay | RNA transcription, protein translation | CS score, function | |
| Rps9 | 76846 | RPS9 | 6203 | -3.16 | translation | RNA transcription, protein translation | CS score, function | |
| Rpsa | 16785 | RPSA | 3921 | -3.06 | ribosomal small subunit assembly | RNA transcription, protein translation | CS score, mouse K.O., function | Han J, et al. MGI Direct Data Submission. 2008 |
| Rsl24d1 | 225215 | RSL24D1 | 51187 | -2.76 | translation | RNA transcription, protein translation | CS score, function | |
| Sars | 20226 | SARS | 6301 | -2.67 | tRNA aminoacylation for protein translation | RNA transcription, protein translation | CS score, function | |
| Sars2 | 71984 | SARS2 | 54938 | -2.25 | seryl-tRNA aminoacylation | RNA transcription, protein translation | CS score, function | |
| Sart1 | 20227 | SART1 | 9092 | -2.13 | maturation of 5S rRNA | RNA transcription, protein translation | CS score, function | |
| Sart3 | 53890 | SART3 | 9733 | -1.88 | RNA processing | RNA transcription, protein translation | CS score, function | |
| Sdad1 | 231452 | SDAD1 | 55153 | -1.96 | ribosomal large subunit export from nucleus | RNA transcription, protein translation | CS score, function | |
| Sf1 | 22668 | SF1 | 7536 | -3.04 | mRNA splicing, via spliceosome | RNA transcription, protein translation | CS score, mouse K.O., function | Shitashige M, et al. Cancer Sci. 2007 Dec;98(12):1862-7 |
| Sf3a1 | 67465 | SF3A1 | 10291 | -3.18 | mRNA 3'-splice site recognition | RNA transcription, protein translation | CS score, function | |
| Sf3a2 | 20222 | SF3A2 | 8175 | -2.66 | mRNA 3'-splice site recognition | RNA transcription, protein translation | CS score, function | |
| Sf3a3 | 75062 | SF3A3 | 10946 | -2.26 | RNA splicing, via transesterification reactions | RNA transcription, protein translation | CS score, function | |
| Sf3b2 | 319322 | SF3B2 | 10992 | -2.51 | mRNA splicing, via spliceosome | RNA transcription, protein translation | CS score, function | |
| Sf3b3 | 101943 | SF3B3 | 23450 | -4.13 | RNA splicing, via transesterification reactions | RNA transcription, protein translation | CS score, function | |
| Sf3b4 | 107701 | SF3B4 | 10262 | -2.60 | RNA splicing, via transesterification reactions | RNA transcription, protein translation | CS score, function | |
| Sfpq | 71514 | SFPQ | 6421 | -2.27 | negative regulation of transcription from RNA polymerase II promoter | RNA transcription, protein translation | CS score, function | |
| Sin3a | 20466 | SIN3A | 25942 | -1.74 | negative regulation of transcription from RNA polymerase II promoter | RNA transcription, protein translation | CS score, mouse K.O., function | Dannenberg JH, et al. Genes Dev. 2005 Jul 1;19(13):1581-95 |
| Smg5 | 229512 | SMG5 | 23381 | -2.35 | nuclear -transcribed mRNA catabolic process, nonsense-mediated decay | RNA transcription, protein translation | CS score, function | |
| Smg6 | 103677 | SMG6 | 23293 | -1.18 | nuclear -transcribed mRNA catabolic process, nonsense-mediated decay | RNA transcription, protein translation | CS score, function | |
| Snrnp25 | 78372 | SNRNP2 5 | 79622 | -2.43 | mRNA processing | RNA transcription, protein translation | CS score, function | |
| Snrnp27 | 66618 | SNRNP2 7 | 11017 | -1.36 | mRNA processing | RNA transcription, protein translation | CS score, function | |
| Snrpd2 | 107686 | SNRPD2 | 6633 | -2.47 | RNA splicing | RNA transcription, protein translation | CS score, function | |
| Snrpf | 69878 | SNRPF | 6636 | -3.58 | mRNA splicing, via spliceosome | RNA transcription, protein translation | CS score, function | |
| Srrm1 | 51796 | SRRM1 | 10250 | -1.81 | mRNA processing | RNA transcription, protein translation | CS score, function | |
| Srsf1 | 110809 | SRSF1 | 6426 | -2.75 | mRNA 5'-splice site recognition | RNA transcription, protein translation | CS score, mouse K.O., function | Xu X, et al. Cell. 2005 Jan 14;120(1):59-72 |
| Srsf2 | 20382 | SRSF2 | 6427 | -3.66 | regulation of alternative mRNA splicing, via spliceosome | RNA transcription, protein translation | CS score, mouse K.O., function | Ding JH, et al. EMBO J. 2004 Feb 25;23(4):885-96 |
| Srsf3 | 20383 | SRSF3 | 6428 | -2.28 | mRNA splicing, via spliceosome | RNA transcription, protein translation | CS score, mouse K.O., function | Jumaa H et al. Curr Biol. 1999 Aug 26;9(16):899-902 |
| Srsf7 | 225027 | SRSF7 | 6432 | -2.06 | mRNA splicing, via spliceosome | RNA transcription, protein translation | CS score, function | |
| Ssu72 | 68991 | SSU72 | 29101 | -2.57 | mRNA polyadenylation | RNA transcription, protein translation | CS score, function | |
| Sugp1 | 70616 | SUGP1 | 57794 | -1.36 | RNA processing | RNA transcription, protein translation | CS score, function | |
| Tars | 110960 | TARS | 6897 | -2.53 | tRNA aminoacylation for protein translation | RNA transcription, protein translation | CS score, function | |
| Tars2 | 71807 | TARS2 | 80222 | -1.91 | threonyl-tRNA aminoacylation | RNA transcription, protein translation | CS score, function | |
| Tbl3 | 213773 | TBL3 | 10607 | -2.41 | maturation of SSU-rRNA from tricistronic rRNA transcript (SSU-rRNA, 5.8S rRNA, LSU-rRNA) | RNA transcription, protein translation | CS score, function | |
| Thoc2 | 331401 | THOC2 | 57187 | -2.52 | mRNA processing | RNA transcription, protein translation | CS score, function | |
| Thoc5 | 107829 | THOC5 | 8563 | -1.57 | mRNA processing | RNA transcription, protein translation | CS score, mouse K.O., function | Mancini A, et al. BMC Biol 2010;8:1 |
| Thoc7 | 66231 | THOC7 | 80145 | -2.23 | mRNA processing | RNA transcription, protein translation | CS score, function | |
| Timeless | 21853 | TIMELES S | 8914 | -2.27 | negative regulation of transcription from RNA polymerase II promoter | RNA transcription, protein translation | CS score, mouse K.O., function | Gotter AL, et al. Nat Neurosci. 2000 Aug;3(8):755-6 |
| Tsen2 | 381802 | TSEN2 | 80746 | -1.41 | tRNA-type intron splice site recognition and cleavage | RNA transcription, protein translation | CS score, function | |
| Tsr1 | 104662 | TSR1 | 55720 | -1.76 | ribosome biogenesis | RNA transcription, protein translation | CS score, function | |
| Tsr2 | 69499 | TSR2 | 90121 | -2.82 | maturation of SSU-rRNA from tricistronic rRNA transcript (SSU-rRNA, 5.8S rRNA, LSU-rRNA) | RNA transcription, protein translation | CS score, function | |
| Tufm | 233870 | TUFM | 7284 | -1.92 | translational elongation | RNA transcription, protein translation | CS score, function | |
| Tut1 | 70044 | TUT1 | 64852 | -2.65 | mRNA polyadenylation | RNA transcription, protein translation | CS score, function | |
| Twistnb | 28071 | TWISTNB | 221830 | -2.17 | transcription from RNA polymerase I promoter | RNA transcription, protein translation | CS score, function | |
| U2af1 | 108121 | U2AF1 | 7307 | -2.41 | mRNA splicing, via spliceosome | RNA transcription, protein translation | CS score, function | |
| U2af2 | 22185 | U2AF2 | 11338 | -2.80 | mRNA processing | RNA transcription, protein translation | CS score, function | |
| Uba52 | 22186 | UBA52 | 7311 | -2.54 | translation | RNA transcription, protein translation | CS score, function | |
| Ubl5 | 66177 | UBL5 | 59286 | -2.56 | mRNA splicing, via spliceosome | RNA transcription, protein translation | CS score, function | |
| Upf1 | 19704 | UPF1 | 5976 | -2.63 | nuclear-transcribed mRNA catabolic process, nonsense-mediated decay | RNA transcription, protein translation | CS score, mouse K.O., function | Medghalchi SM, et al. Hum Mol Genet. 2001 Jan 15;10(2):99-105 |
| Upf2 | 326622 | UPF2 | 26019 | -2.16 | nuclear-transcribed mRNA catabolic process, nonsense-mediated decay | RNA transcription, protein translation | CS score, mouse K.O., function | Weischenfeldt J, et al. Genes Dev. 2008 May 15;22(10):1381-96 |
| Utp15 | 105372 | UTP15 | 84135 | -1.65 | maturation of SSU-rRNA from tricistronic rRNA transcript (SSU-rRNA, 5.8S rRNA, LSU-rRNA) | RNA transcription, protein translation | CS score, function | |
| Utp20 | 70683 | UTP20 | 27340 | -2.28 | endonucleolytic cleavage in ITS1 to separate SSU-rRNA from 5.8S rRNA and LSU-rRNA from tricistronic rRNA transcript (SSU-rRNA, 5.8S rRNA, LSU-rRNA) | RNA transcription, protein translation | CS score, function | |
| Utp23 | 78581 | UTP23 | 84294 | -2.54 | rRNA processing | RNA transcription, protein translation | CS score, function | |
| Utp3 | 65961 | UTP3 | 57050 | -1.58 | maturation of SSU-rRNA from tricistronic rRNA transcript (SSU-rRNA, 5.8S rRNA, LSU-rRNA) | RNA transcription, protein translation | CS score, function | |
| Utp6 | 216987 | UTP6 | 55813 | -1.99 | maturation of SSU-rRNA from tricistronic rRNA transcript (SSU-rRNA, 5.8S rRNA, LSU-rRNA) | RNA transcription, protein translation | CS score, function | |
| Vars | 22321 | VARS | 7407 | -3.35 | tRNA aminoacylation for protein translation | RNA transcription, protein translation | CS score, function | |
| Wars | 22375 | WARS | 7453 | -2.22 | tryptophanyl-tRNA aminoacylation | RNA transcription, protein translation | CS score, function | |
| Wdr12 | 57750 | WDR12 | 55759 | -2.16 | maturation of LSU-rRNA from tricistronic rRNA transcript (SSU-rRNA, 5.8S rRNA, LSU-rRNA) | RNA transcription, protein translation | CS score, function | |
| Wdr3 | 269470 | WDR3 | 10885 | -2.65 | maturation of SSU-rRNA from tricistronic rRNA transcript (SSU-rRNA, 5.8S rRNA, LSU-rRNA) | RNA transcription, protein translation | CS score, function | |
| Wdr33 | 74320 | WDR33 | 55339 | -2.63 | mRNA polyadenylation | RNA transcription, protein translation | CS score, function | |
| Wdr36 | 225348 | WDR36 | 134430 | -2.04 | rRNA processing | RNA transcription, protein translation | CS score, mouse K.O., function | Gallenberger M, et al. Hum Mol Genet. 2011 Feb 1;20(3):422-35 |
| Wdr46 | 57315 | WDR46 | 9277 | -2.41 | maturation of SSU-rRNA from tricistronic rRNA transcript (SSU-rRNA, 5.8S rRNA, LSU-rRNA) | RNA transcription, protein translation | CS score, function | |
| Wdr61 | 66317 | WDR61 | 80349 | -2.63 | nuclear-transcribed mRNA catabolic process, exonucleolytic, 3'-5' | RNA transcription, protein translation | CS score, function | |
| Wdr75 | 73674 | WDR75 | 84128 | -2.12 | regulation of transcription from RNA polymerase II promoter | RNA transcription, protein translation | CS score, function | |
| Xpo1 | 103573 | XPO1 | 7514 | -3.50 | ribosomal large subunit export from nucleus | RNA transcription, protein translation | CS score, function | |
| Yars | 107271 | YARS | 8565 | -2.78 | tRNA aminoacylation for protein translation | RNA transcription, protein translation | CS score, function | |
| Yars2 | 70120 | YARS2 | 51067 | -2.40 | translation | RNA transcription, protein translation | CS score, function | |
| Ythdc1 | 231386 | YTHDC1 | 91746 | -2.35 | mRNA splice site selection | RNA transcription, protein translation | CS score, function | |
| Zbtb8os | 67106 | ZBTB8O S | 339487 | -2.54 | tRNA splicing, via endonucleolytic cleavage and ligation | RNA transcription, , protein translation | CS score, function | |
| Zc3h3 | 223642 | ZC3H3 | 23144 | -1.22 | mRNA polyadenylation | RNA transcription protein translation | CS score, function | |

### Documents Cited

Rossant J, Nagy A. Nature Biotechnology, Jan., pp. 23. (1999)
Thomson JA et al. Science. 282(5391):1145. (1998)
Takahashi K, Yamanaka S. Cell. 126(4):663. (2006)
Takahashi K et al. Cell. 131(5):861. (2007)
Hussein SM et al. Nature. 471(7336):58. (2011)
Laurent LC et al. Stem Cell. 8(1): 106. (2011)
Lister R et al. Nature. 471(7336):68. (2011)
Yoshida Y, Yamanaka S. Circulation. 122(1):80. (2010)
Choo AB et al. STEM CELLS. 26(6):1454. (2008)
Tan HL et al. STEM CELLS. 27(8):1792. (2009)
Ben-David U, Nudel N, Benvenisty N. Nature Communications. 4:. (2013a)
Fong CY, Peh GSL, Gauthaman K, Bongso A. Stem Cell Rev and Rep. 5(1):72. (2009)
Tang C et al. Nature Biotechnology. 29(9):829. (2011)
Blum B, Bar-Nur O, Golan-Lev T, Benvenisty N. Nature Biotechnology. 27(3):281. (2009)
Menendez S et al. Aging Cell. 11(1):41. (2012)
Chung S et al. J Neurochem. 97(5):1467. (2006)
Eiges R et al. Current Biology. 11(7):514. (2001)
Huber I et al. The FASEB Journal. 21(10):2551. (2007)
Rong Z, Fu X, Wang M, Xu Y. Journal of Biological Chemistry. 287(39):32338. (2012)
Schuldiner M, Itskovitz-Eldor J, Benvenisty N. STEM CELLS. 21(3):257. (2003)
Ben-David U et al. Cell Stem Cell. 12(2):167. (2013b)
Dabir DV et al. Developmental Cell. 25(1):81. (2013)
Tohyama S et al. Stem Cell. 12(1):127. (2013)
Ghosh Z et al. Cancer Research. 71(14):5030. (2011)
Ben-David U, Benvenisty N. Nat Protoc. 9(3):729. (2014)
Lim T-T et al. PLoS ONE. 8(7):e70543. (2013)
Halloran PJ, Fenton RG. Cancer Research. 58(17):3855. (1998)
Tomicic MT, Thust R, Kaina B. Oncogene. 21(14):2141. (2002)
Di Stasi A et al. N Engl J Med. 365(18):1673. (2011)
Santamaria D et al. Nature. 448(7155):811. (2007)
Diril MK et al. Proceedings of the National Academy of Sciences. 109(10):3826. (2012)
Scognamiglio R et al. Cell. 164(4):668. (2016)
Fernandez-Miranda G et al. Development. 138(13):2661. (2011)
Pellettieri J, Sánchez Alvarado A. Annu. Rev. Genet. 41:83. (2007)
Bianconi E et al. Ann. Hum. Biol. 40(6):463. (2013)
Wang T et al. Science. 350(6264): 1096. (2015)
Morgan DO. (2007)
FUNG T, POON R. Seminars in Cell and Developmental Biology. 16(3):335. (2005)
Peters J-M. Mol Cell. 9(5):931. (2002)
Fukagawa T. Front Biosci. 13(13):2705. (2007)
Cassimeris L. Current Opinion in Cell Biology. 11(1):134. (1999)
Satyanarayana A et al. Development. 135(20):3389. (2008)
Kittler R et al. Nature. 432(7020): 1036. (2004)
Harborth J et al. J. Cell. Sci. 114(Pt 24):4557. (2001)
Silk AD, Holland AJ, Cleveland DW. The Journal of Cell Biology. 184(5):677. (2009)
Oda H et al. Molecular and Cellular Biology. 29(8):2278. (2009)
Liu Q et al. Genes & Development. 14(12):1448. (2000)
Martin-McCaffrey L et al. Developmental Cell. 7(5):763. (2004)
Uren AG et al. Curr. Biol. 10(21):1319. (2000)
Leung CG et al. J Exp Med. 204(7):1603. (2007)
Cutts SM et al. Human Molecular Genetics. 8(7):1145. (1999)
Howman EV et al. Proceedings of the National Academy of Sciences. 97(3):1148. (2000)
Chauvière M, Kress C, Kress M. Biochem Biophys Res Commun. 372(4):513. (2008)
Miura K et al. Biochem Biophys Res Commun. 341(1):132. (2006)
Magnaghi P et al. Nat Chem Biol. 9(9):548. (2013)
Smith ED et al. Molecular and Cellular Biology. 24(3):1168. (2004)
Morham SG, Kluckman KD, Voulomanos N, Smithies O. Molecular and Cellular Biology. 16(12):6804. (1996)
Akimitsu N et al. Genes Cells. 8(4):393. (2003)
Jeganathan K et al. The Journal of Cell Biology. 179(2):255. (2007)
Ono T et al. Cell. 115(1):109. (2003)
Bharadwaj R, Qi W, Yu H. J. Biol. Chem. 279(13):13076. (2004)
Parisi G, Fornasari MS, Echave J. FEBS Lett. 562(1-3):1. (2004)
Harborth J, Weber K, Osborn M. J. Biol. Chem. 275(41):31979. (2000)
Krull S et al. Mol. Biol. Cell. 15(9):4261. (2004)
Topper LM et al. Cell Cycle. 1(4):282. (2002)
Zhang Y-W et al. J. Biol. Chem. 284(27):18085. (2009)
Simmons AD et al. Human Molecular Genetics. 8(12):2155. (1999)
Plate M et al. Mol Cells. 29(4):355. (2010)
Franco M et al. Proceedings of the National Academy of Sciences. 95(17):9926. (1998)
Ohno M, Shimura Y. Genes & Development. 10(8):997. (1996)
Achsel T et al. EMBO J. 18(20):5789. (1999)
Shichijo S et al. J Exp Med. 187(3):277. (1998)
Jurica MS et al. RNA. 8(4):426. (2002)
Chari A et al. Cell. 135(3):497. (2008)
Xu C et al. J. Biol. Chem. 281(23):15900. (2006)
Yeh H et al. Genomics. 23(2):443. (1994)
Kokkola T et al. FEBS Left. 585(17):2665. (2011)
Chan CC et al. RNA. 10(2):200. (2004)
Zhou M et al. Proceedings of the National Academy of Sciences. 105(47): 18139. (2008)
Takatsu H et al. J. Biol. Chem. 273(38):24693. (1998)
Chen Y et al. Cell Research. 22(2):333. (2012)
Mansfeld J et al. Nat. Cell Biol. 13(10):1234. (2011)
He F, Wang C-T, Gou L-T. Genet. Mol. Res. 8(4):1466. (2009)
Lieu A-S et al. Int J Oncol. 37(2):429. (2010)
Preuβ E et al. Human Gene Therapy. 21(8):929. (2010)
Neschadim A et al. Molecular Therapy. 20(5):1002. (2012)
Hedley D, Ogilvie L, Springer C. Nat Rev Cancer. 7(11):870. (2007)
Nawa A et al. Anticancer Agents Med Chem. 8(2):232. (2008)
Danks MK et al. Cancer Research. 67(1):22. (2007)
Shah K. Adv. Drug Deliv. Rev. 64(8):739. (2012)
Ramnaraine M et al. Cancer Research. 63(20):6847. (2003)
Denny WA. J. Biomed. Biotechnol. 2003(1):48. (2003)
Ireton GC, McDermott G, Black ME, Stoddard BL. J. Mol. Biol. 315(4):687. (2002)
Humerickhouse R et al. Cancer Research. 60(5):1189. (2000)
Wierdl M et al. Cancer Research. 61(13):5078. (2001)
Kojima A, Hackett NR, Ohwada A, Crystal RG. J. Clin. Invest. 101(8):1789. (1998)
Straathof KC et al. Blood. 105(11):4247. (2005)
Soucek L et al. Cancer Research. 62(12):3507. (2002)
Oricchio E et al. CellReports, pp. 1. (2014)
Olive M et al. J. Biol. Chem. 272(30):18586. (1997)
Urlinger S et al. Proceedings of the National Academy of Sciences. 97(14):7963. (2000)
Agha-Mohammadi S et al. J Gene Med. 6(7):817. (2004)
Gossen M et al. Science. 268(5218):1766. (1995)
Banaszynski LA et al. Cell. 126(5):995. (2006)
Maeder ML et al. Nature Methods. 10(3):243. (2013)
Pollock R, Clackson T. Current Opinion in Biotechnology. 13(5):459. (2002)
Mullick A et al. BMC Biotechnology. 6(1):43. (2006)
No D, Yao TP, Evans RM. Proceedings of the National Academy of Sciences, 93(8):3346. (1996)
Stanley SA et al. Nature Medicine. 21(1):92. (2015)
Rollins CT et al. Proceedings of the National Academy of Sciences. 97(13):7096. (2000)
Cong L et al. Science. 339(6121):819. (2013)
Hsu PD et al. Nature Biotechnology. 31(9):827. (2013)
Ran FA et al. Nat Protoc. 8(11):2281. (2013)
Gertsenstein M et al. PLoS ONE. 5(6):e11260. (2010)
Adewumi O et al. Nature Biotechnology. 25(7):803. (2007)
Guy CT, Cardiff RD, Muller WJ. Molecular and Cellular Biology. 12(3):954. (1992)
Szymczak AL et al. Nature Biotechnology. 22(5):589. (2004)
Nagy A. et al. Manipulating the Mouse Embryo. (2003)

## Claims

1. A method of controlling proliferation of an animal cell, the method comprising:
(a) genetically modifying in an animal cell a cell division locus (CDL), the CDL being one or more loci of Table 2, the genetic modification of the CDL comprising an inducible exogenous activator of regulation of a CDL (EARC) system that comprises an inducible activator-based gene expression system that is operably linked to the CDL; and
(b) controlling proliferation of the genetically modified animal cell comprising the EARC system with an inducer of the inducible activator-based gene expression system.

2. The method of claim 1, wherein the controlling proliferation of the genetically modified animal cell comprises:
(a) permitting proliferation of the genetically modified animal cell by exposing the genetically modified animal to an inducer of the inducible activator-based gene expression system; or
(b) preventing or inhibiting proliferation of the genetically modified animal by maintaining the animal cell in the absence of the inducer of the inducible activator-based gene expression system.

3. The method of claim 1 or 2, wherein the genetic modification of the CDL comprises performing targeted modification of the CDL with a DNA vector comprising the EARC system.

4. The method of any one of claims 1 to 3, wherein the genetic modification of the CDL comprising the EARC system results in activation of the CDL solely in the presence of the inducer.

5. The method of any one of claims 1 to 4, wherein the CDL:
(a) is two or more loci recited in Table 2;
(b) encodes a gene product that functions in one or more of: cell cycle, DNA replication, RNA transcription, protein translation, and metabolism;
(c) is one or more of CDK1, TOP2A, CENPA, BIRC5, and EEF2, wherein preferably the CDL is CDK1; or
(d) is two or more of CDK1, TOP2A, CENPA, BIRC5, and EEF2, wherein preferably the CDL is CDK1 and TOP2A or CDK1 and EEF2.

6. The method of any one of claims 1 to 5, wherein:
(a) the EARC system is a dox-bridge system, a cumate switch inducible system, an ecdysone inducible system, a radio wave inducible system, or a ligand-reversible dimerization system, wherein preferably the EARC system is a dox-bridge system;
(b) the animal cell is a mammalian cell selected from the group consisting of a human, mouse, rat, hamster, guinea pig, cat, dog, cow, horse, deer, elk, bison, oxen, camel, llama, rabbit, pig, goat, sheep, and non-human primate cell or is an avian cell; and/or
(c) the animal cell is or is derived from a pluripotent stem cell, a multipotent cell, or a monopotent progenitor cell, or is a terminally differentiated cell.

7. A method of controlling proliferation of an animal cell population according to the method of any one of claims 1 to 6.

8. An animal cell genetically modified to comprise a mechanism for controlling cell proliferation, the genetically modified animal cell comprising a genetic modification of one or more cell division loci (CDL), the CDL being one or more loci of Table 2, the genetic modification being an exogenous activator of regulation of a CDL (EARC) system comprising an inducible activator-based gene expression system that is operably linked to the CDL.

9. The genetically modified animal cell of claim 8, wherein the genetic modification of the CDL was generated by preforming targeted modification of the CDL with a DNA vector comprising the EARC system.

10. The genetically modified animal cell of claim 8 or 9, wherein the genetic modification results in activation of the CDL solely in the presence of an inducer of the inducible activator-based gene expression system.

11. The genetically modified animal cell of any one of claims 8 to 10, wherein the CDL:
(a) is two or more loci recited in Table 2;
(b) encodes a gene product that functions in one or more of: cell cycle, DNA replication, RNA transcription, protein translation, and metabolism;
(c) is one or more of CDK1, TOP2A, CENPA, BIRC5, and EEF2, wherein preferably the CDL is CDK1; or
(e) is two or more of CDK1, TOP2A, CENPA, BIRC5, and EEF2, wherein preferably the CDL is CDK1 and TOP2A or CDK1 and EEF2.

12. The genetically modified animal cell of any one of claims 8 to 11, wherein:
(a) the EARC system is a dox-bridge system, a cumate switch inducible system, an ecdysone inducible system, a radio wave inducible system, or a ligand-reversible dimerization system, wherein preferably the EARC system is a dox-bridge system;
(b) the animal cell is a mammalian cell selected from the group consisting of a human, mouse, rat, hamster, guinea pig, cat, dog, cow, horse, deer, elk, bison, oxen, camel, llama, rabbit, pig, goat, sheep, and non-human primate cell or is an avian cell; and/or
(c) the animal cell is or is derived from a pluripotent stem cell, a multipotent cell, or a monopotent progenitor cell, or is a terminally differentiated cell.

13. The animal cell of any one of claims 8 to 12 for use in medicine.

14. A DNA vector for modifying expression of an endogenous cell division essential locus (CDL), the CDL being one or more loci of Table 2, comprising an exogenous activator of regulation of a CDL (EARC) system that comprises an inducible activator-based gene expression system that is configured to be operably linked to the CDL.

15. The DNA vector of claim 14, wherein the CDL:
(a) is two or more loci recited in Table 2;
(b) encodes a gene product that functions in one or more of: cell cycle, DNA replication, RNA transcription, protein translation, and metabolism;
(c) is one or more of CDK1, TOP2A, CENPA, BIRC5, and EEF2, wherein preferably the CDL is CDK1; or
(d) is two or more of CDK1, TOP2A, CENPA, BIRC5, and EEF2, wherein preferably the CDL is CDK1 and TOP2A or CDK1 and EEF2.

16. The DNA vector of claim 14 or 15, wherein:
(a) the EARC system is a dox-bridge system, a cumate switch inducible system, an ecdysone inducible system, a radio wave inducible system, or a ligand-reversible dimerization system, wherein preferably the EARC system is a dox-bridge system; and/or
(b) wherein integration of the EARC system into proliferating host cells permits proliferation of the host cells upon exposure of the host cells to an inducer of the inducible activator-based gene expression system.
